(19) Europäisches Patentamt
European Patent Office
Office européen des brevets

(11) EP 4 741 386 A1

(12) **EUROPEAN PATENT APPLICATION**
published in accordance with Art. 153(4) EPC

(43) Date of publication:
**13.05.2026 Bulletin 2026/20**

(21) Application number: **24835258.5**

(22) Date of filing: **25.06.2024**

(51) International Patent Classification (IPC):
***C07D 403/14*** (2006.01) ***C07D 401/12*** (2006.01)
***C07D 401/14*** (2006.01) ***C07D 403/12*** (2006.01)
***C07D 498/04*** (2006.01) ***C07D 487/04*** (2006.01)
***C07D 491/048*** (2006.01) ***C07D 239/47*** (2006.01)
***C07D 413/14*** (2006.01) ***C07D 493/08*** (2006.01)
***A61K 31/506*** (2006.01) ***A61K 31/5383*** (2006.01)
***A61K 31/519*** (2006.01) ***A61K 31/5377*** (2006.01)
***A61P 35/00*** (2006.01)

(52) Cooperative Patent Classification (CPC):
**A61K 31/506; A61K 31/519; A61K 31/5377; A61K 31/5383; A61P 35/00; C07D 239/47; C07D 401/12; C07D 401/14; C07D 403/12; C07D 403/14; C07D 413/14; C07D 487/04; C07D 491/048; C07D 493/08; C07D 498/04**

(86) International application number:
**PCT/CN2024/101346**

(87) International publication number:
**WO 2025/007777 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.07.2023 CN 202310824416**

(71) Applicants:
- **Jiangsu Yahong Meditech Co., Ltd.**
**Taizhou, Jiangsu 225316 (CN)**
- **Asieris Pharmaceuticals (Shanghai) Co., Ltd.**
**Pudong, Shanghai 201203 (CN)**

(72) Inventors:
- **WU, Maojiang**
**Taizhou, Jiangsu 225316 (CN)**
- **MENG, Zhi**
**Taizhou, Jiangsu 225316 (CN)**
- **WANG, Tielin**
**Taizhou, Jiangsu 225316 (CN)**

(74) Representative: **Regimbeau**
**20, rue de Chazelles**
**75847 Paris Cedex 17 (FR)**

(54) **PYRIMIDINE COMPOUND, PREPARATION METHOD THEREFOR AND MEDICAL USE THEREOF**

(57) Disclosed are a pyrimidine compound, a preparation method therefor and a medical use thereof. Specifically, disclosed are a compound represented by the general formula (SI), a preparation method therefor, a pharmaceutical composition containing said compound, and a use thereof as a ubiquitin-specific protease 1 (USP1) inhibitor, particularly a use thereof for treating or preventing cancer. The definitions of the groups in the general formula (SI) are the same as those in the description.

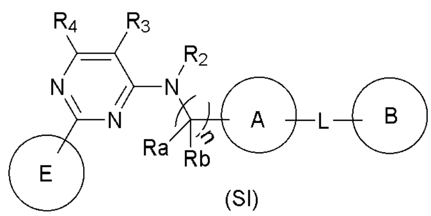

(SI)

EP 4 741 386 A1

**Description**

## FIELD OF THE INVENTION

The present invention relates to a class of compounds with an inhibitory effect on USP1 and compositions comprising the same, and uses thereof in the preparation of a medicament for treating USP1 enzyme-related diseases. Specifically, the present invention relates to a compound of formula (SI) or a pharmaceutically acceptable salt, hydrate, solvate, isotope substitute or stereoisomer thereof.

## BACKGROUND OF THE INVENTION

Ubiquitination, as a dynamic and reversible process, involves a family of deubiquitinases (DUBs). There are approximately 100 DUBs in a human, which can be divided into a cysteine protease family and a metalloproteinase family. Among them, the cysteine protease family mainly includes ubiquitin-specific proteases (USPs), ubiquitin carboxy-terminal hydrolases (UCHs), Machado-Josephin domain proteases (MJDs), MINDY proteases (MINDYs), and ovarian tumor domain proteases (OTUs). The USPs family is the one representing the greatest quantity among known deubiquitinases, with over 50 types encoded by human genes. It plays a role in various physiological functions such as cell cycle, signal transduction, DNA damage repair, chromosome translocation, and gene transcription, by regulating protease substrates.

USP1 belongs to the USP subfamily of DUBs and does not exhibit significant activity on its own. However, it obtains complete enzymatic activity by binding to UAF1 to form a heterodimer complex (USP1/UAF1), and may regulate cellular targets in multiple pathways associated with cancer. For example, the USP1/UAF1 complex enables to deubiquitinate proliferating cell nuclear antigen (PCNA) mono-ubiquitinated by a key protein during translesion synthesis (TLS), to prevent excessive repair thereof, and enables to deubiquitinate fanconi anemia complementation group D2 (FANCD2) mono-ubiquitinated by a key protein in the fanconi anemia (FA) pathway, to prevent improper TLS repair in cells, thereby ensuring genomic stability, etc. These two DNA damage response (DDR) pathways are critical pathways for repairing DNA damage induced by a DNA crosslinker such as cisplatin, mitomycin, and ultraviolet radiation. USP1 can also interact with ID proteins and so on, so as to stabilize their expression in cells through deubiquitination. For example, in osteosarcoma cells, USP1 can deubiquitinate ID1, ID2, and ID3 to promote cell proliferation, and inhibition of USP1 can increase the sensitivity of osteosarcoma cells to chemotherapy. In addition, there are studies to demonstrate that USP1 is closely related to development of resistance to various drugs for tumor treatment. For example, in non-small cell lung cancer (NSCLC) cells with the resistance to cisplatin, USP1 has a high level of expression, and knock down of USP1 can significantly enhance cell sensitivity to cisplatin. In breast cancer cells, USP1 is highly expressed, promotes the proliferation of breast cancer cells and is closely related to the poor prognosis of breast cancer. As reported in the literature (J. Med. Chem. 2014, 57, 8099-8110, Synthesis and Structure-Activity Relationship Studies of N-Benzyl-2-phenylpyrimidin-4-amine Derivatives as Potent USP1/UAF1 Deubiquitinase Inhibitors with Anticancer Activity against Nonsmall Cell Lung Cancer), the compounds such as USP1 inhibitor ML323 can be used for non-small cell lung cancer. As reported in the literature (Cui S-Z, Lei Z-Y, Guan T-P, et al. Targeting USP1-dependent KDM4A protein stability as a potential prostate cancer therapy. Cancer Sci. 2020;00:1-15), USP1 inhibitors are used as potential therapeutic drugs for prostate cancer. In summary, USP1 is expected to become a hot target for treating various cancers and other diseases.

## SUMMARY OF THE INVENTION

The present invention provides a compound of formula (I'),

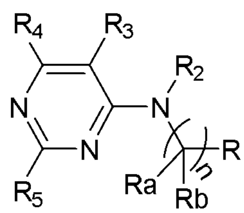

**(I')**

or a pharmaceutically acceptable salt, hydrate, solvate, isotope substitute or stereoisomer thereof, wherein,

R is selected from the group consisting of $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$

hydroxyalkyl, $C_{6-10}$ aryl, $C_{6-10}$ aryl fused with 5 to 10 membered heteroaryl, and

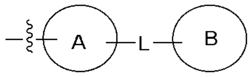,

the above groups are each independently and optionally substituted by one or more $R_1$;
ring A and ring B are each independently selected from the group consisting of $C_{6-10}$ aryl, 5 to 10 membered heteroaryl, $C_{3-8}$ cycloalkyl and 3 to 8 membered heterocyclyl, and ring A and ring B are each independently and optionally substituted by one or more $R_1$;
L is selected from the group consisting of a chemical bond, -O-, -S-, -$C_{1-6}$ alkylene-, -O-$C_{1-6}$ alkylene-, -$C_{1-6}$ alkylene-O-, -S-$C_{1-6}$ alkylene- and -$C_{1-6}$ alkylene-S-;
$R_a$ and $R_b$ are each independently selected from the group consisting of H atom, - CN, $C_{1-6}$ alkyl, -OH, halogen, $C_{2-6}$ alkenyl, $C_{2-6}$ alkynyl, $C_{1-6}$ hydroxyalkyl, $C_{1-6}$ haloalkyl, $C_{1-6}$ alkoxy, $C_{1-6}$ haloalkoxy; or $R_a$ and $R_b$ together form an oxo, $C_{3-8}$ cycloalkyl or 3 to 8 membered heterocyclyl; wherein the $C_{1-6}$ alkyl is optionally substituted by one or more $R_1$;
$R_2$ is selected from the group consisting of H atom, -OH, -CN, $C_{1-6}$ alkyl, -$C_{1-6}$ alkylene-$C_{6-10}$ aryl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{1-6}$ alkyl or -$C_{1-6}$ alkylene-$C_{6-10}$ aryl is optionally substituted by one or more $R_1$;
$R_3$ is selected from the group consisting of H atom, -OH, -COOH, -$NH_2$, -CN, halogen, $C_{1-6}$ alkyl, -S-$C_{1-6}$ alkyl, -S(O)-$C_{1-6}$ alkyl, -$S(O)_2$-$C_{1-6}$ alkyl, phosphoryl, phosphonyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3 to 8 membered heterocyclyl and -$C_{1-6}$ alkylene-C(O)-O-$C_{1-6}$ alkyl, wherein the - $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl are each independently and optionally substituted by one or more $R_1$;
$R_4$ is selected from the group consisting of H atom, -OH, -COOH, -$NH_2$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;
or, $R_2$ and $R_3$ together with the atoms to which they are attached form ring C, the ring C is 5 to 7 membered heteroaryl or 5 to 7 membered heterocyclyl, the heteroatom in the 5 to 7 membered heteroaryl or 5 to 7 membered heterocyclyl is O or N, and further, the 5 to 7 membered heterocyclyl is selected from the group consisting of morpholinyl, 3-morpholinonyl, pyrrolidinyl, 2-oxazolidinonyl and 2-pyrrolidinonyl, and the ring C is optionally substituted by one or more $R_1$;
or, $R_3$ and $R_4$ together with the atoms to which they are attached form ring D, the ring D is 5 to 7 membered heteroaryl or 5 to 7 membered heterocyclyl, and the ring D is optionally substituted by one or more $R_1$;
$R_5$ is selected from the group consisting of $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{6-10}$ aryl fused with 5 to 10 membered heteroaryl, $C_{6-10}$ aryl fused with 3 to 8 membered heterocyclyl, $C_{3-8}$ cycloalkyl and 3 to 8 membered heterocyclyl, wherein the $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl are each independently and optionally substituted by one or more $R_1$;
$R_1$ at each occurrence is independently selected from the group consisting of D atom, -OH, -COOH, -$NH_2$, -CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3 to 8 membered heterocyclyl, -O-$C_{1-6}$alkylene-O-$C_{1-6}$ alkyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of D atom, - OH, -COOH, -$NH_2$, -CN, halogen, $C_{1-6}$ alkyl, $C_{1-6}$ haloalkyl, $C_{1-6}$ alkoxy, $C_{1-6}$ haloalkoxy and $C_{1-6}$ hydroxyalkyl; and
n is an integer from 0 to 8.

In some embodiments, the compound of formula (I') is a compound of formula (I),

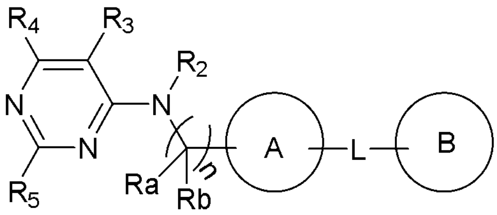

**(I)**

wherein, ring A, ring B, L, $R_a$, $R_b$, $R_2$ to $R_5$ and n are as defined in formula (I').

In some embodiments, provided is the compound of formula (I) or (I'), wherein

$R_2$ is selected from the group consisting of H atom, -OH, -CN, $C_{1-6}$ alkyl, $C_{1-6}$ deuterated alkyl, $C_{2-6}$ alkynyl, -$C_{1-6}$ alkylene-$C_{6-10}$ aryl, $C_{1-6}$ alkoxy, $C_{1-6}$ haloalkyl, $C_{1-6}$ haloalkoxy, $C_{1-6}$ hydroxyalkyl, $C_{3-6}$ cycloalkyl and 5 to 7 membered heterocyclyl, wherein the $C_{1-6}$ alkyl or -$C_{1-6}$ alkylene-$C_{6-10}$ aryl is optionally substituted by one or more $R_1$, $R_1$ is as defined in formula (I');

preferably, $R_2$ is selected from the group consisting of H atom, -CN, methyl, trideuteriomethyl, ethynyl, propynyl, tetrahydrofuranyl, cyclopropyl, methoxy and hydroxy;

and/or

$R_3$ is selected from the group consisting of H atom, -OH, -COOH, -$NH_2$, -CN, halogen, $C_{1-6}$ alkyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{3-8}$ cycloalkyl-O-, 5 to 7 membered heterocyclyl and -$C_{1-6}$ alkylene-C(O)-O-$C_{1-6}$ alkyl, wherein the $C_{1-6}$ hydroxyalkyl and 5 to 7 membered heterocyclyl are each independently and optionally substituted by one or more $C_{1-6}$ alkyl;

preferably, $R_3$ is selected from the group consisting of H atom, methoxy, cyclopropyl-O-, trifluoromethyl, Cl atom, -CN, isopropoxy, ethynyl, difluoromethoxy, morpholinyl,

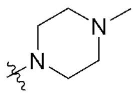

,

-OH,

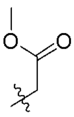

,

F atom, hydroxymethyl and

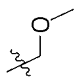

;

and/or

$R_4$ is selected from the group consisting of H atom, -OH, -COOH, -$NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;

preferably, $R_4$ is H atom.

In some embodiments, the compound of formula (I') or (I) is a compound of formula (II),

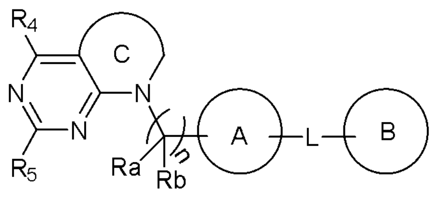

**(II)**

wherein, ring C is selected from the group consisting of

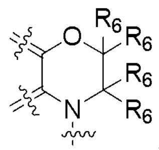, 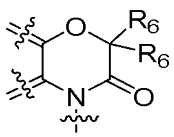,

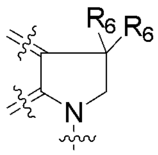, 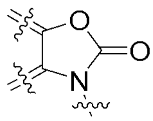 and 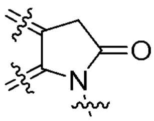;

$R_6$ at each occurrence is independently H or $C_1$-$C_6$ alkyl, or two $R_6$ together with the atoms to which they are attached form a $C_{3-8}$ cycloalkyl or 3 to 8 membered heterocyclyl; and
ring A, ring B, L, $R_a$, $R_b$, $R_4$, $R_5$ and n are as defined in formula (I');
specifically,
$R_4$ is selected from the group consisting of H atom, -OH, -COOH, $-NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; and preferably, $R_4$ is H atom.

In some embodiments, the compound of formula (I') is a compound of formula (III),

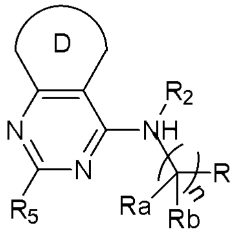

**(III)**

wherein, ring D is selected from the group consisting of

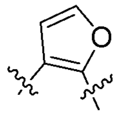, 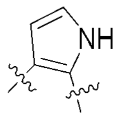,

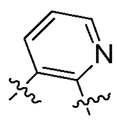, 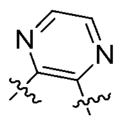 and 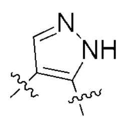,

the above groups are each independently and optionally substituted by one or more $R_1$; and
R, $R_a$, $R_b$, $R_1$, $R_2$, $R_5$ and n are as defined in formula (I');
specifically, $R_2$ is selected from the group consisting of H atom, -OH, $C_{1-6}$ alkyl, $C_{2-6}$ alkynyl, $-C_{1-6}$ alkylene-$C_{6-10}$ aryl,

$C_{1-6}$ alkoxy, $C_{1-6}$ haloalkyl, $C_{1-6}$ haloalkoxy and $C_{1-6}$hydroxyalkyl, wherein the $C_{1-6}$ alkyl or $C_{1-6}$ alkyl-$C_{6-10}$ aryl is optionally substituted by one or more $R_1$, $R_1$ is as defined in formula (I');
preferably, $R_2$ is H atom or

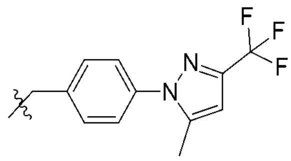
.

In some embodiments, provided is the compound of formula (I'), (I), (II) or (III), wherein

ring A and ring B are each independently selected from the group consisting of phenyl, piperidinyl, cyclohexyl, cyclopropyl, cyclobutyl, pyridinyl, pyrimidinyl, imidazolyl, pyrazolyl, bicyclo[2.2.2]octanyl, 2-oxabicyclo[2.2.2]octanyl, pentacyclooctanyl, isoindolinonyl, imidazo[1,2-a]pyrazinyl, piperidine-2,6-dionyl, thienyl, furanyl, cyclopentyl, pyranyl, pyrrolidinyl, piperazinyl, morpholinyl, naphthyl, pyrrolyl, pyrazinyl, pyridazinyl, triazolyl, tetrazolyl, indolyl, isoindolyl, indolinyl, isoindolinyl, indolinonyl, pyrido[3,2-d]pyrimidinyl, pteridinyl, pyrazolo[4,3-c]pyridinyl, pyrazolo[3,4-d]pyrimidinyl and cubanyl, and ring A and ring B are each independently and optionally substituted by one or more $R_1$, $R_1$ is as defined in formula (I');

L is selected from the group consisting of a chemical bond, -O-, -O-$C_{1-6}$ alkylene and -$C_{1-6}$ alkylene-O-;

specifically,

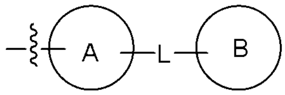

is selected from the group consisting of

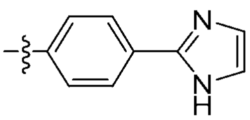 , 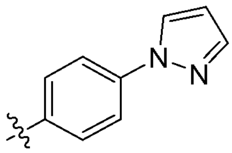 , 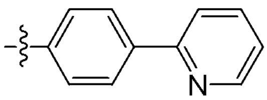 , 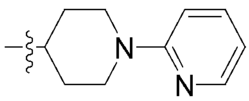 ,

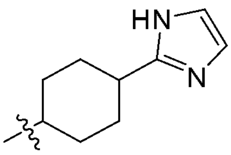 , 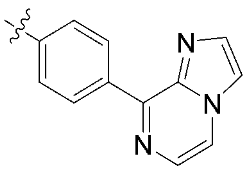 , 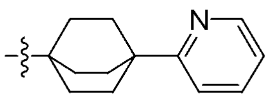 , 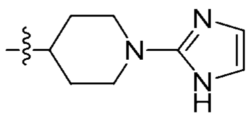 ,

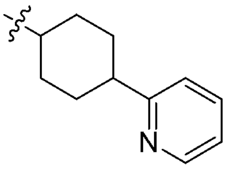 , 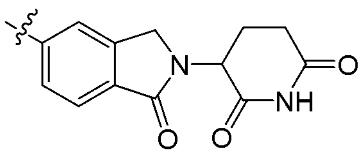 , 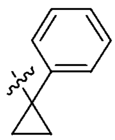 , 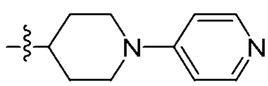 ,

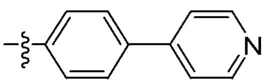 , 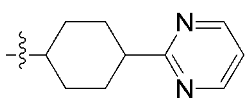 , 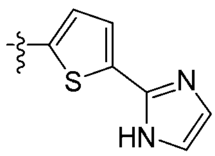 , 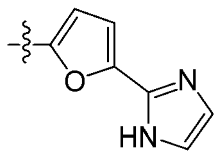 ,

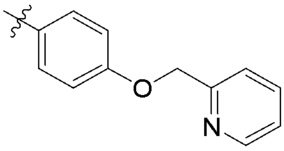, 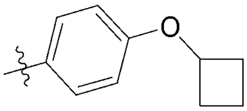, 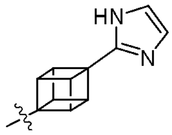, 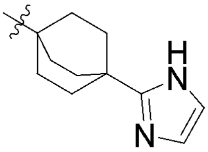,

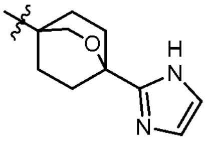 and 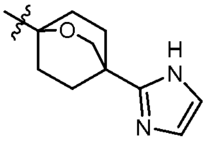,

and ring A and ring B are each independently and optionally substituted by one or more $R_1$, $R_1$ is as defined in formula (I');

more specifically,

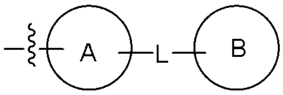

is selected from the group consisting of

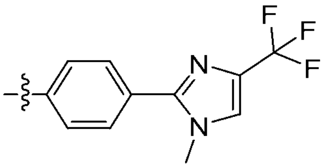, 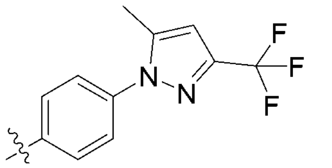, 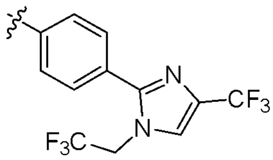,

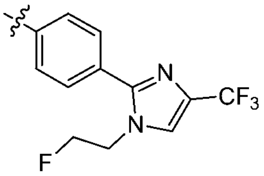, 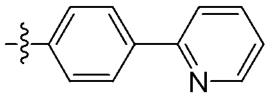, 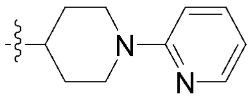,

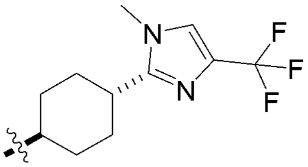, 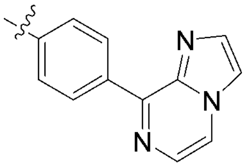, 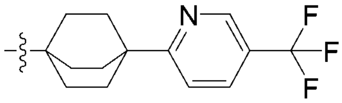,

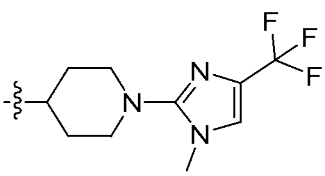, 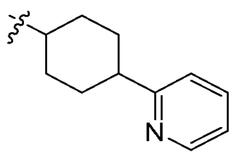, 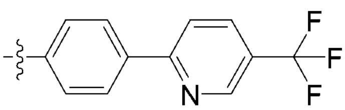,

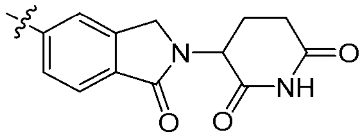, 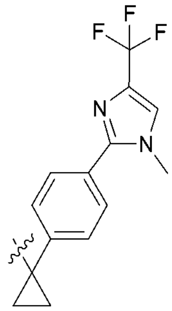, 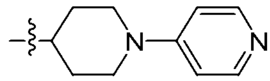,

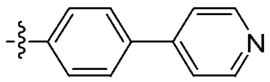, 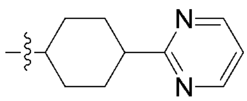, 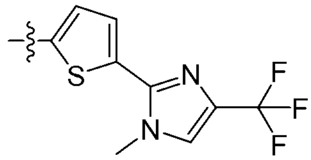,

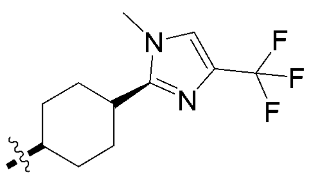, 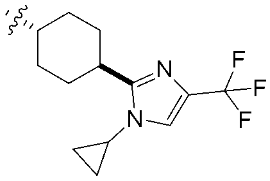, 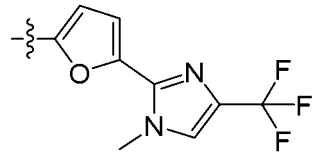,

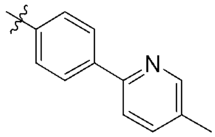, 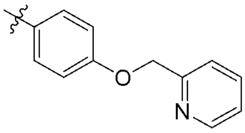, 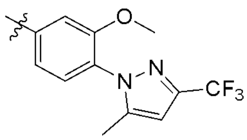, 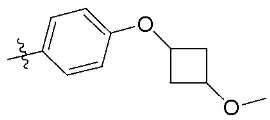,

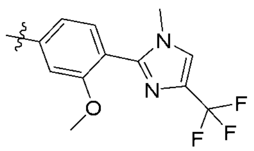, 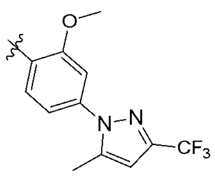, 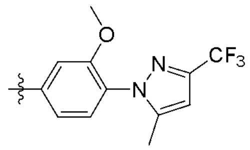, 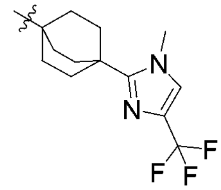,

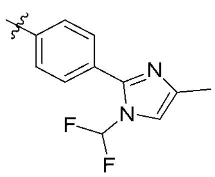, 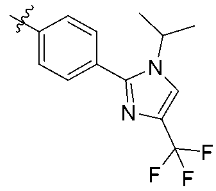, 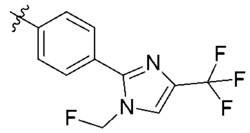, 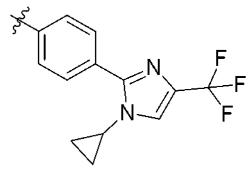,

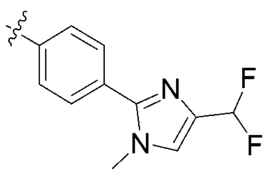 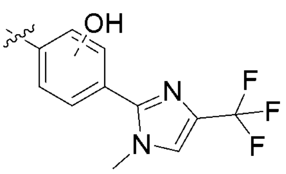, 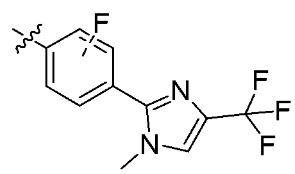,

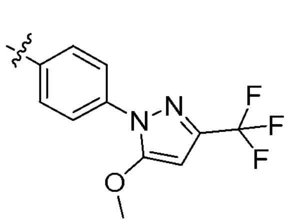, 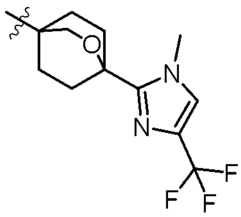, 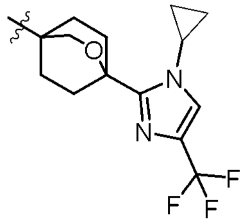, 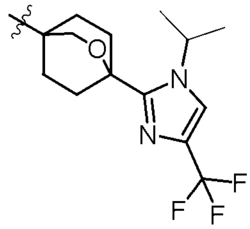,

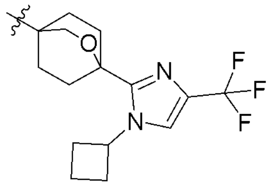, 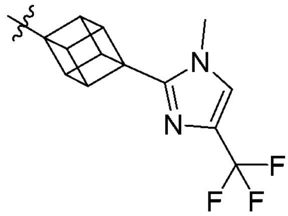, 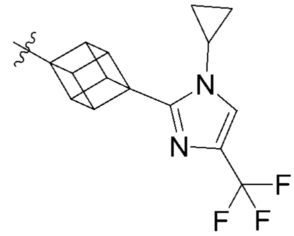

and

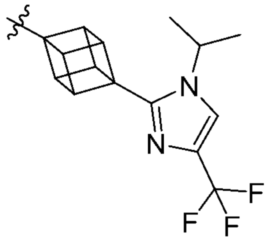.

In some embodiments, provided is the compound of formula (I'), wherein

R is $C_{6-10}$ aryl or $C_{6-10}$ aryl fused with 5 to 6 membered heteroaryl, the above groups are each independently and optionally substituted by one or more substituents selected from the group consisting of oxo, $C_{1-6}$ alkyl, -O-$C_{1-6}$alkylene-O-$C_{1-6}$ alkyl;
specifically, R is

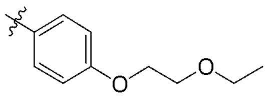 or 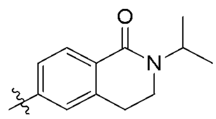 ;

$R_2$ is selected from the group consisting of H atom, -OH, $C_{1-6}$ alkyl, $C_{2-6}$ alkynyl, - $C_{1-6}$ alkylene-$C_{6-10}$ aryl, $C_{1-6}$ alkoxy, $C_{1-6}$ haloalkyl, $C_{1-6}$ haloalkoxy, and $C_{1-6}$ hydroxyalkyl; preferably, $R_2$ is H atom;
$R_3$ is selected from the group consisting of H atom, -OH, -COOH, $-NH_2$, -CN, halogen, $C_{1-6}$ alkyl, $C_{2-6}$ alkynyl, $C_{1-6}$ alkoxy, $C_{1-6}$ haloalkyl, $C_{1-6}$ haloalkoxy, and $C_{1-6}$ hydroxyalkyl; preferably, $R_3$ is methoxy;
$R_4$ is selected from the group consisting of H atom, -OH, -COOH, $-NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $R_4$ is H atom;
or, $R_3$ and $R_4$ together with the atoms to which they are attached form ring D, the ring D is 5 to 7 membered heteroaryl or 5 to 7 membered heterocyclyl, and preferably furanyl.

In some embodiments, provided is the compound of formula (I'), (I), (II) or (III), wherein

$R_5$ is selected from the group consisting of $C_{6-10}$ aryl, 5 to 6 membered heteroaryl, $C_{6-10}$ aryl fused with 5 to 6 membered heterocyclyl, and $C_{6-10}$ aryl fused with 5 to 6 membered heteroaryl, and preferably selected from the group consisting of phenyl, pyridyl, pyrimidinyl, pyrazolyl, imidazolyl, thiazolyl, indolyl, indolinyl and isoxazolyl, wherein the $C_{6-10}$ aryl, 5 to 6 membered heteroaryl, $C_{6-10}$ aryl fused with 5 to 6 membered heterocyclyl, and $C_{6-10}$ aryl fused with 5 to 6 membered heteroaryl are each independently and optionally substituted by one or more substituents selected from the group consisting of -OH, -COOH, $-NH_2$, -CN, halogen, $C_{1-6}$ alkyl, $C_{1-6}$ haloalkyl, $C_{1-6}$ alkoxy, $C_{1-6}$ haloalkoxy,

$C_{1-6}$ hydroxyalkyl and $C_{3-6}$ cycloalkyl;

specifically, $R_5$ is selected from the group consisting of

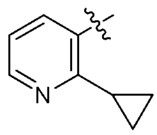, 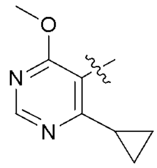,

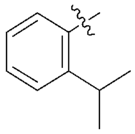, 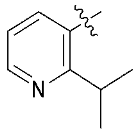, 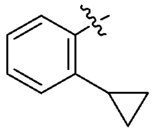, 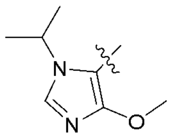, 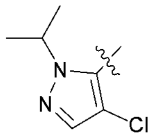, 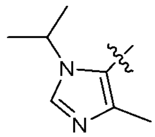,

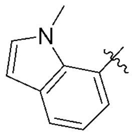, 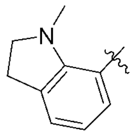, 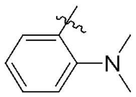, 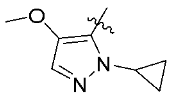, 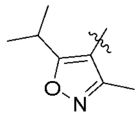, 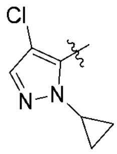, 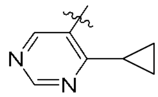,

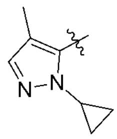, 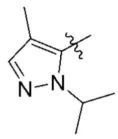, 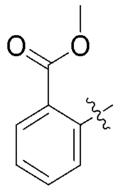, 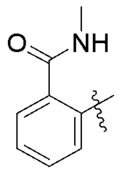 and 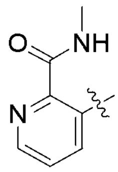.

In some embodiments, provided is the compound of formula (I'), (I), (II) or (III), wherein n is 0 or 1.

In some embodiments, provided is the compound of formula (I'), (I), (II) or (III), wherein $R_a$ and $R_b$ are each independently selected from the group consisting of H atom, -CN, $C_{1-6}$ alkyl, OH and halogen;

preferably, $R_a$ and $R_b$ are each independently H atom or -CN.

Typical compounds of formula (I) or (I') of the present invention include, but are not limited to the following compounds:

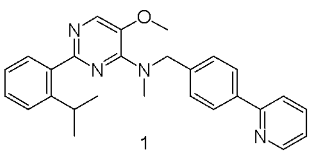
1,

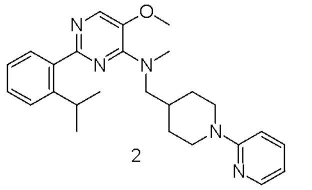
2,

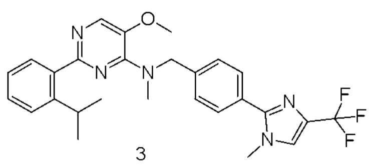
3,

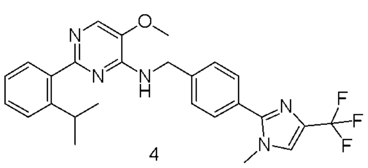
4,

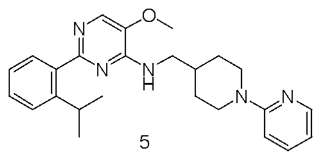
5,

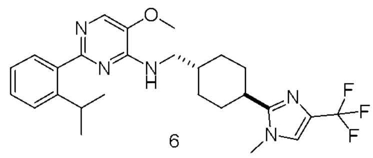
6,

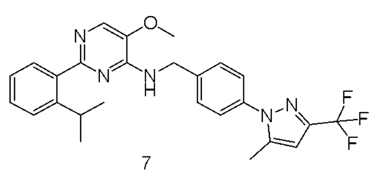
7 ,

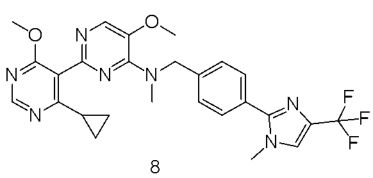
8 ,

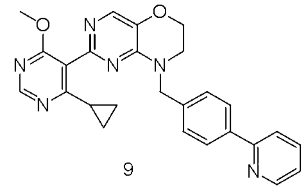
9 ,

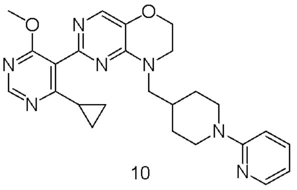
10 ,

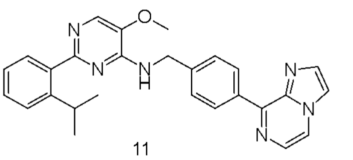
11 ,

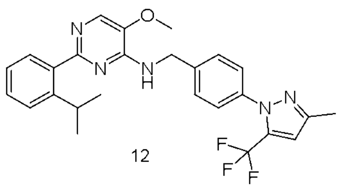
12 ,

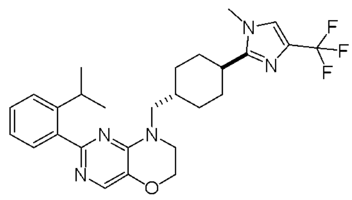
13 ,

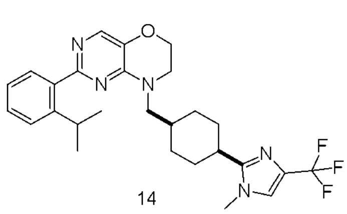
14 ,

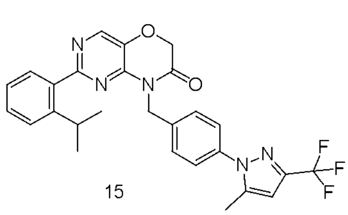
15 ,

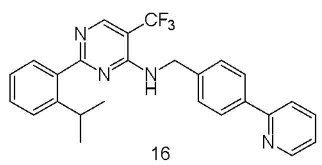
16 ,

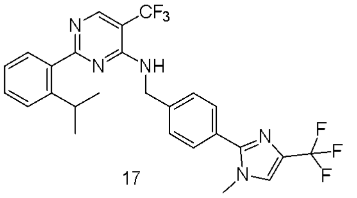
17 ,

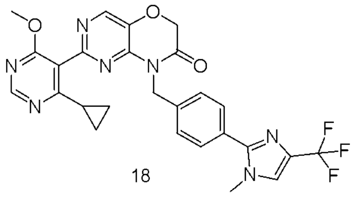
18 ,

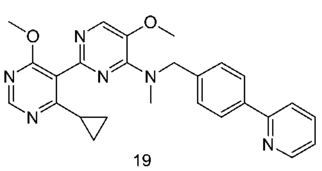
19 ,

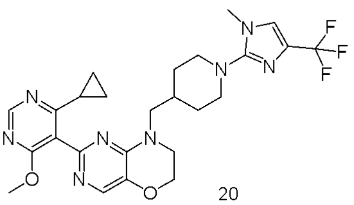
20 ,

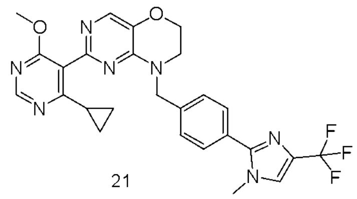
21 ,

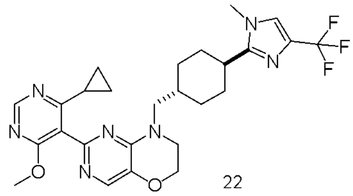
22 ,

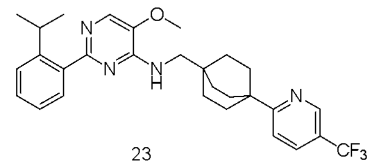
23 ,

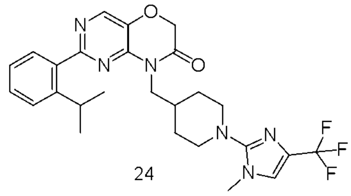
24 ,

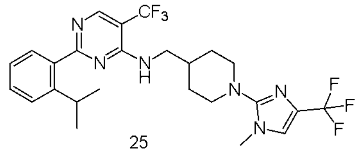
25 ,

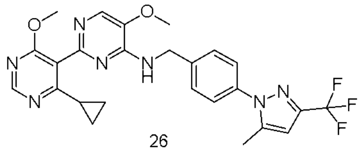
26 ,

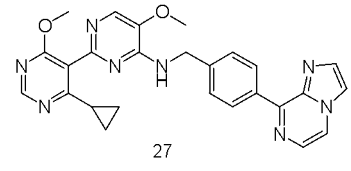
27 ,

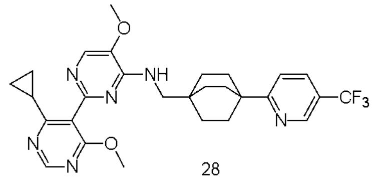
28
,
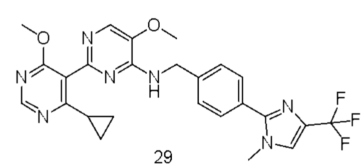
29
,
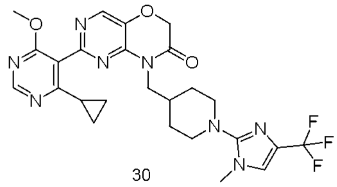
30
,
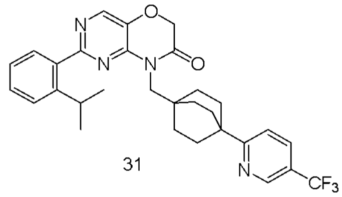
31
,
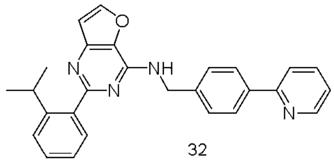
32
,
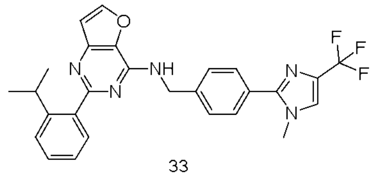
33
,
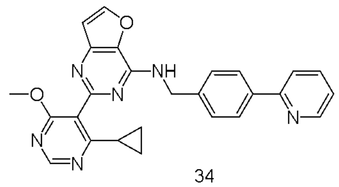
34
,
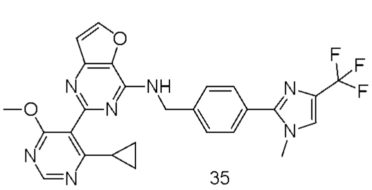
35
,
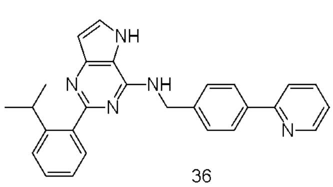
36
,
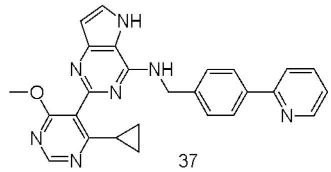
37
,
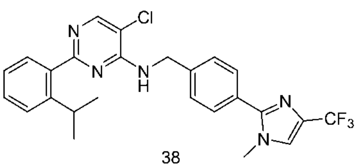
38
,
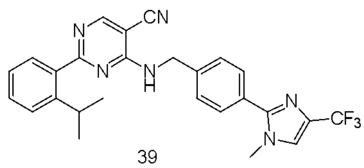
39
,
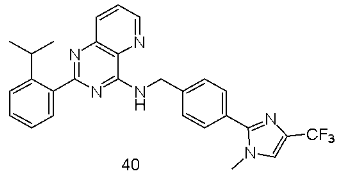
40
,
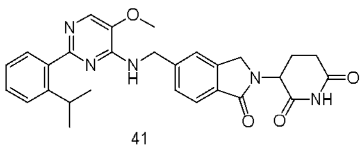
41
,
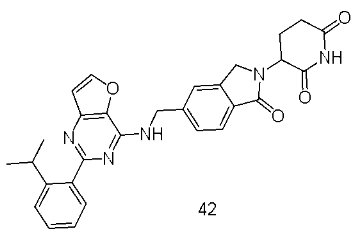
42
,
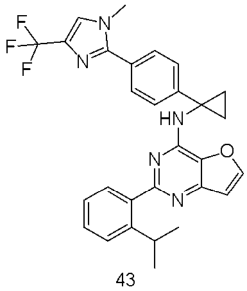
43
,
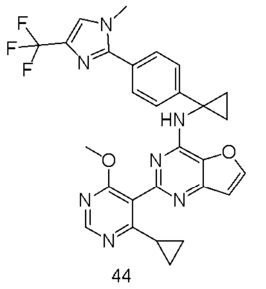
44
,
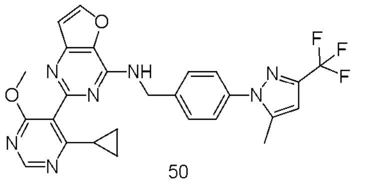
50
,
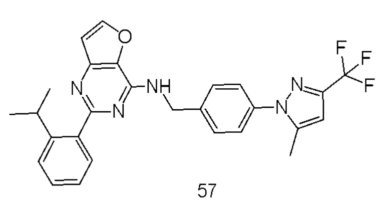
57
,
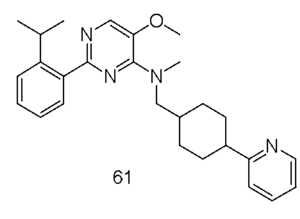
61
,
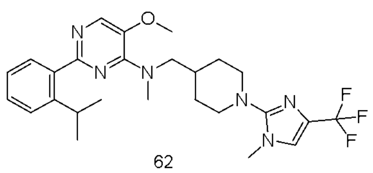
62
,

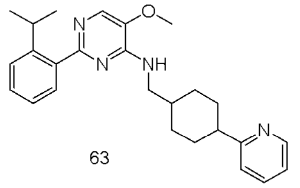
63 ,

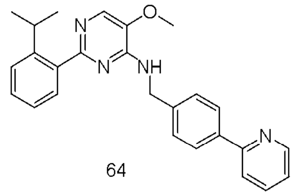
64 ,

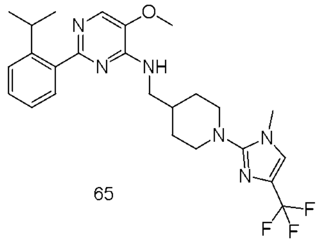
65 ,

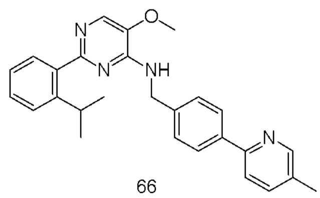
66 ,

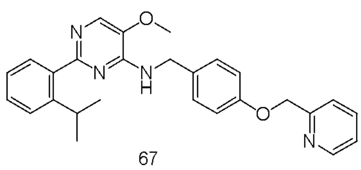
67 ,

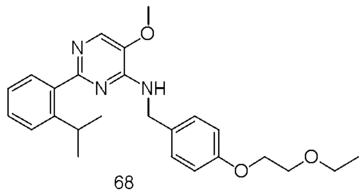
68 ,

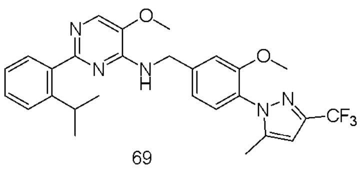
69 ,

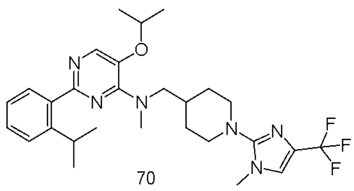
70 ,

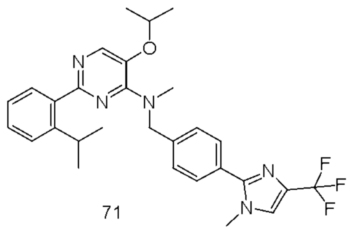
71 ,

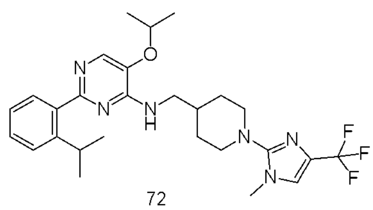
72 ,

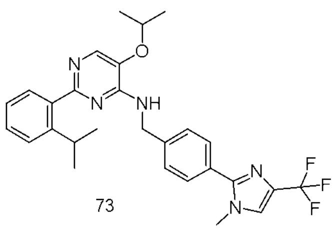
73 ,

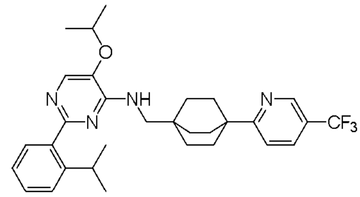
74 ,

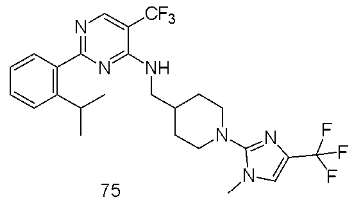
75 ,

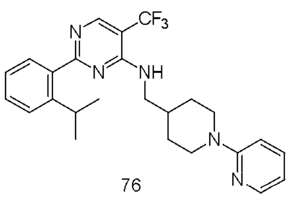
76 ,

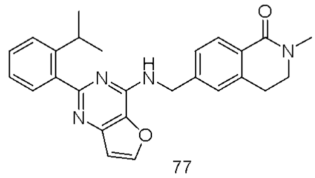
77 ,

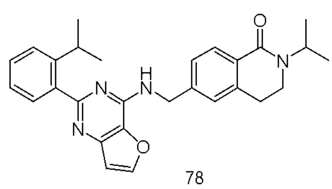
78 ,

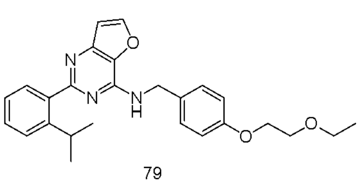
79 ,

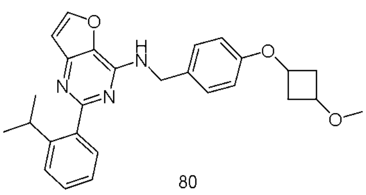
80 ,

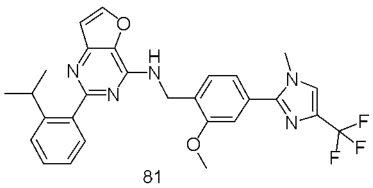
81

,

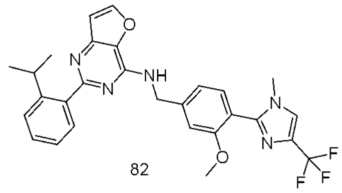
82

,

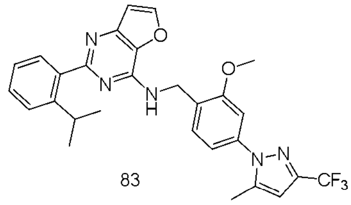
83

,

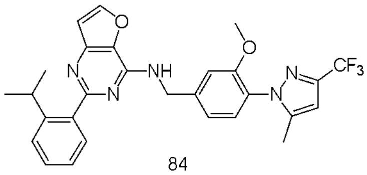
84

,

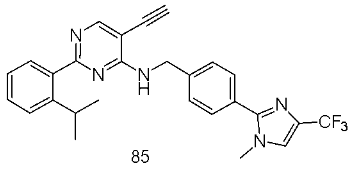
85

,

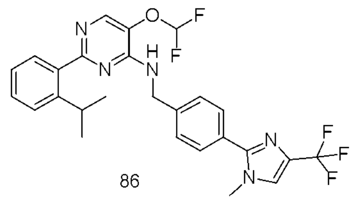
86

,

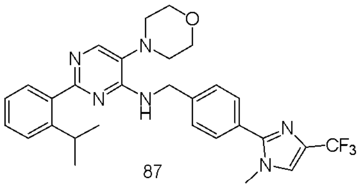
87

,

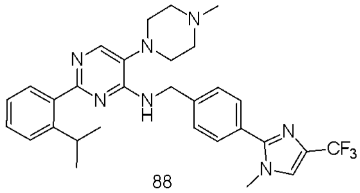
88

,

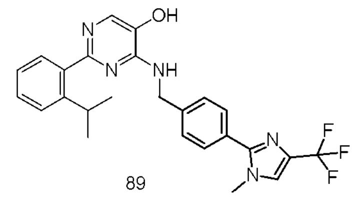
89

,

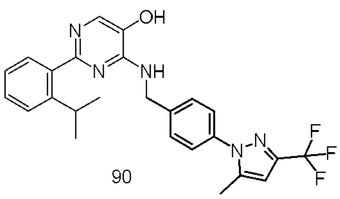
90

,

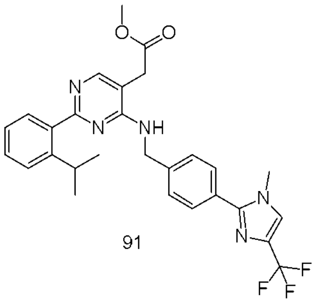
91

,

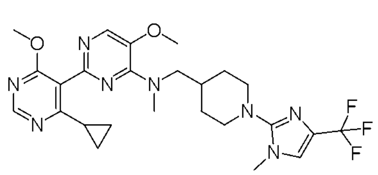
92

,

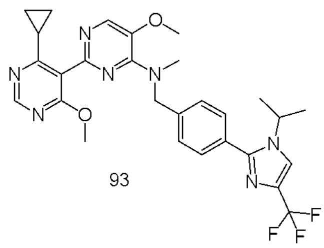
93

,

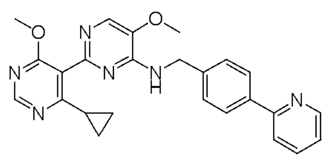
94

,

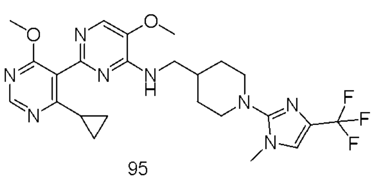
95

,

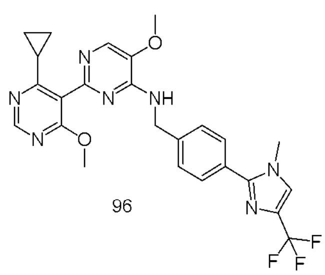
96

,

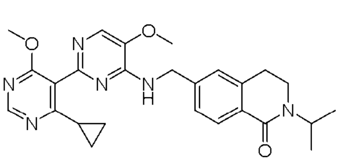
97

,

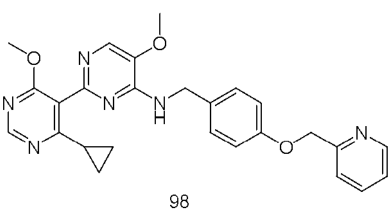
98

,

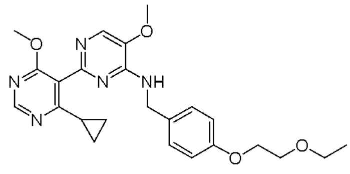
99,
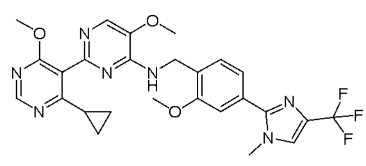
100,
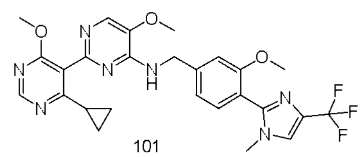
101,

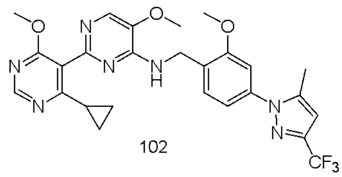
102,
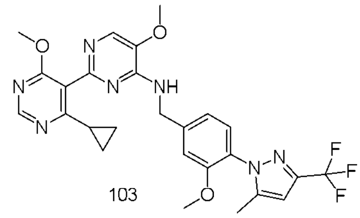
103,
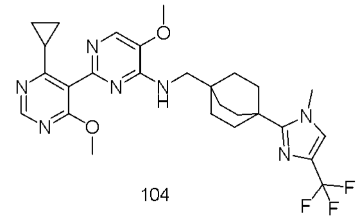
104,

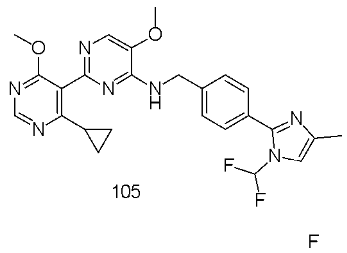
105,
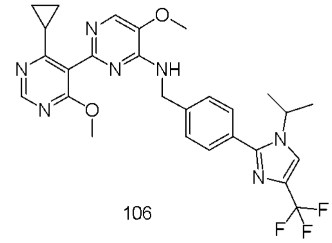
106,
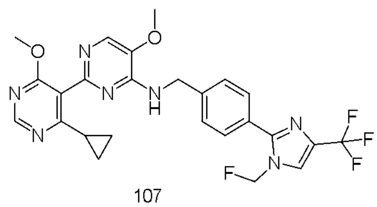
107,

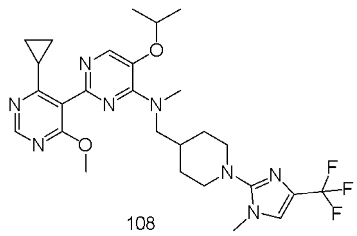
108,
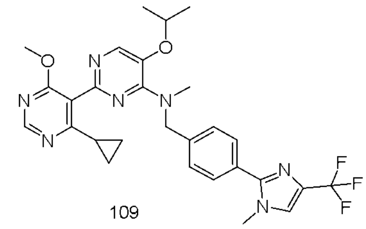
109,
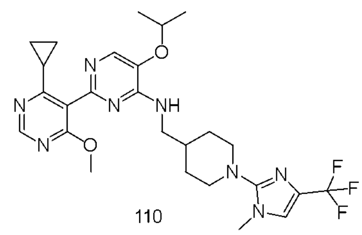
110,

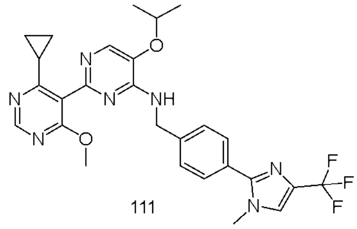
111,
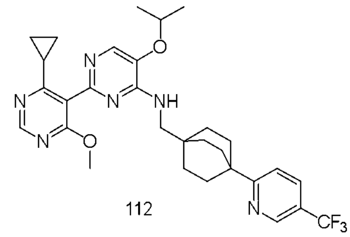
112,
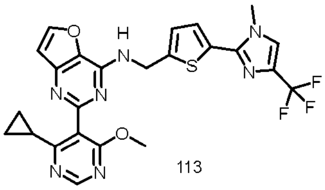
113,

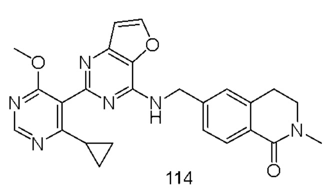
114,
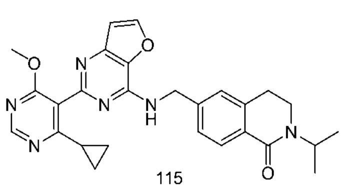
115,
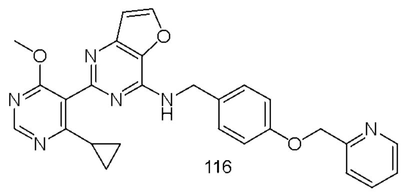
116,

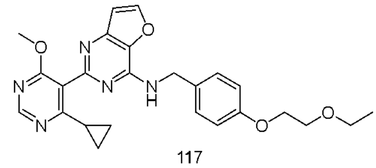
117,
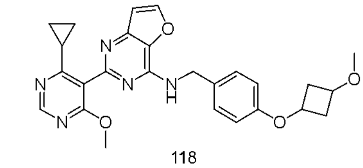
118,
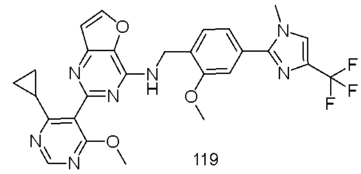
119,

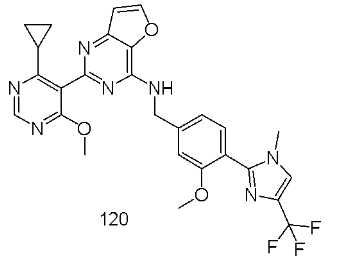
120,
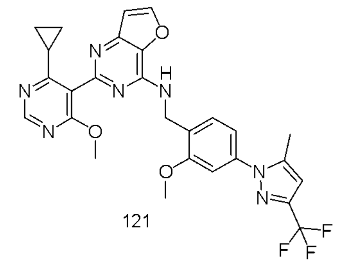
121,
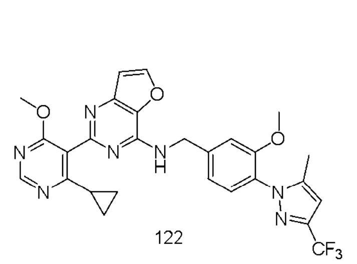
122,

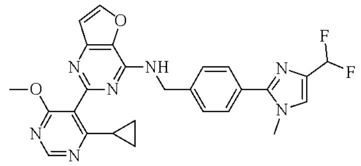
123,
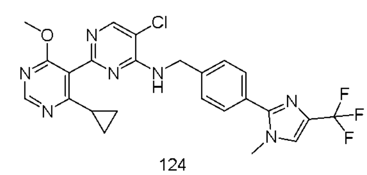
124,
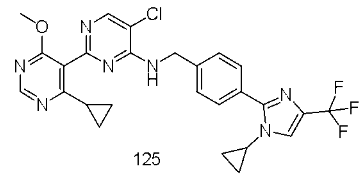
125,

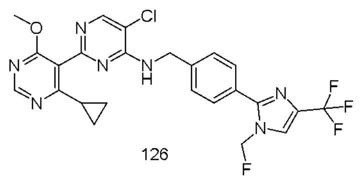
126,
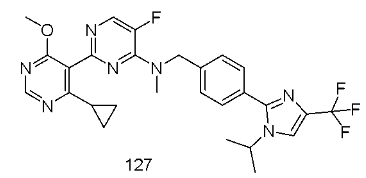
127,
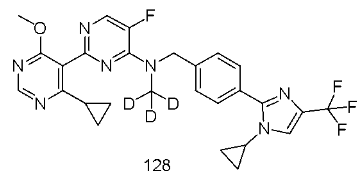
128,

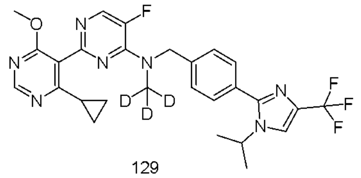
129,
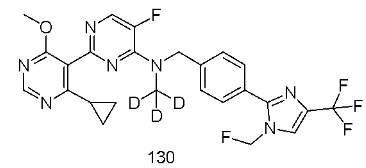
130,
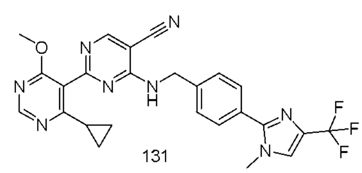
131,

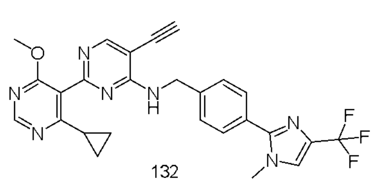
132,
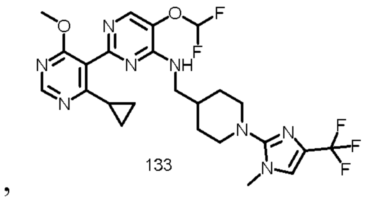
133,
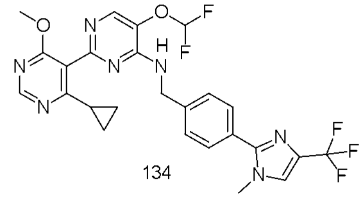
134,

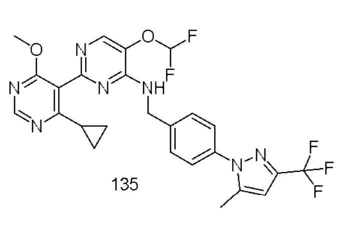
135,
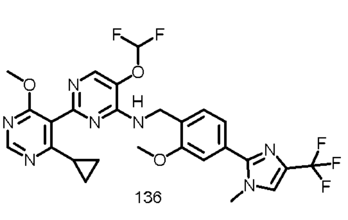
136,
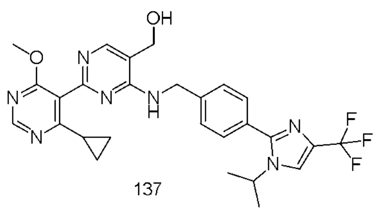
137,

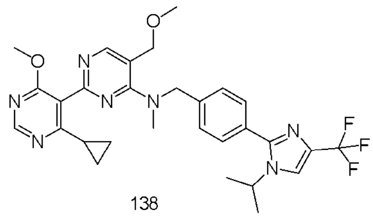
138,

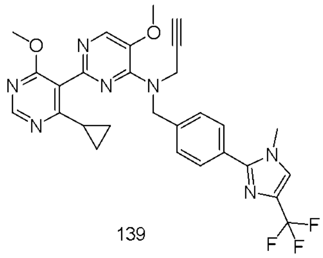
139,

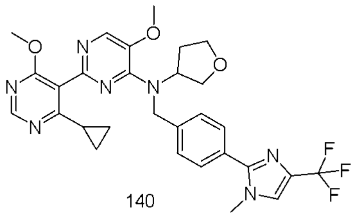
140,

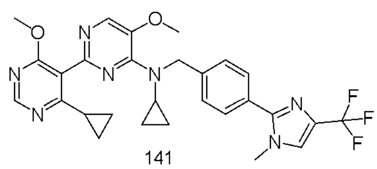
141,

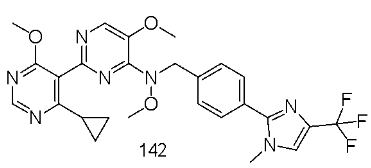
142,

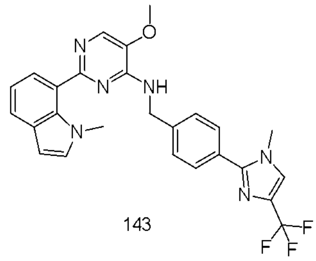
143,

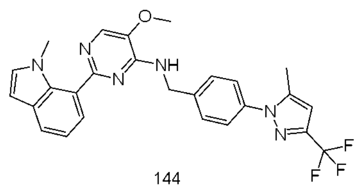
144,

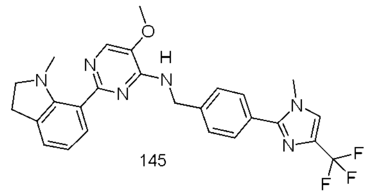
145,

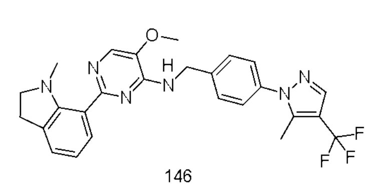
146,

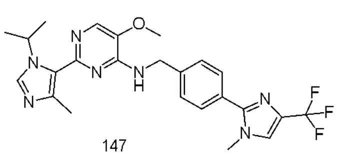
147,

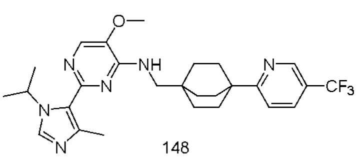
148,

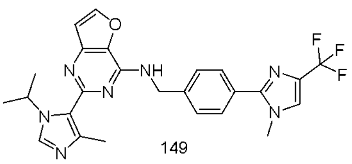
149,

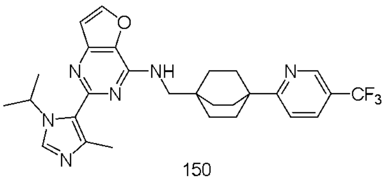
150,

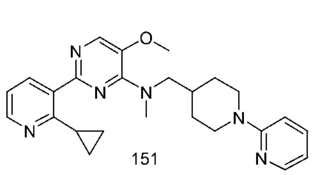
151,

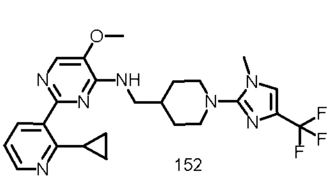
152

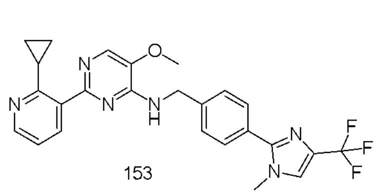
153,

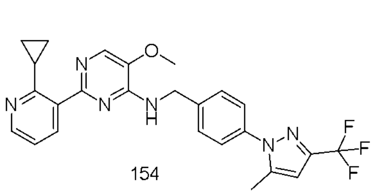
154,

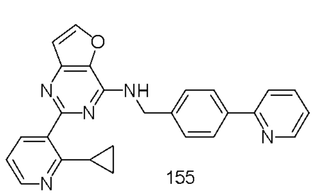
155,

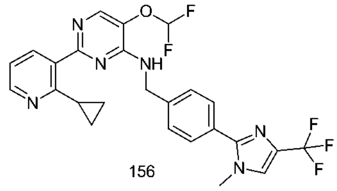
156,

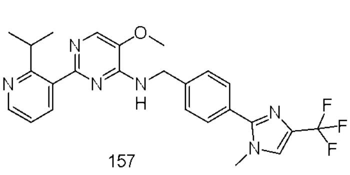
157,

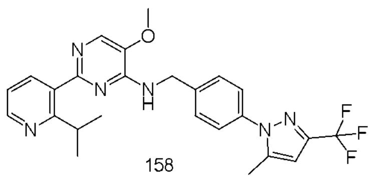
158,

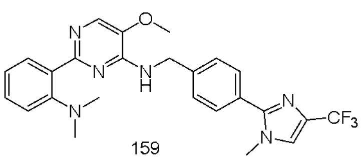
159 ,

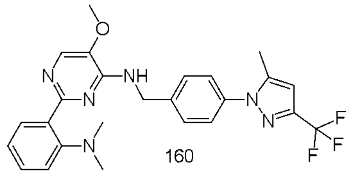
160 ,

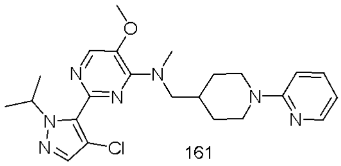
161 ,

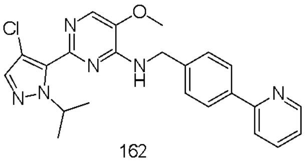
162 ,

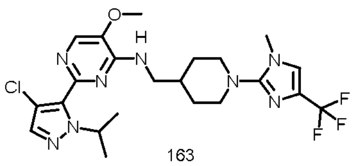
163 ,

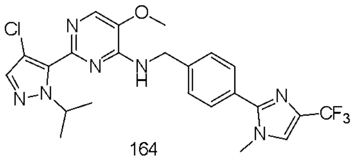
164 ,

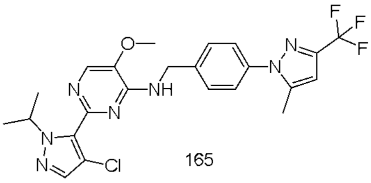
165 ,

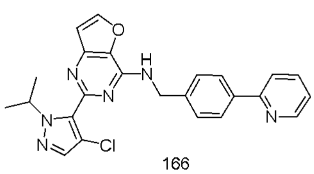
166 ,

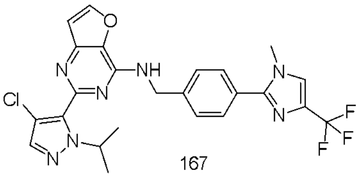
167 ,

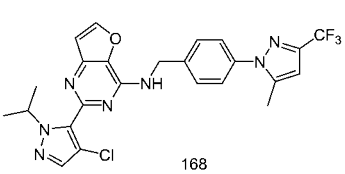
168 ,

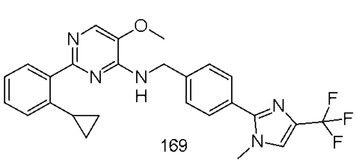
169 ,

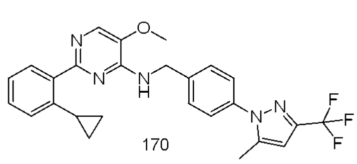
170 ,

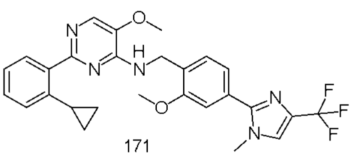
171 ,

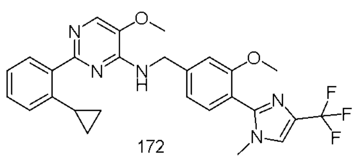
172 ,

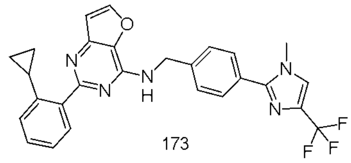
173 ,

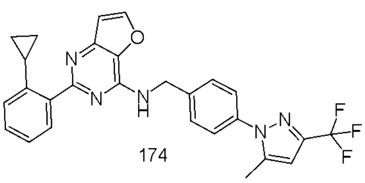
174 ,

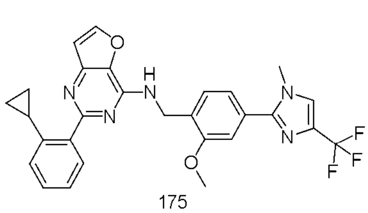
175 ,

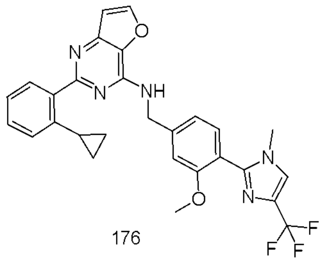
176 ,

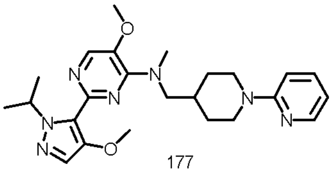
177 ,

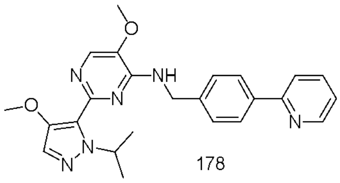
178 ,

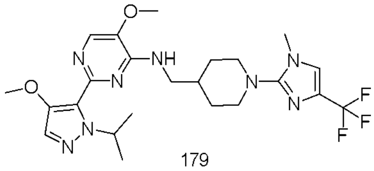
179 ,

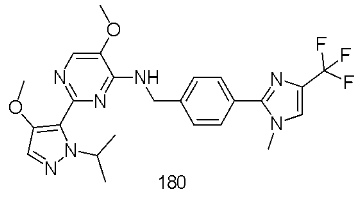
180 ,
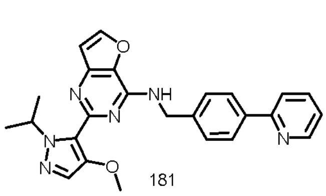
181 ,
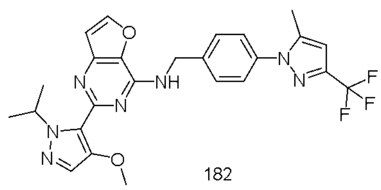
182 ,
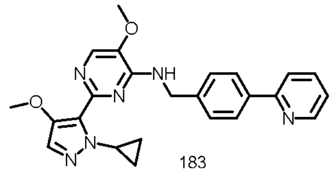
183 ,
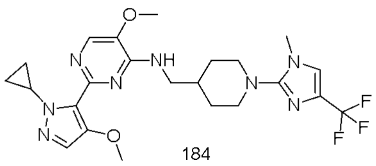
184 ,
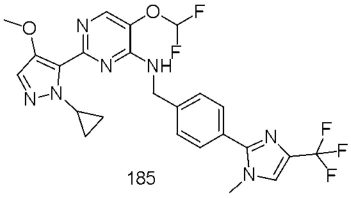
185 ,
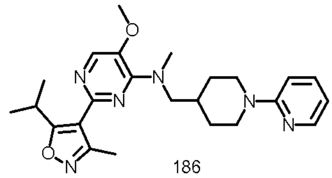
186 ,
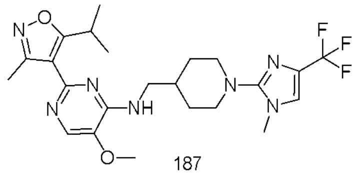
187 ,
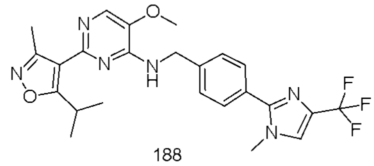
188 ,
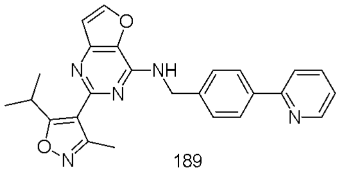
189 ,
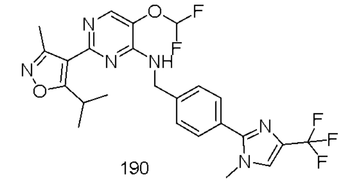
190 ,
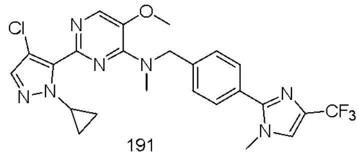
191 ,
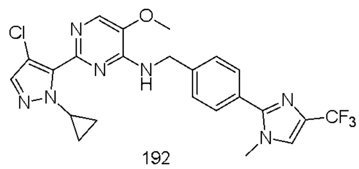
192 ,
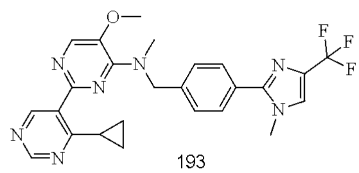
193 ,
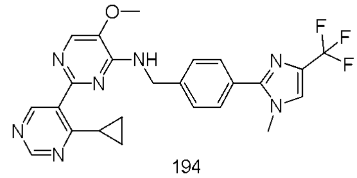
194 ,
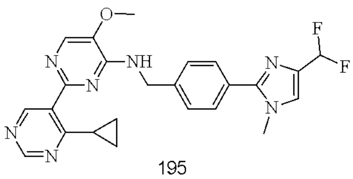
195 ,
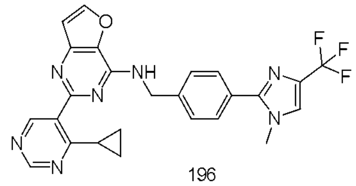
196 ,
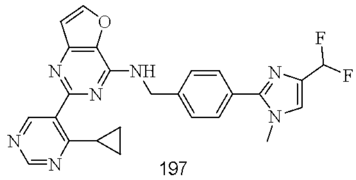
197 ,
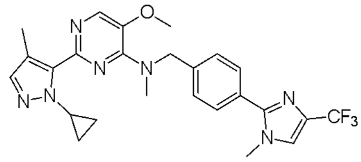
198 ,
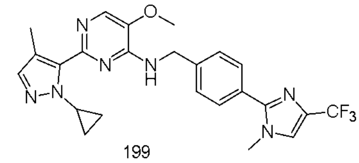
199 ,
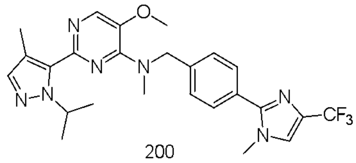
200 ,

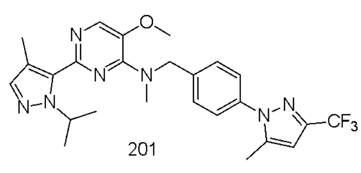
201,

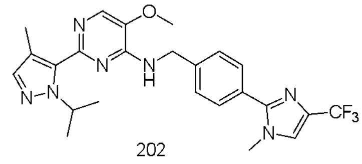
202,

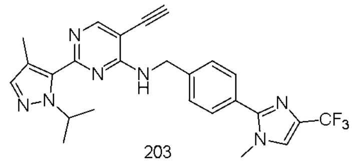
203,

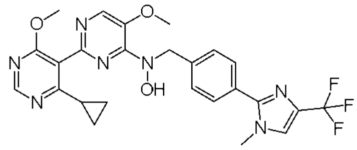
204,

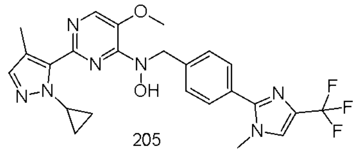
205,

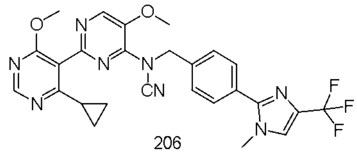
206,

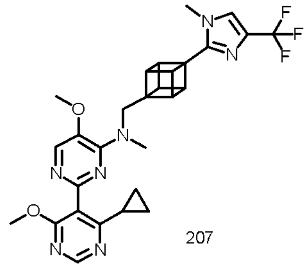
207,

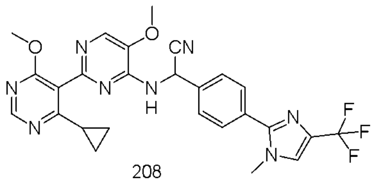
208,

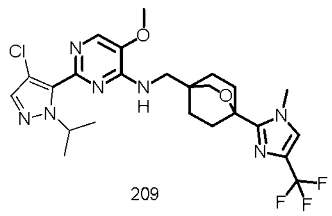
209,

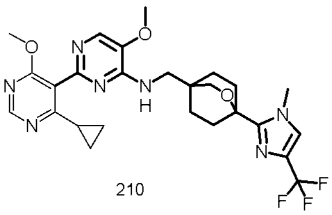
210,

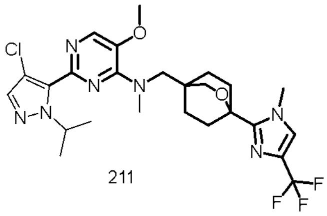
211,

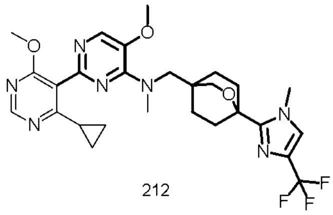
212,

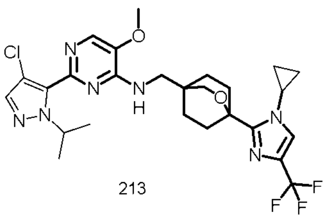
213,

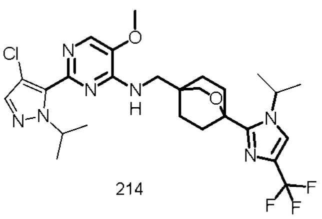
214,

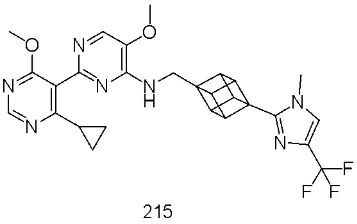
215,

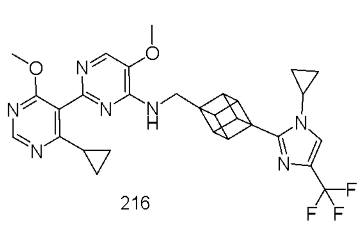
216 and

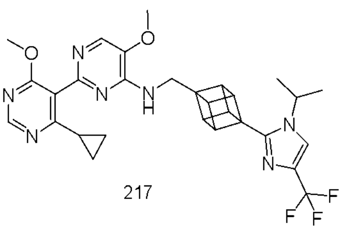
217.

The present invention also provides a method for preparing a compound of formula (I'), comprising:

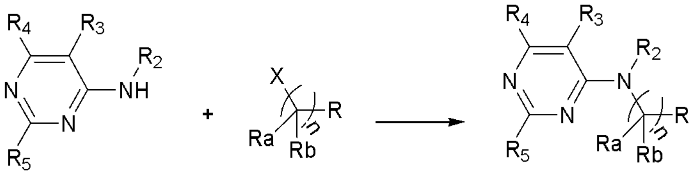

(IA) (IB') **(I')**

reacting a compound of formula (IA) with a compound of formula (IB) to obtain the compound of formula (I');
X is a leaving group selected from the group consisting of halogen, sulfonate, boronic acid and borate; and
R, $R_a$, $R_b$, $R_2$ to $R_5$ and n are as defined in formula (I');
or

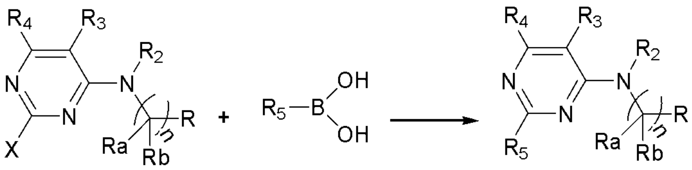

(IC') (ID) **(I')**

reacting a compound of formula (IC') with a compound of formula (ID) to obtain the compound of formula (I');
X is a leaving group selected from the group consisting of halogen, sulfonate, boronic acid and borate; and
R, $R_a$, $R_b$, $R_2$ to $R_5$ and n are as defined in formula (I');
or

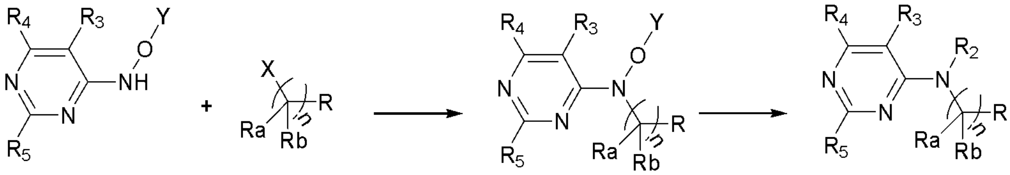

(IE) (IB') **(IF')** **(I')**

reacting a compound of formula (IE) with a compound of formula (IB') to obtain a compound of formula (IF'), and deprotecting the compound of formula (IF') to obtain the compound of formula (I');
X is a leaving group selected from the group consisting of halogen, sulfonate, boronic acid and borate;
Y is a hydroxy protecting group selected from the group consisting of *tert*-butyldimethylsilyl, trimethylsilyl, triethylsilyl, triisopropylsilyl, tert-butyldiphenylsilyl, benzyl, methoxymethyl and ethoxyethyl;
$R_2$ is hydroxy;
R, $R_a$, $R_b$, $R_3$ to $R_5$ and n are as defined in formula (I');
or

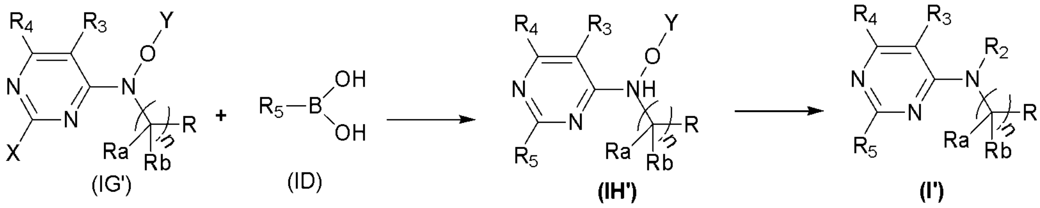

(IG') (ID) **(IH')** **(I')**

reacting a compound of formula (IG') with a compound of formula (ID) to obtain a compound of formula (IH'), and deprotecting the compound of formula (IH') to obtain the compound of formula (I');
X is a leaving group selected from the group consisting of halogen, sulfonate, boronic acid and borate;
Y is a hydroxy protecting group selected from the group consisting of *tert*-butyldimethylsilyl, trimethylsilyl, triethylsilyl, triisopropylsilyl, tert-butyldiphenylsilyl, benzyl, methoxymethyl and ethoxyethyl;
$R_2$ is hydroxy;
R, $R_a$, $R_b$, $R_3$ to $R_5$ and n are as defined in formula (I');

or

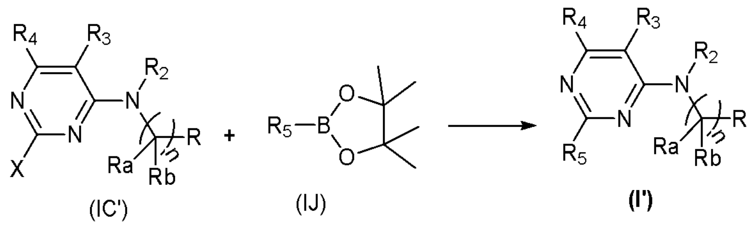

(IC') (IJ) **(I')**

reacting a compound of formula (IC') with a compound of formula (IJ) to obtain the compound of formula (I'); X is a leaving group selected from the group consisting of halogen, sulfonate, boronic acid and borate; and R, $R_a$, $R_b$, $R_2$ to $R_5$ and n are as defined in formula (I').

The present invention also provides a method for preparing a compound of formula (I), comprising:

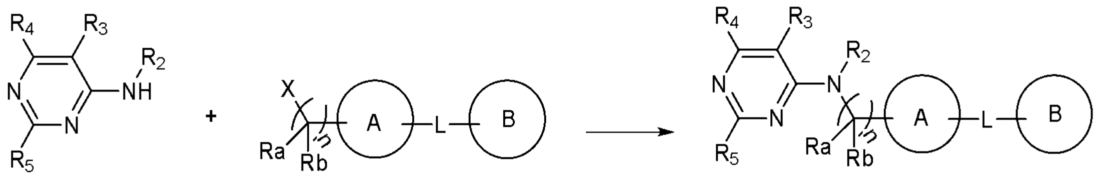

(IA) (IB) **(I)**

reacting a compound of formula (IA) with a compound of formula (IB) to obtain the compound of formula (I); X is a leaving group selected from the group consisting of halogen, sulfonate, boronic acid and borate; and ring A, ring B, L, $R_a$, $R_b$, $R_2$ to $R_5$ and n are as defined in formula (I');
or

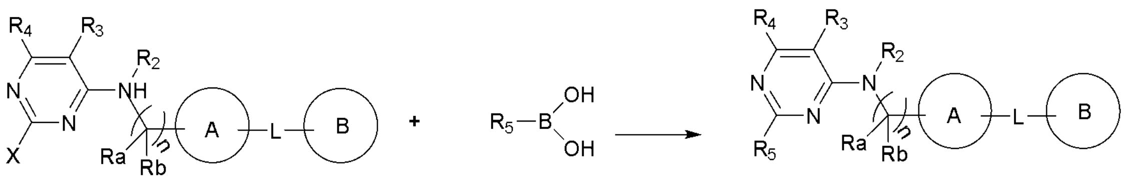

(IC) (ID) **(I)**

reacting a compound of formula (IC) with a compound of formula (ID) to obtain the compound of formula (I); X is a leaving group selected from the group consisting of halogen, sulfonate, boronic acid and borate; and ring A, ring B, L, $R_a$, $R_b$, $R_2$ to $R_5$ and n are as defined in formula (I');
or

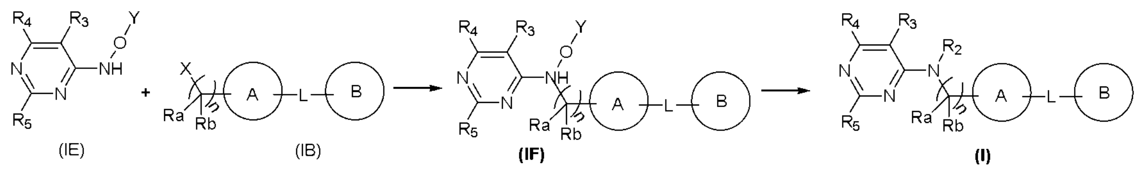

(IE) (IB) **(IF)** **(I)**

reacting a compound of formula (IE) with a compound of formula (IB) to obtain a compound of formula (IF), and deprotecting the compound of formula (IF) to obtain the compound of formula (I);
X is a leaving group selected from the group consisting of halogen, sulfonate, boronic acid and borate;
Y is a hydroxy protecting group selected from the group consisting of *tert*-butyldimethylsilyl, trimethylsilyl, triethylsilyl, triisopropylsilyl, tert-butyldiphenylsilyl, benzyl, methoxymethyl and ethoxyethyl;
$R_2$ is hydroxy;
ring A, ring B, L, $R_a$, $R_b$, $R_3$ to $R_5$ and n are as defined in formula (I');

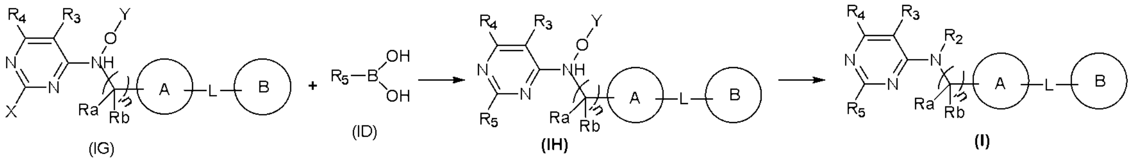

(IG) (ID) (IH) (I)

reacting a compound of formula (IG) with a compound of formula (ID) to obtain a compound of formula (IH), and deprotecting the compound of formula (IH) to obtain the compound of formula (I);
X is a leaving group selected from the group consisting of halogen, sulfonate, boronic acid and borate;
Y is a hydroxy protecting group selected from the group consisting of *tert*-butyldimethylsilyl, trimethylsilyl, triethylsilyl, triisopropylsilyl, *tert*-butyldiphenylsilyl, benzyl, methoxymethyl and ethoxyethyl;
$R_2$ is hydroxy;
ring A, ring B, L, $R_a$, $R_b$, $R_3$ to $R_5$ and n are as defined in formula (I');
or

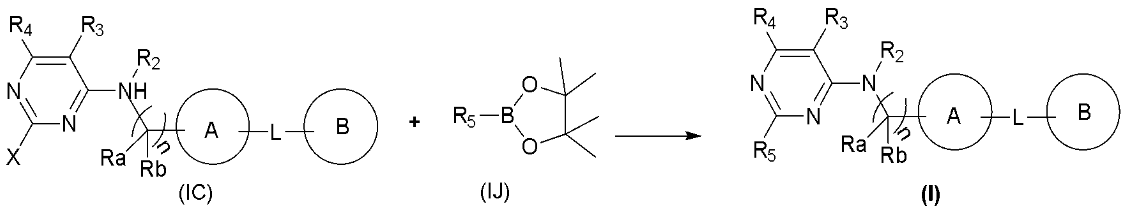

(IC) (IJ) (I)

reacting a compound of formula (IC) with a compound of formula (IJ) to obtain the compound of formula (I);
X is a leaving group selected from the group consisting of halogen, sulfonate, boronic acid and borate; and
ring A, ring B, L, $R_a$, $R_b$, $R_2$ to $R_5$ and n are as defined in formula (I');
The present invention provides a compound of formula (SI),

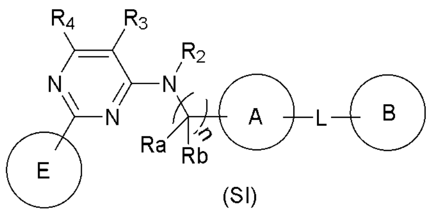

(SI)

or a pharmaceutically acceptable salt, hydrate, solvate, isotope substitute or stereoisomer thereof, wherein,

ring A and ring B are each independently selected from the group consisting of $C_{6-10}$ aryl, 5 to 10 membered heteroaryl, $C_{3-8}$ cycloalkyl and 3 to 8 membered heterocyclyl, and ring A and ring B are each independently and optionally substituted by one or more $R_1$;
ring E is selected from the group consisting of $C_{6-10}$ aryl, 5 to 6 membered heteroaryl, $C_{6-10}$ aryl fused with 5 to 6 membered heterocyclyl, and $C_{6-10}$ aryl fused with 5 to 6 membered heteroaryl, and ring E is optionally substituted by one or more $R_1$;
L is selected from the group consisting of a chemical bond, -O-, -S-, -$C_{1-6}$ alkylene-, -O-$C_{1-6}$ alkylene-, -$C_{1-6}$ alkylene-O-, -S-$C_{1-6}$ alkylene- and -$C_{1-6}$ alkylene-S-;
$R_a$ and $R_b$ are each independently selected from the group consisting of H atom, - CN, $C_{1-6}$ alkyl, -OH, halogen, $C_{2-6}$ alkenyl, $C_{2-6}$ alkynyl, $C_{1-6}$ hydroxyalkyl, $C_{1-6}$ haloalkyl, $C_{1-6}$ alkoxy, $C_{1-6}$ haloalkoxy; or $R_a$ and $R_b$ together form an oxo, $C_{3-8}$ cycloalkyl or 3 to 8 membered heterocyclyl; wherein the $C_{1-6}$ alkyl is optionally substituted by one or more $R_1$;
$R_2$ is selected from the group consisting of H atom, -OH, -CN, $C_{1-6}$ alkyl, -$C_{1-6}$ alkylene-$C_{6-10}$ aryl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{1-6}$ alkyl or -$C_{1-6}$ alkylene-$C_{6-10}$ aryl is optionally substituted by one or more $R_1$;
$R_3$ is selected from the group consisting of H atom, -OH, -COOH, -$NH_2$, -CN, halogen, $C_{1-6}$ alkyl, -S-$C_{1-6}$ alkyl, -S(O)-$C_{1-6}$ alkyl, -$S(O)_2$-$C_{1-6}$ alkyl, phosphoryl, phosphonyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3

to 8 membered heterocyclyl and -$C_{1-6}$ alkylene-C(O)-O-$C_{1-6}$ alkyl, wherein the - $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl are each independently and optionally substituted by one or more $R_1$;

$R_4$ is selected from the group consisting of H atom, -OH, -COOH, -$NH_2$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;

$R_1$ at each occurrence is independently selected from the group consisting of D atom, -OH, -COOH, -$NH_2$, -CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3 to 8 membered heterocyclyl, -O-$C_{1-6}$alkylene-O-$C_{1-6}$ alkyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of D atom, - OH, -COOH, -$NH_2$, -CN, halogen, $C_{1-6}$ alkyl, $C_{1-6}$ haloalkyl, $C_{1-6}$ alkoxy, $C_{1-6}$ haloalkoxy and $C_{1-6}$ hydroxyalkyl; and

n is an integer from 0 to 8.

In some embodiments, provided is the compound of formula (SI), wherein ring B is $C_{6-10}$ aryl or 5 to 6 membered heteroaryl, and ring B is optionally substituted by one or more $R_1$, $R_1$ is as defined in formula (SI); specifically,

ring B is selected from the group consisting of phenyl, naphthyl, pyridinyl, pyrimidinyl, imidazolyl, pyrazolyl, thienyl, piperazinyl, naphthyl, pyrrolyl, pyridazinyl, triazolyl, tetrazolyl, and furanyl, and ring B is optionally substituted by one or more $R_1$, $R_1$ is as defined in formula (SI);

more specifically,

ring B is imidazolyl, and ring B is optionally substituted by one or more $R_1$, $R_1$ is as defined in formula (SI).

In some embodiments, the compound of formula (SI) is a compound of formula (SII),

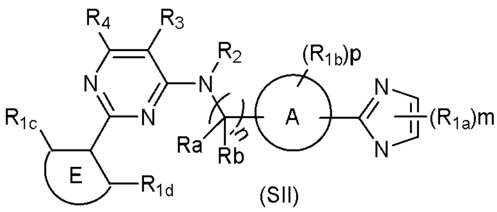

(SII)

wherein,

$R_{1a}$ at each occurrence is independently selected from the group consisting of D atom, -OH, -COOH, -$NH_2$, -CN, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;

$R_{1b}$ at each occurrence is independently selected from the group consisting of D atom, -OH, -COOH, -$NH_2$, -CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;

$R_{1c}$ is selected from the group consisting of H atom, D atom, -OH, -COOH, -$NH_2$, - CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;

$R_{1d}$ is selected from the group consisting of H atom, D atom, -OH, -COOH, -$NH_2$, - CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;

m is 0, 1 or 2;

p is 0, 1 or 2;

ring A, ring E, $R_a$, $R_b$, $R_2$ to $R_4$ and n are as defined in formula (SI).

In some embodiments, provided is the compound of formula (SI) or (SII), wherein

ring A is selected from the group consisting of $C_{6-10}$ aryl, $C_{3-8}$ cycloalkyl and 3 to 8 membered heterocyclyl, and ring A is optionally substituted by one or more $R_1$, $R_1$ is as defined in formula (SI);
specifically,
ring A is selected from the group consisting of phenyl, naphthyl, cyclohexyl, cyclopentyl, cyclobutyl, cyclopropyl, bicyclo[2.2.2]octanyl, 2-oxabicyclo[2.2.2]octanyl, cubanyl, piperidyl, pyranyl, pyrrolidinyl, piperazinyl and morpholinyl, and ring A is optionally substituted by one or more $R_{1b}$, $R_{1b}$ is as defined in formula (SII);
more specifically,
ring A is selected from the group consisting of phenyl, cyclohexyl, bicyclo[2.2.2]octanyl, 2-oxabicyclo[2.2.2]octanyl and cubanyl, and ring A is optionally substituted by one or more $R_{1b}$, $R_{1b}$ is as defined in formula (SII).

In some embodiments, provided is the compound of formula (SI) or (SII), wherein

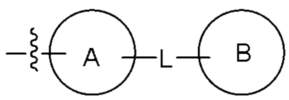 or 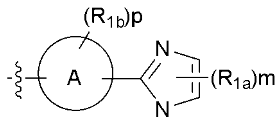

is selected from the group consisting of

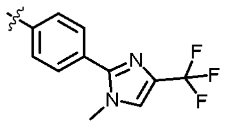 , 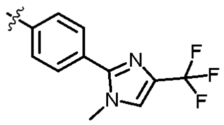 , 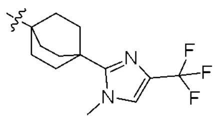 , 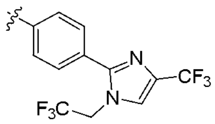 ,

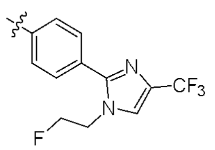 , 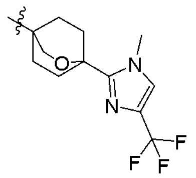 , 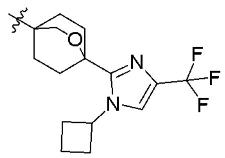 , 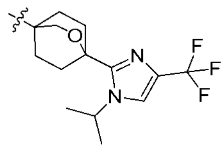 ,

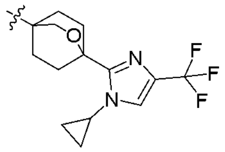 , 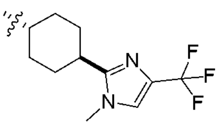 , 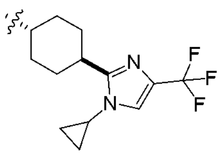 , 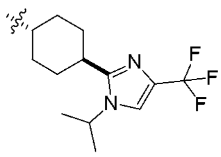 ,

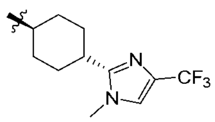 , 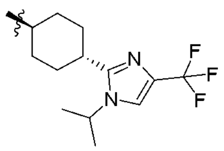 , 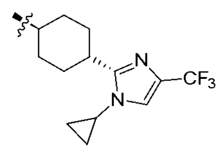 , 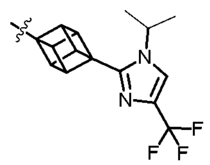 ,

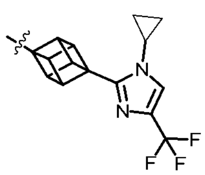 and 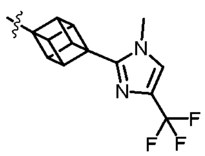 .

In some embodiments, provided is the compound of formula (SI) or (SII), wherein

ring E is $C_{6-10}$ aryl or 5 to 6 membered heteroaryl, and ring E is optionally substituted by one or more $R_1$, $R_1$ is as defined in formula (SI);
specifically,
ring E is selected from the group consisting of phenyl, pyridinyl, pyrimidinyl, pyrazolyl, imidazolyl, thiazolyl and

isoxazolyl, and ring E is optionally substituted by one $R_{1c}$ and one $R_{1d}$, $R_{1c}$ and $R_{1d}$ is as defined in formula (SII); more specifically,

ring E is selected from the group consisting of pyridinyl, pyrimidinyl and imidazolyl, ring E is optionally substituted by one $R_{1c}$ and one $R_{1d}$, $R_{1c}$ and $R_{1d}$ is as defined in formula (SII);

even more specifically,

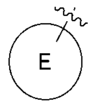

is selected from the group consisting of

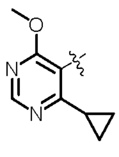, 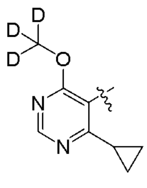, 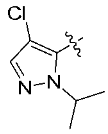,

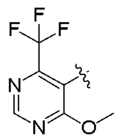, 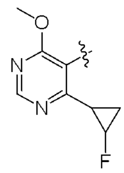, 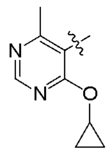, 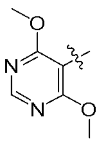, 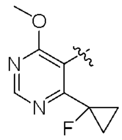 and 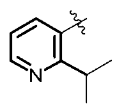.

In some embodiments, provided is the compound of formula (SI) or (SII), wherein

n is 0 or 1;

and/or

$R_a$ and $R_b$ are each independently selected from the group consisting of H atom, - CN, $C_{1-6}$ alkyl, -OH and halogen; preferably, $R_a$ and $R_b$ are each independently H atom or $C_{1-6}$ alkyl;

more preferably, both of $R_a$ and $R_b$ are H atom, or $R_a$ is H atom and $R_b$ is methyl.

In some embodiments, provided is the compound of formula (SI) or (SII), wherein

$R_2$ is selected from the group consisting of H atom, -OH, -CN, $C_{1-6}$ alkyl, $C_{1-6}$ deuterated alkyl, $C_{2-6}$ alkynyl, -$C_{1-6}$ alkylene-$C_{6-10}$ aryl, $C_{1-6}$ alkoxy, $C_{1-6}$ haloalkyl, $C_{1-6}$ haloalkoxy, $C_{1-6}$ hydroxyalkyl, $C_{3-6}$ cycloalkyl and 5 to 7 membered heterocyclyl,

preferably, $R_2$ is selected from the group consisting of H atom, -CN, methyl and trideuteriomethyl;

and/or

$R_3$ is selected from the group consisting of H atom, -OH, -COOH, -$NH_2$, -CN, halogen, $C_{1-6}$ alkyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{3-6}$ cycloalkyl-O-, 5 to 7 membered heterocyclyl and -$C_{1-6}$ alkylene-C(O)-O-$C_{1-6}$ alkyl;

preferably, $R_3$ is methoxy or cyclopropyl-O-;

and/or

$R_4$ is selected from the group consisting of H atom, -OH, -COOH, -$NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;

preferably, $R_4$ is H atom.

In some embodiments, provided is the compound of formula (SI) or (SII), wherein ring A, ring B, ring E, L, $R_a$, $R_b$, $R_2$ to $R_4$, $R_{1a}$, $R_{1b}$, $R_{1c}$, $R_{1d}$, m, n and p are each independently corresponding groups in Compounds S1 to S51.

Typical compounds of formula (SI) or (SII) of the present invention include, but are not limited to the following compounds:

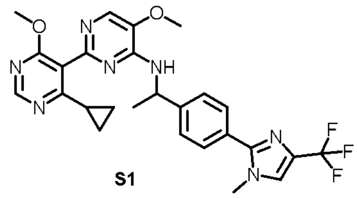
S1,

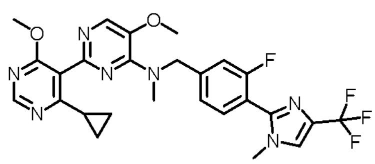
S2,

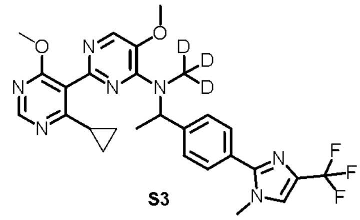
S3,

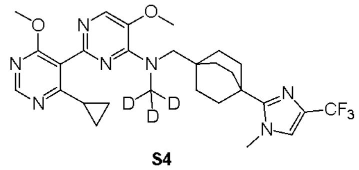
S4,

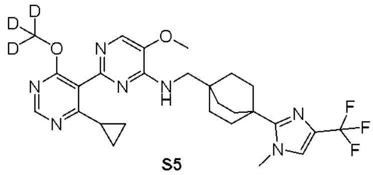
S5,

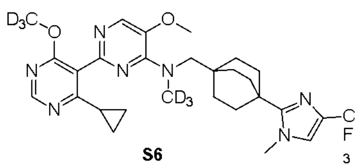
S6,

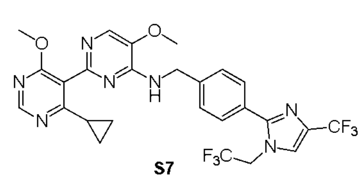
S7,

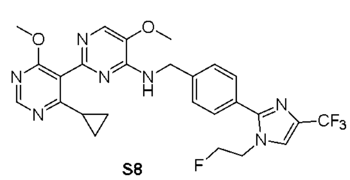
S8,

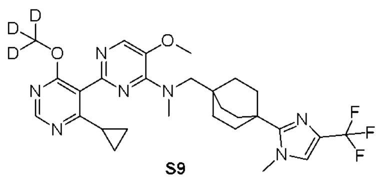
S9,

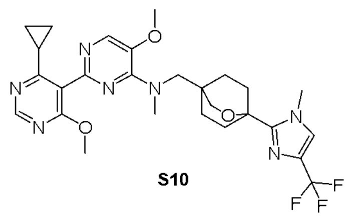
S10,

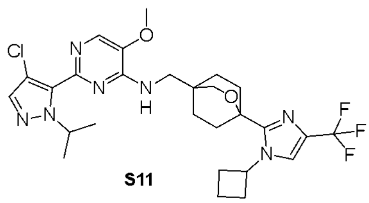
S11,

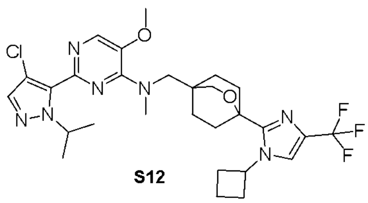
S12,

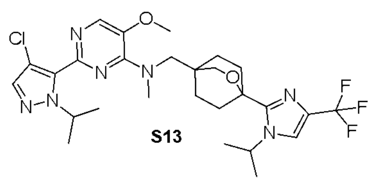
S13,

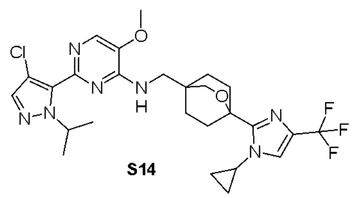
S14,

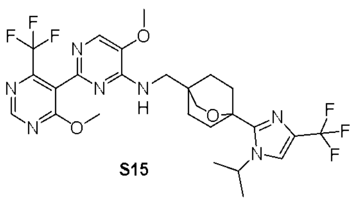
S15,

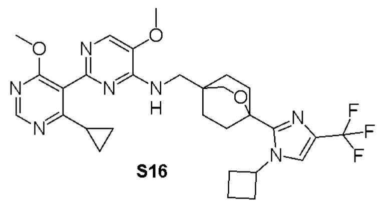
S16,

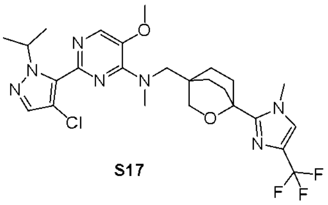
S17,

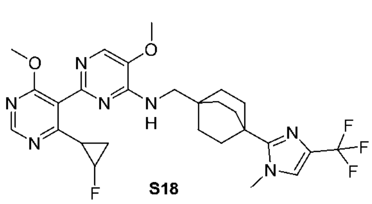
S18,

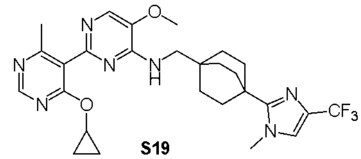
S19,

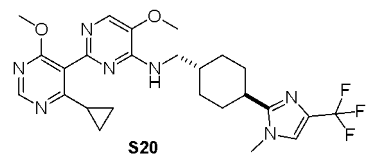
S20,

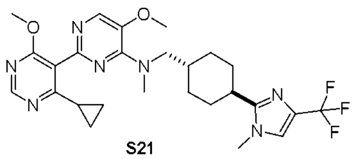
S21,

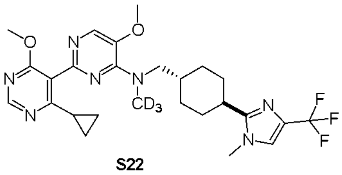
S22,

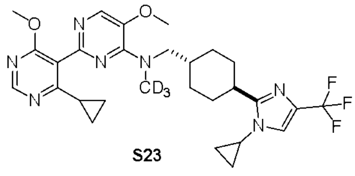
S23,

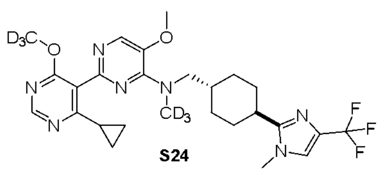
S24,

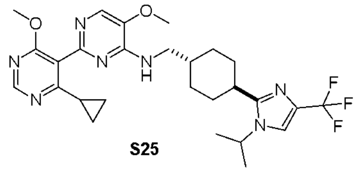
S25,

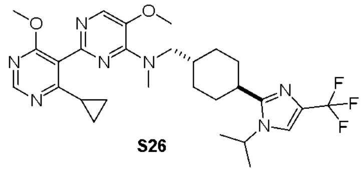
S26,

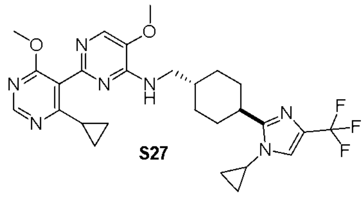
S27,

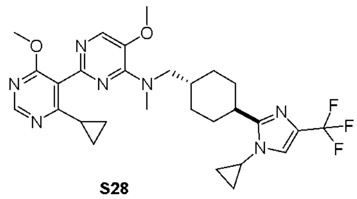
S28,

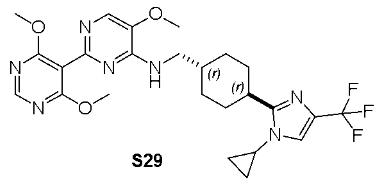
S29,

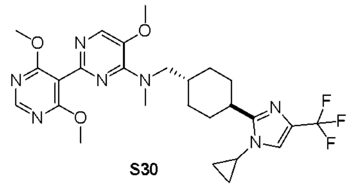
S30,

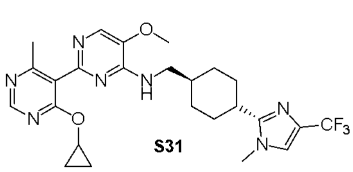
S31,

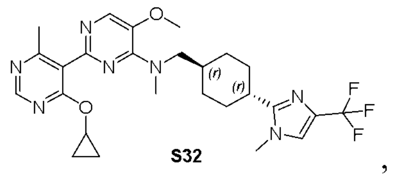
S32,

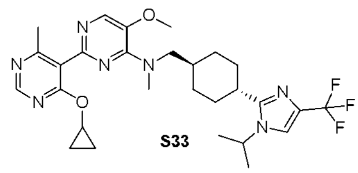
S33,

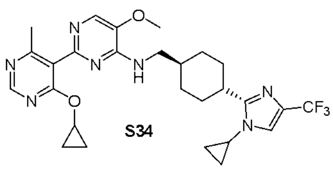
S34,

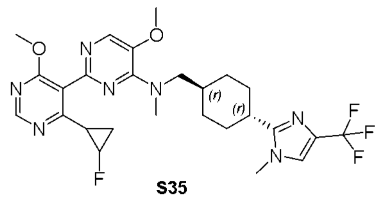
S35,

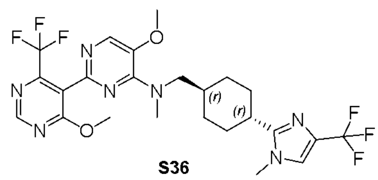
S36,

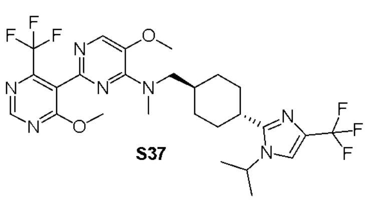
S37,

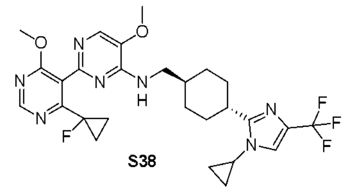
S38,

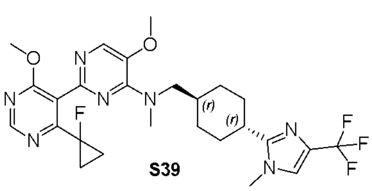
S39,

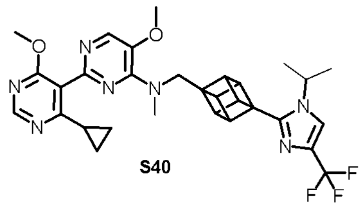
S40,

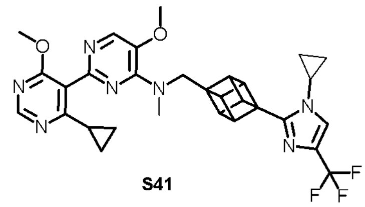
S41,

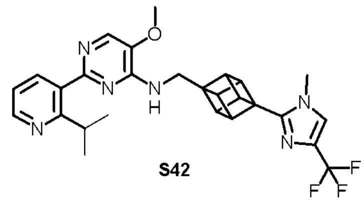
S42,

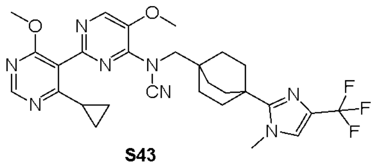
S43,
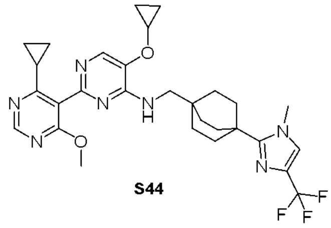
S44,
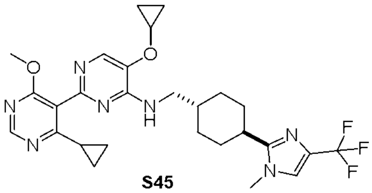
S45,
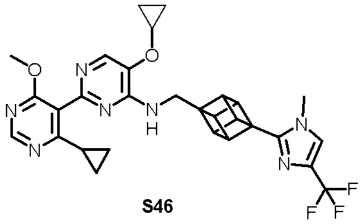
S46,
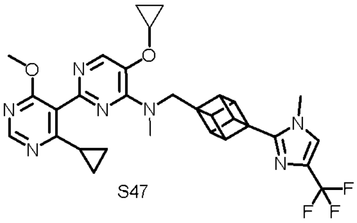
S47,
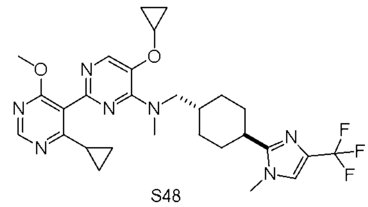
S48,
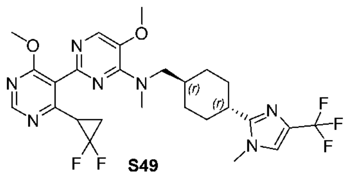
S49,
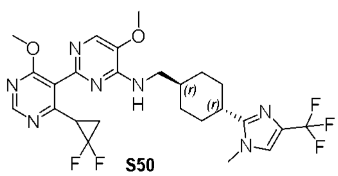
S50
and
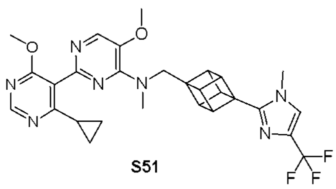
S51.

The present invention also provides a method for preparing a compound of formula (SI), comprising:

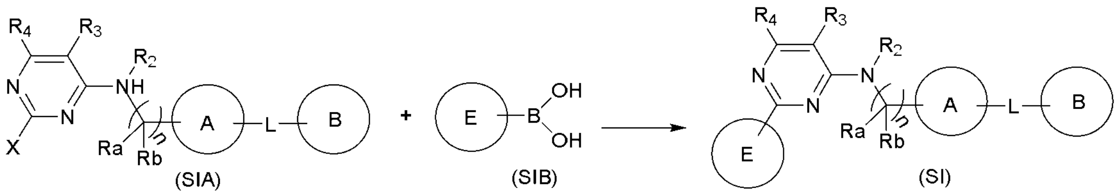
(SIA) (SIB) (SI)

reacting a compound of formula (SIA) with a compound of formula (SIB) to obtain the compound of formula (SI); X is a leaving group selected from the group consisting of halogen, sulfonate, boronic acid and borate; and ring A, ring B, ring E, L, $R_a$, $R_b$, $R_2$ to $R_4$ and n are as defined in formula (SI);
or

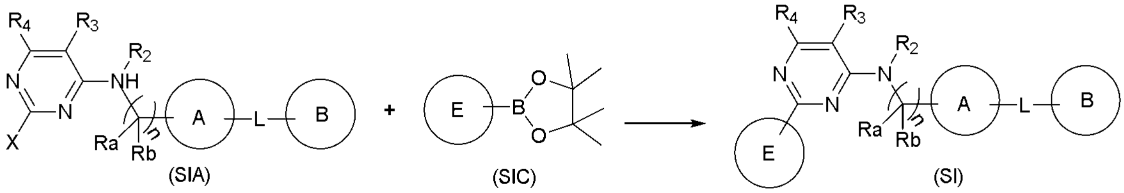
(SIA) (SIC) (SI)

reacting a compound of formula (SIA) with a compound of formula (SIC) to obtain the compound of formula (SI); X is a leaving group selected from the group consisting of halogen, sulfonate, boronic acid and borate; and ring A, ring B, ring E, L, $R_a$, $R_b$, $R_2$ to $R_4$ and n are as defined in formula (SI).

The present invention also provides a method for preparing a compound of formula (SII), comprising:

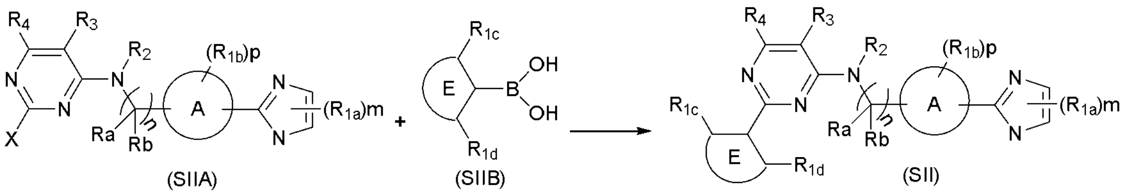

reacting a compound of formula (SIIA) with a compound of formula (SIIB) to obtain the compound of formula (SII); X is a leaving group selected from the group consisting of halogen, sulfonate, boronic acid and borate; and ring A, ring B, ring E, $R_a$, $R_b$, $R_2$ to $R_4$, $R_{1a}$, $R_{1b}$, $R_{1c}$, $R_{1d}$, m, n and p are as defined in formula (SII);

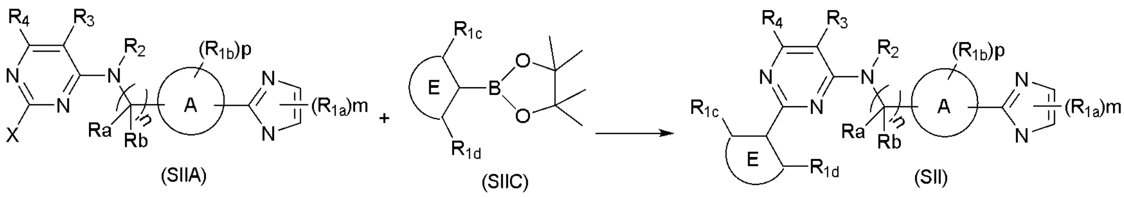

reacting a compound of formula (SIIA) with a compound of formula (SIIC) to obtain the compound of formula (SII); X is a leaving group selected from the group consisting of halogen, sulfonate, boronic acid and borate; and ring A, ring B, ring E, Ra, Rb, R2 to R4, $R_{1a}$, $R_{1b}$, $R_{1c}$, $R_{1d}$, m, n and p are as defined in formula (SII).

The present invention also provides a pharmaceutical composition comprising at least one compound of formula (I'), (I), (II), (III), (SI) or (SII), and one or more pharmaceutically acceptable excipients.

The present invention relates to a use of the compound of formula (I'), (I), (II), (III), (SI) or (SII) or the pharmaceutical composition comprising the same in the preparation of a medicament for treating or preventing diseases or conditions associated with inhibition of ubiquitin-specific protease 1 (USP1).

The present invention relates to a use of the compound of formula (I'), (I), (II), (III), (SI) or (SII) or the pharmaceutical composition comprising the same in the preparation of a medicament for treating or preventing a cancer, specifically selected from the group consisting of lung cancer, non-small cell lung cancer (NSCLC), colon cancer, bladder cancer, osteosarcoma, ovarian cancer, skin cancer, and breast cancer.

The present invention relates to the compound of formula (I'), (I), (II), (III), (SI) or (SII) or the pharmaceutical composition comprising the same, for use as a medicament.

The present invention relates to the compound of formula (I'), (I), (II), (III), (SI) or (SII) or the pharmaceutical composition comprising the same, for use in the treatment or prevention of diseases or conditions associated with inhibition of ubiquitin-specific protease 1 (USP1).

The present invention relates to the compound of formula (I'), (I), (II), (III), (SI) or (SII) or a pharmaceutical composition comprising the same, for use in the treatment or prevention of a cancer, specifically selected from the group consisting of lung cancer, non-small cell lung cancer (NSCLC), colon cancer, bladder cancer, osteosarcoma, ovarian cancer, skin cancer, and breast cancer.

The present invention relates to a method for treating or preventing diseases or conditions associated with inhibition of ubiquitin-specific protease 1 (USP1), comprising administering a therapeutically effective amount of the compound of formula (I'), (I), (II), (III), (SI) or (SII) or the pharmaceutical composition comprising the same to a patient in need thereof.

The present invention also relates to a method for for treating or preventing a cancer, comprising administering a therapeutically effective amount of the compound of formula (I'), (I), (II), (III), (SI) or (SII) or the pharmaceutical composition comprising the same to a patient in need thereof, wherein the cancer is specifically selected from the group consisting of lung cancer, non-small cell lung cancer (NSCLC), colon cancer, bladder cancer, osteosarcoma, ovarian cancer, skin cancer, and breast cancer.

The pharmaceutical composition of the present invention can be various conventional dosage forms, for example, tablet, aqueous suspension, oily suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

It is well known to those skilled in the art that the administration dose of a drug depends on a variety of factors including, but not limited to the following factors: activity of a specific compound used, age of the patient, weight of the patient, general health of the patient, behavior of the patient, diet of the patient, administration time, administration route, excretion rate, drug combination and the like. In addition, the optimal treatment, such as treatment mode, daily dose of the

compound or the type of pharmaceutically acceptable salt thereof can be verified according to the traditional therapeutic regimens.

**Definition of terms**

Unless otherwise specified, the following terms and phrases used herein are intended to have the following meanings. A particular term or phrase should not be considered ambiguous or unclear without special definition, but should be understood according to the ordinary meaning.

The term "pharmaceutically acceptable" refers to those compounds, materials, compositions and/or dosage forms which are, within the scope of sound medical judgment, suitable for use in contact with the tissues of human beings and animals without excessive toxicity, irritation, allergic response or other problems or complications, commensurate with a reasonable benefit/risk ratio.

The term "pharmaceutically acceptable salt" refers to salts of compounds of the present invention, prepared from the compounds of the present invention having specific substituents and relatively nontoxic acids or bases. When the compounds of the present invention contain relatively acidic functional groups, base addition salts can be obtained by contacting such compounds with a sufficient amount of base in a pure solution or a suitable inert solvent. Pharmaceutically acceptable base addition salts include sodium, potassium, calcium, ammonium, organic amine or magnesium salts or similar salts. When the compounds of the present invention contain relatively basic functional groups, acid addition salts can be obtained by contacting such compounds with a sufficient amount of acid in a pure solution or a suitable inert solvent. Examples of pharmaceutically acceptable acid addition salts include inorganic acid salts, the inorganic acid including, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, etc.; and organic acid salts, the organic acid including, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid and methanesulfonic acid, etc.; and also include salts of amino acids (such as arginine, etc.), and salts of organic acids such as glucuronic acid. Certain specific compounds of the present invention contain basic and acidic functional groups, and can be converted into any base or acid addition salt.

The term "isomer" refers to compounds having the same composition and molecular weight, but different physical and/or chemical properties. The structural differences may lie in the configuration (geometric isomers) or the ability to rotate the plane of polarized light (stereoisomers). With respect to stereoisomers, the compounds of formula (I) may have one or more asymmetric carbon atoms and may exist as racemates, racemic mixtures and as individual enantiomers or diastereomers.

The term "optional" or "optionally" means that the event or circumstance described subsequently can, but not necessarily, occur, and such a description includes the situation in which the event or circumstance occurs and the situation in which the event or circumstance does not occur.

The term "solvate" refers to a complex of variable stoichiometry formed by a solute and a solvent. Such a solvent for the purpose of the present application may not interfere with the biological activity of the solute. Examples of suitable solvents include, but are not limited to, water, MeOH, EtOH, and AcOH. Solvates with water as a solvent molecule are commonly referred to as hydrates. Hydrates include compositions containing stoichiometric amounts of water, and compositions containing variable amounts of water.

As used herein, the term "disease" or "disorder" or "condition" refers to a condition for which treatment is needed and/or desired, which is generally regarded as a pathological condition or a functionally disorder and/or abnormality, and which may manifest in a form of specific sign, symptom and/or dysfunction. The compounds of the present invention inhibit USP1 protein and are useful in treating diseases and conditions such as proliferative diseases, for which inhibition of USP1 protein would provide benefit.

"USP1" and "ubiquitin-specific processing protease 1" refer to any native polypeptide or polynucleotide encoding USP1. The term "USP1" encompasses USP1 polypeptides unprocessed over "full-length" and any form of USP1 resulting from processing within a cell (e.g., removal of a signal peptide). The term also encompasses variants of USP1 which naturally exist, such as those encoded by splice variants and allelic variants. The USP1 polypeptides described herein can be isolated from a variety of sources, such as from a human tissue type or from another source, or prepared by recombinant or synthetic methods.

The terms "cancer" and "tumor" refer to or describe a physiological condition in which a population of cells in a mammal is characterized by unregulated cell growth. The terms encompass solid cancers and hematologic/lymphoid cancers. Examples of cancers include, but are not limited to, cancers with defects in DNA damage repair pathways. Other examples of cancers include, but are not limited to, lung cancer, non-small cell lung cancer (NSCLC), colon cancer, bladder cancer, osteosarcoma, ovarian cancer, skin cancer, and breast cancer (including triple-negative breast cancer). The cancer can be BRCA1 or BRCA2 wild type. The cancer can also be BRCA1 or BRCA2 mutant type. The cancer can also be a PARP inhibitor-resistant or refractory cancer, or a PARP inhibitor-resistant or refractory BRCA1 or BRCA2 mutant

cancer.

Where any variable (such as R) appears more than once in the structure of a compound, its definition in each case is independent. For example, if a group is substituted by 0 to 2 R, the group can optionally be substituted by up to two R, and R in each case has independent options.

The term "alkyl" refers to a saturated linear or branched monovalent hydrocarbon group having 1 to 20 (for example, 1, 2, 3, 4, 5, 6, 7, 8, 9, 10, 11, 12, 13, 14, 15, 16, 17, 18, 19 and 20) carbon atoms, preferably a $C_{1-10}$ alkyl, and more preferably a $C_{1-6}$ alkyl. Examples of the alkyl include, but are not limited to, methyl, ethyl, n-propyl, isopropyl, n-butyl, isobutyl, tert-butyl, sec-butyl, n-pentyl, 2,2-dimethylpropyl, 2-methylbutyl, n-hexyl, 2,2-dimethylbutyl, 2-methylpentyl, 3-methylpentyl, 2,3-dimethylbutyl, n-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, n-nonyl, 2-methyl-3-ethylhexyl, n-decyl and 3,3-diethylhexyl.

The term "alkenyl" refers to a linear or branched monovalent hydrocarbon group having 2 to 6 (for example 2, 3, 4, 5 and 6) carbon atoms and at least one carbon-carbon double bond, in which the carbon-carbon double bond may be located anywhere within the alkenyl. The alkenyl is preferably a $C_{2-5}$ alkenyl. Examples of the alkenyl include, but are not limited to, $-CH=CH_2$, $-CH=CH-CH_3$, $-CH_2-CH=CH_2$, $-CH=CH-CH_2-CH_3$, $-CH_2-CH=CH-CH_3$, $-CH=CH-CH=CH_2$, $-CH=C(CH_3)-CH_3$ and $-CH_2-C(CH_3)=CH_2$.

The term "alkynyl" refers to a linear or branched monovalent hydrocarbon group having 2 to 6 (for example 2, 3, 4, 5 and 6) carbon atoms and at least one carbon-carbon triple bond, in which the carbon-carbon triple bond may be located anywhere within the alkynyl. The alkynyl is preferably a $C_{2-5}$ alkynyl. Examples of the alkynyl include, but are not limited to, $-C \equiv CH$, $-C \equiv C-CH_3$, $-CH_2-C \equiv CH$, $-C \equiv C-CH_2-CH_3$, $-CH_2-CH_2-C \equiv CH$, $-CH(CH_3)C \equiv CH$ and $-CH_2-C \equiv C-CH_3$.

The term "cycloalkyl" includes two categories, one is conventional cycloalkyl, and the other is heterostructured cycloalkyl.

The conventional cycloalkyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) carbon atoms, preferably a $C_{3-12}$ conventional cycloalkyl, more preferably a $C_{3-10}$ conventional cycloalkyl, further preferably a $C_{3-8}$ conventional cycloalkyl, and most preferably a $C_{3-6}$ conventional cycloalkyl. The conventional cycloalkyl optionally comprises one or more double or triple bonds.

The conventional cycloalkyl may be a monocyclic cycloalkyl, and examples of the monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl and cyclooctyl. The conventional cycloalkyl may also be a polycyclic cycloalkyl (for example, bicycloalkyl, tricycloalkyl, tetracycloalkyl and pentacycloalkyl), and the polycyclic cycloalkyl includes spiro cycloalkyl, fused cycloalkyl and bridged cycloalkyl.

The term "spiro cycloalkyl" refers to a 5 to 20 membered (for example, 5, 6, 7, 8, 9, 10, 11, 12, 13, 14, 15, 16, 17, 18, 19 and 20 membered) spiro cycloalkyl, preferably a 6 to 14 membered spiro cycloalkyl, and more preferably a 7 to 10 membered spiro cycloalkyl. The spiro cycloalkyl may be a mono-spiro cycloalkyl, a di-spiro cycloalkyl, or a poly-spiro cycloalkyl, preferably a mono-spiro cycloalkyl, and more preferably a 4-membered/4-membered, 4-membered/5-membered, 4-membered/6-membered, 5-membered/5-membered or 5-membered/6-membered mono-spiro cycloalkyl. Examples of the spiro cycloalkyl include, but are not limited to:

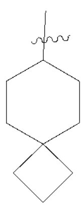 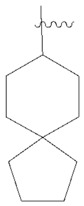 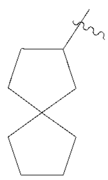 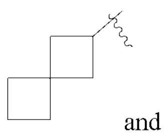 and 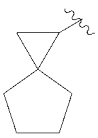 .

The term "fused cycloalkyl" refers to a 5 to 20 membered (for example, 5, 6, 7, 8, 9, 10, 11, 12, 13, 14, 15, 16, 17, 18, 19 and 20 membered) fused cycloalkyl, preferably a 6 to 14 membered fused cycloalkyl, and more preferably a 7 to 10 membered fused cycloalkyl. The fused cycloalkyl may be a bicyclic, tricyclic, tetracyclic, pentacyclic or more fused cycloalkyl, preferably a bicyclic or tricyclic fused cycloalkyl, and more preferably a 5-membered/5-membered, or 5-membered/6-membered fused cycloalkyl. Examples of the fused cycloalkyl include, but are not limited to:

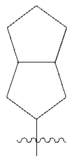 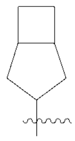 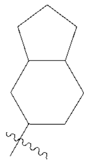 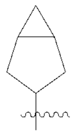 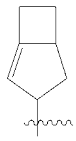 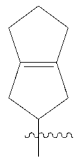 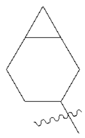 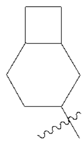 and 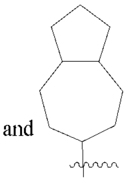.

The term "bridged cycloalkyl" refers to a 5 to 20 membered (for example, 5, 6, 7, 8, 9, 10, 11, 12, 13, 14, 15, 16, 17, 18, 19 and 20 membered) bridged cycloalkyl, preferably a 6 to 14 membered bridged cycloalkyl, and more preferably a 7 to 10 membered bridged cycloalkyl. The bridged cycloalkyl may be a bicyclic, tricyclic, tetracyclic, pentacyclic or more bridged cycloalkyl, preferably a bicyclic, tricyclic or tetracyclic bridged cycloalkyl, and more preferably a bicyclic or tricyclic bridged cycloalkyl. Examples of the bridged cycloalkyl include, but are not limited to:

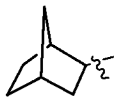 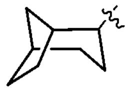 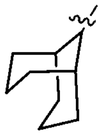 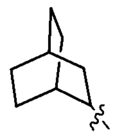 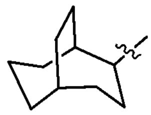

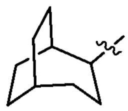 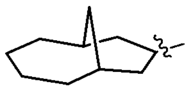 and 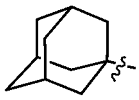

The term "heterostructured cycloalkyl" includes a monocyclic cycloalkyl, spiro cycloalkyl, fused cycloalkyl and bridged cycloalkyl fused with any one selected from the group consisting of conventional aryl, conventional heteroaryl and conventional heterocyclyl, in which the attachment point is located on the corresponding conventional cycloalkyl (referring to a monocyclic cycloalkyl, spiro cycloalkyl, fused cycloalkyl or bridged cycloalkyl). Examples of the heterostructured cycloalkyl include, but are not limited to:

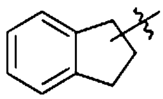 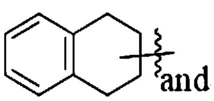 and 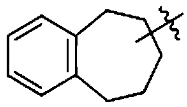.

The term "heterocyclyl" includes two categories, one is conventional heterocyclyl, and the other is heterostructured heterocyclyl.

The conventional heterocyclyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) ring atoms, in which one or more ring atoms are replaced by one or more elements selected from the group consisting of N, O, S, S(O) and $S(O)_2$, and the replacement does not result in formation of -O-O-, -O-S- or -S-S-. The conventional heterocyclyl is preferably a $C_{3-12}$ conventional heterocyclyl in which 1 to 4 (for example 1, 2, 3 and 4) atoms are heteroatoms; more preferably a $C_{3-8}$ conventional heterocyclyl in which 1 to 3 (for example 1, 2 and 3) atoms are heteroatoms; and most preferably a $C_{5-7}$ conventional heterocyclyl in which 1 to 2 or 1 to 3 atoms are heteroatoms.

The conventional heterocyclyl may be a monocyclic heterocyclyl, and examples of the monocyclic heterocyclyl include, but are not limited to oxetanyl, 3-pyrrolinyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and pyranyl, and preferably 1,2,5-oxadiazolyl, pyranyl or morpholinyl. The conventional heterocyclyl may also be a polycyclic heterocyclyl and the polycyclic heterocyclyl includes spiro heterocyclyl, fused heterocyclyl and bridged heterocyclyl.

The term "spiro heterocyclyl" refers to a 5 to 20 membered (for example, 5, 6, 7, 8, 9, 10, 11, 12, 13, 14, 15, 16, 17, 18, 19 and 20 membered) spiro heterocyclyl, preferably a 6 to 14 membered spiro heterocyclyl, and more preferably a 7 to 10 membered spiro heterocyclyl. The spiro heterocyclyl may be a mono-spiro heterocyclyl, a di-spiro heterocyclyl, or a poly-spiro heterocyclyl, preferably a mono-spiro heterocyclyl or a di-spiro heterocyclyl, and more preferably a 3-membered/6-membered, 4-membered/4-membered, 4-membered/5-membered, 4-membered/6-membered, 5-membered/5-membered or 5-membered/6-membered mono-spiro heterocyclyl. Examples of the spiro heterocyclyl include, but are not limited to:

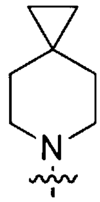 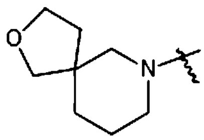 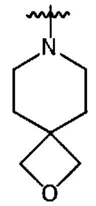 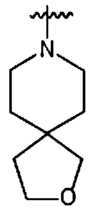 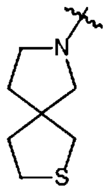 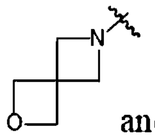 and 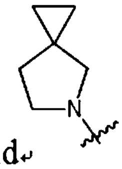

.

The term "fused heterocyclyl" refers to a 5 to 20 membered (for example, 5, 6, 7, 8, 9, 10, 11, 12, 13, 14, 15, 16, 17, 18, 19 and 20 membered) fused heterocyclyl, preferably a 6 to 14 membered fused heterocyclyl, and more preferably a 7 to 10 membered fused heterocyclyl. The fused heterocyclyl may be a bicyclic, tricyclic, tetracyclic, pentacyclic or more fused heterocyclyl, preferably a bicyclic or tricyclic fused heterocyclyl, and more preferably a 5-membered/5-membered or 5-membered/6-membered bicyclic fused heterocyclyl. Examples of the fused heterocyclyl include, but are not limited to:

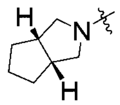 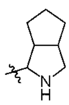 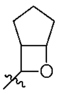 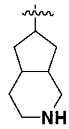 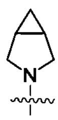 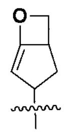 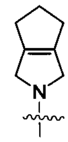 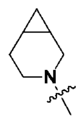

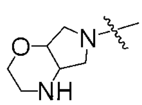 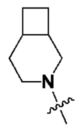 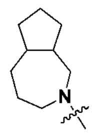 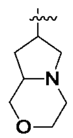 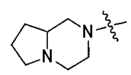 and 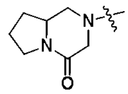 .

The term "bridged heterocyclyl" refers to a 5 to 14 membered (for example, 5, 6, 7, 8, 9, 10, 11, 12, 13 and 14 membered) bridged heterocyclyl, preferably a 6 to 14 membered bridged heterocyclyl, and more preferably a 7 to 10 membered bridged heterocyclyl. The bridged heterocyclyl may be a bicyclic, tricyclic, tetracyclic, pentacyclic or more bridged heterocyclyl, preferably a bicyclic, tricyclic or tetracyclic bridged heterocyclyl, and more preferably a bicyclic or tricyclic bridged heterocyclyl. Examples of the bridged heterocyclyl include, but are not limited to:

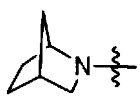 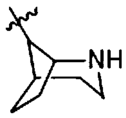 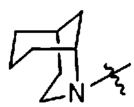 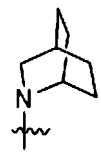

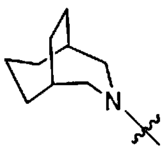 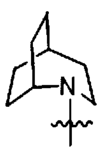 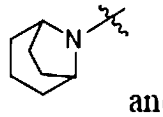 and 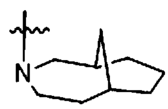

.

The term "heterostructured heterocyclyl" includes a monocyclic heterocyclyl, spiro heterocyclyl, fused heterocyclyl and bridged heterocyclyl fused with any one selected from the group consisting of conventional aryl, conventional heteroaryl and conventional cycloalkyl, in which the attachment point is located on the corresponding conventional heterocyclyl (referring to a monocyclic heterocyclyl, spiro heterocyclyl, fused heterocyclyl or bridged heterocyclyl). Examples of the heterostructured heterocyclyl include, but are not limited to:

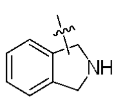 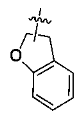 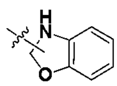 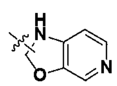 and 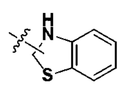 .

The term "aryl" includes two categories, one is conventional aryl, and the other is heterostructured aryl.

The conventional aryl refers to a 6 to 14 membered (for example, 6, 7, 8, 9, 10, 11, 12, 13 and 14 membered) aromatic hydrocarbon group, preferably a $C_{6-10}$ conventional aryl, and more preferably phenyl, naphthyl, phenanthryl or anthracenyl.

The term "heterostructured aryl" includes a conventional aryl fused with any one selected from the group consisting of conventional heteroaryl, conventional heterocyclyl and conventional cycloalkyl, in which the attachment point is located on the conventional aryl. Examples of the heterostructured aryl include, but are not limited to:

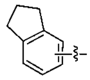 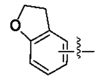 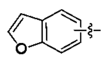 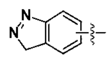 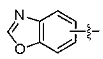 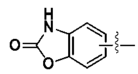 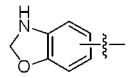 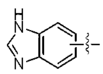

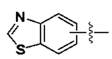 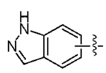 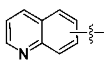 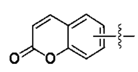 and 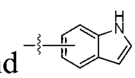.

The term "heteroaryl" includes two categories, one is conventional heteroaryl, and the other is heterostructured heteroaryl.

The conventional heteroaryl refers to a 5 to 14 membered (for example, 5, 6, 7, 8, 9, 10, 11, 12, 13 and 14 membered) aromatic hydrocarbon group in which 1 to 4 (for example, 1, 2, 3 and 4) carbon atoms are replaced by heteroatoms selected from the group consisting of oxygen, sulfur and nitrogen. Preferably, the number of ring atoms is 5 to 10, including 1 to 3 (for example, 1, 2 and 3) heteroatoms. More preferably, the number of ring atoms is 5 or 6, including 1 to 2 heteroatoms. Examples of the conventional heteroaryl include, but are not limited to imidazolyl, furanyl, thienyl, thiazolyl, pyrazolyl, oxazolyl, pyrrolyl, tetrazolyl, pyridyl, pyrimidinyl, thiadiazolyl and pyrazinyl, preferably imidazolyl, thiazolyl, pyrazolyl, pyrimidinyl or thiazolyl, and more preferably pyrazolyl or thiazolyl.

The term "heterostructured heteroaryl" includes a conventional heteroaryl fused with any one selected from the group consisting of conventional aryl, conventional cycloalkyl and conventional heterocyclyl, in which the attachment point is located on the conventional heteroaryl. Examples of the heterostructured heteroaryl include, but are not limited to:

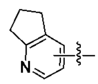 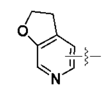 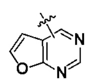 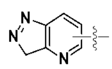 and 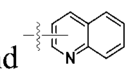.

The term "alkoxy" includes -O-alkyl and -O-cycloalkyl, in which the "alkyl" and "cycloalkyl" are as defined above. Examples of the alkoxy include, but are not limited to: methoxy, ethoxy, propoxy, butoxy, cyclopropyloxy, cyclobutyloxy, cyclopentyloxy and cyclohexyloxy.

The term "haloalkyl" refers to an alkyl substituted by one or more halogens, in which the alkyl is as defined above.

The term "haloalkoxy" refers to an alkoxy substituted by one or more halogens, in which the alkoxy is as defined above.

The term "hydroxy" refers to an -OH group.

The term "halogen" refers to a -F, -Cl, -Br or -I group.

The term "amino" refers to a $-NH_2$ group.

The term "cyano" refers to a -CN group.

The term "nitro" refers to a $-NO_2$ group.

The term "oxo" refers to an =O group.

The term "carboxy" refers to a -C(= O)OH group.

The term "thiol" refers to a -SH group.

The term "alkoxycarbonyl" refers to a -C(=O)O-alkyl or -C(=O)O-cycloalkyl group, in which the alkyl and cycloalkyl are as defined above.

The term "acyl" refers to a -C(= O)R group, in which R is selected from the group consisting of alkyl, cycloalkyl, heterocyclyl, aryl and heteroaryl.

The term "protecting group for hydroxy" refers to a group that is introduced on a hydroxy and is easily removed, for blocking or protecting the hydroxy while a reaction takes place on other functional group(s) of a compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, etc.

The symbol

“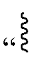”

refers to an attachment point.

The term "stereoisomer" refers to isomers with the same structure but different arrangements of atoms in space. It includes cis and trans (or Z and E) isomers, (-)- and (+)-isomers, (R)- and (S)-enantiomers, diastereomers, (D)- and (L)-isomers, tautomers, atropisomers, conformers and mixtures thereof (e.g., mixtures of racemates and diastereomers). Substituents in the compounds of the present invention may have additional asymmetric atoms. All of these stereoisomers and mixtures thereof are included within the scope of the present invention. Optically active (-)- and (+)-isomers, (R)- and (S)-enantiomers and (D)- and (L)-isomers may be prepared by chiral synthesis, chiral reagents or other conventional techniques. An isomer of a compound of the present invention can be prepared by asymmetric synthesis or chiral auxiliary, or, when the molecule contains a basic functional group (such as amino) or an acidic functional group (such as carboxyl), it can be formed into a diastereoisomer salt with an appropriate optically active acid or base, and then the diastereoisomers can be resolved by conventional methods known in the art to obtain pure isomers. In addition, the resolution of enantiomers and diastereomers is usually achieved by chromatography.

In the chemical structures of the compounds of the present invention, the bond "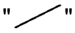" represents an unspecified configuration, and that is, if chiral isomers exist in the chemical structures, the bond "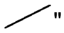" may be "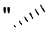" or "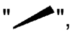", or contains both "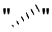" and "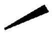" configurations. For all of carbon-carbon double bonds, even if only one configuration is named, both Z and E configurations are included.

The compounds and intermediates of the present invention may also exist in different tautomeric forms, and all such forms are included within the scope of the present invention. The term "tautomer" or "tautomeric form" refers to structural isomers of different energies that can be interconverted via a low energy barrier. For example, proton tautomers (also known as prototropic tautomers) include interconversions via proton migration, such as keto-enol and imine-enamine, lactam-lactimide isomerizations.

The compounds of the present invention includes all suitable isotope substitutes of the compounds. The term "isotope substitute" refers to a compound in which at least one atom is replaced by an atom having the same atomic number but different atomic mass. Examples of isotopes that can be incorporated into the compounds of the present invention include stable and radioactive isotopes of hydrogen, carbon, nitrogen, oxygen, phosphorus, sulphur, fluorine, chlorine, bromine, iodine, etc, such as $^{2}H$ (deuterium, D), $^{3}H$ (tritium, T), $^{11}C$, $^{13}C$, $^{14}C$, $^{15}N$, $^{17}O$, $^{18}O$, $^{32}p$, $^{33}p$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, $^{131}I$, etc., preferably deuterium.

As used herein, the singular forms of "a," "an," and "the" include plural references, and vice versa, unless the context clearly dictates otherwise.

When the term "about" is applied to a parameter such as pH, concentration, temperature, etc., it indicates that the parameter may vary by $\pm 10\%$, and is sometimes more preferably within $\pm 5\%$. As will be appreciated by those skilled in the art, a number is generally given only for illustrative purpose, instead of being limiting.

## DETAILED DESCRIPTION OF THE INVENTION

The present invention will be described in more detail through examples below, but it does not mean that any adverse limitation is imposed on the present invention. The present document has described the present invention in detail, and also has disclosed specific embodiments. It will be apparent to those skilled in the art that various changes and modifications can be made in the specific embodiments of the present invention without departing from the spirit and scope of the present invention.

The compounds of the present invention are prepared by using convenient starting materials and general preparation procedures. The present invention provides typical or preferential reaction conditions, such as reaction temperature, time, solvent, pressure and molar ratio of reactants. However, unless specially specified, other reaction conditions can also be adopted. Optimal conditions may vary with the use of specific reactants or solvents, but under normal circumstances, the steps and conditions optimized for reaction can be determined.

In addition, some protecting groups may be used in the present invention to protect certain functional groups from unnecessary reactions. The protecting groups suitable for various functional groups and their protection or deprotection conditions are already well known to those skilled in the art.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific methods used may be referred to the examples described in the present invention. Of course, other similar isolation and purification methods can also be used. They can be characterized using conventional methods (including physical constants and spectral data).

The purity analysis method is as follows: a Kinetex EVO C18 (50×4.6 mm, 5 μm, 100Å) chromatographic column is used, acetonitrile-water is used as the mobile phase for gradient elution, the flow rate is 1.5 mL/min, and the detection

wavelength is 220 nm.

MS is determined on a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400MHz.

Preparative liquid chromatography is determined using an Agilent 1260 Infinity II high performance liquid chromatograph (manufacturer: Agilent). The chromatographic column is Daisogel C18 10 μm 100A (30 mm×250 mm), and the mobile phase is acetonitrile/water.

GF254 silica gel plates from Qingdao Haiyang Chemical are used for thin-layer chromatography (TLC). For the silica gel plates used, the specification is 0.20 mm to 0.25 mm for the thin-layer chromatography for monitoring of reactions, and 0.5 mm for the thin-layer chromatography for separation and purification.

Silica gels of 100 to 200 mesh, 200 to 300 mesh and 300 to 400 mesh from Qingdao Haiyang Chemical are used as a carrier for silica gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech and the like.

Unless specifically stated in the examples, all reactions are carried out under nitrogen atmosphere.

Nitrogen atmosphere means that a reaction flask is connected to a nitrogen balloon with a volume of about 1 L.

The reaction solvent, organic solvent or inert solvent is each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP).

Unless specifically stated in the examples, a solution refers to an aqueous solution.

The chemical reactions described in the present invention are generally carried out under normal pressure. The reaction time and conditions are, for example, between - 78° C and 200° C at one atmosphere, and completed within about 1 to 24 hours. If the reaction is carried out overnight, the reaction time is generally 16 hours. Unless specifically stated in the examples, the reaction temperature is room temperature, which is 20°C to 30°C.

Unless otherwise defined, all professional and scientific terms used herein have the same meaning as those familiar to those skilled in the art. In addition, any methods and materials similar or equivalent to the content described herein can be applied to the methods of the present invention.

Unless specifically stated, the mixing ratios of different solvents are volume ratios.

Hereinafter, the synthesis of general intermediates, including A series, AA series, B series, BB series, C series, D series, SA series, SB series, SC series and further synthesized intermediate series, will be described respectively.

**Synthesis of general intermediate A1 (2-(2-isopropylphenyl)-5-methoxypyrimidin-4-amine)**

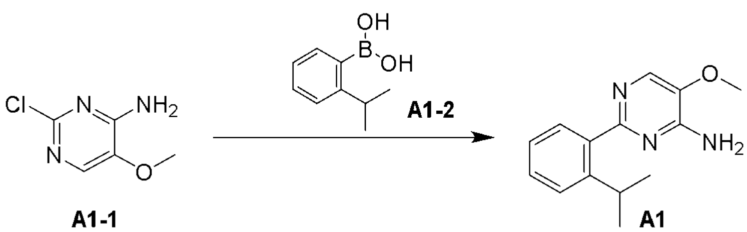


A1-1 A1

2-Chloro-5-methoxypyrimidin-4-amine (compound **Al-1,** 5.0 g, 31.3 mmol, 1.00 eq), (2-isopropylphenyl)boric acid (compound **A1-2,** 6.7 g, 40.7 mmol, 1.30 eq, purchased from Bide Pharmatech), potassium carbonate (13.0 g, 94.0 mmol, 3.00 eq) and 1,1'-bis(diphenylphosphino)ferrocene palladium dichloride (2.3 g, 3.13 mmol, 0.10 eq) were added to dioxane (50.0 mL) and water (12.5 mL) successively. The resulting mixture was purged with nitrogen three times, and reacted at 100°C under nitrogen atmosphere for 16 hours. Then, ethyl acetate (500.0 mL) was added, and the resulting mixture was stirred for 10 minutes and filtered to obtain a filtrate. The filtrate was concentrated under vacuum to obtain a crude product, which was purified by reverse phase HPLC (0.1% $NH_3$•$H_2O$) to obtain compound **A1** (4.9 g, 63.3% yield, 98.2% purity) as a light yellow solid. $^1$H NMR (400 MHz, DMSO) δ 7.93 (s, 1H), 7.32-7.36 (m, 3H), 7.16-7.19 (m, 1H), 6.71 (s, 2H), 3.87 (s, 3H), 3.44-3.50 (m, 1H), 1.12 (d, *J* = 7.20 Hz, 6H); LC-MS: m/z = 244.2 $(M+H)^+$.

**Synthesis of general intermediate A2 (4'-cyclopropyl-5,6'-dimethoxy-N-methyl-[2,5'-bipyrimidin]-4-amine)**

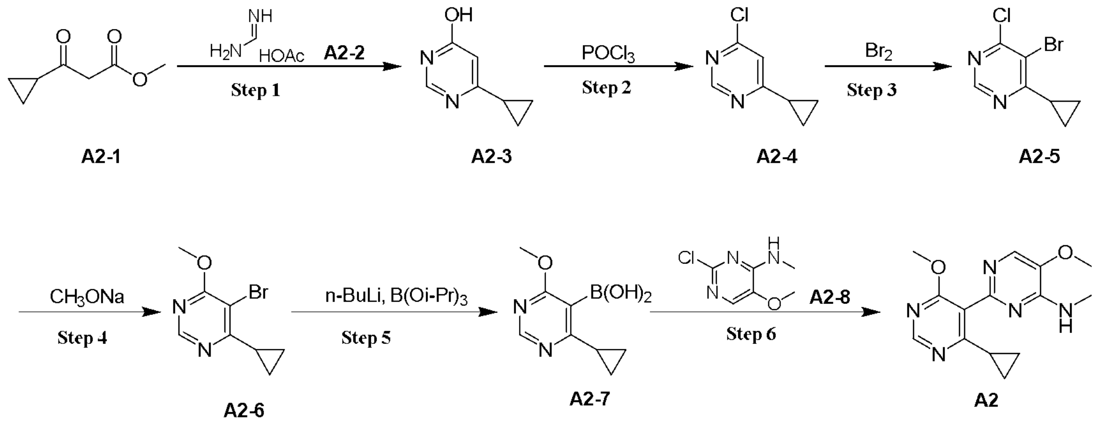


A2-1 A2-3 A2-4 A2-5

A2-6 A2-7 A2

Step 1: Synthesis of compound **A2-3** (6-cyclopropylpyrimidin-4-ol)

Compound **A2-1** (200.0 g, 1.4 mol, 1.00 *eq)* and compound **A2-2** (292.0 g, 2.81 mol, *2.00 eq)* were added to methanol (1.2 L) successively, and a solution of sodium methoxide in methanol (5.4 M, 1.3 L, 5.00 *eq)* was added in portions at 0°C. After the addition was completed, the reaction was heated up to 20°C and stirred for 13 hours. Then, glacial acetic acid was added at 0°C to adjust the pH to 7-8. The resulting mixture was concentrated under vacuum to obtain a crude product, which was purified by column chromatography (eluent: petroleum ether/ethyl acetate = 1/0 - 1/1) to obtain compound **A2-3** (90.3 g). LC-MS: m/z =137.1 $(M+H)^+$.

Step 2: Synthesis of compound **A2-4** (4-chloro-6-cyclopropylpyrimidine)

Compound **A2-3** (40.0 g, 293.0 mmol, 1.00 *eq*) was added to phosphorus oxychloride (180.0 mL) in portions. After the addition was completed, the reaction was heated up to 60°C and stirred for 2 hours. The reaction solution was concentrated under vacuum to obtain a crude product. The crude product was dissolved in ethyl acetate (400.0 mL) and water (400.0 mL), and stirred for 5 minutes. The organic layer was separated, and the aqueous layer was extracted twice with ethyl acetate (400.0 mL). The organic layers were combined, dried over anhydrous sodium sulfate, and filtered to obtain the filtrate. The filtrate was concentrated under vacuum to obtain a crude product, which was purified by column chromatography (eluent: petroleum ether/ethyl acetate = 20/1 - 1/1) to obtain compound **A2-4** (11.0 g). LC-MS: m/z = 155.0 $(M+H)^+$.

Step 3: Synthesis of compound **A2-5** (5-bromo-4-chloro-6-cyclopropylpyrimidine)

Compound **A2-4** (11.0 g, 71.1 mmol, 1.00 *eq)* was dissolved in methanol (150.0 mL), and bromine (34.1 g, 213.0 mmol, 3.00 *eq)* was slowly added at -60°C. After the addition was completed, the reaction was heated up to 20°C and stirred for 2 hours. Then, saturated sodium bicarbonate solution (200.0 mL) and water (100.0 mL) were added at 0°C, the resulting mixture was stirred for 5 minutes, and extracted with dichloromethane (200.0 mL) three times. The organic layers were combined, washed with saturated brine (200.0 mL) twice, dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 3/1) to obtain compound **A2-5** (7.8 g). $^1H$ NMR (400 MHz, $CDCl_3$) $\delta$ 8.61 (s, 1H), 2.56 - 2.62 (m, 1H), 1.23 - 1.26 (m, 2H), 1.16 - 1.21 (m, 2H); LC-MS: m/z = 232.9 $(M+H)^+$.

Step 4: Synthesis of compound **A2-6** (5-bromo-4-cyclopropyl-6-methoxypyrimidine)

Compound **A2-5** (7.8 g, 33.4 mmol, 1.00 *eq)* was dissolved in methanol (240.0 mL), and sodium methoxide (18.0 g, 100.0 mmol, 3.00 *eq)* was added at 0°C. After the addition was completed, the reaction was heated up to 30°C and stirred for 1 hour. The reaction solution was concentrated under vacuum to obtain a crude product, which was purified by column chromatography (eluent: petroleum ether/ethyl acetate = 10/1 - 5/1) to obtain compound **A2-6** (7.3 g). LC-MS: m/z =228.9 $(M+H)^+$.

Step 5: Synthesis of compound **A2-7** ((4-cyclopropyl-6-methoxypyrimidin-5-yl)boronic acid)

Compound **A2-6** (10.3 g, 44.9 mmol, 1.00 *eq)* and triisopropyl borate (11.8 g, 62.9 mmol, 1.40 *eq)* were dissolved in tetrahydrofuran (30.0 mL) and toluene (90.0 mL), and *n*-butyl lithium (2.5 M, 25.1 mL, 1.40 *eq)* was added dropwise at -70°C. After the addition was completed, the reaction was stirred for 3 hours at -70°C. Then, 1 N hydrochloric acid solution (50.0 mL) was added dropwise at -70°C. After the addition was completed, the reaction was heated up to 20°C and stirred for 0.5 hours. Saturated aqueous sodium bicarbonate solution was then added to adjust the pH to 7-8, and the resulting mixture was extracted with ethyl acetate (100.0 mL) three times. The organic layers were combined, washed with saturated brine (100.0 mL) three times, dried over anhydrous sodium sulfate, and filtered to obtain the filtrate, which was concentrated under vacuum to obtain compound **A2-7** (6.9 g). $^1H$ NMR (400 MHz, $CDCl_3$) δ 8.52 (s, 1H), 8.45 (s, 2H), 3.84 (s, 3H), 1.88 - 1.92 (m, 2H), 0.93 - 1.01 (m, 4H).

Step 6: Synthesis of intermediate **A2**

Compound **A2-8** (1.2 g, 6.9 mmol, 1.0 *eq),* 1,1'-bis(diphenylphosphino)ferrocene palladium dichloride (505.0 mg, 0.7 mmol, 0.10 *eq),* and potassium carbonate (2.9 g, 20.7 mmol, 3.00 *eq)* were added to dioxane (100.0 mL) and water (25.0 mL) successively, and the resulting mixture was purged three times with nitrogen. Compound **A2-7** (1.6 g, 8.3 mmol, 1.20 *eq)* dissolved in N,N-dimethylformamide (10.0 mL) was added dropwise at 100°C under nitrogen atmosphere. After the addition was completed, the reaction was reacted at 100°C for 2 hours under nitrogen atmosphere. Then, the reaction solution was concentrated under vacuum, followed by the addition of water (60.0 mL), and the resulting mixture was extracted twice with ethyl acetate (40.0 mL). The organic layers were combined, washed twice with saturated brine (40.0 mL), dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 10/1) to obtain compound **A2** (100.0 mg, 348 μmol, 5.0% yield, 100% purity) as a white solid. LC-MS: m/z =288.0 $(M+H)^+$.

**Synthesis of general intermediate A3 (4-chloro-2-(2-isopropylphenyl)-5-nitropyrimidine)**

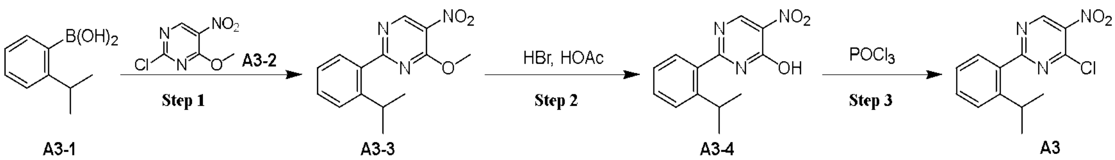


A3-1 A3-3 A3-4 A3

Step 1: Synthesis of compound **A3-3** (2-(2-isopropylphenyl)-4-methoxy-5-nitropyrimidine)

Compound **A3-2** (3.3 g, 17.4 mmol, 1.00 eq), compound **A3-1** (5.7 g, 34.8 mmol, 2.00 eq), 1,1'-bis(diphenylphosphino)ferrocene palladium dichloride (1.3 g, 1.7 mmol, 0.10 eq), and potassium carbonate (4.8 g, 34.8 mmol, 2.00 eq) were dissolved in dioxane (30.0 mL) and water (7.0 mL). The resulting mixture was purged three times with nitrogen, and reacted at 100°C for 12 hours under nitrogen atmosphere. Then, the reaction solution was concentrated under vacuum to obtain a crude product, which was purified by column chromatography (eluent: petroleum ether/ethyl acetate = 10/1 - 5/1) to obtain compound **A3-3** (3.2 g). LC-MS: m/z = 274.1 $(M+H)^+$.

Step 2: Synthesis of compound **A3-4** (2-(2-isopropylphenyl)-5-nitropyrimidin-4-ol)

Compound **A3-3** (2.0 g, 7.3 mmol, 1.00 *eq)* was dissolved in dioxane (30.0 mL), and hydrobromic acid and glacial acetic acid (6 M, 15.0 mL, 12.30 *eq)* was added. After the addition was completed, the reaction was heated up to 50°C and stirred for 1 hour. Then, the reaction solution was concentrated under vacuum to obtain crude compound **A3-4** (2.0 g), which was directly used in the next step. LC-MS: m/z = 260.0 $(M+H)^+$.

Step 3: Synthesis of intermediate **A3**

Compound **A3-4** (2.0 g, 6.8 mmol, 1.00 *eq)* was added to phosphorus oxychloride (18.0 mL) and reacted at 90°C for 2 hours. The reaction solution was concentrated under vacuum to obtain a crude product, which was purified by column chromatography (eluent: petroleum ether/ethyl acetate = 50/1 - 30/1) to obtain compound **A3** (1.0 g, 3.59 mmol, 53.1% yield, 99.8% purity) as a light yellow oil. LC-MS: m/z = 278.1 $(M+H)^+$.

**Synthesis of general intermediate A4 (2-(2-isopropylphenyl)-5-(trifluoromethyl)pyrimidin-4-amine)**

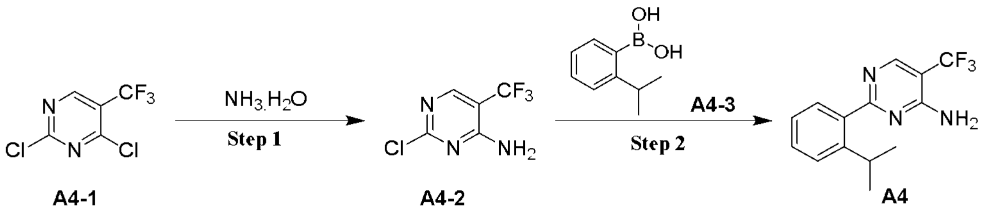


Step 1: Synthesis of compound **A4-2** (2-chloro-5-(trifluoromethyl)pyrimidin-4-amine)

Compound **A4-1** (18.7 g, 86.2 mmol, 1.00 eq) was dissolved in tetrahydrofuran (20.0 mL), and aqueous ammonia (15.1 g, 129 mmol, 30.0% purity, 1.50 eq) was added at 0°C. After the addition was completed, the reaction was heated up to 20°C and stirred for 16 hours. Then, water (30.0 mL) was added, and the resulting mixture was extracted with ethyl acetate (30.0 mL) three times. The organic layers were combined, dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 0/1) to obtain compound **A4-2** (1.73 g). $^1$H NMR (400 MHz, DMSO) δ 8.56 (s, 1H), 7.95 (s, 2H); LC-MS: m/z = 197.9 (M+H)$^+$.

Step 2: Synthesis of intermediate **A4**

Compound **A4-2** (1.15 g, 5.77 mmol, 1.00 eq), compound **A4-3** (1.14 g, 6.92 mmol, 1.20 eq), potassium carbonate (2.39 g, 17.3 mmol, 3.00 eq), and 1,1'-bis(diphenylphosphino)ferrocene palladium dichloride (422 mg, 577 μmol, 0.10 eq) were added to dimethyl sulfoxide (12.0 mL) and water (2.5 mL) successively. The resulting mixture was purged three times with nitrogen, and reacted at 100°C for 12 hours under nitrogen atmosphere. Then, the reaction solution was concentrated under vacuum to obtain a crude product, which was purified by preparative HPLC to obtain compound **A4** (200 mg, 711 μmol, 12.3% yield, 100% purity) as a light yellow oil. $^1$H NMR (400 MHz, DMSO) δ 8.57 (s, 1H), 7.41 - 7.44 (m, 3H), 7.22 - 7.26 (m, 1H), 3.41 - 3.45 (m, 1H), 3.30 (s, 2H), 1.16 (dd, $J_1$ = 7.2Hz, $J_2$ = 13.2Hz, 6H); LC-MS: m/z = 282.1 (M+H)$^+$.

**Synthesis of general intermediate A5 (2-(2-isopropylphenyl)-5-methoxy-N-methylpyrimidin-4-amine)**

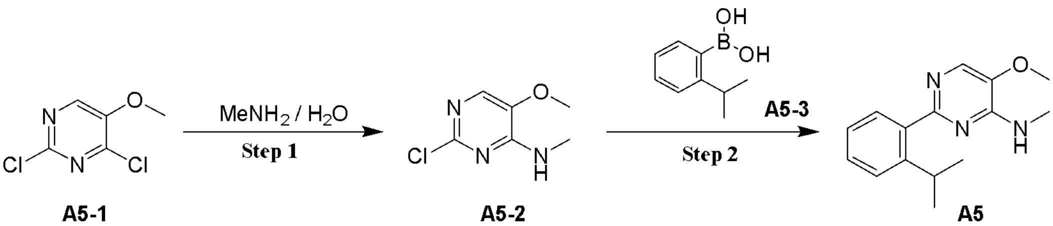


Step 1: Synthesis of compound **A5-2** (2-chloro-5-methoxy-N-methylpyrimidin-4-amine)

Compound **A5-1** (10.0 g, 55.9 mmol, 1.00 eq) was dissolved in tetrahydrofuran (100.0 mL), and methylamine (2.0 M methylamine aqueous solution, 54.5 mL, 1.95 eq) was added at 0°C. After the addition was completed, the reaction was heated up to 20°C and stirred for 2 hours. Water (200.0 mL) was added. and the resulting mixture was stirred for 5 minutes, and extracted with ethyl acetate (100.0 mL) three times. The organic layers were combined, dried over anhydrous sodium sulfate, and filtered to obtain the filtrate, which was concentrated under vacuum to obtain compound **A5-2** (9.0 g). $^1$H NMR (400 MHz, $CDCl_3$) δ 7.50 (s, 1H), 5.48 (s, 1H), 3.86 (s, 3H), 3.05 (d, $J$ = 4.00 Hz, 3H); LC-MS: m/z = 173.9 (M+H)$^+$.

Step 2: Synthesis of intermediate **A5**

Compound **A5-2** (3.0 g, 17.3 mmol, 1.00 *eq),* compound **A5-3** (3.7 g, 22.5 mmol, *1.3 eq),* potassium carbonate

(7.2 g, 51.8 mmol, 3.00 *eq)* and 1,1'-bis(diphenylphosphino)ferrocene palladium dichloride (1.3 g, 1.7 mmol, 0.10 *eq)* were added to dioxane (30.0 mL) and water (7.5 mL) successively. The resulting mixture was purged three times with nitrogen, and reacted at 100°C for 16 hours under nitrogen atmosphere, followed by the addition of ethyl acetate (500.0 mL), and stirred for 10 minutes. The resulting mixture was filtered to obtain a filtrate, which was concentrated under vacuum to obtain a crude product. The crude product was purified by reverse phase HPLC (0.1% $NH_3$•$H_2O$) to obtain compound **A5** (4.3 g, 16.3 mmol, 94.4% yield, 96.9% purity) as a brown oil. $^1$H NMR (400 MHz, $CDCl_3$) δ 7.82 (s, 1H), 7.56 (d, *J* = 7.60 Hz, 1H), 7.33-7.41 (m, 2H), 7.23-7.25 (m, 1H), 5.29 (s, 1H), 3.92 (s, 3H), 3.53-3.60 (m, 1H), 3.07 (d, *J*= 5.20 Hz, 3H), 1.25-1.29 (m, 6H); LC-MS: m/z = 258.0 (M+H)$^+$.

**Synthesis of general intermediate A6 (2-(2-isopropylphenyl)-7,8-dihydro-6H-pyrimido[5,4-b][1,4]oxazine)**

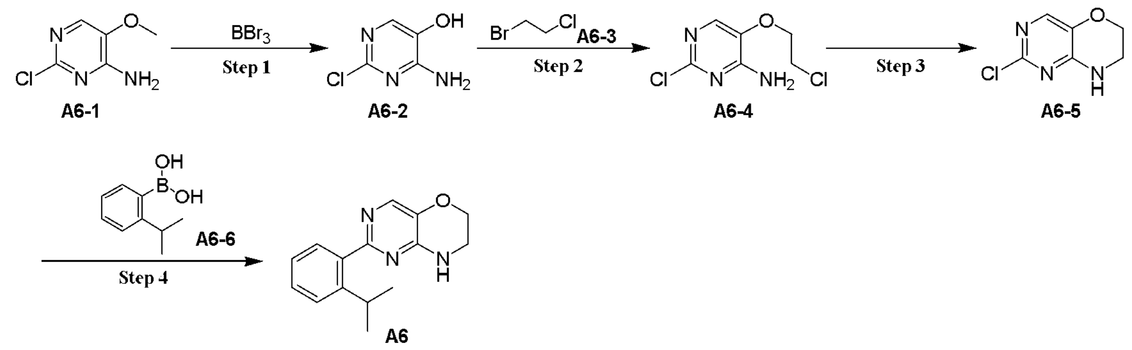


Step 1: Synthesis of compound **A6-2** (4-amino-2-chloropyrimidin-5-ol)

Compound **A6-1** (15.6 g, 97.8 mmol, 1.00 eq) was dissolved in dichloromethane (1.5 L), and boron tribromide (367.0 g, 1.5 mol, 15.0 eq) was slowly added at 0°C. After the addition was completed, the reaction was heated up to 20°C and stirred for 96 hours. Then, methanol (1.0 L) was slowly added at 0°C to quench the reaction. The reaction solution was concentrated under vacuum to obtain a crude product. Dichloromethane (200.0 mL) was added, and the resulting mixture was stirred for 5 minutes, and filtered to obtain a filter cake. The filter cake was dissolved in water (200.0 mL), and saturated aqueous sodium bicarbonate solution was added to adjust the pH to 7. The resulting mixture was filtered to obtain a filter cake, which was washed with water (50.0 mL) twice, and concentrated under vacuum to obtain compound **A6-2** (12.9 g). $^1$H NMR (400 MHz, DMSO) δ 10.0 (d, *J* = 10.0 Hz, 1H), 7.47 (d, *J* = 15.6 Hz, 1H), 7.07 (s, 2H); LC-MS: m/z = 145.9 (M+H)$^+$.

Step 2: Synthesis of compound **A6-4** (2-chloro-5-(2-chloroethoxy)pyrimidin-4-amine)

Compound **A6-2** (19.0 g, 130 mmol, 1.00 eq), compound **A6-3** (28.1 g, 196 mmol, 16.2 mL, 1.50 eq), and potassium carbonate (54.1 g, 392 mmol, 3.00 eq) were added to N,N-dimethylformamide (200.0 mL) successively, and reacted at 20°C for 16 hours to obtain crude compound **A6-4** (27.2 g), and the reaction solution was directly used in the next step. LC-MS: m/z = 207.8 (M+H)$^+$.

Step 3: Synthesis of compound **A6-5** (2-chloro-7,8-dihydro-6H-pyrimido[5,4-b][1,4]oxazine)

The reaction solution of compound **A6-4** (27.2 g) was heated up to 100°C, stirred for 16 hours, and filtered to obtain a filtrate. Water (800.0 mL) was added, the resulting mixture was stirred for 5 minutes, and extracted with ethyl acetate (300.0 mL) three times. The organic layers were combined, washed three times with saturated brine (100.0 mL), dried over anhydrous sodium sulfate, and filtered to obtain a filtrate, which was concentrated under vacuum to obtain a crude product. Methyl *tert*-butyl ether (1000.0 mL) was added, the resulting mixture was stirred for 30 minutes, and filtered to obtain a filter cake, which was concentrated under vacuum to obtain compound **A6-5** (3.4 g). $^1$H NMR (400 MHz, $CDCl_3$) δ 8.33 (d, *J* = 9.60 Hz, 1H), 7.63 (d, *J*= 18.8 Hz, 1H), 4.10-4.34 (m, 2H), 3.43-3.46 (m, 2H); LC-MS: m/z = 171.9 (M+H)$^+$.

Step 4: Synthesis of intermediate **A6**

Compound **A6-5** (1.5 g, 8.74 mmol, 1.00 eq), compound **A6-6** (1.4 g, 8.74 mmol, 1.00 eq), potassium carbonate (3.6 g, 26.2 mmol, 3.00 eq) and 1,1'-bis(diphenylphosphino)ferrocene palladium dichloride (640.0 mg, 874 μmol, 0.10 eq)

were added to N,N-dimethylformamide (15.0 mL) and water (3.8 mL) successively. The resulting mixture was purged three times with nitrogen, and stirred at 100°C for 16 hours under nitrogen atmosphere. The reaction solution was concentrated under vacuum to obtain a crude product, which was purified by preparative HPLC (column: Kromasil Eternity XT 250×80mm×10μm; mobile phase: [water (ammonium hydroxide v/v)-ACN]; B%: 28%-58%, 21 min) to obtain compound **A6** (1.37 g, 5.27 mmol, 60.3% yield, 98.3% purity) as a brown solid. $^1$H NMR (400 MHz, DMSO) δ 7.86 (s, 1H), 7.73-7.83 (m, 1H), 7.54-7.73 (m, 1H), 7.31-7.36 (m, 2H), 7.16-7.20 (m, 1H), 4.16-4.18 (m, 2H), 3.43-3.48 (m, 3H), 1.12-1.23 (m, 6H); LC-MS: m/z = 456.0 (M+H)$^+$.

**Synthesis of general intermediate A7 (2-(2-isopropylphenyl)-6H-pyrimido[5,4-b][1,4]oxazin-7(8H)-one)**

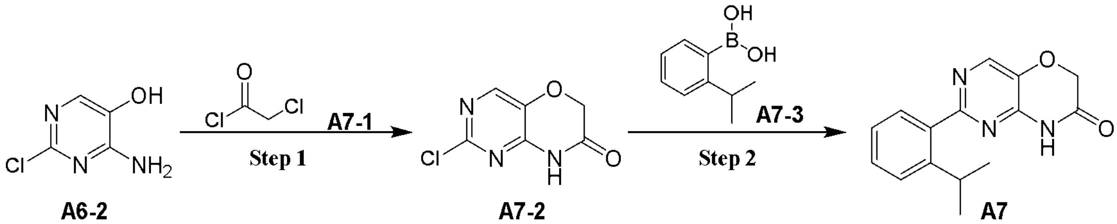


A6-2 A7-2 A7

Step 1: Synthesis of compound **A7-2** (2-chloro-6H-pyrimido[5,4-b][1,4]oxazin-7(8H)-one)

Compound **A6-2** (4.00 g, 27.4 mmol, 1.00 *eq),* compound **A7-1** (4.66 g, 41.2 mmol, 3.28 mL, 1.50 *eq),* and potassium carbonate (11.4 g, 82.4 mmol, 3.00 *eq)* were added to N,N-dimethylformamide (50.0 mL) successively, and reacted at 30°C for 3 hours. Water (150.0 mL) was added, and the resulting mixture was extracted with ethyl acetate (50.0 mL) three times. The organic layers were combined, washed with saturated brine (70.0 mL) three times, dried over anhydrous sodium sulfate, and concentrated under vacuum to obtain a crude product, which was purified by column chromatography (eluent: petroleum ether/ethyl acetate = 20/1 - 5/1) to obtain compound **A7-2** (1.1 g). $^1$H NMR (400 MHz, $CDCl_3$) δ 12.00 (s, 1H), 8.18 - 8.23 (m, 1H), 4.77 - 4.78 (m, 2H); LC-MS: m/z = 185.9 (M+H)$^+$.

Step 2: Synthesis of intermediate **A7**

Compound **A7-2** (500.0 mg, 2.69 mmol, 1.00 *eq),* compound **A7-3** (574.1 mg, 3.50 mmol, 1.30 *eq),* aqueous potassium phosphate solution (1.5 M, 5.39 mL, 3.00 *eq)* and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (231.1 mg, 269 μmol, 0.100 *eq)* were added to tetrahydrofuran (25.0 mL) successively. The resulting mixture was purged three times with nitrogen, and stirred at 60°C for 12 hours under nitrogen atmosphere. The reaction solution was concentrated under vacuum to obtain a crude product, which was purified by preparative HPLC (column: 3_Phenomenex Luna C18 75×30mm×3μm; mobile phase: [water(HCl)-ACN]; B%: 28%-48%, 8 min) to obtain **A7** (90.0 mg, 334 μmol, 12.4% yield) as a white solid. LC-MS: m/z = 270.1 (M+H)$^+$.

**Synthesis of general intermediate A8 (2-(4-cyclopropyl-6-methoxypyrimidin-5-yl)-7,8-dihydro-6H-pyrimido [5,4-b][1,4]oxazine)**

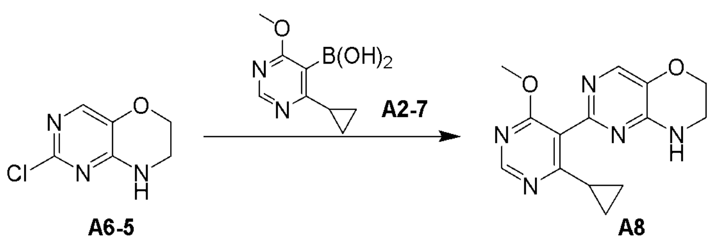


A6-5 A8

Compound **A6-5** (1.50 g, 8.74 mmol, 1.00 eq), compound **A2-7** (1.70 g, 8.74 mmol, 1.00 eq), potassium carbonate (3.62 g, 26.2 mmol, 3.00 eq), and 1,1'-bis(diphenylphosphino)ferrocene palladium dichloride (640 mg, 874 μmol, 0.10 eq) were added to a mixed solvent of N,N-dimethylformamide (15.0 mL) and water (3.75 mL) successively. The

resulting mixture was purged three times with nitrogen, and stirred at 100°C for 16 hours under nitrogen atmosphere. The reaction solution was concentrated under vacuum to obtain a crude product, which was purified by preparative HPLC (column: Waters Xbridge BEH C18 250×50mm×10μm; mobile phase: [water (ammonium hydroxide v/v)-ACN]; B%: 10%-35%, 20 min) to obtain compound **A8** (414 mg, 1.45 mmol, 16.6% yield, 100% purity) as a white solid. $^1$H NMR (400 MHz, DMSO) δ 8.59 (s, 1H), 7.94 (s, 1H), 7.87 (s, 1H), 4.17-4.19 (m, 2H), 3.82 (s, 3H), 3.47-3.49 (m, 2H), 1.67-1.71 (m, 1H), 0.99-1.01 (m, 2H), 0.88-0.90 (m, 2H); LC-MS: m/z = 286.0 (M+H)$^+$.

**Synthesis of general intermediate A9 (4'-cyclopropyl-5,6'-dimethoxy-[2,5'-bipyrimidin]-4-amine)**

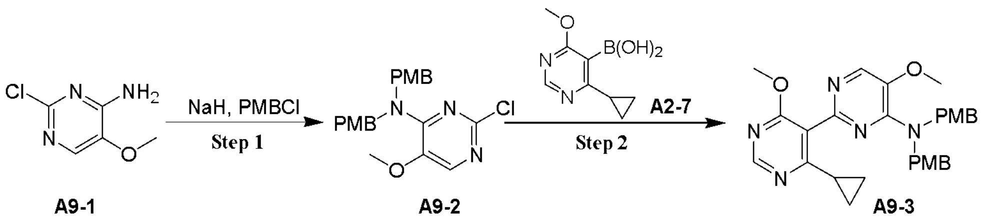


A9-1 A9-2 A9-3

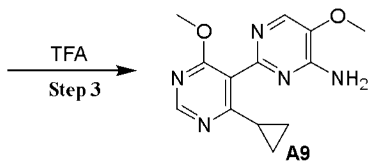


A9

Step 1: Synthesis of compound **A9-2** (2-chloro-5-methoxy-N,N-bis(4-methoxybenzyl)pyrimidin-4-amine)

Compound **A9-1** (6.0 g, 37.6 mmol, 1.00 eq) was dissolved in tetrahydrofuran (60.0 mL), and sodium hydride (3.31 g, 82.7 mmol, 60% purity, 2.20 eq) was added in portions at 0°C. After the addition was completed, the resulting mixture was stirred at 5°C for 30 minutes. 4-Methoxybenzyl chloride (12.9 g, 82.7 mmol, 2.20 eq) was added and stirred at 5°C for 12 hours. Then, saturated aqueous ammonium chloride solution (40.0 mL) was added at 0°C to quench the reaction. Water (150.0 mL) was added, and the resulting mixture was extracted with ethyl acetate (70.0 mL) three times. The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 5/1 - 1/1) to obtain compound **A9-2** (10.3 g). LC-MS: m/z = 400.2 (M+H)$^+$.

Step 2: Synthesis of compound **A9-3** (4'-cyclopropyl-5,6'-dimethoxy-N,N-bis(4-methoxybenzyl)-[2,5'-bipyrimidin]-4-amine)

Compound **A9-2** (2.0 g, 5.00 mmol, 1.00 *eq)*, compound **A2-7** (1.46 g, 7.50 mmol, *1.50 eq)*, aqueous potassium phosphate solution (1.5 M, 10.0 mL, 3.00 *eq)*, and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (430 mg, 500 μmol, 0.100 *eq)* were added to tetrahydrofuran (40.0 mL) successively. The resulting mixture was purged three times with nitrogen, and stirred at 80°C for 12 hours under nitrogen atmosphere. The reaction solution was concentrated under vacuum to obtain a crude product, which was purified by preparative HPLC (column: Phenomenex luna C18 (250×70mm,10 μm); mobile phase: [water (FA)-ACN]; B%: 35%-65%,21 min) to obtain **A9-3** (0.9 g). LC-MS: m/z = 514.3 (M+H)$^+$.

Step 3: Synthesis of intermediate **A9**

Compound **A9-3** (900 mg, 1.75 mmol, 1.00 eq) was added to trifluoroacetic acid (10.0 mL), heated up to 90°C and stirred for 12 hours. The reaction solution was concentrated under vacuum, and saturated aqueous sodium bicarbonate solution (10.0 mL) was added to adjust the pH to 8. The resulting mixture was extracted with ethyl acetate (20.0 mL) three times. The organic layers were combined, dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain **A9** (300.0 mg, 62.5% yield, 100% purity) as a white solid. LC-MS: m/z = 274.1 (M+H)$^+$.

**Synthesis of general intermediate A10 (2-(4-cyclopropyl-6-methoxypyrimidin-5-yl)-6H-pyrimido[5,4-b][1,4]oxazin-7(8H)-one)**

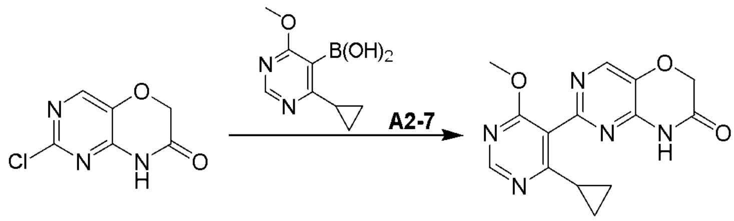


A7-2 A10

Compound **A7-2** (600 mg, 3.23 mmol, 1.00 *eq),* **A2-7** (940 mg, 4.85 mmol, 1.50 *eq),* aqueous potassium phosphate solution (1.5 M, 6.47 mL, 3.00 *eq),* and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (417 mg, 485 μmol, 0.150 *eq)* were added to tetrahydrofuran (10.0 mL) successively. The resulting mixture was purged three times with nitrogen, and stirred at 80°C for 12 hours under nitrogen atmosphere. The reaction solution was concentrated under vacuum to obtain a crude product, which was purified by preparative HPLC (column: Phenomenex luna C18 150×40mm×15μm; mobile phase: [water (TFA)-ACN]; B%: 10%-40%, 10 min) to obtain intermediate **A10** (60.0 mg, 0.2 mmol, 6.2% yield, 100% purity). LC-MS: m/z =300.1 $(M+H)^+$.

**Synthesis of general intermediate AA3 (1-methyl-7-(4,4,5,5-tetramethyl-1,3,2-dioxaborolan-2-yl)-1H-indole)**

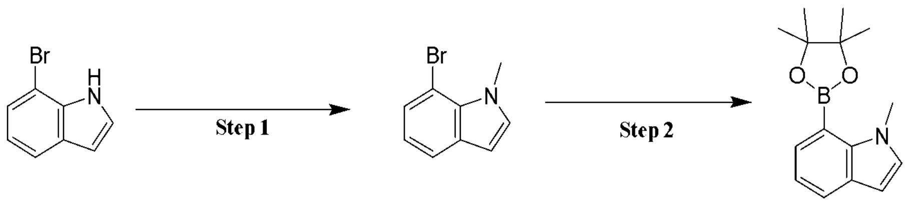


AA3-1 AA3-2 AA3

Step 1: Synthesis of compound **AA3-2** (7-bromo-1-methyl-1H-indole)

7-Bromoindole (compound **AA3-1,** 15.0 g, 76.5 mmol, 1.00 eq) was dissolved in THF (150 mL), and NaH (4.59 g, 115 mmol, purity 60%, 1.50 eq) was added in portions at 0°C. After the addition was completed, the resulting mixture was heated up to room temperature (25°C) and stirred for 1.5 hours. Then, potassium iodide (14.1 g, 99.5 mmol, 6.19 mL, 1.30 eq) was added at 0°C. After the addition was completed, the resulting mixture was heated up to room temperature (25°C) and stirred for 12 hours. 60 mL of ice water was added at 0°C to quench the reaction, and the resulting mixture was extracted with ethyl acetate (70.0 mL) three times. The organic layers were combined, washed three times with saturated brine (100.0 mL), dried over anhydrous sodium sulfate, and filtered to obtain a filtrate, which was concentrated under vacuum to obtain compound **AA3-2** (16.5 g, crude) as a yellow solid. $^1H$ NMR (400 MHz, $CDCl_3$) δ 7.59 (d, *J*= 7.6Hz, 1H), 7.39 - 7.41 (m, 1H), 7.01 - 7.02 (m, 1H), 6.94 - 6.98 (m, 1H), 6.50 - 6.51 (m, 1H), 4.18 (s, 3H); LC-MS: m/z = 212.0 $(M+H)^+$.

Step 2: Synthesis of intermediate **AA3**

Compound **AA3-2** (4.00 g, 19.0 mmol, 1.00 eq), bis(pinacolato)diboron (9.67 g, 38.1 mmol, 2.00 eq), [1,1'-bis(diphenylphosphino)ferrocene]palladium dichloride dichloromethane complex (1.39 g, 1.90 mmol, 0.100 eq) and potassium acetate (3.74 g, 38.1 mmol, 2.00 eq) were dissolved in dioxane (40.0 mL), and stirred at an external temperature of 95°C for 12 hours. Then, water (80.0 mL) was added, and the resulting mixture was extracted with ethyl acetate (50.0 mL) three times. The organic layers were combined, washed twice with saturated brine (50.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain intermediate **AA3** (2.47 g, 9.45 mmol, 49.6% yield, 98.4% purity) as a white solid. $^1H$ NMR (400 MHz, $CDCl_3$) δ 7.75 (d, *J*= 8.0Hz, 1H), 7.70 (d,

$J$= 6.0Hz, 1H), 7.11 - 7.15 (m, 1H), 7.03 - 7.04 (m, 1H), 6.51 - 6.52 (m, 1H), 4.00 (s, 3H), 1.43 - 1.50 (m, 12H); LC-MS: m/z = 258.1 $(M+H)^+$.

**Synthesis of general intermediate AA4 ((1-methylindolin-7-yl)boronic acid)**

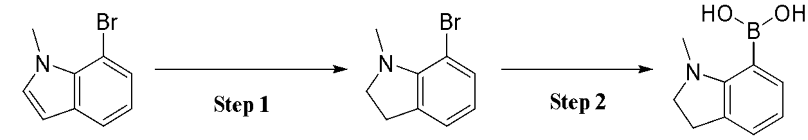


**AA3-2** **AA4-1** **AA4**

Step 1: Synthesis of compound **AA4-1** (7-bromo-1-methylindole)

Compound **AA3-2** (6.70 g, 31.8 mmol, 1.00 eq) was added to acetic acid (50.0 mL), followed by the addition of sodium cyanoborohydride (16.0 g, 255 mmol, 8.00 eq) at 10°C, and the mixture was stirred at 20°C for 12 hours. 1 M aqueous sodium hydroxide solution was added to adjust the pH to about 8, and the resulting mixture was extracted with dichloromethane (60 mL) three times. The organic layers were combined, washed twice with saturated brine (50 mL), dried over anhydrous sodium sulfate, and filtered to obtain a filtrate, which was concentrated under vacuum to obtain compound **AA4-1** (8.00 g, crude) as a brown oil. LC-MS: m/z = 212.1 $(M+H)^+$.

Step 2: Synthesis of intermediate **AA4**

Compound **AA4-1** (500 mg, 2.36 mmol, 1.00 eq) and triisopropyl borate (576 mg, 3.06 mmol, 704 μL, 1.30 eq) were added to tetrahydrofuran (10.0 mL), and n-butyl lithium (2.5 M, 1.23 mL, 1.30 eq) was added dropwise at -78°C. After the addition was completed, the resulting mixture was heated up to 25°C and stirred for 4 hours. Then, saturated aqueous ammonium chloride solution (20 mL) was added dropwise at 0°C to quench the reaction. Water (30.0 mL) was added, and the resulting mixture was extracted with ethyl acetate (15 mL) three times. The organic layers were combined, washed twice with saturated brine (20 mL), dried over anhydrous sodium sulfate, and filtered to obtain a filtrate. The filtrate was concentrated under vacuum to obtain a crude product, which was purified by reverse phase HPLC (0.1% FA) to obtain intermediate **AA4** (120 mg, 588.45 μmol, 24.96% yield, 86.8% purity) as a white solid. $^1H$ NMR (400MHz, DMSO-$d_6$) δ 8.06 (s, 1H), 7.02 (d, $J$ = 6.8Hz, 2H), 6.52 - 6.60 (m, 2H), 3.24 (t, $J$ = 8.0Hz, 2H), 2.84 (t, $J$= 8.0Hz, 2H), 2.76(s, 3H); LC-MS: m/z = 178.1 $(M+H)^+$.

**Synthesis of general intermediate AA5 ((1-isopropyl-4-methyl-1H-imidazol-5-yl)boronic acid)**

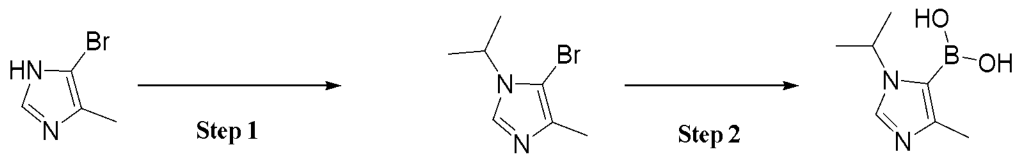


**AA5-1** **AA5-2** **AA5**

Step 1: Synthesis of compound **AA5-2** (5-bromo-1-isopropyl-4-methyl-1H-imidazole)

4-Methyl-5-bromoimidazole **(AA5-1,** 40.0 g, 248 mmol, 1.00 eq) was dissolved in tetrahydrofuran (800 mL), and sodium hydride (7.15 g, 178 mmol, 60% purity, 0.720 eq) was added in portions at -15°C. After the addition was completed, the resulting mixture was stirred at -15°C for 30 minutes. 2-Iodopropane (42.2 g, 248 mmol, 24.8 mL, 1.00 eq) was added, and the resulting mixture was stirred at 0°C for 3 hours. Saturated aqueous ammonium chloride solution (100 mL) was added to quench the reaction. Water (800.0 mL) was added, and the resulting mixture was extracted with ethyl acetate (500 mL) three times. The organic layers were combined, washed twice with saturated brine (500 mL), dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain compound **AAS-2** (7.00 g, 34.4 mmol,

46.6% yield) as a brown oil. $^1$H NMR (400MHz, $CDCl_3$) δ 7.56 (s, 1H), 4.32 - 4.39 (m, 1H), 2.20 (s, 3H), 1.47 (d, *J*= 4.4 Hz, 6H); LC-MS: m/z = 203.0 $(M+H)^+$.

Step 2: Synthesis of intermediate **AA5**

Compound **AAS-2** (7.00 g, 34.5 mmol, 1.00 eq) and triisopropyl borate (32.4 g, 172 mmol, 39.6 mL, 5.00 eq) were added to tetrahydrofuran (150.0 mL), and *n*-butyl lithium (2.50 M, 27.6 mL, 2.00 eq) was added dropwise at -78°C. After the addition was completed, the resulting mixture was heated up to 25°C and stirred for 4 hours. Then, saturated aqueous ammonium chloride solution (200 mL) was added dropwise at 0°C to quench the reaction. Water (300.0 mL) was added, and the resulting mixture was extracted with ethyl acetate (300 mL) three times. The organic layers were combined, washed twice with saturated brine (300 mL), dried over anhydrous sodium sulfate, and filtered to obtain a filtrate, which was concentrated under vacuum to obtain compound **AA5** (6.5 g, crude) as a yellow solid. $^1$H NMR (400MHz, DMSO-$d_6$) δ 8.22 - 8.26 (m, 2H), 8.20 (s, 1H), 5.12 (s, 1H),2.30 (s, 3H), 1.37 - 1.39 (m, 6H); LC-MS: m/z = 169.1 $(M+H)^+$.

**Synthesis of general intermediate AA6 (2-cyclopropyl-3-(4,4,5,5-tetramethyl-1,3,2-dioxaborolan-2-yl)pyridine)**

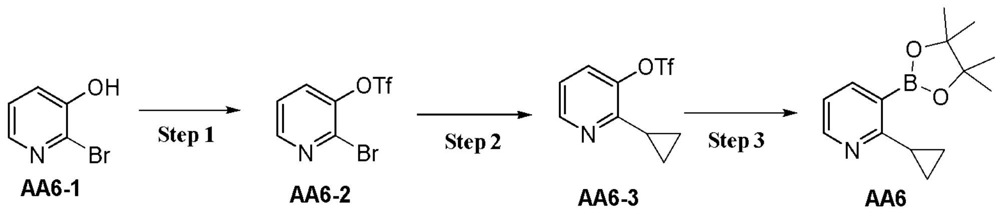


AA6-1 AA6-2 AA6-3 AA6

Step 1: Synthesis of compound **AA6-2** (2-bromopyridin-3-yl trifluoromethanesulfonate)

5-Bromo-3-hydroxypyridine (compound **AA6-1,** 1.0 g, 7.72 mmol), N-phenylbis(trifluoromethanesulfonyl)imide (2.76 g, 7.72 mmol, purchased from Bide Pharmatech), and triethylamine (1.1 mL, 8.1 mmol) were added to dichloromethane (20 mL) successively. The resulting mixture was stirred at 0°C for 1 hour under nitrogen atmosphere, and then heated up to 25°C and stirred for 1.5 hours. The reaction solution was washed once with 1 M sodium hydroxide (100 mL), washed twice with saturated brine (100 mL), dried over anhydrous sodium sulfate, and filtered to obtain a filtrate, which was concentrated under vacuum to obtain compound **AA6-2** (2.8 g, crude) as a light yellow oil. LC-MS: m/z = 305.9 $(M+H)^+$.

Step 2: Synthesis of compound **AA6-3** (2-cyclopropylpyridin-3-yl trifluoromethanesulfonate)

Compound **AA6-2** (2.5 g), tetrakis(triphenylphosphine)palladium (199 mg), and cyclopropylzinc chloride (0.4 M THF solution, 23 mL) were added to tetrahydrofuran (15 mL) successively. The resulting mixture was stirred at 70°C for 3 hours under nitrogen atmosphere, and cooled to room temperature. Saturated aqueous sodium bicarbonate solution (60 mL) was added, and the resulting mixture was extracted with ethyl acetate (50 mL) three times. The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compound **AA6-3** (1.5 g) as a colorless transparent oil. LC-MS: m/z = 268.1 $(M+H)^+$.

Step 3: Synthesis of intermediate **AA6**

Compound **AA6-3** (0.5 g), bis(pinacolato)diboron (0.57 g), potassium carbonate (0.525 g) and [1,1'-bis(diphenylphosphino)ferrocene]palladium dichloride dichloromethane complex (0.156 g) were added to dioxane (80 mL). The resulting mixture was stirred at 100°C for 20 hours, and cooled to room temperature. Ethyl acetate (200 mL) was added, the resulting mixture was washed with saturated brine (100 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **AA6** (0.2 g) as a white solid.

**Synthesis of general intermediate AA7 (2-isopropyl-3-(4,4,5,5-tetramethyl-1,3,2-dioxaborolan-2-yl)pyridine)**

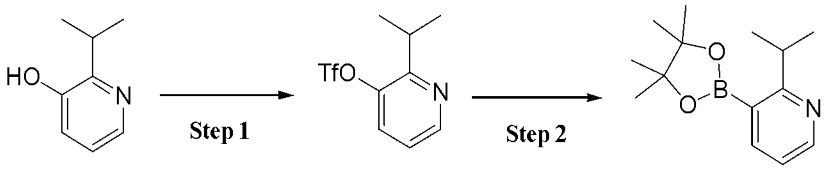


AA7-1 AA7-2 AA7

Step 1: Synthesis of compound **AA7-2** (2-isopropylpyridin-3-yl trifluoromethanesulfonate)

2-Isopropylpyridin-3-ol (compound **AA7-1,** 3.00 g, 21.8 mmol, 1.00 eq) was added to pyridine (30.0 mL), followed by the addition of trifluoromethanesulfonic anhydride (6.17 g, 21.8 mmol, 3.61 mL, 1.00 eq) at 0°C, and stirred at 15°C for 2 hours. Water (30 mL) was added, and the resulting mixture was extracted with ethyl acetate (60 mL) three times. The organic layers were combined, washed three times with saturated brine (100 mL), dried over anhydrous sodium sulfate, and filtered to obtain a filtrate, which was concentrated under vacuum to obtain compound **AA7-2** (3.62 g, crude) as a yellow oil. $^1H$ NMR (400MHz, $CDCl_3$) δ 8.59 (t, *J* = 3.6 Hz, 1H), 7.55 - 7.58 (m, 1H), 7.21 - 7.24 (m, 1H), 3.39 - 3.46 (m, 1H), 1.30 (d, *J*= 6.8 Hz, 6H); LC-MS: m/z = 270.2 $(M+H)^+$.

Step 2: Synthesis of compound **AA7**

Compound **AA7-2** (3.62 g, 13.4 mmol, 1.00 eq), bis(pinacolato)diboron (6.83 g, 26.8 mmol, 2.00 eq), potassium acetate (2.64 g, 26.8 mmol, 2.00 eq) and [1,1'-bis(diphenylphosphino)ferrocene]palladium dichloride (983 mg, 1.34 mmol, 0.100 eq) were added to dioxane (60.0 mL) successively. The resulting mixture was stirred at 100°C for 20 hours, and cooled to room temperature. Ethyl acetate (300 mL) was added, the resulting mixture was washed with saturated brine (150 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA7** (2.00 g, 8.09 mmol, 60.2% yield) as a light yellow oil. $^1H$ NMR (400MHz, $CDCl_3$) δ 8.60 (dd, $J_1$ = 2.0Hz, $J_2$ = 3.2Hz, 1H), 7.99 (dd, $J_1$ = 2.0Hz, $J_2$ = 5.2Hz, 1H), 7.07 (dd, $J_1$ = 4.8Hz, $J_2$ = 2.8Hz, 1H), 3.71 - 3.78 (m, 1H), 1.35 (s, 12H), 1.24 - 1.28 (m, 6H).

**General intermediate AA8 ((2-(dimethylamino)phenyl)boronic acid)**

Intermediate **AA8** was purchased from Bide Pharmatech.

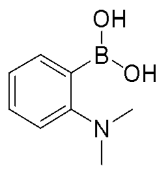

AA8

**Synthesis of general intermediate AA9 (4-chloro-1-isopropyl-5-(4,4,5,5-tetramethyl-1,3,2-dioxaborolan-2-yl)-1H-pyrazole)**

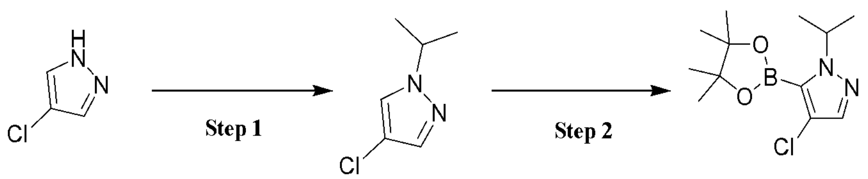


AA9-1 AA9-2 AA9

Step 1: Synthesis of compound **AA9-2** (4-chloro-1-isopropyl-1H-pyrazole)

Compound **AA9-1** (50.00 g, 487.70 mmol, 1.00 *eq),* 2-iodopropane (124.36 g, 731.55 mmol, 73.15 mL, *1.50 eq)* and cesium carbonate (317.80 g, 975.40 mmol, 2.00 *eq)* were added to acetonitrile (500.00 mL), and stirred at 80°C for 2 hours under $N_2$ atmosphere. Then, the reaction solution was filtered to obtain a filtrate. The filtrate was concentrated under vacuum to obtain a crude product, which was purified by column chromatography (eluent: petroleum ether/ethyl acetate = 1/0 - 5/1) to obtain compound **AA9-2** (45.00 g, 306.53 mmol, 62.85% yield, 98.50% purity) as a light yellow oil. $^1$H NMR (400 MHz, $CDCl_3$) δ 7.41-7.40 (m, 2H), 4.46 - 4.41(m, 1H), 1.48 (d, *J* = 6.8 Hz, 6H); LC-MS: m/z = 144.0 $(M+H)^+$.

Step 2: Synthesis of intermediate **AA9**

Compound **AA9-2** (10 g, 69.16 mmol, 1 eq) was dissolved in anhydrous tetrahydrofuran (100 mL), and *n*-butyl lithium (2.5 M, 33.19 mL, 1.2 eq) was added dropwise at 0°C. After the addition was completed, the resulting mixture was heated up to 25°C and stirred for 1 hour. Isopropyl pinacolyl borate (15.44 g, 82.99 mmol, 16.93 mL, 1.2 eq) was added at -78°C, and the resulting mixture was heated up to 25°C and stirred for 2 hours. Then, saturated aqueous ammonium chloride solution (100 mL) was added at 0°C to quench the reaction. Water (200 mL) was added, and the resulting mixture was extracted with ethyl acetate (50.0 mL) three times. The organic layers were combined, washed twice with saturated brine (60.0 mL), dried over anhydrous sodium sulfate, and filtered to obtain a filtrate. The filtrate was concentrated under vacuum to obtain a crude product, which was purified by prep-HPLC (column: Welch Ultimate XB-CN 250×70×10μm; mobile phase: [hexane-ethanol]; B%: 1%-1%, 15 min) to obtain compound **AA9** (6.5 g, 18.33 mmol, 26.51% yield, 76.3% purity) as a light yellow oil. $^1$H NMR (400MHz, $CDCl_3$) δ 7.45 (s, 1H), 5.07 - 5.00(m, 1H), 1.46 (d, *J*= 6.8 Hz, 6H), 1.36 (s, 12H); LC-MS: m/z = 271.2 $(M+H)^+$.

**General intermediate AA10 ((2-cyclopropylphenyl)boronic acid)**

Intermediate **AA10** was purchased from Bide Pharmatech.

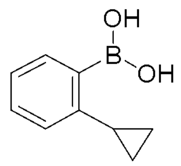

**AA10**

**Synthesis of general intermediate AA11 ((1-isopropyl-4-methoxy-1H-pyrazol-5-yl)boronic acid)**

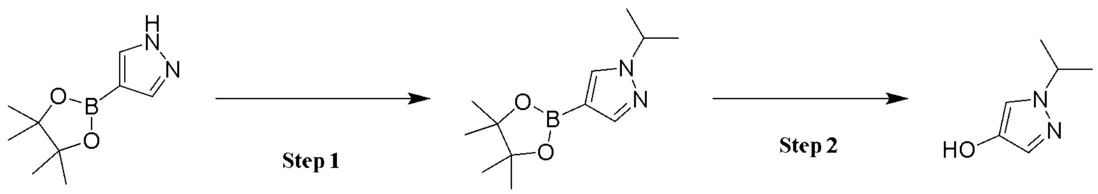


**AA11-1** **AA11-2** **AA11-3**

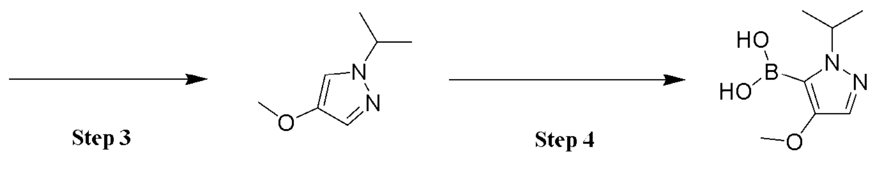


**AA11-4** **AA11**

Step 1: Synthesis of compound **AA11-2** (1-isopropyl-4-(4,4,5,5-tetramethyl-1,3,2-dioxaborolan-2-yl)-1H-pyrazole)

4-Pyrazolyl pinacolyl borate (compound **AA11-1,** 130 g, 669 mmol, 1.00 eq) was dissolved in N,N-dimethylformamide (114 g, 669 mmol, 67.0 mL, 1.00 eq). 2-Iodopropane (114 g, 669 mmol, 67.0 mL, 1.00 eq) and cesium carbonate (327 g, 1.00 mol, 1.50 eq) were added, and the mixture was stirred at an external temperature of 90°C for 12 hours. Then, the reaction solution was filtered to obtain a filtrate. The filtrate was concentrated under vacuum to obtain a crude product, which was purified by column chromatography (eluent: petroleum ether/ethyl acetate = 1/0 - 0/1) to obtain compound **AA11-2** (120 g, 508 mmol, 75.9% yield) as a colorless transparent oil. $^1$H NMR (400MHz, $CDCl_3$) δ 7.74 (m, 2H), 4.46 - 4.53 (m, 1H), 1.48 (d, *J*= 6.8Hz, 6H), 1.29 (s, 12H); LC-MS: m/z = 237.1 $(M+H)^+$.

Step 2: Synthesis of compound **AA11-3** (1-isopropyl-1H-pyrazol-4-ol)

Compound **AA11-2** (60.0 g, 254 mmol, 1.00 eq) was dissolved in tetrahydrofuran (600 mL), followed by the addition of aqueous sodium hydroxide solution (2.50 M, 203 mL, 2.00 eq) and hydrogen peroxide (72.0 g, 635 mmol, 61.0 mL, 30.0% purity, 2.50 eq). The mixture was stirred at 25°C for 3 hours. 1 M aqueous hydrochloric acid solution was added to adjust the pH to about 2. Then, anhydrous sodium sulfite (50.0 g) was added at 0°C to quench the reaction. The resulting mixture was concentrated under vacuum to remove tetrahydrofuran, and extracted three times with dichloromethane/-methanol = 10/1 (1500 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA11-3** (17.5 g, 138 mmol, 54.6% yield) as a light yellow oil. $^1$H NMR (400MHz, $CDCl_3$) δ7.12 (s, 1H), 7.09 (d, *J* = 0.8 Hz, 1H), 4.32 - 4.39 (m, 1H), 1.43 (s, 3H), 1.41 (s, 3H).

Step 3: Synthesis of compound **AA11-4** (1-isopropyl-4-methoxy-1H-pyrazole)

Compound **AA11-3** (17.5 g, 139 mmol, 1.00 eq) was dissolved in N,N-dimethylformamide (350 mL), followed by the addition of cesium carbonate (67.8 g, 208 mmol, 1.50 eq) and iodomethane (29.5 g, 208 mmol, 13.0 mL, 1.50 eq). The mixture was stirred at room temperature (25°C) for 3 hours. Then, water (1000 mL) was added, and the resulting mixture was extracted three times with ethyl acetate (600 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **AA11-4** (12.3 g, 87.7 mmol, 63.3% yield) as a light yellow solid. $^1$H NMR (400MHz, $CDCl_3$) δ 7.17 (s, 1H), 7.05 (d, *J* = 0.8 Hz, 1H), 4.30 - 4.36 (m, 1H), 3.70 (s, 3H), 1.42 (s, 3H), 1.40 (s, 3H).

Step 4: Synthesis of compound **AA11**

Compound **AA11-4** (1.00 g, 7.13 mmol, 1.00 eq) was dissolved in anhydrous tetrahydrofuran (15.0 mL). *n*-Butyl lithium (2.50 M, 4.28 mL, 1.50 eq) was added dropwise at -70°C under nitrogen atmosphere. After the addition was completed, the resulting mixture was stirred at -70°C for 1 hour. Isopropyl pinacolyl borate (2.01 g, 10.8 mmol, 2.2 mL, 1.50 eq) was then added at -70°C. The resulting mixture was stirred at - 70°C for 1 hour, heated up to 25°C and stirrred for 12 hours. Then, saturated aqueous ammonium chloride solution (20.0 mL) was added at 0°C, and the resulting mixture was extracted with ethyl acetate (100 mL) three times. The organic layers were combined, dried over anhydrous sodium sulfate, and filtered to obtain a filtrate. The filtrate was concentrated under vacuum to obtain a crude product, which was triturated with petroleum ether (20.00 mL) for 15 minutes to obtain intermediate **AA11** (200 mg, 1.09 mmol, 15.2% yield) as an off-white solid. LC-MS: m/z = 185.2 $(M+H)^+$.

**Synthesis of** general **intermediate AA12 ((1-cyclopropyl-4-methoxy-1H-pyrazol-5-yl)boronic acid)**

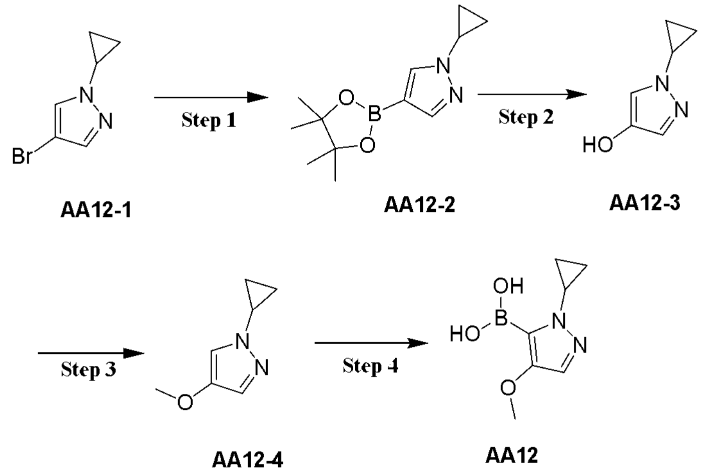


**AA12-1** **AA12-2** **AA12-3**

**AA12-4** **AA12**

Step 1: Synthesis of compound **AA12-2** (1-cyclopropyl-4-(4,4,5,5-tetramethyl-1,3,2-dioxaborolan-2-yl)-1H-pyrazole)

Compound **AA12-1** (19.0 g, 102 mmol, 1.00 *eq)* was dissolved in dioxane (250 mL), followed by the addition of bis(pinacolato)diboron (36.1 g, 142 mmol, 1.40 *eq),* potassium acetate (39.9 g, 406 mmol, 4.00 *eq)* and [1,1'-bis(diphenylphosphino)ferrocene]palladium dichloride dichloromethane complex (4.15 g, 5.08 mmol, 0.05 *eq)* successively. The mixture was stirred at 85°C for 12 hours under nitrogen atmosphere. Then, water (800 mL) and ethyl acetate (500 mL) were added, and the resulting mixture was filtered to obtain a filtrate, which was extracted with ethyl acetate (500 mL) three times. The organic layers were combined, washed twice with saturated brine (500 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/0) to obtain compound **AA12-2** (4.40 g) as a light yellow oil. $^1$H NMR (400MHz, $CDCl_3$) $\delta$ 7.75 (d, *J* = 3.2Hz, 2H), 3.50 - 3.60 (m, 1H), 1.32 (s, 12H), 1.09 - 1.12 (m, 2H), 1.01 - 1.04 (m, 2H); LC-MS: m/z = 235.1 $(M+H)^+$.

Step 2: Synthesis of compound **AA12-3** (1-cyclopropyl-1H-pyrazol-4-ol)

Compound **AA12-2** (3.30 g, 14.1 mmol, 1.00 *eq)* was dissolved in tetrahydrofuran (43.0 mL), followed by the addition of aqueous sodium hydroxide solution (2.50 M, 11.3 mL, 2.00 *eq)* and hydrogen peroxide (5.84 g, 51.5 mmol, 4.95 mL, 30.0% purity, 3.65 *eq)* were added. The mixture was stirred at 25°C for 3 hours. 1 M aqueous hydrochloric acid solution was added to adjust the pH to about 2. Then, anhydrous sodium sulfite (50.0 g) was added at 0°C to quench the reaction. The resulting mixture was concentrated under vacuum to remove tetrahydrofuran, and extracted three times with dichloromethane/methanol = 10/1 (150 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA12-3** (0.980 g, crude) as a light yellow oil. $^1$H NMR (400MHz, $CDCl_3$) $\delta$ 7.12 (d, *J*= 3.6Hz, 2H), 3.46 - 3.48 (m, 1H), 0.95 - 0.96 (m, 2H), 0.94 - 0.95 (m, 2H).

Step 3: Synthesis of compound **AA12-4** (1-cyclopropyl-4-methoxy-1H-pyrazole)

Compound **AA12-3** (0.980 g, 7.89 mmol, 1.00 *eq)* was dissolved in N,N-dimethylformamide (19.5 mL), followed by the addition of cesium carbonate (3.86 g, 11.8 mmol, 1.50 *eq)* and iodomethane (1.68 g, 11.8 mmol, 737 μL, 1.50 *eq).* The mixture was stirred at room temperature (25°C) for 3 hours. Then, water (10.0 mL) was added, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **AA12-4** (0.460 g, 3.33 mmol, 42.2% yield) as a light yellow solid. $^1$H NMR (400MHz, $CDCl_3$) $\delta$ 7.21 (s, 1H), 7.13 (s, 1H), 3.74 (s, 3H), 3.49 - 3.53 (m, 1H), 1.08 - 1.09 (m, 2H), 0.96 - 0.99 (m, 2H); LC-MS: m/z = 139.2 $(M+H)^+$.

Step 4: Synthesis of intermediate **AA12**

Compound **AA12-4** (0.300 g, 2.17 mmol, 1.00 *eq)* was dissolved in anhydrous tetrahydrofuran (8.50 mL), and n-butyl lithium (2.50 M, 1.74 mL, 2.00 *eq)* was added dropwise at -70°C under nitrogen atmosphere. After the addition was

completed, the resulting mixture was stirred at -70°C for 1 hour. Isopropyl pinacolyl borate (808 mg, 4.34 mmol, 886 μL, 2.00 *eq)* was then added at -70°C, and the resulting mixture was stirred at -70°C for 1 hour, heated up to 25°C and stirred for 12 hours. Then, saturated aqueous ammonium chloride solution (30.0 mL) was added at 0°C, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by prep-HPLC (chromatographic column: Waters Xbridge C18 150×50mm× 10μm; mobile phase: [water ($NH_4HCO_3$)-ACN]; B%: 3%-33%, 10 min) to obtain compound **AA12** (0.400 g, crude) as a light yellow oil. LC-MS: m/z = 183.1 $(M+H)^+$.

**Synthesis of general intermediate AA13 ((5-isopropyl-3-methylisoxazol-4-yl)boronic acid)**

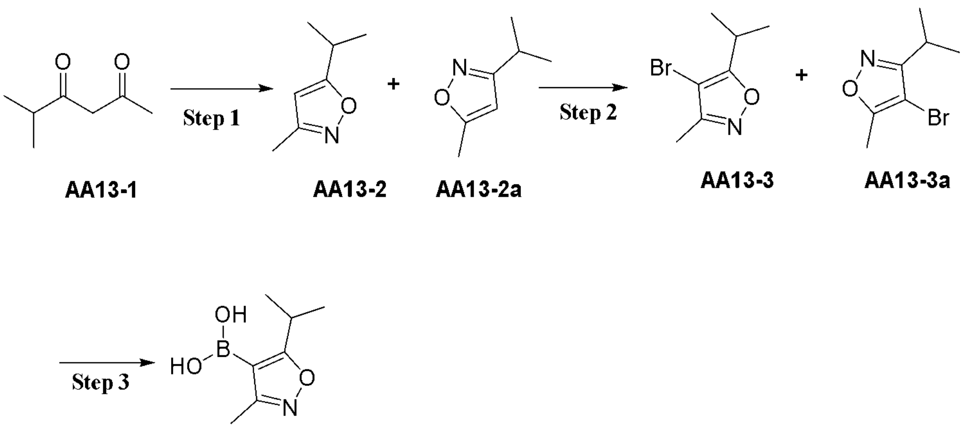


AA13-1 AA13-2 AA13-2a AA13-3 AA13-3a

AA13

Step 1: Synthesis of compound **AA13-2** (5-isopropyl-3-methylisoxazole)

Isobutyrylacetone (compound **AA13-1,** 2.00 g, 15.6 mmol, 1.00 *eq)* and hydroxylamine hydrochloride (1.36 g, 19.6 mmol, 1.25 *eq)* were added to ethanol (10.0 mL) successively, and stirred at 130°C for 10 minutes. Dichloromethane (100 mL) was added, and the resulting mixture was washed twice with saturated brine (50.0 mL). The organic layer was separated, dried over anhydrous sodium sulfate, and filtered to obtain a filtrate, which was concentrated under vacuum to obtain compound **AA13-2** and compound **AA13-2a** (4.80 g in total, crude) as a colorless transparent liquid. Compound **AA13-2:** $^1H$ NMR (400MHz, $CDCl_3$) δ 5.76 (s, 1H), 3.04 - 2.97 (m,1H), 2.35 (s, 3H), 1.28 - 1.23 (m, 6H).

Step 2: Synthesis of compound **AA13-3** (4-bromo-5-isopropyl-3-methylisoxazole)

Compound **AA13-2** and compound **AA13-2a** (3.50 g, 28.0 mmol, 1.00 *eq)* were dissolved in N,N-dimethylformamide (15 mL), followed by the addition of N-bromosuccinimide (7.94 g, 44.6 mmol, 1.59 *eq).* The mixture was stirred at 25°C for 15 hours. Ethyl acetate (250 mL) was added, and the resulting mixture was washed once with saturated sodium thiosulfate (100 mL) and three times with saturated brine (200 mL). The organic layer was separated, dried over anhydrous sodium sulfate, and filtered to obtain a filtrate, which was concentrated under vacuum to obtain compound **AA13-3** and compound **AA13-3a** (5.24 g, crude) as a light yellow oil. Compound **AA13-3:** $^1H$ NMR (400MHz, $CDCl_3$) δ 3.20 - 3.13 (m, 1H), 2.24 (s, 3H), 1.33 - 1.30 (m, 6H); LC-MS: m/z = 203.9 $(M+H)^+$.

Step 3: Synthesis of compound **AA13**

Compound **AA13-3,** compound **AA13-3a** (3.00 g, 14.7 mmol, 1.00 *eq)* and triisopropyl borate (3.59 g, 19.1 mmol, 4.39 mL, 1.30 *eq)* were dissolved in tetrahydrofuran (30.0 mL), and *n*-butyl lithium (2.5 M, 7.64 mL, 1.30 eq) was added dropwise at -70°C. After the addition was completed, the resulting mixture was heated up to 25°C and stirred for 4 hours. Water (10.0 mL) was added at 0°C to quench the reaction, and the resulting mixture was extracted three times with ethyl acetate (30.0 mL). The organic layers were combined, dried over anhydrous sodium sulfate, and filtered to obtain a filtrate. The filtrate was concentrated under vacuum to obtain a crude product, which was purified by prep-HPLC (chromatographic column: Phenomenex luna C18 250×80mm×10 μm; mobile phase: [water (FA)-ACN]; B%: 20%-50%, 20 min) to obtain compound **AA13** (470 mg, 2.73 mmol, 18.5% yield, 98.0% purity) as a white solid. $^1H$ NMR (400MHz, $CDCl_3$) δ 3.83

- 3.76 (m, 1H), 2.49 (s, 3H), 1.38 (s, 3H), 1.36 (s, 3H); LC-MS: m/z = 170.1 $(M+H)^+$.

**Synthesis of general intermediate AA14 (4-chloro-1-cyclopropyl-5-(4,4,5,5-tetramethyl-1,3,2-dioxaborolan-2-yl)-1H-pyrazole)**

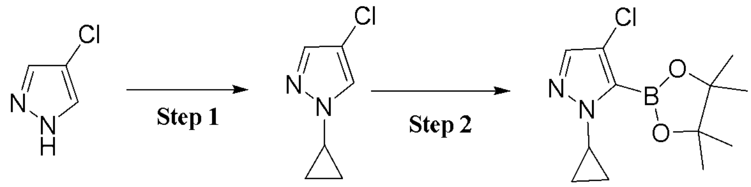


AA14-1 AA14-2 AA14

Step 1: Synthesis of compound **AA14-2** (4-chloro-1-cyclopropyl-1H-pyrazole)

4-Chloropyrazole (compound **AA14-1,** 10.0 g, 97.54 mmol, 1.0 eq), cyclopropyl bromide (21.2 g, 175.57 mmol, 1.8 eq) and cesium carbonate (63 g, 195.08 mmol, 2.0 eq) were added to dioxane (50 mL) successively, and stirred at an external temperature of 140°C for 16 hours. The reaction solution was cooled to room temperature, and concentrated under vacuum to obtain a crude product, which was purified by column chromatography (eluent: petroleum ether/ethyl acetate = 1/0 - 1/2) to obtain compound **AA14-2** (6.4 g) as a light yellow oil. $^1$H NMR (400MHz, $CDCl_3$) δ 7.42 (s, 1H), 7.37 (s, 1H), 3.54 (tt, J = 7.3, 3.8 Hz, 1H), 1.13 - 1.05 (m, 2H), 1.05 - 0.95 (m, 2H). LC-MS: m/z = 143.1 (M+H)+.

Step 2: Synthesis of intermediate **AA14**

Compound **AA14-2** (8.3 g, 58.21 mmol, 1.0 eq) was dissolved in tetrahydrofuran (100 mL), and lithium diisopropylamide (58 mL, 116.42 mmol, 2.0 eq) was added dropwise under nitrogen atmosphere at -70°C. After the addition was completed, the resulting mixture was stirred at -70°C for 1 hour. Isopropyl pinacolyl borate (17.3 g, 93.14 mmol, 1.6 eq) was added, and the resulting mixture was stirred at -70°C for 1 hour, heated up to 25°C and stirred for 2 hours. Then, water (100 mL) was added to quench the reaction, and the resulting mixture was extracted three times with dichloromethane (200 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain intermediate **AA14** (4.2 g) as a colorless and transparent oil. $^1$H NMR (400MHz, $CDCl_3$) δ 7.42 (s, 1H), 7.37 (s, 1H), 3.54 (tt, J = 7.3, 3.8 Hz, 1H), 1.13 - 1.05 (m, 2H), 1.05 - 0.95 (m, 2H).

**General intermediate AA15 ((4-cyclopropylpyrimidin-5-yl)boronic acid)**

Intermediate **AA15** was purchased from Leyan Reagents.

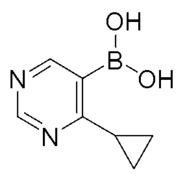

**AA15**

**Synthesis of general intermediate AA16 (1-cyclopropyl-4-methyl-5-(4,4,5,5-tetramethyl-1,3,2-dioxaborolan-2-yl)-1H-pyrazole)**

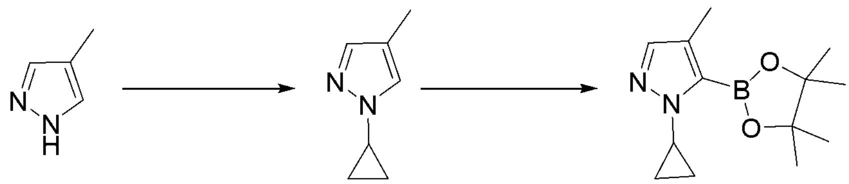

AA16-1 AA16-2 AA16

In accordance with the same steps of intermediate **AA14,** intermediate **AA16** (2.9 g) was obtained using 4-methylpyrazole (compound **AA16-1)** as a starting material. $^1$H NMR (400 MHz, DMSO-$d_6$) δ 7.24 (s, 1H), 4.03 (td, *J* = 7.5, 3.8 Hz, 1H), 2.15 (s, 3H), 1.35 (s, 12H), 1.12 - 1.01 (m, 2H), 0.99 - 0.89 (m, 2H).

**Synthesis of general intermediate AA17 (1-isopropyl-4-methyl-5-(4,4,5,5-tetramethyl-1,3,2-dioxaborolan-2-yl)-1H-pyrazole)**

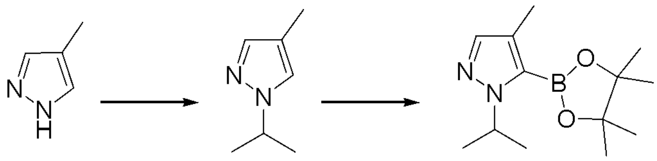

AA16-1 AA17-2 AA17

In accordance with the same steps of intermediate **AA14,** intermediate **AA17** (4.1 g) was obtained using 4-methylpyrazole (compound **AA16-1)** as a starting material. $^1$H NMR (400 MHz, DMSO-$d_6$) δ 7.27 (s, 1H), 4.94-4.98 (m, 1H), 2.12 (s, 3H), 1.34 (d, *J*= 6.6 Hz, 6H), 1.29 (s, 12H).

**Synthesis of general intermediate B1 (3-(4-(chloromethyl)piperidin-1-yl)pyridine)**

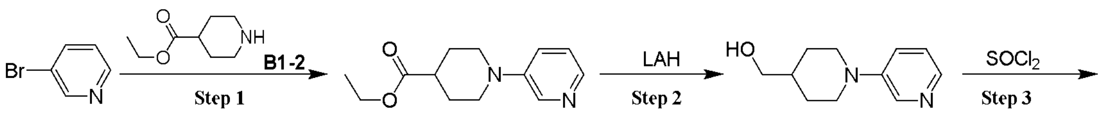


B1-1 B1-3 B1-4

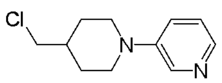

B1

Step 1: Synthesis of compound **B1-3** (ethyl 1-(pyridin-3-yl)piperidine-4-carboxylate)

Compound **B1-1** (6.4 g, 40.7 mmol, 1.28 eq), compound **B1-2** (5.0 g, 31.8 mmol, 1.00 eq), 4,5-bis(diphenylphosphino)-9,9-dimethylxanthene (1.1 g, 1.91 mmol, 0.06 eq), cesium carbonate (13.9 g, 42.90 mmol, 1.35 eq), and tris(dibenzylideneacetone)dipalladium (582.0 mg, 0.6 mmol, 0.02 eq) were added to anhydrous dioxane (50.0 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% FA) to obtain compound **B1-3** (3.2 g). $^1$H NMR (400 MHz, $CDCl_3$) δ 8.29 (d, *J* = 1.6 Hz, 1H), 8.05 (d, *J* = 3.2 Hz, 1H), 7.12 - 7.19 (m, 2H), 4.11 - 4.17 (m, 2H), 3.60 - 3.65 (m, 2H), 2.80 - 2.86 (m, 2H), 2.39 - 2.48 (m, 2H), 2.00 - 2.04 (m, 2H), 1.83 - 1.87 (m, 2H), 1.25 (t, J = 7.2 Hz, 3H); LC-MS: m/z =235.1 (M+H)$^+$.

Step 2: Synthesis of compound **B1-4** ((1-(pyridin-3-yl)piperidin-4-yl)methanol)

Compound **B1-3** (2.0 g, 8.5 mmol, 1.00 eq) was dissolved in tetrahydrofuran (20.0 mL), and lithium aluminum tetrahydride (356.0 mg, 9.4 mmol, 1.10 eq) was added at 0°C. After the addition was completed, the resulting mixture was heated up to 25°C and stirred for 4 hours. Then, water (1.6 mL) and aqueous sodium hydroxide solution (1 M, 0.4 mL) were added at 0°C. After the addition was completed, the resulting mixture was stirred at 0°C for 0.5 hours. Tetrahydrofuran (10.0 mL) and anhydrous sodium sulfate (1.0 g) were added, and the resulting mixture was stirred for 5 minutes, and filtered to obtain a filtrate, which was concentrated under vacuum to obtain crude compound **B1-4** (1.2 g), which was directly used in the next step. $^1H$ NMR (400 MHz, $CDCl_3$) δ 8.29 (d, *J* = 2.8 Hz, 1H), 8.03 (d, *J* = 4.4 Hz, 1H), 7.19 - 7.21 (m, 1H), 7.12 - 7.16 (m, 1H), 3.71 - 3.74 (m, 2H), 3.53 - 3.55 (m, 2H), 2.73 - 2.80 (m, 2H), 2.60 (s, 1H), 1.86 - 1.89 (m, 2H), 1.67 - 1.70 (m, 1H), 1.25 - 1.44 (m, 2H); LC-MS: m/z = 193.2 $(M+H)^+$.

Step 3: Synthesis of intermediate **B1**

Compound **B1-4** (0.5 g, 2.6 mmol, 1.00 eq) was dissolved in dichloromethane (5.0 mL), and thionyl chloride (3.1 g, 26.0 mmol, 10.0 eq) was added at 0°C. After the addition was completed, the resulting mixture was heated up to 20°C and stirred for 15 hours. The reaction solution was concentrated under vacuum to obtain compound **B1** (520 mg, 2.31 mmol, 88.9% yield, 93.7% purity) as a light yellow solid. LC-MS: m/z =211.2 $(M+H)^+$.

**Synthesis of general intermediate B2 (4-(4-(chloromethyl)piperidin-1-yl)pyridine)**

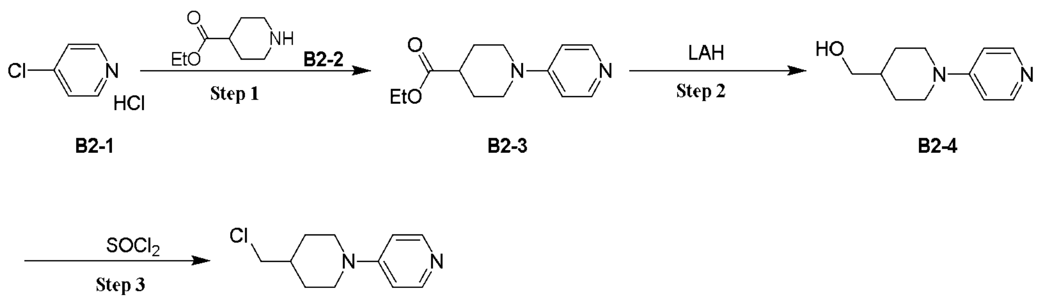


B2-1 B2-3 B2-4 B2

Step 1: Synthesis of compound **B2-3** (ethyl 1-(pyridin-4-yl)piperidine-4-carboxylate)

Compound **B2-1** (5.0 g, 33.3 mmol, 1.00 eq), compound **B2-2** (5.2 g, 33.3 mmol, 1.00 eq), and triethylamine (10.1 g, 100.0 mmol, 3.00 eq) were added to ethanol (50.0 mL), the mixture was heated up to 100°C and stirred for 16 hours. Then, water (50.0 mL) was added at 25°C, and the resulting mixture was extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **B2-3** (2.7 g). $^1H$ NMR (400 MHz, $CDCl_3$) δ 8.25 (d, *J* = 5.2 Hz, 2H), 6.65 (d, *J* = 5.6 Hz, 2H), 4.15 (q, *J* = 7.2 Hz, 2H), 3.82 (d, *J* = 13.2 Hz, 2H), 2.96 (d, *J* = 11.6 Hz, 2H), 2.51 - 2.58 (m, 1H), 1.99 - 2.02 (m, 2H), 1.77 - 1.84 (m, 2H), 1.27 (d, *J* = 7.2 Hz, 3H); LC-MS: m/z =235.2 $(M+H)^+$.

Step 2: Synthesis of compound **B2-4** ((1-(pyridin-4-yl)piperidin-4-yl)methanol)

Compound **B2-3** (1.7 g, 7.3 mmol, 1.00 eq) was dissolved in tetrahydrofuran (20.0 mL), and lithium aluminum hydride (302.0 mg, 8.0 mmol, 1.10 eq) was added at 0°C. After the addition was completed, the resulting mixture was heated up to 25°C and stirred for 4 hours. Aqueous sodium hydroxide solution (0.25 M, 2 mL) was added at 0°C, and the resulting mixture was stirred at 0°C for 0.5 hours. Tetrahydrofuran (10.0 mL) and anhydrous sodium sulfate (1.0 g) were added, and the resulting mixture was stirred for 5 minutes, and filtered to obtain a filtrate, which was concentrated under vacuum to obtain crude compound **B2-4** (1.1 g), which was directly used in the next step. $^1H$ NMR (400 MHz, $CDCl_3$) δ 8.25 (t, *J* = 1.6 Hz, 2H), 6.67 (t, *J* = 1.2 Hz, 2H), 3.92 (d, *J* = 16.0 Hz, 2H), 3.55 (d, *J* = 2.4 Hz, 2H), 2.84 - 2.90 (m, 2H), 1.78 - 1.87 (m, 3H), 1.30 - 1.37 (m, 2H); LC-MS: m/z = 193.0 $(M+H)^+$.

Step 3: Synthesis of compound **B2**

Compound **B2-4** (500.0 mg, 2.60 mmol, 1.00 eq) was dissolved in dichloromethane (5.0 mL), and thionyl chloride (2.5 g, 20.8 mmol, 8.00 eq) was added at 0°C. After the addition was completed, the resulting mixture was heated up to 20°C and stirred for 15 hours. The reaction solution was concentrated under vacuum to obtain compound **B2** (530 mg, 2.52 mmol, 96.7% yield) as a white solid. LC-MS: m/z =193.0 $(M+H)^+$.

**Synthesis of general intermediate B3 (3-(4-(chloromethyl)phenyl)pyridine)**

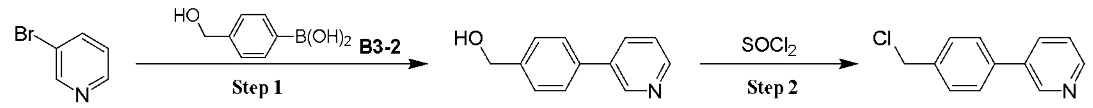


B3-1 B3-3 B3

Step 1: Synthesis of compound **B3-3** ((4-(pyridin-3-yl)phenyl)methanol)

Compound **B3-1** (3.0 g, 18.9 mmol, 1.00 eq), compound **B3-2** (3.8 g, 24.6 mmol, 1.30 eq), sodium carbonate (14.8 g, 140.0 mmol, 7.40 eq), tetrakis(triphenylphosphine)palladium (2.2 g, 1.89 mmol, 0.10 eq) were added to a mixed solvent of toluene (15.0 mL), water (15.0 mL) and ethanol (3.0 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% $NH_3 \cdot H_2O$) to obtain compound **B3-3** (2.3 g). $^1H$ NMR (400 MHz, $CDCl_3$) δ 8.80 (s, 1H), 8.59 (d, *J* = 4.8 Hz, 1H), 7.87 - 7.90 (m, 1H), 7.59 (d, *J* = 8.0 Hz, 2H), 7.50 (d, *J* = 8.0 Hz, 2H), 7.36 - 7.39 (m, 1H), 4.78 (s, 2H), 1.96 - 2.06 (m, 1H); LC-MS: m/z = 186.1 $(M+H)^+$.

Step 2: Synthesis of intermediate **B3**

Compound **B3-3** (1.0 g, 5.4 mmol, 1.00 eq) was dissolved in dichloromethane (5.0 mL), and thionyl chloride (6.4 g, 53.9 mmol, 10.0 eq) was added at 0°C. After the addition was completed, the resulting mixture was heated up to 20°C and stirred for 15 hours. The reaction solution was concentrated under vacuum to obtain compound **B3** (1.1 g, 5.35 mmol, 99.1% yield) as a white solid. LC-MS: m/z = 204.0 $(M+H)^+$.

**Synthesis of general intermediate B4 (4-(4-(chloromethyl)phenyl)pyridine)**

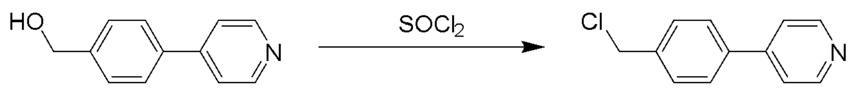


B4-1 B4

Compound **B4-1** (250.0 mg, 1.4 mmol, 1.00 eq) was dissolved in dichloromethane (5.0 mL), and thionyl chloride (802.0 mg, 6.8 mmol, 5.00 eq) was added at 0°C. After the addition was completed, the resulting mixture was heated up to 20°C and stirred for 15 hours. The reaction solution was concentrated under vacuum to obtain compound **B4** (260 mg, 1.28 mmol, 94.5% yield) as a white solid. LC-MS: m/z = 204.0 $(M+H)^+$.

**Synthesis** of **general intermediate B5 (5-(4-(chloromethyl)phenyl)-2-methylpyridine)**

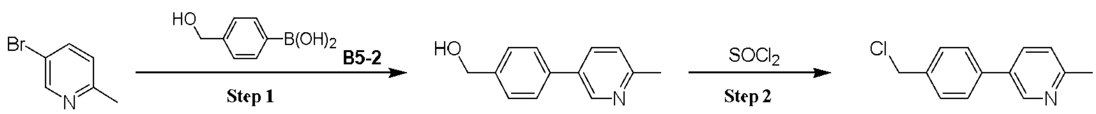


B5-1 B5-3 B5

Step 1: Synthesis of compound **B5-3** ((4-(6-methylpyridin-3-yl)phenyl)methanol)

Compound **B5-1** (3.0 g, 17.4 mmol, 1.00 eq), compound B5-2 (3.5 g, 22.6 mmol, 1.30 eq), sodium carbonate (13.6 g, 129.0 mmol, 7.40 eq), and tetrakis(triphenylphosphine)palladium (2.0 g, 1.74 mmol, 0.10 eq) were added to a mixed solvent of toluene (15.0 mL), water (15.0 mL) and ethanol (3.00 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% $NH_3$•$H_2O$) to obtain compound **B5-3** (3.0 g). $^1$H NMR (400 MHz, $CDCl_3$) δ 8.59 - 8.63 (m, 1H), 7.76 - 7.78 (m, 1H), 7.54 (d, *J* = 8.0 Hz, 2H), 7.47 (t, *J* = 4.0 Hz, 2H), 7.22 (d, *J* = 8.0 Hz, 1H), 4.76 (s, 2H), 2.61 (s, 3H), 2.33 - 2.37 (m, 1H); LC-MS: m/z = 200.1 $(M+H)^+$.

Step 2: Synthesis of intermediate **B5**

Compound **B5-3** (0.5 g, 2.5 mmol, 1.00 eq) was dissolved in dichloromethane (5.0 mL), and thionyl chloride (1.5 g, 12.5 mmol, 5.00 eq) was added at 0°C. After the addition was completed, the resulting mixture was heated up to 20°C and stirred for 15 hours. The reaction solution was concentrated under vacuum to obtain compound **B5** (530 mg, 2.43 mmol, 97.0% yield) as a white solid. LC-MS: m/z = 218.1 $(M+H)^+$.

**Synthesis of general intermediate B6 (2-(4-(chloromethyl)phenyl)-1-methyl-4-(trifluoromethyl)-1H-imidazole)**

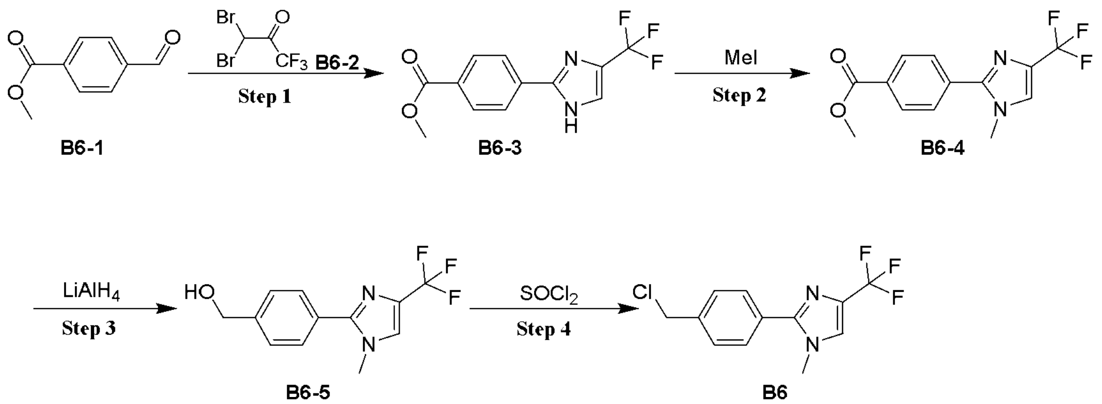


B6-1 B6-3 B6-4

B6-5 B6

Step 1: Synthesis of compound **B6-3** (methyl 4-(4-(trifluoromethyl)-1H-imidazol-2-yl)benzoate)

Compound **B6-2** (7.2 g, 26.8 mmol, 1.10 eq) and sodium acetate (2.2 g, 27.3 mmol, 1.12 eq) were dissolved in water (8.0 mL), and stirred at 100°C for 1 hour. Compound **B6-1** (4.0 g, 24.4 mmol, 1.00 eq) dissolved in methanol (80.0 mL) and ammonia water (22.0 mL) was added at 25°C. After the addition was completed, the resulting mixture was stirred at 25°C for 40 minutes, heated up to 100°C and stirred for 2 hours. Then, water (60.0 mL) was added to quench the reaction, and the resulting mixture was extracted three times with ethyl acetate (80.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **B6-3** (4.5 g). $^1$HNMR (400 MHz, $CDCl_3$) δ 13.4 (s, 1H), 8.10 - 8.12 (m, 2H), 8.04 - 8.06 (m, 2H), 7.99 (s, 1H), 3.87 (s, 3H); LC-MS: m/z = 271.0 $(M+H)^+$.

Step 2: Synthesis of compound **B6-4** (methyl 4-(1-methyl-4-(trifluoromethyl)-1H-imidazol-2-yl)benzoate)

Compound **B6-3** (2.5 g, 9.3 mmol, 1.00 eq) was dissolved in tetrahydrofuran (20.0 mL), and sodium hydride (444.1 mg, 11.1 mmol, 1.20 eq) was added in portions at 0°C. After the addition was completed, the resulting mixture was reacted at 0°C for 30 minutes. Then, iodomethane (6.8 g, 48.1 mmol, 5.20 eq) was added. After the addition was completed, the resulting mixture was heated up to 20°C and stirred for 15 hours. Then, ice water (20.0 mL) was added at 0°C to quench the reaction, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **B6-4** (1.1 g). $^1$H NMR (400 MHz, $CDCl_3$) δ 8.07 (d, *J* = 8.4Hz, 2H), 7.99 (s, 1H), 7.90 (d, *J* = 8.4Hz, 2H), 3.89 (s, 3H), 3.84 (s, 3H); LC-MS: m/z =285.1 $(M+H)^+$.

Step 3: Synthesis of compound **B6-5** ((4-(1-methyl-4-(trifluoromethyl)-1H-imidazol-2-yl)phenyl)methanol)

Compound **B6-4** (0.6 g, 2.1 mmol, 1.00 eq) was dissolved in tetrahydrofuran (6.0 mL), and lithium aluminum hydride (88.1 mg, 2.32 mmol, 1.10 eq) was added at 0°C. After the addition was completed, the resulting mixture was heated up to 25°C and stirred for 4 hours. Water (1.6 mL) and aqueous sodium hydroxide solution (1 M, 0.4 mL) were added at 0°C, and the resulting mixture was stirred at 0°C for 0.5 hours. Tetrahydrofuran (10.0 mL) and anhydrous sodium sulfate (1.0 g) were added, and the resulting mixture was filtered to obtain a filtrate. The filtrate was concentrated under vacuum to obtain crude product **B6-5** (505.4 mg), which was directly used in the next step. $^1$H NMR (400 MHz, $CDCl_3$) δ 7.92 (s, 1H), 7.67 (d, *J* = 8.4 Hz, 2H), 7.45 (d, *J* = 8.0 Hz, 2H), 5.30 - 5.33 (m, 1H), 4.58 (d, *J* = 5.6 Hz, 2H), 3.78 (s, 3H); LC-MS: m/z = 257.1 $(M+H)^+$.

Step 4: Synthesis of intermediate B6

Compound **B6-5** (500.0 mg, 2.0 mmol, 1.00 eq) was dissolved in dichloromethane (5.0 mL), and thionyl chloride (1.9 g, 15.6 mmol, 8.00 eq) was added at 0°C. After the addition was completed, the resulting mixture was heated up to 20°C and stirred for 15 hours. The reaction solution was concentrated under vacuum to obtain compound **B6** (523 mg, 1.90 mmol, 97.6% yield) as a brown solid. LC-MS: m/z = 275.1 $(M+H)^+$.

**Synthesis of general intermediate B7 (2-(4-(chloromethyl)cyclohexyl)pyrimidine)**

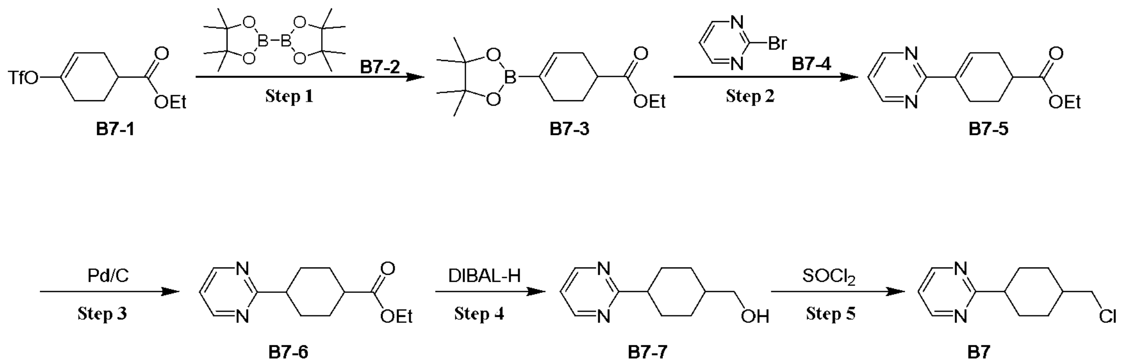


Step 1: Synthesis of compound **B7-3** (ethyl 4-(4,4,5,5-tetramethyl-1,3,2-dioxaborolan-2-yl)cyclohex-3-ene-1-carboxylate)

Compound **B7-1** (10.0 g, 33.1 mmol, 1.00 eq), compound **B7-2** (9.2 g, 36.4 mmol, 1.10 eq), [1,1'-bis(diphenylphosphino)ferrocene]palladium dichloride dichloromethane complex (1.4 g, 1.7 mmol, 0.05 eq), and potassium acetate (9.8 g, 99.3 mmol, 3.00 eq) were added to dioxane (80.0 mL) successively. The resulting mixture was purged three times with nitrogen, and stirred at 80°C for 2 hours under nitrogen atmosphere. Then, water (200.0 mL) was added, and the resulting mixture was extracted three times with ethyl acetate (70.0 mL). The organic layers were combined, washed twice with saturated brine (100.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 30/1) to obtain compound **B7-3** (4.5 g). $^1$H NMR (400 MHz, $CDCl_3$) δ 6.55 (s, 1H), 4.12-4.17 (m, 2H), 2.50-2.51 (m, 1H), 2.26-2.35 (m, 3H), 2.01-2.05 (m, 2H), 1.58-1.63 (m, 1H), 1.27-1.28 (m, 15H); LC-MS: m/z = 281.1 $(M+H)^+$.

Step 2: Synthesis of compound **B7-5** (ethyl 4-(pyrimidin-2-yl)cyclohex-3-ene-1-carboxylate)

Compound **B7-3** (4.9 g, 17.3 mmol, 1.10 *eq*), compound **B7-4** (2.5 g, 15.7 mmol, 1.00 *eq*), sodium carbonate (5.0 g, 47.2 mmol, 3.00 *eq*), and tetrakis(triphenylphosphine)palladium (1.8 g, 1.6 mmol, 0.10 *eq*) were added to dioxane (50.0 mL) and water (12.5 mL) successively. The resulting mixture was purged three times with nitrogen, and stirred at 90°C for 20 hours under nitrogen atmosphere. Then, water (100.0 mL) was added, and the resulting mixture was extracted three times with ethyl acetate (100.0 mL). The organic layers were combined, washed three times with saturated brine (100.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to

obtain compound **B7-5** (3.1 g). $^1$H NMR (400 MHz, $CDCl_3$) δ 8.67 (d, *J* = 4.80 Hz, 2H), 7.28-7.29 (m, 1H), 7.07-7.10 (m, 1H), 4.12-4.20 (m, 2H), 2.82-2.83 (m, 1H), 2.58-2.63 (m, 1H), 2.55-2.58 (m, 3H), 2.04-2.21 (m, 1H), 1.81-1.84 (m, 1H), 1.23-1.30 (m, 3H); LC-MS: m/z = 233.1 $(M+H)^+$.

Step 3: Synthesis of compound **B7-6** (ethyl 4-(pyrimidin-2-yl)cyclohexane-1-carboxylate)

Compound **B7-5** (3.0 g, 13.1 mmol, 1.00 *eq*) and wet palladium on carbon (1.4 g, 1.3 mmol, 10.0% purity, 0.10 *eq*) were added to ethanol (90.0 mL) successively, and stirred at 25°C under hydrogen atmosphere for 16 hours. The reaction solution was filtered to obtain a filtrate. The filtrate was concentrated under vacuum to obtain a crude product, which was purified by column chromatography (eluent: petroleum ether/ethyl acetate = 1/0 - 0/1) to obtain compound **B7-6** (2.1 g). $^1$H NMR (400 MHz, $CDCl_3$) δ 8.64-8.66 (m, 2H), 7.07-7.12 (m, 1H), 4.10-4.15 (m, 2H), 2.95-2.97 (m, 1H), 2.59-2.61 (m, 1H), 2.10-2.15 (m, 3H), 1.98-2.02 (m, 1H), 1.90-1.98 (m, 1H), 1.67-1.68 (m, 2H), 1.26-1.68 (m, 1H), 1.21-1.26 (m, 3H); LC-MS: m/z = 235.1 $(M+H)^+$.

Step 4: Synthesis of compound **B7-7** ((4-(pyrimidin-2-yl)cyclohexyl)methanol)

Compound **B7-6** (500.0 mg, 2.1 mmol, 1.00 eq) was dissolved in tetrahydrofuran (20.0 mL), and diisobutylaluminum hydride (1.0 M, 3.20 eq) was added at -65°C. After the addition was completed, the resulting mixture was reacted at -65°C for 0.5 hours, heated up to 20°C and stirred for 1 hour. Then, saturated aqueous ammonium chloride solution (20.0 mL) was added at 0°C. After the addition was completed, the resulting mixture was heated to 20°C and stirred for 1 hour. The reaction solution was filtered to obtain a filtrate. The organic layer was separated, dried over anhydrous sodium sulfate, and filtered to obtain a filtrate. The filtrate was concentrated under vacuum to obtain crude compound **B7-7** (263.5 mg), which was directly used in the next step. $^1$H NMR (400 MHz, $CDCl_3$) δ 8.69 (d, *J* = 4.80 Hz, 2H), 7.11-7.14 (m, 1H), 3.64 (d, *J* = 7.20 Hz, 2H), 3.04-3.08 (m, 1H), 2.05-2.08 (m, 2H), 1.83-1.85 (m, 3H), 1.64-1.69 (m, 4H), 1.14-1.26 (m, 1H); LC-MS: m/z = 193.0 $(M+H)^+$.

Step 5: Synthesis of intermediate **B7**

Compound **B7-7** (263.0 mg, 1.4 mmol, 1.00 eq) was dissolved in dichloromethane (5.0 mL), and thionyl chloride (814.0 mg, 6.8 mmol, 5.00 eq) was added at 0°C. After the addition was completed, the resulting mixture was heated up to 20°C and stirred for 15 hours. The reaction solution was concentrated under vacuum to obtain compound **B7** (300 mg, crude) as a light yellow oil. $^1$H NMR (400 MHz, $CDCl_3$) δ 8.79-8.98 (m, 2H), 7.17-7.25 (m, 1H), 3.59-3.76 (m, 1H), 3.26-3.47 (m, 2H), 2.05-2.37 (m, 2H), 1.73-1.88 (m, 4H), 1.47-1.56 (m, 1H), 1.45-1.47 (m, 1H), 1.27-1.45 (m, 1H); LC-MS: m/z = 211.2 $(M+H)^+$.

**Synthesis of general intermediate B8 ((4-(1-methyl-4-(trifluoromethyl)-1H-imidazol-2-yl)phenyl)methanamine)**

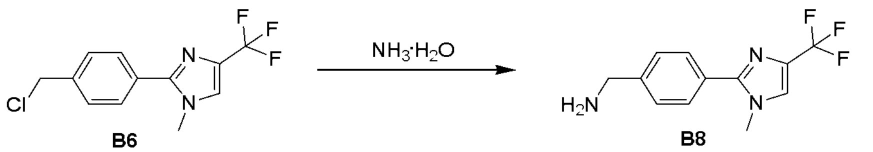


B6 B8

Intermediate **B6** (700.0 mg, 2.55 mmol, 1.00 *eq*) and aqueous ammonia (27.3 g, 233.0 mmol, 30% purity, 91.70 *eq*) were added to dioxane (15.0 mL), and the resulting mixture was reacted at 50°C for 12 hours in a sealed tube. The reaction solution was concentrated under vacuum to obtain a crude product, which was purified by reverse phase HPLC (0.1% $NH_3 \cdot H_2O$) to obtain **B8** (580 mg, 1.99 mmol, 78.0% yield, HCl) as a white solid. $^1$H NMR (400 MHz, $CDCl_3$) δ 7.73 - 7.76 (m, 3H), 7.63 - 7.65 (m, 2H), 4.22 (s, 2H), 3.80 (s, 3H); LC-MS: m/z = 256.0 $(M+H)^+$.

**Synthesis of general intermediate B9 (2-(5-(chloromethyl)thiophen-2-yl)-1-methyl-4-(trifluoromethyl)-1H-imidazole)**

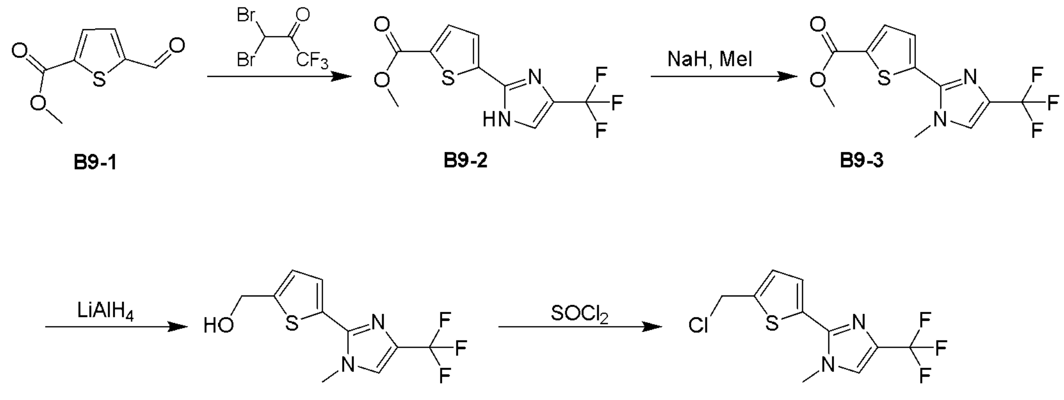


B9-1 B9-2 B9-3 B9-4 B9

In accordance with the synthesis of intermediate **B6,** general intermediate **B9** was obtained by replacing **B6-1** with an equimolar amount of **B9-1,** while other experimental conditions remained unchanged. Compound **B9** (330.0 mg, crude) was finally obtained as a white solid. LC-MS: m/z = 281.0 $(M+H)^+$.

**Synthesis of general intermediate B10 (2-(4-(chloromethyl)phenyl)pyridine)**

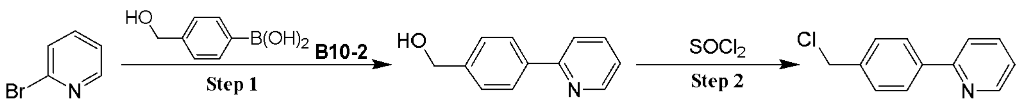


B10-1 B10-3 B10

Step 1: Synthesis of compound **B10-3** ((4-(pyridin-2-yl)phenyl)methanol)

Compound **B10-1** (10.0 g, 63.3 mmol, 6.02 mL, 1.00 eq), compound **B10-2** (12.5 g, 82.3 mmol, 1.30 eq), tetrakis(triphenylphosphine)palladium (7.31 g, 6.33 mmol, 0.100 eq), and sodium carbonate (49.6 g, 468 mmol, 7.40 eq) were added to a mixed solvent of toluene (50.0 mL), water (50.0 mL) and ethanol (10.0 mL) successively. The resulting mixture was purged three times with nitrogen, and stirred at 100°C for 12 hours under nitrogen atmosphere. The reaction solution was filtered to obtain a filtrate. The filtrate was concentrated under vacuum to obtain a crude product, which was purified by reverse phase HPLC (0.1% $NH_3$•$H_2O$) to obtain compound **B10-3** (9.63 g). $^1H$ NMR (400 MHz, $CDCl_3$) δ 8.67 (d, *J*=4.4Hz, 1H), 7.75 - 7.95 (m, 2H), 7.70 - 7.74 (m, 2H), 7.42 - 7.44 (m, 2H), 7.22 - 7.25 (m, 1H), 4.73 (s, 2H), 2.70 (s, 1H); LC-MS: m/z = 186.2 $(M+H)^+$.

Step 2: Synthesis of intermediate **B10**

Compound **B10-3** (1.0 g, 5.40 mmol, 1.00 eq) was dissolved in dichloromethane (10.0 mL), and thionyl chloride (3.2 g, 27.0 mmol, 1.96 mL, 5.00 eq) was added at 0°C. After the addition was completed, the resulting mixture was heated up to 20°C and stirred for 12 hours. The reaction solution was concentrated under vacuum to obtain **B10** (1.1 g, crude) as a white solid. LC-MS: m/z = 204.1 $(M+H)^+$.

**Synthesis of general intermediate B11 (2-(4-(chloromethyl)piperidin-1-yl)pyridine)**

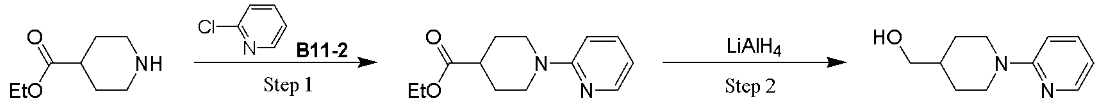


B11-1 B11-3 B11-4

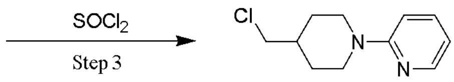


**B11**

Step 1: Synthesis of compound **B11-3** (ethyl 1-(pyridin-2-yl)piperidine-4-carboxylate)

Compound **B11-1** (20.8 g, 132 mmol, 20.4 mL, 1.00 eq), compound **B11-2** (15.0 g, 132.0 mmol, 1.00 eq) and triethylamine (26.7 g, 264 mmol, 36.8 mL, 2.00 eq) were dissolved in DMSO (50.0 mL) and the resulting mixture was reacted at 120 to 150°C for 16 hours. The reaction solution was cooled down to quench the reaction. Water (50 ml) was added at room temperature to quench the reaction, and the resulting mixture was extracted with ethyl acetate (60.0 mL) three times. The organic layers were combined, washed twice with saturated brine (30.0 mL), dried over anhydrous sodium sulfate, and filtered to obtain a filtrate. The filtrate was concentrated under vacuum to obtain a crude product, which was purified by silica gel column chromatography (petroleum ether: ethyl acetate 2:1) to obtain compound B11-32 (6.0 g, 19.4% yield, 100% purity) as a yellow oil. $^1H$ NMR (400 MHz, $CDCl_3$) δ 8.17 - 8.19 (m, 1H), 7.44 - 7.48 (m, 1H), 6.66 (d, J=8.4Hz, 1H), 6.59 - 6.61 (m, 1H), 4.21 - 4.24 (m, 2H), 4.13 - 4.18 (m, 2H), 2.92 - 2.99 (m, 2H), 2.52 - 2.53 (m, 1H), 1.98 - 2.02 (m, 2H), 1.75 - 1.79 (m, 2H), 1.25 - 1.28 (m, 3H). **LC-MS:** m/z =235.2$(M+H)^+$.

Step 2: Synthesis of compound **B11-4** ((1-(pyridin-2-yl)piperidin-4-yl)methanol)

Compound **B11-3** (6.00 g, 25.6 mmol) was dissolved in THF (60.0 mL), and lithium aluminum hydride ($LiAlH_4$, 1.07 g, 28.2 mmol, 1.10 eq) was carefully added at 0°C, and then the resulting mixture was stirred at room temperature for 16 hours. Additional lithium aluminum hydride ($LiAlH_4$, 486 mg, 12.8 mmol, 0.500 eq) was added and the resulting mixture was further stirred for 6 hours. $H_2O$ (20.0 mL) was carefully added at 0°C to quench the reaction. The resulting mixture was extracted with ethyl acetate (60.0 mL × 3). The organic layers were combined, washed twice with saturated brine (20.0 mL), dried over anhydrous sodium sulfate, and filtered to obtain a filtrate. The filtrate was concentrated under vacuum to obtain a crude product, which was purified by silica gel column chromatography (petroleum ether: ethyl acetate 1:1) to obtain compound **B11-4** (1.20 g, yield 23.8%, purity 97.8%) as a yellow oil. **$^1$H NMR** (400 MHz, $CDCl_3$) δ 8.18 (d, J=3.6Hz, 1H), 7.43 - 7.48 (m, 1H), 6.67 (d, J=8.8Hz, 1H), 6.58 - 6.59 (m, 1H),4.33 (d, J=12.8Hz, 2H), 3.54 (d, J=6.0Hz, 2H), 2.81 - 2.88 (m, 2H), 1.77 - 1.86 (m, 2H), 1.73 - 1.76 (m, 1H), 1.55 (s, 1H), 1.28 - 1.35 (m, 2H). **LC-MS:** m/z =193.2$(M+H)^+$.

Step 3: Synthesis of intermediate **B11**

Compound **B11-4** (1.20 g, 6.24 mmol, 1.00 eq) was dissolved in dichloromethane (12.0 mL), and thionyl chloride (3.71 g, 31.2 mmol, 2.26 mL, 5.00 eq) was added at 0°C. After the addition was completed, the resulting mixture was heated up to 20°C and stirred for 12 hours. The reaction solution was concentrated under vacuum to obtain compound B11 (1.57 g, crude) as a white solid. LC-MS: m/z = 211.1.

**Synthesis of general intermediate B12 (2-((1r,4r)-4-(chloromethyl)cyclohexyl)-1-methyl-4-(trifluoromethyl)-1H-imidazole)**

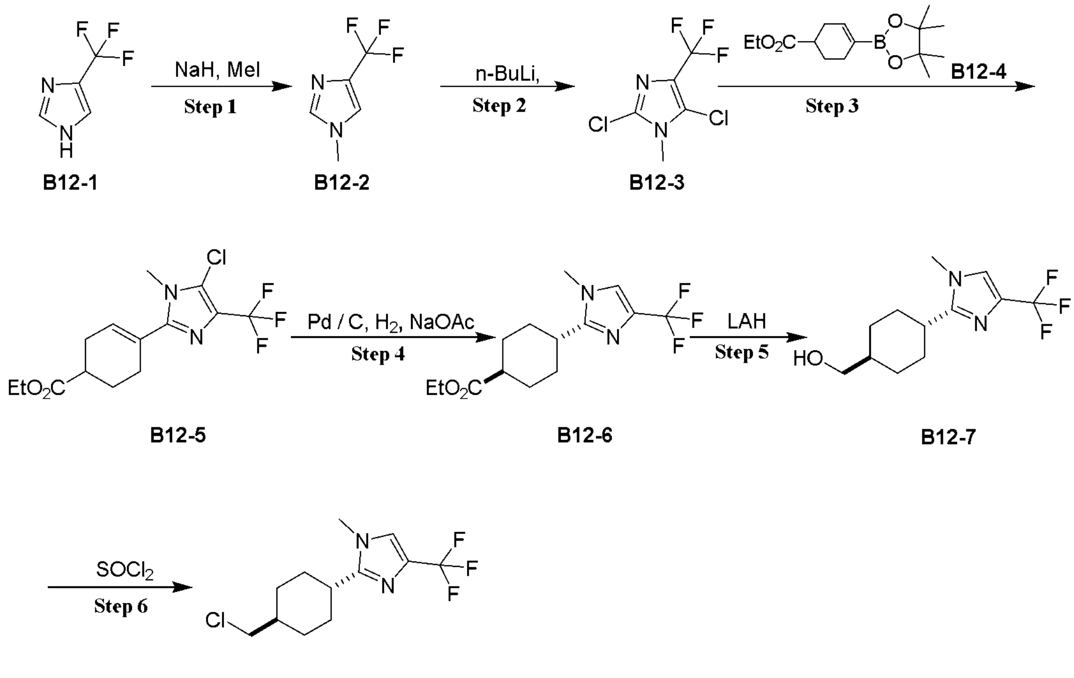


B12-1 B12-2 B12-3

B12-5 B12-6 B12-7

B12

Step 1: Synthesis of compound **B12-2** (1-methyl-4-(trifluoromethyl)-1H-imidazole)

Compound **B12-1** (33.0 g, 242.0 mmol, 1.00 eq) was dissolved in tetrahydrofuran (330.0 mL), and sodium hydride (9.7 g, 242.0 mmol, 60.0% purity, 1.00 eq) was added in portions at 0°C. After the addition was completed, the resulting mixture was reacted at 0°C for 30 minutes. Then, iodomethane (34.4 g, 242.0 mmol, 15.1 mL, 1.00 eq) was added. After the addition was completed, the resulting mixture was heated up to 25°C and stirred for 5 hours. Saturated aqueous ammonium chloride solution (30.0 mL) was added at 0°C to quench the reaction. Water (30.0 mL) was added, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain compound **B12-2** (21.0 g). $^1H$ NMR (400 MHz, $CDCl_3$) δ 7.48 (s, 1H), 7.24 (s, 1H), 3.75 (s, 3H); LC-MS: m/z = 151.1 $(M+H)^+$.

Step 2: Synthesis of compound **B12-3** (2,5-dichloro-1-methyl-4-(trifluoromethyl)-1H-imidazole)

Compound **B12-2** (16.8 g, 111.0 mmol, 1.00 eq) was dissolved in anhydrous tetrahydrofuran (370.0 mL), and *n*-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **B12-3** (12.0 g). LC-MS: m/z = 219.0 $(M+H)^+$.

Step 3: Synthesis of compound **B12-5** (ethyl 4-(5-chloro-1-methyl-4-(trifluoromethyl)-1H-imidazol-2-yl)cyclohex-3-ene-1-carboxylate)

Compound **B12-3** (7.0 g, 31.9 mmol, 1.00 eq), compound **B12-4** (9.0 g, 31.9 mmol, 1.00 eq), potassium phosphate (20.3 g, 95.8 mmol, 3.00 eq), chloro(2-dicyclohexylphosphino-2',4',6'-triisopropyl-1,1'-biphenyl)[2-(2'-amino-1,1'-biphenyl)]palladium (II) (1.3 g, 1.6 mmol, 0.05 eq) and 2-dicyclohexylphosphino-2',4',6'-triisopropylbiphenyl (5.9 g, 12.4 mmol, 0.39 eq) were added to a mixed solvent of dioxane (70.0 mL) and water (10.0 mL) successively. The resulting mixture was purged three times with nitrogen, and stirred at 100°C for 5 hours under nitrogen atmosphere. The reaction

solution was filtered to obtain a filtrate. The filtrate was concentrated under vacuum to obtain a crude product, which was purified by reverse phase HPLC (0.1% FA) to obtain compound **B12-5** (1.9 g). $^1$H NMR (400 MHz, $CDCl_3$) δ 6.05 (d, *J* = 2.0 Hz, 1H), 4.18 (q, *J* = 7.2 Hz, 2H), 3.61 (s, 3H), 2.50 - 2.67 (m, 5H), 2.13 - 2.14 (m, 1H), 1.86 - 1.89 (m, 1H), 1.28 (t, *J* = 7.2 Hz, 3H); LC-MS: m/z = 337.2 $(M+H)^+$.

Step 4: Synthesis of compound **B12-6** (ethyl (1R,4R)-4-(1-methyl-4-(trifluoromethyl)-1H-imidazol-2-yl)cyclohexane-1-formate)

Compound **B12-5** (1.9 g, 5.6 mmol, 1.00 eq), sodium acetate (925.0 mg, 11.2 mmol, 2.00 eq) and wet palladium on carbon (0.5 g, 1.4 mmol, 10.0% purity, 0.25 eq) were added to ethanol (30.0 mL) successively, and the resulting mixture was reacted at 50°C under hydrogen atmosphere (15 psi) for 5 hours. The reaction solution was filtered to obtain a filtrate, which was concentrated under vacuum to obtain a crude product. The crude product was purified by preparative HPLC (column: Waters Xbridge C18 150×50mm×10μm; mobile phase: [water ($NH_4HCO_3$)-ACN]; B%: 34%-64%, 10 min) to obtain compound **B12-6** (0.3 g). $^1$H NMR (400 MHz, $CDCl_3$) δ 7.13 (s, 1H), 4.15(q, *J* = 6.8 Hz, 2H), 3.64(s, 3H), 2.60 - 2.65 (m, 1H), 2.41 - 2.44 (m, 1H), 2.12 - 2.15 (m, 2H), 1.97 - 1.98 (m, 2H), 1.77 - 1.80 (m, 2H), 1.54 - 1.59 (m, 2H), 1.27 (t, *J* = 8.8 Hz, 3H); LC-MS: m/z = 305.0 $(M+H)^+$.

Step 5: Synthesis of compound **B12-7** (((1R,4R)-4-(1-methyl-4-(trifluoromethyl)-1H-imidazol-2-yl)cyclohexyl)methanol)

Compound **B12-6** (0.6 g, 2.0 mmol, 1.00 eq) was dissolved in tetrahydrofuran (10.0 mL), and lithium aluminum hydride (224.0 mg, 5.9 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **B12-7** (0.5 g), which was directly used in the next step. $^1$H NMR (400 MHz, $CDCl_3$) δ 7.12 (s, 1H), 3.63 (s, 3H), 3.52 (d, *J* = 5.6 Hz, 2H), 2.59 - 2.65 (m, 1H), 1.95 - 1.98 (m, 4H), 1.77 - 1.80 (m, 3H), 1.07 - 1.17 (m, 2H); LC-MS: m/z = 263.1 $(M+H)^+$.

Step 6: Synthesis of intermediate **B12**

Compound **B12-7** (500.0 mg, 1.9 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain compound **B12** (550 mg, 1.36 mmol, 71.5% yield, HCl) as a light yellow solid. LC-MS: m/z = 281.0 $(M+H)^+$.

**Synthesis of general intermediate B13 (4-(chloromethyl)-1-(1-methyl-4-(trifluoromethyl)-1H-imidazol-2-yl)piperidine hydrochloride)**

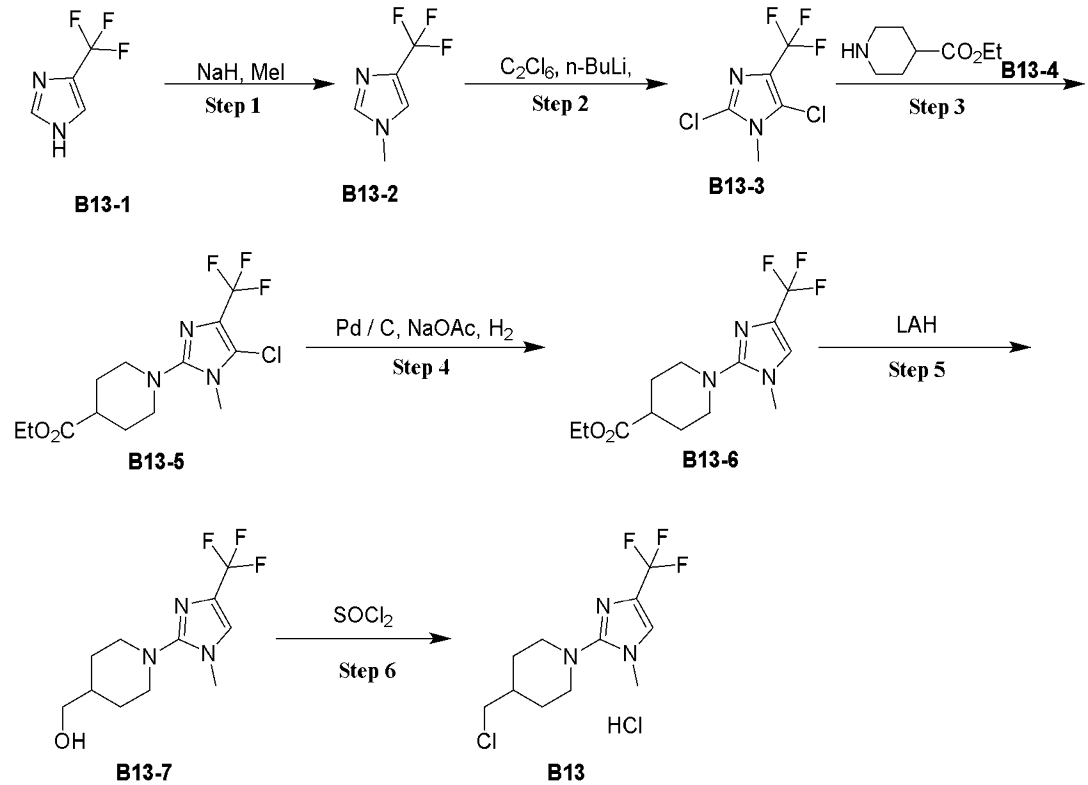


Step 1: Synthesis of compound **B13-2** (1-methyl-4-(trifluoromethyl)-1H-imidazole)

Compound **B13-1** (33.0 g, 242 mmol, 1.00 eq) was dissolved in tetrahydrofuran (330.0 mL), and sodium hydride (9.70 g, 242 mmol, 60.0% purity, 1.00 eq) was added in portions at 0°C. After the addition was completed, the resulting mixture was reacted at 0°C for 30 minutes. Then, iodomethane (34.4 g, 242 mmol, 15.1 mL, 1.00 eq) was added. After the addition was completed, the resulting mixture was heated up to 25°C and stirred for 4 hours. Then, ice water (200.0 mL) was added at 0°C to quench the reaction, and the resulting mixture was extracted three times with ethyl acetate (200.0 mL). The organic layers were combined, dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain compound **B13-2** (21.0 g). $^1H$ NMR (400 MHz, $CDCl_3$) δ 7.48 (s, 1H), 7.24 (s, 1H), 3.75 (s, 3H); LC-MS: m/z = *151.1* $(M+H)^+$.

Step 2: Synthesis of compound **B13-3** (2,5-dichloro-1-methyl-4-(trifluoromethyl)-1H-imidazole)

Compound **B13-2** (16.8 g, 111 mmol, 1.00 eq) was dissolved in anhydrous tetrahydrofuran (370.0 mL), and *n*-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **B13-3** (12.0 g). LC-MS: m/z = 219.0 $(M+H)^+$.

Step 3: Synthesis of compound **B13-5** (ethyl 1-(5-chloro-1-methyl-4-(trifluoromethyl)-1H-imidazol-2-yl)piperidine-4-carboxylate)

Compound **B13-3** (7.0 g, 31.9 mmol, 1.00 eq), compound **B13-4** (7.54 g, 47.9 mmol, 7.39 mL, 1.50 eq), N,N-diisopropylethylamine (16.5 g, 127 mmol, 22.2 mL, 4.00 eq) and sodium iodide (479 mg, 3.20 mmol, 0.100 eq) were added to N,N-dimethylformamide (70.0 mL) successively, and the resulting mixture was reacted at 130°C for 72 hours. Water (350 mL) was added, the resulting mixture was stirred for 5 minutes, and extracted three times with ethyl acetate (100 mL).

The organic layers were combined, dried over anhydrous sodium sulfate, and filtered to obtain a filtrate. The filtrate was concentrated under vacuum to obtain a crude product, which was purified by reverse phase HPLC (0.1% FA) to obtain compound **B13-5** (2.2 g). $^1$H NMR (400 MHz, $CDCl_3$) δ 4.17 (q, *J* = 7.2Hz, 2H), 3.45 (s, 3H), 3.24 - 3.28 (m, 2H), 2.90 - 2.97 (m, 2H), 2.40 - 2.45 (m, 1H), 2.02 - 2.06 (m, 2H), 1.85 - 1.88 (m, 2H), 1.28 (t, *J* = 8.0Hz, 2H); LC-MS: m/z = 340.1 $(M+H)^+$.

Step 4: Synthesis of compound **B13-6** (ethyl 1-(1-methyl-4-(trifluoromethyl)-1H-imidazol-2-yl)piperidine-4-carboxylate)

Compound **B13-5** (2.2 g, 6.48 mmol, 1.00 eq), sodium acetate (1.06 g, 12.9 mmol, 2.00 eq) and wet palladium on carbon (0.5 g, 1.94 mmol, 10.0% purity, 0.300 eq) were added to ethanol (40.0 mL) successively, and the resulting mixture was reacted at 50°C under hydrogen atmosphere (15 psi) for 36 hours. The reaction solution was filtered to obtain a filtrate, which was concentrated under vacuum to obtain compound **B13-6** (1.9 g). $^1$H NMR (400 MHz, $CDCl_3$) δ 7.02 (s, 1H), 4.15 (q, *J* = 7.2Hz, 2H), 3.52 (s, 3H), 3.25 - 3.29 (m, 2H), 2.92 - 2.98 (m, 2H), 2.40 - 2.45 (m, 1H), 2.01 - 2.05 (m, 2H), 1.85 - 1.89 (m, 2H), 1.28 (t, *J* = 6.8Hz, 3H); LC-MS: m/z = 306.1 $(M+H)^+$.

Step 5: Synthesis of compound **B13-7** ((1-(1-methyl-4-(trifluoromethyl)-1H-imidazol-2-yl)piperidin-4-yl)methanol)

Compound **B13-6** (1.2 g, 3.93 mmol, 1.00 eq) was dissolved in tetrahydrofuran (20.0 mL), and lithium aluminum hydride (447 mg, 11.8 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **B13-7** (1.0 g), which was directly used in the next step. $^1$H NMR (400 MHz, $CDCl_3$) δ 7.01 (s, 1H), 3.57 (d, *J* = 6.4Hz, 2H), 3.51 (s, 3H), 3.27 - 3.30 (m, 2H), 2.90 - 2.96 (m, 2H), 1.84 - 1.87 (m, 2H), 1.65 - 1.70 (m, 1H), 1.39 - 1.42(m, 2H); LC-MS: m/z = 264.1 $(M+H)^+$.

Step 6: Synthesis of intermediate **B13**

Compound **B13-7** (1.0 g, 3.80 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain compound **B13** (1.1 g, 3.46 mmol, 91.0% yield, HCl) as a brown solid. LC-MS: m/z = 282.1 $(M+H)^+$.

**Synthesis of general intermediate B14 (2-(5-(chloromethyl)furan-2-yl)-1-methyl-4-(trifluoromethyl)-1H-imidazole)**

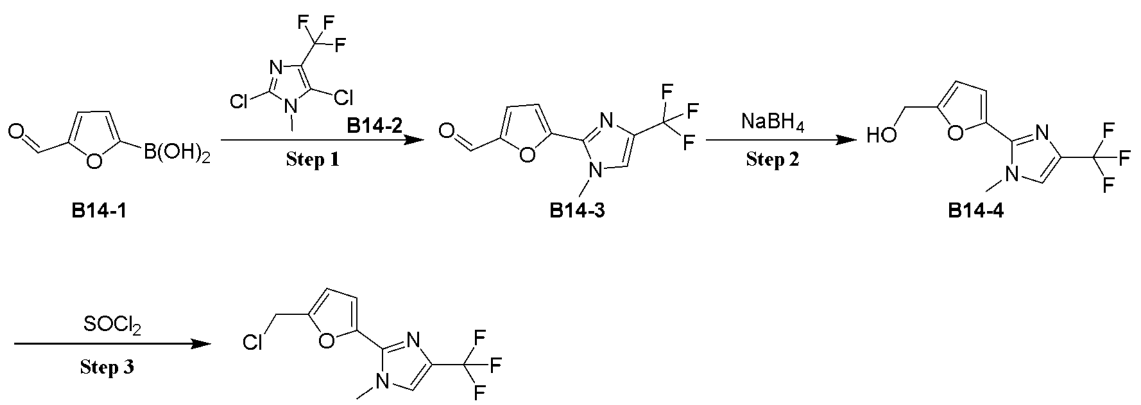


B14-1 B14-3 B14-4 B14

Step 1: Synthesis of compound **B14-3** (5-(1-methyl-4-(trifluoromethyl)-1H-imidazol-2-yl)furan-2-carbaldehyde)

Compound **B14-2** (400 mg, 1.83 mmol, 1.00 eq), **B14-1** (255 mg, 1.83 mmol, 1.00 eq), methanesulfonato (2-dicyclohexylphosphino-2',6'-dimethoxy-1,1'-biphenyl)(2'-amino-1,1'-biphenyl-2-yl)palladium (II) (71.2 mg, 91.3 μmol, 0.0500 eq), and potassium phosphate (1.16 g, 5.48 mmol, 3.00 eq) were added to n-butanol (3.0 mL) and water (0.5

mL) successively. The resulting mixture was purged three times with nitrogen, and reacted at 60°C for 12 hours under nitrogen atmosphere. The reaction solution was concentrated under vacuum to obtain a crude product, which was purified by preparative HPLC (column: Phenomenex C18 75×30mm×3μm; mobile phase: [water (FA)-ACN]; B%: 22%-52%, 7 min) to obtain compound **B14-3** (60.0 mg). $^1$H NMR (400 MHz, $CDCl_3$) δ 9.70 (s, 1H), 7.37 (d, *J* = 3.6Hz, 1H), 7.32 (s, 1H), 7.22 (d, *J* = 3.6 Hz, 1H), 4.07 (s, 3H); LC-MS: m/z = 245.2 (M+H)+.

Step 2: Synthesis of compound **B14-4** ((5-(1-methyl-4-(trifluoromethyl)-1H-imidazol-2-yl)furan-2-yl)methanol)

Compound **B14-3** (60.0 mg, 245 μmol, 1.00 eq) was dissolved in methanol (1.0 mL), followed by the addition of sodium borohydride (90.0 mg, 2.38 mmol, 9.68 eq), the resulting mixture was stirred at 15°C for 30 minutes. Then saturated aqueous ammonium chloride solution (10.0 mL) was added at 0°C and water (20.0 mL) was added, and the resulting mixture was extracted three times with ethyl acetate (10.0 mL). The organic layers were combined, dried over anhydrous sodium sulfate, and filtered to obtain a filtrate, which was concentrated under vacuum to obtain compound **B14-4** (50.0 mg). $^1$H NMR (400 MHz, $CDCl_3$) δ 7.25 (s, 1H), 6.92 (d, *J* = 3.2Hz, 1H), 6.42 (d, *J* = 3.2Hz, 1H), 4.70 (s, 2H), 3.93 (s, 3H); LC-MS: m/z = 247.1 $(M+H)^+$.

Step 3: Synthesis of intermediate **B14**

Compound **B14-4** (50.0 mg, 203 μmol, 1.00 eq) was dissolved in dichloromethane (2.0 mL), and thionyl chloride (1.2 g, 10.1 mmol, 50.0 eq) was added at 0°C. After the addition was completed, the resulting mixture was heated up to 25°C and reacted for 24 hours. The reaction solution was concentrated under vacuum to obtain compound **B14** (45 mg, crude) as a light yellow oil. LC-MS: m/z = 265.1 $(M+H)^+$.

**Synthesis of general intermediate B15 (8-(4-(chloromethyl)phenyl)imidazo[1,2-a]pyrazine)**

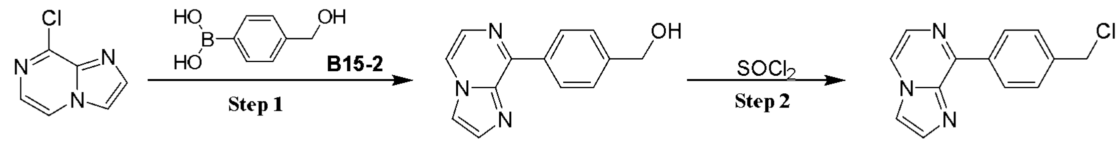


B15-1 B15-3 B15

Step 1: Synthesis of compound **B15-3** ((4-(imidazo[1,2-a]pyrazin-8-yl)phenyl)methanol)

Compound **B15-1** (9.6 g, 62.5 mmol, 1.00 *eq*), compound **B15-2** (12.4 g, 81.3 mmol, 1.30 *eq*), tetrakis(triphenylphosphine)palladium (7.2 g, 6.25 mmol, 0.10 *eq*), and sodium carbonate (49.0 g, 463 mmol, 7.40 *eq*) were added to a mixed solvent of toluene (48.0 mL), water (48.0 mL) and ethanol (9.6 mL) successively. The resulting mixture was purged three times with nitrogen, and stirred at 100°C for 12 hours under nitrogen atmosphere. The reaction solution was filtered to obtain a filtrate, which was concentrated under vacuum to obtain a crude product. The crude product was purified by reverse phase HPLC (0.1% $NH_3$•$H_2O$) to obtain compound **B15-3** (12.7 g). LC-MS: m/z = 226.2 $(M+H)^+$.

Step 2: Synthesis of intermediate **B15**

Compound **B15-3** (6.0 g, 26.6 mmol, 1.00 *eq*) was dissolved in dichloromethane (10.0 mL), and thionyl chloride (15.9 g, 133 mmol, 5.00 *eq*) was added at 0°C. After the addition was completed, the resulting mixture was heated up to 20°C and stirred for 12 hours. The reaction solution was concentrated under vacuum to obtain compound **B15** (7.3 g, 26.2 mmol, 98.2% yield, HCl) as an off-white solid. LC-MS: m/z = 244.1 $(M+H)^+$.

**Synthesis of general intermediate B16 ((4-(5-(trifluoromethyl)pyridin-2-yl)bicyclo[2.2.2]octan-1-yl)methyl-4-methylbenzenesulfonate)**

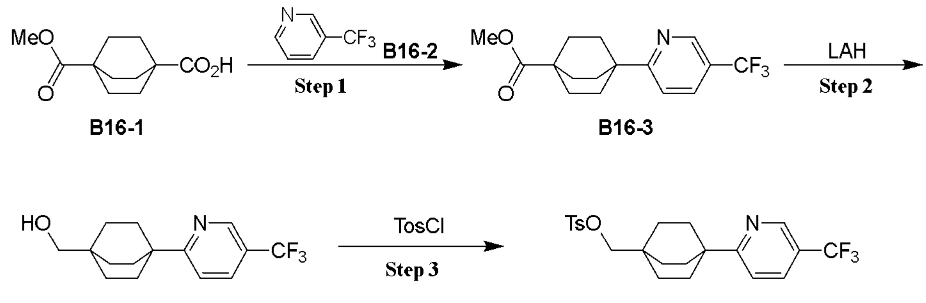


B16-1 B16-3

B16-4 B16

Step 1: Synthesis of compound **B16-3** (methyl 4-(5-(trifluoromethyl)pyridin-2-yl)bicyclo[2.2.2]octane-1-carboxylate)

Compound **B16-1** (25.0 g, 118 mmol, 1.00 eq), compound **B16-2** (20.8 g, 141 mmol, 1.20 eq), ammonium persulfate (26.9 g, 118 mmol, 25.6 mL, 1.00 eq) and silver nitrate (4.01 g, 23.6 mmol, 0.20 eq) were added to a mixed solvent of dichloromethane (750.0 mL) and water (750.0 mL) successively. The resulting mixture was reacted at 20°C for 16 hours, followed by the addition of dichloromethane (250.0 mL), and filtered to obtain a filtrate, which was washed with water (100.0 mL) three times. The organic layer was dried over anhydrous sodium sulfate, and filtered to obtain a filtrate, which was concentrated under vacuum to obtain a crude product. The crude product was purified by preparative HPLC (alkaline conditions, $NH_3$•$H_2O$/MeCN/$H_2O$) to obtain compound **B16-**3 (7.5 g). $^1$H NMR (400 MHz, $CDCl_3$) δ 8.81 (s, , 1H), 7.85 (dd, $J_1$ = 4.0Hz, $J_2$ = 4.0Hz, 1H), 7.37 (d, $J$ = 4.0Hz, 1H), 3.69 (s, 3H), 1.96 (s, 12H).

Step 2: Synthesis of compound **B16-4** ((4-(5-(trifluoromethyl)pyridin-2-yl)bicyclo[2.2.2]octan-1-yl)methanol)

Compound **B16-3** (2.5 g, 7.98 mmol, 1.00 eq) was dissolved in tetrahydrofuran (50.0 mL), and lithium aluminum hydride (908 mg, 23.9 mmol, 3.00 eq) was slowly added at 0°C. After the addition was completed, the resulting mixture was heated up to 20°C and stirred for 3 hours. Then, ice water (50.0 mL) was added at 0°C and the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **B16-4** (2.3 g). LC-MS: m/z = 286.1 $(M+H)^+$.

Step 3: Synthesis of intermediate **B16**

Compound **B16-4** (1.6 g, 5.61 mmol, 1.00 eq) and *p*-toluenesulfonyl chloride (1.28 g, 6.73 mmol, 1.20 eq) were added to pyridine (25.0 mL), and the resulting mixture was reacted at 20°C for 12 hours. The reaction solution was concentrated under vacuum. Water (10.0 mL) was added, and the resulting mixture was stirred for 5 minutes, and extracted three times with dichloromethane (5.0 mL). The organic layers were combined, dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 3/1) to obtain compound **B16** (1.5 g, 3.38 mmol, 60.2% yield, 98.9% purity) as a white solid. $^1$H NMR (400 MHz, $CDCl_3$) δ 8.79 (s, 1H), 7.84-7.79 (m, 3H), 7.38 (m, 3H), 3.72 (s, 2H), 2.47 (s, 3H), 1.93-1.89 (m, 6H), 1.57-1.54 (m, 6H); LC-MS: m/z = 440.2 $(M+H)^+$.

**Synthesis of general intermediate B17 (1-(4-(chloromethyl)phenyl)-5-methyl-3-(trifluoromethyl)-1H-pyrazole)**

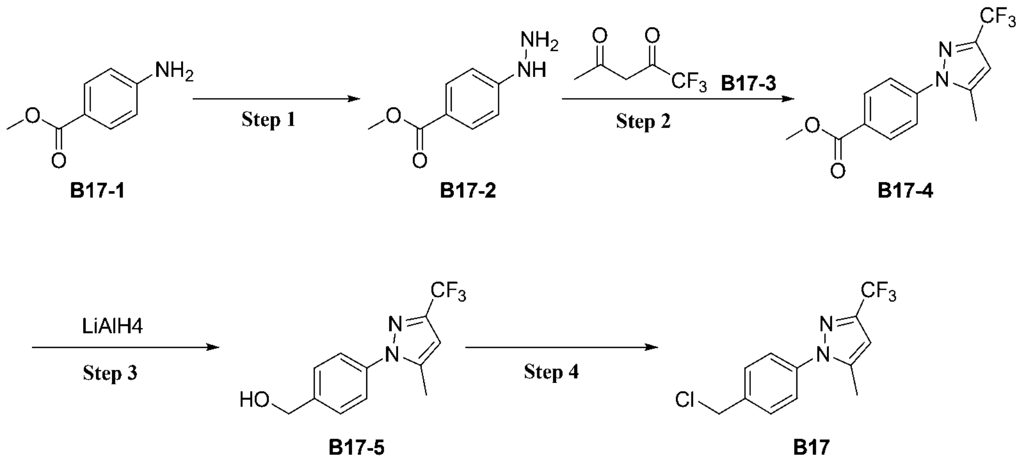


B17-1 B17-2 B17-4

B17-5 B17

Step 1: Synthesis of compound **B17-2** (methyl 4-hydrazinylbenzoate)

Compound **B17-1** (15.0 g, 90.32 mmol, 1.0 eq) was dissolved in 37% aqueous hydrochloric acid solution (25.0 mL), followed by the addition of sodium nitrite (6.2 g, 90.32 mmol, 1.0 eq) dissolved in water (10.0 mL) at 0°C. After the addition was completed, the resulting mixture was stirred for 10 minutes. Then, tin chloride (85.6 g, 451.62 mmol, 5.0 eq) dissolved in 37% aqueous hydrochloric acid solution (75.0 mL) was added dropwise at 0°C. After the addition was completed, the resulting mixture was heated up to 25°C and stirred for 2 hours. The reaction solution was filtered to obtain a filter cake, which was washed three times with ethyl acetate (50.0 mL), and concentrated under vacuum to obtain compound **B17-2** (16.5 g). LC-MS: m/z = 167.1 $(M+H)^+$.

Step 2: Synthesis of compound **B17-4** (methyl 4-(5-methyl-3-(trifluoromethyl)-1H-pyrazol-1-yl)benzoate)

Compound **B17-2** (5 g, 24.75 mmol, 1.0 eq) and compound **B17-3** (3.8 g, 24.75 mmol, 1.0 eq) were added to hexafluoroisopropanol (25.0 mL). Triethylamine (5.0 g, 49.50 mmol, 2.0 *eq*) dissolved in hexafluoroisopropanol (25.0 mL) was added dropwise at 0°C. After the addition was completed, the resulting mixture was heated up to 25°C and stirred for 1 hour. Then, water (10 mL) was added, and the resulting mixture was extracted three times with dichloromethane (200.0 mL). The organic layers were combined, washed twice with saturated brine (200.0 mL), dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 1/1) to obtain compound **B17-4** (5.3 g). LC-MS: m/z = 285.0 $(M+H)^+$.

Step 3: Synthesis of compound **B17-5** ((4-(5-methyl-3-(trifluoromethyl)-1H-pyrazol-1-yl)phenyl)methanol)

Compound **B17-4** (5.3 g, 18.65 mmol, 1.0 *eq*) was dissolved in tetrahydrofuran (100.0 mL), and lithium aluminum hydride (1.9 g, 46.64 mmol, 2.5 *eq*) was slowly added at 0°C. After the addition was completed, the resulting mixture was heated up to 20°C and stirred for 6 hours. Then, ice water (50.0 mL) was added at 0°C, and the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 1/2) to obtain compound **B17-5** (3.6 g). LC-MS: m/z = 257.1 $(M+H)^+$.

Step 4: Synthesis of intermediate **B17**

Compound **B17-5** (1.0 g, 3.91 mmol, 1.0 *eq*) was dissolved in dichloroethane (25.0 mL), and thionyl chloride (1.4 g, 11.73 mmol, 5.2 *eq*) was added at 0°C. After the addition was completed, the resulting mixture was heated up to 50°C and stirred for 2 hours. The reaction solution was concentrated under vacuum to obtain compound **B17** (1.0 g, 3.64 mmol, 93.1% yield, 96.9% purity) as a white solid. $^1H$ NMR (400 MHz, $CDCl_3$) $\delta$ 7.53 (d, *J* = 8.7Hz, 2H), 7.46 (d, *J* = 8.7Hz, 2H), 6.47 (s, 1H), 4.64 (s, 2H), 2.37 (s, 3H); LC-MS: m/z = 275.1 $(M+H)^+$.

**General intermediate BB1 (2-chloro-5-methoxy-N-methylpyrimidin-4-amine)**

Intermediate **BB1** was purchased from Leyan Reagents.

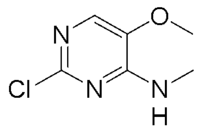

BB1

**General intermediate BB2 (2-chloro-5-methoxypyrimidin-4-amine)**

Intermediate **BB2** was purchased from Leyan Reagents.

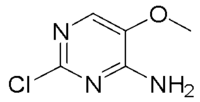

BB2

**General intermediate BB3 (2-chloro-5-isopropoxy-N-methylpyrimidin-4-amine)**

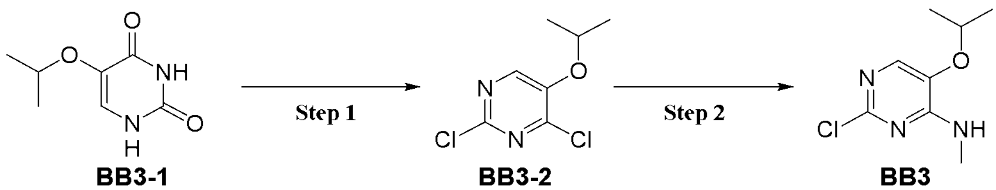


BB3-1 BB3-2 BB3

Step 1: Synthesis of compound **BB3-2** (2,4-dichloro-5-isopropoxypyrimidine)

2, 4-Dichloro-5-isopropylpyrimidine (compound **BB3-1,** 0.5 g, 2.41 mmol, 1.00 eq) was added to phosphorus oxychloride (5.0 mL), and refluxed under nitrogen atmosphere for 4 hours. The reaction solution was cooled to room temperature, and concentrated under vacuum to remove phosphorus oxychloride. Ice water (100.0 mL) was added, and the resulting mixture was extracted three times with ethyl acetate (100.0 mL). The organic layers were combined, dried over anhydrous sodium sulfate, and concentrated under vacuum to obtain compound **BB3-2** (551.0 mg, crude) as a light yellow oil. LC-MS: m/z = 207.1 $(M+H)^+$.

Step 2: Synthesis of intermediate **BB3**

Compound **BB3-2** (100 mg, 0.48 mmol, 1.00 eq), methylamine hydrochloride (97.8 mg, 1.45 mmol, 3.00 eq) and cesium carbonate (472.4 mg, 1.45 mmol, 3.00 eq) were added to N,N-dimethylformamide (2.0 mL) successively, and the resulting mixture was stirred at 60°C for 6 hours. Ethyl acetate (15.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 0/1) to obtain intermediate **BB3** (38.8 mg, 0.19 mmol, 40.1% yield). LC-MS: m/z = 202.1 $(M+H)^+$.

**General intermediate BB4 (2-chloro-5-isopropoxypyrimidin-4-amine)**

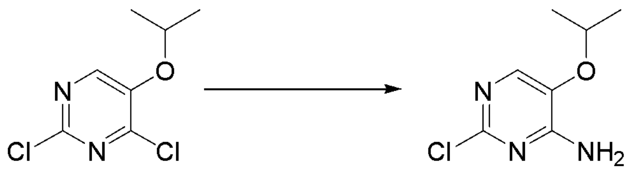

BB3-2 BB4

Compound **BB3-2** (100 mg, 0.48 mmol, 1.00 eq) and aqueous ammonia (1.68 g, 48.0 mmol, 30% purity, 100.0 *eq*) were added to dioxane (3.0 mL), and the resulting mixture was reacted at 50°C in a sealed tube for 12 hours. The reaction solution was concentrated under vacuum to obtain a crude product, which was purified by reverse phase HPLC (0.1% $NH_3$•$H_2O$) to obtain intermediate **BB4** (27.3 mg, 0.15 mmol, 30.3% yield) as a white solid. LC-MS: m/z = 188.2 $(M+H)^+$.

**General intermediate BB12 (2-chloro-5-(trifluoromethyl)pyrimidin-4-amine)**

Intermediate **BB12** was purchased from Bide Pharmatech.

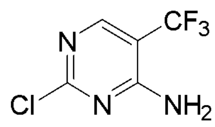

**BB12**

**Synthesis of general intermediate BB13 (2-chlorofuro[3,2-d]pyrimidin-4-amine)**

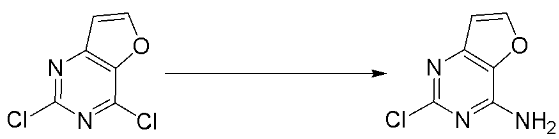

**BB13-1** **BB13**

2,4-Dichlorofuro[3,2-d]pyrimidine (compound **BB13-1,** 5.0 g, 26.46 mmol, 1.00 *eq*) and aqueous ammonia (30.9 g, 264.6 mmol, 30% purity, 100.0 *eq*) were added to dioxane (100.0 mL), and the resulting mixture was reacted at 50°C in a sealed tube for 12 hours. The reaction solution was concentrated under vacuum to obtain a crude product, which was purified by reverse phase HPLC (0.1% $NH_3$•$H_2O$) to obtain compound **BB13** (3.2 g, 18.87 mmol, 71.3% yield) as a white solid. LC-MS: m/z = 170.1 $(M+H)^+$.

**Synthesis of general intermediate BB14 (2-chloropyrido[3,2-d]pyrimidin-4-amine)**

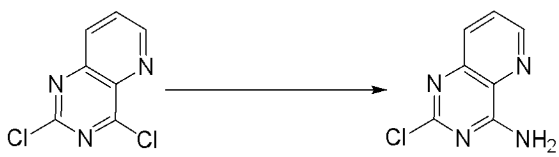

**BB14-1** **BB14**

In accordance with the same steps of intermediate **BB13,** intermediate **BB14** (1.1 g, 6.09 mmol, 40.6% yield) was obtained using 2,4-dichloropyrido[3,2-d]pyrimidine **(BB14-1,** 3.0 g, 15.0 mmol, 1.00 *eq*) as a starting material. LC-MS: m/z = 181.0 $(M+H)^+$.

**General intermediate BB15 (2,5-dichloropyrimidin-4-amine)**

Intermediate **BB15** was purchased from Bide Pharmatech.

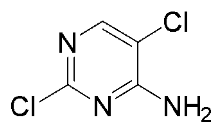

**BB15**

**Synthesis of general intermediate BB16 (2-chloro-5-fluoro-N-methylpyrimidin-4-amine)**

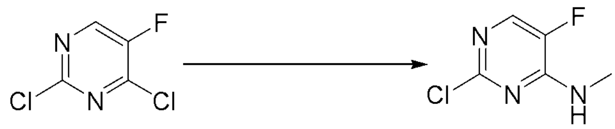

BB16-1 BB16

2,4-Dichloro-5-fluoropyrimidine (compound **BB16-1,** 3.0 g, 18.07 mmol, 1.00 *eq*), methylamine hydrochloride (3.66 g, 54.23 mmol, 3.00 *eq)* and cesium carbonate (23.55 g, 72.28 mmol, 4.00 *eq*) were added to N,N-dimethylformamide (500.0 mL) successively, and the resulting mixture was stirred at 60°C for 6 hours. Ethyl acetate (1000.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 0/1) to obtain compound **BB16** (1.1 g, 6.81 mmol, 37.7% yield). LC-MS: m/z = 162.0 $(M+H)^+$.

**Synthesis of general intermediate BB16-D3 (2-chloro-5-fluoro-N-(methyl-D3)pyrimidin-4-amine)**

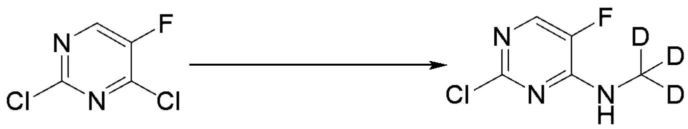

BB16-1 BB16-D3

In accordance with the same steps of intermediate **BB16,** intermediate **BB16-D3** (1.47 g, 8.93 mmol, 49.4% yield) was obtained using 2,4-dichloro-5-fluoropyrimidine **(BB16-1,** 3.0 g, 18.07 mmol, 1.00 *eq*) as a starting material. LC-MS: m/z = 165.1 $(M+H)^+$,

**General intermediate BB17 (4-amino-2-chloropyrimidine-5-carbonitrile)**

Intermediate **BB17** was purchased from Bide Pharmatech.

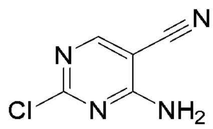

BB17

**Synthesis of general intermediate BB18 (2,4-dichloro-5-((trimethylsilyl)ethynyl)pyrimidine)**

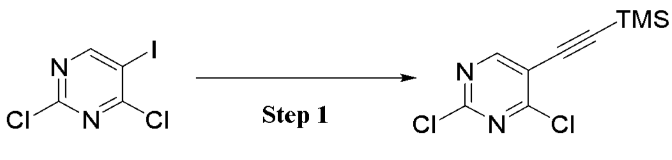


BB18-1 BB18

Compound 2,4-dichloro-5-iodopyrimidine **(BB18-1,** 1.0 g, 3.6 mmol, 1.00 eq), bis(triphenylphosphine)palladium dichloride (256 mg, 0.3 mmol, 0.1 eq), cuprous iodide (139 mg, 0.7 mmol, 0.2 eq), trimethylethynylsilane (700 mg, 7.2 mmol, 2.0 eq) and triethylamine (1.1 g, 10.9 mmol, 3.0 eq) were added to tetrahydrofuran (15 mL) successively. The reaction mixture was stirred at 40°C for 3 hours, and concentrated under vacuum to obtain a crude product, which was

purified by column chromatography (eluent: petroleum ether/ethyl acetate = 1/0 - 1/1) to obtain compound **BB18** (590 mg, 2.4 mmol) as a white solid. $^1$HNMR (400MHz, DMSO) δ 8.97 - 8.89 (m, 1H), 0.28 (s, 9H).

**Synthesis of general intermediate BB19 (2-chloro-5-(difluoromethoxy)pyrimidin-4-amine)**

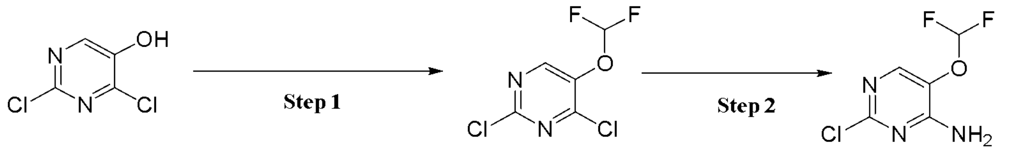


**BB19-1** **BB19-2** **BB19**

Step 1: Synthesis of compound **BB19-2** (2,4-dichloro-5-(difluoromethoxy)pyrimidine)

2,4-Dichloropyrimidine-5-ol (compound **BB19-1,** 3.40 g, 20.6 mmol, 1.00 eq) and potassium hydroxide (13.3 g, 237 mmol, 11.5 *eq*) were added to acetonitrile (60.0 mL) and water (60.0 mL) successively, and diethyl bromofluoromethylphosphonate (9.35 g, 35.0 mmol, 1.70 *eq*) was added dropwise. After the addition was completed, the resulting mixture was stirred at 25°C for 1.5 hours. Saturated aqueous citric acid solution (10.0 mL) was added to quench the reaction, and water (10.0 mL) was added. The resulting mixture was extracted three times with ethyl acetate (40 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 2/1) to obtain intermediate **BB19-2** (3.70 g, 17.2 mmol, 83.5% yield) as a light yellow oil. $^1$H NMR (400MHz, $CDCl_3$) δ 8.53 (s, 1H), 6.84 - 6.49 (t, *J* =70.8 Hz, 1H); LC-MS: m/z = 215.0 $(M+H)^+$.

Step 2: Synthesis of intermediate **BB19**

In accordance with the same steps of intermediate **BB13,** intermediate **BB19** (298.5 mg, 1.53 mmol, 65.5% yield) was obtained using compound **BB19-2** (500.0 mg, 2.33 mmol, 1.00 *eq*) as a starting material. LC-MS: m/z = 196.1 $(M+H)^+$.

**General intermediate BB21 (4-(2,4-dichloropyrimidin-5-yl)morpholine)**

Intermediate **BB21** was purchased from Bide Pharmatech.

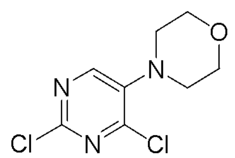

**BB21**

**Synthesis of general intermediate BB22 (2,4-dichloro-5-(4-methylpiperazin-1-yl)pyrimidine)**

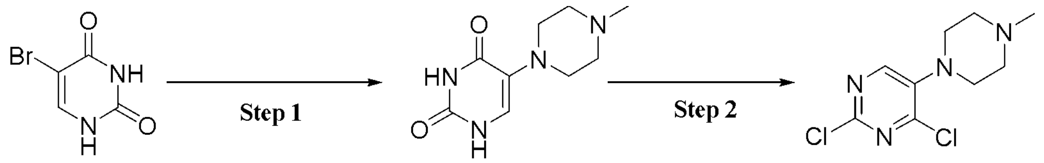


**BB22-1** **BB22-2** **BB22**

Step 1: Synthesis of compound **BB22-2** (5-(4-methylpiperazin-1-yl)pyrimidine-2,4(1H,3H)-dione)

5-Bromouracil **(BB22-1,** 5.0 g, 26.18 mmol, 1.00 eq) was dissolved in pyridine (25 mL), and N-methylpiperazine (3.9 g, 39.2 mmol, 1.5 eq) was added. The resulting mixture was heated up to 110°C and stirred for 4 hours, then cooled to room temperature. The resulting mixture was concentrated under vacuum to obtain a crude product, which was triturated with ethyl acetate (50 mL). The resulting mixture was filtered to obtain a solid, which was concentrated under vacuum to obtain compound **BB22-2** (5.1 g, 24.26 mmol, 92.7% yield) as a brown solid. LC-MS: m/z = 211.1 $(M+H)^+$.

Step 2: Synthesis of compound **BB22**

**BB22-2** (5.0 g, 23.8 mmol, 1.0 *eq*) was added to phosphorus oxychloride (200 mL), and the mixture was stirred at 90°C for 16 hours. The resulting mixture was cooled to room temperature, and concentrated under vacuum. Saturated aqueous sodium bicarbonate solution was added to adjust pH to about 7, and the resulting mixture was extracted with dichloromethane (300 mL) three times. The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain intermediate **BB22** (1.3 g, 5.26 mmol, 22.1% yield, 97.8% purity) as a yellow solid. $^1$H NMR (400 MHz, DMSO-d6) δ 8.51 (s, 1H), 3.16 - 3.05 (m, 4H), 2.50 - 2.42 (m, 4H), 2.24 (s, 3H); LC-MS: m/z =247.1 $(M+H)^+$.

**General intermediate BB24 (2,4-dichloropyrimidin-5-ol)**

Intermediate **BB24** was purchased from Bide Pharmatech.

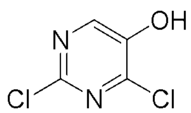

**BB24**

**General intermediate BB35 ((2,4-dichloropyrimidin-5-yl)methanol)**

Intermediate **BB35** was purchased from Bide Pharmatech.

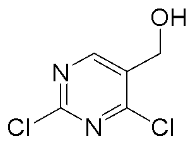

**BB35**

**Synthesis of general intermediate BB36 (2-chloro-5-(methoxymethyl)-N-methylpyrimidin-4-amine)**

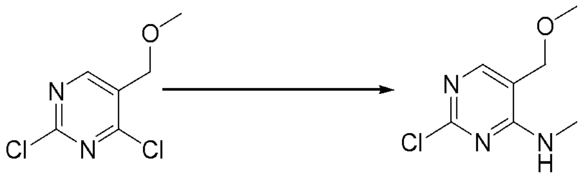

**BB36-1** **BB36**

In accordance with the same steps of intermediate **BB16,** intermediate **BB36** (1.89 g, 10.07 mmol, 67.2% yield) was obtained using 2,4-dichloro-5-(methoxymethyl)pyrimidine **(BB36-1,** 2.9 g, 15.0 mmol, 1.00 *eq*) as a starting material. LC-MS: m/z = 188.1 $(M+H)^+$.

**Synthesis of general intermediate BB41 (methyl 2-(2,4-dichloropyrimidin-5-yl)acetate)**

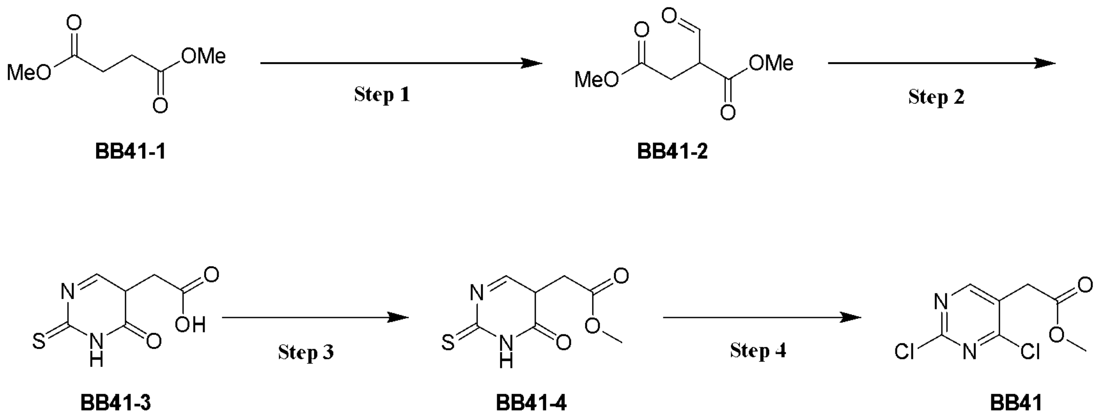


BB41-1 BB41-2 BB41-3 BB41-4 BB41

Step 1: Synthesis of compound **BB41-2** (dimethyl 2-formylsuccinate)

Dimethyl succinate (compound **BB41-1,** 200 g, 1.37 mol, 179 mL, 1.00 *eq*), ethyl formate (203 g, 2.74 mol, 220 mL, 2.00 *eq*) and sodium methoxide (259 g, 1.44 mol, 30.0% purity, 1.05 *eq*) were added to tetrahydrofuran (850 mL) successively, and the resulting mixture was stirred at 25°C for 12 hours. Then, the reaction solution was concentrated under vacuum to obtain a crude product, which was washed twice with petroleum ether (400 mL). 1 M hydrochloric acid was added to adjusted the pH to about 6, and the resulting mixture was extracted three times with methyl *tert*-butyl ether (300 mL). The organic layers were combined, dried over anhydrous sodium sulfate, and filtered to obtain a filtrate, which was concentrated under vacuum to obtain compound **BB41-2** (70.0 g, 402 mmol, 29.4% yield) as a yellow oil. $^1$H NMR (400MHz, $CDCl_3$) δ 7.69 (m, 1H), 4.03-3.58 (m, 1H), 3.57-3.56 (m, 6H), 1.17-1.10 (m, 1H), 1.07-1.03, (m, 1H).

Step 2: Synthesis of compound **BB41-3** (2-(6-oxo-2-thioxo-1,2,5,6-tetrahydropyrimidin-5-yl)acetic acid)

Compound **BB41-2** (60.0 g, 345 mmol, 1.00 eq) was dissolved in methanol (525 mL), sodium methoxide (124 g, 689 mmol, 30.0% purity, 2.00 eq) and thiourea (26.2 g, 345 mmol, 1.00 eq) were added. The resulting mixture was stirred at 100°C for 12 hours, and filtered to obtain a filter cake. 1 M aqueous hydrochloric acid solution (600 mL) was added, and the resulting mixture was stirred at 0°C for 30 minutes. The resulting mixture was filtered to obtain a filter cake, which was washed with water (100 mL) twice to obtain compound **BB41-3** (30.0 g, 161 mmol, 46.8% yield, 100% purity) as a white solid. $^1$H NMR (400MHz, $CDCl_3$) δ 12.5 (s, 1H), 12.3-12.2 (m, 2H), 7.42 (s, 1H), 3.20 (s, 2H).

Step 3: Synthesis of compound **BB41-4** (methyl 2-(6-oxo-2-thioxo-1,2,5,6-tetrahydropyrimidin-5-yl)acetate)

Compound **BB41-3** (15.0 g, 80.6 mmol, 1.00 eq) was dissolved in methanol (100 mL), and concentrated sulfuric acid (23.7 g, 242 mmol, 12.9 mL, 3.00 eq) was added. The resulting mixture was stirred at 80°C for 12 hours, and filtered to obtain a filtrate, which was concentrated under vacuum to obtain compound **BB41-4** (13.0 g, 64.9 mmol, 80.6% yield) as a white solid. $^1$H NMR (400MHz, $CDCl_3$) δ 12.5 (s, 1H), 12.3(s, 1H), 7.40-7.60 (m, 1H), 3.59 (s, 3H), 3.30 (s, 2H).

Step 4: Synthesis of intermediate **BB41**

Compound **BB41-3** (12.0 g, 59.9 mmol, 1.00 eq) was dissolved in phosphorus oxychloride (158 g, 1.03 mol, 96.0 mL, 17.2 eq), and stirred at 110°C for 12 hours under nitrogen atmosphere. The reaction solution was cooled to 20°C, and ice water (50.0 mL) was added. Saturated aqueous sodium bicarbonate solution was added to adjust the pH to about 7, and the resulting mixture was extracted three times with ethyl acetate (30 mL). The organic layers were combined, washed three times with saturated brine (35.0 mL), and filtered to obtain a filtrate. The filtrate was concentrated under vacuum to obtain a crude product, which was purified by column chromatography (eluent: petroleum ether/ethyl acetate = 1/0 - 1/1) to obtain compound **BB41** (3.60 g, 15.7 mmol, 26.2% yield, 96.4% purity) as a light yellow oil. LC-MS: m/z =220.9 $(M+H)^+$.

**Synthesis of general intermediate BB42 (2-chloro-5-methoxy-N-(prop-2-yn-1-yl)pyrimidin-4-amine)**

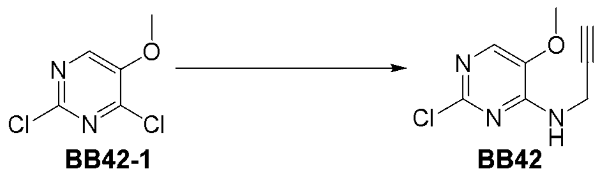

**BB42-1** **BB42**

In accordance with the same steps of intermediate **BB16,** intermediate **BB42** (140.8 mg, 0.71 mmol, 25.6% yield) was obtained using 2,4-dichloro-5-methoxypyrimidine **(BB42-1,** 500 mg, 2.79 mmol, 1.00 *eq*) and propargylamine (153.7 mg, 2.79 mmol, 1.00 *eq*) as starting materials. LC-MS: m/z = 198.2 $(M+H)^+$.

**Synthesis of general intermediate BB43 (2-chloro-5-methoxy-N-(tetrahydrofuran-3-yl)pyrimidin-4-amine)**

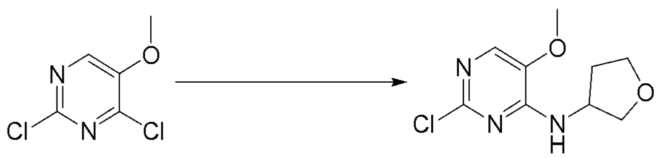

**BB42-1** **BB43**

In accordance with the same steps of intermediate **BB16,** intermediate BB43 (910.5 mg, 3.96 mmol, 59.2% yield) was obtained using 2,4-dichloro-5-methoxypyrimidine **(BB42-1,** 1.2 g, 6.7 mmol, 1.00 *eq)* and 3-aminotetrahydrofuran (584.0 mg, 6.7 mmol, 1.00 *eq)* as starting materials. LC-MS: m/z = 230.2 $(M+H)^+$.

**Synthesis of general intermediate BB44 (2-chloro-N-cyclopropyl-5-methoxypyrimidin-4-amine)**

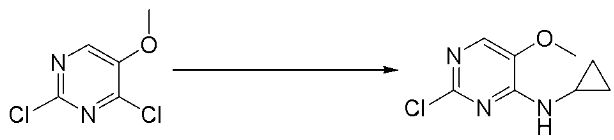

**BB42-1** **BB44**

In accordance with the same steps of intermediate **BB16,** intermediate **BB44** (556.3 mg, 2.79 mmol, 62.3% yield) was obtained using 2,4-dichloro-5-methoxypyrimidine **(BB42-1,** 0.8 g, 4.47 mmol, 1.00 *eq)* and cyclopropylamine (255.2 mg, 4.47 mmol, 1.00 *eq*) as starting materials. LC-MS: m/z = 200.1 $(M+H)^+$.

**Synthesis of general intermediate BB45 (N-(2-chloro-5-methoxypyrimidin-4-yl)-O-methylhydroxylamine)**

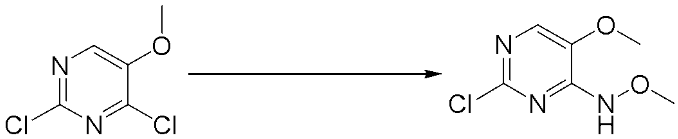

**BB45-1** **BB45**

In accordance with the same steps of intermediate **BB16,** intermediate **BB45** (490.0 mg, 2.58 mmol, 92.3% yield) was obtained using 2,4-dichloro-5-methoxypyrimidine (compound **BB45-1,** 500 mg, 2.8 mmol, 1.0 eq) and methoxyamine hydrochloride (352 mg, 4.2 mmol, 1.5 eq) as starting materials. LC-MS: m/z = 190.1 $(M+H)^+$.

**Synthesis of general intermediate C2 (2-(4-(chloromethyl)cyclohexyl)pyridine)**

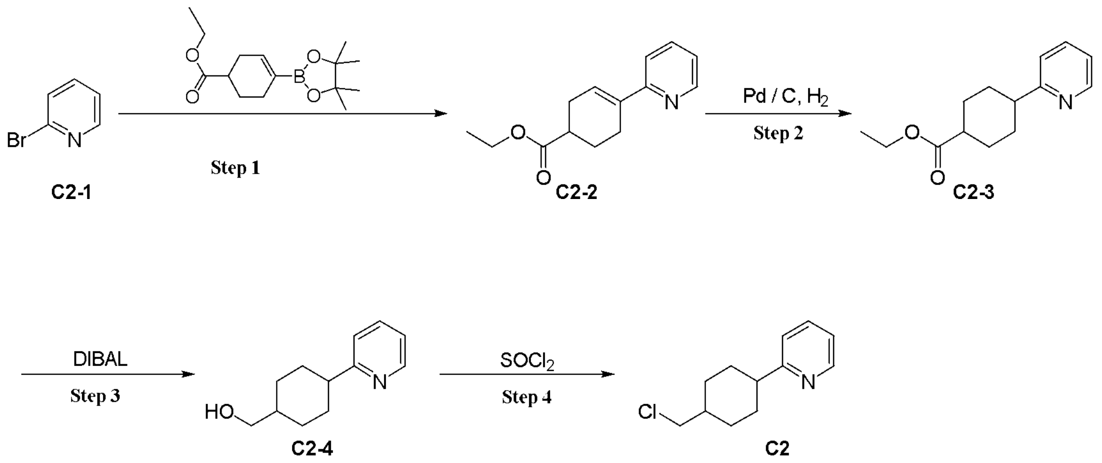


Step 1: Synthesis of compound C2-2 (ethyl 4-(pyridin-2-yl)cyclohex-3-ene-1-carboxylate)

2-Bromopyridine **(C2-1,** 15.0 g, 94.9 mmol, 9.04 mL, 1.00 eq), 1-ethoxycarbonylcyclohex-3-ene-4-pinacolyl borate (26.9 g, 94.9 mmol, 99.0% purity, 1.00 eq), potassium carbonate (39.4 g, 284 mmol, 3.00 eq) and [1,1'-bis(diphenylphosphino)ferrocene]palladium dichloride (6.95 g, 9.49 mmol, 0.10 eq) were added to water (50.0 mL) and dioxane (200 mL) successively, and the resulting mixture was stirred at 100°C for 16 hours under nitrogen atmosphere. Then, water (30.0 mL) was added, and the resulting mixture was extracted three times with ethyl acetate (100 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C2-2** (9.00 g, 38.9 mmol, 41.0% yield) as a colorless transparent oil. $^1$H NMR (400 MHz, $CDCl_3$) δ 8.54 - 8.55 (m, 1H), 7.60 - 7.64 (m, 1H), 7.37 (d, J = 8.8Hz, 1H), 7.10 - 7.13 (m, 1H), 6.66 - 6.68 (m, 1H), 4.11 - 4.20 (m, 2H), 2.62 - 2.72 (m, 5H), 2.50 - 2.53 (m, 1H), 1.79 - 1.87 (m, 1H), 1.25 - 1.29 (m, 3H); LC-MS: m/z = 232.0 $(M+H)^+$.

Step 2: Synthesis of compound **C2-3** (ethyl 4-(pyridin-2-yl)cyclohexane-1-carboxylate)

Compound **C2-2** (9.00 g, 38.9 mmol, 1.00 eq) was dissolved in methanol (288.0 mL), and palladium on carbon (4.14 g, 3.89 mmol, 10% purity, 0.10 eq) was added under nitrogen atmosphere. The resulting mixture was purged with hydrogen three times, and stirred at 25°C for 16 hours under hydrogen atmosphere (15 Psi). The reaction mixture was filtered to obtain a filtrate, which was concentrated under vacuum to obtain compound **C2-3** (8.13 g, 34.9 mmol, 89.6% yield) as a colorless transparent oil. $^1$H NMR (400 MHz, $CDCl_3$) δ 8.51 - 8.54 (m, 1H), 7.58 - 7.62 (m, 1H), 7.09 - 7.14 (m, 2H), 4.14 - 4.20 (m, 2H), 2.67 - 2.78 (m, 1H), 2.22 - 2.25 (m, 3H), 1.62 - 1.87 (m, 6H), 1.26 - 1.29 (m, 3H); LC-MS: m/z = 234.0 $(M+H)^+$.

Step 3: Synthesis of compound **C2-4** ((4-(pyridin-2-yl)cyclohexyl)methanol)

Compound **C2-3** (8.13 g, 34.8 mmol, 1.00 eq) was dissolved in tetrahydrofuran (300.0 mL), and diisobutylaluminum hydride (1 M, 112 mL, 3.20 eq) was added dropwise at -70°C. After the addition was completed, the resulting mixture was stirred at -70°C for 30 minutes, heated up to 25°C and stirred for 1 hour. Water (100 mL) was added at 0°C to quench the reaction, and the resulting mixture was extracted three times with ethyl acetate (300 mL). The organic layers were combined, washed twice with saturated brine (200 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C2-4** (4.80 g, 25.1 mmol, 72.0% yield) as a colorless transparent oil. $^1$H NMR (400 MHz, $CDCl_3$) δ 8.54 - 8.55 (m, 1H), 7.59 - 7.63 (m, 1H), 7.10 - 7.20 (m, 2H), 3.52 - 3.71 (m, 2H), 2.80 - 2.85(m, 1H), 1.67 - 1.84 (m, 8H), 1.15 - 1.23 (m, 1H); LC-MS: m/z = 192.0 $(M+H)^+$.

Step 4: Synthesis of compound **C2**

Compound **C2-4** (800 mg, 4.18 mmol, 1.00 eq) was dissolved in dichloromethane (16.0 mL), and thionyl chloride (2.49 g, 20.9 mmol, 1.52 mL, 5.00 eq) was added at 0°C. After the addition was completed, the resulting mixture was heated up to 20°C and stirred for 12 hours. The reaction solution was concentrated under vacuum to obtain intermediate **C2** (900 mg, crude) as a white solid. LC-MS: m/z = 210.0 $(M+H)^+$.

**Synthesis of general intermediate C3 (2-(4-(chloromethyl)phenyl)pyridine)**

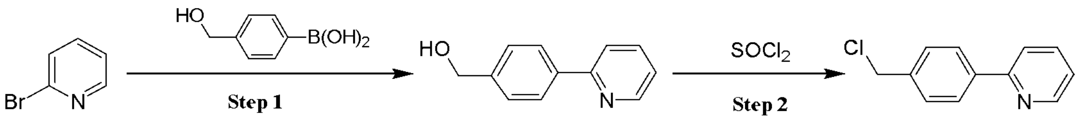


C3-1 C3-2 C3

Step 1: Synthesis of compound **C3-2** ((4-(pyridin-2-yl)phenyl)methanol)

Compound 2-bromopyridine **(C3-1,** 10.0 g, 63.3 mmol, 6.02 mL, 1.00 eq), 2-(4-hydroxymethylphenyl)pyridine (12.5 g, 82.3 mmol, 1.30 eq), tetrakis(triphenylphosphine)palladium (7.31 g, 6.33 mmol, 0.100 eq), and sodium carbonate (49.6 g, 468 mmol, 7.40 eq) were added to a mixed solvent of toluene (50.0 mL), water (50.0 mL) and ethanol (10.0 mL) successively. The resulting mixture was purged three times with nitrogen, and stirred at 100°C for 12 hours under nitrogen atmosphere. The reaction solution was filtered to obtain a filtrate. The filtrate was concentrated under vacuum to obtain a crude product, which was purified by reverse phase HPLC (0.1% $NH_3$•$H_2O$) to obtain compound **C3-2** (9.63 g) as a light yellow oil. $^1H$ NMR (400 MHz, $CDCl_3$) δ 8.67 (d, *J*=4.4Hz, 1H), 7.75 - 7.95 (m, 2H), 7.70 - 7.74 (m, 2H), 7.42 - 7.44 (m, 2H), 7.22 - 7.25 (m, 1H), 4.73 (s, 2H), 2.70 (s, 1H); LC-MS: m/z = 186.2 $(M+H)^+$.

Step 2: Synthesis of intermediate **C3**

Compound **C3-2** (1.0 g, 5.40 mmol, 1.00 eq) was dissolved in dichloromethane (10.0 mL), and thionyl chloride (3.2 g, 27.0 mmol, 1.96 mL, 5.00 eq) was added at 0°C. After the addition was completed, the resulting mixture was heated up to 20°C and stirred for 12 hours. The reaction solution was concentrated under vacuum to obtain intermediate C3 (1.1 g, crude) as a white solid. LC-MS: m/z = 204.1 $(M+H)^+$.

**Synthesis of general intermediate C4 (4-(chloromethyl)-1-(1-methyl-4-(trifluoromethyl)-1H-imidazol-2-yl)piperidine)**

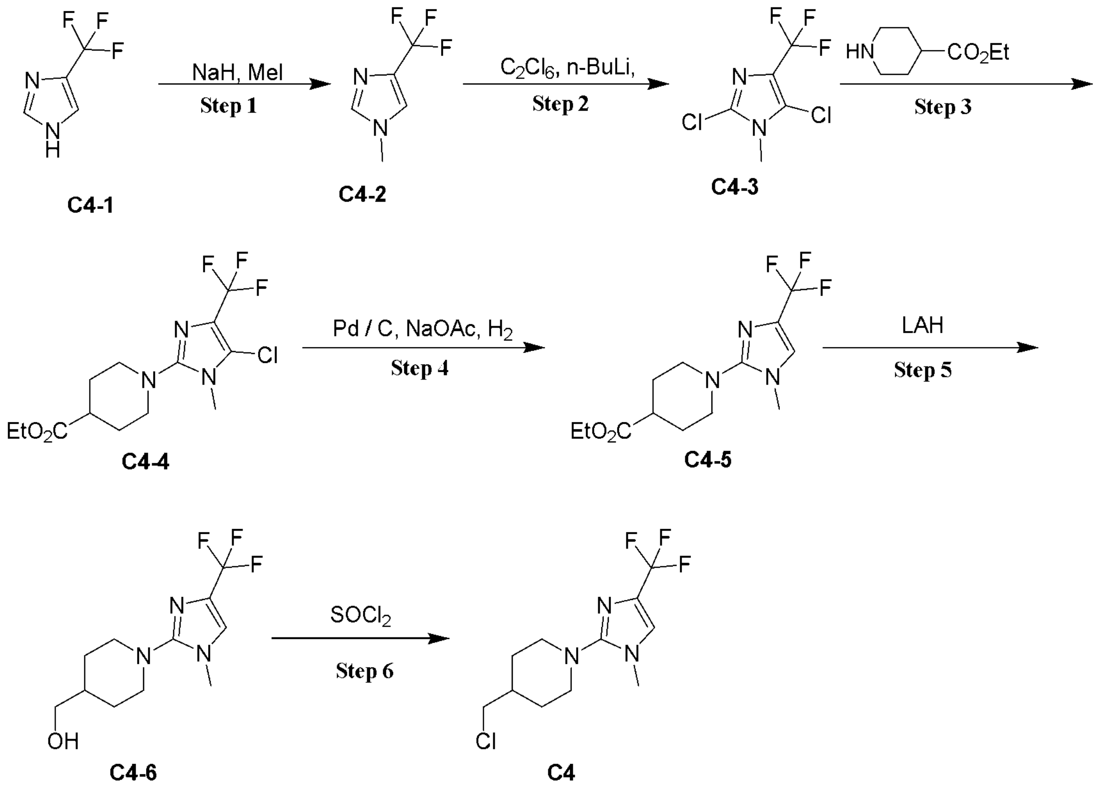


C4-1 C4-2 C4-3 C4-4 C4-5 C4-6 C4

Step 1: Synthesis of compound **C4-2** (1-methyl-4-(trifluoromethyl)-1H-imidazole)

Compound 4-(trifluoromethyl)-1H-imidazole (compound **C4-1,** 33.0 g, 242 mmol, 1.00 eq) was dissolved in tetrahydrofuran (330.0 mL), and sodium hydride (9.70 g, 242 mmol, 60.0% purity, 1.00 eq) was added in portions at 0°C. After the addition was completed, the resulting mixture was reacted at 0°C for 30 minutes. Then, iodomethane (34.4 g, 242 mmol, 15.1 mL, 1.00 eq) was added. After the addition was completed, the resulting mixture was heated up to 25°C and stirred for 4 hours. Then, ice water (200.0 mL) was added at 0°C to quench the reaction, and the resulting mixture was extracted three times with ethyl acetate (200.0 mL). The organic layers were combined, dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain compound **C4-2** (21.0 g). $^1$H NMR (400 MHz, $CDCl_3$) δ 7.48 (s, 1H), 7.24 (s, 1H), 3.75 (s, 3H); LC-MS: m/z = 151.1 $(M+H)^+$.

Step 2: Synthesis of compound **C4-3** (2,5-dichloro-1-methyl-4-(trifluoromethyl)-1H-imidazole)

Compound **C4-2** (16.8 g, 111 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **C4-3** (12.0 g). LC-MS: m/z = 219.0 $(M+H)^+$.

Step 3: Synthesis of compound **C4-4** (ethyl 1-(5-chloro-1-methyl-4-(trifluoromethyl)-1H-imidazol-2-yl)piperidine-4-carboxylate)

Compound **C4-3** (7.0 g, 31.9 mmol, 1.00 eq), ethyl 4-piperidinylcarboxylate (7.54 g, 47.9 mmol, 7.39 mL, 1.50 eq), N,N-diisopropylethylamine (16.5 g, 127 mmol, 22.2 mL, 4.00 eq) and sodium iodide (479 mg, 3.20 mmol, 0.100 eq) were added to N,N-dimethylformamide (70.0 mL) successively, and the resulting mixture was reacted at 130°C for 72 hours. Water (350 mL) was added, the resulting mixture was stirred for 5 minutes, and extracted three times with ethyl

acetate (100 mL). The organic layers were combined, dried over anhydrous sodium sulfate, and filtered to obtain a filtrate. The filtrate was concentrated under vacuum to obtain a crude product, which was purified by reverse phase HPLC (0.1% FA) to obtain compound **C4-4** (2.2 g). $^1H$ NMR (400 MHz, $CDCl_3$) δ 4.17 (q, *J* = 7.2Hz, 2H), 3.45 (s, 3H), 3.24 - 3.28 (m, 2H), 2.90 - 2.97 (m, 2H), 2.40 - 2.45 (m, 1H), 2.02 - 2.06 (m, 2H), 1.85 - 1.88 (m, 2H), 1.28 (t, *J* = 8.0Hz, 2H); LC-MS: m/z = 340.1 $(M+H)^+$.

Step 4: Synthesis of compound **C4-5** (ethyl 1-(1-methyl-4-(trifluoromethyl)-1H-imidazol-2-yl)piperidine-4-carboxylate)

Compound **C4-4** (2.2 g, 6.48 mmol, 1.00 eq), sodium acetate (1.06 g, 12.9 mmol, 2.00 eq) and wet palladium on carbon (0.5 g, 1.94 mmol, 10.0% purity, 0.300 eq) were added to ethanol (40.0 mL) successively, and the resulting mixture was reacted at 50°C under hydrogen atmosphere (15 psi) for 36 hours. The reaction solution was filtered to obtain a filtrate, which was concentrated under vacuum to obtain compound **C4-5** (1.9 g). $^1H$ NMR (400 MHz, $CDCl_3$) δ 7.02 (s, 1H), 4.15 (q, *J* = 7.2Hz, 2H), 3.52 (s, 3H), 3.25 - 3.29 (m, 2H), 2.92 - 2.98 (m, 2H), 2.40 - 2.45 (m, 1H), 2.01 - 2.05 (m, 2H), 1.85 - 1.89 (m, 2H), 1.28 (t, *J* = 6.8Hz, 3H); LC-MS: m/z = 306.1 $(M+H)^+$.

Step 5: Synthesis of compound **C4-6** ((1-(1-methyl-4-(trifluoromethyl)-1H-imidazol-2-yl)piperidin-4-yl)methanol)

Compound **C4-5** (1.2 g, 3.93 mmol, 1.00 eq) was dissolved in tetrahydrofuran (20.0 mL), and lithium aluminum hydride (447 mg, 11.8 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain compound **C4-6** (1.0 g, crude), which was directly used in the next step. $^1H$ NMR (400 MHz, $CDCl_3$) δ 7.01 (s, 1H), 3.57 (d, *J* = 6.4Hz, 2H), 3.51 (s, 3H), 3.27 - 3.30 (m, 2H), 2.90 - 2.96 (m, 2H), 1.84 - 1.87 (m, 2H), 1.65 - 1.70 (m, 1H), 1.39 - 1.42(m, 2H); LC-MS: m/z = 264.1 $(M+H)^+$.

Step 6: Synthesis of intermediate **C4**

Compound **C4-6** (1.0 g, 3.80 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain intermediate **C4** (1.1 g, 3.46 mmol, 91.0% yield, HCl) as a brown solid. LC-MS: m/z = 282.1 $(M+H)^+$.

**Synthesis of general intermediate C5 (2-(4-(chloromethyl)phenyl)-1-methyl-4-(trifluoromethyl)-1H-imidazole)**

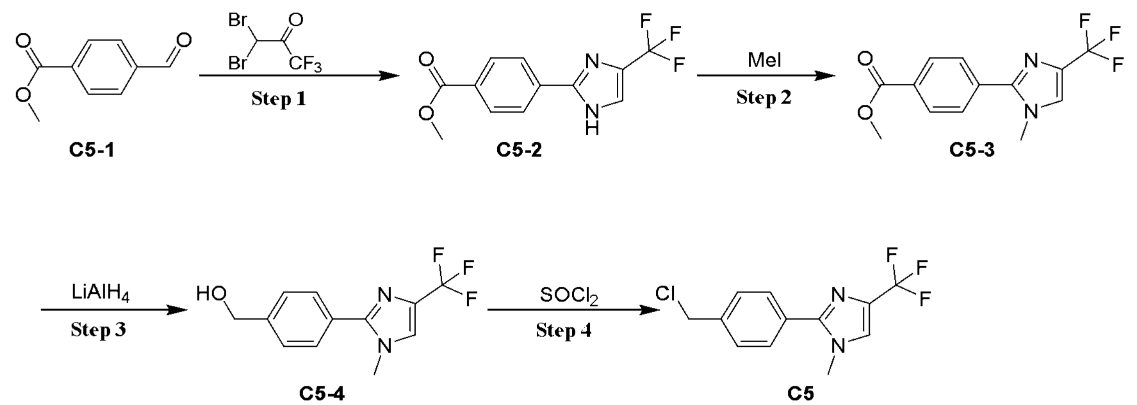


C5-1 C5-2 C5-3 C5-4 C5

Step 1: Synthesis of compound C5-2 (methyl 4-(4-(trifluoromethyl)-1H-imidazol-2-yl)benzoate)

1,1-Dibromo-3,3-trifluoroacetone (7.2 g, 26.8 mmol, 1.10 eq) and sodium acetate (2.2 g, 27.3 mmol, 1.12 eq) were dissolved in water (8.0 mL), and the resulting mixture was stirred at 100°C for 1 hour. Compound methyl *p*-formylbenzoate **(C5-1,** 4.0 g, 24.4 mmol, 1.00 eq) dissolved in methanol (80.0 mL) and ammonia water (22.0 mL) was added at 25°C. After the addition was completed, the resulting mixture was reacted at 25°C for 40 minutes, heated up to

100°C and stirred for 2 hours. Then, water (60.0 mL) was added to quench the reaction, and the resulting mixture was extracted three times with ethyl acetate (80.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **C5-2** (4.5 g). $^1$H NMR (400 MHz, $CDCl_3$) δ 13.4 (s, 1H), 8.10 - 8.12 (m, 2H), 8.04 - 8.06 (m, 2H), 7.99 (s, 1H), 3.87 (s, 3H); LC-MS: m/z = 271.0 $(M+H)^+$.

Step 2: Synthesis of compound **C5-3** (methyl 4-(1-methyl-4-(trifluoromethyl)-1H-imidazol-2-yl)benzoate)

Compound **C5-2** (2.5 g, 9.3 mmol, 1.00 eq) was dissolved in tetrahydrofuran (20.0 mL), and sodium hydride (444.1 mg, 11.1 mmol, 1.20 eq) was added in portions at 0°C. After the addition was completed, the resulting mixture was reacted at 0°C for 30 minutes. Then, iodomethane (6.8 g, 48.1 mmol, 5.20 eq) was added. After the addition was completed, the resulting mixture was heated up to 20°C and stirred for 15 hours. Then, ice water (20.0 mL) was added at 0°C to quench the reaction, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **C5-3** (1.1 g). $^1$H NMR (400 MHz, $CDCl_3$) δ 8.07 (d, *J* = 8.4Hz, 2H), 7.99 (s, 1H), 7.90 (d, *J* = 8.4Hz, 2H), 3.89 (s, 3H), 3.84 (s, 3H); LC-MS: m/z =285.1 $(M+H)^+$.

Step 3: Synthesis of compound **C5-4** ((4-(1-methyl-4-(trifluoromethyl)-1H-imidazol-2-yl)phenyl)methanol)

Compound **C5-3** (0.6 g, 2.1 mmol, 1.00 eq) was dissolved in tetrahydrofuran (6.0 mL), and lithium aluminum hydride (88.1 mg, 2.32 mmol, 1.10 eq) was added at 0°C. After the addition was completed, the resulting mixture was heated up to 25°C and stirred for 4 hours. Water (1.6 mL) and aqueous sodium hydroxide solution (1 M, 0.4 mL) were added at 0°C, and the resulting mixture was stirred at 0°C for 0.5 hours. Tetrahydrofuran (10.0 mL) and anhydrous sodium sulfate (1.0 g) were added, and the resulting mixture was filtered to obtain a filtrate. The filtrate was concentrated under vacuum to obtain a crude product **C5-4** (505.4 mg), which was directly used in the next step. $^1$H NMR (400 MHz, $CDCl_3$) δ 7.92 (s, 1H), 7.67 (d, *J* = 8.4 Hz, 2H), 7.45 (d, *J* = 8.0 Hz, 2H), 5.30 - 5.33 (m, 1H), 4.58 (d, *J* = 5.6 Hz, 2H), 3.78 (s, 3H); LC-MS: m/z = 257.1 (M+H)+.

Step 4: Synthesis of intermediate **C5**

Compound **C5-4** (500.0 mg, 2.0 mmol, 1.00 eq) was dissolved in dichloromethane (5.0 mL), and thionyl chloride (1.9 g, 15.6 mmol, 8.00 eq) was added at 0°C. After the addition was completed, the resulting mixture was heated up to 20°C and stirred for 15 hours. The reaction solution was concentrated under vacuum to obtain compound **C5** (523 mg, 1.90 mmol, 97.6% yield) as a brown solid. $^1$H NMR (400 MHz, DMSO-d6) δ 7.94 (d, J = 1.4 Hz, 1H), 7.74 (d, J = 8.2 Hz, 2H), 7.58 (d, J = 8.1 Hz, 2H), 4.84 (s, 2H), 3.80 (s, 3H); LC-MS: m/z = 275.1 $(M+H)^+$.

**Synthesis of general intermediate C6 (2-(4-(chloromethyl)piperidin-1-yl)pyridine)**

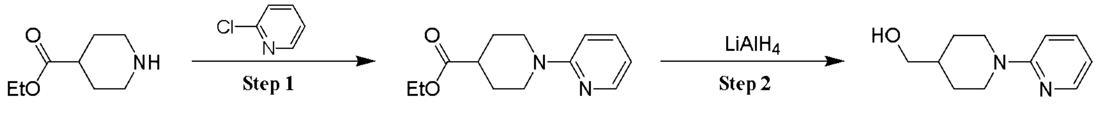


**C6-1** **C6-2** **C6-3**

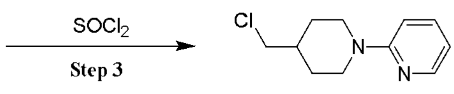


**C6**

Step 1: Synthesis of compound **C6-2** (ethyl 1-(pyridin-2-yl)piperidine-4-carboxylate)

Ethyl piperidine-4-carboxylate (compound **C6-1,** 20.8 g, 132 mmol, 20.4 mL, 1.00 eq), 2-chloropyridine (15.0 g,

132.0 mmol, 1.00 eq) and triethylamine (26.7 g, 264 mmol, 36.8 mL, 2.00 eq) were dissolved in dimethyl sulfoxide (50.0 mL), and the resulting mixture was reacted at 130°C for 16 hours. The reaction was cooled down and stopped. Water (50 ml) was added at room temperature to quench the reaction, and the resulting mixture was extracted three times with ethyl acetate (60.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by silica gel column chromatography (eluent: petroleum ether/ethyl acetate = 1/0 - 1/1) to obtain compound **C6-2** (6.0 g, yield 19.4%, purity 100%) as a yellow oil. $^1$H NMR (400 MHz, $CDCl_3$) δ 8.17 - 8.19 (m, 1H), 7.44 - 7.48 (m, 1H), 6.66 (d, J=8.4Hz, 1H), 6.59 - 6.61 (m, 1H), 4.21 - 4.24 (m, 2H), 4.13 - 4.18 (m, 2H), 2.92 - 2.99 (m, 2H), 2.52 - 2.53 (m, 1H), 1.98 - 2.02 (m, 2H), 1.75 - 1.79 (m, 2H), 1.25 - 1.28 (m, 3H). LC-MS: m/z = 235.2$(M+H)^+$.

Step 2: Synthesis of compound **C6-3** ((1-(pyridin-2-yl)piperidin-4-yl)methanol)

Compound **C6-2** (6.00 g, 25.6 mmol) was dissolved in THF (60.0 mL), and lithium aluminum hydride (1.07 g, 28.2 mmol, 1.10 eq) was added in portions at 0°C. The resulting mixture was heated up to room temperature, and stirred for 16 hours. Additional lithium aluminum hydride (486 mg, 12.8 mmol, 0.500 eq) was added, and the resulting mixture was further stirred for 6 hours. Water (20.0 mL) was added at 0°C to quench the reaction, and the resulting mixture was extracted three times with ethyl acetate (60.0 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by silica gel column chromatography (eluent: petroleum ether/ethyl acetate = 1/0 - 1/1) to obtain compound **C6-3** (1.20 g, yield 23.8%, purity 97.8%) as a yellow oil. $^1$H NMR (400 MHz, $CDCl_3$) δ 8.18 (d, J=3.6Hz, 1H), 7.43 - 7.48 (m, 1H), 6.67 (d, J=8.8Hz, 1H), 6.58 - 6.59 (m, 1H),4.33 (d, J=12.8Hz, 2H), 3.54 (d, J=6.0Hz, 2H), 2.81 - 2.88 (m, 2H), 1.77 - 1.86 (m, 2H), 1.73 - 1.76 (m, 1H), 1.55 (s, 1H), 1.28 - 1.35 (m, 2H). LC-MS: m/z =193.2$(M+H)^+$.

Step 3: Synthesis of intermediate **C6**

Compound **C6-3** (1.20 g, 6.24 mmol, 1.00 eq) was dissolved in dichloromethane (12.0 mL), and thionyl chloride (3.71 g, 31.2 mmol, 2.26 mL, 5.00 eq) was added at 0°C. After the addition was completed, the resulting mixture was heated up to 20°C and stirred for 12 hours. The reaction solution was concentrated under vacuum to obtain intermediate **C6** (1.57 g, crude) as a white solid. LC-MS: m/z = 211.1.

**Synthesis of general intermediate C7 (2-(5-(chloromethyl)thiophen-2-yl)-1-methyl-4-(trifluoromethyl)-1H-imidazole)**

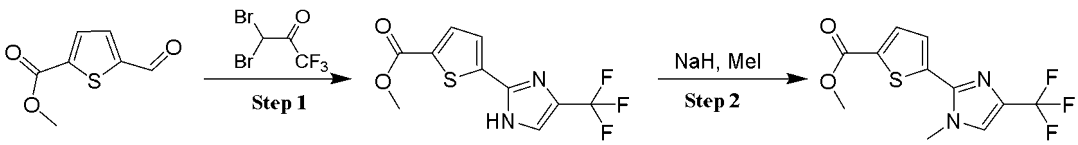


C7-1 C7-2 C7-3

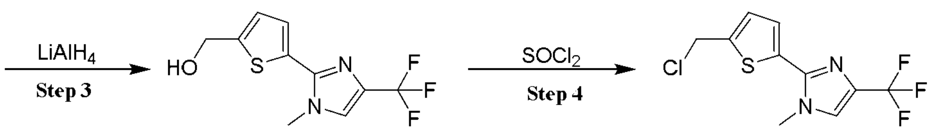


C7-4 C7

In accordance with the synthesis of intermediate **C5,** general intermediate **C7** was obtained by replacing compound **C5-1** with an equimolar amount of methyl 5-formylthiophene-2-carboxylate (compound **C7-1),** while other experimental conditions remained unchanged. Intermediate **C7** (330.0 mg, crude) was finally obtained as a white solid. LC-MS: m/z = 281.0 $(M+H)^+$.

**Synthesis of general intermediate C9 (2-((1R,4R)-4-(chloromethyl)cyclohexyl)-1-methyl-4-(trifluoromethyl)-1H-imidazole)**

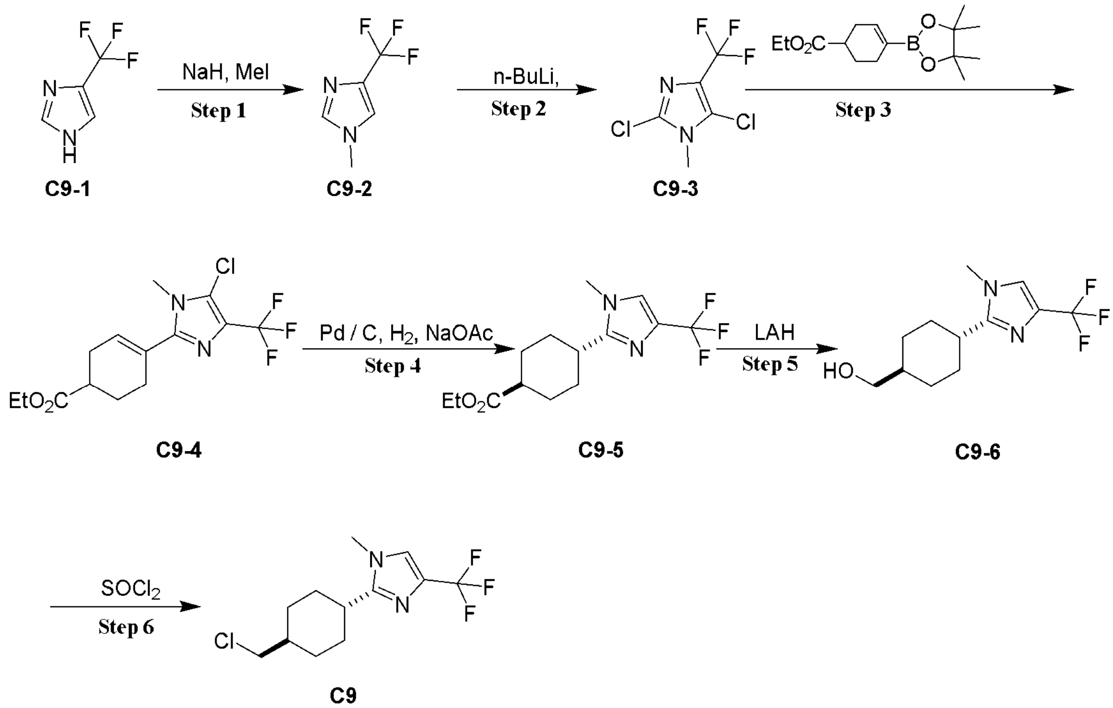


C9-1 C9-2 C9-3 C9-4 C9-5 C9-6 C9

Step 1: Synthesis of compound **C9-2** (1-methyl-4-(trifluoromethyl)-1H-imidazole)

4-(Trifluoromethyl)-1H-imidazole (compound **C9-1,** 33.0 g, 242.0 mmol, 1.00 eq) was dissolved in tetrahydrofuran (330.0 mL), and sodium hydride (9.7 g, 242.0 mmol, 60.0% purity, 1.00 eq) was added in portions at 0°C. After the addition was completed, the resulting mixture was reacted at 0°C for 30 minutes. Then, iodomethane (34.4 g, 242.0 mmol, 15.1 mL, 1.00 eq) was added. After the addition was completed, the resulting mixture was heated up to 25°C and stirred for 5 hours. Saturated aqueous ammonium chloride solution (30.0 mL) was added at 0°C to quench the reaction. Water (30.0 mL) was added, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by reverse phase HPLC (0.1% $NH_3$•$H_2O$) to obtain compound **C9-2** (21.0 g). $^1$H NMR (400 MHz, $CDCl_3$) δ 7.48 (s, 1H), 7.24 (s, 1H), 3.75 (s, 3H); LC-MS: m/z = 151.1 $(M+H)^+$.

Step 2: Synthesis of compound **C9-3** (2,5-dichloro-1-methyl-4-(trifluoromethyl)-1H-imidazole)

Compound **C9-2** (16.8 g, 111.0 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **C9-3** (12.0 g). LC-MS: m/z = 219.0 $(M+H)^+$.

Step 3: Synthesis of compound **C9-4** (ethyl 4-(5-chloro-1-methyl-4-(trifluoromethyl)-1H-imidazol-2-yl)cyclohex-3-ene-1-carboxylate)

Compound **C9-3** (7.0 g, 31.9 mmol, 1.00 eq), 1-ethoxycarbonylcyclohex-3-ene-4-pinacolyl borate (8.94 g, 31.9 mmol, 1.00 eq), potassium phosphate (20.3 g, 95.8 mmol, 3.00 eq), chloro(2-dicyclohexylphosphino-2',4',6'-triisopropyl-1,1'-biphenyl)[2-(2'-amino-1,1'-biphenyl)]palladium(II) (1.3 g, 1.6 mmol, 0.05 eq) and 2-dicyclohexylphosphino-2',4',6'-triisopropylbiphenyl (5.9 g, 12.4 mmol, 0.39 eq) were added to a mixed solvent of dioxane (70.0 mL) and water (10.0 mL) successively. The resulting mixture was purged three times with nitrogen, and stirred at 100°C for 5 hours under nitrogen atmosphere. The reaction solution was filtered to obtain a filtrate, which was concentrated under vacuum to obtain a crude product. The crude product was purified by reverse phase HPLC (0.1% FA) to obtain compound **C9-4** (1.9 g). $^1$H NMR (400 MHz, $CDCl_3$) δ 6.05 (d, *J* = 2.0 Hz, 1H), 4.18 (q, *J* = 7.2 Hz, 2H).3.61 (s, 3H), 2.50 - 2.67 (m, 5H), 2.13 - 2.14 (m, 1H), 1.86 - 1.89 (m, 1H), 1.28 (t, *J* = 7.2 Hz, 3H); LC-MS: m/z = 337.2 $(M+H)^+$.

Step 4: Synthesis of compound **C9-5** (ethyl (1R,4R)-4-(1-methyl-4-(trifluoromethyl)-1H-imidazol-2-yl)cyclohexane-1-carboxylate)

Compound **C9-4** (1.9 g, 5.6 mmol, 1.00 eq), sodium acetate (925.0 mg, 11.2 mmol, 2.00 eq) and wet palladium on carbon (0.5 g, 1.4 mmol, 10.0% purity, 0.25 eq) were added to ethanol (30.0 mL) successively, and the resulting mixture was reacted at 50°C under hydrogen atmosphere (15 psi) for 5 hours. The reaction solution was filtered to obtain a filtrate, which was concentrated under vacuum to obtain a crude product. The crude product was purified by preparative HPLC (column: water Xbridge C18 150×50mm×10μm; mobile phase: [water ($NH_4HCO_3$)-ACN]; B%: 34%-64%, 10 min) to obtain compound **C9-5** (0.3 g). $^1$H NMR (400 MHz, $CDCl_3$) δ 7.13 (s, 1H), 4.15(q, *J* = 6.8 Hz, 2H), 3.64(s, 3H), 2.60 - 2.65 (m, 1H), 2.41 - 2.44 (m, 1H), 2.12 - 2.15 (m, 2H), 1.97 - 1.98 (m, 2H), 1.77 - 1.80 (m, 2H), 1.54 - 1.59 (m, 2H), 1.27 (t, *J* = 8.8 Hz, 3H); LC-MS: m/z = 305.0 $(M+H)^+$.

Step 5: Synthesis of compound **C9-6** (((1R,4R)-4-(1-methyl-4-(trifluoromethyl)-1H-imidazol-2-yl)cyclohexyl)methanol)

Compound **C9-5** (0.6 g, 2.0 mmol, 1.00 eq) was dissolved in tetrahydrofuran (10.0 mL), and lithium aluminum hydride (224.0 mg, 5.9 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **C9-6** (0.5 g), which was directly used in the next step. $^1$H NMR (400 MHz, $CDCl_3$) δ 7.12 (s, 1H), 3.63 (s, 3H), 3.52 (d, *J* = 5.6 Hz, 2H), 2.59 - 2.65 (m, 1H), 1.95 - 1.98 (m, 4H), 1.77 - 1.80 (m, 3H), 1.07 - 1.17 (m, 2H); LC-MS: m/z = 263.1 (M+H)+.

Step 6: Synthesis of intermediate **C9**

Compound **C9-6** (500.0 mg, 1.9 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain intermediate C9 (550 mg, 1.36 mmol, 71.5% yield) as a light yellow solid. LC-MS: m/z = 281.0 $(M+H)^+$.

**Synthesis of general intermediate C11 (1-(4-(chloromethyl)phenyl)-5-methyl-3-(trifluoromethyl)-1H-pyrazole)**

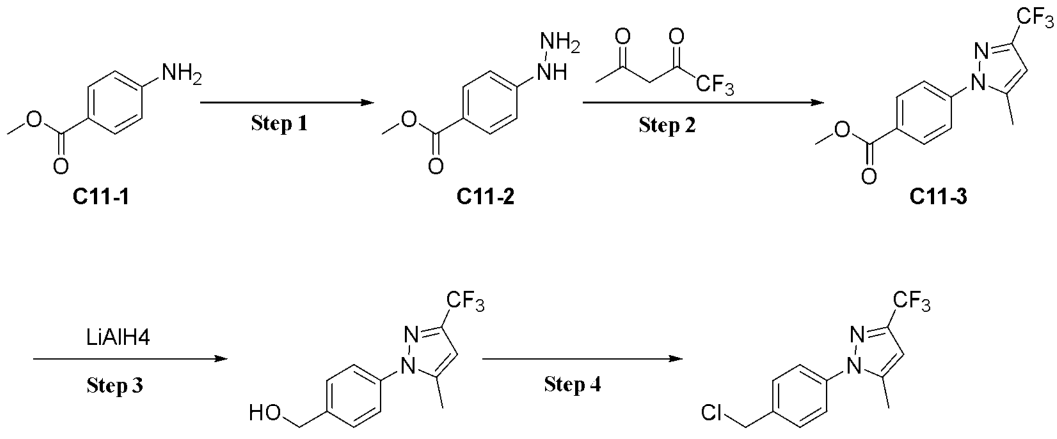


C11-1 C11-2 C11-3

C11-4 C11

Step 1: Synthesis of compound **C11-2** (methyl 4-hydrazinylbenzoate)

Compound **C11-1** (15.0 g, 90.32 mmol, 1.0 eq) was dissolved in 37% aqueous hydrochloric acid solution (25.0 mL), and sodium nitrite (6.2 g, 90.32 mmol, 1.0 eq) dissolved in water (10.0 mL) was added at 0°C. After the addition was completed, the resulting mixture was stirred for 10 minutes. Then, tin chloride (85.6 g, 451.62 mmol, 5.0 eq) dissolved in 37% aqueous hydrochloric acid solution (75.0 mL) was added dropwise at 0°C. After the addition was completed, the resulting mixture was heated up to 25°C and stirred for 2 hours. The reaction mixture was filtered to obtain a filter cake, which was washed three times with ethyl acetate (50.0 mL), and concentrated under vacuum to obtain compound **C11-2** (16.5 g). LC-MS: m/z = 167.1 $(M+H)^+$.

Step 2: Synthesis of compound **C11-3** (methyl 4-(5-methyl-3-(trifluoromethyl)-1H-pyrazol-1-yl)benzoate)

Compound **C11-2** (5 g, 24.75 mmol, 1.0 eq) and 1,1,1-trifluoro-2,4-pentanedione (3.8 g, 24.75 mmol, 1.0 eq) were added to hexafluoroisopropanol (25.0 mL), and triethylamine (5.0 g, 49.50 mmol, 2.0 *eq*) dissolved in hexafluoroisopropanol (25.0 mL) was added dropwise at 0°C. After the addition was completed, the resulting mixture was heated up to 25°C and stirred for 1 hour. Then, water (10 mL) was added, and the resulting mixture was extracted three times with dichloromethane (200.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C11-3** (5.3 g). LC-MS: m/z = 285.0 $(M+H)^+$.

Step 3: Synthesis of compound **C11-4** ((4-(5-methyl-3-(trifluoromethyl)-1H-pyrazol-1-yl)phenyl)methanol)

Compound **C11-3** (5.3 g, 18.65 mmol, 1.0 *eq*) was dissolved in tetrahydrofuran (100.0 mL), and lithium aluminum hydride (1.9 g, 46.64 mmol, 2.5 *eq*) was slowly added at 0°C. After the addition was completed, the resulting mixture was heated up to 20°C and stirred for 6 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **C11-4** (3.6 g). LC-MS: m/z = 257.1 $(M+H)^+$.

Step 4: Synthesis of intermediate **C11**

Compound **C11-4** (1.0 g, 3.91 mmol, 1.0 *eq*) was dissolved in dichloroethane (25.0 mL), and thionyl chloride (1.4 g, 11.73 mmol, 5.2 *eq*) was added at 0°C. After the addition was completed, the resulting mixture was heated up to 50°C and stirred for 2 hours. The reaction solution was concentrated under vacuum to obtain intermediate **C11** (1.0 g, 3.64 mmol, 93.1% yield, 96.9% purity) as a white solid. $^1H$ NMR (400 MHz, $CDCl_3$) δ 7.53 (d, *J* = 8.7Hz, 2H), 7.46 (d, *J* = 8.7Hz, 2H), 6.47 (s, 1H), 4.64 (s, 2H), 2.37 (s, 3H); LC-MS: m/z = 275.1 $(M+H)^+$.

**Synthesis of general intermediate C12 (8-(4-(chloromethyl)phenyl)imidazo[1,2-a]pyrazine)**

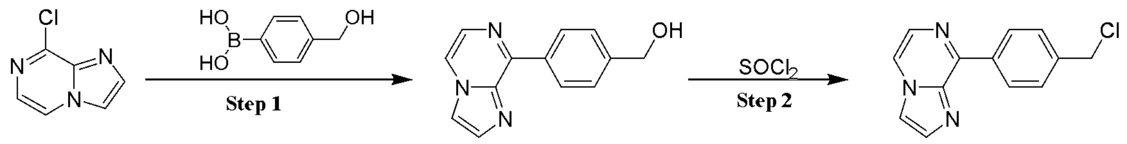


C12-1 C12-2 C12

Step 1: Synthesis of compound **C12-2** ((4-(imidazo[1,2-a]pyrazin-8-yl)phenyl)methanol)

8-Chloroimidazo[1,2-A]pyrazine (compound **C12-1,** 9.6 g, 62.5 mmol, 1.00 *eq*), 4-hydroxymethylphenylboronic acid (12.4 g, 81.3 mmol, 1.30 *eq*), tetrakis(triphenylphosphine)palladium (7.2 g, 6.25 mmol, 0.10 *eq*), and sodium carbonate (49.0 g, 463 mmol, 7.40 *eq*) were added to a mixed solvent of toluene (48.0 mL), water (48.0 mL) and ethanol (9.6 mL) successively. The resulting mixture was purged three times with nitrogen, and stirred at 100°C for 12 hours under nitrogen atmosphere. The reaction mixture was filtered to obtain a filtrate, which was concentrated under vacuum to obtain a crude product. The crude product was purified by reverse phase HPLC (0.1% $NH_3$•$H_2O$) to obtain compound **C12-2** (12.7 g). LC-MS: m/z = 226.2 $(M+H)^+$.

Step 2: Synthesis of intermediate **C12**

Compound **C12-2** (6.0 g, 26.6 mmol, 1.00 *eq*) was dissolved in dichloromethane (10.0 mL), and thionyl chloride (15.9 g, 133 mmol, 5.00 *eq*) was added at 0°C. After the addition was completed, the resulting mixture was heated up to 20°C and stirred for 12 hours. The reaction solution was concentrated under vacuum to obtain intermediate **C12** (7.3 g, 26.2 mmol, 98.2% yield, HCl) as an off-white solid. LC-MS: m/z = 244.1 (M+H)+.

**Synthesis of general intermediate C13 (2-(4-(chloromethyl)phenyl)-5-(trifluoromethyl)pyridine)**

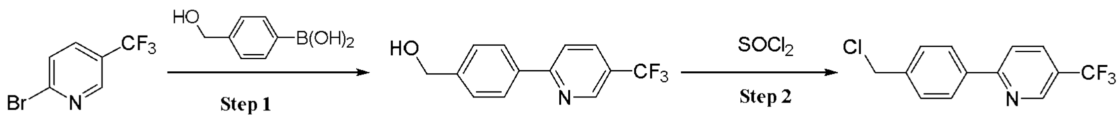


C13-1 C13-2 C13

In accordance with the same steps of intermediate **C3,** intermediate **C13** (2.0 g) was obtained using 2-bromo-5-(trifluoromethyl)pyridine (compound **C13-1)** as a starting material. LC-MS: m/z = 272.0 $(M+H)^+$.

**Synthesis of general intermediate C14 (2-(4-(chloromethyl)phenyl)-5-methylpyridine)**

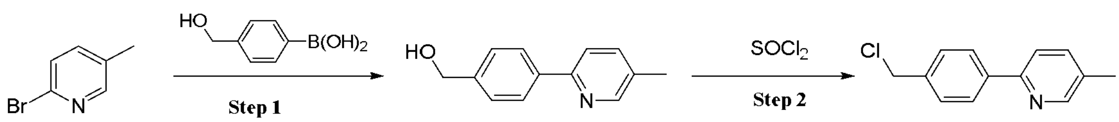


C14-1 C14-2 C14

In accordance with the same steps of intermediate **C3,** intermediate **C14** (10.0 g) was obtained using 2-bromo-5-methylpyridine (compound **C14-1)** as a starting material. LC-MS: m/z = 218.0 $(M+H)^+$.

**Synthesis of general intermediate C19 ((4-(5-(trifluoromethyl)pyridin-2-yl)bicyclo[2.2.2]octan-1-yl)methyl-4-methylbenzenesulfonate)**

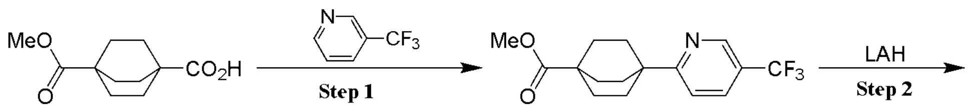


C19-1 C19-2

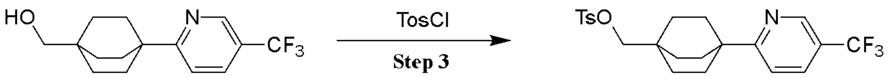


C19-3 C19

Step 1: Synthesis of compound **C19-2** (methyl 4-(5-(trifluoromethyl)pyridin-2-yl)bicyclo[2.2.2]octane-1-carboxylate)

Monomethyl hydrogen bicyclo[2.2.2]octane-1,4-dicarboxylate (compound **C19-1,** 25.0 g, 118 mmol, 1.00 eq), 3-(trifluoromethyl)pyridine (20.8 g, 141 mmol, 1.20 eq), ammonium persulfate (26.9 g, 118 mmol, 25.6 mL, 1.00 eq) and silver nitrate (4.01 g, 23.6 mmol, 0.20 eq) were added to a mixed solvent of dichloromethane (750.0 mL) and water (750.0 mL) successively, and the resulting mixture was reacted at 20°C for 16 hours. Dichloromethane (250.0 mL) was added, the resulting mixture was filtered to obtain a filtrate, and washed three times with water (100.0 mL). The organic layer was dried over anhydrous sodium sulfate, and filtered to obtain a filtrate. The filtrate was concentrated under vacuum to obtain a crude product, which was purified by preparative HPLC (purification conditions, $NH_3.H_2O/MeCN/H_2O$) to obtain compound **C19-2** (7.5 g). $^1H$ NMR (400 MHz, $CDCl_3$) δ 8.81 (s, , 1H), 7.85 (dd, $J_1$ = 4.0Hz, $J_2$ = 4.0Hz, 1H), 7.37 (d, $J$ = 4.0Hz, 1H), 3.69 (s, 3H), 1.96 (s, 12H).

Step 2: Synthesis of compound **C19-3** ((4-(5-(trifluoromethyl)pyridin-2-yl)bicyclo[2.2.2]octan-1-yl)methanol)

Compound **C19-2** (2.5 g, 7.98 mmol, 1.00 eq) was dissolved in tetrahydrofuran (50.0 mL), and lithium aluminum hydride (908 mg, 23.9 mmol, 3.00 eq) was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **C19-3** (2.3 g) as a white solid. LC-MS: m/z = 286.1 $(M+H)^+$.

Step 3: Synthesis of intermediate **C19**

Compound **C19-3** (1.6 g, 5.61 mmol, 1.00 eq) and *p*-toluenesulfonyl chloride (1.28 g, 6.73 mmol, 1.20 eq) were added to pyridine (25.0 mL), and the resulting mixture was reacted at 20°C for 12 hours. The reaction solution was concentrated under vacuum. Water (10.0 mL) was added, the resulting mixture was stirred for 5 minutes, and extracted three times with dichloromethane (5.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain intermediate **C19** (1.5 g, 3.38 mmol, 60.2% yield, 98.9% purity) as a white solid. $^1H$ NMR (400 MHz, $CDCl_3$) δ 8.79 (s, 1H), 7.84-7.79 (m, 3H), 7.38 (m, 3H), 3.72 (s, 2H), 2.47 (s, 3H), 1.93-1.89 (m, 6H), 1.57-1.54 (m, 6H); LC-MS: m/z = 440.2 $(M+H)^+$.

**Synthesis of general intermediate C25 (6-(chloromethyl)-2-methyl-3,4-dihydroisoquinolin-1(2H)-one)**

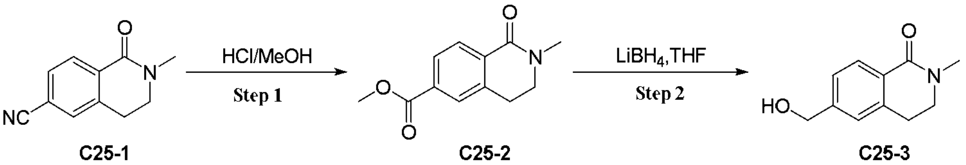


C25-1 C25-2 C25-3

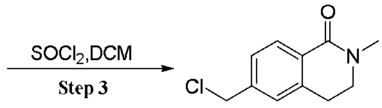


C25

Step 1: Synthesis of compound **C25-2** (methyl 2-methyl-1-oxo-1,2,3,4-tetrahydroisoquinoline-6-carboxylate)

2-Methyl-1-oxo-3,4-dihydroisoquinoline-6-carbonitrile (compound **C25-1,** 500 mg, 2.69 mmol, 1 eq) was dissolved in hydrochloric acid in methanol (4 M, 6.71 mL, 10 eq), and the mixture was stirred at 70°C for 3 hours. The reaction solution was concentrated under vacuum to obtain a crude product, which was purified by prep-HPLC (FA) to obtain compound **C25-2** (0.16 g, 715.21 μmol, 26.64% yield, 98% purity) as a white solid. LC-MS: m/z = 220.2 $(M+H)^+$.

Step 2: Synthesis of compound **C25-3** (6-(hydroxymethyl)-2-methyl-3,4-dihydroisoquinolin-1 (2H)-one)

Compound **C25-2** (75 mg, 342.10 μmol, 1 eq) was dissolved in tetrahydrofuran (4 mL), and lithium borohydride (2 M, 1 mL, 5.85 eq) was added at 0°C. After the addition was completed, the resulting mixture was stirred at 25°C for 3 hours. Then, hydrochloric acid (2 mL, 1M) was added for quenching three times, and the resulting mixture was extracted three times with ethyl acetate (15.0 mL). The organic layers were combined, dried over anhydrous sodium sulfate, and concentrated under vacuum to obtain compound **C25-3** (50 mg, crude) as a white solid. LC-MS: m/z = 192.2 $(M+H)^+$.

Step 3: Synthesis of intermediate **C25**

Compound **C25-3** (50 mg, 261.47 μmol, 1 eq) was dissolved in dichloromethane (10.0 mL), and thionyl chloride (155.54 mg, 1.31 mmol, 94.84 μL, 5 eq) was added at 0°C. After the addition was completed, the resulting mixture was heated up to 20°C and stirred for 12 hours. The reaction solution was concentrated under vacuum to obtain intermediate **C25** (50 mg, 238.47 μmol, 91.20% yield) as an off-white solid. LC-MS: m/z = 210.0 $(M+H)^+$.

**Synthesis of general intermediate C26 (6-(chloromethyl)-2-isopropyl-3,4-dihydroisoquinolin-1(2H)-one)**

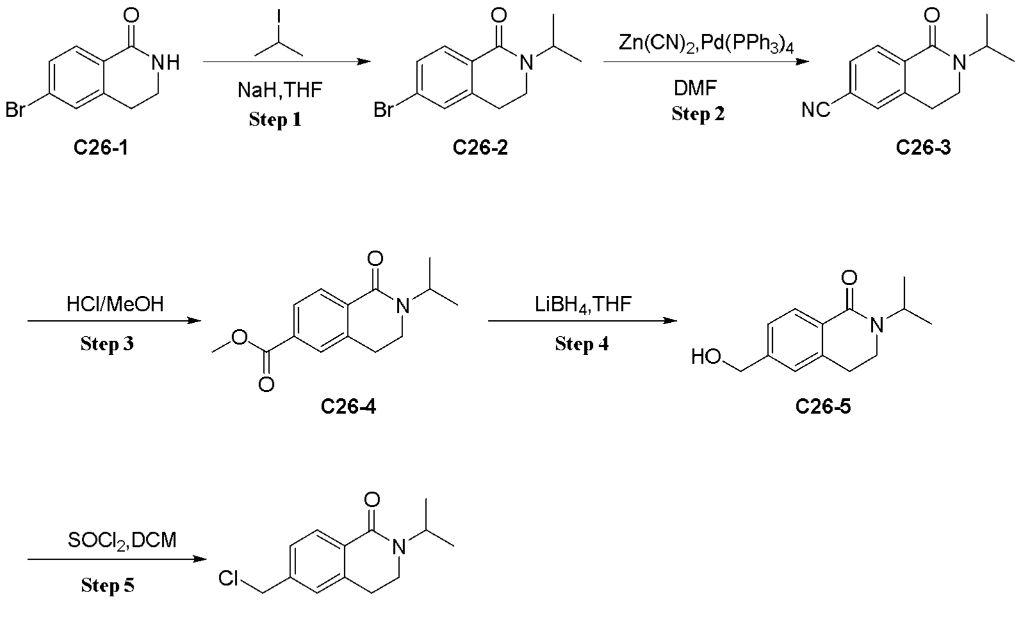


C26

Step 1: Synthesis of compound **C26-2** (6-bromo-2-isopropyl-3,4-dihydroisoquinolin-1(2H)-one)

6-Bromo-3,4-dihydro-2H-isoquinolin-1-one (compound **C26-1,** 500 mg, 2.69 mmol, 1 eq) was dissolved in tetrahydrofuran (40 mL), followed by the addition of sodium hydride (1.06 g, 26.54 mmol, 60% purity, 1.5 eq) at 0°C, and the resulting mixture was stirred for 30 minutes. 2-Iodopropane (6.02 g, 35.39 mmol, 3.54 mL, 2 eq) was added dropwise at 0°C. After the addition was completed, the resulting mixture was stirred at 25°C for 2 hours under nitrogen atmosphere. Water (30 mL) was added at 0°C to quench the reaction, and the resulting mixture was extracted three times with ethyl acetate (50 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 -1/2) to obtain compound **C26-2** (1.7 g, 6.19 mmol, 35.01% yield, 97.7% purity) as a light yellow oil. $^1$H NMR (400 MHz, $CDCl_3$) $\delta$ 7.94 (d, $J$=8.4Hz, 1H), 7.47-7.45 (m, 1H), 7.33 (s, 1H), 5.09-5.03(m, 1H), 3.42 (t, $J_1$=6.4Hz, $J_2$=6.8Hz, 2H), 2.91 (t, $J_1$=6.8Hz, $J_2$=6.4Hz, 2H), 1.19 (d, $J$=6.8Hz, 6H); LC-MS: m/z = 268.0 (M+H)+.

Step 2: Synthesis of compound **C26-3** (2-isopropyl-1-oxo-1,2,3,4-tetrahydroisoquinoline-6-carbonitrile)

Compound **C26-2** (2.6 g, 9.70 mmol, 1 eq) was dissolved in N,N-dimethylformamide (30 mL), followed by the addition of zinc cyanide (797.00 mg, 6.79 mmol, 430.81 $\mu$L, 0.7 eq) and tetrakis(triphenylphosphine)palladium (1.12 g, 969.61 $\mu$mol, 0.1 eq), and the resulting mixture was stirred at 80°C for 12 hours under nitrogen atmosphere. Water (30 mL) was added, and the resulting mixture was extracted three times with ethyl acetate (20 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 -0/1) to obtain compound **C26-3** (1.9 g, 8.84 mmol, 91.18% yield, 99.7% purity) as a white solid. $^1$H NMR (400 MHz, $CDCl_3$) $\delta$ 8.19 (d, $J$=8.4Hz, 1H), 7.64-7.62 (m, 1H), 7.50 (s,1H), 5.11-5.04(m, 1H), 3.48(t, $J_1$=6.4Hz, $J_2$=6.4Hz, 2H), 2.99(t, $J_1$=6.4Hz, $J_2$=6.4Hz, 2H), 1.22 (d, $J$=6.8Hz, 6H); LC-MS: m/z = 215.1 $(M+H)^+$.

Steps 3 to 5: Synthesis of intermediate **C26**

In accordance with the same steps of intermediate **C25,** intermediate **C26** (433.4 mg, 1.82 mmol, 45.1% yield) was obtained using compound **C26-3** as a starting material. LC-MS: m/z = 238.2 $(M+H)^+$.

**Synthesis of general intermediate C27 (2-((4-(chloromethyl)phenoxy)methyl)pyridine)**

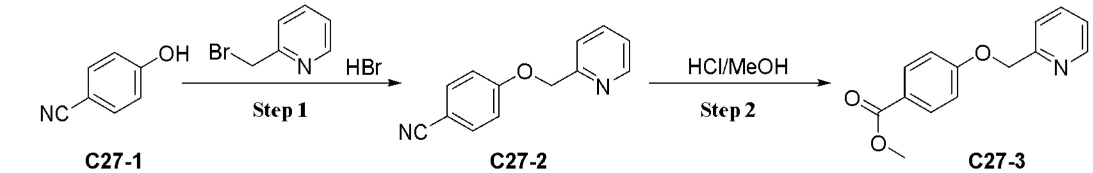


C27-1 C27-2 C27-3

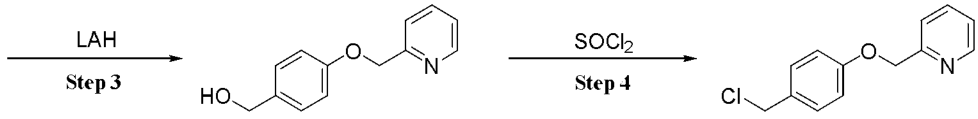


C27-4 C27

In accordance with the same steps of intermediate **C28,** intermediate C27 (800.7 mg, crude, a light yellow oil) was obtained using 4-hydroxybenzonitrile (compound C27-1) and 2-(bromomethyl)pyridine hydrobromide as starting materials. LC-MS: m/z = 234.0 $(M+H)^+$.

**Synthesis of general intermediate C28 (1-(chloromethyl)-4-(2-ethoxyethoxy)benzene)**

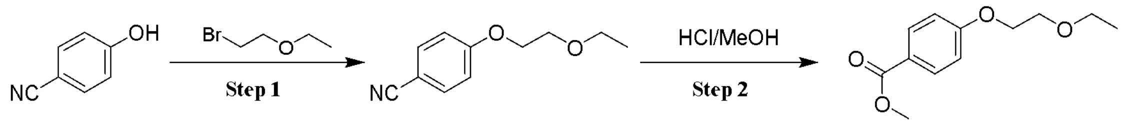


C28-1 C28-2 C28-3

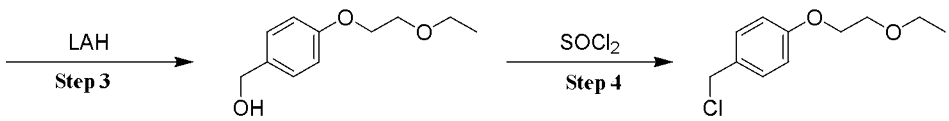


C28-4 C28

Step 1: Synthesis of compound **C28-2** (4-(2-ethoxyethoxy)benzonitrile)

4-Hydroxybenzonitrile (compound **C28-1,** 4.00 g, 33.6 mmol, 1.00 eq), 2-bromoethyl ethyl ether (10.0 g, 65.4 mmol, 7.35 mL, 1.95 eq) and potassium carbonate (9.28 g, 67.2 mmol, 2.00 eq) were added to acetonitrile (142 mL) successively, and the resulting mixture was stirred at 80°C for 12 hours. The reaction solution was concentrated under vacuum to obtain a crude product. Water (30.0 mL) was added, and the pH was adjusted to about 5 with 1 N HCl aqueous solution. The resulting mixture was extracted three times with dichloromethane (50.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 2/1) to obtain compound **C28-2** (6.69 g, crude) as a light yellow oil. $^1H$ NMR (400MHz, DMSO) δ 7.75 (dd, $J_1$ = 1.6Hz, $J_2$ = 6.8Hz, 2H), 7.11 (d, $J$ = 8.8Hz, 2H), 4.16 - 4.19 (m, 2H), 3.69 - 3.71 (m, 2H), 3.46 - 3.51 (m, 2H), 1.11 (t, $J$ = 7.0Hz, 3H).

Step 2: Synthesis of compound **C28-3** (methyl 4-(2-ethoxyethoxy)benzoate)

Compound **C28-2** (2.00 g, 10.5 mmol, 1.00 eq) was dissolved in HCl/MeOH (4 M, 105 mL, 40.0 eq), the resulting mixture was stirred at 80°C for 36 hours, and concentrated under vacuum to obtain a crude product. Water (20.0 mL) was added, and the pH was adjusted to about 7 with saturated aqueous sodium bicarbonate solution. The resulting mixture was extracted three times with ethyl acetate (50.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C28-3** (1.46 g, 6.51 mmol, 62.3% yield) as a light yellow oil. $^1H$ NMR (400MHz, $CDCl_3$) δ 7.99 (d, $J$ =

8.4Hz, 2H), 6.95 (d, *J* = 8.8Hz, 2H), 4.17 - 4.19 (m, 2H), 3.89 (s, 3H), 3.81 - 3.83 (m, 2H), 3.59 - 3.64 (m, 2H), 1.26 (t, *J* = 7.0Hz, 3H); LC-MS: m/z = 225.3 $(M+H)^+$.

Step 3: Synthesis of compound **C28-4** ((4-(2-ethoxyethoxy)phenyl)methanol)

Compound **C28-3** (0.700 g, 3.12 mmol, 1.00 *eq*) was dissolved in tetrahydrofuran (7.00 mL), and lithium aluminum hydride (237 mg, 6.24 mmol, 2.00 *eq*) was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **C28-4** (0.62 g, crude) as a colorless and transparent oil. $^1H$ NMR (400MHz, $CDCl_3$) δ 7.28 - 7.29 (m, 2H), 6.90 - 6.94 (m, 2H), 4.61 (s, 2H), 4.11 - 4.14 (m, 2H), 3.78 - 3.81 (m, 2H), 3.59 - 3.64 (m, 2H), 1.25 (t, *J* = 7.0Hz, 3H).

Step 4: Synthesis of intermediate **C28**

Compound **C28-4** (0.560 g, 2.85 mmol, 1.00 *eq*) was dissolved in dichloromethane (10.0 mL), and thionyl chloride (2.46 g, 20.7 mmol, 1.50 mL, 7.25 *eq*) was added at 0°C. After the addition was completed, the resulting mixture was heated up to 20°C and stirred for 12 hours. The reaction solution was concentrated under vacuum to obtain compound **C28** (0.60 g, crude) as a light yellow oil. $^1H$ NMR (400MHz, DMSO) δ 7.35 - 7.37 (m, 2H), 6.93 - 6.95 (m, 2H), 4.73 (s, 2H), 4.08 - 4.10 (m, 2H), 3.68 - 3.70 (m, 2H), 3.48 - 3.53 (m, 2H), 1.13 (t, *J* = 7.0Hz, 3H); LC-MS: m/z = 215.0 $(M+H)^+$.

**Synthesis of general intermediate C29 (1-(chloromethyl)-4-(3-methoxycyclobutoxy)benzene)**

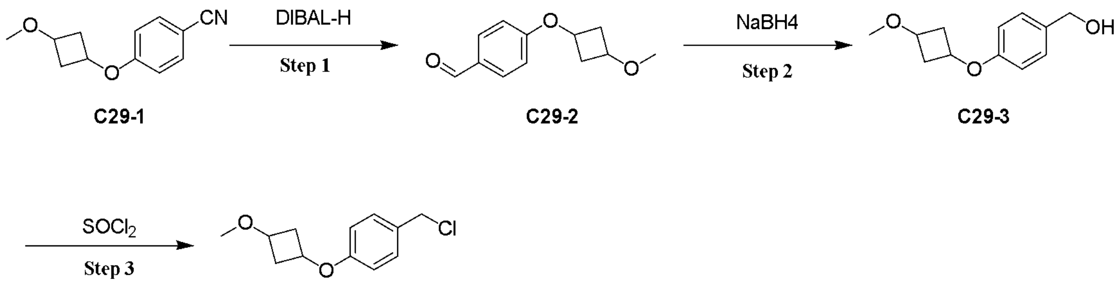


C29-1 C29-2 C29-3

C29

Step 1: Synthesis of compound **C29-2** (4-(3-methoxycyclobutoxy)benzaldehyde)

4-(3-Methoxycyclobutoxy)benzonitrile (compound **C29-1,** 0.560 g, 2.85 mmol, 1.00 *eq*) was dissolved in tetrahydrofuran (6.50 mL), and diisobutylaluminum hydride (1 M, 7.75 mL, 2.50 *eq*) was added dropwise at 0°C. After the addition was completed, the resulting mixture was stirred at 25°C for 2 hours. Saturated aqueous ammonium chloride solution (20.0 mL) was added dropwise at 0°C to quench the reaction, and the resulting mixture was extracted three times with ethyl acetate (50.0 mL). The organic layers were combined, dried over anhydrous sodium sulfate, and filtered to obtain a filtrate, which was concentrated under vacuum to obtain compound **C29-2** (0.65 g, crude) as a light yellow oil. $^1H$ NMR (400MHz, DMSO) δ 9.87 (s, 1H), 7.85 - 7.87 (m, 2H), 7.04 - 7.06 (m, 2H), 4.50 - 4.54 (m, 1H), 4.07 - 4.10 (m, 1H), 3.61 - 3.67 (m, 3H), 2.90 - 2.92 (m, 2H), 1.91 - 1.96 (m, 2H).

Step 2: Synthesis of compound **C29-3** ((4-(3-methoxycyclobutoxy)phenyl)methanol)

Compound **C29-2** (0.65 g, 3.15 mmol, 1.00 *eq*) was dissolved in methanol (6.00 mL), and sodium borohydride (0.210 g, 5.55 mmol, 1.76 *eq*) was added in portions at 0°C. After the addition was completed, the resulting mixture was heated up to 25°C and stirred for 2 hours. Water (10.0 mL) was added, and the resulting mixture was extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **C29-3** (0.12 g, crude) as a light yellow oil. $^1H$ NMR (400MHz, DMSO) δ 7.22 - 7.24 (m, 2H), 6.77 - 6.79 (m, 2H), 4.28 - 4.32 (m, 1H), 3.73 (s, 2H), 3.65 - 3.68 (m, 1H), 3.28 (s, 3H), 2.86 - 2.91 (m, 2H), 2.05 - 2.16 (m, 2H).

Step 3: Synthesis of compound **C29**

Compound **C29-3** (0.12 g, 576 μmol, 1.00 *eq*) was dissolved in dichloromethane (3.0 mL), and thionyl chloride (686 mg, 5.76 mmol, 418 μL, 10.0 *eq*) was added at 0°C. After the addition was completed, the resulting mixture was heated up to 20°C and stirred for 12 hours. The reaction solution was concentrated under vacuum to obtain compound **C29** (0.120 g, 529 μmol, 91.9% yield) as a light yellow oil. $^1$H NMR (400MHz, $CDCl_3$) δ 7.28 - 7.29 (m, 1H), 7.25 - 7.27 (m, 1H), 6.80 - 6.83 (m, 2H), 4.62 (s, 2H), 4.30 - 4.33 (m, 1H), 3.65 - 3.69 (m, 1H), 3.28 (s, 3H), 2.87 - 2.91 (m, 2H), 2.05 - 2.16 (m, 2H); LC-MS: m/z = 227.1 $(M+H)^+$.

**Synthesis of general intermediate C30 (2-(4-(chloromethyl)-3-methoxyphenyl)-1-methyl-4-(trifluoromethyl)-1H-imidazole)**

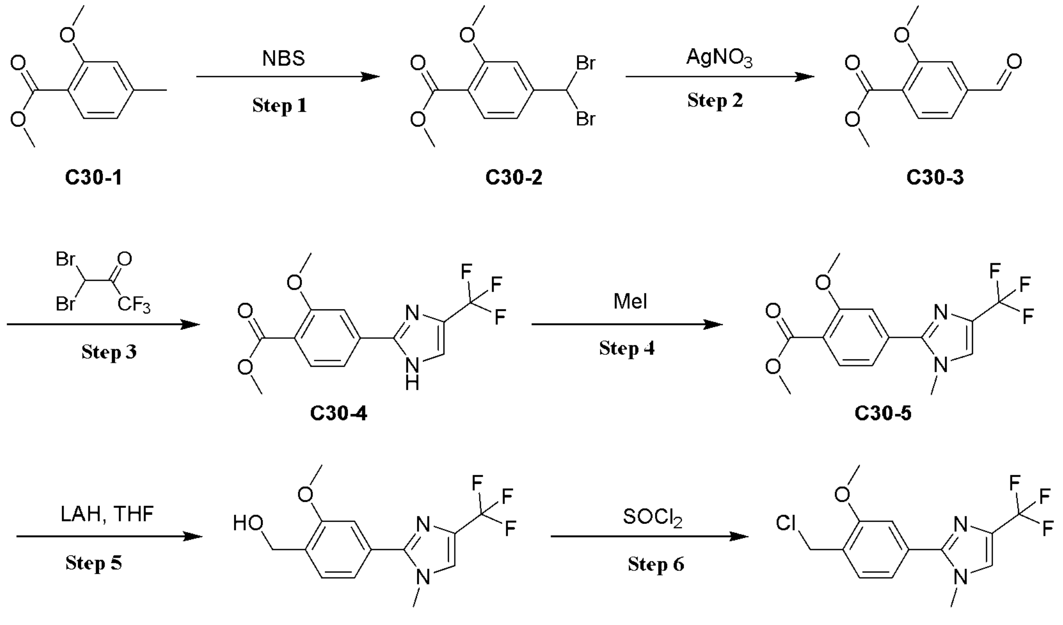


C30-1, C30-2, C30-3, C30-4, C30-5, C30-6, C30

Step 1: Synthesis of compound **C30-2** (methyl 4-(dibromomethyl)-2-methoxybenzoate)

Methyl 2-methoxy-4-methylbenzoate (compound **C30-1,** 5.00 g, 27.8 mmol, 1.00 eq) was added to tetrachloromethane (75.0 mL), and N-bromosuccinimide (10.9 g, 61.0 mmol, 2.20 eq) was added in portions at room temperature. After the addition was completed, the resulting mixture was heated up to 85°C and stirred for 12 hours. The reaction solution was filtered to obtain a filtrate, which was concentrated under vacuum to obtain a crude product. The crude product was purified by column chromatography (eluent: petroleum ether/ethyl acetate = 1/0 - 1/1) to obtain compound **C30-2** (6.50 g, 19.2 mmol, 69.3% yield) as a colorless transparent oil. $^1$H NMR (400MHz, $CDCl_3$) δ 7.77 (d, *J* = 8.0Hz, 1H), 7.21 (d, *J* = 1.6Hz, 1H), 7.13 (dd, $J_1$ = 1.8Hz, $J_2$ = 8.2Hz, 1H), 6.62 (s, 1H), 3.96 (s, 3H), 3.90 (s, 3H).

Step 2: Synthesis of compound **C30-3** (methyl 4-formyl-2-methoxybenzoate)

Compound **C30-2** (5.00 g, 14.8 mmol, 1.00 eq) was dissolved in acetone (60.0 mL), and silver nitrate (7.57 g, 44.6 mmol, 3.01 eq) and water (15.0 mL) were added. The resulting mixture was stirred at 20°C for 3 hours, and filtered to obtain a filtrate. The filtrate was concentrated under vacuum to obtain a crude product, which was purified by column chromatography (eluent: petroleum ether/ethyl acetate = 1/0 - 1/1) to obtain compound **C30-3** (2.60 g, 13.4 mmol, 90.5% yield) as a colorless transparent oil. $^1$H NMR δ 10.0 (s, 1H), 7.89 - 7.91 (m, 1H), 7.48 - 7.50 (m, 2H), 3.98 (s, 3H), 3.93 (s, 3H).

Steps 3 to 6: Synthesis of intermediate **C30**

Regarding Steps 3 to 6, in accordance with the same steps of intermediate **C5,** intermediate **C30** (320 mg, 938

μmol, 75.0% yield, a white solid) was obtained using **C30-3** as a starting material. LC-MS: m/z = 305.1 $(M+H)^+$.

**Synthesis of general intermediate C31 (2-(4-(chloromethyl)-2-methoxyphenyl)-1-methyl-4-(trifluoromethyl)-1H-imidazole)**

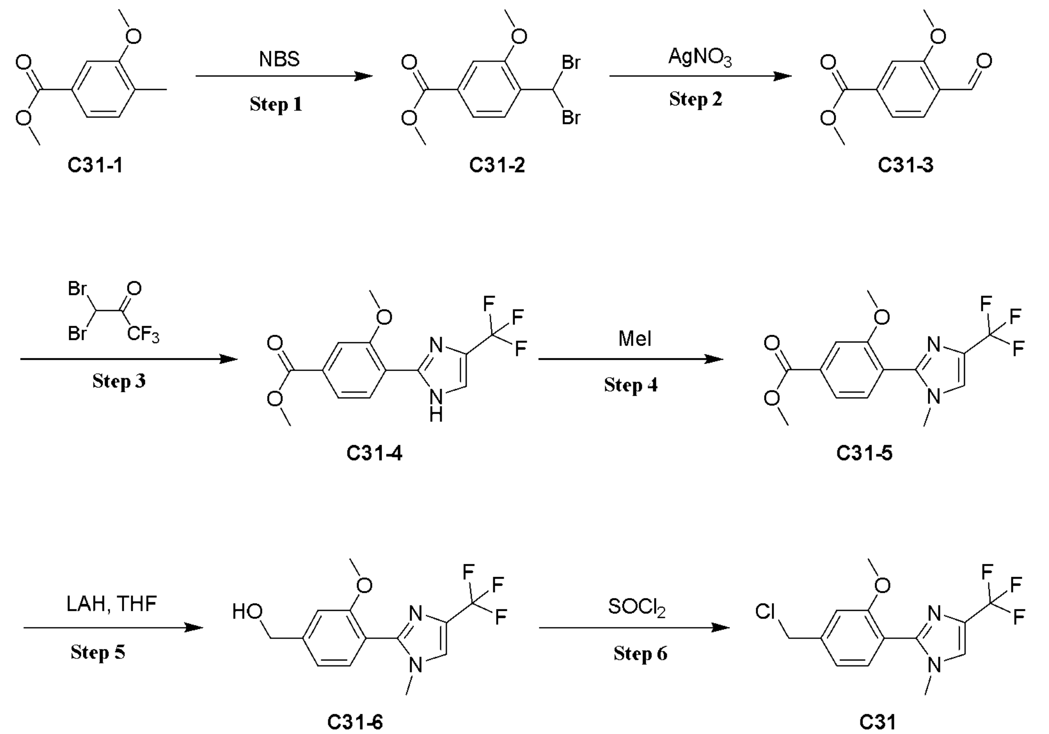


C31-1 C31-2 C31-3 C31-4 C31-5 C31-6 C31

In accordance with the same steps of intermediate **C30,** intermediate **C31** (450 mg, a white solid) was obtained using methyl 3-methoxy-4-methylbenzoate (compound **C31-1)** as a starting material. LC-MS: m/z = 305.1 $(M+H)^+$.

**Synthesis of general intermediate C32 (1-(4-(chloromethyl)-3-methoxyphenyl)-5-methyl-3-(trifluoromethyl)-1H-pyrazole)**

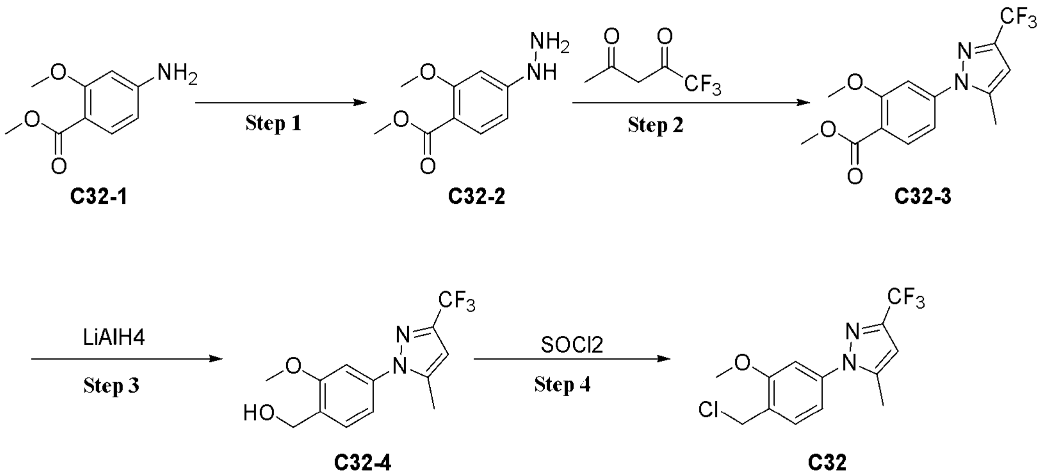


C32-1 C32-2 C32-3 C32-4 C32

In accordance with the same steps of intermediate **C11,** intermediate **C32** (1.18 g, 3.87 mmol, 88.5% yield, a white solid) was obtained using methyl 2-methoxy-4-aminobenzoate (compound **C32-1)** as a starting material. LC-MS:

m/z = 305.1 $(M+H)^+$.

**Synthesis of general intermediate C33 (1-(4-(chloromethyl)-2-methoxyphenyl)-5-methyl-3-(trifluoromethyl)-1H-pyrazole)**

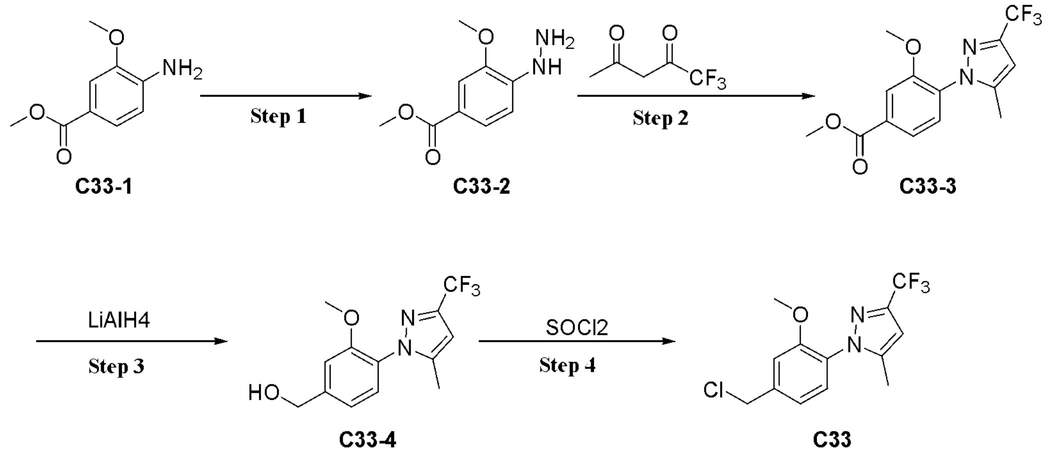


C33-1 C33-2 C33-3 C33-4 C33

In accordance with the same steps of intermediate **C11,** intermediate **C33** (796.0 mg, 2.61 mmol, 79.6% yield, a white solid) was obtained using methyl 4-amino-3-methoxybenzoate (compound **C33-1)** as a starting material. LC-MS: m/z = 305.0 $(M+H)^+$.

**Synthesis of general intermediate C35 ((4-(1-methyl-4-(trifluoromethyl)-1H-imidazol-2-yl)bicyclo[2.2.2]octan-1-yl)methyl-4-methylbenzenesulfonate)**

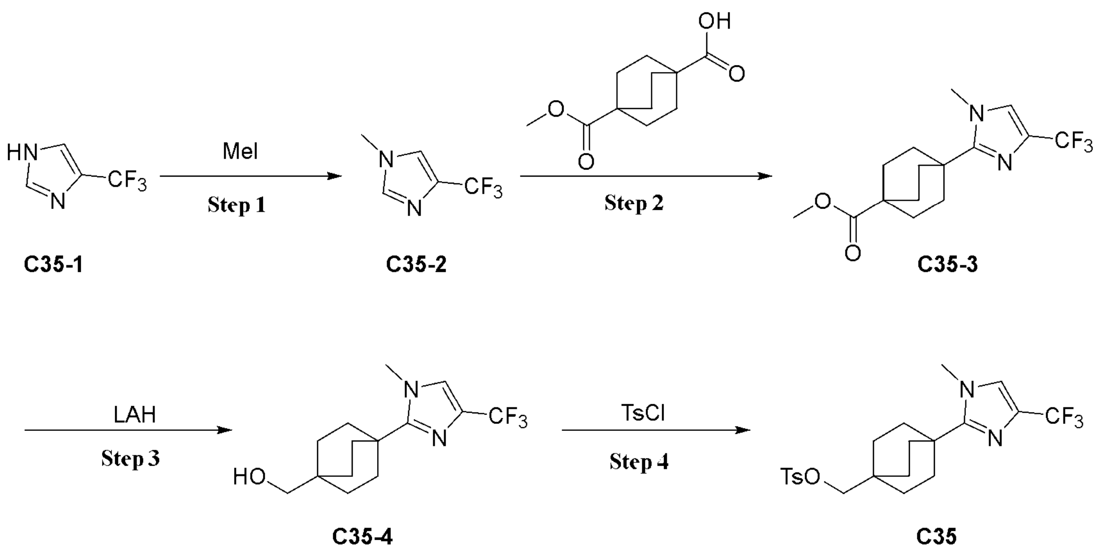


C35-1 C35-2 C35-3 C35-4 C35

Step 1: Synthesis of compound **C35-2** (1-methyl-4-(trifluoromethyl)-1H-imidazole)

Compound 4-(trifluoromethyl)-1H-imidazole (compound **C35-1,** 26 g, 0.19 mol) and potassium carbonate (105.5 g, 0.76 mol, 4.0 eq) were added to acetonitrile (390 mL), and the resulting mixture was stirred at 0°C for 30 minutes. Iodomethane (32.6 g, 0.23 mol, 1.2 eq) was added dropwise. After the addition was completed, the resulting mixture was stirred at room temperature for 16 hours. Then, the reaction solution was concentrated under vacuum to obtain a crude product. Water (260 mL) was added, the resulting mixture was stirred for 10 minutes, and extracted three times with ethyl acetate (200 mL). The organic layers were combined, washed twice with saturated brine (200 mL), dried over anhydrous

sodium sulfate, and filtered to obtain a filtrate, which was concentrated under vacuum to obtain compound **C35-2** (26 g, crude). LC-MS: m/z = 151.0 $(M+H)^+$.

Step 2: Synthesis of compound **C35-3** (methyl 4-(1-methyl-4-(trifluoromethyl)-1H-imidazol-2-yl)bicyclo[2.2.2]octane-1-carboxylate)

Compound **C35-2** (14.75 g, 98.3 mmol) and monomethyl hydrogen bicyclo[2.2.2]octane-1,4-dicarboxylate (25 g, 117.9 mmol, 1.2 eq) were dissolved in dichloromethane (295 mL) and water (295 mL), followed by the addition of silver nitrate (6.0 g, 35.4 mmol, 0.3 eq.) and ammonium persulfate (44.9 g, 78.7 mmol, 2.0 eq.), and the resulting mixture was stirred at 25°C for 16 hours. The reaction solution was filtered through diatomaceous earth, and washed twice with dichloromethane (100 mL). The organic layer was separated, and the aqueous layer was extracted three times with dichloromethane (200 mL). The organic layers were combined, washed twice with saturated brine (200 mL), dried over anhydrous sodium sulfate, and filtered to obtain a filtrate, which was concentrated under vacuum to obtain compound **C35-3** (35.2 g, crude) as a yellow oil. $^1$H NMR (400MHz, $CDCl_3$) 7.09 - 7.08 (m, 1H), 3.77 (s, 3H), 3.67 (s, 3H), 2.07 - 2.03 (m, 6H), 1.93 - 1.89 (m, 6H); LC-MS: m/z = 317.2 $(M+H)^+$.

Step 3: Synthesis of compound **C35-4** ((4-(1-methyl-4-(trifluoromethyl)-1H-imidazol-2-yl)bicyclo[2.2.2]octan-1-yl) methanol)

Compound **C35-3** (35.2 g, 111.4 mmol) was dissolved in tetrahydrofuran (180.0 mL), and lithium aluminum hydride (2.5 M, 111.4 mL, 278.5 mmol, 2.5 eq) was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (500.0 mL). The organic layers were combined, dried over anhydrous sodium sulfate, and filtered to obtain a filtrate, which was concentrated under vacuum to obtain compound **C35-4** (25.1 g, crude) as a yellow oil. LC-MS: m/z = 289.1 $(M+H)^+$.

Step 4: Synthesis of compound **C35**

Compound **C35-4** (25.1 g, 87.2 mmol), *p*-toluenesulfonyl chloride (33.2 g, 174.4 mmol, 2.0 eq) and 4-dimethylaminopyridine (32.0 g, 261.6 mmol, 3.0 eq) were added to dichloromethane (251 mL) successively, and the resulting mixture was stirred at 25°C for 16 hours. The reaction solution was washed twice with water (100 mL), and the organic layer was separated. The organic layer was dried over anhydrous sodium sulfate, and filtered to obtain a filtrate. The filtrate was concentrated under vacuum to obtain a crude product, which was purified by column chromatography (eluent: petroleum ether/dichloromethane = 1/0 - 0/1) to obtain intermediate C35 (10.0 g) as a white solid. $^1$H NMR (400 MHz, DMSO-d6) $\delta$ 7.82 (d, J = 8.2 Hz, 2H), 7.67 (s, 1H), 7.52 (d, J = 8.0 Hz, 2H), 3.77 (s, 3H), 3.72 (s, 2H), 2.46 (s, 3H), 1.95 - 1.86 (m, 6H), 1.49 - 1.37 (m, 6H); LC-MS: m/z = 443.3 $(M+H)^+$.

**Synthesis of general intermediate C48 (2-(4-(chloromethyl)phenyl)-4-(difluoromethyl)-1-methyl-1H-imidazole)**

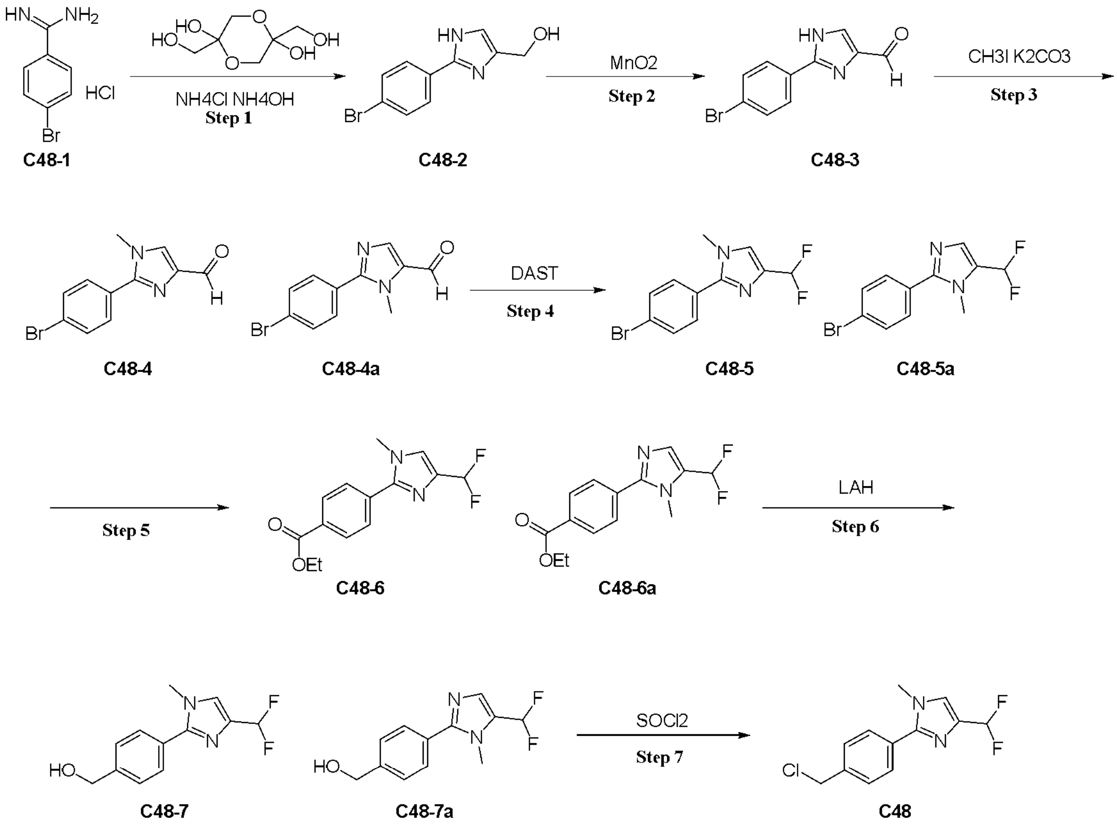


C48-1 C48-2 C48-3 C48-4 C48-4a C48-5 C48-5a C48-6 C48-6a C48-7 C48-7a C48

Step 1: Synthesis of compound **C48-2** ((2-(4-bromophenyl)-1H-imidazol-4-yl)methanol)

4-Bromobenzimidamide hydrochloride (compound **C48-1,** 17.8 g, 75.36 mmol), 1,3-dihydroxyacetone dimer (15 g, 83.26 mmol, 1.1 eq), ammonium chloride (20 g, 374 mmol, 5 eq) and sodium hydroxide (3 g, 75.36 mmol, 1 eq) were added to aqueous ammonia (500 mL) successively, and the resulting mixture was stirred at an external temperature of 80°C for 2 hours. The reaction solution was cooled to room temperature, and filtered to obtain a solid, which was concentrated under vacuum to obtain compound **C48-2** (15 g, crude) as a white solid. LC-MS: m/z = 252.6 $(M+H)^+$.

Step 2: Synthesis of compound **C48-3** (2-(4-bromophenyl)-1H-imidazole-4-carbaldehyde)

Compound **C48-2** (14 g, 55.56 mmol) was dissolved in tetrahydrofuran (300 mL), and manganese dioxide (48 g, 555.6 mmol, 10 eq) was added and stirred at an external temperature of 60°C for 16 hours. The reaction solution was cooled to room temperature, and filtered to obtain a filtrate, which was concentrated under vacuum to obtain compound **C48-3** (11.1 g, crude) as a white solid. LC-MS: m/z = 250.8 $(M+H)^+$.

Step 3: Synthesis of compound **C48-4** (2-(4-bromophenyl)-1-methyl-1H-imidazole-4-carbaldehyde)

Compound **C48-3** (11.1 g, 44.4 mmol), iodomethane (8.2 g, 57.7 mmol, 1.3 eq), and potassium carbonate (24.6 g, 177.6 mmol, 4 eq) were added to N,N-dimethylformamide (120 mL). The reaction solution was stirred at an external temperature of 45°C for 3 hours, and filtered to obtain a filtrate. Water (1000 mL) was added, and the resulting mixture was extracted three times with ethyl acetate (500 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compound **C48-4** and compound **C48-4a** (6.8 g in total) as a white solid. LC-MS: m/z = 265.0 $(M+H)^+$.

Step 4: Synthesis of compound **C48-5** (2-(4-bromophenyl)-4-(difluoromethyl)-1-methyl-1H-imidazole)

Compound **C48-4** and compound **C48-4a** (6.7 g, 25.38 mmol) were dissolved in anhydrous dichloromethane (200 mL), and diethylaminosulfur trifluoride (41 g, 253.8 mmol, 10 eq) was slowly added dropwise at 0°C. After the addition was completed, the resulting mixture was stirred at room temperature (25°C) for 5 hours. Then, the reaction solution was

slowly added to saturated aqueous sodium bicarbonate solution, and extracted three times with dichloromethane (500 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compounds **C48-5** and **C48-5a** (4.1 g) as a white solid. LC-MS: m/z = 286.9 $(M+H)^+$.

Step 5: Synthesis of compound **C48-6** (ethyl 4-(4-(difluoromethyl)-1-methyl-1H-imidazol-2-yl)benzoate)

Compounds **C48-5** and **C48-5a** (800 mg, 2.8 mmol), [1,1'-bis(diphenylphosphino)ferrocene]palladium dichloride dichloromethane complex (1.36 g, 1.68 mmol, 0.6 eq), 4,5-bis(diphenylphosphino)-9,9-dimethylxanthene (730 mg, 1.26 mmol, 0.45 eq) and triethylamine (1.42 g, 14 mmol, 5 eq) were added to anhydrous ethanol (32 mL) successively, and the resulting mixture was stirred at an external temperature of 85°C for 20 hours under carbon monoxide atmosphere (double-layer balloon, 25 psi). Then, EA (100 mL) was added, the resulting mixture was stirred for 10 minutes, and filtered to obtain a filtrate. The filtrate was mixed with silica gel, and purified by column chromatography (eluent: petroleum ether/ethyl acetate = 1/0 - 1/1) to obtain compound **C48-6** and compound **C48-6a** (630 mg). LC-MS: m/z = 281.1 $(M+H)^+$.

Step 6: Synthesis of compound **C48-7** ((4-(4-(difluoromethyl)-1-methyl-1H-imidazol-2-yl)phenyl)methanol)

Compound **C48-6** and compound **C48-6a** (630 mg) were dissolved in anhydrous THF (7 mL), and a solution of lithium aluminum hydride in THF (2.5 M, 2 mL) was added dropwise at 0°C. After the addition was completed, the resulting mixture was selfheated to room temperature (25°C) and stirred for 2 hours. Water (10 mL) was added at 0°C to quench the reaction, and the pH was adjusted to about 5 with 1 M aqueous hydrochloric acid solution. The resulting mixture was extracted three times with dichloromethane (50 mL). The organic layers were combined, dried over anhydrous sodium sulfate, and concentrated under vacuum to obtain crude compound **C48-7** and compound **C48-7a** (500 mg). LC-MS: m/z = 239.1 $(M+H)^+$.

Step 7: Synthesis of intermediate **C48**

Compound **C48-7** and compound **C48-7a** (500 mg) were dissolved in anhydrous dichloromethane (5 mL), and thionyl chloride (1.5 mL) was added and the resulting mixture was stirred at room temperature of 25°C for 1.5 hours. Then, the reaction solution was directly concentrated under vacuum to obtain a crude product, which was purified by column chromatography (eluent: petroleum ether/ethyl acetate = 1/0 - 0/1) to obtain compound **C48** (130 mg). LC-MS: m/z = 257.1 $(M+H)^+$.

**Synthesis of general intermediate C49 (2-(chloromethyl)-5-(1-methyl-4-(trifluoromethyl)-1H-imidazol-2-yl)pyridine)**

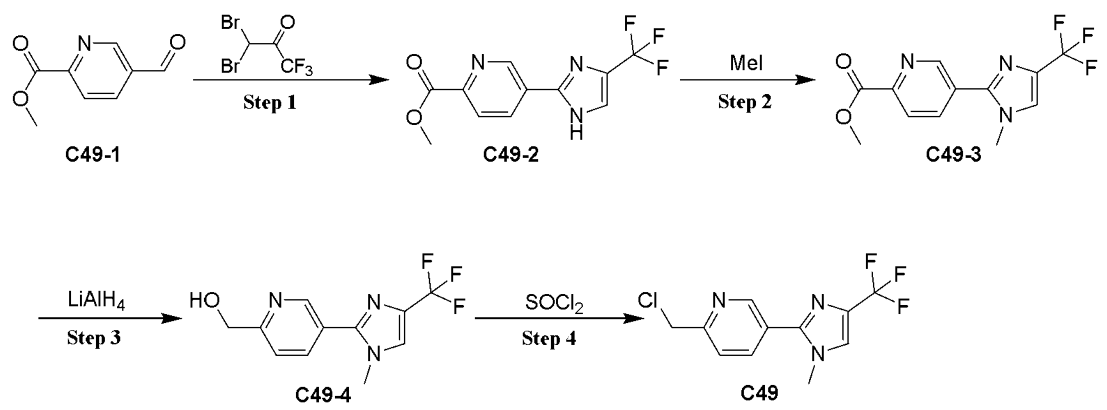

C49-1 C49-2 C49-3
C49-4 C49

In accordance with the same steps of intermediate **C5,** intermediate **C49** (2.0 g) was obtained using methyl 5-formylpyridine-2-carboxylate (compound C49-1) as a starting material. LC-MS: m/z = 276.0 $(M+H)^+$.

**Synthesis of general intermediate C57 (2-(4-(chloromethyl)phenyl)-1-cyclopropyl-4-(trifluoromethyl)-1H-imidazole)**

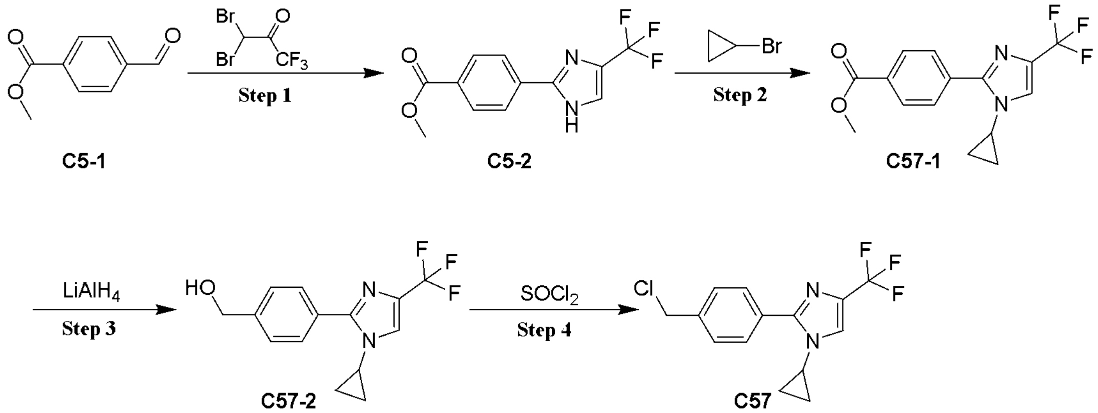


C5-1 C5-2 C57-1 C57-2 C57

In accordance with the same steps of intermediate **C5,** intermediate **C57** (1.83 g) was obtained using methyl p-formylbenzoate (compound **C5-1**) as a starting material. LC-MS: m/z = 301.2 $(M+H)^+$.

**Synthesis of general intermediate C58 (2-(4-(chloromethyl)phenyl)-1-isopropyl-4-(trifluoromethyl)-1H-imidazole)**

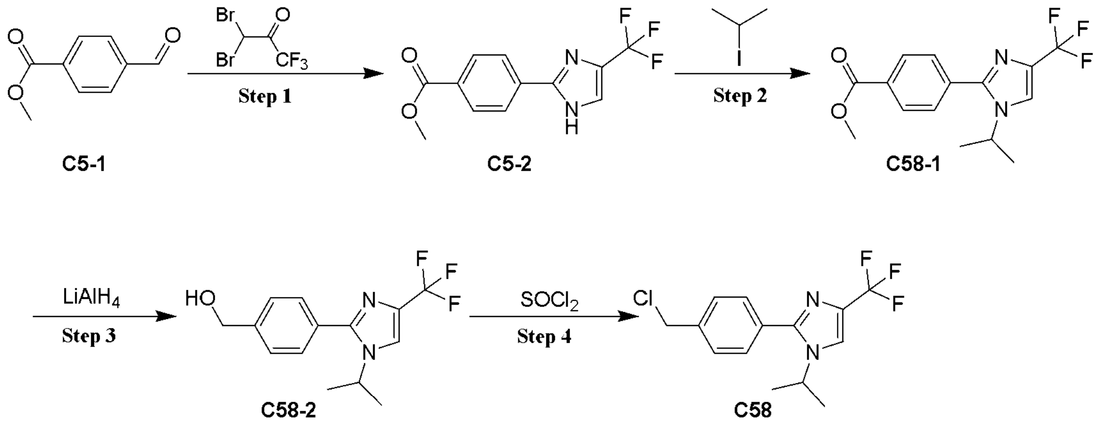


C5-1 C5-2 C58-1 C58-2 C58

In accordance with the same steps of intermediate **C5,** intermediate **C58** (0.6 g) was obtained using methyl *p*-formylbenzoate (compound **C5-1**) as a starting material. $^1$H NMR (400 MHz, DMSO-$d_6$) δ 8.22 (s, 1H), 7.61-7.64 (m, 4H), 4.88 (d, *J* = 2.4 Hz, 2H), 4.50-4.55 (m, 1H), 1.44 (d, *J* = 6.6 Hz, 6H); LC-MS: m/z = 303.1 $(M+H)^+$.

**Synthesis of general intermediate C59 (2-(4-(chloromethyl)phenyl)-1-(fluoromethyl)-4-(trifluoromethyl)-1H-imidazole)**

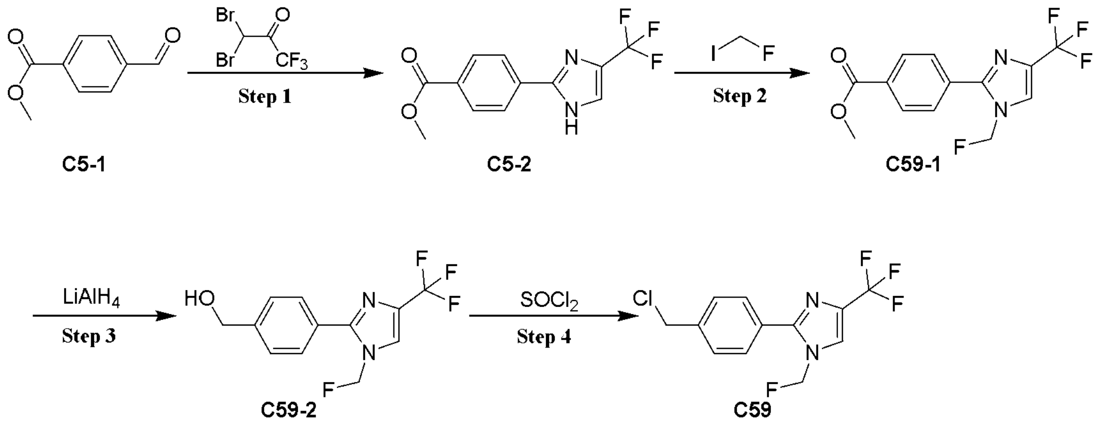


C5-1 C5-2 C59-1 C59-2 C59

In accordance with the same steps of intermediate **C5,** intermediate **C59** (0.6 g) was obtained using methyl *p*-formylbenzoate (compound **C5-1**) as a starting material. LC-MS: m/z = 293.1 $(M+H)^+$.

**Synthesis of general intermediate D5 ((4-(1-methyl-4-(trifluoromethyl)-1H-imidazol-2-yl)phenyl)methanamine)**

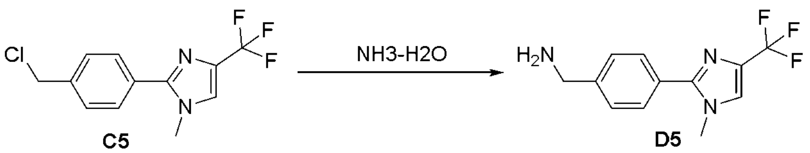


C5 D5

In accordance with the same steps of intermediate **BB13,** intermediate **D5** (0.16 g, 0.63 mmol, 85.9% yield) was obtained using compound **C5** (0.2 g, 0.73 mmol, 1.00 *eq)* as a starting material. $^1H$ NMR (400 MHz, DMSO-d6) δ 7.92 (t, J = 1.4 Hz, 1H), 7.75 - 7.61 (m, 2H), 7.50 (d, J = 8.0 Hz, 2H), 3.83 (s, 2H), 3.80 (s, 3H); LC-MS: m/z = 256.0 $(M+H)^+$.

**Synthesis of general intermediate D11 ((4-(5-methyl-3-(trifluoromethyl)-1H-pyrazol-1-yl)phenyl)methanamine)**

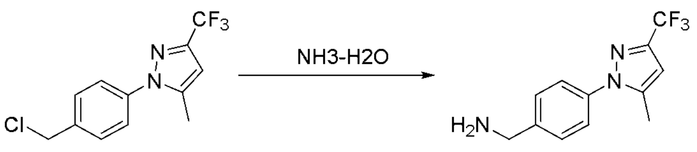


C11 D11

In accordance with the same steps of intermediate **BB13,** intermediate **D11** (1.59 g, 6.23 mmol, 77.6% yield) was obtained using compound **C11** (2.2 g, 8.03 mmol, 1.00 *eq)* as a starting material. $^1H$ NMR (400 MHz, DMSO-d6) δ 7.60 - 7.47 (m, 4H), 6.76 (s, 1H), 3.84 (s, 2H), 2.35 (s, 3H); LC-MS: m/z = 256.1 $(M+H)^+$.

**Synthesis of general intermediate D58 ((4-(1-isopropyl-4-(trifluoromethyl)-1H-imidazol-2-yl)phenyl)methanamine)**

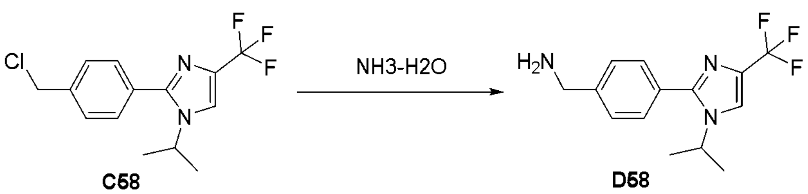


C58 D58

In accordance with the same steps of intermediate **BB13,** intermediate **D58** (0.98 g, 3.46 mmol, 69.9% yield) was obtained using compound **C58** (1.5 g, 4.95 mmol, 1.00 *eq)* as a starting material. LC-MS: m/z = 284.3 $(M+H)^+$.

**Synthesis of intermediate BB1C2 (2-chloro-5-methoxy-N-methyl-N-((4-(pyridin-2-yl)cyclohexyl)methyl)pyrimidin-4-amine)**

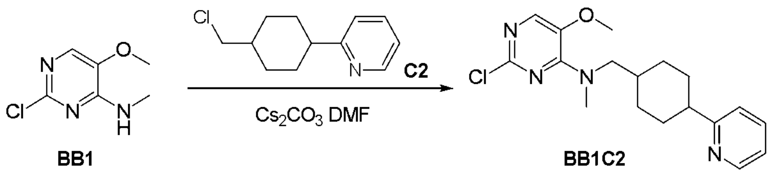


Compound **BB1** (500.0 mg, 2.89 mmol, 1.00 eq), compound C2 (634.4 mg, 3.04 mmol, 1.05 eq) and cesium carbonate (3.77 g, 11.56 mmol, 4.0 eq) were added to N,N-dimethylformamide (10.0 mL) successively, and the resulting mixture was stirred at 80°C for 2 hours. The reaction solution was cooled to 25°C. Ethyl acetate (100.0 mL) was added, the resulting mixture was stirred for 5 minutes, and filtered to obtain a filtrate. The filtrate was washed three times with saturated brine (100.0 mL), dried over anhydrous sodium sulfate, and concentrated under vacuum to obtain a crude product, which was purified by column chromatography (eluent: petroleum ether/ethyl acetate = 1/0 - 1/2) to obtain intermediate **BB1C2** (910.3 mg, 2.63 mmol, 91.0% yield) as a light yellow solid. LC-MS: m/z = 347.2 $(M+H)^+$.

**Synthesis of intermediate BB1C3 (2-chloro-5-methoxy-N-methyl-N-(4-(pyridin-2-yl)benzyl)pyrimidin-4-amine)**

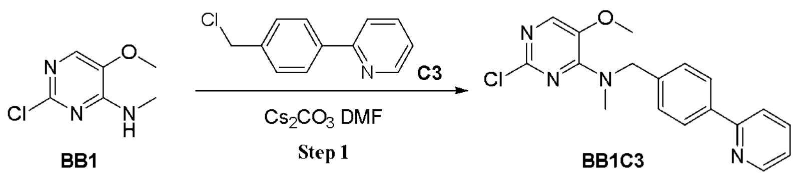


In accordance with the same steps of intermediate **BB1C2,** intermediate **BB1C3** (689.9 mg, 2.03 mmol, 87.5% yield, a light yellow solid) was obtained using intermediate **BB1** and compound **C3** as starting materials. LC-MS: m/z = 341.2 $(M+H)^+$.

**Synthesis of intermediate BB1C4 (2-chloro-5-methoxy-N-methyl-N-((1-(1-methyl-4-(trifluoromethyl)-1H-imidazol-2-yl)piperidin-4-yl)methyl)pyrimidin-4-amine)**

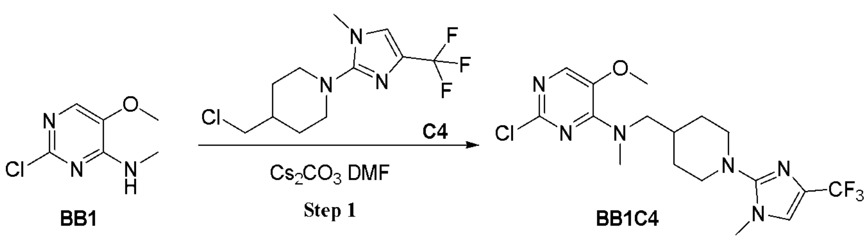


In accordance with the same steps of intermediate **BB1C2,** intermediate **BB1C4** (352.5 mg, 0.843 mmol, 69.5% yield, a light yellow solid) was obtained using intermediate **BB1** and compound **C4** as starting materials. LC-MS: m/z = 419.2 $(M+H)^+$.

**Synthesis of intermediate BB1C5 (2-chloro-5-methoxy-N-methyl-N-(4-(1-methyl-4-(trifluoromethyl)-1H-imidazol-2-yl)benzyl)pyrimidin-4-amine)**

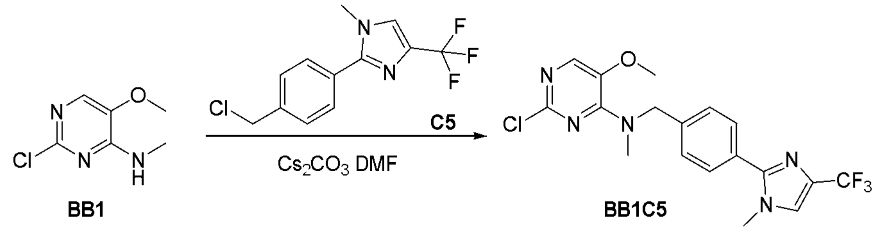


In accordance with the same steps of intermediate **BB1C2,** intermediate **BB1C5** (2.39 g, 5.81 mmol, 81.6% yield, a light yellow solid) was obtained using intermediate **BB1** and compound **C5** as starting materials. $^1$H NMR (400 MHz, DMSO-$d_6$) δ 8.03 - 7.90 (m, 2H), 7.80 - 7.72 (m, 2H), 7.41 (d, *J* = 8.3 Hz, 2H), 4.98 (s, 2H), 3.18 (s, 3H), 2.92 (s, 3H), 2.76 (d, *J*= 0.6 Hz, 3H); LC-MS: m/z = 412.1 (M+H)$^+$.

**Synthesis of intermediate BB1C6 (2-chloro-5-methoxy-N-methyl-N-(1-(pyridin-2-yl)piperidin-4-yl)methyl)pyrimidin-4-amine)**

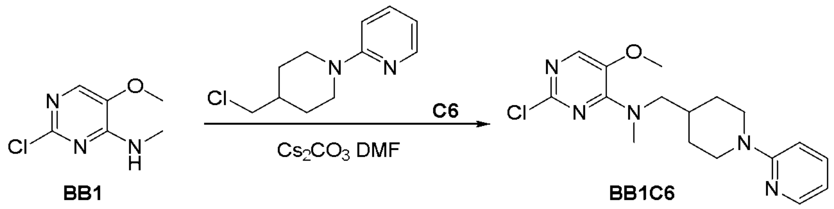


In accordance with the same steps of intermediate **BB1C2,** intermediate **BB1C6** (169.7 mg, 0.49 mmol, 59.9% yield, a light yellow solid) was obtained using intermediate **BB1** and compound **C6** as starting materials. LC-MS: m/z = 348.2 (M+H)$^+$.

**Synthesis of intermediate BB1C11 (2-chloro-5-methoxy-N-methyl-N-(4-(5-methyl-3-(trifluoromethyl)-1H-pyrazol-1-yl)benzyl)pyrimidin-4-amine)**

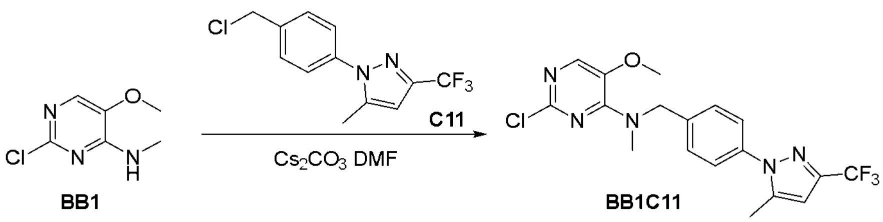


In accordance with the same steps of intermediate **BB1C2,** intermediate **BB1C11** (3.31g, 8.04 mmol, 73.2% yield, an off-white solid) was obtained using intermediate **BB1** and compound **C11** as starting materials. LC-MS: m/z = 412.8 (M+H)$^+$.

**Synthesis of intermediate BB2C2 (2-chloro-5-methoxy-N-((4-(pyridin-2-yl)cyclohexyl)methyl)pyrimidin-4-amine)**

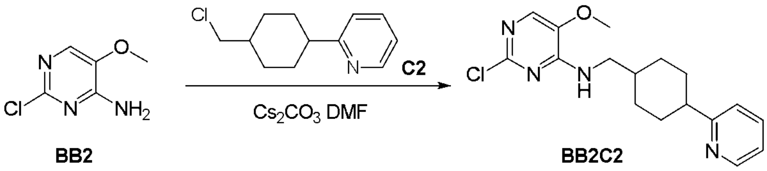

BB2 BB2C2

In accordance with the same steps of intermediate **BB1C2,** intermediate **BB2C2** (134.1 mg, 0.40 mmol, 46.6% yield, a light yellow oil) was obtained using intermediate **BB2** and compound **C2** as starting materials. LC-MS: m/z = 333.1 $(M+H)^+$.

**Synthesis of intermediate BB2C3 (2-chloro-5-methoxy-N-(4-(pyridin-2-yl)benzyl)pyrimidin-4-amine)**

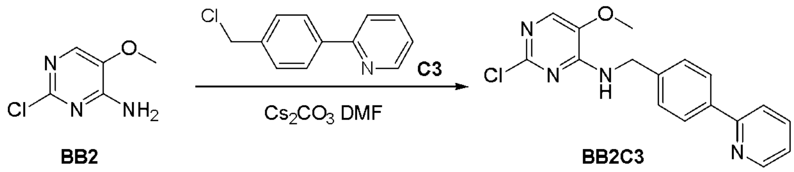

BB2 BB2C3

In accordance with the same steps of intermediate **BB1C2,** intermediate **BB2C3** (255.5 mg, 0.78 mmol, 87.7% yield, a light yellow solid) was obtained using intermediate **BB2** and compound **C3** as starting materials. LC-MS: m/z = 327.0 $(M+H)^+$.

**Synthesis of intermediate BB2C4 (2-chloro-5-methoxy-N-((1-(1-methyl-4-(trifluoromethyl)-1H-imidazol-2-yl)piperidin-4-yl)methyl)pyrimidin-4-amine)**

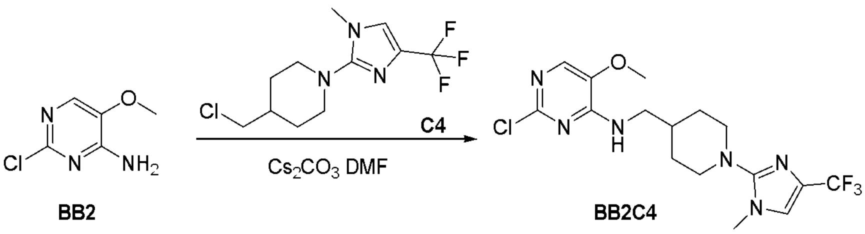

BB2 BB2C4

In accordance with the same steps of intermediate **BB1C2,** intermediate **BB2C4** (98.8 mg, 0.24 mmol, 39.2% yield, a light yellow solid) was obtained using intermediate **BB2** and compound **C4** as starting materials. LC-MS: m/z = 405.1 $(M+H)^+$.

**Synthesis of intermediate BB2CS (2-chloro-5-methoxy-N-((4-(1-methyl-4-(trifluoromethyl)-1H-imidazol-2-yl) benzyl)pyrimidin-4-amine)**

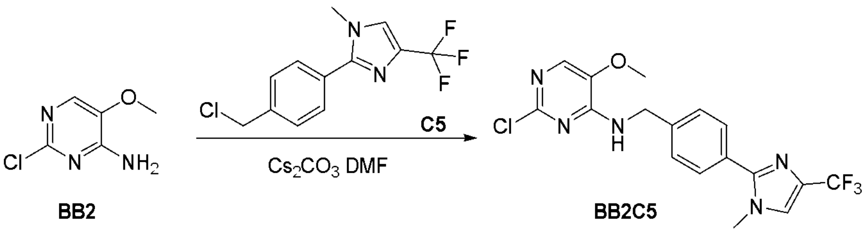

BB2 BB2C5

In accordance with the same steps of intermediate **BB1C2,** intermediate **BB2CS** (18.9 g, 47.60 mmol, 69.8% yield, a light yellow solid) was obtained using intermediate **BB2** and compound **C5** as starting materials. $^1H$ NMR (400

MHz, $CDCl_3$) δ 7.60 - 7.62 (m, 2H), 7.57 (s, 1H), 7.42 - 7.44 (m, 2H), 7.27 - 7.31 (m, 1H), 5.82 (s, 1H), 4.73 - 4.74 (m, 2H), 3.87 (s, 3H), 3.76 (s, 3H); LC-MS: m/z = 398.6 $(M+H)^+$.

**Synthesis of intermediate BB2C6 (2-chloro-5-methoxy-N-((1-(pyridin-2-yl)piperidin-4-yl)methyl)pyrimidin-4-amine)**

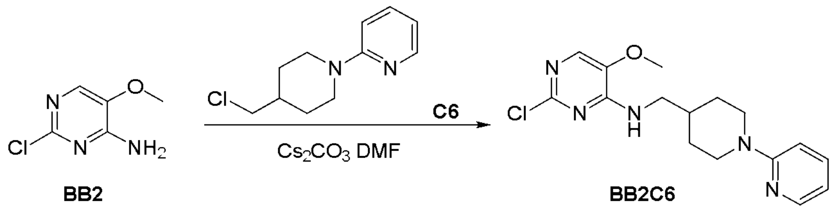


BB2 BB2C6

In accordance with the same steps of intermediate **BB1C2,** intermediate **BB2C6** (100.6 mg, 0.30 mmol, 35.3% yield, a light yellow solid) was obtained using intermediate **BB2** and compound **C6** as starting materials. LC-MS: m/z = 344.1 $(M+H)^+$.

**Synthesis of intermediate BB2C9 (2-chloro-5-methoxy-N-(((1R,4R)-4-(1-methyl-4-(trifluoromethyl)-1H-imidazol-2-yl)cyclohexyl)methyl)pyrimidin-4-amine)**

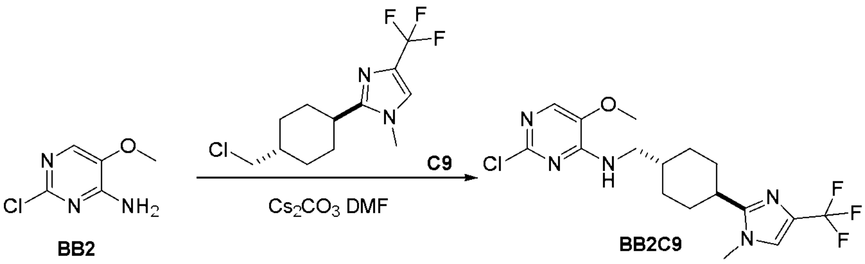


BB2 BB2C9

In accordance with the same steps of intermediate **BB1C2,** intermediate **BB2C9** (31.1 mg, 0.077 mmol, 13.2% yield, a light yellow solid) was obtained using intermediate **BB2** and compound **C9** as starting materials. LC-MS: m/z = 404.2 $(M+H)^+$.

**Synthesis of intermediate BB2C11 (2-chloro-5-methoxy-N-(4-(5-methyl-3-(trifluoromethyl)-1H-pyrazol-1-yl)benzyl)pyrimidin-4-amine)**

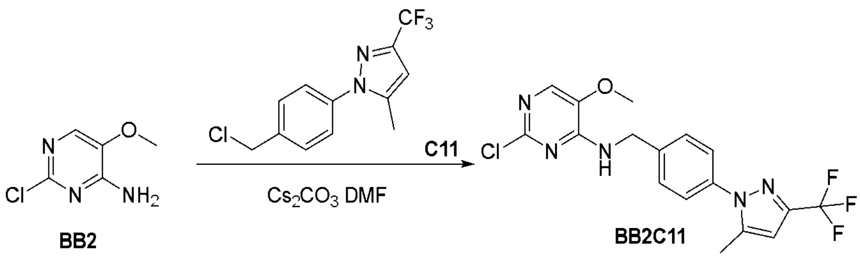


BB2 BB2C11

In accordance with the same steps of intermediate **BB1C2,** intermediate **BB2C11** (5.6 g, 14.10 mmol, 77.8% yield, a white solid) was obtained using intermediate **BB2** and compound **C11** as starting materials. $^1H$ NMR (400 MHz, $CDCl_3$) δ 7.49 (s, 1H), 7.43 - 7.47 (m, 4H), 6.47 - 6.49 (m, 1H), 5.76 (s, 1H), 4.75 - 4.82 (m, 2H), 3.90 (s, 3H), 2.36 (s, 3H); LC-MS: m/z = 398.0 $(M+H)^+$.

**Synthesis of intermediate BB2C12 (2-chloro-N-(4-(imidazo[1,2-a]pyrazin-8-yl)benzyl)-5-methoxypyrimidin-4-amine)**

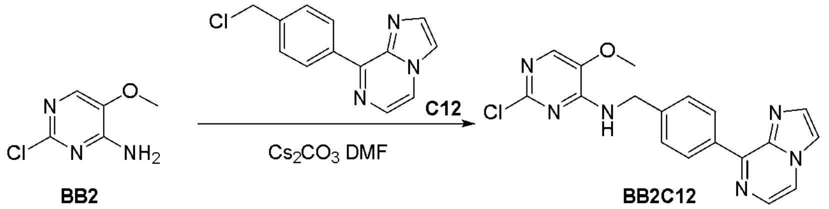


BB2 BB2C12

In accordance with the same steps of intermediate **BB1C2,** intermediate **BB2C12** (130.3 mg, 0.36 mmol, 88.2% yield, a light yellow solid) was obtained using intermediate **BB2** and compound **C12** as starting materials. $^1$H NMR (400 MHz, DMSO) δ 8.71 (d, *J* = 8.4 Hz, 2H), 8.60 (d, *J* = 4.4 Hz, 1H), 8.19 - 8.21 (m, 2H), 7.99 (d, *J* = 4.4 Hz, 1H), 7.87 (s, 1H), 7.72 (s, 1H), 7.46 (d, *J* = 8.4 Hz, 2H), 4.61 (d, *J* = 4.4 Hz, 2H), 3.88 (s, 3H); LC-MS: m/z = 367.0 $(M+H)^+$.

**Synthesis of intermediate BB2C13 (2-chloro-5-methoxy-N-(4-(5-(trifluoromethyl)pyridin-2-yl)benzyl)pyrimidin-4-amine)**

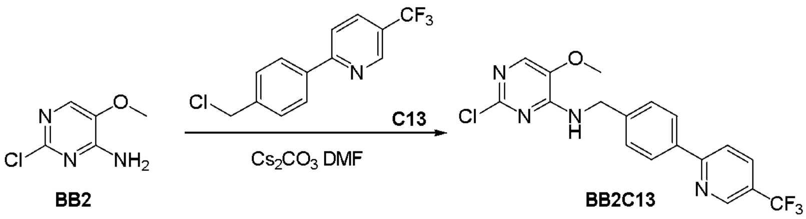


BB2 BB2C13

In accordance with the same steps of intermediate **BB1C2,** intermediate **BB2C13** (455.1 mg, 1.16 mmol, 69.9% yield, a light yellow solid) was obtained using intermediate **BB2** and compound **C13** as starting materials. LC-MS: m/z = 395.0 $(M+H)^+$.

**Synthesis of intermediate BB2C14 (2-chloro-5-methoxy-N-(4-(5-methylpyridin-2-yl)benzyl)pyrimidin-4-amine)**

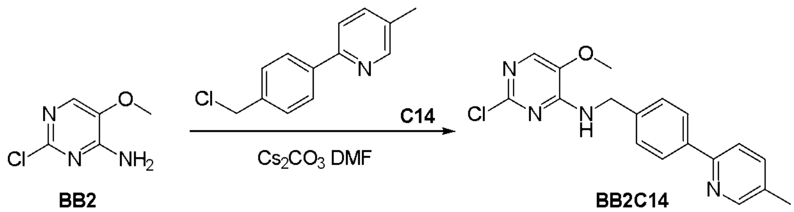


BB2 BB2C14

In accordance with the same steps of intermediate **BB1C2,** intermediate **BB2C14** (173.45 mg, 0.51 mmol, 90.3% yield, a light yellow solid) was obtained using intermediate **BB2** and compound **C14** as starting materials. LC-MS: m/z = 341.1 $(M+H)^+$.

**Synthesis of intermediate BB2C19 (2-chloro-5-methoxy-N-((4-(5-(trifluoromethyl)pyridin-2-yl)bicyclo [2.2.2] octan-1-yl)methyl)pyrimidin-4-amine)**

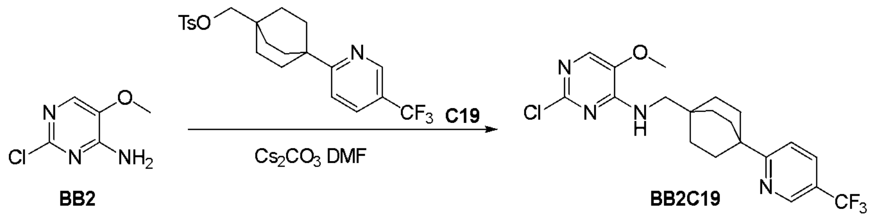


BB2 BB2C19

In accordance with the same steps of intermediate **BB1C2,** intermediate **BB2C19** (281.3 mg, 0.66 mmol, 35.6% yield, a light yellow solid) was obtained using intermediate **BB2** and compound **C19** as starting materials. LC-MS: m/z = 427.3 $(M+H)^+$.

**Synthesis of intermediate BB2C26 (6-((2-chloro-5-methoxypyrimidin-4-yl)amino)methyl)-2-isopropyl-3,4-dihydroisoquinolin-1(2H)-one)**

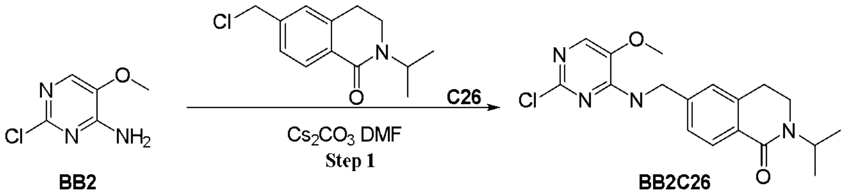


BB2 BB2C26

In accordance with the same steps of intermediate **BB1C2,** intermediate **BB2C26** (379.2 mg, 1.05 mmol, 82.0% yield, a light yellow solid) was obtained using intermediate **BB2** and compound **C26** as starting materials. LC-MS: m/z = 361.1 $(M+H)^+$.

**Synthesis of intermediate BB2C27 (2-chloro-5-methoxy-N-(4-(pyridin-2-ylmethoxy)benzyl)pyrimidin-4-amine)**

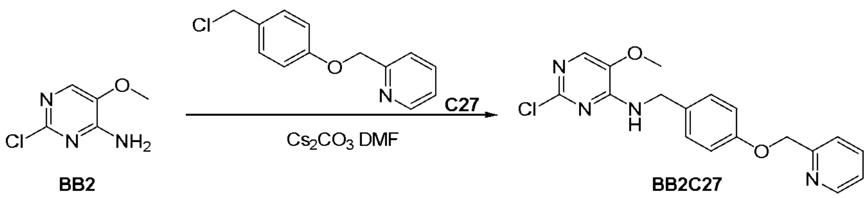


BB2 BB2C27

In accordance with the same steps of intermediate **BB1C2,** intermediate **BB2C27** (268.5 mg, 0.61 mmol, 93.6% yield, a light yellow solid) was obtained using intermediate **BB2** and compound **C27** as starting materials. $^1$H NMR (400 MHz, $CDCl_3$) δ 8.62 (d, *J* = 4.8Hz, 1H), 7.72 - 7.76 (m, 1H), 7.72 - 7.75 (m, 2H), 7.29 (s, 1H), 7.24 - 7.25 (m, 1H), 6.99 (d, *J*= 8.4Hz, 2H), 5.61 (s, 1H), 5.23 (s, 2H), 4.60 (d, *J* = 5.6Hz, 2H), 3.85 (s, 3H); LC-MS: m/z = 357.0 $(M+H)^+$.

**Synthesis of intermediate BB2C28 (2-chloro-N-(4-(2-ethoxyethoxy)benzyl)-5-methoxypyrimidin-4-amine)**

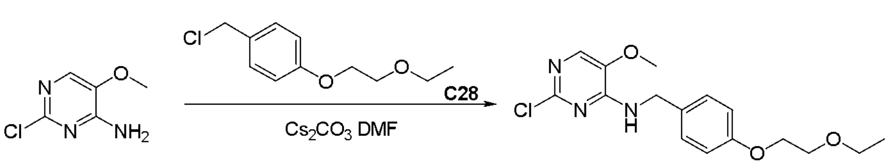


BB2 BB2C28

In accordance with the same steps of intermediate **BB1C2,** intermediate **BB2C28** (316.9 mg, 0.94 mmol, 78.8% yield, a white solid) was obtained using intermediate **BB2** and compound **C28** as starting materials. $^1$H NMR (400 MHz, $CDCl_3$) δ 7.53 (s, 1H), 7.24 - 7.29 (m, 2H), 6.89 - 6.94 (m, 2H), 5.61 (s, 1H), 4.59 (d, *J* = 5.6 Hz, 2H), 4.10 - 4.14 (m, 2H), 3.84

(s, 3H), 3.76 - 3.81 (m, 2H), 3.60 - 3.62 (m, 2H), 1.25 (t, *J* = 6.8 Hz, 3H); LC-MS: m/z = 338.1 $(M+H)^+$.

**Synthesis of intermediate BB2C30 (2-chloro-5-methoxy-N-(2-methoxy-4-(1-methyl-4-(trifluoromethyl)-1H-imidazol-2-yl)benzyl)pyrimidin-4-amine)**

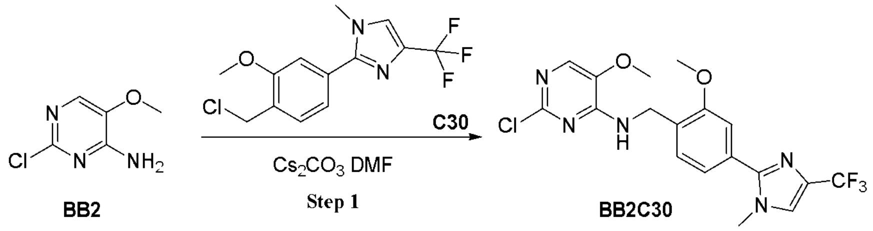


BB2 BB2C30

In accordance with the same steps of intermediate **BB1C2,** intermediate **BB2C30** (662.0 mg, 1.55 mmol, 86.7% yield, a light yellow solid) was obtained using intermediate **BB2** and compound **C30** as starting materials. LC-MS: m/z = 428.0 $(M+H)^+$.

**Synthesis of intermediate BB2C31 (2-chloro-5-methoxy-N-(3-methoxy-4-(1-methyl-4-(trifluoromethyl)-1H-imidazol-2-yl)benzyl)pyrimidin-4-amine)**

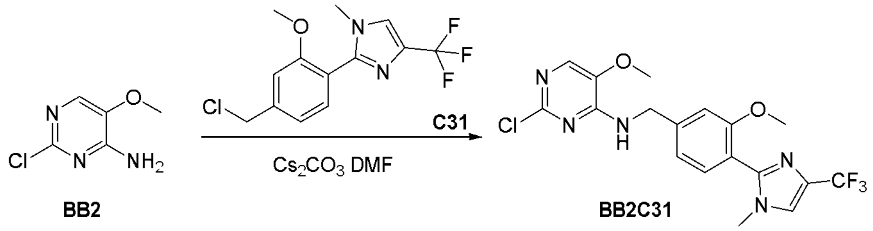


BB2 BB2C31

In accordance with the same steps of intermediate **BB1C2,** intermediate **BB2C31** (337.4 mg, 0.79 mmol, 93.9% yield, a white solid) was obtained using intermediate **BB2** and compound **C31** as starting materials. LC-MS: m/z = 428.1 $(M+H)^+$.

**Synthesis of intermediate BB2C33 (2-chloro-5-methoxy-N-(3-methoxy-4-(5-methyl-3-(trifluoromethyl)-1H-pyrazol-1-yl)benzyl)pyrimidin-4-amine)**

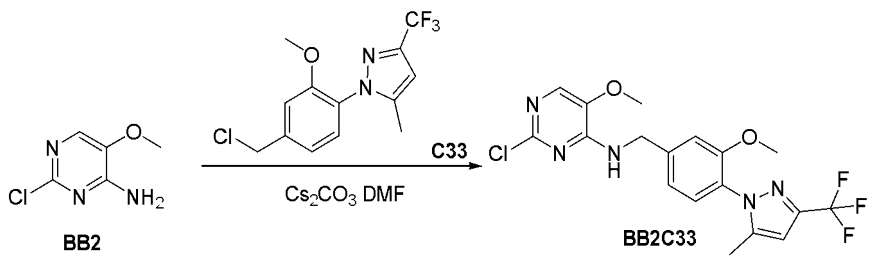


BB2 BB2C33

In accordance with the same steps of intermediate **BB1C2,** intermediate **BB2C33** (3.02 g, 2.66 mmol, 88.3% yield, a white solid) was obtained using intermediate **BB2** and compound **C33** as starting materials. LC-MS: m/z = 428.0 $(M+H)^+$.

**Synthesis of intermediate BB2C35 (2-chloro-5-methoxy-N-((4-(1-methyl-4-(trifluoromethyl)-1H-imidazol-2-yl) bicyclo [2.2.2] octan-1-yl)methyl)pyrimidin-4-amine)**

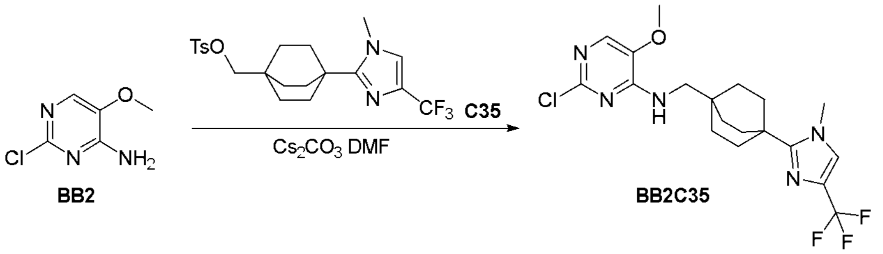


Compound **BB2** (1.0 g, 6.29 mmol, 1.00 eq), compound **C35** (2.78 g, 6.29 mmol, 1.0 eq) and cesium carbonate (10.2 g, 31.45 mmol, 5.0 eq) were added to N,N-dimethylformamide (20.0 mL) successively, and the resulting mixture was stirred at 130°C for 36 hours. The reaction solution was cooled to 25°C. Dichloromethane (100.0 mL) was added, the resulting mixture was stirred for 5 minutes, and filtered to obtain a filtrate. The filtrate was washed three times with saturated brine (50.0 mL), dried over anhydrous sodium sulfate, and concentrated under vacuum to obtain a crude product, which was purified by column chromatography (eluent: petroleum ether/dichloromethane = 1/0 - 0/1) to obtain intermediate **BB2C35** (1.03 g, 2.41 mmol, 38.3% yield) as a white solid. LC-MS: m/z = 430.1 $(M+H)^+$.

**Synthesis of intermediate BB2C48 (2-chloro-N-(4-(4-(difluoromethyl)-1-methyl-1H-imidazol-2-yl)benzyl)-5-methoxypyrimidin-4-amine)**

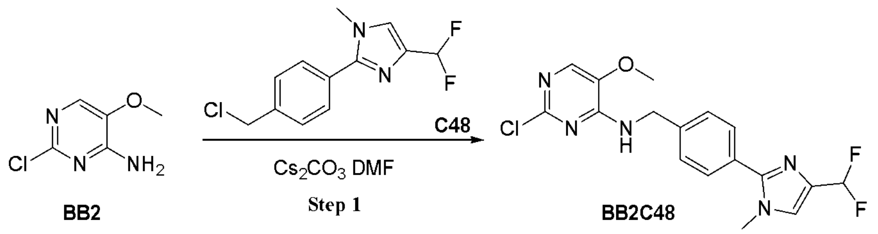


In accordance with the same steps of intermediate **BB1C2,** intermediate **BB2C48** (371.5 mg, 0.98 mmol, 77.5% yield, a white solid) was obtained using intermediate **BB2** and compound **C48** as starting materials. LC-MS: m/z = 380.3 $(M+H)^+$.

**Synthesis of intermediate BB2C58 (2-chloro-N-(4-(1-isopropyl-4-(trifluoromethyl)-1H-imidazol-2-yl)benzyl)-5-methoxypyrimidin-4-amine)**

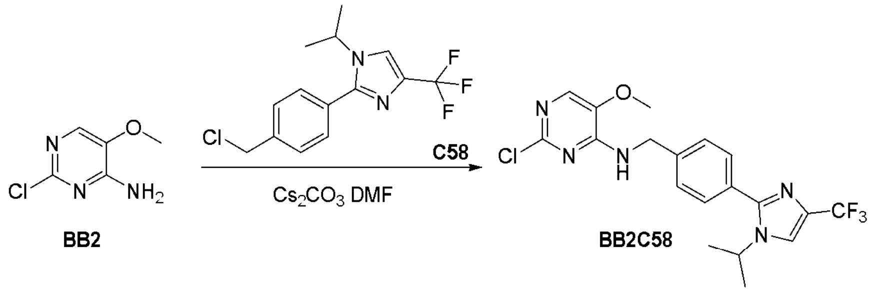


In accordance with the same steps of intermediate **BB1C2,** intermediate **BB2C58** (357.1 mg, 0.84 mmol, 97.0% yield, a light yellow solid) was obtained using intermediate **BB2** and compound **C58** as starting materials. $^1H$ NMR (400 MHz, DMSO-$d_6$) δ 8.31 - 8.15 (m, 2H), 7.75 (s, 1H), 7.54 (d, *J* = 8.1 Hz, 2H), 7.45 (d, *J* = 8.1 Hz, 2H), 4.63 (d, *J* = 6.3 Hz, 2H),

4.56 - 4.38 (m, 1H), 3.90 (s, 3H), 1.42 (d, *J* = 6.6 Hz, 6H); LC-MS: m/z = 426.1 $(M+H)^+$.

**Synthesis of intermediate BB2C59 (2-chloro-N-(4-(1-(fluoromethyl)-4-(trifluoromethyl)-1H-imidazol-2-yl)benzyl)-5-methoxypyrimidin-4-amine)**

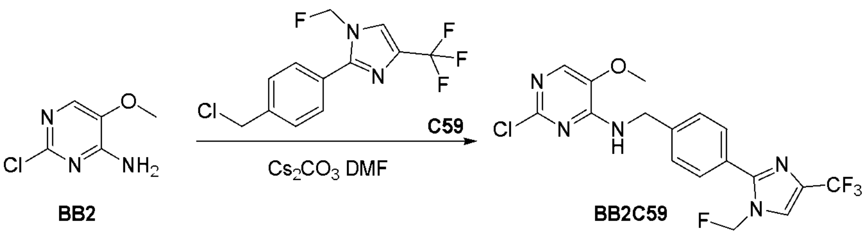


BB2 BB2C59

In accordance with the same steps of intermediate **BB1C2,** intermediate **BB2C59** (91.3 mg, 0.22 mmol, 78.8% yield, a white solid) was obtained using intermediate **BB2** and compound C59 as starting materials. LC-MS: m/z = 426.1 $(M+H)^+$.

**Synthesis of intermediate BB3C4 (2-chloro-5-isopropoxy-N-methyl-N-((1-(1-methyl-4-(trifluoromethyl)-1H-imidazol-2-yl)piperidin-4-yl)methyl)pyrimidin-4-amine)**

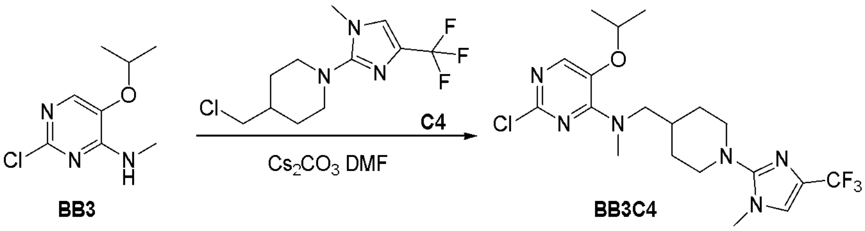


BB3 BB3C4

In accordance with the same steps of intermediate **BB2C35,** intermediate **BB3C4** (178.5 mg, 0.40 mmol, 28.7% yield, a white solid) was obtained using intermediate **BB3** and compound **C4** as starting materials. LC-MS: m/z = 447.3 $(M+H)^+$.

**Synthesis of intermediate BB3CS (2-chloro-5-isopropoxy-N-methyl-N-(4-(1-methyl-4-(trifluoromethyl)-1H-imidazol-2-yl)benzyl)pyrimidin-4-amine)**

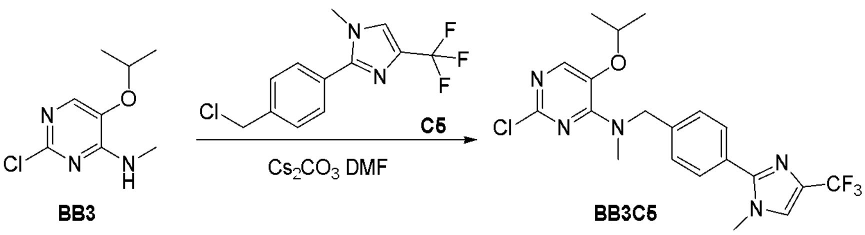


BB3 BB3C5

In accordance with the same steps of intermediate **BB1C2,** intermediate **BB3C5** (2.1 g, 4.78 mmol, 86.4% yield, a white solid) was obtained using intermediate **BB3** and compound **C5** as starting materials. LC-MS: m/z = 440.2 $(M+H)^+$.

**Synthesis of intermediate BB4C4 (2-chloro-5-isopropoxy-N-((1-(1-methyl-4-(trifluoromethyl)-1H-imidazol-2-yl) piperidin-4-yl)methyl)pyrimidin-4-amine)**

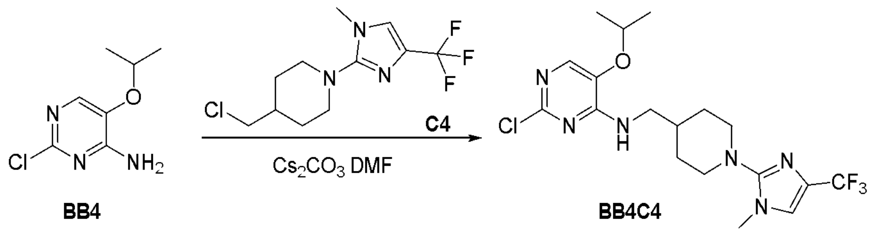


BB4 BB4C4

In accordance with the same steps of intermediate **BB2C35,** intermediate **BB4C4** (159.9 mg, 0.37 mmol, 39.6% yield, a white solid) was obtained using intermediate **BB4** and compound **C4** as starting materials. LC-MS: m/z = 433.2 $(M+H)^+$.

**Synthesis of intermediate BB4C5 (2-chloro-5-isopropoxy-N-(4-(1-methyl-4-(trifluoromethyl)-1H-imidazol-2-yl) benzyl)pyrimidin-4-amine)**

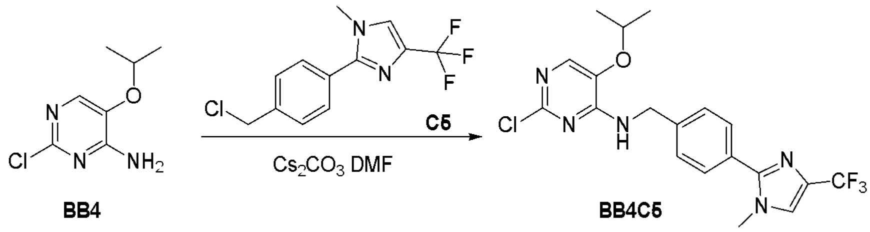


BB4 BB4C5

In accordance with the same steps of intermediate **BB1C2,** intermediate **BB4C5** (2.03 g, 4.78 mmol, 86.4% yield, a white solid) was obtained using intermediate **BB4** and compound **C5** as starting materials. LC-MS: m/z = 426.1 $(M+H)^+$.

**Synthesis of intermediate BB4C19 (2-chloro-5-isopropoxy-N-((4-(5-(trifluoromethyl)pyridin-2-yl)bicyclo [2.2.2] octan-1-yl)methyl)pyrimidin-4-amine)**

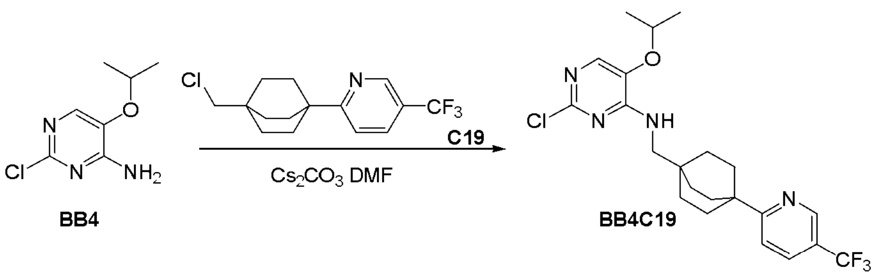


BB4 BB4C19

In accordance with the same steps of intermediate **BB1C2,** intermediate **BB4C19** (349.7 mg, 0.77 mmol, 83.2% yield, a white solid) was obtained using intermediate **BB4** and compound **C19** as starting materials. LC-MS: m/z = 455.2 $(M+H)^+$.

**Synthesis of intermediate BB12C2 (2-chloro-N-((4-(pyridin-2-yl)cyclohexyl)methyl)-5-(trifluoromethyl)pyrimidin-4-amine)**

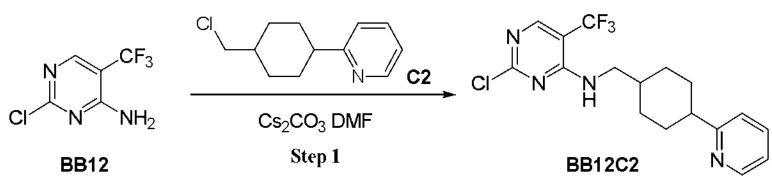


BB12 BB12C2

In accordance with the same steps of intermediate **BB2C35,** intermediate **BB12C2** (51.8 mg, 0.14 mmol, 35.4% yield, a white solid) was obtained using intermediate **BB12** and compound **C2** as starting materials. LC-MS: m/z = 371.1 $(M+H)^+$.

**Synthesis of intermediate BB12C3 (2-chloro-N-(4-(pyridin-2-yl)benzyl)-5-(trifluoromethyl)pyrimidin-4-amine)**

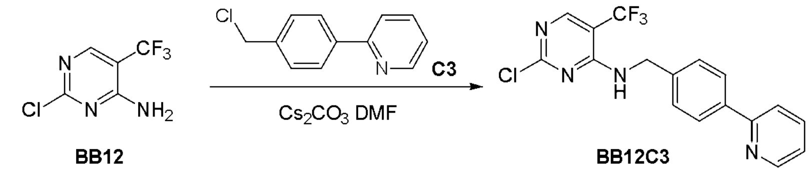


In accordance with the same steps of intermediate **BB1C2,** intermediate **BB12C3** (396.8 mg, 1.09 mmol, 76.6% yield, a light yellow solid) was obtained using intermediate **BB12** and compound **C3** as starting materials. LC-MS: m/z = 365.0 $(M+H)^+$.

**Synthesis of intermediate BB12C4 (2-chloro-N-((1-(1-methyl-4-(trifluoromethyl)-1H-imidazol-2-yl)piperidin-4-yl)methyl)-5-(trifluoromethyl)pyrimidin-4-amine)**

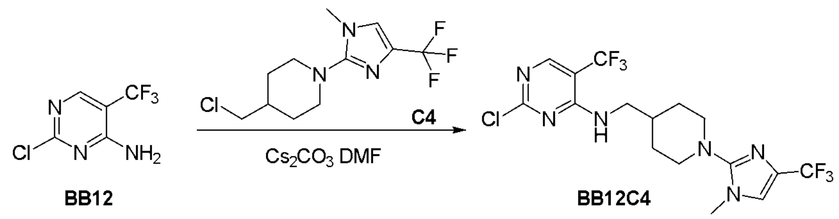


In accordance with the same steps of intermediate **BB2C35,** intermediate **BB12C4** (53.1 mg, 0.12 mmol, 21.1% yield, a light yellow oil) was obtained using intermediate **BB12** and **C4** as starting materials. LC-MS: m/z = 443.0 $(M+H)^+$.

**Synthesis of intermediate BB12C5 (2-chloro-N-(4-(1-methyl-4-(trifluoromethyl)-1H-imidazol-2-yl)benzyl)-5-(trifluoromethyl)pyrimidin-4-amine)**

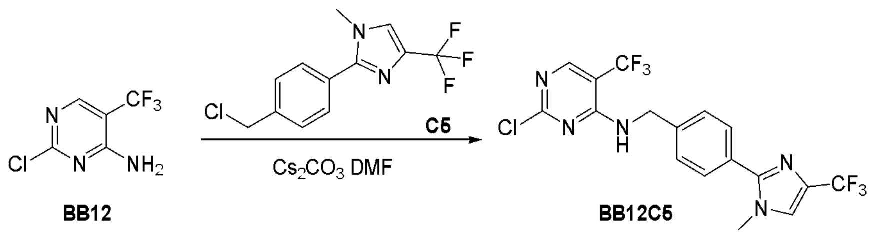


In accordance with the same steps of intermediate **BB1C2,** intermediate **BB12C5** (248.0 mg, 0.57 mmol, 88.8% yield, a light yellow solid) was obtained using intermediate **BB12** and compound **C5** as starting materials. LC-MS: m/z = 436.1 $(M+H)^+$.

**Synthesis of intermediate BB12C6 (2-chloro-N-((1-(pyridin-2-yl)piperidin-4-yl)methyl)-5-(trifluoromethyl)pyrimidin-4-amine)**

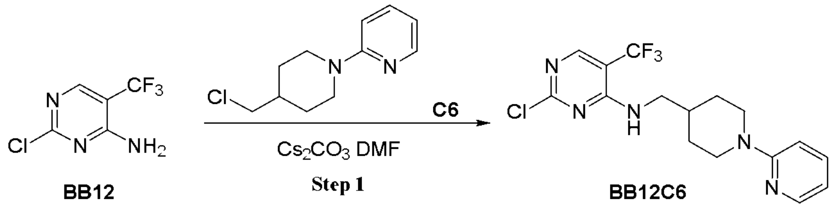


In accordance with the same steps of intermediate **BB2C35,** intermediate **BB12C6** (29.7 mg, 0.08 mmol, 18.0% yield, a light yellow oil) was obtained using intermediate **BB12** and compound **C6** as starting materials. LC-MS: m/z = 372.3 $(M+H)^+$.

**Synthesis of intermediate BB13C3 (2-chloro-N-(4-(pyridin-2-yl)benzyl)furo [3,2-d] pyrimidin-4-amine)**

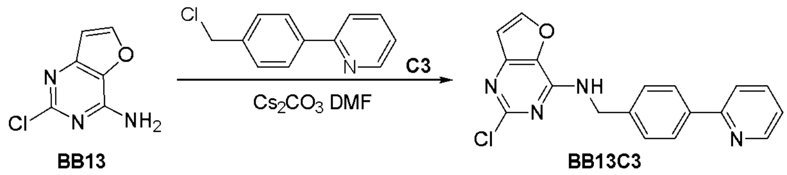


In accordance with the same steps of intermediate **BB1C2,** intermediate **BB13C3** (1.19 g, 3.55 mmol, 89.4% yield, a light yellow solid) was obtained using intermediate **BB13** and compound **C3** as starting materials. LC-MS: m/z = 337.1 $(M+H)^+$.

**Synthesis of intermediate BB13C5 (2-chloro-N-(4-(1-methyl-4-(trifluoromethyl)-1H-imidazol-2-yl)benzyl)furo [3,2-d] pyrimidin-4-amine)**

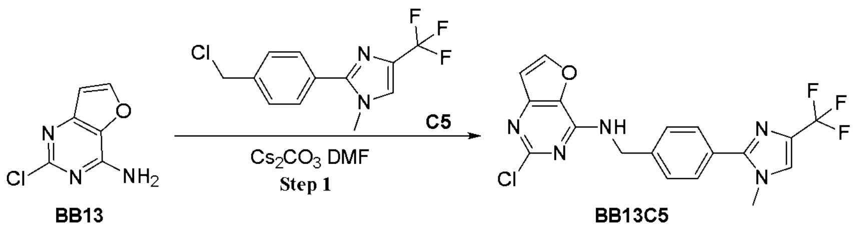


In accordance with the same steps of intermediate **BB1C2,** intermediate **BB13C5** (761.1 mg, 1.87 mmol, 76.7% yield, a light yellow solid) was obtained using intermediate **BB13** and compound **C5** as starting materials. $^1H$ NMR (400 MHz, DMSO) δ 9.00 (s, 1H), 8.29-8.33 (m, 1H), 7.90-7.94 (m, 1H), 7.67-7.72 (m, 2H), 7.46-7.52 (m, 2H), 6.96-7.00 (m, 1H), 4.74 (s, 2H), 3.77 (s, 3H); C-MS: m/z = 407.9 $(M+H)^+$.

**Synthesis of intermediate BB13C7 (2-chloro-N-((5-(1-methyl-4-(trifluoromethyl)-1H-imidazol-2-yl)thiophen-2-yl)methyl)furo [3,2-d] pyrimidin-4-amine)**

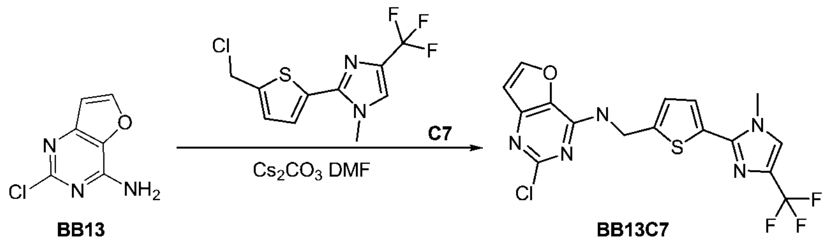

In accordance with the same steps of intermediate **BB1C2,** intermediate **BB13C7** (342.6 mg, 0.83 mmol, 56.9% yield, a white solid) was obtained using intermediate **BB13** and compound **C7** as starting materials. LC-MS: m/z = 414.0 $(M+H)^+$.

**Synthesis of intermediate BB13C11 (2-chloro-N-(4-(5-methyl-3-(trifluoromethyl)-1H-pyrazol-1-yl)benzyl)furo [3,2-d] pyrimidin-4-amine)**

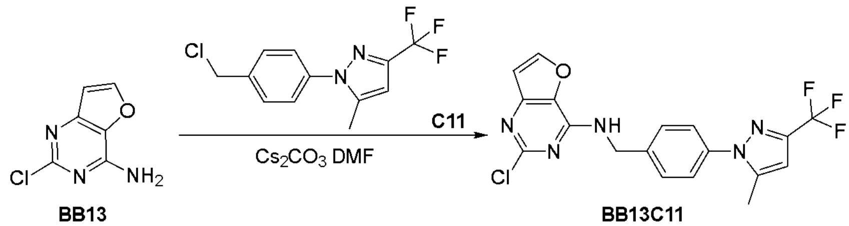


BB13 BB13C11

In accordance with the same steps of intermediate **BB1C2,** intermediate **BB13C11** (4.23 g, 10.39 mmol, 76.9% yield, a light yellow solid) was obtained using intermediate **BB13** and compound **C11** as starting materials. LC-MS: m/z = 408.0 $(M+H)^+$.

**Synthesis of intermediate BB13C12 (2-chloro-N-(4-(imidazo[1,2-a]pyrazin-8-yl)benzyl)furo [3,2-d] pyrimidin-4-amine)**

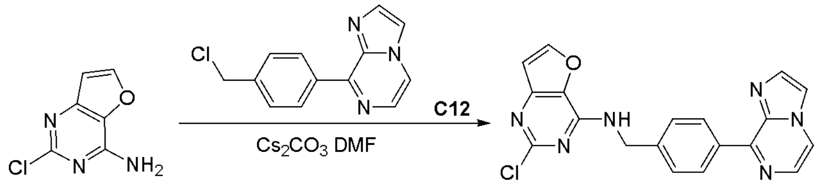


BB13 BB13C12

In accordance with the same steps of intermediate **BB1C2,** intermediate **BB13C12** (458.8 mg, 1.22 mmol, 88.7% yield, a white solid) was obtained using intermediate **BB13** and compound **C12** as starting materials. $^1H$ NMR (400 MHz, DMSO) δ 9.07 (s, 1H), 8.74 (d, *J* = 8.4Hz, 2H), 8.60 (d, *J* = 4.4Hz, 1H), 8.27 - 8.31 (m, 1H), 8.21 (s, 1H), 7.99 (d, *J* = 8.4Hz, 1H), 7.87 (s, 1H), 7.53 (d, *J* = 8.0Hz, 2H), 6.93-6.98 (m, 1H), 4.76 (s, 2H); LC-MS: m/z = 377.1 $(M+H)^+$.

**Synthesis of intermediate BB13C19 (2-chloro-N-((4-(5-(trifluoromethyl)pyridin-2-yl)bicyclo [2.2.2] octan-1-yl) methyl)furo [3,2-d]pyrimidin-4-amine)**

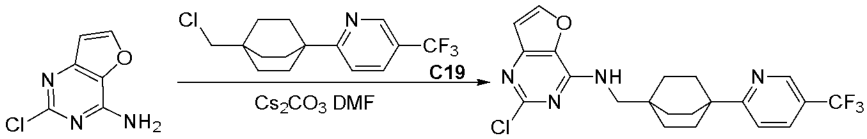


BB13 BB13C19

In accordance with the same steps of intermediate **BB2C35,** intermediate **BB13C19** (383.8 mg, 0.88 mmol, 22.3% yield, a light yellow oil) was obtained using intermediate **BB13** and compound **C19** as starting materials. LC-MS: m/z = 437.0 $(M+H)^+$.

**Synthesis of intermediate BB13C25 (6-(((2-chlorofuro[3,2-d]pyrimidin-4-yl)amino)methyl)-2-methyl-3,4-dihydroisoquinolin-1(2H)-one)**

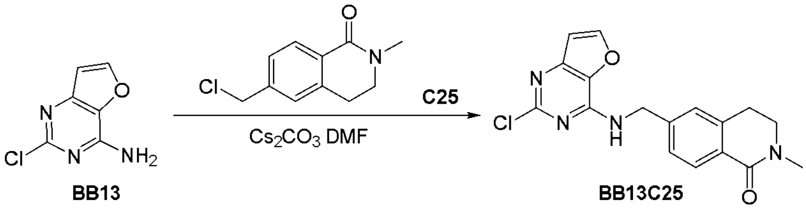


BB13 BB13C25

In accordance with the same steps of intermediate **BB1C2,** intermediate **BB13C25** (195.0 mg, 0.57 mmol, 90.4% yield, a white solid) was obtained using intermediate **BB13** and compound **C25** as starting materials. $^1$H NMR (400 MHz, DMSO) δ 8.94 (s, 1H), 8.30 (s, 1H), 7.91 (d, *J*=8.0Hz, 1H), 7.29 (d, *J*=8.0Hz, 1H), 7.24 (s, 1H), 6.97 (d, *J*=2.0Hz, 1H), 4.69 (s, 2H),3.52 (t, $J_1$=6.4Hz, $J_2$=6.8Hz, 2H), 3.00 (s, 3H), 2.95 (t, $J_1$=6.4Hz, $J_2$=6.8Hz, 2H); LC-MS: m/z = 343.2 $(M+H)^+$.

**Synthesis of intermediate BB13C26 (6-(((2-chlorofuro[3,2-d]pyrimidin-4-yl)amino)methyl)-2-isopropyl-3,4-dihydroisoquinolin-1(2H)-one)**

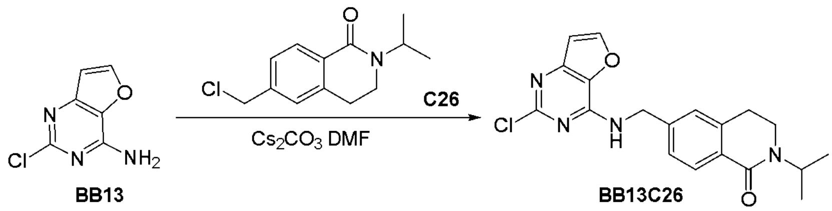


BB13 BB13C26

In accordance with the same steps of intermediate **BB1C2,** intermediate **BB13C26** (233.1 mg, 0.63 mmol, 82.6% yield, a light yellow solid) was obtained using intermediate **BB13** and compound C26 as starting materials. LC-MS: m/z = 371.2 $(M+H)^+$.

**Synthesis of intermediate BB13C27 (2-chloro-N-(4-(pyridin-2-ylmethoxy)benzyl)furo[3,2-d]pyrimidin-4-amine)**

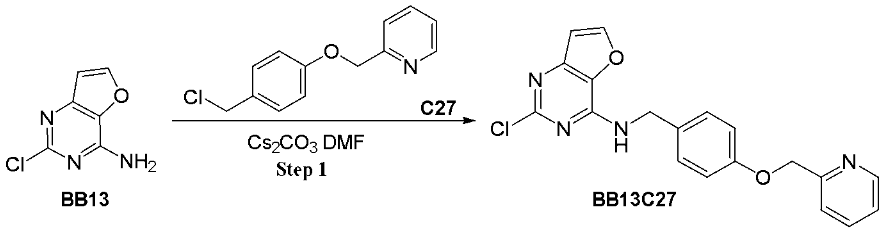


BB13 BB13C27

In accordance with the same steps of intermediate **BB1C2,** intermediate **BB13C27** (256.3 mg, 0.70 mmol, 66.8% yield, a light yellow solid) was obtained using intermediate **BB13** and compound **C27** as starting materials. LC-MS: m/z = 366.9 $(M+H)^+$.

**Synthesis of intermediate BB13C28 (2-chloro-N-(4-(2-ethoxyethoxy)benzyl)furo[3,2-d]pyrimidin-4-amine)**

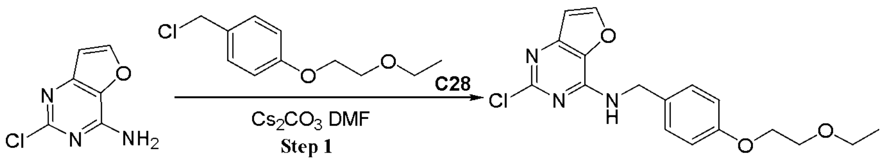


BB13 BB13C28

In accordance with the same steps of intermediate **BB1C2,** intermediate **BB13C28** (118.0 mg, 0.34 mmol, 84.5% yield, a light yellow solid) was obtained using intermediate **BB13** and **C28** as starting materials. LC-MS: m/z = 348.2 $(M+H)^+$.

**Synthesis of intermediate BB13C29 (2-chloro-N-(4-(3-methoxycyclobutoxy)benzyl)furo[3,2-d]pyrimidin-4-amine)**

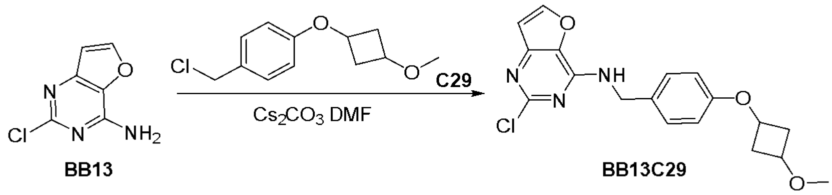


BB13 BB13C29

In accordance with the same steps of intermediate **BB1C2,** intermediate **BB13C29** (244.2 mg, 0.68 mmol, 93.5% yield, a light yellow solid) was obtained using intermediate **BB13** and compound **C29** as starting materials. LC-MS: m/z = 360.2 $(M+H)^+$.

**Synthesis of intermediate BB13C30 (2-chloro-N-(2-methoxy-4-(1-methyl-4-(trifluoromethyl)-1H-imidazol-2-yl) benzyl)furo[3,2-d]pyrimidin-4-amine)**

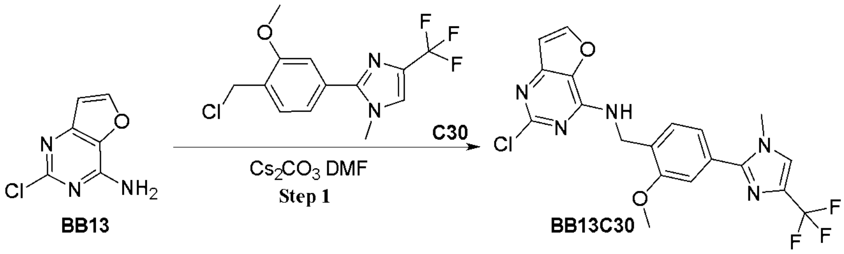


BB13 BB13C30

In accordance with the same steps of intermediate **BB1C2,** intermediate **BB13C30** (292.9 mg, 0.67 mmol, 78.8% yield, a white solid) was obtained using intermediate **BB13** and compound **C30** as starting materials. LC-MS: m/z = 438.1 $(M+H)^+$.

**Synthesis of intermediate BB13C31 (2-chloro-N-(3-methoxy-4-(1-methyl-4-(trifluoromethyl)-1H-imidazol-2-yl) benzyl)furo [3,2-d] pyrimidin-4-amine)**

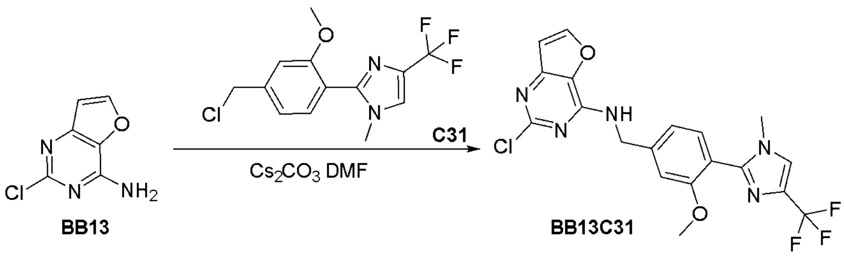

BB13
BB13C31

In accordance with the same steps of intermediate **BB1C2,** intermediate **BB13C31** (214.2 mg, 0.49 mmol, 73.9% yield, a white solid) was obtained using intermediate **BB13** and compound **C31** as starting materials. LC-MS: m/z = 438.0 $(M+H)^+$.

**Synthesis of intermediate BB13C32 (2-chloro-N-(2-methoxy-4-(5-methyl-3-(trifluoromethyl)-1H-pyrazol-1-yl) benzyl)furo [3,2-d] pyrimidin-4-amine)**

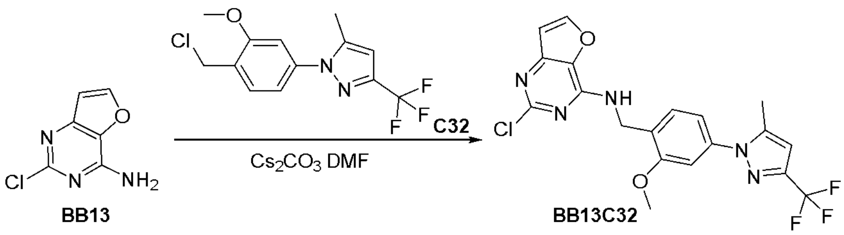

BB13
BB13C32

In accordance with the same steps of intermediate **BB1C2,** intermediate **BB13C32** (100.5 mg, 0.23 mmol, 70.0% yield, a white solid) was obtained using intermediate **BB13** and compound **C32** as starting materials. LC-MS: m/z = 438.0 $(M+H)^+$.

**Synthesis of intermediate BB13C33 (2-chloro-N-(3-methoxy-4-(5-methyl-3-(trifluoromethyl)-1H-pyrazol-1-yl) benzyl)furo [3,2-d] pyrimidin-4-amine)**

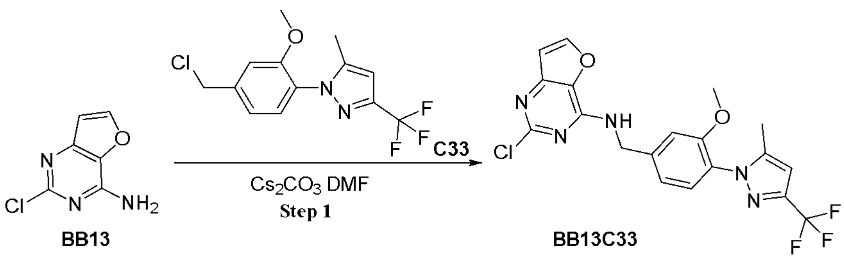

BB13
BB13C33

In accordance with the same steps of intermediate **BB1C2,** intermediate **BB13C33** (257.6 mg, 0.59 mmol, 84.2% yield, a light yellow solid) was obtained using intermediate **BB13** and compound **C33** as starting materials. LC-MS: m/z = 437.9 $(M+H)^+$.

**Synthesis of intermediate BB13C35 (2-chloro-N-((4-(1-methyl-4-(trifluoromethyl)-1H-imidazol-2-yl)bicyclo [2.2.2]octan-1-yl)methyl)furo[3,2-d]pyrimidin-4-amine)**

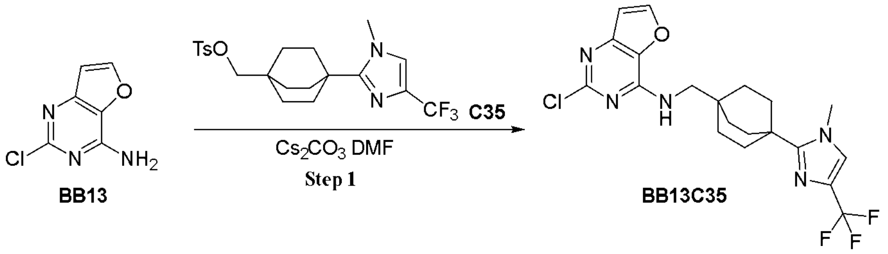


In accordance with the same steps of intermediate **BB2C35,** intermediate **BB13C35** (48.3 mg, 0.11 mmol, 28.8% yield, a white solid) was obtained using intermediate **BB13** and compound **C35** as starting materials. LC-MS: m/z = 440.2 $(M+H)^+$.

**Synthesis of intermediate BB13C48 (2-chloro-N-(4-(4-(difluoromethyl)-1-methyl-1H-imidazol-2-yl)benzyl)furo [3,2-d]pyrimidin-4-amine)**

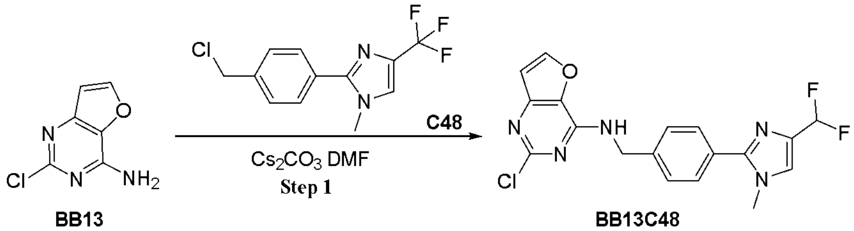


In accordance with the same steps of intermediate **BB1C2,** intermediate **BB13C48** (886.2 mg, 2.28 mmol, 77.9% yield, a white solid) was obtained using intermediate **BB13** and **C48** as starting materials. LC-MS: m/z = 390.1 $(M+H)^+$.

**Synthesis of intermediate BB14C5 (2-chloro-N-(4-(1-methyl-4-(trifluoromethyl)-1H-imidazol-2-yl)benzyl)pyrido [3,2-d]pyrimidin-4-amine)**

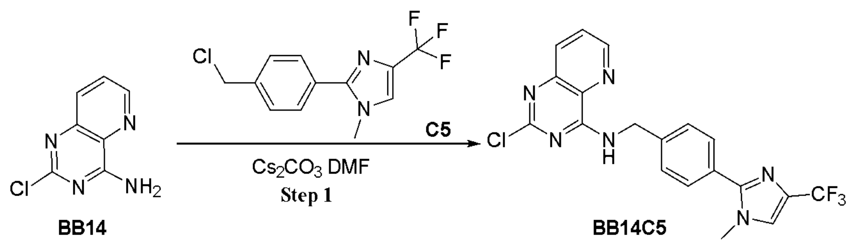


In accordance with the same steps of intermediate **BB1C2,** intermediate **BB14C5** (1.22 g, 2.92 mmol, 93.3% yield, a light yellow solid) was obtained using intermediate **BB14** and compound **C5** as starting materials. LC-MS: m/z = 419.1 $(M+H)^+$.

**Synthesis of intermediate BB15C5 (2,5-dichloro-N-(4-(1-methyl-4-(trifluoromethyl)-1H-imidazol-2-yl)benzyl) pyrimidin-4-amine)**

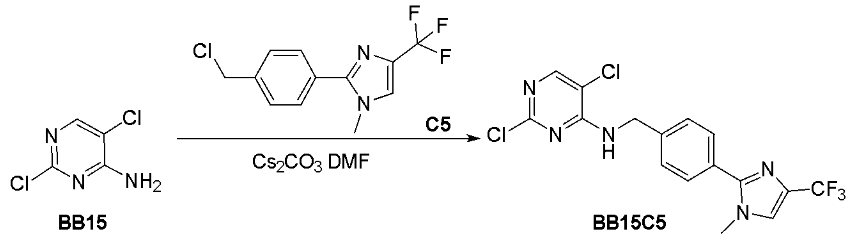


BB15 BB15C5

In accordance with the same steps of intermediate **BB1C2,** intermediate **BB15C5** (398.7 mg, 0.99 mmol, 80.4% yield, a white solid) was obtained using intermediate **BB15** and compound C5 as starting materials. $^1$H NMR (400 MHz, DMSO-d6) δ 8.60 (s, 1H), 7.96 (d, J = 1.3 Hz, 1H), 7.87 - 7.68 (m, 2H), 7.45 (d, J = 8.3 Hz, 2H), 5.00 (s, 2H), 3.21 (s, 3H); LC-MS: m/z = 402.0 (M+H)$^+$.

**Synthesis of intermediate BB15C59 (2,5-dichloro-N-(4-(1-(fluoromethyl)-4-(trifluoromethyl)-1H-imidazol-2-yl)benzyl)pyrimidin-4-amine)**

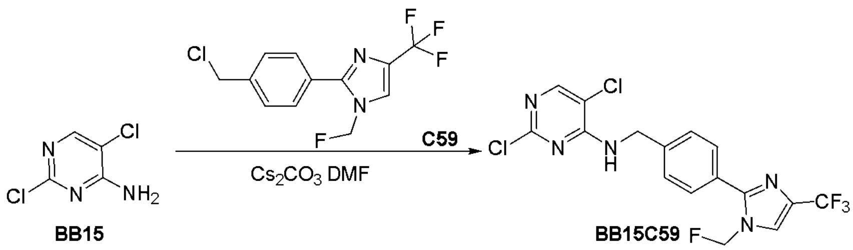


BB15 BB15C59

In accordance with the same steps of intermediate **BB1C2,** intermediate **BB15C59** (124.0 mg, 0.30 mmol, 58.9% yield, a white solid) was obtained using intermediate **BB15** and compound **C59** as starting materials. LC-MS: m/z = 420.0 (M+H)$^+$.

**Synthesis of intermediate BB16C58 (2-chloro-5-fluoro-N-(4-(1-isopropyl-4-(trifluoromethyl)-1H-imidazol-2-yl)benzyl)-N-methylpyrimidin-4-amine)**

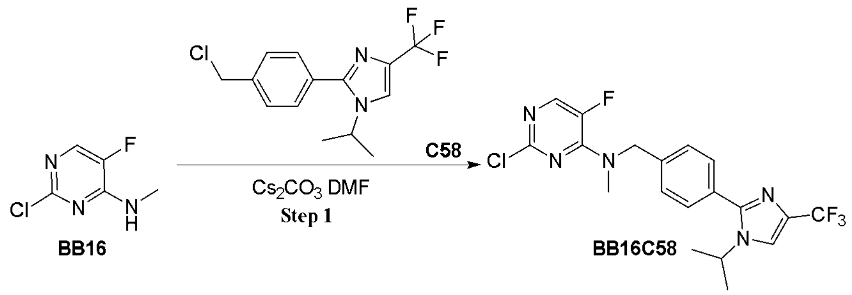


BB16 BB16C58

In accordance with the same steps of intermediate **BB1C2,** intermediate **BB16C58** (6.66 g, 16.69 mmol, 93.3% yield, a light yellow solid) was obtained using intermediate **BB16** and compound **C58** as starting materials. LC-MS: m/z = 428.2 (M+H)$^+$.

**Synthesis of intermediate BB16-D3C57 (2-chloro-N-(4-(1-cyclopropyl-4-(trifluoromethyl)-1H-imidazol-2-yl)benzyl)-5-fluoro-N-(methyl-d3)pyrimidin-4-amine)**

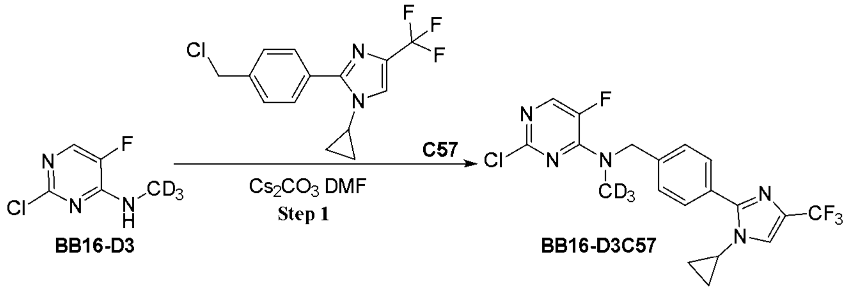


In accordance with the same steps of intermediate **BB1C2,** intermediate **BB16-D3C57** (1.33 g, 3.10 mmol, 89.8% yield, an off-white solid) was obtained using intermediate **BB16-D3** and compound **C57** as starting materials. LC-MS: m/z = 429.3 $(M+H)^+$.

**Synthesis of intermediate BB16-D3C58 (2-chloro-5-fluoro-N-(4-(1-isopropyl-4-(trifluoromethyl)-1H-imidazol-2-yl)benzyl)-N-(methyl-d3)pyrimidin-4-amine)**

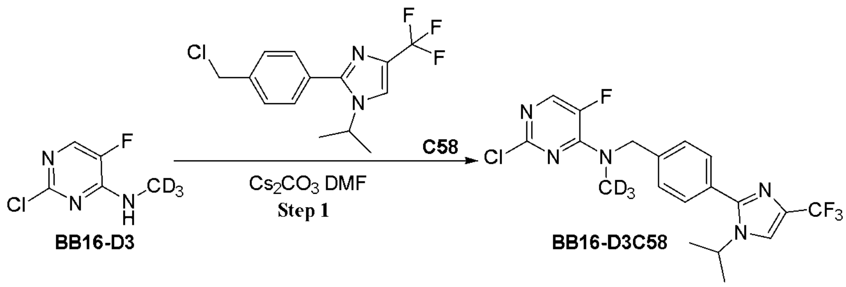


In accordance with the same steps of intermediate **BB1C2,** intermediate **BB16-D3C58** (1.54 g, 3.57 mmol, 79.6% yield, an off-white solid) was obtained using intermediate **BB16-D3** and compound **C58** as starting materials. LC-MS: m/z = 431.1 $(M+H)^+$.

**Synthesis of intermediate BB16-D3C59 (2-chloro-5-fluoro-N-(4-(1-(fluoromethyl)-4-(trifluoromethyl)-1H-imidazol-2-yl)benzyl)-N-(methyl-D3)pyrimidin-4-amine)**

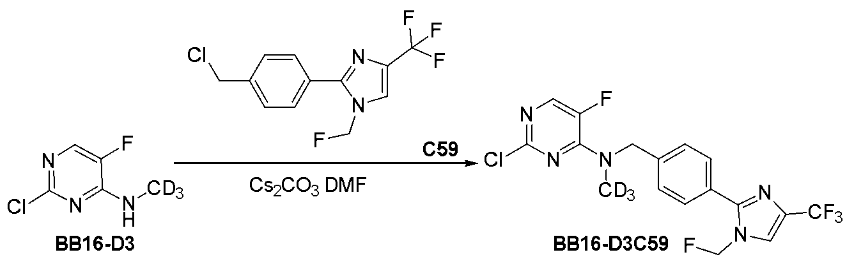


In accordance with the same steps of intermediate **BB1C2,** intermediate **BB16-D3C59** (874.2 mg, 2.08 mmol, 84.1% yield, a yellow solid) was obtained using intermediate **BB16-D3** and compound **C59** as starting materials. LC-MS: m/z = 421.1 $(M+H)^+$.

**Synthesis of intermediate BB17C5 (2-chloro-4-((4-(1-methyl-4-(trifluoromethyl)-1H-imidazol-2-yl)benzyl)amino)pyrimidine-5-carbonitrile)**

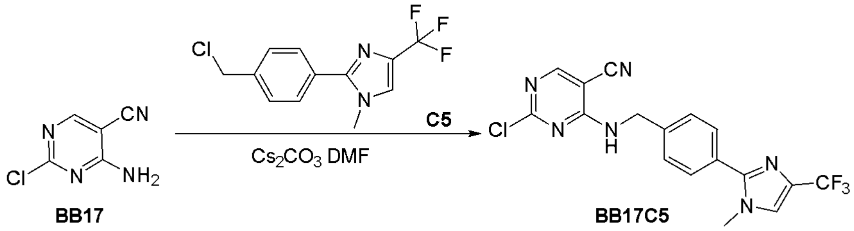


BB17 BB17C5

In accordance with the same steps of intermediate **BB1C2,** intermediate **BB17C5** (149.0 mg, 0.38 mmol, 48.7% yield, an off-white solid) was obtained using intermediate **BB17** and compound **C5** as starting materials. LC-MS: m/z = 393.0 $(M+H)^+$.

**Synthesis of intermediate BB19C4 (2-chloro-5-(difluoromethoxy)-N-((1-(1-methyl-4-(trifluoromethyl)-1H-imidazol-2-yl)piperidin-4-yl)methyl)pyrimidin-4-amine)**

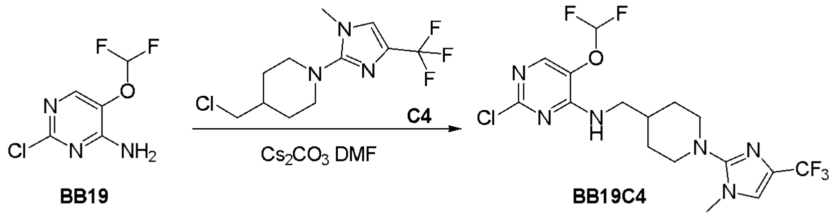


BB19 BB19C4

In accordance with the same steps of intermediate **BB2C35,** intermediate **BB19C4** (44.0 mg, 0.10 mmol, 17.7% yield, a colorless transparent oil) was obtained using intermediate **BB19** and compound **C4** as starting materials. LC-MS: m/z = 441.0 $(M+H)^+$.

**Synthesis of intermediate BB19C5 (2-chloro-5-(difluoromethoxy)-N-(4-(1-methyl-4-(trifluoromethyl)-1H-imidazol-2-yl)benzyl)pyrimidin-4-amine)**

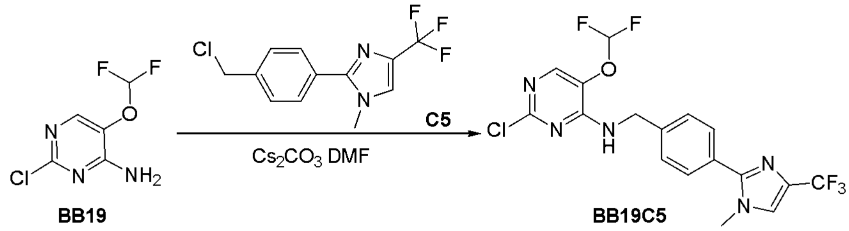


BB19 BB19C5

In accordance with the same steps of intermediate **BB1C2,** intermediate **BB19C5** (1.03 g, 2.33 mmol, 82.2% yield, a white solid) was obtained using intermediate **BB19** and compound **C5** as starting materials. LC-MS: m/z = 434.0 $(M+H)^+$.

**Synthesis of intermediate BB19C11 (2-chloro-5-(difluoromethoxy)-N-(4-(5-methyl-3-(trifluoromethyl)-1H-pyrazol-1-yl)benzyl)pyrimidin-4-amine)**

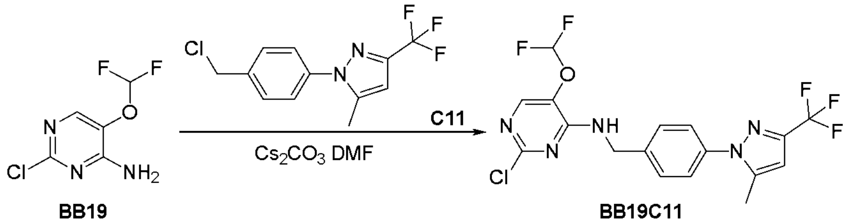


BB19 BB19C11

In accordance with the same steps of intermediate **BB1C2,** intermediate **BB19C11** (545.7 mg, 1.26 mmol, 77.3% yield, a white solid) was obtained using intermediate **BB19** and compound **C11** as starting materials. LC-MS: m/z = 434.2 $(M+H)^+$.

**Synthesis of intermediate BB19C30 (2-chloro-5-(difluoromethoxy)-N-(2-methoxy-4-(1-methyl-4-(trifluoromethyl)-1H-imidazol-2-yl)benzyl)pyrimidin-4-amine)**

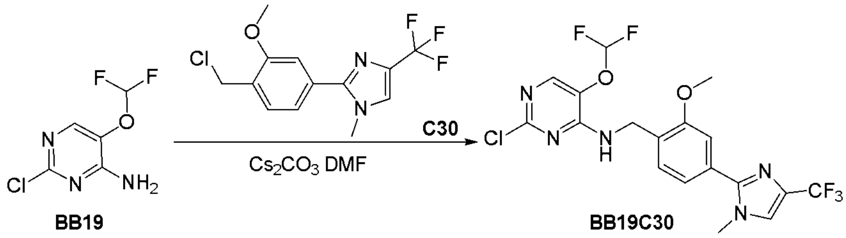


BB19 BB19C30

In accordance with the same steps of intermediate **BB1C2,** intermediate **BB19C30** (546.4 mg, 1.18 mmol, 66.5% yield, an off-white solid) was obtained using intermediate **BB19** and compound **C30** as starting materials. LC-MS: m/z = 464.0 $(M+H)^+$.

**Synthesis of intermediate BB36C58 (2-chloro-N-(4-(1-isopropyl-4-(trifluoromethyl)-1H-imidazol-2-yl)benzyl)-5-(methoxymethyl)-N-methylpyrimidin-4-amine)**

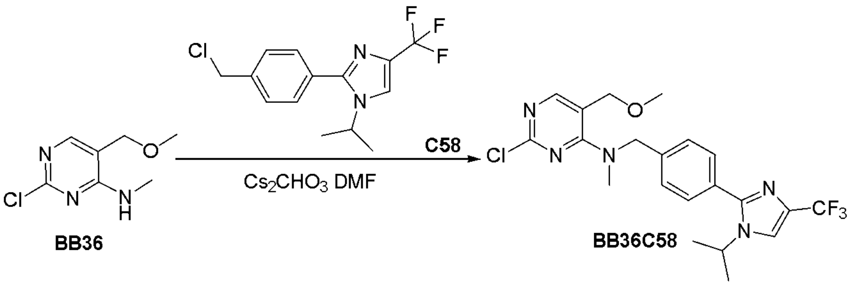


BB36 BB36C58

In accordance with the same steps of intermediate **BB1C2,** intermediate **BB36C58** (382.0 mg, 0.84 mmol, 69.3% yield, a colorless transparent oil) was obtained using intermediate **BB36** and compound **C58** as starting materials. LC-MS: m/z = 454.0 $(M+H)^+$.

**Synthesis of intermediate BB42CS (2-chloro-5-methoxy-N-(4-(1-methyl-4-(trifluoromethyl)-1H-imidazol-2-yl)benzyl)-N-(prop-2-yn-1-yl)pyrimidin-4-amine)**

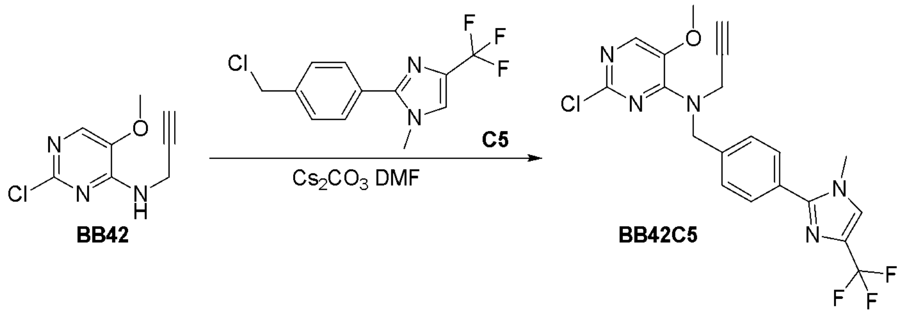


BB42 BB42C5

In accordance with the same steps of intermediate **BB1C2,** intermediate **BB42C5** (332.6 mg, 0.76 mmol, 44.5% yield, an off-white solid) was obtained using intermediate **BB42** and compound **C5** as starting materials. LC-MS: m/z = 436.2 $(M+H)^+$.

**Synthesis of intermediate BB43C5 (2-chloro-5-methoxy-N-(4-(1-methyl-4-(trifluoromethyl)-1H-imidazol-2-yl)benzyl)-N-(tetrahydrofuran-2-yl)pyrimidin-4-amine)**

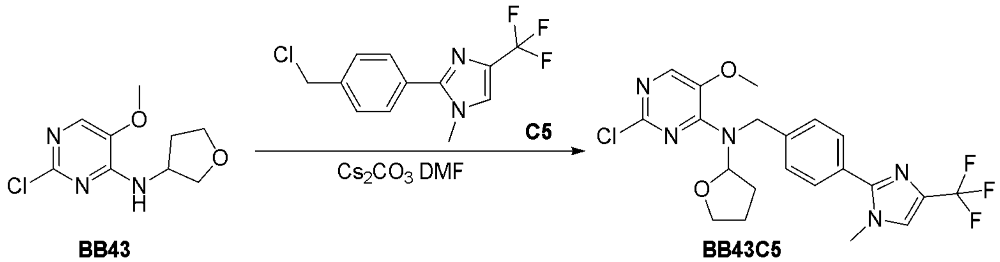


BB43 BB43C5

In accordance with the same steps of intermediate **BB1C2,** intermediate **BB43CS** (101.1 mg, 0.22 mmol, 37.3% yield, a white solid) was obtained using intermediate **BB43** and compound **C5** as starting materials. LC-MS: m/z = 468.1 $(M+H)^+$.

**Synthesis of intermediate BB44CS (2-chloro-N-cyclopropyl-5-methoxy-N-(4-(1-methyl-4-(trifluoromethyl)-1H-imidazol-2-yl)benzyl)pyrimidin-4-amine)**

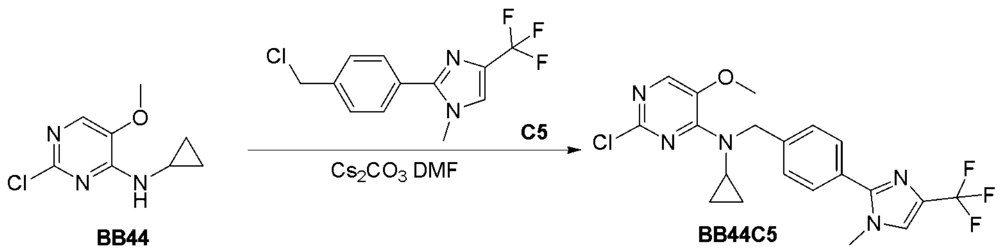


BB44 BB44C5

In accordance with the same steps of intermediate **BB1C2,** intermediate **BB44C5** (258.3 mg, 0.59 mol, 74.8% yield, a light yellow solid) was obtained using intermediate **BB44** and compound **C5** as starting materials. LC-MS: m/z = 438.1 $(M+H)^+$.

**Synthesis of intermediate BB45C5 (N-(2-chloro-5-methoxypyrimidin-4-yl)-O-methyl-N-(4-(1-methyl-4-(trifluoromethyl)-1H-imidazol-2-yl)benzyl)hydroxylamine)**

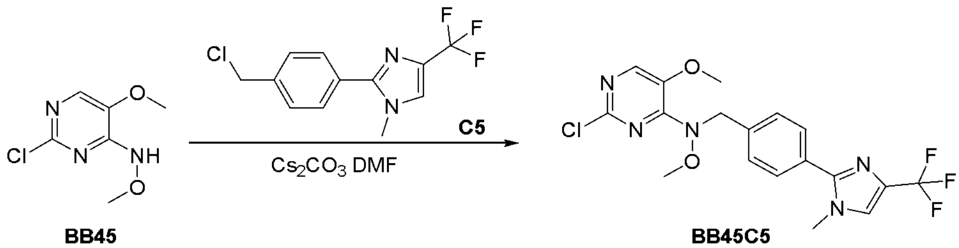


BB45 BB45C5

Compound **BB45** (250 mg, 1.3 mmol, 1.0 eq), compound **C5** (392 mg, 1.43 mmol, 1.1 eq) and potassium carbonate (548 mg, 4.0 mmol, 3.0 eq) were added to dimethyl sulfoxide (5.0 mL) successively, and the resulting mixture was stirred at 85°C for 1 hour. The reaction solution was cooled to 25°C. Ethyl acetate (20.0 mL) was added, the resulting mixture was stirred for 5 minutes, and filtered to obtain a filtrate. The filtrate was washed three times with saturated brine (10.0 mL), dried over anhydrous sodium sulfate, and concentrated under vacuum to obtain a crude product, which was purified by column chromatography (eluent: petroleum ether/ethyl acetate = 1/0 - 3/1) to obtain intermediate **BB45C5** (160.5 mg, 0.38 mmol, 28.9% yield) as a light yellow oil. $^1$H NMR (400 MHz, DMSO-d6) δ 8.18 (s, 1H), 7.96 (d, J = 1.5 Hz, 1H), 7.77 - 7.71 (m, 2H), 7.51 (d, J = 8.2 Hz, 2H), 4.96 (s, 2H), 3.95 (s, 3H), 3.81 (s, 3H), 3.68 (s, 3H); LC-MS: m/z = 428.1 $(M+H)^+$.

## Synthesis of intermediate BB18D5 (2-chloro-5-ethynyl-N-(4-(1-methyl-4-(trifluoromethyl)-1H-imidazol-2-yl)benzyl)pyrimidin-4-amine)

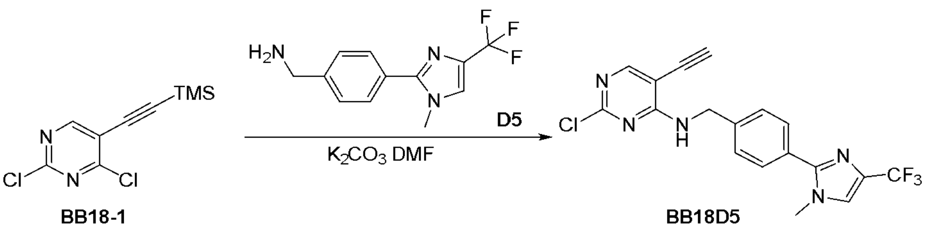


BB18-1 BB18D5

2-(2,4-Dichloropyrimidin-5-yl)ethynyl-trimethylsilane (compound **BB18-1,** 2.2 g, 8.16 mmol, 1.00 eq), compound **D5** (2.19 g, 8.57 mmol, 1.05 eq) and potassium carbonate (4.51 g, 32.64 mmol, 4.0 eq) were added to N,N-dimethylformamide (40.0 mL) successively, and the resulting mixture was stirred at 50°C for 2 hours. The reaction solution was cooled to 25°C. Ethyl acetate (100.0 mL) was added, the resulting mixture was stirred for 5 minutes, and filtered to obtain a filtrate. The filtrate was washed three times with saturated brine (100.0 mL), dried over anhydrous sodium sulfate, and concentrated under vacuum to obtain a crude product, which was purified by column chromatography (eluent: petroleum ether/ethyl acetate = 1/0 - 0/1) to obtain intermediate **BB18D5** (2.24 g, 5.73 mmol, 70.2% yield) as a white solid. LC-MS: m/z = 392.2 $(M+H)^+$.

## Synthesis of intermediate BB21D5 (2-chloro-N-(4-(1-methyl-4-(trifluoromethyl)-1H-imidazol-2-yl)benzyl)-5-morpholinopyrimidin-4-amine)

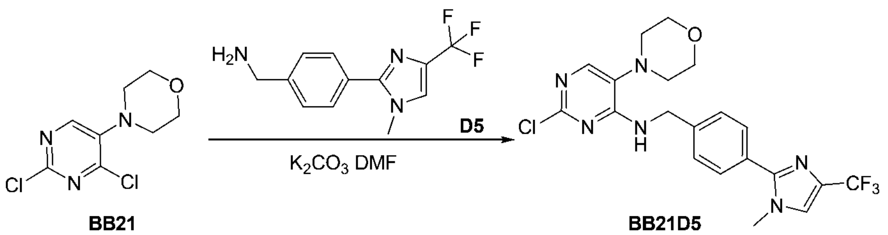


BB21 BB21D5

In accordance with the same steps of intermediate **BB18D5,** intermediate **BB21D5** (880.0 mg, 1.95 mmol, 88.6% yield, a white solid) was obtained using intermediate **BB21** and compound **D5** as starting materials. LC-MS: m/z = 453.1 $(M+H)^+$.

**Synthesis of intermediate BB22D5 (2-chloro-N-(4-(1-methyl-4-(trifluoromethyl)-1H-imidazol-2-yl)benzyl)-5-(4-methylpiperazin-1-yl)pyrimidin-4-amine)**

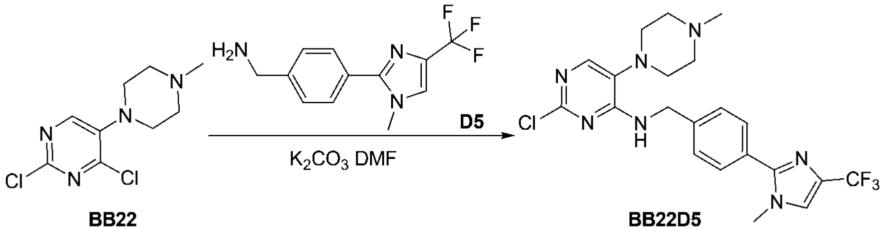


BB22 BB22D5

In accordance with the same steps of intermediate **BB18D5,** intermediate **BB22D5** (441.9 mg, 0.95 mmol, 87.4% yield, an off-white solid) was obtained using intermediate **BB22** and compound **D5** as starting materials. LC-MS: m/z = 466.2 $(M+H)^+$.

**Synthesis of intermediate BB24D5 (2-chloro-4-((4-(1-methyl-4-(trifluoromethyl)-1H-imidazol-2-yl)benzyl)amino)pyrimidin-5-ol)**

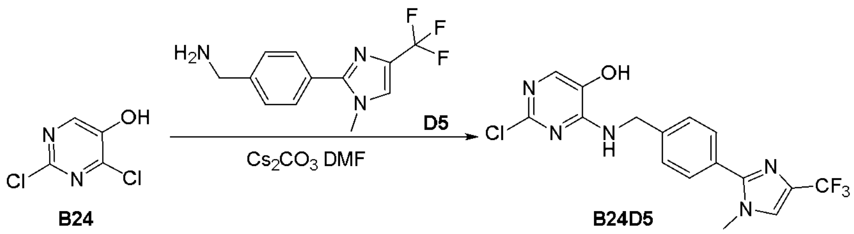


B24 B24D5

In accordance with the same steps of intermediate **BB18D5,** intermediate **BB24DS** (26.8 mg, 0.07 mmol, 22.8% yield, a light yellow solid) was obtained using intermediate **BB24** and compound **D5** as starting materials. LC-MS: m/z = 384.1 $(M+H)^+$.

**Synthesis of intermediate BB24D11 (2-chloro-4-((4-(5-methyl-3-(trifluoromethyl)-1H-pyrazol-1-yl)benzyl)amino)pyrimidin-5-ol)**

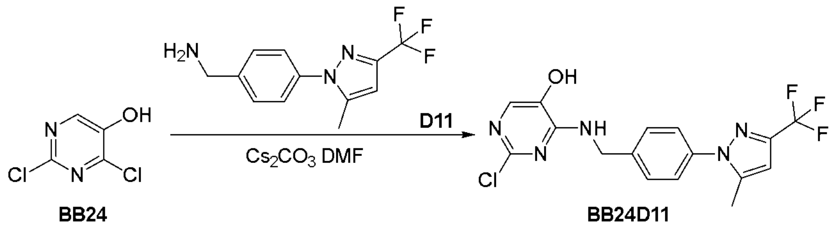


BB24 BB24D11

In accordance with the same steps of intermediate **BB18D5,** intermediate **BB24D11** (115.1 mg, 0.3 mmol, 83.0% yield, a white solid) was obtained using intermediate **BB24** and **D11** as starting materials. LC-MS: m/z = 384.2 $(M+H)^+$.

**Synthesis of intermediate BB35D5 ((2-chloro-4-((4-(1-methyl-4-(trifluoromethyl)-1H-imidazol-2-yl)benzyl)amino)pyrimidin-5-yl)methanol)**

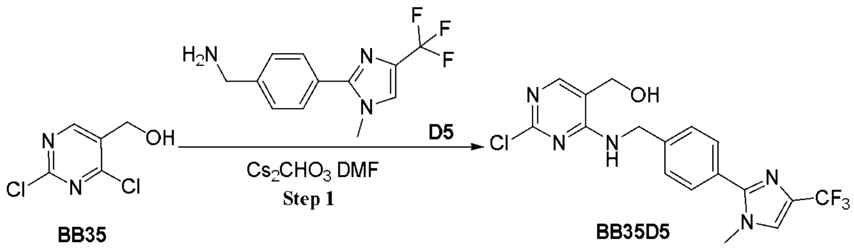


BB35 BB35D5

In accordance with the same steps of intermediate **BB18D5,** intermediate **BB35D5** (333.8 mg, 1.86 mmol, 55.6% yield, a colorless transparent oil) was obtained using intermediate **BB35** and compound **D5** as starting materials. LC-MS: m/z = 398.1 $(M+H)^+$.

**Synthesis of intermediate BB41C5 (methyl 2-(2-chloro-4-((4-(1-methyl-4-(trifluoromethyl)-1H-imidazol-2-yl) benzyl)amino)pyrimidin-5-yl)acetate)**

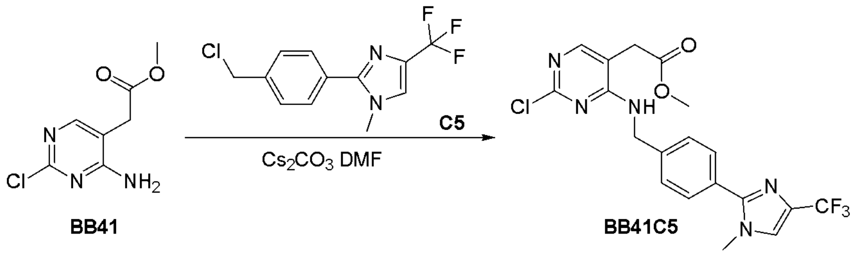


BB41 BB41C5

In accordance with the same steps of intermediate **BB18D5,** intermediate **BB41D5** (133.9 mg, 0.30 mmol, 69.9% yield, a light yellow solid) was obtained using intermediate **BB41** and compound **D5** as starting materials. LC-MS: m/z = 440.2 $(M+H)^+$.

**Example 1: Synthesis of compound 1 (2-(2-isopropylphenyl)-5-methoxy-N-methyl-N-(4-(pyridin-2-yl)benzyl) pyrimidin-4-amine)**

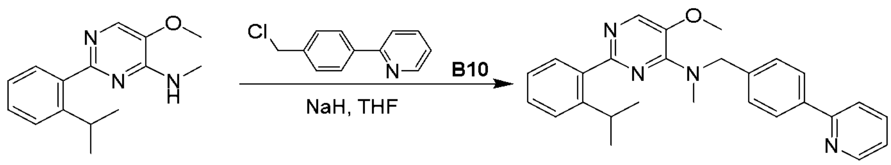


A5 1

Compound **A5** (100 mg, 389 μmol, 1.00 eq) was dissolved in tetrahydrofuran (2.5 mL), and sodium hydride (62.2 mg, 1.55 mmol, 60.0% purity, 4.00 eq) was added in portions at 0°C. After the addition was completed, the resulting mixture was reacted at 0°C for 30 minutes. Then, compound **B10** (95.0 mg, 466 μmol, 1.20 eq) was added. After the addition was completed, the resulting mixture was heated up to 25°C and stirred for 12 hours. Then, ice water (2.0 mL) was added at 0°C to quench the reaction, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by preparative HPLC (column: 3_Phenomenex Luna C18 75x30mmx3μm; mobile phase: [water (HCl)-ACN]; B%: 15%-35%, 8 min) to obtain compound **1** (136 mg, 277 μmol, 71.3% yield, HCl) as a light yellow solid. $^1H$ NMR (400 MHz, $CDCl_3$) δ 8.91 (d, *J* = 4.40 Hz, 1H), 8.40 (s, 1H), 8.18-8.19 (m, 3H), 7.80 (s, 1H), 7.65 (s, 1H), 7.43-7.46 (m, 6H), 5.09-5.21 (m, 2H), 3.85-3.94 (m, 3H), 3.54 (s, 1H), 3.26-3.34 (m, 3H), 1.01-1.23 (m, 6H); LC-MS: m/z = 425.2 $(M+H)^+$.

In accordance with the preparation method of compound **1,** the following compounds were obtained using different general intermediates A and B together with cesium carbonate or sodium hydride or potassium carbonate.

| Example | Structure and name of compound | Reaction moiety | Analyzed data |
|---|---|---|---|
| 2 | 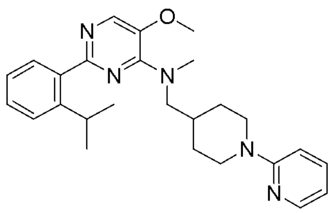 2-(2-Isopropylphenyl)-5-methoxy-N-methyl-N-((1-(pyridin-2-yl)piperidin-4-yl)methyl)pyrimidin-4-amine | A5 B11 | LC-MS: m/z =432.2 $(M+H)^+$ |
| 3 | 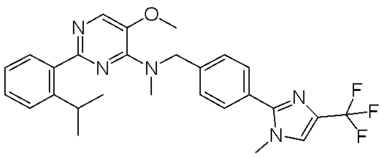 2-(2-Isopropylphenyl)-5-methoxy-N-methyl-N-(4-(1-methyl-4-(trifluoromethyl)-1H-imidazol-2-yl)benzyl) pyrimidin-4-amine | A5 B6 | LC-MS: m/z =496.3 $(M+H)^+$; HNMR (400MHz, DMSO) δ 8.14 (s, 1H), 7.96 (s, 1H), 7.70-7.72 (m, 2H), 7.37-7.57 (m, 6H), 5.18 (s, 2H), 3.95 (s, 3H), 3.75 (s, 3H), 3.41-3.43 (m, 3H), 3.13 (s, 1H), 1.02-1.23 (m, 6H). |
| 4 | 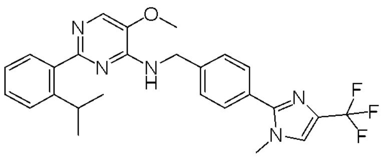 2-(2-Isopropylphenyl)-5-methoxy-N-(4-(1-methyl-4-(trifluoromethyl)-1H-imidazol-2-yl)benzyl) pyrimidin-4-amine | A1 B6 | LC-MS: m/z =482.3 $(M+H)^+$; HNMR (400MHz, DMSO) δ 8.02 (s, 1H), 7.69-7.74 (m, 3H), 7.52-7.54 (m, 1H), 7.39-7.48 (m, 5H), 7.10 (s, 1H), 4.94 (d, *J* = 5.20 Hz, 2H), 4.05 (s, 3H), 3.82 (s, 3H), 3.33-3.36 (m, 1H), 1.21 (d, *J* = 6.80 Hz, 6H). |
| 5 | 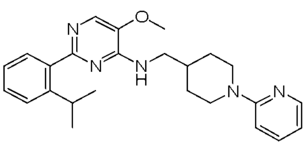 2-(2-Isopropylphenyl)-5-methoxy-N-((1-(pyridin-2-yl) piperidin-4-yl)methyl)pyrimidin-4-amine | A1 B11 | LC-MS: m/z =418.1 $(M+H)^+$ |
| 6 | 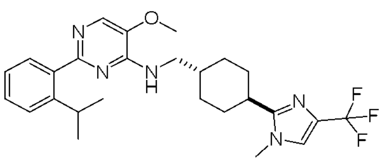 2-(2-Isopropylphenyl)-5-methoxy-N-(((1R,4R)-4-(1-methyl-4-(trifluoromethyl)-1H-imidazol-2-yl)cyclohexyl)methyl)pyrimidin-4-amine | A1 B12 | LC-MS: m/z =488.4 $(M+H)^+$ |
| 7 | 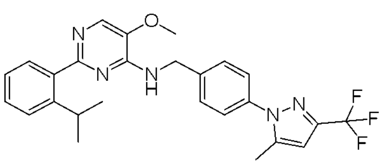 2-(2-Isopropylphenyl)-5-methoxy-N-(4-(5-methyl-3-(trifluoromethyl)-1H-pyrazol-1-yl)benzyl) pyrimidin-4-amine | A1 B17 | LC-MS: m/z =482.2 $(M+H)^+$ |

(continued)

| Example | Structure and name of compound | Reaction moiety | Analyzed data |
|---|---|---|---|
| 8 | 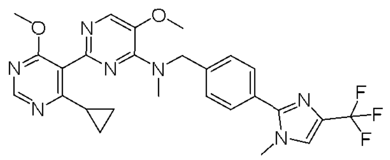 4'-Cyclopropyl-5,6'-dimethoxy-N-methyl-N-(4-(1-methyl-4-(trifluoromethyl)-1H-imidazol-2-yl)benzyl)-[2,5'-bipyrimidin]-4-amine | A2 B6 | LC-MS: m/z =526.2 (M+H)+; $^1$ H NMR (400 MHz, DMSO) δ 8.62 (s, 1H), 8.16 (s, 1H), 7.98 - 7.93 (m, 1H), 7.70 (d, *J* = 8.2 Hz, 2H), 7.42 (d, *J* = 8.1 Hz, 2H), 4.94 (s, 2H), 3.90 (s, 3H), 3.86 (s, 3H), 3.80 (s, 3H), 3.18 (s, 3H), 1.79-1.72 (m, 1H), 1.06 - 0.99 (m, 2H), 0.90-0.81 (m, 2H). |
| 9 | 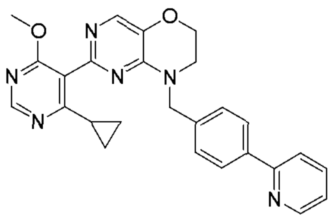 2-(4-Cyclopropyl-6-methoxypyrimidin-5-yl)-8-(4-(pyridin-2-yl)benzyl)-7,8-dihydro-6H-pyrimido[5,4-b][1,4]oxazine | A8 B10 | LC-MS: m/z =453.1 (M+H)+; $^1$HNMR (400 MHz, DMSO) δ 8.65 (d, *J* = 4.4 Hz, 1H), 8.59 (s, 1H), 8.04 (d, *J* = *8.00* Hz, 2H), 7.94-7.95 (m, 2H), 7.85-7.89 (m, 1H), 7.36-7.42 (m, 2H), 7.33-7.36 (m, 1H), 4.85 (s, 2H), 4.28 (s, 2H), 3.85 (s, 3H), 3.59 (s, 2H), 1.74-1.76 (m, 1H), 0.97 (s, 2H), 0.81-0.83 (m, 2H). |
| 10 | 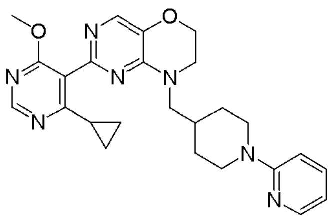 2-(4-Cyclopropyl-6-methoxypyrimidin-5-yl)-8-((1-(pyridin-2-yl)piperidin-4-yl)methyl)-7,8-dihydro-6H-pyrimido[5,4-b][1,4]oxazine | A8 B11 | LC-MS: m/z =460.2 (M+H)+; $^1$HNMR (400 MHz, DMSO) δ 8.57 (s, 1H), 8.06-8.07 (m, 1H), 7.86 (s, 1H), 7.45-7.49 (m, 1H), 6.78 (d, *J* = 8.80 Hz, 1H), 6.54-6.57 (m, 1H), 4.23-4.29 (m, 4H), 3.81 (s, 3H), 3.61-3.63 (m, 2H), 3.46 (d, *J* = 7.60 Hz, 2H), 2.67-2.77 (m, 2H), 2.05-2.07 (m, 1H), 1.74-1.75 (m, 1H), 1.61 (d, *J* = 11.2 Hz, 2H), 1.16-1.17 (m, 2H), 0.96-0.99 (m, 2H), 0.88-0.90 (m, 2H). |
| 11 | 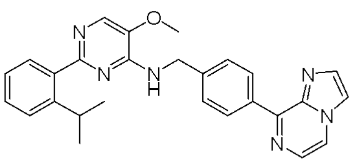 N-(4-(Imidazo[1,2-a]pyrazin-8-yl)benzyl)-2-(2-isopropylphenyl)-5-methoxypyrimidin-4-amine | A1 B15 | LC-MS: m/z =451.1 (M+H)+ |
| 12 | 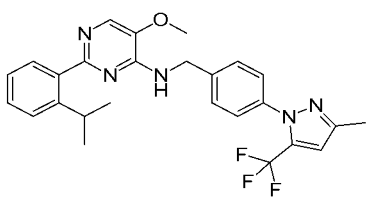 2-(2-Isopropylphenyl)-5-methoxy-N-(4-(3-methyl-5-(trifluoromethyl)-1H-pyrazol-1-yl)benzyl)pyrimidin-4-amine | A1 B17 | LC-MS: m/z =482.2 (M+H)+ |

(continued)

| Example | Structure and name of compound | Reaction moiety | Analyzed data |
|---|---|---|---|
| 13 | 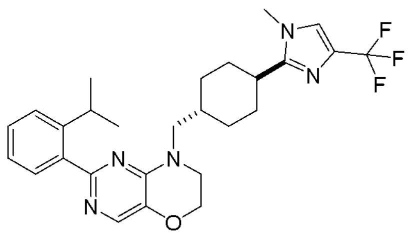 2-(2-Isopropylphenyl)-8-(((1R,4R)-4-(1-methyl-4-(trifluoromethyl)-1H-imidazol-2-yl)cyclohexyl) methyl)-7,8-dihydro-6H-pyrimido [5,4-b][1,4] oxazine | A6 B12 | LC-MS: m/z =500.4 (M+H)+ |
| 14 | 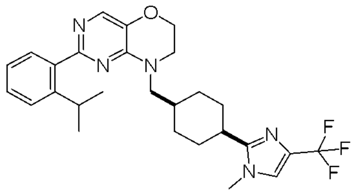 6H-pyrimido[5,4-b][1,4] 2-(2-Isopropylphenyl)-8-(((1s,4s)-4-(1-methyl-4-(trifluoromethyl)-1H-imidazol-2-yl)cyclohexyl)methyl)-7,8-dihydro-6H-pyrimido[5,4-b][1,4] oxazine | A6 B12 | LC-MS: m/z =500.3 (M+H)+ |
| 15 | 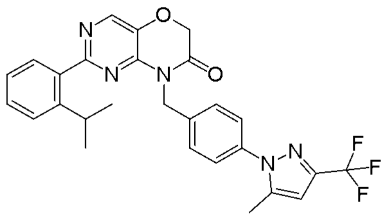 2-(2-Isopropylphenyl)-8-(4-(5-methyl-3-(trifluoromethyl)-1H-pyrazol-1-yl)benzyl)-6H-pyrimido[5,4-b] [1,4]oxazin-7(8H)-one | A7 B17 | LC-MS: m/z = 508.3 (M+H)+ |
| 16 | 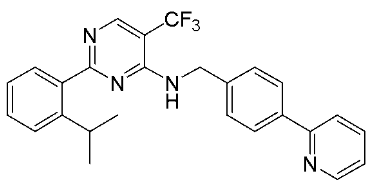 2-(2-Isopropylphenyl)-N-(4-(pyridin-2-yl)benzyl)-5-(trifluoromethyl)pyrimidin-4-amine | A4 B10 | LC-MS: m/z =449.1 (M+H)+ |
| 17 | 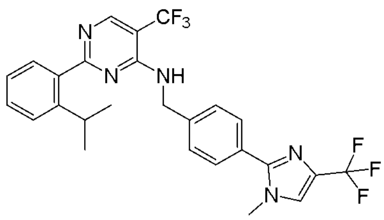 (trifluoromethyl)pyrimidin-4-amine 2-(2-Isopropylphenyl)-N-(4-(1-methyl-4-(trifluoromethyl)-1H-imidazol-2-yl)benzyl)-5-(trifluoromethyl)pyrimidin-4-amine | A4 B6 | LC-MS: m/z =520.1 (M+H)+ |

(continued)

| Example | Structure and name of compound | Reaction moiety | Analyzed data |
|---|---|---|---|
| **18** | 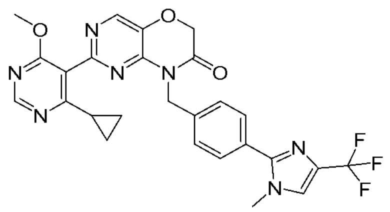 2-(4-Cyclopropyl-6-methoxypyrimidin-5-yl)-8-(4-(1-methyl-4-(trifluoromethyl)-1H-imidazol-2-yl)benzyl)-6H-pyrimido[5,4-b][1,4]oxazin-7(8H)-one | **A10** **B6** | LC-MS: m/z = 538.3 (M+H)+ |
| **19** | 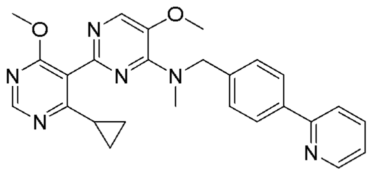 4'-Cyclopropyl-5,6'-dimethoxy-N-methyl-N-(4-(pyridin-2-yl)benzyl)-[2,5'-bipyrimidin]-4-amine | **A2** **B10** | LC-MS: m/z =455.4 (M+H)+ |
| **20** | 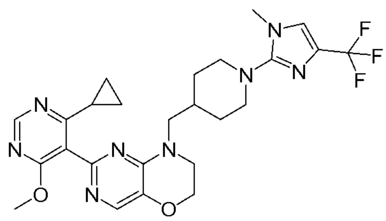 2-(4-Cyclopropyl-6-methoxypyrimidin-5-yl)-8-((1-(1-methyl-4-(trifluoromethyl)-1H-imidazol-2-yl)piperidin-4-yl)methyl)-7,8-dihydro-6H-pyrimido[5,4-b][1,4] oxazine | **A8** **B13** | LC-MS: m/z =531.2 (M+H)+ |
| **21** | 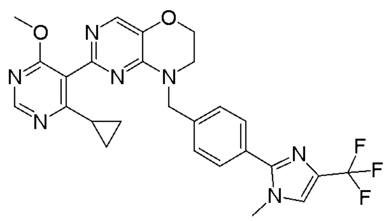 2-(4-Cyclopropyl-6-methoxypyrimidin-5-yl)-8-(4-(1-methyl-4-(trifluoromethyl)-1H-imidazol-2-yl)benzyl)-7,8-dihydro-6H-pyrimido[5,4-b][1,4]oxazine | **A8** **B6** | LC-MS:m/z=524.2 (M+H)+; 1HNMR (400 MHz, DMSO) δ8.60 (s, 1H), 7.95 (d, *J* = 7.20 Hz, 2H), 7.68 (d, *J* = 8.00 Hz, 2H), 7.44 (d, *J* = 8.00 Hz, 2H), 4.87 (s, 2H), 4.29 (s, 2H), 3.80 (d, *J* = 30.0 Hz, 6H), 3.59 (s, 2H), 1.76 (s, 1H), 0.98 (s, 2H), 0.82-0.84 (m, 2H). |
| **22** | 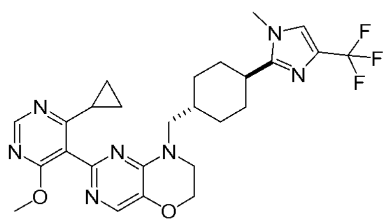 2-(4-Cyclopropyl-6-methoxypyrimidin-5-yl)-8-(((1r,4r)-4-(1-methyl-4-(trifluoromethyl)-1H-imidazol-2-yl)cyclohexyl)methyl)-7,8-dihydro-6H-pyrimido[5,4-b][1,4]oxazine | **A8** **B12** | LC-MS: m/z = 530.4 (M+H)+ |

(continued)

| Example | Structure and name of compound | Reaction moiety | Analyzed data |
|---|---|---|---|
| 23 | 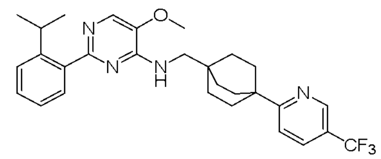 yl)methyl)pyrimidin-4-amine 2-(2-Isopropylphenyl)-5-methoxy-N-((4-(5-(trifluoromethyl)pyridin-2-yl)bicyclo[2.2.2]octan-1-yl)methyl)pyrimidin-4-amine | A1 B16 | LC-MS: m/z =511.2 $(M+H)^+$ |
| 24 | 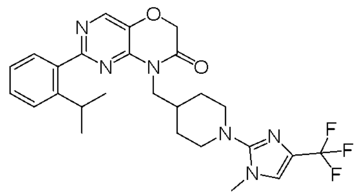 2-(2-Isopropylphenyl)-8-((1-(1-methyl-4-(trifluoromethyl)-1H-imidazol-2-yl)piperidin-4-yl)methyl)-6H-pyrimido[5,4-b][1,4]oxazin-7(8H)-one | A7 B13 | LC-MS: m/z =515.2 $(M+H)^+$ |
| 25 | 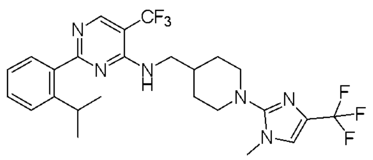 (trifluoromethyl)pyrimidin-4-amine 2-(2-Isopropylphenyl)-N-((1-(1-methyl-4-(trifluoromethyl)-1H-imidazol-2-yl)piperidin-4-yl)methyl)-5-(trifluoromethyl) pyrimidin-4-amine | A4 B13 | LC-MS: m/z =527.2 $(M+H)^+$ |
| 26 | 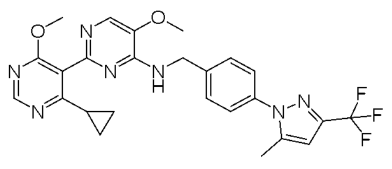 4'-Cyclopropyl-5,6'-dimethoxy-N-(4-(5-methyl-3-(trifluoromethyl)-1H-pyrazol-1-yl)benzyl)-[2,5'-bipyrimidin]-4-amine | A9 B17 | LC-MS: m/z = 512.3 $(M+H)^+$; $^1H$ NMR (400 MHz, DMSO-$d_6$) $\delta$ 8.58 (s, 1H), 7.97 (s, 1H), 7.85 (t, *J* = 6.3 Hz, 1H), 7.47 (t, *J* = 8.3 Hz, 4H), 6.74 (s, 1H), 4.63 (d, *J* = 6.3 Hz, 2H), 3.94 (s, 3H), 3.80 (s, 3H), 2.31 (s, 3H), 1.67 (tt, *J* = 8.3, 4.7 Hz, 1H), 0.94 (dq, *J* = 6.3, 3.5 Hz, 2H), 0.76 (dt, *J* = 8.2, 3.3 Hz, 2H). |
| 27 | 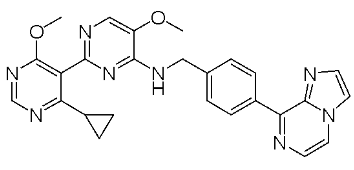 4'-Cyclopropyl-N-(4-(imidazo[1,2-a]pyrazin-8-yl)benzyl)-5,6'-dimethoxy-[2,5'-bipyrimidin]-4-amine | A9 B15 | LC-MS: m/z = 481.3 (M+H)+ |

(continued)

| Example | Structure and name of compound | Reaction moiety | Analyzed data |
|---|---|---|---|
| **28** | 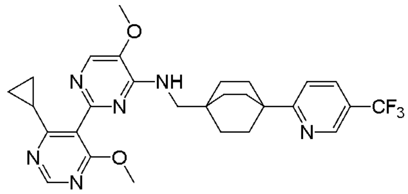 4'-Cyclopropyl-5,6'-dimethoxy-N-(4-(5-(trifluoromethyl)pyridin-2-yl)bicyclo[2.2.2]octan-1-yl) methyl)-[2,5'-bipyrimidin]-4-amine | **A9** **B16** | LC-MS: m/z = 541.3 (M+H)+ |
| **29** | 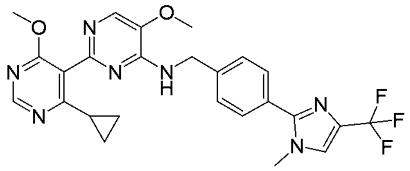 4'-Cyclopropyl-5,6'-dimethoxy-N-(4-(1-methyl-4-(trifluoromethyl)-1H-imidazol-2-yl)benzyl)-[2,5'-bipyrimidin]-4-amine | **A9** **B6** | LC-MS: m/z = 512.2 $(M+H)^+$; $^1$H NMR (400 MHz, DMSO-$d_6$) δ 8.60 (s, 1H), 7.96 (d, *J* = 16.5 Hz, 2H), 7.86 (t, *J* = 6.4 Hz, 1H), 7.65 (d, *J* = 8.0 Hz, 2H), 7.43 (d, *J* = 8.0 Hz, 2H), 4.64 (d, *J* = 6.3 Hz, 2H), 3.96 (s, 3H), 3.83 (s, 3H), 3.78 (s, 3H), 1.70 (tt, *J* = 8.5, 4.7 Hz, 1H), 0.97 (s, 2H), 0.78 (dd, *J* = 7.8, 3.3 Hz, 2H). |
| **30** | 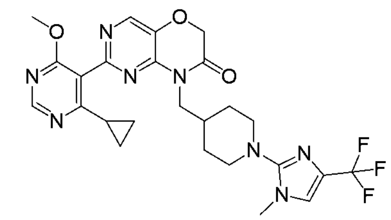 2-(4-Cyclopropyl-6-methoxypyrimidin-5-yl)-8-((1-(1-methyl-4-(trifluoromethyl)-1H-imidazol-2-yl)piperidin-4-yl)methyl)-6H-pyrimido[5,4-b][1,4]oxazin-7(8H)-one | **A10** **B13** | LC-MS: m/z = 545.3 $(M+H)^+$ |
| **31** | 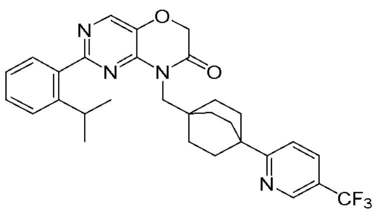 yl)bicyclo[2.2.2]octan-1-2-(2-Isopropylphenyl)-8-((4-(5-(trifluoromethyl)pyridin-2-yl)bicyclo [2.2.2]octan-1-yl)methyl)-6H-pyrimido[5,4-b][1,4]oxazin-7(8H)-one | **A7** **B16** | LC-MS: m/z = 537.3 $(M+H)^+$ |

**Example 32: Synthesis of compound 32 (2-(2-isopropylphenyl)-N-(4-(pyridin-2-yl)benzyl)furo [3,2-d] pyrimidin-4-amine)**

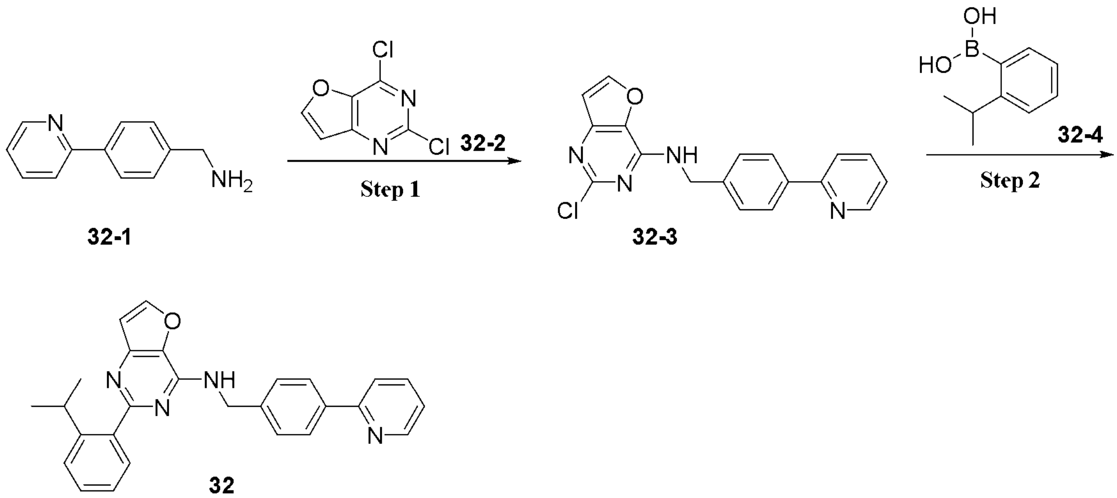


32-1

32-3

32

Step 1: Synthesis of compound **32-3**

Compound **32-1** (200.0 mg, 1.09 mmol, 1.00 *eq),* compound **32-2** (205.0 mg, 1.09 mmol, 1.00 *eq*) and cesium carbonate (1.1 g, 3.27 mmol, 3 *eq*) were added to N,N-dimethylformamide (4.0 mL) successively, and the resulting mixture was stirred at 60°C for 1 hour. Ethyl acetate (30.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 2/1) to obtain compound **32-3** (360.9 mg). LC-MS: m/z =337.0 $(M+H)^+$.

Step 2: Synthesis of compound **32**

Compound **32-3** (100.0 mg, 0.3 mmol, 1.00 *eq),* compound **32-4** (97.7 mg, 0.6 mmol, *2.00 eq),* potassium phosphate (191.0 mg, 0.9 mmol, 3.00 *eq),* and tetrakis(triphenylphosphine)palladium (34.7 mg, 0.03 mmol, 0.10 *eq)* were added to dioxane (2.0 mL) and water (0.4 mL) successively. The resulting mixture was purged three times with nitrogen, and stirred at 90°C for 16 hours under nitrogen atmosphere. Water (10.0 mL) was added, and the resulting mixture was extracted three times with ethyl acetate (20.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **32** (87.5 mg, 0.21 mmol, 69.4% yield, 98.6% purity) as a white solid. $^1H$ NMR (400 MHz, DMSO) δ 10.05 (s, 1H), 8.71 (d, *J* = 4.9 Hz, 1H), 8.59 (d, *J* = 2.2 Hz, 1H), 8.06 (d, *J* = 8.0 Hz, 2H), 8.03 - 7.95 (m, 2H), 7.58 - 7.48 (m, 5H), 7.47 - 7.42 (m, 1H), 7.40 - 7.33 (m, 1H), 7.20 (d, *J* = 2.2 Hz, 1H), 4.93 (d, *J* = 6.0 Hz, 2H), 3.29 - 3.22 (m, 1H), 1.07 (d, *J*= 6.8 Hz, 6H); LC-MS: m/z = 421.2 $(M+H)^+$.

The following compounds were obtained in accordance with the preparation method of compound **32,** wherein the reaction condition of step 1 was cesium carbonate or sodium hydride or potassium carbonate, and the reaction condition of step 2 was $Pd(PPh_3)_4/K_3PO_4$ or XPhos-Pd-G2/XPhos/$K_3PO_4$.

| Example | Structure and name of compound | Reaction moiety | Analyzed data |
|---|---|---|---|
| **33** | 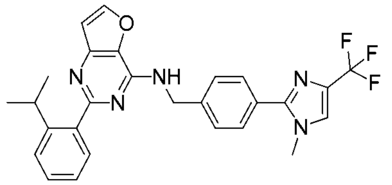 2-(2-Isopropylphenyl)-N-(4-(1-methyl-4-(trifluoromethyl)-1H-imidazol-2-yl)benzyl)furo[3,2-d]pyrimidin-4-amine | **B8** 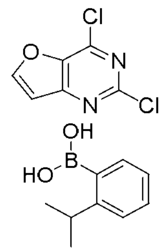 | LC-MS: m/z = 492.3 $(M+H)^+$; $^1H$ NMR (400 MHz, DMSO) δ 9.59 (s, 1H), 8.50 (s, 1H), 7.92 (d, *J* = 1.4 Hz, 1H), 7.68 (d, *J* = 8.1 Hz, 2H), 7.46-7.51 (m, 5H), 7.29 - 7.31 (m, 1H), 7.14 (d, *J* = 2.2 Hz, 1H), 4.88 (d, *J* = 6.1 Hz, 2H), 3.75 (s, 3H), 3.32 - 3.25 (m, 1H), 1.05 (d, *J* = 6.8 Hz, 6H). |
| **34** | 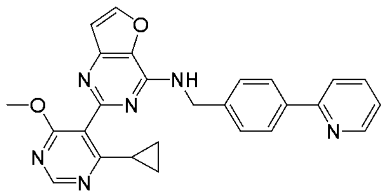 2-(4-Cyclopropyl-6-methoxypyrimidin-5-yl)-N-(4-(pyridin-2-yl)benzyl)furo[3,2-d]pyrimidin-4-amine | 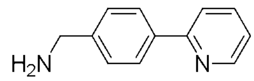 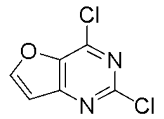 **A2-7** | LC-MS: m/z = 450.2 $(M+H)^+$ |
| **35** | 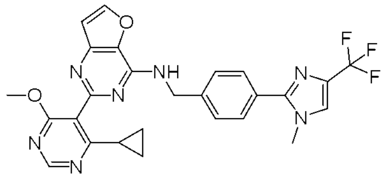 2-(4-Cyclopropyl-6-methoxypyrimidin-5-yl)-N-(4-(1-methyl-4-(trifluoromethyl)-1H-imidazol-2-yl)benzyl) furo[3,2-d]pyrimidin-4-amine | **B8** 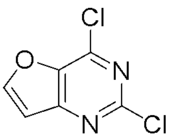 **A2-7** | LC-MS: m/z = 522.2 $(M+H)^+$; $^1H$ NMR (400 MHz, DMSO) δ 9.29 (s, 1H), 8.69 (s, 1H), 8.46 (d, *J* = 2.2 Hz, 1H), 7.95 (d, *J* = 1.4 Hz, 1H), 7.69 (d, *J* = 8.1 Hz, 2H), 7.50 (d, *J* = 7.9 Hz, 2H), 7.15 (d, *J* = 2.2 Hz, 1H), 4.83 (s, 2H), 3.87 (s, 3H), 3.78 (s, 3H), 1.91 - 1.77 (m, 1H), 1.02 (s, 2H), 0.83 (s, 2H). |

(continued)

| Example | Structure and name of compound | Reaction moiety | Analyzed data |
|---|---|---|---|
| **36** | 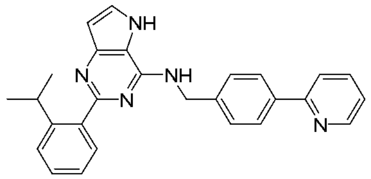 2-(2-Isopropylphenyl)-N-(4-(pyridin-2-yl)benzyl)-5H-pyrrolo[3,2-d]pyrimidin-4-amine | 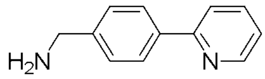 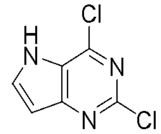 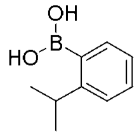 | LC-MS: m/z = 420.3 $(M+H)^+$ |
| **37** | 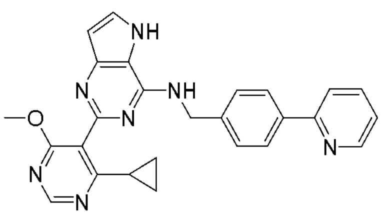 2-(4-Cyclopropyl-6-methoxypyrimidin-5-yl)-N-(4-(pyridin-2-yl)benzyl)-5H-pyrrolo[3,2-d]pyrimidin-4-amine | 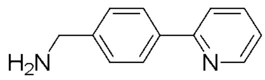 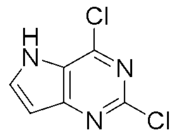 **A2-7** | LC-MS: m/z = 450.2 $(M+H)^+$; $^1$H NMR (400 MHz, DMSO) δ 12.11 (s, 1H), 9.61 (s, 1H), 8.80 (s, 1H), 8.69 (dd, *J* = 4.6, 1.6 Hz, 1H), 8.15 - 8.06 (m, 2H), 8.04 - 7.91 (m, 3H), 7.53 (d, *J* = 8.1 Hz, 2H), 7.44 - 7.36 (m, 1H), 6.73 - 6.64 (m, 1H), 4.96 (d, *J* = 5.7 Hz, 2H), 3.95 (s, 3H), 1.99 - 1.90 (m, 1H), 1.12 - 1.06 (m, 2H), 0.93 - 0.83 (m, 2H). |
| **38** | 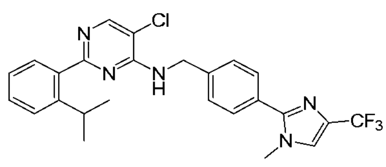 5-Chloro-2-(2-isopropylphenyl)-N-(4-(1-methyl-4-(trifluoromethyl)-1H-imidazol-2-yl)benzyl) pyrimidin-4-amine | **B8** 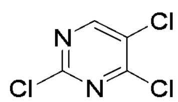 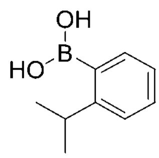 | LC-MS: m/z = 486.2 $(M+H)^+$; $^1$H NMR (400 MHz, DMSO) δ 8.4 (s, 1H), 8.15-8.19 (m, 1H), 7.9 (s, 1H), 7.65-7.67 (d, 2H), 7.42-7.44 (d, 1H), 7.39-7.41 (d, 2H), 7.35-7.36 (d, 2H), 7.17-7.21 (m,1H), 4.73-4.74 (d, 2H), 3.75(s, 3H), 3.44-3.47 (m, 1H), 0.99-1.01 (m, 6H). |

(continued)

| Example | Structure and name of compound | Reaction moiety | Analyzed data |
|---|---|---|---|
| 39 | 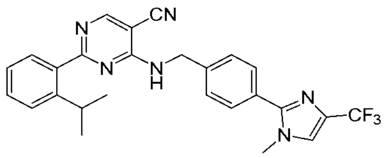 2-(2-Isopropylphenyl)-4-((4-(1-methyl-4-(trifluoromethyl)-1H-imidazol-2-yl)benzyl)amino)pyrimidine-5-carbonitrile | B8 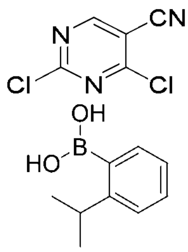 | LC-MS: m/z = 477.1 $(M+H)^+$; $^1$ H NMR (400 MHz, DMSO) δ 8.79-8.81 (m, 2H),7.91 (s, 1H), 7.67 (d, 2H), 7.49-7.51 (d, 1H), 7.40-7.43 (m, 4H), 7.21-7.25 (m, 1H), 4.74-4.75 (d, 2H), 3.75 (s, 3H),3.48-3.58 (m, 1H) 1.01-1.02 (d,6H). |
| 40 | 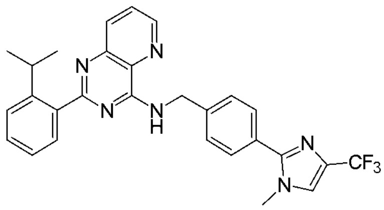 2-(2-Isopropylphenyl)-N-(4-(1-methyl-4-(trifluoromethyl)-1H-imidazol-2-yl)benzyl)pyrido[3,2-d]pyrimidin-4-amine | B8 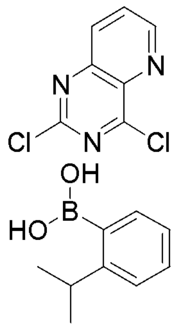 | LC-MS: m/z = 503.3 $(M+H)^+$; $^1$ H NMR (400 MHz, DMSO) δ9.22-9.25 (m, 1H),8.85-8.86 (m, 1H), 8.15-8.16 (m, 1H), 7.86-7.90 (m, 2H),7.65-7.67 (d, 2H),7.53-7.55 (m, 1H),7.46-7.48 (m, 2H),7.38-7.39 (m, 2H), 7.23-7.25 (m, 1H), 4.86-4.88 (d, mH), 3.72 (s, 3H), 3.52-3.57 (m, 1H), 1.04-1.06 (m, 6H). |

(continued)

| Example | Structure and name of compound | Reaction moiety | Analyzed data |
|---|---|---|---|
| **41** | 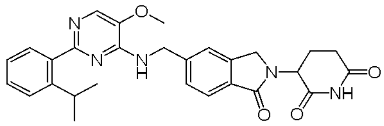<br>3-(5-(((2-(2-Isopropylphenyl)-5-methoxypyrimidin-4-yl)amino)methyl)-1-oxoisoindolin-2-yl)piperidine-2,6-dione | 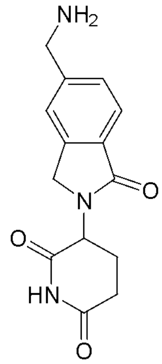<br>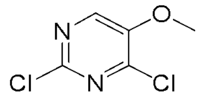<br>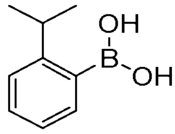 | LC-MS: m/z = 500.4 (M+H)$^+$ |
| **42** | 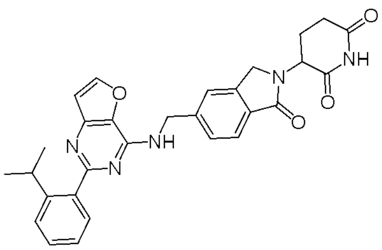<br>3-(5-(((2-(2-Isopropylphenyl)furo[3,2-d]pyrimidin-4-yl)amino)methyl)-1-oxoisoindolin-2-yl)piperidine-2,6-dione | 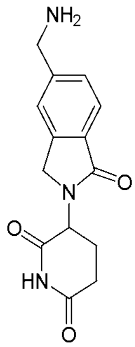<br>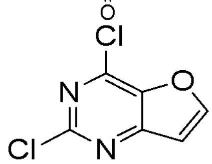<br>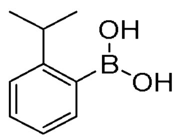 | LC-MS: m/z = 510.1 (M+H)$^+$ |

(continued)

| Example | Structure and name of compound | Reaction moiety | Analyzed data |
|---|---|---|---|
| **43** | 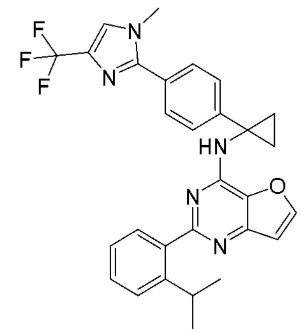<br>2-(2-Isopropylphenyl)-N-(1-(4-(1-methyl-4-(trifluoromethyl)-1H-imidazol-2-yl)phenyl)cyclopropyl)furo[3, 2-d]pyrimidin-4-amine | 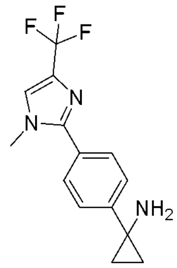<br>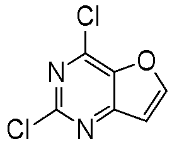<br>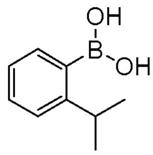 | LC-MS: m/z = 518.2 $(M+H)^+$ |
| **44** | 2-d]pyrimidin-4-amine<br>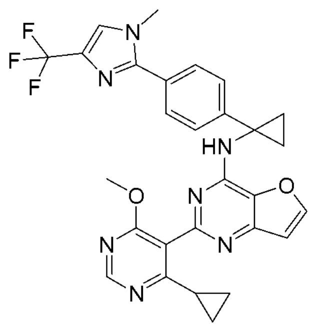<br>2-(4-Cyclopropyl-6-methoxypyrimidin-5-yl)-N-(1-(4-(1-methyl-4-(trifluoromethyl)-1H-imidazol-2-yl)phenyl)cyclopropyl)furo[3, 2-d]pyrimidin-4-amine | 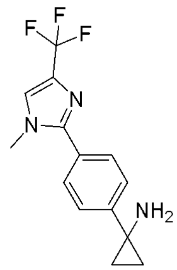<br>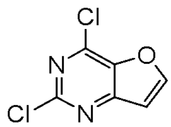<br>**A2-7** | LC-MS: m/z = 548.1 $(M+H)^+$ |
| **50** | 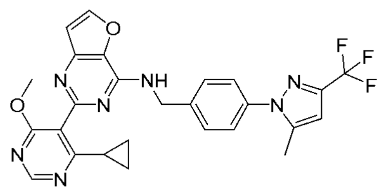<br>2-(4-Cyclopropyl-6-methoxypyrimidin-5-yl)-N-(4-(5-methyl-3-(trifluoromethyl)-1H-pyrazol-1-yl)benzyl) furo[3,2-d]pyrimidin-4-amine | 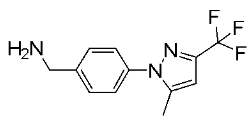<br>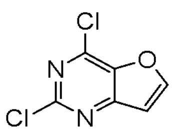<br>**A2-7** | LC-MS: m/z = 522.1 $(M+H)^+$ $^1$H NMR (400 MHz, DMSO) 8.60 (s, 1H), 8.32 (d, *J*=2.0Hz, 1H), 7.50 - 7.54 (m, 4H), 6.98 - 7.04 (m, 1H), 6.74 (m, 1H), 4.75 (s, 2H), 3.81 (s, 3H), 2.29 (s, 3H), 1.64 - 1.66 (m, 1H), 0.95 - 0.98 (m, 2H), 0.76 - 0.79 (m, 2H). |

(continued)

| Example | Structure and name of compound | Reaction moiety | Analyzed data |
|---|---|---|---|
| **57** | 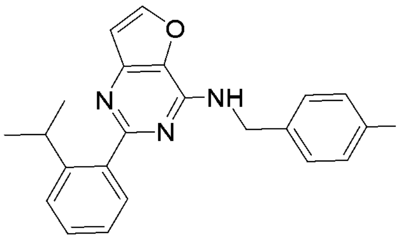 2-(2-Isopropylphenyl)-N-(4-(5-methyl-3-(trifluoromethyl)-1H-pyrazol-1-yl)benzyl)furo[3,2-d]pyrimidin-4-amine | 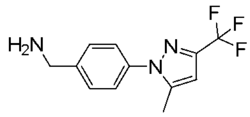 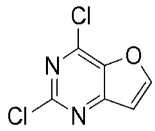 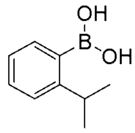 | LC-MS: m/z = 492.1 $(M+H)^+$ |

**Example 61: Synthesis of compound 61 (2-(2-isopropylphenyl)-5-methoxy-N-methyl-N-((4-(pyridin-2-yl)cyclohexyl)methyl)pyrimidin-4-amine)**

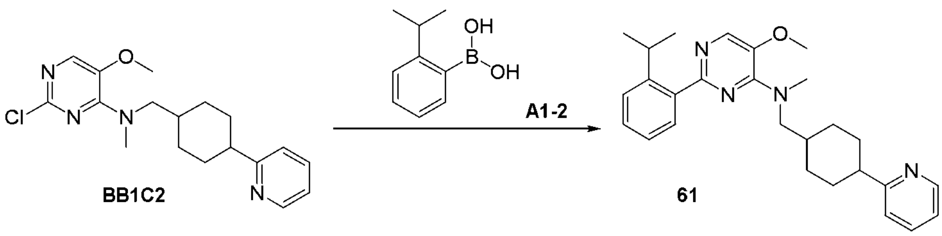


BB1C2

61

Compound **BB1C2** (125.0 mg, 0.36 mmol, 1.00 *eq),* compound **A1-2** (118.1 mg, 0.72 mmol, 2.00 *eq*), chloro(2-dicyclohexylphosphino-2',4',6'-triisopropyl-1,1'-biphenyl)[2-(2'-amino-1,1'-biphenyl)]palladium (II) (XPhos-Pd-G2, 56.58 mg, 0.072 mmol, 0.20 *eq*), 2-dicyclohexylphosphino-2',4',6'-triisopropylbiphenyl (XPhos, 68.65 mg, 0.144 mmol, 0.40 *eq)* and potassium phosphate (229.3 mg, 1.08 mmol, 3.00 *eq)* were added to dioxane (2.5 mL) and water (0.5 mL) successively. The resulting mixture was purged three times with nitrogen, and stirred at 95°C for 16 hours under nitrogen atmosphere. Water (15.0 mL) was added, and the resulting mixture was extracted three times with ethyl acetate (30.0 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **61** (93.5 mg, 0.22 mmol, 60.3% yield, 97.3% purity) as a white solid. LC-MS: m/z = 431.2 $(M+H)^+$.

In accordance with the preparation method of compound 61, the following compounds were obtained using intermediates **A series, AA series, BBC series or A series, AA series, BBD series** as starting materials, XPhos-Pd-G2/XPhos/$K_3PO_4$ as reaction reagents, and dioxane and water as solvents.

| Example | Structure and name of compound | Reaction moiety | Analyzed data |
|---|---|---|---|
| **62** | 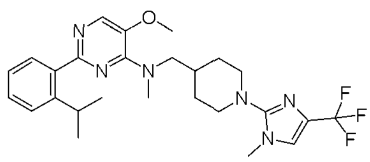 2-(2-Isopropylphenyl)-5-methoxy-N-methyl-N-((1-(1-methyl-4-(trifluoromethyl)-1H-imidazol-2-yl)piperidin-4-yl)methyl)pyrimidin-4-amine | **A1-2 and BB1C4** | LC-MS: m/z =503.2 $(M+H)^+$ |

(continued)

| Example | Structure and name of compound | Reaction moiety | Analyzed data |
|---|---|---|---|
| **63** | 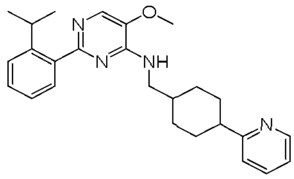 2-(2-Isopropylphenyl)-5-methoxy-N-((4-(pyridin-2-yl)cyclohexyl)methyl)pyrimidin-4-amine | **A1-2 and BB2C2** | LC-MS: m/z =417.2 $(M+H)^+$ |
| **64** | 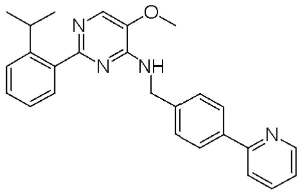 2-(2-Isopropylphenyl)-5-methoxy-N-(4-(pyridin-2-yl) benzyl)pyrimidin-4-amine | **A1-2 and BB2C3** | LC-MS: m/z =411.2 $(M+H)^+$ |
| **65** | 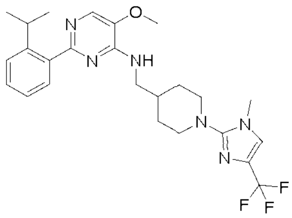 2-(2-Isopropylphenyl)-5-methoxy-N-((1-(1-methyl-4-(trifluoromethyl)-1H-imidazol-2-yl)piperidin-4-yl)methyl) pyrimidin-4-amine | **A1-2 and BB2C4** | LC-MS: m/z =489.2 $(M+H)^+$ |
| **66** | 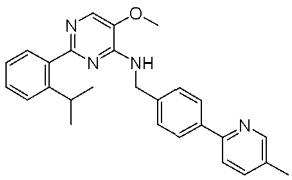 2-(2-Isopropylphenyl)-5-methoxy-N-(4-(5-methylpyridin-2-yl)benzyl)pyrimidin-4-amine | **A1-2 and BB2C14** | LC-MS: m/z =425.3 $(M+H)^+$ |
| **67** | 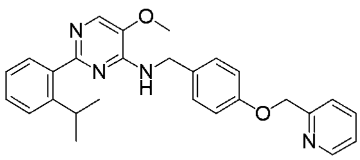 2-(2-Isopropylphenyl)-5-methoxy-N-(4-(pyridin-2-ylmethoxy)benzyl)pyrimidin-4-amine | **A1-2 and BB2C27** | LC-MS: m/z =441.1 $(M+H)^+$ |

(continued)

| Example | Structure and name of compound | Reaction moiety | Analyzed data |
|---|---|---|---|
| **68** | 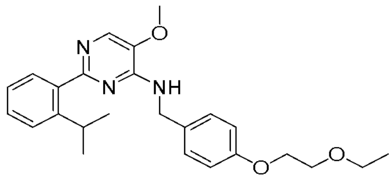 N-(4-(2-Ethoxyethoxy)benzyl)-2-(2-isopropylphenyl)-5-methoxypyrimidin-4-amine | **A1-2 and BB2C28** | LC-MS: m/z =422.2 $(M+H)^+$; $^1H$ NMR (400 MHz, DMSO) δ 7.92 (s, 1H), 7.50 - 7.56 (m, 1H), 7.38-7.43 (m, 1H), 7.28 - 7.36 (m, 2H), 7.13 - 7.23 (m, 3H), 6.84 - 6.86 (m, 2H), 4.49 - 4.54 (m, 2H), 3.99 - 4.05 (m, 2H), 3.90 (s, 3H), 3.63 - 3.68 (m, 2H), 3.44 - 3.55 (m, 3H), 1.11 (t, *J* = 7.0 Hz, 3H), 1.04 (d, *J* = 6.8 Hz, 6H). |
| **69** | 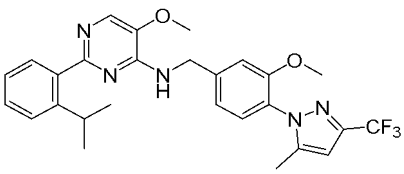 2-(2-Isopropylphenyl)-5-methoxy-N-(3-methoxy-4-(5-methyl-3-(trifluoromethyl)-1H-pyrazol-1-yl)benzyl)pyrimidin-4-amine | **A1-2 and BB2C33** | LC-MS: m/z =512.1 $(M+H)^+$; $^1$ H NMR (400 MHz, CDC13) δ 7.93 (s, 1H), 7.54 - 7.52 (m, 1H), 7.38 - 7.35 (m, 2H), 7.31 (s, 1H), 7.29 - 7.27 (m, 1H), 7.04 - 7.02 (m, 2H), 6.41 (s, 1H), 5.68 (s, 1H), 4.79 - 4.78 (m, 2H), 3.97 (s, 3H), 3.71 (s, 3H), 3.56 - 3.53 (m, 1H), 2.15 (s, 3H), 1.20 - 1.19 (m, 6H). |
| **70** | 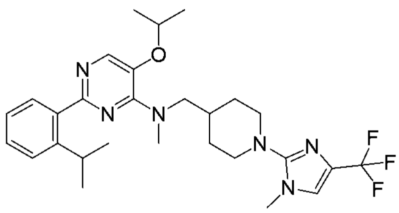 5-Isopropoxy-2-(2-isopropylphenyl)-N-methyl-N-((1-(1-methyl-4-(trifluoromethyl)-1H-imidazol-2-yl)piperidin-4-yl)methyl)pyrimidin-4-amine | **A1-2 and BB3C4** | LC-MS: m/z =531.3 $(M+H)^+$ |
| **71** | 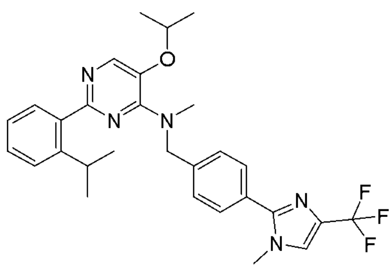 5-Isopropoxy-2-(2-isopropylphenyl)-N-methyl-N-(4-(1-methyl-4-(trifluoromethyl)-1H-imidazol-2-yl)benzyl)pyrimidin-4-amine | **A1-2 and BB3C5** | LC-MS: m/z =524.4 $(M+H)^+$; $^1$ H NMR (400 MHz, $CDCl_3$) δ 8.14 (s, 1H), 7.93 (s, 1H), 7.70 (d, *J* = 8.4 Hz, 2H), 7.49 - 7.47 (m, 1H), 7.39 - 7.32 (m, 4H), 7.21 - 7.17 (m, 1H), 4.97 (s, 2H), 4.72 - 4.66 (m, 1H), 3.77 (s, 3H), 3.63 - 3.56 (m, 1H), 3.17 (s, 3H), 1.23 (d, *J* = 6.0 Hz, 6H), 1.11 (d, *J*= 6.8 Hz, 6H). |

(continued)

| Example | Structure and name of compound | Reaction moiety | Analyzed data |
|---|---|---|---|
| 72 | 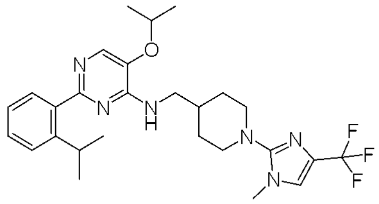 5-Isopropoxy-2-(2-isopropylphenyl)-N-((1-(1-methyl-4-(trifluoromethyl)-1H-imidazol-2-yl)piperidin-4-yl)methyl)pyrimidin-4-amine | A1-2 and BB4C4 | LC-MS: m/z =517.2 $(M+H)^+$ |
| 73 | 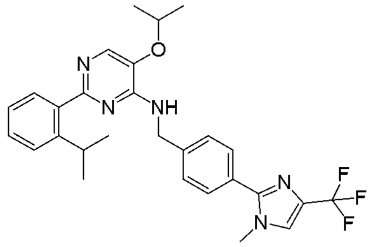 5-Isopropoxy-2-(2-isopropylphenyl)-N-(4-(1-methyl-4-(trifluoromethyl)-1H-imidazol-2-yl)benzyl)pyrimidin-4-amine | A1-2 and BB4C5 | LC-MS: m/z =510.3 $(M+H)^+$ |
| 74 | 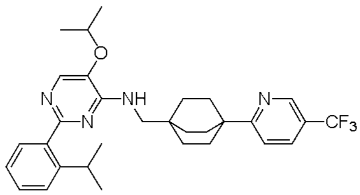 5-Isopropoxy-2-(2-isopropylphenyl)-N-((4-(5-(trifluoromethyl)pyridin-2-yl)bicyclo[2.2.2]octan-1-yl)methyl)pyrimidin-4-amine | A1-2 and BB4C19 | LC-MS: m/z =539.4 $(M+H)^+$ |
| 75 | (trifluoromethyl)pyrimidin-4-amine 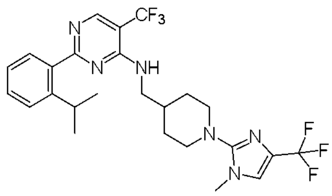 2-(2-Isopropylphenyl)-N-((1-(1-methyl-4-(trifluoromethyl)-1H-imidazol-2-yl)piperidin-4-yl)methyl)-5-(trifluoromethyl)pyrimidin-4-amine | A1-2 and BB12C4 | LC-MS: m/z =527.2 $(M+H)^+$ |
| 76 | 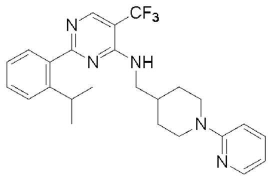 2-(2-Isopropylphenyl)-N-((1-(pyridin-2-yl)piperidin-4-yl)methyl)-5-(trifluoromethyl)pyrimidin-4-amine | A1-2 and BB12C6 | LC-MS: m/z =456.5 (M+H)+ |

(continued)

| Example | Structure and name of compound | Reaction moiety | Analyzed data |
|---|---|---|---|
| **77** | 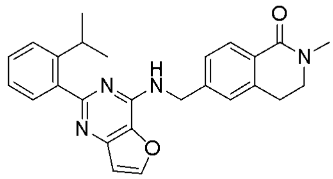 6-(((2-(2-Isopropylphenyl)furo[3,2-d]pyrimidin-4-yl)amino)methyl)-2-methyl-3,4-dihydroisoquinolin-1 (2H)-one | **A1-2 and BB13C25** | LC-MS: m/z =427.2 (M+H)+ |
| **78** | 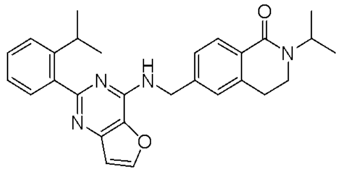 2-Isopropyl-6-(((2-(2-isopropylphenyl)furo[3,2-d]pyrimidin-4-yl)amino)methyl)-3,4-dihydroisoquinolin-1(2H)-one | **A1-2 and BB13C26** | LC-MS: m/z =455.1 (M+H)+ |
| **79** | 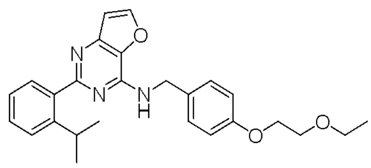 N-(4-(2-Ethoxyethoxy)benzyl)-2-(2-isopropylphenyl) furo[3,2-d]pyrimidin-4-amine | **A1-2 and BB13C28** | LC-MS: m/z =432.1 (M+H)+ |
| **80** | 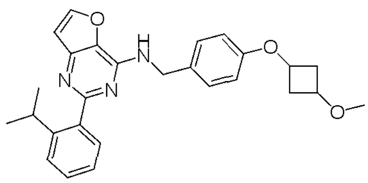 2-(2-Isopropylphenyl)-N-(4-(3-methoxycyclobutoxy) benzyl)furo[3, 2-d]pyrimidin-4-amine | **A1-2 and BB13C29** | LC-MS: m/z =444.2 (M+H)+ |
| **81** | 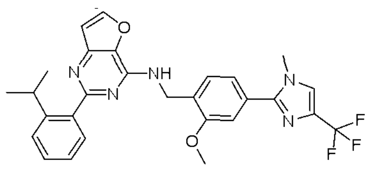 2-(2-Isopropylphenyl)-N-(2-methoxy-4-(1-methyl-4-(trifluoromethyl)-1H-imidazol-2-yl)benzyl)furo[3,2-d]pyrimidin-4-amine | **A1-2 and BB13C30** | LC-MS: m/z =522.2 (M+H)+ |
| **82** | 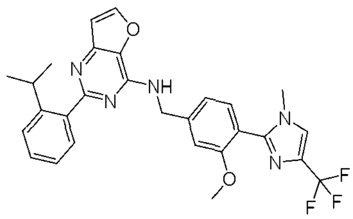 2-(2-Isopropylphenyl)-N-(3-methoxy-4-(1-methyl-4-(trifluoromethyl)-1H-imidazol-2-yl)benzyl)furo[3,2-d]pyrimidin-4-amine | **A1-2 and BB13C31** | LC-MS: m/z =522.0 (M+H)+ |

(continued)

| Example | Structure and name of compound | Reaction moiety | Analyzed data |
|---|---|---|---|
| **83** | 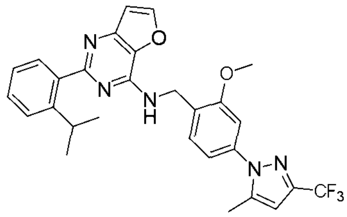 2-(2-Isopropylphenyl)-N-(2-methoxy-4-(5-methyl-3-(trifluoromethyl)-1H-pyrazol-1-yl)benzyl)furo[3,2-d]pyrimidin-4-amine | **A1-2 and BB13C32** | LC-MS: m/z =522.2 (M+H)+ |
| **84** | 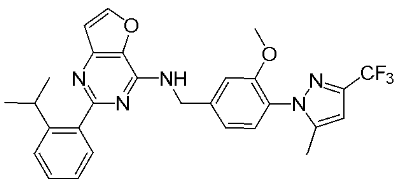 2-(2-Isopropylphenyl)-N-(3-methoxy-4-(5-methyl-3-(trifluoromethyl)-1H-pyrazol-1-yl)benzyl)furo[3,2-d]pyrimidin-4-amine | **A1-2 and BB13C33** | LC-MS: m/z =522.1 (M+H)+; $^1$ H NMR (400 MHz, $CDCl_3$) δ 7.80 (s, 1H), 7.79-7.55 (m, 1H), 7.41-7.40 (m, 2H), 7.38 (s, 1H), 7.33-7.27 (m, 1H), 7.08 (s, 2H), 6.94-6.93 (m, 1H), 6.42 (s, 1H), 5.60-5.57 (m, 1H), 4.95-4.94 (m, 2H), 3.71 (s, 3H), 3.52-3.49 (m, 1H), 2.15 (s, 3H), 1.22-1.19 (m, 6H). |
| **85** | 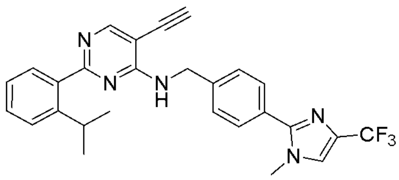 5-Ethynyl-2-(2-isopropylphenyl)-N-(4-(1-methyl-4-(trifluoromethyl)-1H-imidazol-2-yl)benzyl)pyrimidin-4-amine | **A1-2 and BB18D5** | LC-MS: m/z =476.1 (M+H)+; $^1$ H NMR (400 MHz, DMSO-$d_6$) δ 9.14 (s, 1H), 7.90 (s, 1H), 7.80 (d, J = 3.6 Hz, 1H), 7.67 (d, J = 8.3 Hz, 2H), 7.61 - 7.56 (m, 1H), 7.43 (dd, J = 14.1, 7.4 Hz, 2H), 7.36 (d, J = 8.3 Hz, 2H), 7.31 - 7.24 (m, 1H), 6.74 (d, J = 3.5 Hz, 1H), 5.58 (s, 2H), 3.73 (s, 3H), 3.55 - 3.49 (m, 1H), 1.12 (d, J = 6.9 Hz, 6H). |
| **86** | 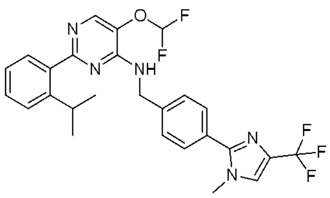 5-(Difluoromethoxy)-2-(2-isopropylphenyl)-N-(4-(1-methyl-4-(trifluoromethyl)-1H-imidazol-2-yl)benzyl)pyrimidin-4-amine | **A1-2 and BB19C5** | LC-MS: m/z =518.0 (M+H)+ |

(continued)

| Example | Structure and name of compound | Reaction moiety | Analyzed data |
|---|---|---|---|
| **87** | 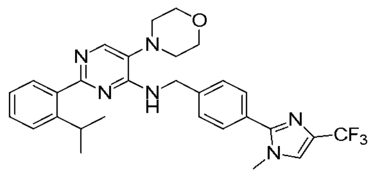<br>2-(2-Isopropylphenyl)-N-(4-(1-methyl-4-(trifluoromethyl)-1H-imidazol-2-yl)benzyl)-5-morpholinopyrimidin-4-amine | **A1-2 and BB21D5** | LC-MS: m/z =537.4 (M+H)+; $^1$ H NMR (400 MHz, DMSO-$d_6$) δ 8.07 (s, 1H), 7.90 (s, 1H), 7.65 (d, *J* = 8.2 Hz, 2H), 7.41 (d, *J* = 6.9 Hz, 2H), 7.38 (d, *J* = 8.2 Hz, 2H), 7.32 (d, *J* = 1.4 Hz, 1H), 7.18 - 7.12 (m, 1H), 4.73 (d, *J= 6.3* Hz, 2H), 3.83 - 3.80 (m, 4H), 3.75 (s, 3H), 3.57 - 3.48 (m, 1H), 2.97 - 2.93 (m, 4H), 0.99 (d, *J* = 6.9 Hz, 6H). |
| **88** | 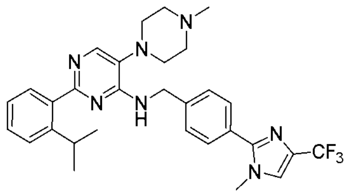<br>2-(2-Isopropylphenyl)-N-(4-(1-methyl-4-(trifluoromethyl)-1H-imidazol-2-yl)benzyl)-5-(4-methylpiperazin-1-yl)pyrimidin-4-amine | **A1-2 and BB22D5** | LC-MS: m/z =550.1 $(M+H)^+$; $^1$ H NMR (400 MHz, DMSO-$d_6$) δ 8.06 (s, 1H), 7.90 (s, 1H), 7.65 (d, J = 8.2 Hz, 2H), 7.41 (d, J = 8.3 Hz, 1H), 7.38 (d, J = 8.2 Hz, 2H), 7.31 (d, J = 1.4 Hz, 1H), 7.22 (s, 1H), 7.18 - 7.11 (m, 1H), 4.72 (d, J = 6.2 Hz, 2H), 3.75 (s, 3H), 3.54 (d, J = 6.8 Hz, 1H), 2.96 (s, 4H), 2.56 (s, 4H), 2.26 (s, 3H), 1.00 (d, J = 6.9 Hz, 6H). |
| **89** | 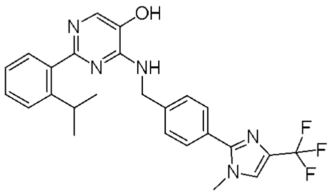<br>2-(2-Isopropylphenyl)-4-((4-(1-methyl-4-(trifluoromethyl)-1H-imidazol-2-yl)benzyl)amino)pyrimidin-5-ol | **A1-2 and BB24D5** | LC-MS: m/z =468.1 (M+H)+ |
| **90** | yl)benzyl)amino)pyrimidin-5-ol<br>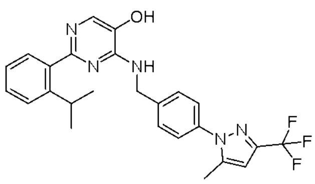<br>2-(2-Isopropylphenyl)-4-((4-(5-methyl-3-(trifluoromethyl)- 1H-pyrazol-1-vl)benzyl)amino)pyrimidin-5-ol | **A1-2 and BB24D11** | **LC-MS:** m/z =468.3 M+H)+ |

(continued)

| Example | Structure and name of compound | Reaction moiety | Analyzed data |
|---|---|---|---|
| **91** | 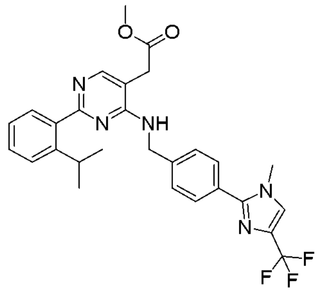 Methyl 2-(2-(2-isopropylphenyl)-4-((4-(1-methyl-4-(trifluoromethyl)-1H-imidazol-2-yl)benzyl)amino)pyrimidin-5-yl)acetate | **A1-2 and BB41D5** | LC-MS: m/z =524.2 (M+H)+ |
| **92** | 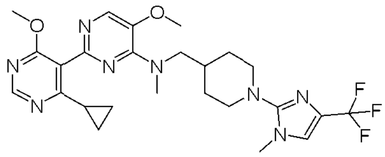 4'-Cyclopropyl-5,6'-dimethoxy-N-methyl-N-((1-(1-methyl-4-(trifluoromethyl)- 1 H-imidazol-2-yl)piperidin-4-yl)methyl)-[2,5'-bipyrimidin]-4-amine | **A2-7 and BB1C4** | LC-MS: m/z =533.4 (M+H)+ |
| **93** | 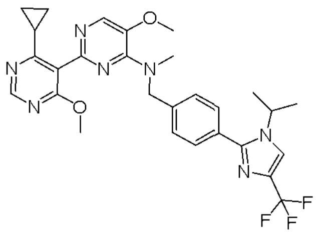 4'-Cyclopropyl-N-(4-(1-isopropyl-4-(trifluoromethyl)-1H-imidazol-2-yl)benzyl)-5,6'-dimethoxy-N-methyl-[2,5'-bipyrimidin]-4-amine | **A2-7 and BB1C58** | LC-MS: m/z =554.4 (M+H)+; $^1$H NMR (400 MHz, DMSO-$d_6$) δ 8.62 (s, 1H), 8.20 (d, *J* = 1.4 Hz, 1H), 8.16 (s, 1H), 7.61 - 7.52 (m, 2H), 7.43 (d, *J* = 8.2 Hz, 2H), 4.95 (s, 2H), 4.61 - 4.35 (m, 1H), 3.90 (s, 3H), 3.85 (s, 3H), 3.19 (s, 3H), 1.77 (s, 1H), 1.43 (d, *J* = 6.6 Hz, 6H), 1.00 (dt, *J* = 4.7, 3.0 Hz, 2H), 0.86 (dt, *J* = 8.1, 3.3 Hz, 2H). |
| **94** | 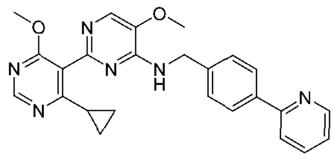 4'-Cyclopropyl-5,6'-dimethoxy-N-(4-(pyridin-2-yl)benzyl)-[2,5'-bipyrimidin]-4-amine | **A2-7 and BB2C3** | LC-MS: m/z =441.2 (M+H)+ |
| **95** | 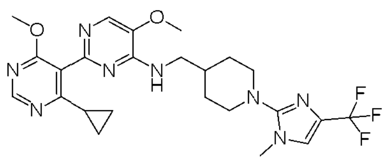 4'-Cyclopropyl-5,6'-dimethoxy-N-((1-(1-methyl-4-(trifluoromethyl)-1H-imidazol-2-yl)piperidin-4-yl) methyl)-[2,5'-bipyrimidin]-4-amine | **A2-7 and BB2C4** | LC-MS: m/z =519.3 (M+H)+ |

(continued)

| Example | Structure and name of compound | Reaction moiety | Analyzed data |
|---|---|---|---|
| **96** | 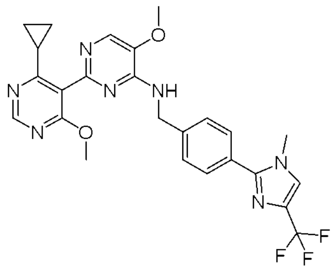 4'-Cyclopropyl-5,6'-dimethoxy-N-(4-(1-methyl-4-(trifluoromethyl)-1H-imidazol-2-yl)benzyl)-[2,5'-bipyrimidin]-4-amine | **A2-7 and BB2C5** | LC-MS: m/z =512.2 (M+H)+; $^1$H NMR (400 MHz, DMSO-$d_6$) δ 8.60 (s, 1H), 7.96 (d, *J* = 16.5 Hz, 2H), 7.86 (t, *J* = 6.4 Hz, 1H), 7.65 (d, *J* = 8.0 Hz, 2H), 7.43 (d, *J* = 8.0 Hz, 2H), 4.64 (d, *J* = 6.3 Hz, 2H), 3.96 (s, 3H), 3.83 (s, 3H), 3.78 (s, 3H), 1.70 (tt, *J* = 8.5, 4.7 Hz, 1H), 0.97 (s, 2H), 0.78 (dd, *J* = 7.8, 3.3 Hz, 2H). |
| **97** | 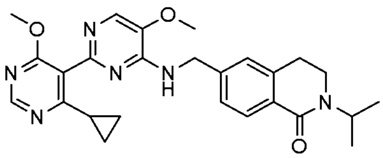 6-(((4'-Cyclopropyl-5,6'-dimethoxy-[2,5'-bipyrimidin]-4-yl)amino)methyl)-2-isopropyl-3,4-dihydroisoquinolin-1(2H)-one | **A2-7 and BB2C26** | LC-MS: m/z = 475.2 (M+H)+ |
| **98** | 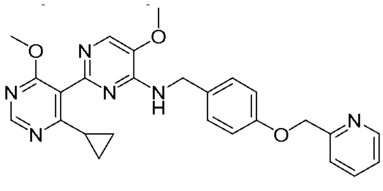 4'-Cyclopropyl-5,6'-dimethoxy-N-(4-(pyridin-2-ylmethoxy)benzyl)-[2,5'-bipyrimidin]-4-amine | **A2-7 and BB2C27** | LC-MS: m/z =471.1 (M+H)+ |
| **99** | 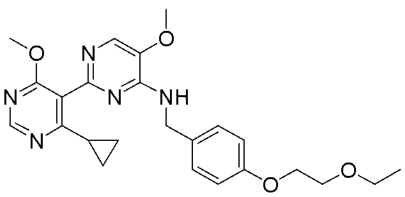 4'-Cyclopropyl-N-(4-(2-ethoxyethoxy)benzyl)-5,6'-dimethoxy-[2,5'-bipyrimidin]-4-amine | **A2-7 and BB2C28** | LC-MS: m/z =452.1 (M+H)+; $^1$H NMR (400 MHz, DMSO) δ 8.58 (s, 1H), 7.91 (s, 1H), 7.63 (t, *J* = 6.0 Hz, 1H), 7.16-7.22 (m, 2H), 6.81-6.86 (m, 2H), 4.43-4.47 (m, 2H), 4.00-4.04 (m, 2H), 3.89 (s, 3H), 3.82 (s, 3H), 3.63-3.68 (m, 2H), 3.48 (q, *J*= 6.8 Hz, 2H), 1.62-1.72 (m, 1H), 1.11 (t, *J*= 6.8 Hz, 3H), 0.93-0.99 (m, 2H), 0.75-0.81 (m, 2H). |

(continued)

| Example | Structure and name of compound | Reaction moiety | Analyzed data |
|---|---|---|---|
| **100** | 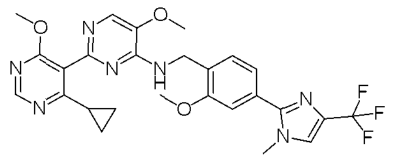 4'-Cyclopropyl-5,6'-dimethoxy-N-(2-methoxy-4-(1-methyl-4-(trifluoromethyl)-1H-imidazol-2-yl)benzyl)-[2,5'-bipyrimidin]-4-amine | **A2-7 and BB2C30** | LC-MS: m/z =542.1 (M+H)+ |
| **101** | 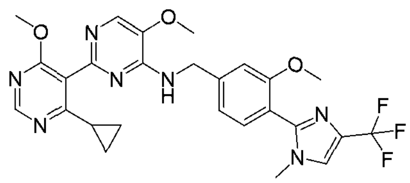 **4'-Cyclopropyl-5,6'-dimethoxy-N-(3-methoxy-4-(1-methyl-4-(trifluoromethyl)-1H-imidazol-2-yl)benzyl)-[2,5'-bipyrimidin]-4-amine** | **A2-7 and BB2C31** | LC-MS: m/z =542.0 (M+H)+ |
| **102** | 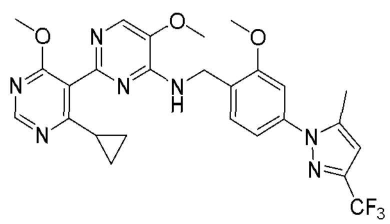 4'-Cyclopropyl-5,6'-dimethoxy-N-(2-methoxy-4-(5-methyl-3-(trifluoromethyl)-1H-pyrazol-1-yl)benzyl)-[2,5'-bipyrimidin]-4-amine | **A2-7 and BB2C32** | LC-MS: m/z =542.1 (M+H)+ |
| **103** | 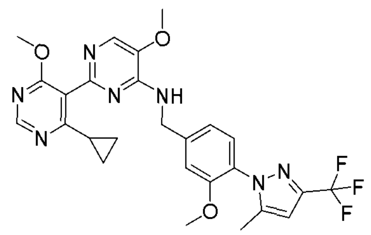 4'-Cyclopropyl-5,6'-dimethoxy-N-(3-methoxy-4-(5-methyl-3-(trifluoromethyl)-1H-pyrazol-1-yl)benzyl)-[2,5'-bipyrimidin]-4-amine | **A2-7 and BB2C33** | LC-MS: m/z =542.1 (M+H)+; $^1H$ NMR (400 MHz, CDCl3) δ 8.61 (s, 1H), 7.94 (s, 1H), 7.31 - 7.27 (m, 1H), 7.06 - 7.03 (m, 2H), 6.42 (s, 1H), 5.74 - 5.73 (m, 1H), 4.77 - 4.75 (m, 2H), 3.97 - 3.93 (m, 6H), 3.75 (s, 3H), 2.16 (m, 3H), 1.82 - 1.80 (m, 1H), 1.19 - 1.16 (m, 2H), 1.89 - 1.84 (m, 2H). |

(continued)

| Example | Structure and name of compound | Reaction moiety | Analyzed data |
|---|---|---|---|
| **104** | 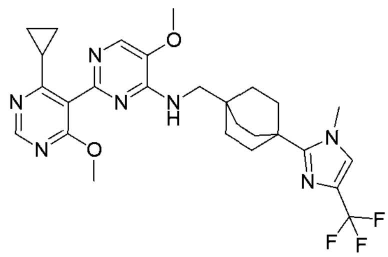 4'-Cyclopropyl-5,6'-dimethoxy-N-((4-(1-methyl-4-(trifluoromethyl)-1H-imidazol-2-yl)bicyclo[2.2.2]octan-1-yl) methyl)-[2,5'-bipyrimidin]-4-amine | **A2-7 and BB2C35** | LC-MS: m/z =544.3 (M+H)+; $^1$H NMR (400 MHz, DMSO-$d_6$) δ 8.62 (s, 1H), 7.90 (s, 1H), 7.65 (s, 1H), 6.99 (t, *J* = 6.5 Hz, 1H), 3.93 (s, 3H), 3.84 (s, 3H), 3.77 (s, 3H), 3.21 (d, *J*= 6.5 Hz, 2H), 1.94-1.85 (m, 6H), 1.77-1.69 (m, 1H), 1.52-1.46 (m, 6H), 1.06 - 1.01 (m, 2H), 0.92 - 0.85 (m, 2H). |
| **105** | 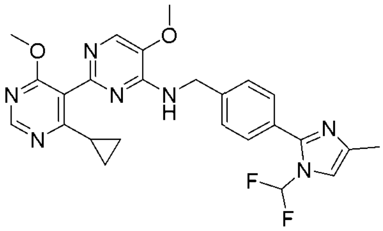 4'-Cyclopropyl-N-(4-(1-(difluoromethyl)-4-methyl-1H-imidazol-2-yl)benzyl)-5,6'-dimethoxy-[2,5'-bipyrimidin]-4-amine | **A2-7 and BB2C48** | LC-MS: m/z =494.2 (M+H)+; $^1$H NMR (400 MHz, DMSO-$d_6$) δ 8.60 (s, 1H), 7.98 (s, 1H), 7.82 (t, *J* = 6.3 Hz, 1H), 7.67 - 7.62 (m, 3H), 7.42 (d, *J* = 8.1 Hz, 2H), 7.07 - 6.79 (m, 1H),4.64 (d, *J* = 6.3 Hz, 2H), 3.96 (s, 3H), 3.83 (s, 3H), 3.76 (s, 3H), 1.71 (dt, *J* = 8.1, 3.5 Hz, 1H), 1.02 - 0.93 (m, 2H), 0.86 - 0.71 (m, 2H). |
| **106** | 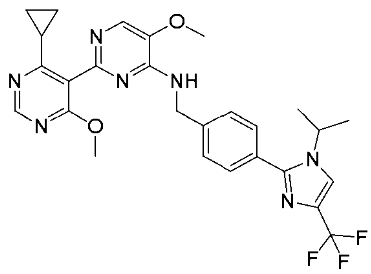 4'-Cyclopropyl-N-(4-(1-isopropyl-4-(trifluoromethyl)-1H-imidazol-2-yl)benzyl)-5,6'-dimethoxy-[2,5'-bipyrimidin]-4-amine | **A2-7 and BB2C58** | LC-MS: m/z =540.2 (M+H)+; $^1$H NMR (400 MHz, DMSO-$d_6$) δ 8.59 (s, 1H), 8.19 (d, *J* = 1.4 Hz, 1H), 7.98 (s, 1H), 7.87 (t, *J* = 6.3 Hz, 1H), 7.49 (d, *J* = 8.0 Hz, 2H), 7.43 (d, *J* = 8.1 Hz, 2H), 4.64 (d, *J* = 6.3 Hz, 2H), 4.54 - 4.34 (m, 1H), 3.96 (s, 3H), 3.82 (s, 3H), 1.69 (s, 1H), 1.41 (d, *J* = 6.6 Hz, 6H), 0.95 (t, *J* = 3.8 Hz, 2H), 0.80 - 0.69 (m, 2H). |

(continued)

| Example | Structure and name of compound | Reaction moiety | Analyzed data |
|---|---|---|---|
| **107** | 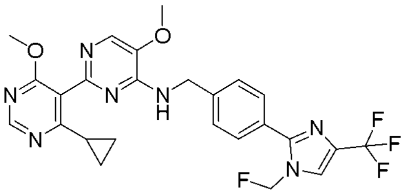<br>4'-Cyclopropyl-N-(4-(1-(fluoromethyl)-4-(trifluoromethyl)-1H-imidazol-2-yl)benzyl)-5,6'-dimethoxy-[2,5'-bipyrimidin]-4-amine | **A2-7 and BB2C59** | LC-MS: m/z =530.2 $(M+H)^+$;<br>$^1H$ NMR (400 MHz, $CDCl_3$) $\delta$ 8.58 (s, 1H), 7.91 (s, 1H), 7.67 (d, *J* = 8.1 Hz, 2H), 7.52 (s, 1H), 7.48 (d, *J* = 8.0 Hz, 2H), 5.92 (s, 1H), 5.77 - 5.69 (m, 1H), 5.73 (s, 1H), 4.77 (d, *J* = 6.1 Hz, 2H), 3.94 (s, 3H), 3.91 (s, 3H), 1.83 - 1.73 (m, 1H), 1.17 - 1.12 (m, 2H), 0.85 - 0.78 (m, 2H). |
| **108** | 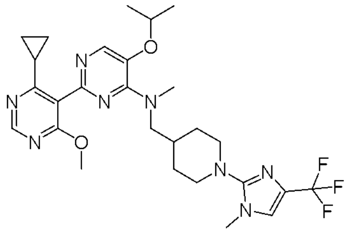<br>4'-Cyclopropyl-5-isopropoxy-6'-methoxy-N-methyl-N-((1-(1-methyl-4-(trifluoromethyl)-1H-imidazol-2-yl)piperidin-4-yl)methyl)-[2,5'-bipyrimidin]-4-amine | **A2-7 and BB3C4** | LC-MS: m/z =561.2 (M+H)+ |
| **109** | 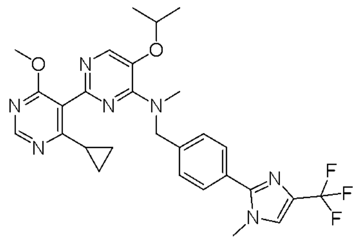<br>4'-Cyclopropyl-5-isopropoxy-6'-methoxy-N-methyl-N-(4-(1-methyl-4-(trifluoromethyl)-1H-imidazol-2-yl)benzyl)-[2,5'-bipyrimidin]-4-amine | **A2-7 and BB3C5** | LC-MS: m/z =554.3 (M+H)+ |
| **110** | 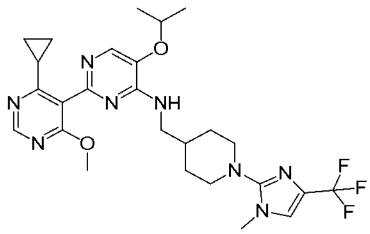<br>4'-Cyclopropyl-5-isopropoxy-6'-methoxy-N-((1-(1-methyl-4-(trifluoromethyl)- 1H-imidazol-2-yl)piperidin-4-yl)methyl)-[2,5'-bipyrimidin]-4-amine | **A2-7 and BB4C4** | LC-MS: m/z =547.4 (M+H)+ |

(continued)

| Example | Structure and name of compound | Reaction moiety | Analyzed data |
|---|---|---|---|
| **111** | 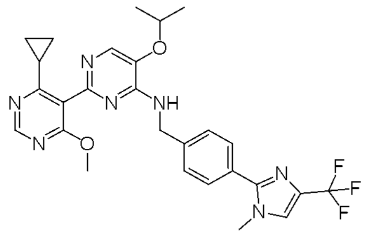 4'-Cyclopropyl-5-isopropoxy-6'-methoxy-N-(4-(1-methyl-4-(trifluoromethyl)-1H-imidazol-2-yl)benzyl)-[2,5'-bipyrimidin]-4-amine | **A2-7 and BB4C5** | LC-MS: m/z =540.2 (M+H)+ |
| **112** | 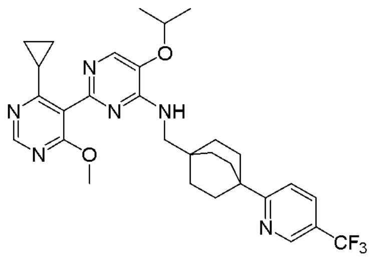 4'-Cyclopropyl-5-isopropoxy-6'-methoxy-N-((4-(5-(trifluoromethyl)pyridin-2-yl)bicyclo[2.2.2]octan-1-yl)methyl)-[2,5'-bipyrimidin]-4-amine | **A2-7 and BB4C19** | LC-MS: m/z =569.4 (M+H)+ |
| **113** | 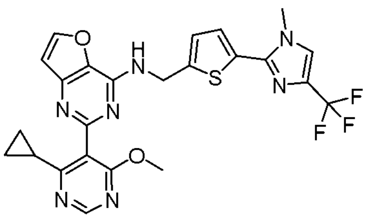 2-(4-Cyclopropyl-6-methoxypyrimidin-5-yl)-N-((5-(1-methyl-4-(trifluoromethyl)- 1H-imidazol-2-yl)thiophen-2-yl)methyl)furo[3,2-d]pyrimidin-4-amine | **A2-7 and BB13C7** | LC-MS: m/z =528.3 (M+H)+ |
| **114** | 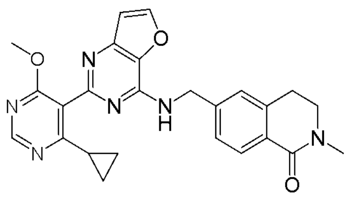 6-(((2-(4-Cyclopropyl-6-methoxypyrimidin-5-yl)furo[3,2-d]pyrimidin-4-yl)amino)methyl)-2-methyl-3,4-dihydroisoquinolin-1(2H)-one | **A2-7 and BB13C25** | LC-MS: m/z =457.1 (M+H)+ |
| **115** | 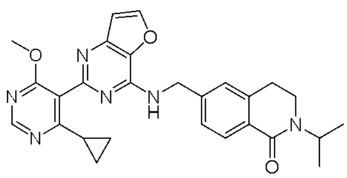 6-(((2-(4-Cyclopropyl-6-methoxypyrimidin-5-yl)furo[3,2-d]pyrimidin-4-yl)amino)methyl)-2-isopropyl-3,4-dihydroisoquinolin-1(2H)-one | **A2-7 and BB13C26** | LC-MS: m/z =485.2 (M+H)+ |

(continued)

| Example | Structure and name of compound | Reaction moiety | Analyzed data |
|---|---|---|---|
| **116** | 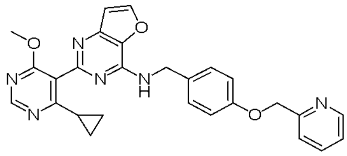<br>2-(4-Cyclopropyl-6-methoxypyrimidin-5-yl)-N-(4-(pyridin-2-ylmethoxy)benzyl)furo[3,2-d]pyrimidin-4-amine | **A2-7 and BB13C27** | LC-MS: m/z =481.1 (M+H)+ |
| **117** | 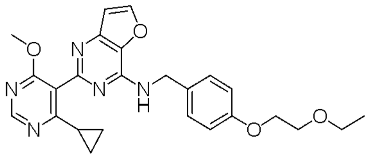<br>2-(4-Cyclopropyl-6-methoxypyrimidin-5-yl)-N-(4-(2-ethoxyethoxy)benzyl)furo[3,2-d]pyrimidin-4-amine | **A2-7 and BB13C28** | LC-MS: m/z =462.1 (M+H)+ |
| **118** | 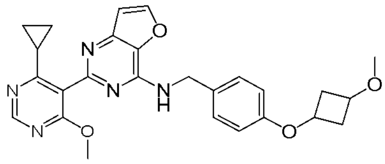<br>2-(4-Cyclopropyl-6-methoxypyrimidin-5-yl)-N-(4-(3-methoxycyclobutoxy)benzyl)furo[3, 2-d]pyrimidin-4-amine | **A2-7 and BB13C29** | LC-MS: m/z =474.2 (M+H)+ |
| **119** | 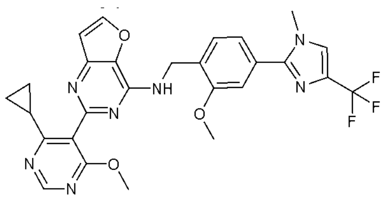<br>2-(4-Cyclopropyl-6-methoxypyrimidin-5-yl)-N-(2-methoxy-4-(1-methyl-4-(trifluoromethyl)- 1H-imidazol-2-yl) benzyl)furo[3,2-d]pyrimidin-4-amine | **A2-7 and BB13C30** | LC-MS: m/z =552.0 (M+H)+ |
| **120** | 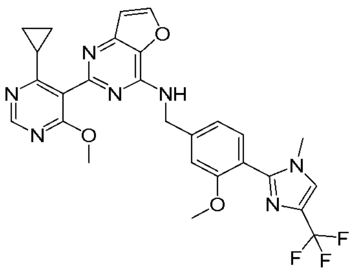<br>2-(4-Cyclopropyl-6-methoxypyrimidin-5-yl)-N-(3-methoxy-4-(1-methyl-4-(trifluoromethyl)- 1H-imidazol-2-yl) benzyl)furo[3,2-d]pyrimidin-4-amine | **A2-7 and BB13C31** | LC-MS: m/z =552.0 (M+H)+ |

(continued)

| Example | Structure and name of compound | Reaction moiety | Analyzed data |
|---|---|---|---|
| **121** | 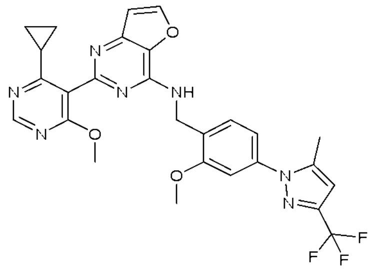 2-(4-Cyclopropyl-6-methoxypyrimidin-5-yl)-N-(2-methoxy-4-(5-methyl-3-(trifluoromethyl)-1H-pyrazol-1-yl) benzyl)furo[3,2-d]pyrimidin-4-amine | **A2-7 and BB13C32** | LC-MS: m/z =552.1 (M+H)+ |
| **122** | 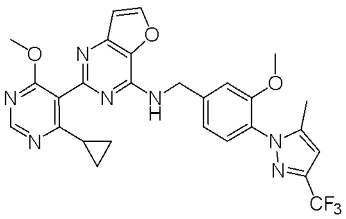 2-(4-Cyclopropyl-6-methoxypyrimidin-5-yl)-N-(3-methoxy-4-(5-methyl-3-(trifluoromethyl)-1H-pyrazol-1-yl) benzyl)furo[3,2-d]pyrimidin-4-amine | **A2-7 and BB13C33** | LC-MS: m/z =552.2 (M+H)+; $^1H$ NMR (400 MHz, CDCl3) δ 8.64 (s, 1H), 7.82 (s, 1H), 7.33 - 7.27 (m, 1H), 7.11 - 7.08 (m, 2H), 6.45 (s, 1H), 6.42 (s, 1H), 5.67 (s, 1H), 4.97-4.93 (m, 2H), 3.93 (s, 3H), 3.76 (s, 3H), 2.15 (s, 3H), 1.79 - 1.76 (m, 1H), 1.21 - 1.19 (m, 2H), 0.88 - 0.86 (m, 2H). |
| **123** | 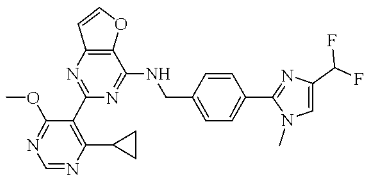 2-(4-Cyclopropyl-6-methoxypyrimidin-5-yl)-N-(4-(4-(difluoromethyl)-1-methyl-1H-imidazol-2-yl)benzyl)furo [3,2-d]pyrimidin-4-amine | **A2-7 and BB13C48** | LC-MS: m/z =504.2 (M+H)+; $^1H$ NMR (400 MHz, DMSO-$d_6$) δ 8.66 (s, 1H), 8.62 (s, 1H), 8.33 (d, *J* = 2.2 Hz, 1H), 7.63 (d, *J* = 8.1 Hz, 2H), 7.48 (d, *J* = 8.0 Hz, 2H), 7.42 - 7.34 (m, 1H), 7.12 - 7.07 (m, 1H), 7.05 (d, *J* = 2.2 Hz, 1H), 4.77 (d, *J* = 6.1 Hz, 2H), 3.83 (s, 3H), 3.73 (s, 3H), 1.68 (t, *J* = 7.8 Hz, 1H), 0.98 (d, *J* = 4.2 Hz, 2H), 0.78 (s, 2H). |

(continued)

| Example | Structure and name of compound | Reaction moiety | Analyzed data |
|---|---|---|---|
| **124** | 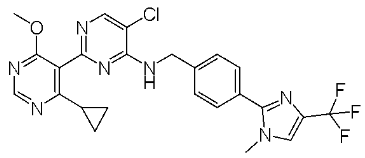<br>5-Chloro-4'-cyclopropyl-6'-methoxy-N-(4-(1-methyl-4-(trifluoromethyl)-1H-imidazol-2-yl)benzyl)-[2,5'-bipyrimidin]-4-amine | **A2-7 and BB15C5** | LC-MS: m/z =516.0 (M+H)+; $^1$H NMR (400 MHz, DMSO-*d*$_6$) δ 8.61 (s, 1H), 8.42 (s, 1H), 8.29 (t, *J* = 6.2 Hz, 1H), 7.92 (d, *J* = 1.4 Hz, 1H), 7.76 - 7.54 (m, 2H), 7.41 (d, *J* = 8.2 Hz, 2H), 4.67 (d, *J* = 6.1 Hz, 2H), 3.81 (s, 3H), 3.75 (s, 3H), 1.77 - 1.59 (m, 1H), 0.95 (p, *J* = 3.7 Hz, 2H), 0.77 (dq, *J* = 7.0, 3.5 Hz, 2H). |
| **125** | 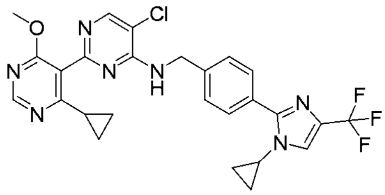<br>5-Chloro-4'-cyclopropyl-N-(4-(1-cyclopropyl-4-(trifluoromethyl)-1H-imidazol-2-yl)benzyl)-6'-methoxy-[2,5'-bipyrimidin]-4-amine | **A2-7 and BB15C57** | LC-MS: m/z =542.4 (M+H)$^+$; $^1$H NMR (400 MHz, MeOH-*d*$_4$) δ 8.53 (s, 1H), 8.29 (s, 1H), 7.74 (d, *J* = 8.2 Hz, 2H), 7.69 (s, 1H), 7.46 (d, *J* = 7.9 Hz, 2H), 4.78 (s, 2H), 3.88 (s, 3H), 3.66 - 3.56 (m, 1H), 1.74 - 1.63 (m, 1H), 1.07 - 1.02 (m, 2H), 1.01 - 0.96 (m, 2H), 0.85 - 0.77 (m, 4H). |
| **126** | 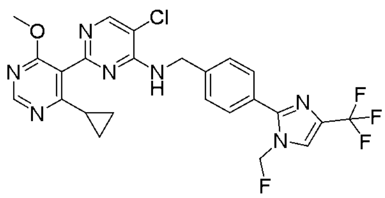<br>5-Chloro-4'-cyclopropyl-N-(4-(1-(fluoromethyl)-4-(trifluoromethyl)-1H-imidazol-2-yl)benzyl)-6'-methoxy-[2,5'-bipyrimidin]-4-amine | **A2-7 and BB15C59** | LC-MS: m/z =534.3 (M+H)$^+$; $^1$H NMR (400 MHz, DMSO-*d*$_6$) δ 8.57 (s, 1H), 8.39 (s, 1H), 8.33 - 8.22 (m, 2H), 7.60 (d, *J* = *8.2* Hz, 2H), 7.43 (d, *J* = 8.0 Hz, 2H), 6.05 (d, *J* = 51.7 Hz, 2H), 4.64 (d, *J* = 6.1 Hz, 2H), 3.77 (s, 3H), 1.71 - 1.58 (m, 1H), 0.96 - 0.88 (m, 2H), 0.78 - 0.69 (m, 2H).. |

(continued)

| Example | Structure and name of compound | Reaction moiety | Analyzed data |
|---|---|---|---|
| **127** | 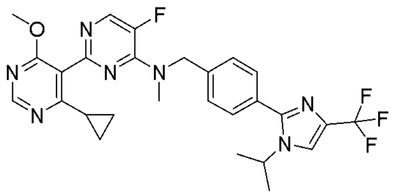 4'-Cyclopropyl-5-fluoro-N-(4-(1-isopropyl-4-(trifluoromethyl)-1H-imidazol-2-yl)benzyl)-6'-methoxy-N-methyl-[2,5'-bipyrimidin]-4-amine | **A2-7 and BB16C58** | LC-MS: m/z =542.0 (M+H)+; $^1$H NMR (400 MHz, DMSO-$d_6$) δ 8.61 (s, 1H), 8.39 (d, *J* = 6.8 Hz, 1H), 8.18 (s, 1H), 7.54 (d, *J* = 8.0 Hz, 2H), 7.43 (d, *J* = 8.1 Hz, 2H), 4.90 (s, 2H), 4.50 - 4.40 (m, 1H), 3.84 (s, 3H), 3.27 - 3.23 (m, 3H), 1.78 - 1.70 (m, 1H), 1.40 (d, *J* = 6.6 Hz, 6H), 1.01 - 0.95 (m, 2H), 0.86 - 0.79 (m, 2H). |
| **128** | 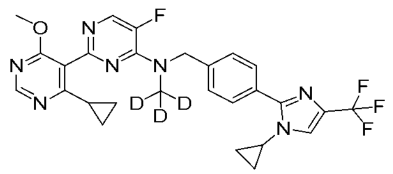 4'-Cyclopropyl-N-(4-(1-cyclopropyl-4-(trifluoromethyl)-1H-imidazol-2-yl)benzyl)-5-fluoro-6'-methoxy-N-(methyl-d3)-[2,5'-bipyrimidin]-4-amine | **A2-7 and BB16-D3C57** | LC-MS: m/z =543.2 (M+H)+; $^1$H NMR (400 MHz, MeOH-$d_4$) δ 8.53 (s, 1H), 8.21 (d, *J* = 6.9 Hz, 1H), 7.79 (d, *J* = 8.3 Hz, 2H), 7.71 (s, 1H), 7.45 (d, *J* = *8.2* Hz, 2H), 4.97 (s, 2H), 3.90 (s, 3H), 3.69 - 3.56 (m, 1H), 1.81 - 1.71 (m, 1H), 1.10 - 1.05 (m, 2H), 1.04 - 0.98 (m, 2H), 0.89 - 0.83 (m, 4H). |
| **129** | 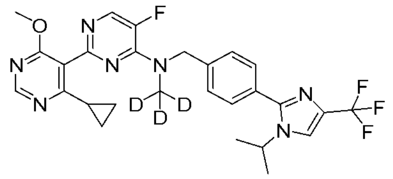 4'-Cyclopropyl-5-fluoro-N-(4-(1-isopropyl-4-(trifluoromethyl)-1H-imidazol-2-yl)benzyl)-6'-methoxy-N-(methyl-d3)-[2,5'-bipyrimidin]-4-amine | **A2-7 and BB16-D3C58** | LC-MS: m/z =545.4 (M+H)+; $^1$H NMR (400 MHz, DMSO-$d_6$) δ 8.61 (s, 1H), 8.38 (d, *J* = 6.8 Hz, 1H), 8.18 (s, 1H), 7.54 (d, *J* = 8.1 Hz, 2H), 7.43 (d, *J* = 7.9 Hz, 2H), 4.90 (s, 2H), 4.52 - 4.40 (m, 1H), 3.84 (s, 3H), 1.78 - 1.70 (m, 1H), 1.40 (d, *J* = 6.6 Hz, 6H), 1.00 - 0.96 (m, 2H), 0.85 - 0.81 (m, 2H). |

(continued)

| Example | Structure and name of compound | Reaction moiety | Analyzed data |
|---|---|---|---|
| **130** | 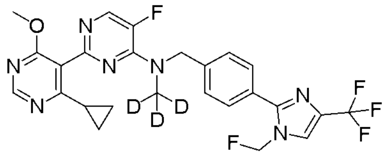 4'-Cyclopropyl-5-fluoro-N-(4-(1-(fluoromethyl)-4-(trifluoromethyl)-1H-imidazol-2-yl)benzyl)-6'-methoxy-N-(methyl-d3)-[2,5'-bipyrimidin]-4-amine | **A2-7 and BB16-D3C59** | LC-MS: m/z =535.1 (M+H)+; $^1$H NMR (400 MHz, DMSO-$d_6$) δ 8.58 (s, 1H), 8.35 (d, *J* = 6.7 Hz, 1H), 8.27 (s, 1H), 7.64 (d, *J* = 8.0 Hz, 2H), 7.45 (d, *J* = 8.0 Hz, 2H), 6.07 (d, *J* = 51.6 Hz, 2H), 4.87 (s, 2H), 3.80 (s, 3H), 1.77 - 1.67 (m, 1H), 0.99 - 0.93 (m, 2H), 0.85 - 0.77 (m, 2H). |
| **131** | 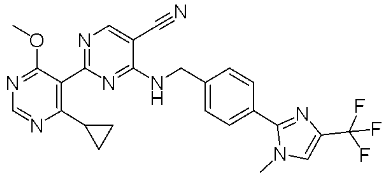 4'-Cyclopropyl-6'-methoxy-4-((4-(1-methyl-4-(trifluoromethyl)-1H-imidazol-2-yl)benzyl)amino)-[2,5'-bipyrimidine]-5-carbonitrile | **A2-7 and BB17C5** | LC-MS: m/z =507.3 (M+H)+ |
| **132** | 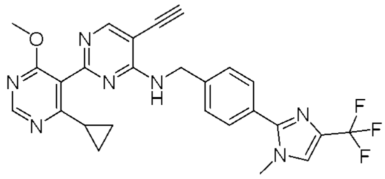 4'-Cyclopropyl-5-ethynyl-6'-methoxy-N-(4-(1-methyl-4-(trifluoromethyl)-1H-imidazol-2-yl)benzyl)-[2,5'-bipyrimidin]-4-amine | **A2-7 and BB18D5** | LC-MS: m/z =506.4 (M+H)+ |
| **133** | 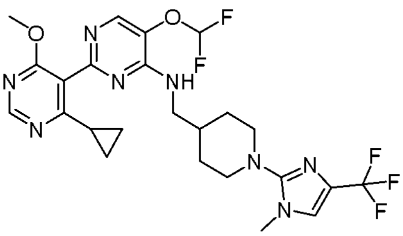 4'-Cyclopropyl-5-(difluoromethoxy)-6'-methoxy-N-((1-(1-methyl-4-(trifluoromethyl)- 1H-imidazol-2-yl)piperidin-4-yl)methyl)-[2,5'-bipyrimidin]-4-amine | **A2-7 and BB19C4** | LC-MS: m/z =555.1 (M+H)+; $^1$H NMR (400 MHz, $CDCl_3$) δ 8.63 (s, 1H), 8.18 (s, 1H), 7.01 - 7.00 (m, 1H), 6.80 - 6.44 (m, 1H), 5.43 (s, 1H), 3.94 (s, 3H), 3.54 - 3.52 (m, 2H), 3.51 (s, 3H), 3.29 - 3.26 (m, 2H), 2.93 - 2.87 (m, 2H), 1.85 - 1.79 (m, 4H), 1.47 - 1.43 (m, 2H), 1.22 - 1.21 (m, 2H), 0.94 - 0.91 (m, 2H). |

(continued)

| Example | Structure and name of compound | Reaction moiety | Analyzed data |
|---|---|---|---|
| **134** | 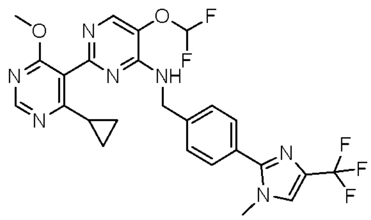 4'-Cyclopropyl-5-(difluoromethoxy)-6'-methoxy-N-(4-(1-methyl-4-(trifluoromethyl)-1H-imidazol-2-yl)benzyl)-[2,5'-bipyrimidin]-4-amine | **A2-7 and BB19C5** | LC-MS: m/z =548.4 (M+H)+ |
| **135** | 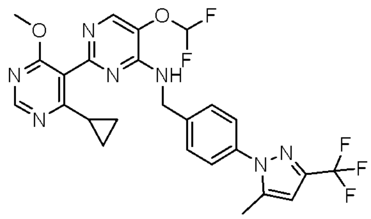 4'-Cyclopropyl-5-(difluoromethoxy)-6'-methoxy-N-(4-(5-methyl-3-(trifluoromethyl)-1H-pyrazol-1-yl)benzyl)-[2,5'-bipyrimidin]-4-amine | **A2-7 and BB19C11** | LC-MS: m/z =548.2 (M+H)+ |
| **136** | 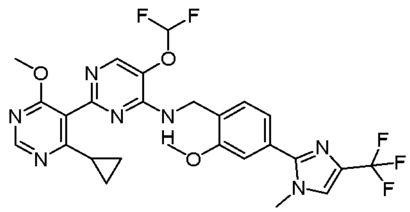 4'-Cyclopropyl-5-(difluoromethoxy)-6'-methoxy-N-(2-methoxy-4-(1-methyl-4-(trifluoromethyl)-1H-imidazol-2-yl)benzyl)-[2,5'-bipyrimidin]-4-amine | **A2-7 and BB19C30** | LC-MS: m/z =578.0 (M+H)+ |
| **137** | 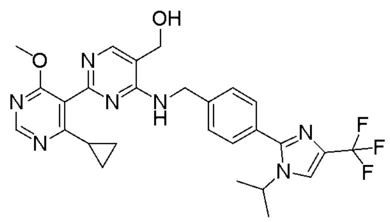 (4'-Cyclopropyl-4-((4-(1-isopropyl-4-(trifluoromethyl)-1H-imidazol-2-yl)benzyl)amino)-6'-methoxy-[2,5'-bipyrimidin]-5-yl)methanol | **A2-7 and BB35D58** | LC-MS: m/z =540.1 (M+H)+; $^1$H NMR (400 MHz, $CDCl_3$) δ 8.60 (s, 1H), 7.90 (s, 1H), 7.45 - 7.38 (m, 4H), 7.26 (s, 1H), 6.7 - 6.63 (m, 1H), 4.80 - 4.72 (m, 2H), 4.58 - 4.46 (m, 3H), 4.32 - 4.12 (m, 1H), 3.92 (s, 3H), 1.81 - 1.72 (m, 1H), 1.44 (d, *J* = 6.6 Hz, 6H), 1.19 - 1.11 (m, 2H), 0.83 - 0.76 (m, 2H). |

(continued)

| Example | Structure and name of compound | Reaction moiety | Analyzed data |
|---|---|---|---|
| **138** | 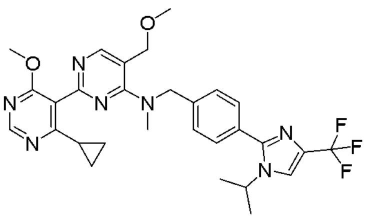<br>4'-Cyclopropyl-N-(4-(1-isopropyl-4-(trifluoromethyl)-1H-imidazol-2-yl)benzyl)-6'-methoxy-5-(methoxymethyl)-N-methyl-[2,5'-bipyrimidin]-4-amine | **A2-7 and BB36C58** | LC-MS: m/z =568.4 $(M+H)^+$; $^1H$ NMR (400 MHz, DMSO-$d_6$) δ 8.60 (s, 1H), 8.31 (s, 1H), 8.17 (s, 1H), 7.53 (d, *J*= 7.9 Hz, 2H), 7.38 (d, *J* = 7.8 Hz, 2H), 4.95 (s, 2H), 4.49 - 4.43 (m, 1H), 4.42 (s, 2H), 3.82 (s, 3H), 3.31 (s, 3H), 3.27 (s, 3H), 1.76 - 1.68 (m, 1H), 1.40 (d, *J*= 6.6 Hz, 6H), 0.99 - 0.94 (m, 2H), 0.84 - 0.78 (m, 2H). |
| **139** | 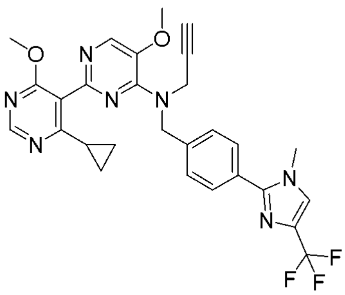<br>4'-Cyclopropyl-5,6'-dimethoxy-N-(4-(1-methyl-4-(trifluoromethyl)-1H-imidazol-2-yl)benzyl)-N-(prop-2-yn-1-yl)-[2,5'-bipyrimidin]-4-amine | **A2-7 and BB42C5** | LC-MS: m/z =550.2 $(M+H)^+$; $^1H$ NMR (400 MHz, MeOH-$d_4$) δ 8.53 (s, 1H), 8.09 (s, 1H), 7.67 (s, 1H), 7.60 (d, *J* = 8.2 Hz, 2H), 7.50 (d, *J*= 8.2 Hz, 2H), 5.07 (s, 2H), 4.45 (d, *J* = 2.3 Hz, 2H), 3.92 (s, 3H), 3.89 (s, 3H), 3.76 (s, 3H), 2.66 - 2.62 (m, 1H), 1.83 - 1.75 (m, 1H), 1.12 - 1.02 (m, 2H), 0.95 - 0.82 (m, 2H). |
| **140** | 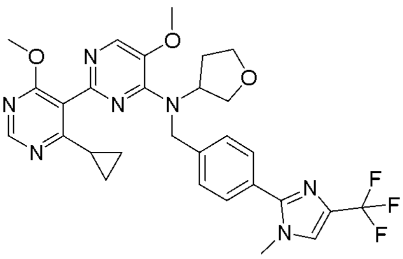<br>4'-Cyclopropyl-5,6'-dimethoxy-N-(4-(1-methyl-4-(trifluoromethyl)-1H-imidazol-2-yl)benzyl)-N-(tetrahydrofuran-3-yl)-[2,5'-bipyrimidin]-4-amine | **A2-7 and BB43C5** | LC-MS: m/z =582.2 (M+H)+ |
| **141** | bipyrimidin]-4-amine<br>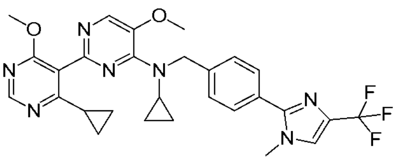<br>N,4'-Dicyclopropyl-5,6'-dimethoxy-N-(4-(1-methyl-4-(trifluoromethyl)-1H-imidazol-2-yl)benzyl)-[2,5'-bipyrimidin]-4-amine | **A2-7 and BB44C5** | LC-MS: m/z =552.3 (M+H)+ |

(continued)

| Example | Structure and name of compound | Reaction moiety | Analyzed data |
|---|---|---|---|
| **142** | 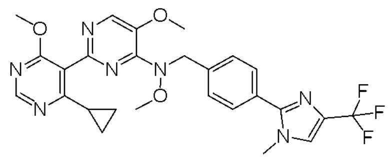 N-(4'-Cyclopropyl-5,6'-dimethoxy-[2,5'-bipyrimidin]-4-yl)-O-methyl-N-(4-(1-methyl-4-(trifluoromethyl)-1H-imidazol-2-yl)benzyl)hydroxylamine | **A2-7 and BB45C5** | LC-MS: m/z =542.2 (M+H)+; 1H NMR (400 MHz, DMSO-d6) δ 8.65 (s, 1H), 8.43 (s, 1H), 7.95 (d, J = 1.4 Hz, 1H), 7.79 - 7.60 (m, 2H), 7.58 - 7.43 (m, 2H), 4.92 (s, 2H), 4.02 (s, 3H), 3.84 (s, 3H), 3.80 (s, 3H), 3.67 (s, 3H), 1.62 (d, J = 4.4 Hz, 1H), 1.09 - 0.95 (m, 2H), 0.85 (dt, J = 8.3, 3.4 Hz, 2H). |
| **143** | 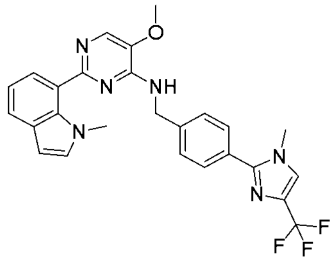 5-Methoxy-2-(1-methyl-1H-indol-7-yl)-N-(4-(1-methyl-4-(trifluoromethyl)-1H-imidazol-2-yl)benzyl)pyrimidin-4-amine | **AA3 and BB2C5** | LC-MS: m/z =493.1 (M+H)+ |
| **144** | 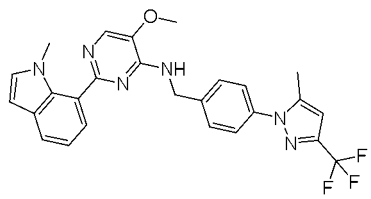 5-Methoxy-2-(1-methyl-1H-indol-7-yl)-N-(4-(5-methyl-3-(trifluoromethyl)-1H-pyrazol-1-yl)benzyl)pyrimidin-4-amine | **AA3 and BB2C11** | LC-MS: m/z =493.1 (M+H)+ |
| **145** | yl)pyrimidin-4-amine 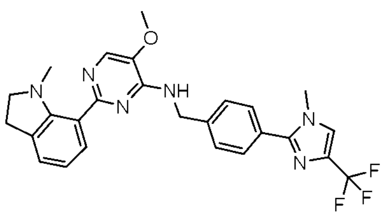 5-Methoxy-N-(4-(1-methyl-4-(trifluoromethyl)-1H-imidazol-2-yl)benzyl)-2-(1-methylindolin-7-yl)pyrimidin-4-amine | **AA4 and BB2C5** | LC-MS: m/z =495.2 (M+H)+ |

(continued)

| Example | Structure and name of compound | Reaction moiety | Analyzed data |
|---|---|---|---|
| 146 | yl)pyrimidin-4-amine 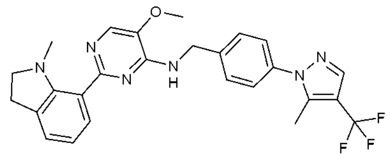 5-Methoxy-N-(4-(5-methyl-4-(trifluoromethyl)-1H-pyrazol-1-yl)benzyl)-2-(1-methylindolin-7-yl)pyrimidin-4-amine | **AA4 and BB2C11** | LC-MS: m/z =495.0 (M+H)+ |
| 147 | 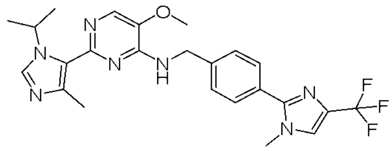 2-(1-Isopropyl-4-methyl-1H-imidazol-5-yl)-5-methoxy-N-(4-(1-methyl-4-(trifluoromethyl)-1H-imidazol-2-yl)benzyl)pyrimidin-4-amine | **AA5 and BB2C5** | LC-MS: m/z =486.3 (M+H)+ |
| 148 | 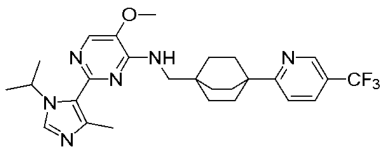 2-(1-Isopropyl-4-methyl-1H-imidazol-5-yl)-5-methoxy-N-((4-(5-(trifluoromethyl)pyridin-2-yl)bicyclo[2.2.2]octan-1-yl)methyl)pyrimidin-4-amine | **AA5 and BB2C19** | LC-MS: m/z =515.5 (M+H)+ |
| 149 | 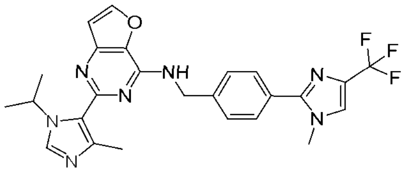 2-(1-Isopropyl-4-methyl-1H-imidazol-5-yl)-N-(4-(1-methyl-4-(trifluoromethyl)- 1H-imidazol-2-yl)benzyl)furo[3,2-d]pyrimidin-4-amine | **AA5 and BB13C5** | LC-MS: m/z =496.2 (M+H)+ |
| 150 | 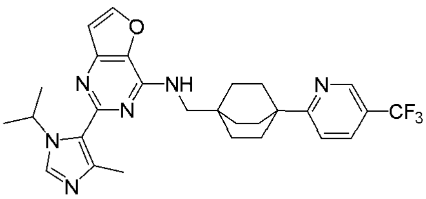 2-(1-Isopropyl-4-methyl-1H-imidazol-5-yl)-N-((4-(5-(trifluoromethyl)pyridin-2-yl)bicyclo[2.2.2]octan-1-yl)methyl)furo[3,2-d]pyrimidin-4-amine | **AA5 and BB13C19** | LC-MS: m/z =525.4 (M+H)+ |

(continued)

| Example | Structure and name of compound | Reaction moiety | Analyzed data |
|---|---|---|---|
| **151** | 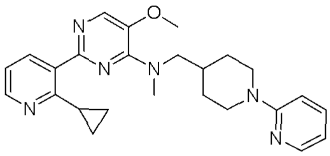 2-(2-Cyclopropylpyridin-3-yl)-5-methoxy-N-methyl-N-((1-(pyridin-2-yl)piperidin-4-yl)methyl)pyrimidin-4-amine | **AA6 and BB1C6** | LC-MS: m/z =431.2 (M+H)+ |
| **152** | 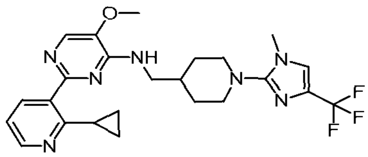 2-(2-Cyclopropylpyridin-3-yl)-5-methoxy-N-((1-(1-methyl-4-(trifluoromethyl)-1H-imidazol-2-yl)piperidin-4-yl)methyl)pyrimidin-4-amine | **AA6 and BB2C4** | LC-MS: m/z =488.1 (M+H)+ |
| **153** | 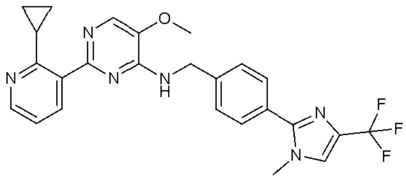 2-(2-Cyclopropylpyridin-3-yl)-5-methoxy-N-(4-(1-methyl-4-(trifluoromethyl)- 1H-imidazol-2-yl)benzyl)pyrimidin-4-amine | **AA6 and BB2C5** | LC-MS: m/z =481.3 (M+H)+ |
| **154** | 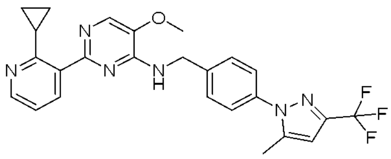 2-(2-Cyclopropylpyridin-3-yl)-5-methoxy-N-(4-(5-methyl-3-(trifluoromethyl)-1H-pyrazol-1-yl)benzyl)pyrimidin-4-amine | **AA6 and BB2C11** | LC-MS: m/z =481.4 (M+H)+ |
| **155** | 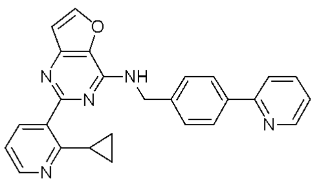 2-(2-Cyclopropylpyridin-3-yl)-N-(4-(pyridin-2-yl)benzyl) furo[3,2-d]pyrimidin-4-amine | **AA6 and BB13C3** | LC-MS: m/z =420.1 (M+H)+ |
| **156** | 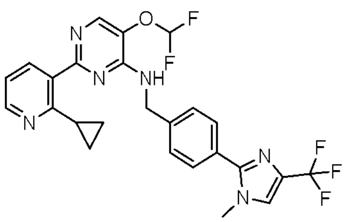 2-(2-Cyclopropylpyridin-3-yl)-5-(difluoromethoxy)-N-(4-(1-methyl-4-(trifluoromethyl)-1H-imidazol-2-yl) benzyl)pyrimidin-4-amine | **AA6 and BB19C5** | LC-MS: m/z =517.3 (M+H)+ |

(continued)

| Example | Structure and name of compound | Reaction moiety | Analyzed data |
|---|---|---|---|
| 157 | 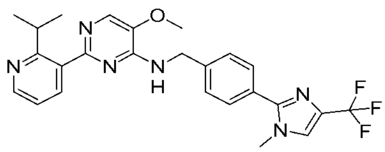 2-(2-Isopropylpyridin-3-yl)-5-methoxy-N-(4-(1-methyl-4-(trifluoromethyl)-1H-imidazol-2-yl)benzyl)pyrimidin-4-amine | AA7 and BB2C5 | LC-MS: m/z =483.2 (M+H)+ |
| 158 | 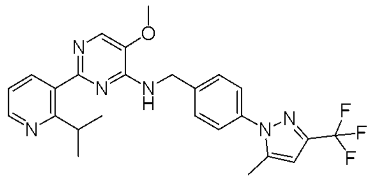 2-(2-Isopropylpyridin-3-yl)-5-methoxy-N-(4-(5-methyl-3-(trifluoromethyl)-1H-pyrazol-1-yl)benzyl)pyrimidin-4-amine | AA7 and BB2C11 | LC-MS: m/z =483.0 (M+H)+ |
| 159 | 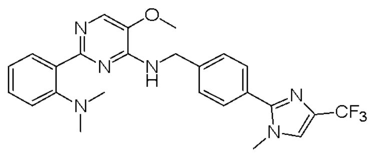 2-(2-(Dimethylamino)phenyl)-5-methoxy-N-(4-(1-methyl-4-(trifluoromethyl)- 1H-imidazol-2-yl)benzyl)pyrimidin-4-amine | AA8 and BB2C5 | LC-MS: m/z =483.4 (M+H)+ |
| 160 | 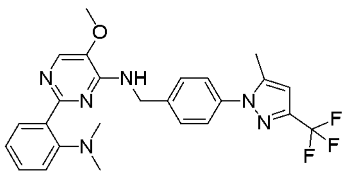 2-(2-(Dimethylamino)phenyl)-5-methoxy-N-(4-(5-methyl-3-(trifluoromethyl)-1H-pyrazol-1-yl)benzyl)pyrimidin-4-amine | AA8 and BB2C11 | LC-MS: m/z =483.1 (M+H)+ |
| 161 | 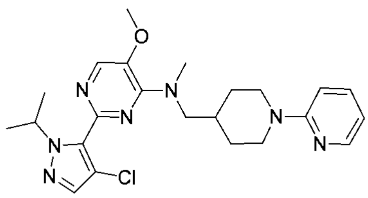 2-(4-Chloro-1-isopropyl-1H-pyrazol-5-yl)-5-methoxy-N-methyl-N-((1-(pyridin-2-yl)piperidin-4-yl)methyl)pyrimidin-4-amine | AA9 and BB1C6 | LC-MS: m/z =456.2 (M+H)+ |
| 162 | 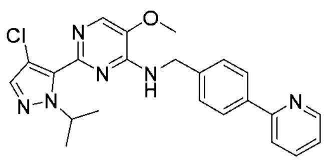 2-(4-Chloro-1-isopropyl-1H-pyrazol-5-yl)-5-methoxy-N-(4-(pyridin-2-yl)benzyl)pyrimidin-4-amine | AA9 and BB2C3 | LC-MS: m/z =435.1 (M+H)+ |

(continued)

| Example | Structure and name of compound | Reaction moiety | Analyzed data |
|---|---|---|---|
| **163** | 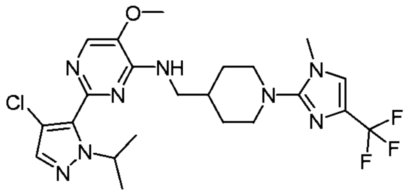 2-(4-Chloro-1-isopropyl-1H-pyrazol-5-yl)-5-methoxy-N-((1-(1-methyl-4-(trifluoromethyl)-1H-imidazol-2-yl)piperidin-4-yl)methyl)pyrimidin-4-amine | **AA9 and BB2C4** | LC-MS: m/z =513.3 (M+H)+ |
| **164** | 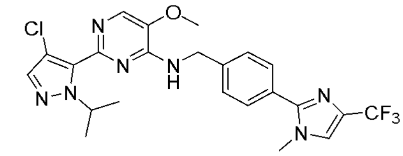 2-(4-Chloro-1-isopropyl-1H-pyrazol-5-yl)-5-methoxy-N-(4-(1-methyl-4-(trifluoromethyl)-1H-imidazol-2-yl) benzyl)pyrimidin-4-amine | **AA9 and BB2C5** | LC-MS: m/z =506.3 (M+H)+ |
| **165** | 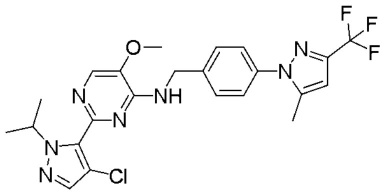 2-(4-Chloro-1-isopropyl-1H-pyrazol-5-yl)-5-methoxy-N-(4-(5-methyl-3-(trifluoromethyl)-1H-pyrazol-1-yl)benzyl)pyrimidin-4-amine | **AA9 and BB2C11** | LC-MS: m/z =506.1 (M+H)+ |
| **166** | 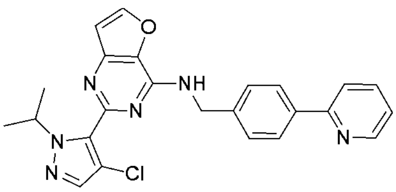 2-(4-Chloro-1-isopropyl-1H-pyrazol-5-yl)-N-(4-(pyridin-2-yl)benzyl)furo[3,2-d]pyrimidin-4-amine | **AA9 and BB13C3** | LC-MS: m/z =445.1 (M+H)+ |
| **167** | 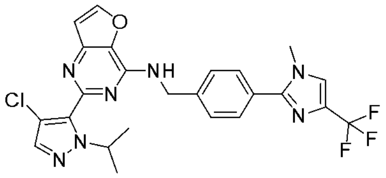 2-(4-Chloro-1-isopropyl-1H-pyrazol-5-yl)-N-(4-(1-methyl-4-(trifluoromethyl)-1H-imidazol-2-yl)benzyl)furo[3,2-d]pyrimidin-4-amine | **AA9 and BB13C5** | LC-MS: m/z =516.1 (M+H)+; $^1$H NMR (400 MHz, DMSO) δ 8.81 (s, 1H), 8.36 (d, *J* = 2.0 Hz, 1H), 7.89-7.93 (m, 1H), 7.64-7.70 (m, 2H), 7.59 (s, 1H), 7.44-7.50 (m, 2H), 7.11 (d, *J* = 2.4 Hz, 1H), 5.01-5.12 (m, 1H), 4.80-4.86 (m, 2H), 3.75 (s, 3H), 1.23 (d, *J* = 6.8 Hz, 6H). |

(continued)

| Example | Structure and name of compound | Reaction moiety | Analyzed data |
|---|---|---|---|
| **168** | 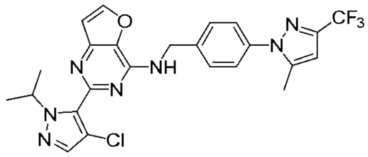 2-(4-Chloro-1-isopropyl-1H-pyrazol-5-yl)-N-(4-(5-methyl-3-(trifluoromethyl)-1H-pyrazol-1-yl)benzyl)furo [3,2-d]pyrimidin-4-amine | **AA9 and BB13C11** | LC-MS: m/z =516.2 (M+H)+ |
| **169** | 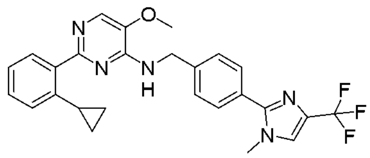 2-(2-Cyclopropylphenyl)-5-methoxy-N-(4-(1-methyl-4-(trifluoromethyl)-1H-imidazol-2-yl)benzyl)pyrimidin-4-amine | **AA10 and BB2C5** | LC-MS: m/z =480.4 (M+H)+ |
| **170** | 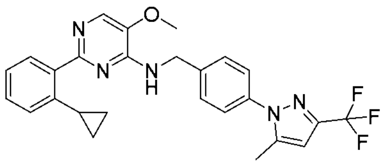 2-(2-Cyclopropylphenyl)-5-methoxy-N-(4-(5-methyl-3-(trifluoromethyl)-1H-pyrazol-1-yl)benzyl)pyrimidin-4-amine | **AA10 and BB2C11** | LC-MS: m/z =480.2 (M+H)+; $^1$H NMR (400 MHz, $CDCl_3$) δ 7.88 (s, 1H), 7.60 - 7.62 (m, 1H), 7.47 - 7.49 (m, 2H), 7.40 - 7.42 (m, 2H), 7.21 - 7.23 (m, 1H), 6.97 - 6.99 (m, 1H), 6.49 (s, 1H), 5.66 - 5.75 (m, 1H), 4.84 (d, *J* = 6.0Hz, 2H), 3.99 (s, 3H), 2.46 - 2.53 (m, 1H), 2.35 (s, 3H), 0.70 - 0.73 (m, 2H), 0.59 - 0.61 (m, 2H). |
| **171** | 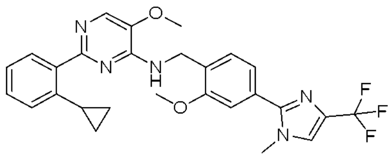 2-(2-Cyclopropylphenyl)-5-methoxy-N-(2-methoxy-4-(1-methyl-4-(trifluoromethyl)- 1H-imidazol-2-yl)benzyl)pyrimidin-4-amine | **AA10 and BB2C30** | LC-MS: m/z =510.2 (M+H)+ |
| **172** | 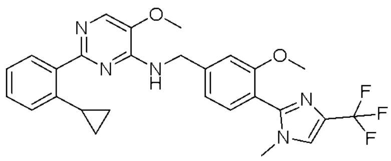 2-(2-Cyclopropylphenyl)-5-methoxy-N-(3-methoxy-4-(1-methyl-4-(trifluoromethyl)-1H-imidazol-2-yl)benzyl)pyrimidin-4-amine | **AA10 and BB2C31** | LC-MS: m/z =510.3 (M+H)+ |

(continued)

| Example | Structure and name of compound | Reaction moiety | Analyzed data |
|---|---|---|---|
| **173** | 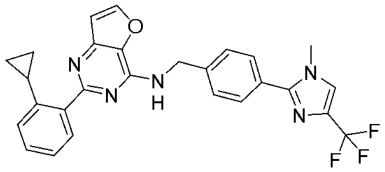 2-(2-Cyclopropylphenyl)-N-(4-(1-methyl-4-(trifluoromethyl)- 1H-imidazol-2-yl)benzyl)furo[3,2-d]pyrimidin-4-amine | **AA10 and BB13C5** | LC-MS: m/z =490.4 (M+H)+ |
| **174** | 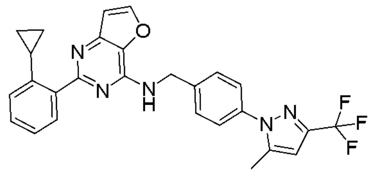 2-(2-Cyclopropylphenyl)-N-(4-(5-methyl-3-(trifluoromethyl)- 1H-pyrazol-1-yl)benzyl)furo[3,2-d]pyrimidin-4-amine | **AA10 and BB13C11** | LC-MS: m/z =490.2 (M+H)+ |
| **175** | 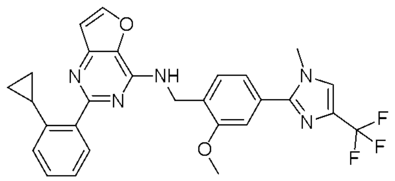 2-(2-Cyclopropylphenyl)-N-(2-methoxy-4-(1-methyl-4-(trifluoromethyl)-1H-imidazol-2-yl)benzyl)furo [3,2-d]pyrimidin-4-amine | **AA10 and BB13C30** | LC-MS: m/z =520.0 (M+H)+ |
| **176** | 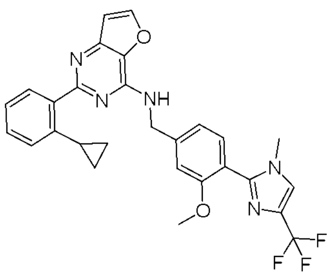 2-(2-Cyclopropylphenyl)-N-(3-methoxy-4-(1-methyl-4-(trifluoromethyl)-1H-imidazol-2-yl)benzyl)furo [3,2-d]pyrimidin-4-amine | **AA10 and BB13C31** | LC-MS: m/z =520.2 (M+H)+ |
| **177** | 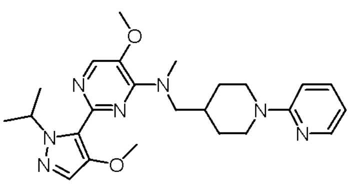 2-(1-Isopropyl-4-methoxy-1H-pyrazol-5-yl)-5-methoxy-N-methyl-N-((1-(pyridin-2-yl)piperidin-4-yl)methyl)pyrimidin-4-amine | **AA11 and BB1C6** | LC-MS: m/z =452.2 (M+H)+ |

(continued)

| Example | Structure and name of compound | Reaction moiety | Analyzed data |
|---|---|---|---|
| 178 | 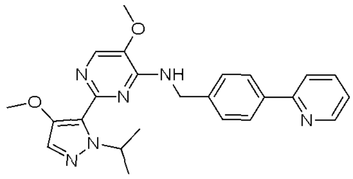 2-(1-Isopropyl-4-methoxy-1H-pyrazol-5-yl)-5-methoxy-N-(4-(pyridin-2-yl)benzyl)pyrimidin-4-amine | **AA11 and BB2C3** | LC-MS: m/z =431.4 (M+H)+ |
| 179 | 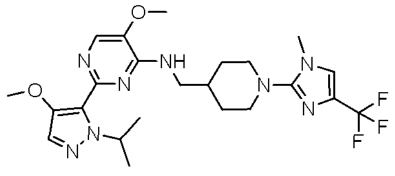 2-(1-Isopropyl-4-methoxy-1H-pyrazol-5-yl)-5-methoxy-N-((1-(1-methyl-4-(trifluoromcthyl)-1H-imidazol-2-yl)piperidin-4-yl)methyl)pyrimidin-4-amine | **AA11 and BB2C4** | LC-MS: m/z =509.1 (M+H)+ |
| 180 | 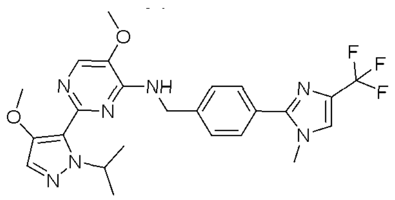 2-(1-Isopropyl-4-methoxy- 1H-pyrazol-5-yl)-5-methoxy-N-(4-(1-methyl-4-(trifluoromethyl)-1H-imidazol-2-yl) benzyl)pyrimidin-4-amine | **AA11 and BB2C5** | LC-MS: m/z =502.1 (M+H)+ |
| 181 | 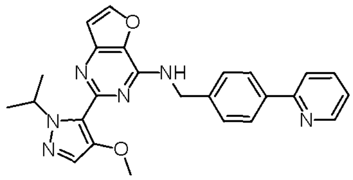 2-(1-Isopropyl-4-methoxy-1H-pyrazol-5-yl)-N-(4-(pyridin-2-yl)benzyl)furo[3,2-d]pyrimidin-4-amine | **AA11 and BB13C3** | LC-MS: m/z =441.4 (M+H)+ |
| 182 | 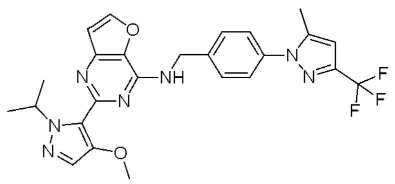 2-(1-Isopropyl-4-methoxy-1H-pyrazol-5-yl)-N-(4-(5-methyl-3-(trifluoromethyl)-1H-pyrazol-1-yl)benzyl)furo [3,2-d]pyrimidin-4-amine | **AA11 and BB13C11** | LC-MS: m/z =512.3 (M+H)+ |
| 183 | 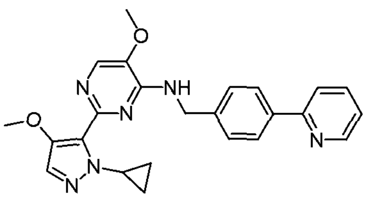 2-(1-Cyclopropyl-4-methoxy-1H-pyrazol-5-yl)-5-methoxy-N-(4-(pyridin-2-yl)benzyl)pyrimidin-4-amine | **AA12 and BB2C3** | LC-MS: m/z =429.2 (M+H)+ |

(continued)

| Example | Structure and name of compound | Reaction moiety | Analyzed data |
|---|---|---|---|
| **184** | 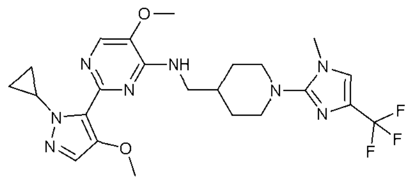 2-(1-Cyclopropyl-4-methoxy-1H-pyrazol-5-yl)-5-methoxy-N-((1-(1-methyl-4-(trifluoromethyl)-1H-imidazol-2-yl)piperidin-4-yl)methyl)pyrimidin-4-amine | **AA12 and BB2C4** | LC-MS: m/z =507.3 (M+H)+ |
| **185** | 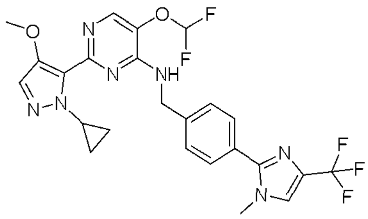 2-(1-Cyclopropyl-4-methoxy-1H-pyrazol-5-yl)-5-(difluoromethoxy)-N-(4-(1-methyl-4-(trifluoromethyl)-1H-imidazol-2-yl)benzyl)pyrimidin-4-amine | **AA12 and BB19C5** | LC-MS: m/z =536.3 (M+H)+ |
| **186** | 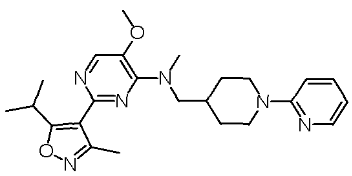 2-(5-Isopropyl-3-methylisoxazol-4-yl)-5-methoxy-N-methyl-N-((1-(pyridin-2-yl)piperidin-4-yl)methyl)pyrimidin-4-amine | **AA13 and BB1C6** | LC-MS: m/z =437.2 (M+H)+ |
| **187** | 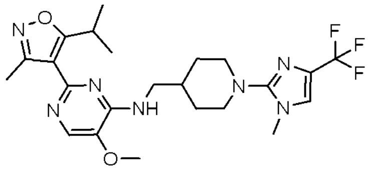 2-(5-Isopropyl-3-methylisoxazol-4-yl)-5-methoxy-N-((1-(1-methyl-4-(trifluoromethyl)-1H-imidazol-2-yl)piperidin-4-yl)methyl)pyrimidin-4-amine | **AA13 and BB2C4** | LC-MS: m/z =494.1 (M+H)+; $^1$H NMR (400 MHz, CDCl3) δ 7.79 (s, 1H), 7.01 (s, 1H), 5.41 - 5.38 (m, 1H), 4.08 - 4.01 (m, 1H), 3.92 (s, 3H), 3.51 (s, 3H), 3.50 - 3.47 (m, 2H), 3.31 - 3.28 (m, 2H), 2.95 - 2.88 (m, 2H), 2.56 (s, 3H), 1.89 (s, 1H), 1.85 - 1.80 (m, 2H), 1.50 - 1.40 (m, 2H), 1.37 (s, 3H), 1.36 (s, 3H). |
| **188** | 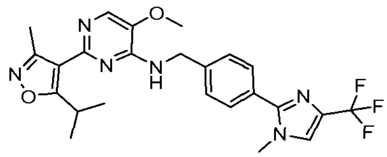 2-(5-Isopropyl-3-methylisoxazol-4-yl)-5-methoxy-N-(4-(1-methyl-4-(trifluoromethyl)-1H-imidazol-2-vl) benzyl)pyrimidin-4-amine | **AA13 and BB2C5** | LC-MS: m/z =487.0 (M+H)+ |

(continued)

| Example | Structure and name of compound | Reaction moiety | Analyzed data |
|---|---|---|---|
| **189** | 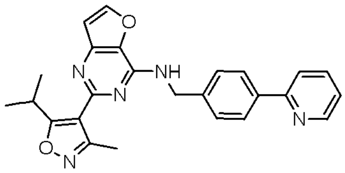 2-(5-Isopropyl-3-methylisoxazol-4-yl)-N-(4-(pyridin-2-yl) benzyl)furo[3,2-d]pyrimidin-4-amine | **AA13 and BB13C3** | LC-MS: m/z =426.1 (M+H)+ |
| **190** | 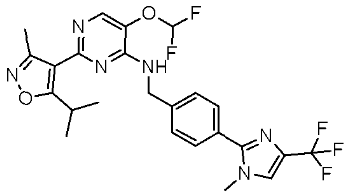 5-(Difluoromethoxy)-2-(5-isopropyl-3-methylisoxazol-4-yl)-N-(4-(1-methyl-4-(trifluoromethyl)-1H-imidazol-2-yl) benzyl)pyrimidin-4-amine | **AA13 and BB19C5** | LC-MS: m/z =523.5 (M+H)+ |
| **191** | 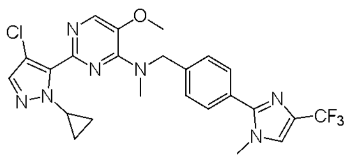 2-(4-Chloro-1-cyclopropyl-1H-pyrazol-5-yl)-5-methoxy-N-methyl-N-(4-(1-methyl-4-(trifluoromethyl)-1H-imidazol-2-yl)benzyl)pyrimidin-4-amine | **AA14 and BBIC5** | LC-MS: m/z =518.3 (M+H)+ |
| **192** | 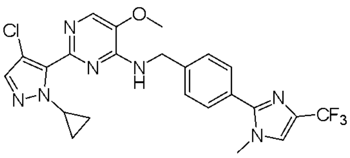 2-(4-Chloro-1-cyclopropyl-1H-pyrazol-5-yl)-5-methoxy-N-(4-(1-methyl-4-(trifluoromethyl)-1H-imidazol-2-yl) benzyl)pyrimidin-4-amine | **AA14 and BB2C5** | LC-MS: m/z =504.2 (M+H)+; $^1$H NMR (400 MHz, DMSO-$d_6$) δ 8.06 (d, *J* = 4.9 Hz, 2H), 8.00 - 7.86 (m, 1H), 7.76 - 7.59 (m, 2H), 7.50 (s, 1H), 7.44 (d, *J*= 8.0 Hz, 2H), 4.71 (d, *J* = 6.2 Hz, 2H), 4.00 (s, 3H), 3.95 (tt, *J* = 7.5, 3.8 Hz, 1H), 3.77 (s, 3H), 0.97 - 0.77 (m, 2H), 0.71 - 0.50 (m, 2H). |
| **193** | 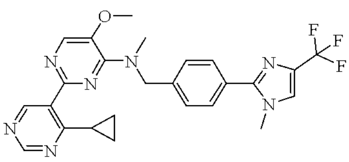 4'-Cyclopropyl-5-methoxy-N-methyl-N-(4-(1-methyl-4-(trifluoromethyl)-1H-imidazol-2-yl)benzyl)-[2,5'-bipyrimidin]-4-amine | **AA15 and BB1C5** | LC-MS: m/z =496.5 (M+H)+ |

(continued)

| Example | Structure and name of compound | Reaction moiety | Analyzed data |
|---|---|---|---|
| **194** | 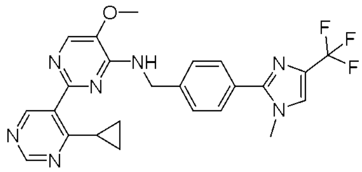 4'-Cyclopropyl-5-methoxy-N-(4-(1-methyl-4-(trifluoromethyl)-1H-imidazol-2-yl)benzyl)-[2,5'-bipyrimidin]-4-amine | **AA15 and BB2C5** | LC-MS: m/z =482.1 (M+H)+; $^1$H NMR (400 MHz, DMSO-$d_6$) δ 8.93 (s, 1H), 8.88 (s, 1H), 8.08 (s, 1H), 8.01 (d, *J*= 6.1 Hz, 1H), 7.93 (s, 1H), 7.68 (d, *J*= 8.0 Hz, 2H), 7.45 (d, *J*= 8.0 Hz, 2H), 4.73 (d, *J*= 6.3 Hz, 2H), 4.00 (s, 3H), 3.79 (s, 3H), 3.06 - 2.99 (m, 1H), 1.05 (s, 2H), 0.86 - 0.80 (m, 2H). |
| **195** | 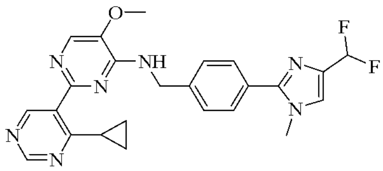 4'-Cyclopropyl-N-(4-(4-(difluoromethyl)-1-methyl-1H-imidazol-2-yl)benzyl)-5-methoxy-[2,5'-bipyrimidin]-4-amine | **AA15 and BB2C48** | LC-MS: m/z =464.4 (M+H)+; $^1$H NMR (400 MHz, DMSO-$d_6$) δ 8.92 (s, 1H), 8.86 (s, 1H), 8.07 (s, 1H), 7.99 (t, *J* = 6.3 Hz, 1H), 7.69 - 7.59 (m, 3H), 7.42 (d, *J*= 8.1 Hz, 2H), 6.78 - 7.05 (m, 1H), 4.71 (d, *J*= 6.2 Hz, 2H), 3.98 (s, 3H), 3.75 (s, 3H), 3.00 (dt, *J* = 8.1, 3.9 Hz, 1H), 1.02 (d, *J* = 4.4 Hz, 2H), 0.79 (dt, *J* = 7.0, 3.4 Hz, 2H). |
| **196** | 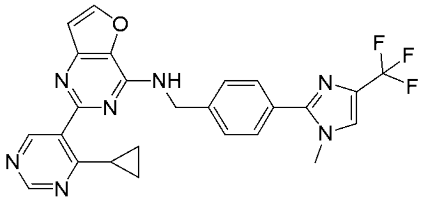 2-(4-Cyclopropylpyrimidin-5-yl)-N-(4-(1-methyl-4-(trifluoromethyl)-1H-imidazol-2-yl)benzyl)furo[3,2-d]pyrimidin-4-amine | **AA15 and BB13C5** | LC-MS: m/z =492.0 (M+H)+; $^1$H NMR (400 MHz, DMSO-$d_6$) δ 8.94 (m, 2H), 8.81 (s, 1H), 8.38 (d, *J* = 2.2 Hz, 1H), 7.93 (d, *J* = 1.4 Hz, 1H), 7.70 (d, *J* = 8.1 Hz, 2H), 7.51 (d, *J* = 8.1 Hz, 2H), 7.14 (d, *J* = 2.2 Hz, 1H), 4.86 (d, *J* = 6.2 Hz, 2H), 3.79 (s, 2H), 3.07 - 2.97 (m, 1H), 1.32 - 1.25 (m, 1H), 1.11 - 1.04 (m, 2H), 0.86 (s, 2H). |

(continued)

| Example | Structure and name of compound | Reaction moiety | Analyzed data |
|---|---|---|---|
| 197 | 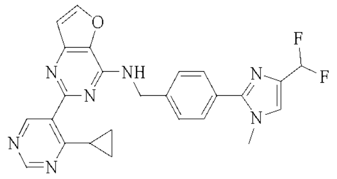 2-(4-Cyclopropylpyrimidin-5-yl)-N-(4-(4-(difluoromethyl)-1-methyl-1H-imidazol-2-yl)benzyl)furo[3,2-d] pyrimidin-4-amine | AA15 and BB13C48 | LC-MS: m/z =474.1 (M+H)+; $^1$H NMR (400 MHz, DMSO-$d_6$) δ 8.96 (s, 1H), 8.90 (s, 1H), 8.80 (s, 1H), 8.37 (d, *J* = 2.2 Hz, 1H), 7.65 (d, *J* = 8.2 Hz, 2H), 7.50 (d, *J*= 8.0 Hz, 2H), 7.39 - 7.35 (m, 1H), 7.28 - 7.12 (m, 1H), 6.75 - 7.03 (m, 1H), 4.85 (d, *J* = 6.2 Hz, 2H), 3.74 (s, 3H), 2.96 (dq, *J*= 8.6, 5.1, 4.5 Hz, 1H), 1.05 (m, 2H), 0.83 (m, 2H). |
| 198 | 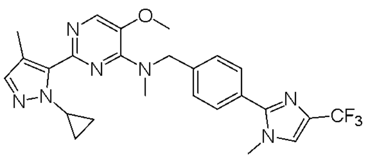 2-(1-Cyclopropyl-4-methyl-1H-pyrazol-5-yl)-5-methoxy-N-methyl-N-(4-(1-methyl-4-(trifluoromethyl)-1H-imidazol-2-yl)benzyl)pyrimidin-4-amine | AA16 and BBIC5 | LC-MS: m/z =498.2 (M+H)+; $^1$H NMR (400 MHz, DMSO-$d_6$) δ 8.21 (s, 1H), 7.95 (d, *J* = 1.5 Hz, 1H), 7.72 (d, *J*= 8.2 Hz, 2H), 7.41 (d, *J* = 8.1 Hz, 2H), 7.23 (s, 1H), 5.02 (s, 2H), 4.31 (dt, *J* = 7.4, 3.6 Hz, 1H), 3.91 (s, 3H), 3.80 (s, 3H), 3.24 (s, 3H), 2.15 (s, 3H), 1.06 - 0.87 (m, 2H), 0.87 - 0.62 (m, 2H). |
| 199 | 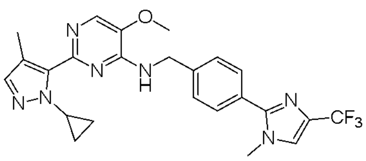 2-(1-Cyclopropyl-4-methyl-1H-pyrazol-5-yl)-5-methoxy-N-(4-(1-methyl-4-(trifluoromethyl)- 1H-imidazol-2-yl) benzyl)pyrimidin-4-amine | AA16 and BB2C5 | LC-MS: m/z =484.4 (M+H)+ |
| 200 | 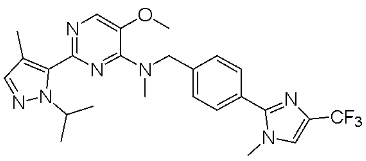 2-(1-Isopropyl-4-methyl-1H-pyrazol-5-yl)-5-methoxy-N-methyl-N-(4-(1-methyl-4-(trifluoromethyl)-1H-imidazol-2-yl)benzyl)pyrimidin-4-amine | AA17 and BB1C5 | LC-MS: m/z =500.5 (M+H)+ |

(continued)

| Example | Structure and name of compound | Reaction moiety | Analyzed data |
|---|---|---|---|
| **201** | 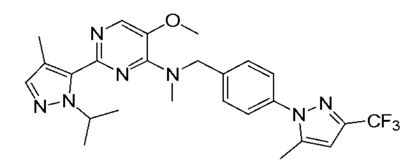 2-(1-Isopropyl-4-methyl-1H-pyrazol-5-yl)-5-methoxy-N-methyl-N-(4-(5-methyl-3-(trifluoromethyl)-1H-pyrazol-1-yl)benzyl)pyrimidin-4-amine | **AA17 and BB1C11** | LC-MS: m/z =500.1 (M+H)+ |
| **202** | 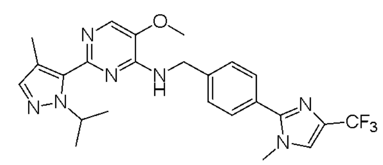 2-(1-Isopropyl-4-methyl-1H-pyrazol-5-yl)-5-methoxy-N-(4-(1-methyl-4-(trifluoromethyl)- 1H-imidazol-2-yl) benzyl)pyrimidin-4-amine | **AA17 and BB2C5** | LC-MS: m/z =486.3 (M+H)+ |
| **203** | 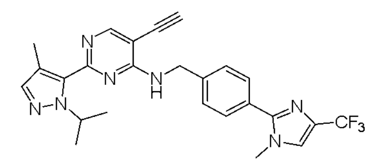 5-Ethynyl-2-(1-isopropyl-4-methyl-1H-pyrazol-5-yl)-N-(4-(1-methyl-4-(trifluoromethyl)-1H-imidazol-2-vl) benzyl)pyrimidin-4-amine | **AA17 and BB18D5** | LC-MS: m/z =480.2 (M+H)+ |

**Example 204: Synthesis of compound 204 (N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)hydroxylamine)**

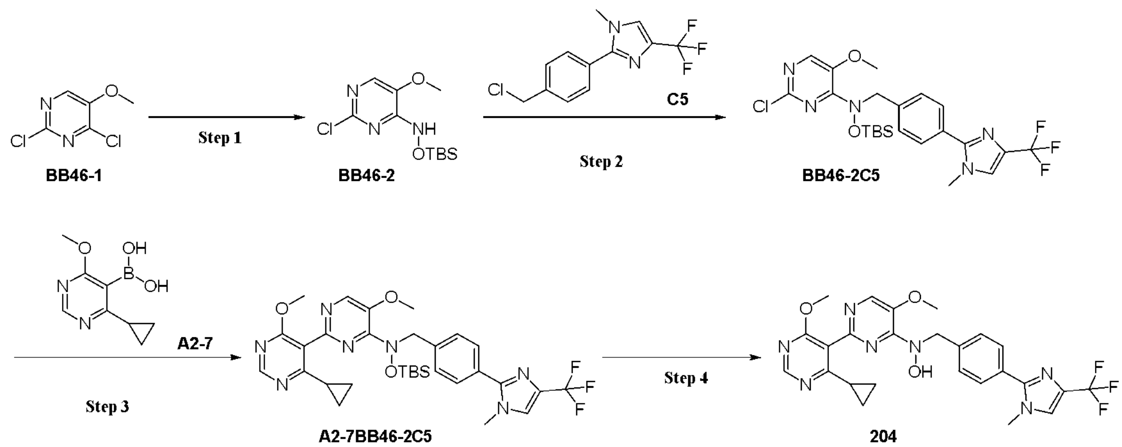

BB46-1
BB46-2
BB46-2C5
A2-7BB46-2C5
204

Step 1: Synthesis of compound **BB46-2** (O-(tert-butyldimethylsilyl)-N-(2-chloro-5-methoxypyrimidin-4-yl)hydroxylamine)

Compound 2,4-dichloro-5-fluoropyrimidine (compound **BB46-1,** 1.0 g, 5.6 mmol, 1.0 eq), O-(tert-butyldimethylsilyl)hydroxylamine (0.9 g, 6.2 mmol, 1.1 eq) and N,N-diisopropylethylamine (2.1 g, 16.8 mmol, 3.0 eq) were added to dioxane (20 mL) successively, and the resulting mixture was stirred at 80°C for 4 hours. The reaction solution was cooled to

room temperature, and filtered to obtain a filtrate. The filtrate was concentrated under vacuum to obtain a crude product, which was purified by column chromatography (eluent: petroleum ether/ethyl acetate = 1/0 - 1/1) to obtain compound **BB46-2** (430.2 mg, 1.49 mmol, 26.6% yield) as a light yellow oil. LC-MS: m/z = 290.0 $(M+H)^+$.

Step 2: Synthesis of compound **BB46-2C5** (O-(tert-butyldimethylsilyl)-N-(2-chloro-5-methoxypyrimidin-4-yl)-N-(4-(1-methyl-4-(trifluoromethyl)-1H-imidazol-2-yl)benzyl)hydroxylamine)

In accordance with the same steps of intermediate **BB1C2**, intermediate **BB46-2C5** (506.8 mg, 0.96 mmol, 64.1% yield, a light yellow solid) was obtained using intermediate **BB46-2** (430 mg,1.5 mmol,1.0 *eq)* and compound **C5** (452 mg, 1.65 mmol, 1.1 *eq)* as starting materials. LC-MS: m/z = 528.3 $(M+H)^+$.

Step 3: Synthesis of compound **A2-7BB46-2C5** (O-(tert-butyldimethylsilyl)-N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)hydroxylamine)

In accordance with the same steps of compound **A1-2BB1C2,** intermediate **A2-7BB46-2C5** (323.3 mg, 0.50 mmol, 56.0% yield, a yellow solid) was obtained using intermediate **BB46-2** (486.0 mg, 0.9 mmol,1.0 *eq)* and compound **A2-7** (262.1 mg, 1.35 mmo, 1.5 *eq)* as starting materials. LC-MS: m/z = 642.3 $(M+H)^+$.

Step 4: Synthesis of compound **204**

Intermediate **A2-7BB46-2C5** (323.0 mg, 0.5 mmol, 1.0 *eq)* was dissolved in a 4 M solution of hydrochloric acid in methanol, and the mixture wa stirred at 25°C for 1 hour. Saturated aqueous sodium bicarbonate solution (30.0 mL) was added, and the resulting mixture was extracted three times with dichloromethane (20.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **204** (109.9 mg, 0.21 mmol, 41.7% yield) as a white solid. $^1H$ NMR (400MHz, DMSO-$d_6$) $\delta$ 9.51 (s, 1H), 8.62 (s, 1H), 8.28 (s, 1H), 7.92 (d, *J*= 1.4 Hz, 1H), 7.75 - 7.57 (m, 2H), 7.56 - 7.41 (m, 2H), 4.87 (s, 2H), 3.94 (s, 3H), 3.82 (s, 3H), 3.77 (s, 3H), 1.62 (dt, *J* = 8.1, 3.5 Hz, 1H), 0.99 (dq, *J* = 5.9, 3.5 Hz, 2H), 0.84 (dq, *J* = 10.0, 3.4 Hz, 2H); LC-MS: m/z = 528.3 $(M+H)^+$.

**Example 205: Synthesis of compound 205 (N-(2-(1-cyclopropyl-4-methyl-1H-pyrazol-5-yl)-5-methoxypyrimidin-4-yl)-N-(4-(1-methyl-4-(trifluoromethyl)-1H-imidazol-2-yl)benzyl)hydroxylamine)**

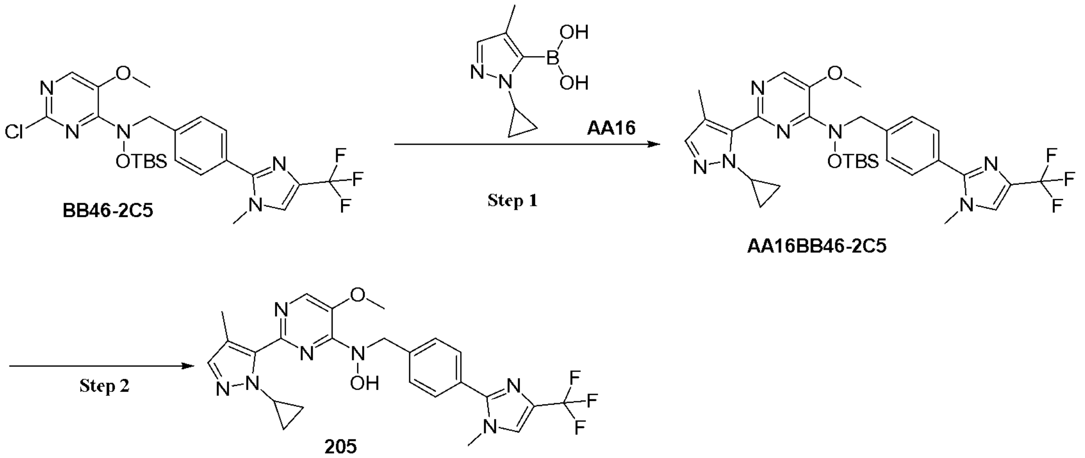


BB46-2C5

AA16BB46-2C5

205

In accordance with the same steps of compound **204,** compound **205** (37.2 mg, 0.074 mmol, 66.9% yield, a white solid) was obtained using intermediate **BB46-2** and compound **AA16** as starting materials. LC-MS: m/z = 500.3 $(M+H)^+$.

**Example 206: Synthesis of compound 206 (N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)cyanamide)**

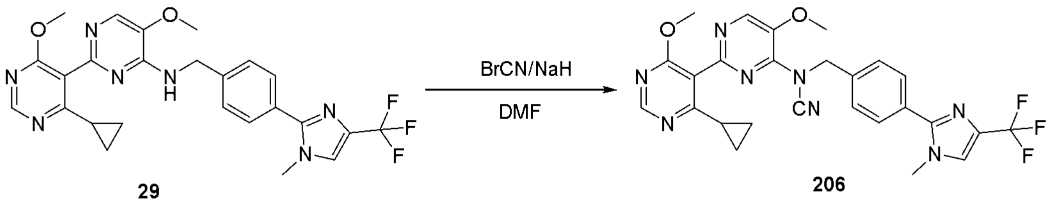


29 206

Compound **29** (50 mg, 0.092 mmol, 1.0 eq) was dissolved in N,N-dimethylformamide (1.0 mL), and sodium hydride (60% content, 36.8 mg, 0.92 mmol, 10 eq) was added in portions at 0°C. The reaction solution was naturally heated up to 25°C, and stirred for one hour. Cyanogen bromide (48.7 mg, 0.46 mmol, 5 eq) was added, and the resulting mixture was stirred at room temperature of 25°C for 2 hours. The reaction solution was concentrated under vacuum to obtain a crude product, which was purified by column chromatography (eluent: petroleum ether/ethyl acetate = 1/0 - 0/1) to obtain compound **206** (6.7 mg, 0.0125 mmol, 13.6% yield) as a pale white solid. $^1H$ NMR (400MHz, DMSO-$d_6$) δ $^1H$ NMR (400 MHz, DMSO-d6) δ 8.58 (s, 1H), 7.96 (s, 1H), 7.92 (s, 1H), 7.60 (d, *J* = 7.8 Hz, 2H), 7.40 (d, *J* = 7.8 Hz, 2H), 5.39 (d, *J* = 6.3 Hz, 2H), 3.93 (s, 3H), 3.81 (s, 3H), 3.70 (s, 3H), 1.68 (tt, J = 8.2, 4.8 Hz, 1H), 0.96 (s, 2H), 0.78-0.73 (m, 2H); LC-MS: m/z = 537.2 $(M+H)^+$.

**Example 207: Synthesis of compound 207 (2-(4-cyclopropyl-6-methoxy-pyrimidin-5-yl)-5-methoxy-N-methyl-N-[[4-[1-methyl-4-(trifluoromethyl)imidazol-2-yl]cuban-1-yl]methyl]pyrimidin-4-amine)**

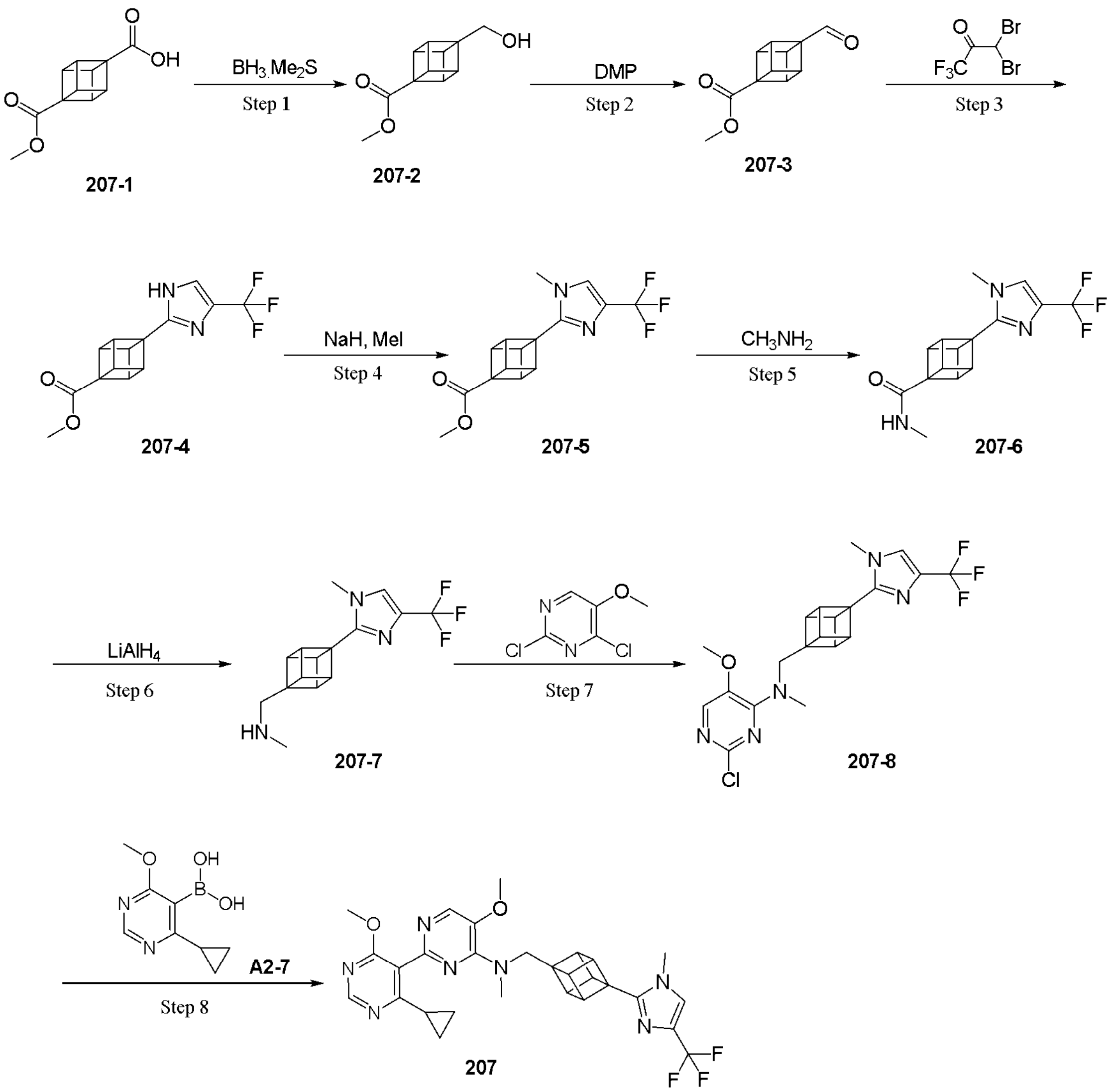


207-1 207-2 207-3 207-4 207-5 207-6 207-7 207-8 207

Step **1:** Synthesis of compound **207-2** (methyl (2R,3R,4S,5S)-4-(hydroxymethyl)cubane-1-carboxylate)

Compound (1S,2R,3R,8S)-4-(methoxycarbonyl)cubane-1-carboxylic acid **(207-1,** 10.0 g, 48.5 mmol, 1.00 eq) was dissolved in tetrahydrofuran (300 mL). A 10 M solution of borane dimethyl sulfide in tetrahydrofuran (5.82 mL, 1.20 eq) was added at 20°C, and the resulting mixture was stirred at 50°C for 13 hours. Methanol (300 mL) was slowly added at 0°C to quench the reaction, and the resulting mixture was filtered to obtain a filtrate, which was concentrated under vacuum to obtain compound **207-2** (11.0 g, crude) as a white solid. $^1$H NMR (400MHz, $CDCl_3$) δ 4.15 - 4.13 (m, 3H), 3.89 - 3.87 (m, 3H), 3.77 - 3.75 (m, 2H), 3.70 (s, 3H).

Step **2:** Synthesis of compound **207-3** (methyl (2R,3R,4S,5S)-4-formylcubane-1-carboxylate)

Compound **207-2** (11.0 g, 57.2 mmol, 1.00 eq) was dissolved in dichloromethane (150 mL), followed by the addition of Dess-Martin periodinane (DMP, 29.1 g, 68.6 mmol, 21.2 mL, 1.20 eq) at 0°C, and the resulting mixture was stirred at 0°C for 2 hours. Saturated aqueous sodium bicarbonate solution (100 mL) was added, and the resulting mixture was extracted three times with dichloromethane (150 mL). The organic layers were combined, washed twice with saturated brine (120 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **207-3** (5.8 g, 30.5 mmol, 53.3% yield) as a white solid. $^1$H NMR (400MHz, $CDCl_3$) δ 9.75 (s, 1H), 4.38 - 4.36 (m, 3H), 4.27 - 4.25 (m, 3H), 3.72 (s, 3H).

Step **3:** Synthesis of compound **207-4** (methyl (2R,3R,4S,5S)-4-(4-(trifluoromethyl)-1H-imidazol-2-yl)cubane-1-carboxylate)

Sodium acetate (5.25 g, 64.0 mmol, 2.1 eq) was dissolved in water (25.0 mL), and 1,1-dibromo-3,3,3-trifluoroacetone (9.05 g, 33.5 mmol, 1.1 eq) was added at 20°C. The reaction solution was heated up to 100°C, and stirred for 1 hour. Compound **207-3** (5.8 g, 30.5 mmol, 1.0 eq) dissolved in methanol (65.0 mL) and ammonia water (25.0 mL) was added at 20°C. After the addition was completed, the resulting mixture was stirred for 11 hours at 20°C. The reaction solution was filtered to obtain a filtrate, which was concentrated under vacuum to obtain compound **207-4** (6.0 g, 18.5 mmol, 60.8% yield) as a white solid. LC-MS: m/z = 297.0 $(M+H)^+$.

Step **4:** Synthesis of compound **207-5** (methyl (2R,3R,4S,5S)-4-(1-methyl-4-(trifluoromethyl)-1H-imidazol-2-yl)cubane-1-carboxylate)

Compound **207-4** (4.5 g, 15.2 mmol, 1.0 eq) was dissolved in tetrahydrofuran (40.0 mL), and sodium hydride (911 mg, 22.7 mmol, 60% content, 1.50 eq) was added in portions at 0°C. After the addition was completed, the resulting mixture was stirred at 20°C for 30 minutes. Iodomethane (2.16 g, 15.2 mmol, 945 μL, 1.0 eq) was added, and the resulting mixture was reacted at 20°C for 2 hours. Saturated aqueous ammonium chloride solution (50.0 mL) was added at 0°C to quench the reaction. Water (100 mL) was added, and the resulting mixture was extracted three times with ethyl acetate (70.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by reverse phase chromatography HPLC (0.1% FA) to obtain compound **207-5** (1.6 g, 4.95 mmol, 32.5% yield) as a white solid. $^1$H NMR (400MHz, $CD_3OD$) δ 7.54 (d, *J*= 1.2 Hz, 1H), 4.45 - 4.42 (m, 3H), 4.32 - 4.30 (m, 3H), 3.73 (s, 3H), 3.66 (s, 3H); LC-MS: m/z = 311.1 $(M+H)^+$.

Step **5:** Synthesis of compound **207-6** ((2R,3R,4S,5S)-N-methyl-4-(1-methyl-4-(trifluoromethyl)-1H-imidazol-2-yl)cubane-1-carboxamide)

Compound **207-5** (120 mg, 386.76 mmol, 1 eq) was dissolved in ethanol (24.0 mL), and a solution of methylamine in ethanol (16.51 g, 159.48 mmol, 30% content, 412.35 eq) was added at 25°C. The resulting mixture was stirred at 70°C under 50 Psi pressure for 12 hours. The reaction solution was cooled to room temperature, and ice water (15.0 mL) was added. The resulting mixture was concentrated under vacuum to remove ethanol, and extracted three times with ethyl acetate (30.0 mL). The organic layers were combined, dried over anhydrous sodium sulfate, and filtered to obtain a filtrate, which was concentrated under vacuum to obtain compound **207-6** (0.11 g, crude) as a white solid. LC-MS: m/z = 310.1 $(M+H)^+$.

Step **6:** Synthesis of compound **207-7** (N-methyl-1-((2R,3R,4S,5S)-4-(1-methyl-4-(trifluoromethyl)-1H-imidazol-2-yl) cuban-1-yl)methylamine)

Compound **207-6** (0.1 g, 323.33 mmol, 1 *eq)* was dissolved in tetrahydrofuran (10.0 mL), and a solution of lithium aluminum hydride in tetrahydrofuran (2.5 mmol, 387.99 μL, 3 *eq)* was added at 0°C. The resulting mixture was stirred at 0°C for 30 minutes, then heated up to 50°C and stirred for 3 hours. Sodium sulfate decahydrate (1.0 g) was added at 0°C, and the resulting mixture was stirred for 30 minutes, followed by the addition of tetrahydrofuran (5.0 mL), and stirred for 5 minutes. The resulting mixture was filtered to obtain a filtrate, which was concentrated under vacuum to obtain compound **207-7** (86 mg, crude) as a white solid. LC-MS: m/z = 296.3 $(M+H)^+$.

Step **7:** Synthesis of compound **207-8** (2-chloro-5-methoxy-N-methyl-N-[[4-[1-methyl-4-(trifluoromethyl)imidazol-2-yl] cuban-1-yl]methyl]pyrimidin-4-amine)

Compound **207-7** (40 mg, 135.45 mmol, 1 *eq),* N,N-diisopropylethylamine (70.03 mg, 541.82 mmol, 94.37 μL, 4 *eq)* and 2,4-dichloro-5-methoxypyrimidine (38.80 mg, 216.73 mmol, 1.6 *eq)* were added to dioxane (2.0 mL) successively, and the resulting mixture was stirred at an external temperature of 60°C for 8 hours. The reaction solution was cooled to room temperature. Water (10.0 mL) was added, and the resulting mixture was extracted three times with ethyl acetate (10.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **207-8** (32.0 mg, 73.09 mmol, 53.96% yield) as a white solid. LC-MS: m/z = 438.2 $(M+H)^+$.

Step **8:** Synthesis of compound **207** (4'-cyclopropyl-5,6'-dimethoxy-N-methyl-N-(4-(1-methyl-4-(trifluoromethyl)-1H-imidazol-2-yl)cuban-1-yl)methyl)-[2,5'-bipyrimidin]-4-amine)

In accordance with the same steps of compound **61,** compound **207** (2.66 mg, 4.32 mmol, 7.56% yield, a white solid) was obtained using compound **207-8** (25.0 mg, 57.10 mmol, 1 eq) and intermediate **A2-7** (22.15 mg, 114.20 mmol, 2.0 eq) as starting materials. LC-MS: m/z = 552.3 $(M+H)^+$.

**Example 208: Synthesis of compound 208 (2-((4'-cyclopropyl-5,6'-dimethoxy-[2,5'-bipyrimidin]-4-yl)amino)-2-(4-(1-methyl-4-(trifluoromethyl)-1H-imidazol-2-yl)phenyl)acetonitrile)**

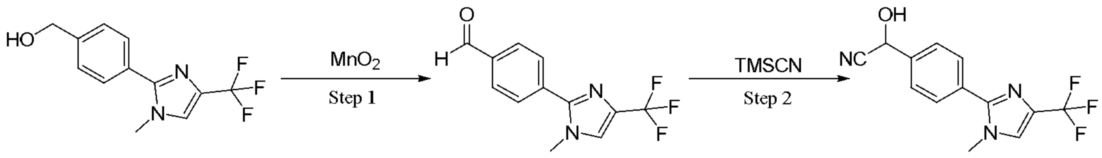


C5-4 208-1 208-2

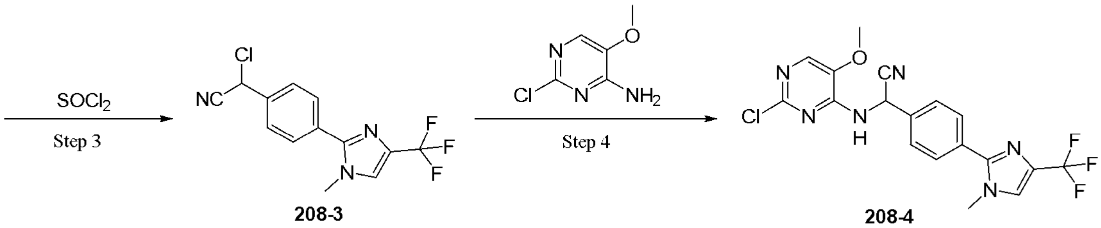


208-3 208-4

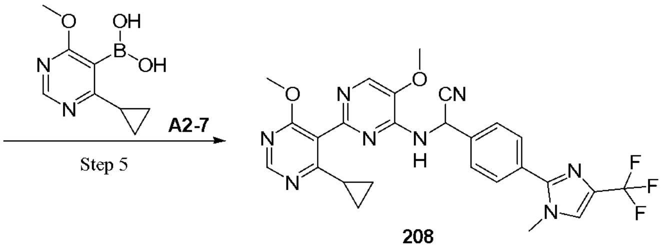


208

Step **1:** Synthesis of compound **208-1** (4-(1-methyl-4-(trifluoromethyl)-1H-imidazol-2-yl)benzaldehyde)

Compound **C5-4** (1.0 g, 3.91 mmol, 1.0 eq) and manganese dioxide (3.4 g, 39.1 mmol, 10.0 eq) were added to tetrahydrofuran (30.0 mL) successively, and the resulting mixture was stirred at an external temperature of 45°C for 16 hours. The reaction solution was filtered to obtain a filtrate. The filtrate was concentrated under vacuum to obtain a crude product which was purified by column chromatogranhv (eluent: petroleum ether/ethyl acetate = 1/0 - 1/1) to obtain compound **208-1** (823.1 mg, 3.24 mmol, 82.8% yield) as a white solid. LC-MS: m/z = 255.1 $(M+H)^+$.

Step **2:** Synthesis of compound **208-2** (2-hydroxy-2-(4-(1-methyl-4-(trifluoromethyl)-1H-imidazol-2-yl)phenyl)acetonitrile)

Compound **208-1** (400.0 mg, 1.57 mmol, 1.0 eq), trimethylsilyl cyanide (203.0 mg, 2.05 mmol, 1.3 eq) and 1-octyl-3-methylimidazolium hexafluorophosphate (3.21 g, 9.42 mmol, 6.0 eq) were added to tetrahydrofuran (8.0 mL) successively, and the resulting mixture was stirred at an external temperature of 35°C for 16 hours. The reaction solution was concentrated under vacuum to obtain a crude product, which was purified by column chromatography (eluent: petroleum ether/ethyl acetate = 1/0 - 0/1) to obtain compound **208-2** (303.3 mg, 1.08 mmol, 68.7% yield) as a colorless transparent oil. LC-MS: m/z = 282.2 $(M+H)^+$.

Step **3:** Synthesis of compound **208-3** (2-chloro-2-(4-(1-methyl-4-(trifluoromethyl)-1H-imidazol-2-yl)phenyl)acetonitrile)

Compound **208-2** (300.0 mg, 1.07 mmol, 1.00 eq) was dissolved in dichloromethane (6.0 mL), and thionyl chloride (635.0 mg, 5.33 mmol, 5.00 eq) was added at 0°C. After the addition was completed, the resulting mixture was heated up to room temperature (25°C) and stirred for 4 hours. The reaction solution was concentrated under vacuum to obtain compound **208-3** (288.8 mg, crude) as a colorless transparent oil. LC-MS: m/z = 300.2 $(M+H)^+$.

Step **4:** Synthesis of compound **208-4** (2-((2-chloro-5-methoxypyrimidin-4-yl)amino)-2-(4-(1-methyl-4-(trifluoromethyl)-1H-imidazol-2-yl)phenyl)acetonitrile)

Compound **208-3** (260.0 mg, 0.87 mmol, 1.0 eq), 2-chloro-4-amino-5-methoxypyrimidine (415.3 mg, 2.61 mmol, 3.0 eq) and pyridine (206.4 mg, 2.61 mmol, 3.0 eq) were added to toluene (5.2 mL) successively, and the resulting mixture was reacted at 140°C under microwave for 1 hour. The reaction solution was cooled to room temperature, and

concentrated under vacuum to obtain a crude product, which was purified by column chromatography (eluent: petroleum ether/ethyl acetate = 1/0 - 0/1) to obtain compound **208-4** (96.0 mg, 0.23 mmol, 26.1% yield) as a light yellow solid. LC-MS: m/z = 423.1 $(M+H)^+$.

Step **5:** Synthesis of compound **208** (2-((4'-cyclopropyl-5,6'-dimethoxy-[2,5'-bipyrimidin]-4-yl)amino)-2-(4-(1-methyl-4-(trifluoromethyl)-1H-imidazol-2-yl)phenyl)acetonitrile)

Compound **208-4** (90.0 mg, 0.21 mmol, 1.00 *eq),* compound **A2-7** (83.4 mg, 0.43 mmol, 2.0 *eq),* chloro(2-dicyclohexylphosphino-2',4',6'-triisopropyl-1,1'-biphenyl)[2-(2'-amino-1,1'-biphenyl)]palladium (II) (XPhos-Pd-G2, 33.8 mg, 0.043 mmol, 0.20 *eq*), 2-dicyclohexylphosphino-2',4',6'-triisopropylbiphenyl (XPhos, 41.0 mg, 0.086 mmol, 0.40 *eq)* and sodium bicarbonate (72.2 mg, 0.86 mmol, 4.0 *eq)* were added to dioxane (2.0 mL) and water (0.4 mL) successively. The resulting mixture was purged three times with nitrogen, and stirred at 95°C for 16 hours under nitrogen atmosphere. Water (10.0 mL) was added, and the resulting mixture was extracted three times with ethyl acetate (10.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **208** (38.2 mg, 0.071 mmol, 33.9% yield) as a white solid. $^1H$ NMR (400 MHz, DMSO-$d_6$) δ 8.70 (s, 1H), 8.60 (s, 1H), 8.01 (s, 1H), 7.92 - 7.89 (m, 2H), 7.86 - 7.81 (m, 2H), 7.53 (s, 1H),7.09 (s, 1H), 3.98 (s, 3H), 3.88 (s, 3H), 3.83 (s, 3H), 1.84-1.80 (m, 1H), 1.18 - 0.82 (m, 4H); LC-MS: m/z = 537.2 $(M+H)^+$.

**Example 209: Synthesis of compound 209 (2-(4-chloro-1-isopropyl-1H-pyrazol-5-yl)-5-methoxy-N-((1-(1-methyl-4-(trifluoromethyl)-1H-imidazol-2-yl)-2-oxabicyclo [2.2.2] octan-4-yl)methyl)pyrimidin-4-amine)**

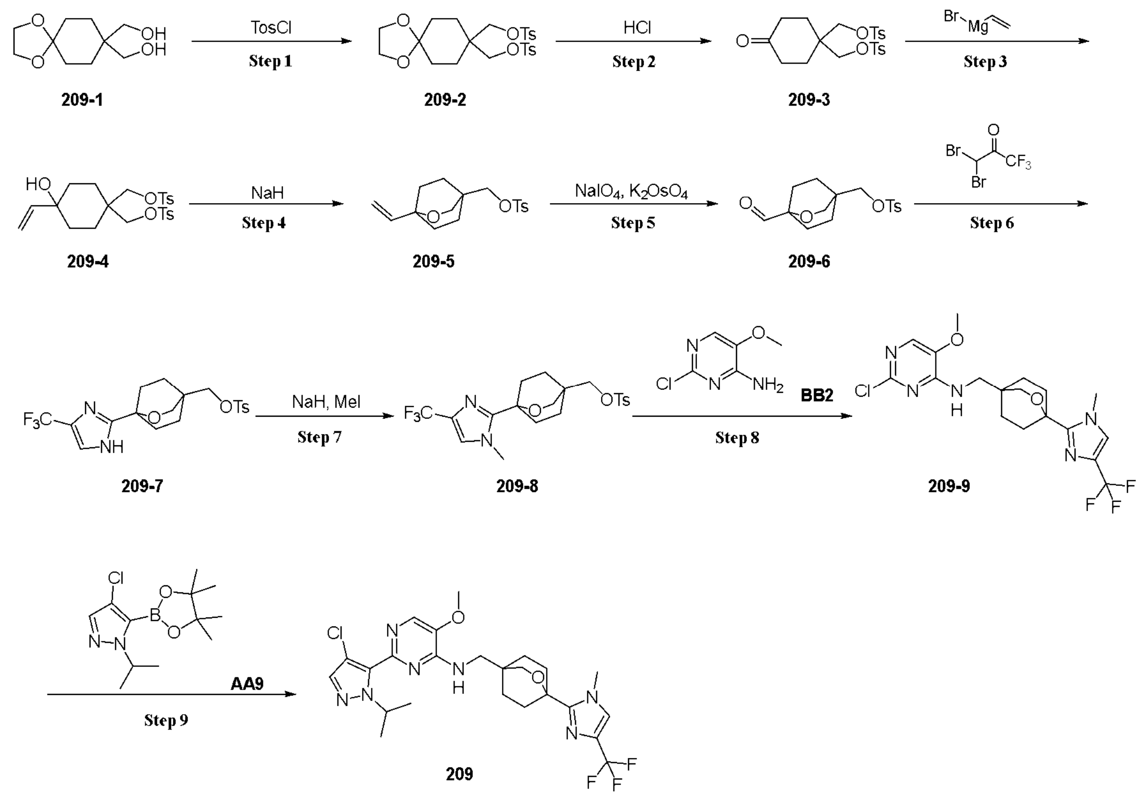


209-1, 209-2, 209-3, 209-4, 209-5, 209-6, 209-7, 209-8, 209-9, 209

Step **1:** Synthesis of compound **209-2** ((1,4-dioxaspiro[4.5]decane-8,8-diyl)bis(methylene) bis(4-methylbenzenesulfonate))

Compound **209-1** ([8-(hydroxymethyl)-1,4-dioxaspiro[4.5]decane-8-yl]methanol, 50.0 g, 247 mmol, 1.00 eq) and p-toluenesulfonyl chloride (104 g, 544 mmol, 2.20 eq) were added to pyridine (250.0 mL) successively, and the resulting mixture was stirred at 25°C for 12 hours. Ethyl acetate (500.0 mL) was added, the resulting mixture was washed

with 10% citric acid aqueous solution (500.0 mL) three times and saturated brine (500.0 mL) once, dried over anhydrous sodium sulfate, and filtered to obtain a filtrate, which was concentrated under vacuum to obtain a crude product. The crude product was triturated with ethanol (500.0 mL) for 1 hour, and filtered to obtain compound **209-2** (116.0 g, 227.0 mmol, 91.9% yield) as a white solid. $^1$H NMR (400 MHz, $CDCl_3$) δ 7.73 (d, *J* = 8.40 Hz, 4H), 7.36 (d, *J* = 8.00 Hz, 4H), 3.89 (s, 4H), 3.84 (s, 4H), 2.47 (s, 6H), 1.49 (s, 8H).

Step **2:** Synthesis of compound **209-3** ((4-oxocyclohexane-1,1-diyl)bis(methylene) bis(4-methylbenzenesulfonate))

Compound **209-2** (116 g, 227 mmol, 1.00 eq) was dissolved in tetrahydrofuran (1000 mL), followed by the addition of 1 M hydrochloric acid (579 mL, 2.55 eq), and the resulting mixture was stirred at 70°C for 12 hours. Saturated brine (200 mL) was added, and the resulting mixture was extracted three times with ethyl acetate (1000 mL). The organic layers were combined, washed twice with saturated brine (400 mL), dried over anhydrous sodium sulfate, and filtered to obtain a filtrate, which was concentrated under vacuum to obtain compound **209-3** (110 g, crude) as a white solid. $^1$H NMR (400 MHz, DMSO-$d_6$) δ 7.76 (d, *J* = 8.00 Hz, 4H), 7.48 (d, *J* = 8.40 Hz, 4H), 3.98 (s, 4H), 2.46 (d, *J* = 27.6 Hz, 6H), 2.15 - 2.11 (t, *J*= 7.20 Hz, 4H), 1.62 - 1.54 (m, 4H); LC-MS: m/z = 467.1 $(M+H)^+$.

Step **3:** Synthesis of compound **209-4** ((4-hydroxy-4-vinylcyclohexane-1,1-diyl)bis(methylene)bis(4-methylbenzenesulfonate))

Compound **209-3** (55.0 g, 118 mmol, 1.00 eq) was dissolved in tetrahydrofuran (500 mL), and a 1 M solution of vinylmagnesium bromide in tetrahydrofuran (236 mL, 2.00 eq) was slowly added dropwise at -70°C. After the addition was completed, the resulting mixture was stirred at -70°C for 2 hours. Saturated aqueous ammonium chloride solution (300 mL) was added at 0°C to quench the reaction. The resulting mixture was concentrated under vacuum to remove the solvent. Water (100 mL) was added, and the resulting mixture was extracted three times with ethyl acetate (300 mL). The organic layers were combined, dried over anhydrous sodium sulfate, and filtered to obtain a filtrate, which was concentrated under vacuum to obtain compound **209-4** (100 g, crude) as a yellow solid. $^1$H NMR (400 MHz, $CDCl_3$) δ 7.74 (dd, $J_1$ = 8.0 Hz, $J_2$ = 12.8 Hz, 4H), 7.36 (t, *J* = 8.0 Hz, 4H), 5.84 (dd, $J_1$ = 10.8 Hz, $J_2$ = 17.2 Hz, 1H), 5.17 (d, *J* = 17.2 Hz, 1H), 5.04 (d, *J* = 10.8 Hz, 1H), 3.92 (s, 2H), 3.76 (s, 2H), 2.46 (d, *J* = 1.60 Hz, 6H), 1.56-1.26 (m, 8H).

Step **4:** Synthesis of compound **209-5** ((1-vinyl-2-oxabicyclo[2.2.2]octan-4-yl)methyl-4-methylbenzenesulfonate)

Compound **209-4** (30.0 g, 60.7 mmol, 1.00 eq) was dissolved in ethylene glycol dimethyl ether (500 mL), and sodium hydride (4.85 g, 121 mmol, 60% content, 2.00 eq) was added in portions at 0°C. After the addition was completed, the resulting mixture was stirred at 0°C for 30 minutes, heated up to 110°C and stirred for 16 hours. Saturated aqueous ammonium chloride solution (400 mL) was added at 0°C to quench the reaction, and water (500 mL) was added. The resulting mixture was concentrated under vacuum to remove the organic solvent, and extracted three times with ethyl acetate (600 mL). The organic layers were combined, washed twice with saturated brine (200 mL), dried over anhydrous sodium sulfate, and filtered to obtain a filtrate, which was concentrated under vacuum to obtain compound **209-5** (27.4 g, crude) as a light yellow solid. $^1$H NMR (400 MHz, $CDCl_3$) δ 7.77 (d, *J* = 8.00 Hz, 2H), 7.36 (d, *J* = 8.00 Hz, 2H), 5.85 - 5.76 (m, 1H), *5.15*- 5.11 (t, *J*= 1.60 Hz, 1H), 5.04 - 5.00 (d, $J_1$ = 1.60 Hz, $J_2$ = 11.2 Hz, 1H), 3.69 (d, *J* = 6.80 Hz, 4H), 2.46 (s, 3H), 1.90 - 1.86 (m, 2H), 1.72 - 1.65 (m, 4H), 1.52 - 1.51 (m, 2H).

Step **5:** Synthesis of compound **209-6** ((1-formyl-2-oxabicyclo[2.2.2]octan-4-yl)methyl-4-methylbenzenesulfonate)

Compound **209-5** (4.00 g, 12.4 mmol, 1.00 *eq)* was dissolved in tetrahydrofuran (80.0 mL), and sodium periodate (7.96 g, 37.2 mmol, 2.06 mL, 3.00 *eq)* was added at 0°C. Potassium osmate (1.14 g, 3.10 mmol, 0.250 *eq)* dissolved in water (16.0 mL) was added, and the resulting mixture was stirred at 20°C for 12 hours, and filtered to obtain a filtrate. The filtrate was washed three times with saturated brine (100 mL), dried over anhydrous sodium sulfate, and filtered to obtain a filtrate, which was concentrated under vacuum to obtain **209-6** (4.0 g, crude) as a yellow oil. $^1$H NMR (400 MHz, $CDCl_3$) δ 9.53 (s, 1H), 7.77 - 7.74 (m, 2H), 7.37 - 7.34 (m, 2H), 3.74 - 3.67 (m, 4H), 2.46 (s, 3H),1.91 1.49 (m, 8H).

Step 6: Synthesis of compound **209-7** ((1-(4-(trifluoromethyl)-1H-imidazol-2-yl)-2-oxabicyclo[2.2.2]octan-4-yl) methyl-4-methylbenzenesulfonate)

3,3-Dibromo-1,1,1-trifluoropropane-2-one (3.99 g, 14.8 mmol, 1.20 *eq)* and sodium acetate (2.12 g, 25.9 mmol, 2.10 *eq)* were added to water (8.0 mL) successively, and the resulting mixture was stirred at 100°C for 1 hour. Compound **209-6** (4.00 g, 12.3 mmol, 1.00 eq) dissolved in ammonia water (11.5 g, 98.7 mmol, 12.7 mL, 30.0% content, 8.0 eq) and methanol (40.0 mL) was added at 25°C. After the addition was completed, the resulting mixture was stirred at 25°C for 5

hours. Water (100 mL) was added, and the resulting mixture was extracted three times with ethyl acetate (180 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **209-7** (2.2 g, 5.05 mmol, 41.0% yield) as a white solid. LC-MS: m/z = 431.2 $(M+H)^+$.

Step **7:** Synthesis of compound **209-8** ((1-(1-methyl-4-(trifluoromethyl)-1H-imidazol-2-yl)-2-oxabicyclo[2.2.2]octan-4-yl)methyl-4-methylbenzenesulfonate)

Compound **209-7** (2.20 g, 5.11 mmol, 1.00 *eq)* was dissolved in tetrahydrofuran (30.0 mL), and sodium hydride (307 mg, 7.67 mmol, 60.0% content, 1.50 *eq*) was added in portions at 0°C. After the addition was completed, the resulting mixture was stirred at 0°C for 30 minutes. Iodomethane (725 mg, 5.11 mmol, 1.0 *eq)* was added, and the resulting mixture was heated up to 25°C and reacted for 2 hours. Ice water (30.0 mL) was added at 0°C to quench the reaction. Water (20.0 mL) was added, and the resulting mixture was extracted with ethyl acetate (150 mL) three times. The organic layers were combined, washed twice with saturated brine (60.0 mL), dried over anhydrous sodium sulfate, and filtered to obtain a filtrate. The filtrate was concentrated under vacuum to obtain a crude product, which was purified by prep-HPLC (column: Phenomenex luna C18 150×40mm×15μm; mobile phase: [water (TFA)-ACN]; gradient: 45%-75%) to obtain compound **209-8** (1.5 g, 3.37 mmol, 66.0% yield) as a white solid. $^1H$ NMR (400 MHz, $CDCl_3$) δ 7.78 (d, *J*= 8.4 Hz, 2H), 7.37 (d, *J*= 8.0 Hz, 2H), 7.11 (d, *J*= 1.2 Hz, 1H), 3.81 (s, 5H), 3.73 (s, 2H), 2.47 (s, 3H), 2.28 - 2.22 (m, 2H), 2.19 - 2.13 (m, 2H), 1.78 - 1.71 (m, 2H), 1.62 - 1.55 (m, 2H); LC-MS: m/z = 445.3 $(M+H)^+$.

Step **8:** Synthesis of compound **209-9** (2-chloro-5-methoxy-N-((1-(1-methyl-4-(trifluoromethyl)-1H-imidazol-2-yl)-2-oxabicyclo[2.2.2]octan-4-yl)methyl)pyrimidin-4-amine)

Compound **209-8** (1.0 g, 2.25 mmol, 1.0 eq), compound **BB2** (716.0 mg, 4.50 mmol, 2.0 eq), sodium iodide (1.01 g, 6.75 mmol, 3.0 eq) and cesium carbonate (3.67 g, 11.25 mmol, 5.0 eq) were added to N,N-dimethylformamide (20.0 mL) successively, and the mixture was stirred at 130°C for 24 hours. The reaction solution was cooled to 25°C. Dichloromethane (100.0 mL) was added, the resulting mixture was stirred for 5 minutes, and filtered to obtain a filtrate. The filtrate was washed three times with saturated brine (50.0 mL), dried over anhydrous sodium sulfate, and concentrated under vacuum to obtain a crude product. The crude product was purified by column chromatography (eluent: petroleum ether/dichloromethane = 1/0 - 0/1) to obtain compound **209-9** (135.6 mg, 0.31 mmol, 14.0% yield) as a white solid. LC-MS: m/z = 432.3 $(M+H)^+$.

Step **9:** Synthesis of compound **209** (2-(4-chloro-1-isopropyl-1H-pyrazol-5-yl)-5-methoxy-N-((1-(1-methyl-4-(trifluoromethyl)-1H-imidazol-2-yl)-2-oxabicyclo[2.2.2]octan-4-yl)methyl)pyrimidin-4-amine)

Compound **209-9** (40.0 mg, 92.6 μmol, 1.00 *eq),* compound **AA9** (75.2 mg, 277.8 μmol, 3.00 *eq),* and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (Xphos Pd G4, 16.0 mg, 18.52 μmol, 0.2 *eq),* 2-dicyclohexylphosphino-2',4',6'-triisopropylbiphenyl (Xphos, 17.7 mg, 37.04 μmol, 0.4 *eq)* and potassium phosphate (78.6 mg, 370.4 μmol, 4 *eq)* were added to dioxane (0.8 mL) and water (0.16 mL) successively, and the resulting mixture was stirred at an external temperature of 95°C for 16 hours. The reaction solution was concentrated under vacuum to obtain a crude product, which was purified by prep-HPLC (column: Phenomenex luna C18 150×25mm×10μm; mobile phase: [water (FA)-ACN]; gradient: 61%-91%) to obtain compound **209** (16.6 mg, 30.8 μmol, 33.3% yield) as a white solid. $^1H$ NMR (400 MHz, $CDCl_3$) δ 8.21 (s, 1H), 7.55 (s, 1H),7.13 (s, 1H), 6.01-5.88 (m, 1H), 5.32 - 5.26 (m, 1H), 4.27 (s, 2H), 4.01 (s, 2H), 4.00 (s, 3H), 3.87 (s, 3H), 2.39 - 2.31 (m, 2H), 2.29 - 2.21 (m, 2H), 1.97 - 1.91 (m, 2H), 1.83 - 1.76 (m, 2H), 1.51 (s, 3H), 1.49 (s, 3H); LC-MS: m/z =540.3 $(M+H)^+$.

**Example 210: Synthesis of compound 210 (4'-cyclopropyl-5,6'-dimethoxy-N-(1-(1-methyl-4-(trifluoromethyl)-1H-imidazol-2-yl)-2-oxabicyclo [2.2.2] octan-4-yl)methyl)-[2,5'-bipyrimidin]-4-amine)**

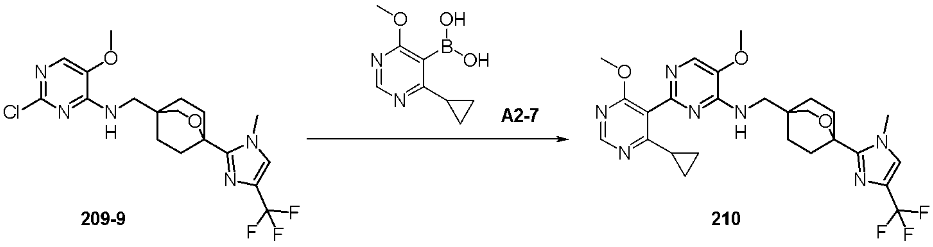


209-9 210

In accordance with the same steps of compound **209,** compound **210** (7.8 mg, 0.0143 mmol, 12.3% yield, a white solid) was obtained using compound **209-9** (50.0 mg, 0.116 mmol, 1 eq) and intermediate **A2-7** (67.4 mg, 0.348 mmol, 3.0 eq) as starting materials. $^1$H NMR (400 MHz, $CDCl_3$) δ 8.63 (s, 1H), 7.91 (s, 1H),7.11 (s, 1H), 5.58 (s, 1H), 3.99 (s, 3H), 3.95 (s, 3H), 3.87 (s, 2H), 3.83 (s, 3H), 3.43 (d, *J*= 6.4 Hz, 2H), 2.29 - 2.21 (m, 4H), 1.82 - 1.71 (m, 5H), 1.23 - 1.21 (m, 2H), 0.94 - 0.91 (m, 2H); LC-MS: m/z = 546.3 (M+H)$^+$.

**Example 211: Synthesis of compound 211 (2-(4-chloro-1-isopropyl-1H-pyrazol-5-yl)-5-methoxy-N-methyl-N-((1-(1-methyl-4-(trifluoromethyl)-1H-imidazol-2-yl)-2-oxabicyclo [2.2.2] octan-4-yl)methyl)pyrimidin-4-amine)**

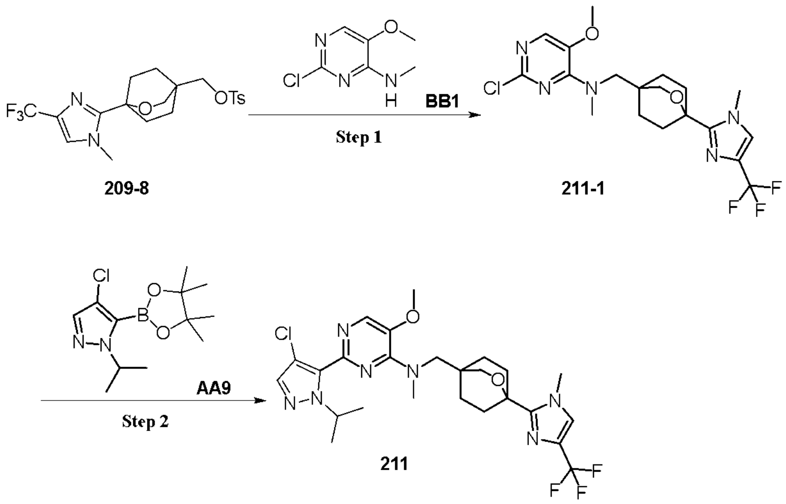


209-8 211-1

211

In accordance with the same steps of compound **209,** compound **211** (44.3 mg, 0.08 mmol, 18.9% yield, a light yellow solid) was obtained using compound **209-8** (300.0 mg, 0.675 mmol, 1 eq) and intermediate **BB-1** (351.5 mg, 2.02 mmol, 3.0 eq) as starting materials. LC-MS: m/z = 554.2 (M+H)$^+$.

**Example 212: Synthesis of compound 212 (4'-cyclopropyl-5,6'-dimethoxy-N-methyl-N-((1-(1-methyl-4-(trifluoromethyl)-1H-imidazol-2-yl)-2-oxabicyclo[2.2.2]octan-4-yl)methyl)-[2,5'-bipyrimidin]-4-amine)**

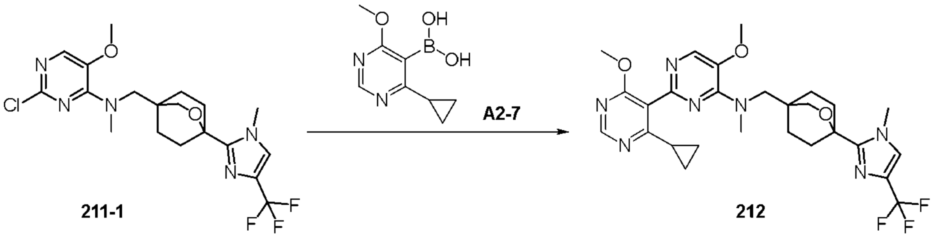


211-1 212

In accordance with the same steps of compound **209,** compound **212** (5.7 mg, 0.01 mmol, 15.2% yield, a white solid) was obtained using compound **211-1** (30.0 mg, 0.067 mmol, 1 eq) and intermediate **A2-7** (39.2 mg, 0.20 mmol, 3.0 eq) as starting materials. LC-MS: m/z = 560.3 (M+H)$^+$.

**Example 213: Synthesis of compound 213 (2-(4-chloro-1-isopropyl-1H-pyrazol-5-yl)-N-((1-(1-cyclopropyl-4-(trifluoromethyl)-1H-imidazol-2-yl)-2-oxabicyclo [2.2.2] octan-4-yl)methyl)-5-methoxypyrimidin-4-amine)**

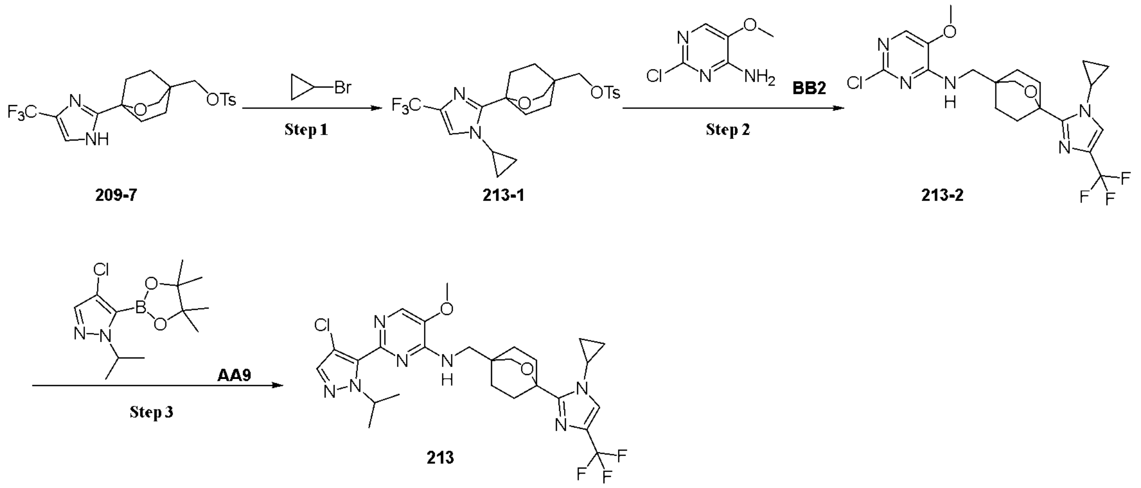

209-7 213-1 213-2 213

In accordance with the same steps of compound **209,** compound **213** (11.6 mg, 0.02 mmol, 17.2% yield, a white solid) was obtained using compound **209-7** (1.0 g, 2.32 mmol, 1 eq) and cyclopropyl bromide (843.2 mg, 6.97 mmol, 3.0 eq) as starting materials. LC-MS: m/z = 566.3 (M+H)$^+$.

**Example 214: Synthesis of compound 214 (2-(4-chloro-1-isopropyl-1H-pyrazol-5-yl)-N-((1-(1-isopropyl-4-(trifluoromethyl)-1H-imidazol-2-yl)-2-oxabicyclo [2.2.2] octan-4-yl)methyl)-5-methoxypyrimidin-4-amine)**

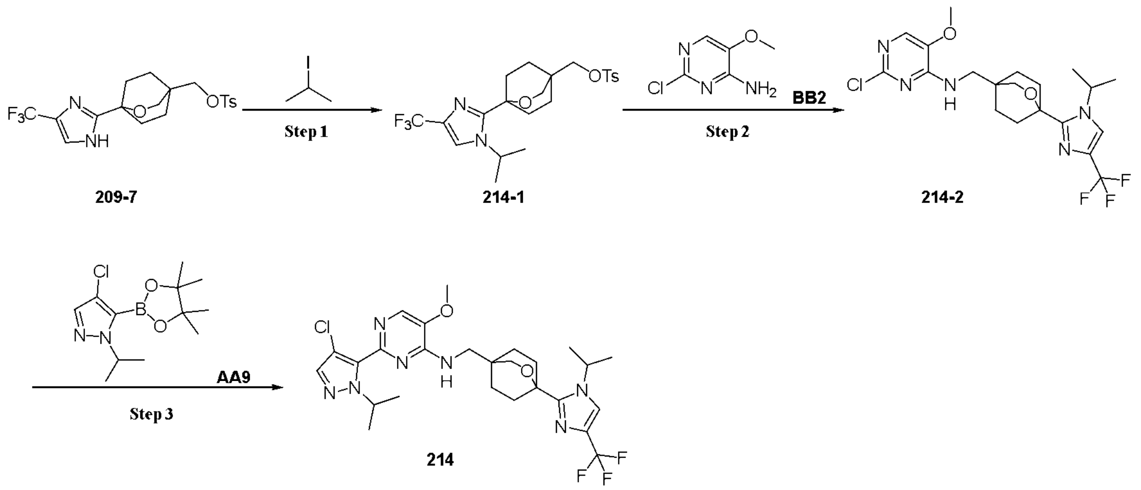

209-7 214-1 214-2 214

In accordance with the same steps of compound **209,** compound **214** (10.4 mg, 0.018 mmol, 20.4% yield, a white solid) was obtained using compound **209-7** (1.0 g, 2.32 mmol, 1 eq) and isopropyl iodide (1.18 g, 6.97 mmol, 3.0 eq) as starting materials. LC-MS: m/z = 568.4 (M+H)$^+$.

**Example 215: Synthesis of compound 215 (4'-cyclopropyl-5,6'-dimethoxy-N-(4-(1-methyl-4-(trifluoromethyl)-1H-imidazol-2-yl)cuban-1-yl)methyl)-[2,5'-bipyrimidin]-4-amine)**

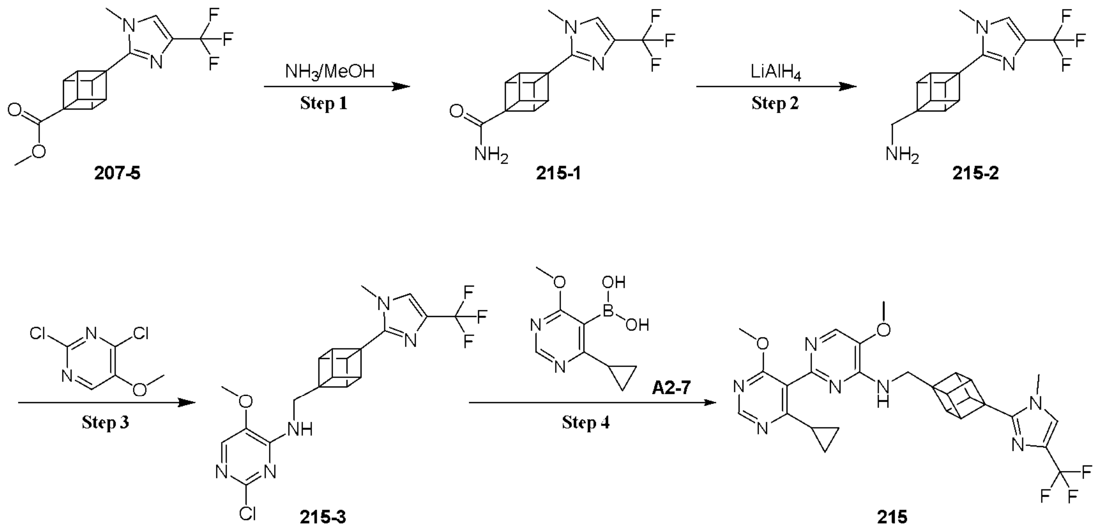


207-5 215-1 215-2 215-3 215

In accordance with the same steps of compound **207,** compound **215** (7.77 mg, 14.0 μM, 11.9% yield, a white solid) was obtained using compound **207-5** (500 mg, 1.61 mmol, 1.0 eq) and 7 M ammonia in methanol (40.0 mL, 173 eq) as starting materials. $^{1}$H NMR (400 MHz, $CDCl_3$) δ 8.61 (s, 1H), 7.87 (s, 1H), 7.19 (s, 1H), 5.46 (t, J = 6.4 Hz, 1H), 4.31 - 4.28 (m, 3H), 3.97-3.95 (m, 6H), 3.93 (s, 3H), 3.85 (d, J = 6.0 Hz, 2H), 3.58 (s, 3H), 1.81-1.75 (m, 1H), 1.21-1.17 (m, 2H), 0.92-0.87 (m, 2H); LC-MS: m/z = 538.3 $(M+H)^+$.

**Example 216: Synthesis of compound 216 (4'-cyclopropyl-N-((4-(1-cyclopropyl-4-(trifluoromethyl)-1H-imidazol-2-yl)cuban-1-yl)methyl)-5,6'-dimethoxy-[2,5'-bipyrimidin]-4-amine)**

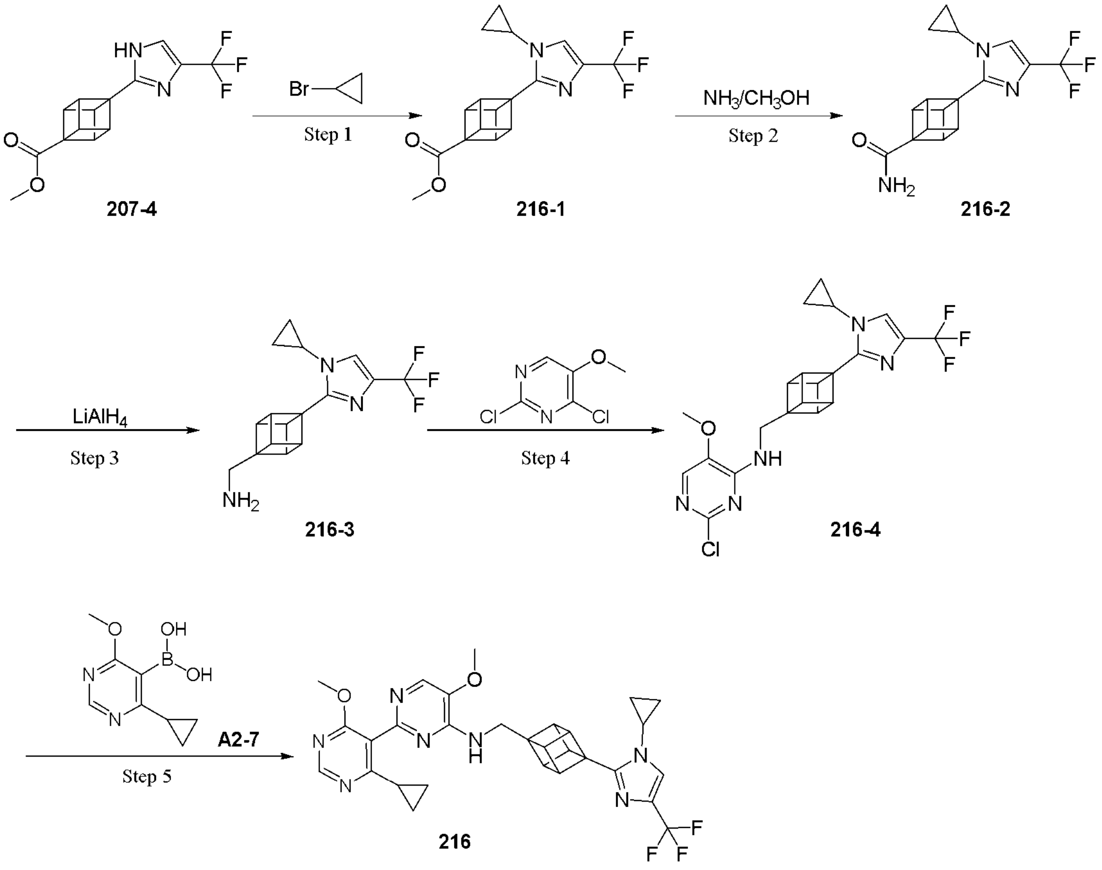


207-4 216-1 216-2 216-3 216-4 216

In accordance with the same steps of compound **207,** compound **216** (2.5 mg, 4.45 μmol, 10.5% yield, a white solid) was obtained using compound **207-4** (200 mg, 0.68 mmol, 1.0 eq) and cyclopropyl bromide (246.8 mg, 2.04 mmol, 3.0 eq) as starting materials. LC-MS: m/z = 564.2 $(M+H)^+$.

**Example 217: Synthesis of compound 217 (4'-cyclopropyl-N-((4-(1-isopropyl-4-(trifluoromethyl)-1H-imidazol-2-yl)cuban-1-yl)methyl)-5,6'-dimethoxy-[2,5'-bipyrimidin]-4-amine)**

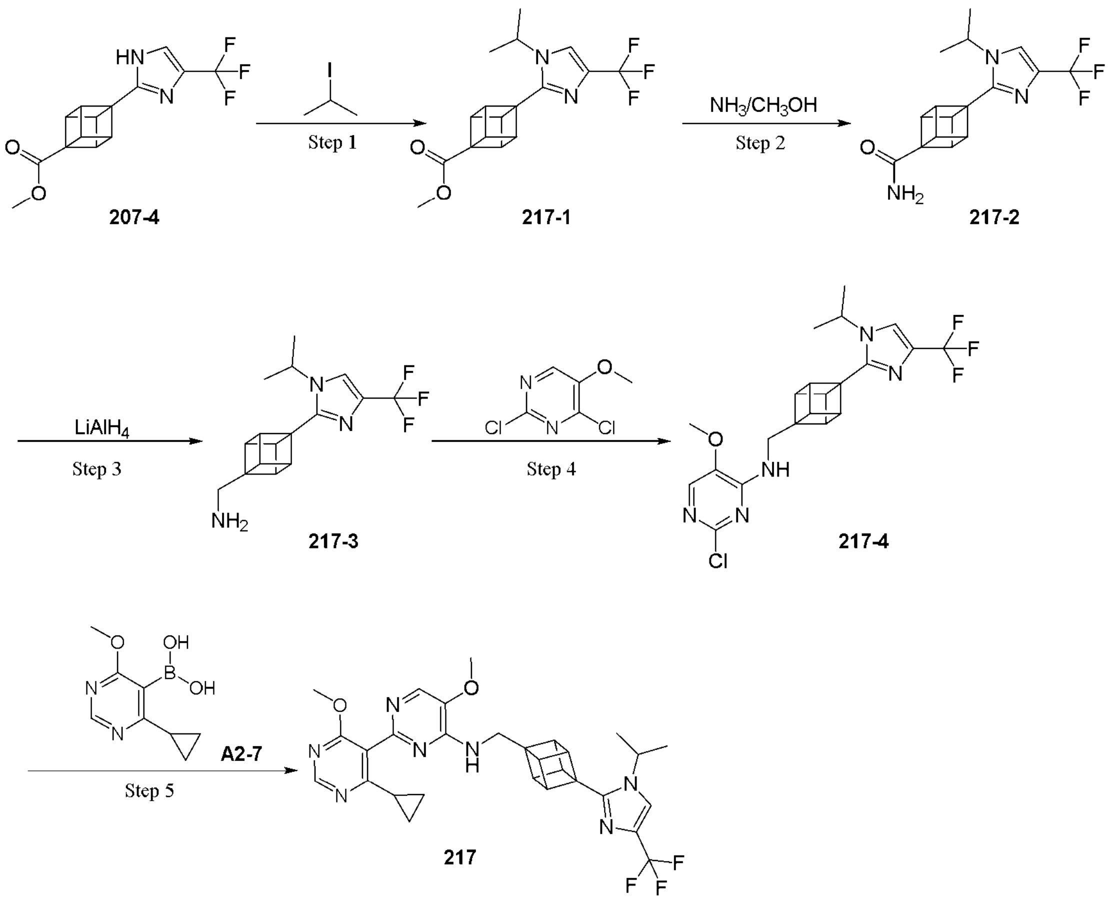


**207-4** **217-1** **217-2** **217-3** **217-4** **A2-7** **217**

In accordance with the same steps of compound **207,** compound **217** (10.5 mg, 0.019 mmol, 13.8% yield, a white solid) was obtained using compound **207-4** (600 mg, 2.03 mmol, 1.0 eq) and isopropyl iodide (1.04 g, 6.09 mmol, 3.0 eq) as starting materials. LC-MS: m/z = 566.3 $(M+H)^+$.

**Synthesis of general intermediate SA2 ((4-cyclopropyl-6-(methoxy-D3)pyrimidin-5-yl)boronic acid)**

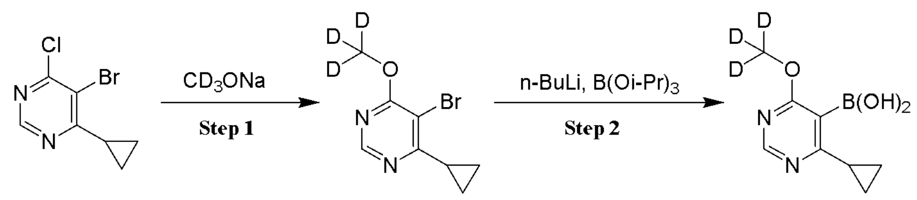


**A2-5** **SA2-1** **SA2**

In accordance with the same steps of intermediate **A2-7,** intermediate **SA2** (5.8 g, 34.4 mmol, 78.5% yield) was obtained using compound **A2-5** as a starting material. $^1H$ NMR (400 MHz, $CDCl_3$) δ 8.51 (s, 1H), 8.33 (s, 2H), 1.84-1.75 (m, 1H), 1.20-1.13 (m, 2H), 0.91-0.87 (m, 2H); LC-MS: m/z = 198.0 $(M+H)^+$.

**Synthesis of general intermediate SA4 ((4-methoxy-6-(trifluoromethyl)pyrimidin-5-yl)boronic acid)**

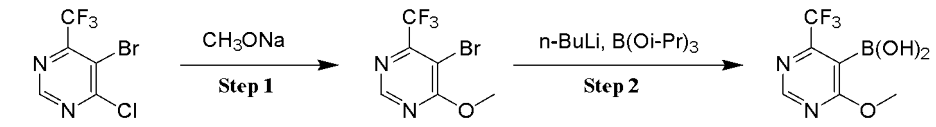


**SA4-1** **SA4-2** **SA4**

In accordance with the same steps of intermediate **A2-7,** intermediate **SA4** (2.4 g, 10.8 mmol, 57.1% yield) was obtained using 5-bromo-4-chloro-6-trifluoromethyl pyrimidine (compound **SA4-1)** as a starting material. $^1$H NMR (400 MHz, $CDCl_3$) δ 8.80 (s, 1H), 4.01 (s, 3H); LC-MS: m/z = 223.0 $(M+H)^+$.

**Synthesis of general intermediate SA5 ((4-(2-fluorocyclopropyl)-6-methoxypyrimidin-5-yl)boronic acid)**

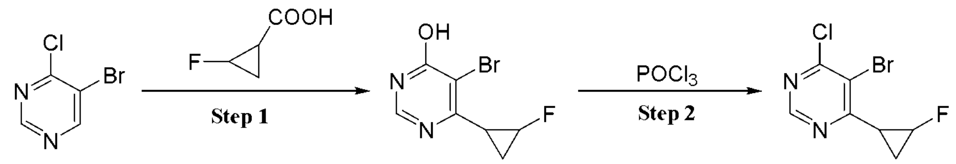


**SA5-1** **SA5-2** **SA5-3**

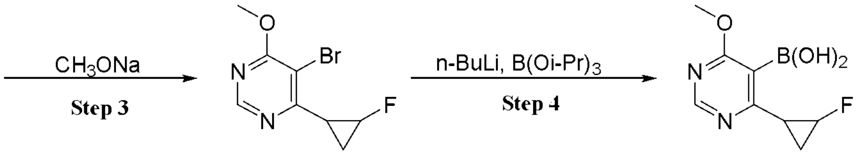


**SA5-4** **SA5**

Step 1: Synthesis of compound **SA5-2** (5-bromo-6-(2-fluorocyclopropyl)pyrimidin-4-ol)

5-Bromo-4-chloropyrimidine (compound **SA5-1,** 4.0 g, 20.68 mol) was added to acetonitrile (60.0 mL) and water (45.0 mL), followed by the addition of 2-fluorocyclopropanecarboxylic acid (6.46g, 62.04 mmol) and silver nitrate (7.03g, 41.36 mmol), and the dropwise addition of ammonium persulfate (9.44 g, 41.36 mmol) dissolved in water (15.0 mL) under stirring at 80°C under nitrogen atmosphere. After the addition was completed, the resulting mixture was stirred for 16 hours at 80°C, and cooled to room temperature. Water (150.0 mL) was added, and the resulting mixture was extracted with ethyl acetate (100.0 mL) three times. The organic layers were combined, washed with saturated brine (100.0 mL) twice, dried over anhydrous sodium sulfate, concentrated under vacuum to obtain a crude product, which was purified by column chromatography (eluent: petroleum ether/ethyl acetate = 1/0 - 1/2) to obtain compound **SA5-2** (1.4 g, 6.0 mol, 29.1% yield) as a light yellow oil. LC-MS: m/z =233.0 $(M+H)^+$.

Steps 2-4: Synthesis of intermediate S**A5**

In accordance with the same steps of intermediate **A2-7,** intermediate **SA5** (354.7 mg, 1.67 mmol, 39.9% yield) was obtained using compound **SA5-2** as a starting material. $^1$H NMR (400 MHz, $CDCl_3$) δ 8.79 (s, 1H), 6.10 - 5.91 (m, 1H), 4.02 (s, 3H), 3.19 - 3.23 (m, 1H), 1.23 - 1.49 (m, 2H); LC-MS: m/z = 213.1 $(M+H)^+$.

**Synthesis of general intermediate SA6 ((4-cyclopropoxy-6-methylpyrimidin-5-yl)boronic acid)**

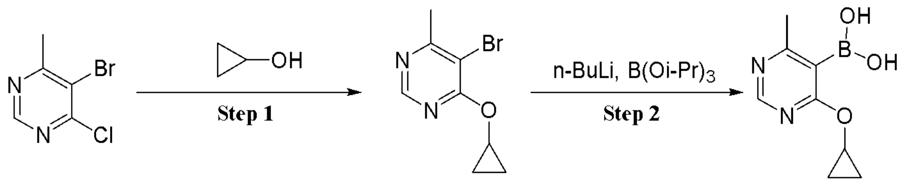


SA6-1 SA6-2 SA6

Step 1: Synthesis of compound **SA6-2** (5-bromo-4-cyclopropoxy-6-methylpyrimidine)

5-Bromo-4-chloro-6-methylpyrimidine (compound **SA6-1,** 3.0 g, 14.46 mol, 1.0 eq), cyclopropanol (2.52 g, 43.38 mol, 3.0 eq) and potassium carbonate (6.0 g, 43.38 mol, 3.0 eq) were added successively to N,N-dimethylformamide (45.0 mL), stirred for 6 hours at 60°C, and cooled to room temperature. Ethyl acetate (300.0 mL) was added, and the resulting mixture was filtered to obtain a filtrate. The filtrate was washed with saturated brine (150.0 mL) twice, dried over anhydrous sodium sulfate, concentrated under vacuum to obtain a crude product, which was purified by column chromatography (eluent: petroleum ether/ethyl acetate = 1/0 - 1/1) to obtain compound **SA6-2** (2.7 g, 11.79 mol, 81.54% yield) as a light yellow solid. LC-MS: m/z =229.0 $(M+H)^+$.

Step 2: Synthesis of intermediate S**A6**

In accordance with the same steps of intermediate **A2-7,** intermediate **SA6** (1.34 g, 6.91 mmol, 58.0% yield) was obtained using compound **SA6-2** as a starting material. $^1$H NMR (400 MHz, $CDCl_3$) δ 9.00 (s, 1H), 4.32-4.28 (m, 1H), 2.46 (s, 3H), 1.20 - 0.76 (m, 4H); LC-MS: m/z = 195.0 $(M+H)^+$.

**General intermediate SA7 ((4,6-dimethoxypyrimidin-5-yl)boronic acid)** was purchased from Leyan Reagents.

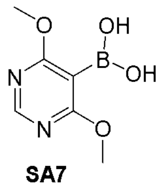

SA7

**Synthesis of general intermediate SA8 ((4-(2-fluorocyclopropyl)-6-methoxypyrimidin-5-yl)boronic acid)**

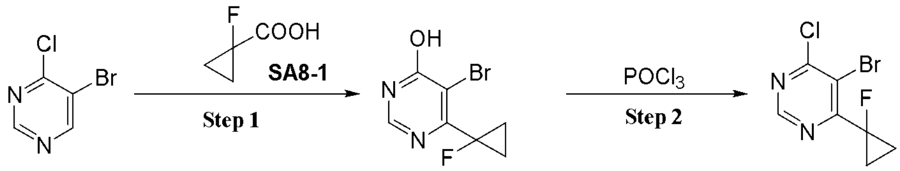


SA5-1 SA8-2 SA8-3

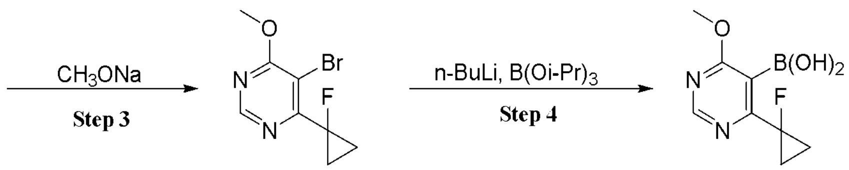


SA8-4 SA8

In accordance with the same steps of intermediate **A5,** intermediate **SA8** (0.88 g, 4.15 mmol, 39.7% yield) was obtained using compound **SA5-1** and 1-fluorocyclopropane-1-carboxylic acid (compound **SA8-1)** as starting materials. LC-MS: m/z =213.1 $(M+H)^+$.

**Synthesis of general intermediate SA10 ((4-(2,2-difluorocyclopropyl)-6-methoxypyrimidin-5-yl)boronic acid)**

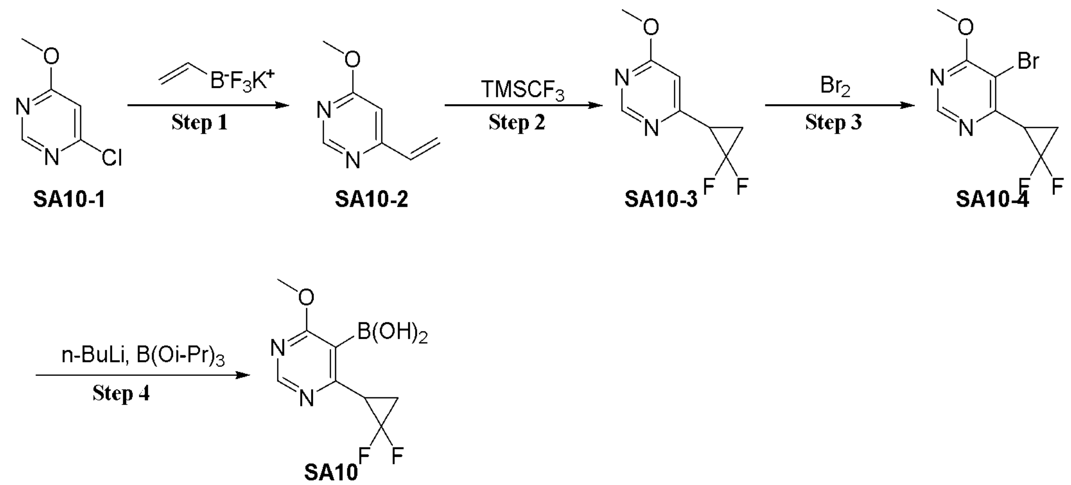


Step 1: Synthesis of compound **SA10-2** (4-methoxy-6-vinylpyrimidine)

4-Chloro-6-methoxypyrimidine (compound **SA10-1,** 5.0 g, 34.59 mmol, 1.0 eq), potassium vinyltrifluoroborate (9.27 g, 69.18 mmol, 2.0 eq), [1,1'-bis(diphenylphosphino)ferrocene]palladium dichloride (Pd(dppf)$Cl_2$, 2.5 g, 3.5 mmol, 0.1 eq) and cesium carbonate (33.8 g, 103.8 mmol, 3.0 eq) were added successively to dioxane (150.0 mL) and water (20.0 mL), purged with nitrogen three times, and stirred for 16 hours at 95°C under nitrogen atmosphere. The resulting mixture was cooled to room temperature, followed by the addition of ethyl acetate (300.0 mL), stirred for 5 minutes, and filtered to obtain a filtrate. The filtrate was washed with saturated brine (100.0 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate= 1/0 - 3/1) to obtain compound **SA10-2** (3.5 g, 25.7 mmol, 74.3% yield) as a yellow oil. LC-MS: m/z =137.1 $(M+H)^+$.

Step 2: Synthesis of compound S**A10-3** (4-(2,2-difluorocyclopropyl)-6-methoxypyrimidine)

Compound **SA10-2** (3.0 g, 22.03 mmol, 1.0 eq) and sodium iodide (1.65 g, 11.0 mmol, 0.5 eq) were added to tetrahydrofuran (50.0 mL), followed by the addition of (trifluoromethyl)trimethylsilane (12.5 g, 88.12 mmol, 4.0 eq), heated up to 60°C and stirred for 1 hour, followed by the further addition of sodium iodide (1.65 g, 11.0 mmol, 0.5 eq) and (trifluoromethyl)trimethylsilane (12.5 g, 88.12 mmol, 4.0 eq), and further stirred for 2 hours at 60°C. The resulting mixture was cooled to room temperature, followed by the addition of ethyl acetate (300.0 mL), washed with saturated brine (100.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate= 1/0 - 3/1) to obtain compound **SA10-3** (2.6 g, 14.0 mmol, 63.4% yield) as a yellow oil. LC-MS: m/z =187.1 $(M+H)^+$.

Steps 3-4: Synthesis of intermediate S**A10**

In accordance with a method with the same Steps 3 and 5 of intermediate **A2-7,** intermediate **SA10** (648.5 mg, 2.82 mmol, 41.1% yield) was obtained using compound **SA10-3** (2.0 g, 10.74 mmol) as a starting material. LC-MS: m/z =231.1 $(M+H)^+$.

**General intermediate SB3 (2-chloro-5-methoxy-N-(methyl-d3)pyrimidin-4-amine)**

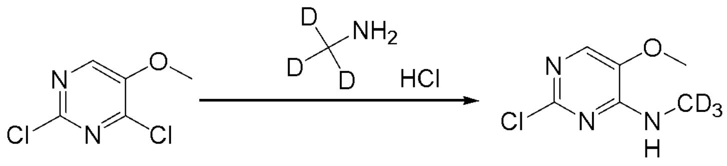


**SB3-1** **SB3**

2,4-Dichloro-5-methoxypyrimidine (compound **SB3-1,** 1.0 g, 5.59 mmol, 1.00 eq), methan-d3-amine hydrochloride (590.8 mg, 8.38 mmol, 1.5 eq) and potassium carbonate (2.32 g, 16.77 mmol, 3.0 eq) were added successively to N,N-dimethylformamide (20.0 mL), reacted for 8 hours at an external temperature of 50°C, and cooled to room temperature. Ethyl acetate (100.0 mL) was added, and the resulting mixture was washed with saturated brine (50.0 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate= 1/0 - 1/1) to obtain compound **SB3** (893.1 mg, 5.06 mmol, 90.5% yield) as a white solid. $^1$H NMR (400MHz, $CDCl_3$) δ 6.91 (s, 1H), 5.73 (s, 1H),3.79 (s, 3H); LC-MS: m/z = 177.8 $(M+H)^+$.

**Synthesis of general intermediate SC1 (2-(4-(1-chloroethyl)phenyl)-1-methyl-4-(trifluoromethyl)-1H-imidazole)**

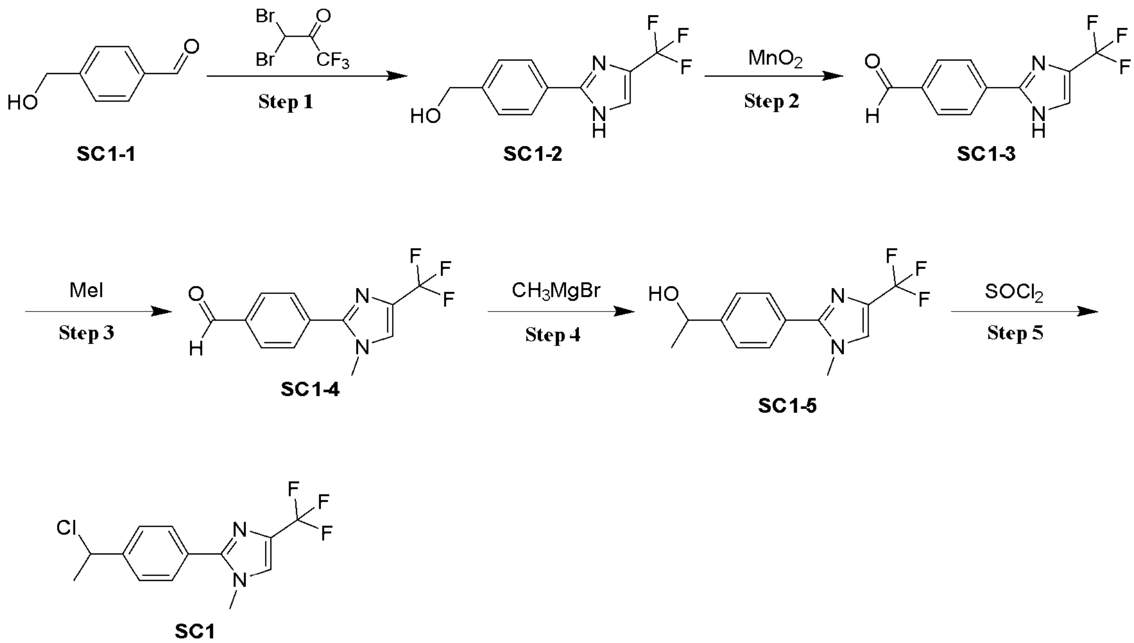


**SC1-1** **SC1-2** **SC1-3** **SC1-4** **SC1-5** **SC1**

Step 1: Synthesis of compound **SC1-2** ((4-(4-(trifluoromethyl)-1H-imidazol-2-yl)phenyl)methanol)

1,1-Dibromo-3,3,3-trifluoroacetone (7.2 g, 26.8 mmol, 1.10 eq) and sodium acetate (2.2 g, 27.3 mmol, 1.12 eq) were dissolved in water (8.0 mL), and stirred for 1 hour at 100°C, followed by the addition of *p*-(hydroxymethyl) benzaldehyde (compound **SC1-1,** 3.32 g, 24.4 mmol, 1.0 eq) dissolved in methanol (80.0 mL) and ammonia water (22.0 mL) at 25°C. After the addition was completed, the resulting mixture was reacted for 40 minutes at 25°C, heated up to 100°C and stirred for 2 hours. Then, water (60.0 mL) was added to quench the reaction, and the resulting mixture was extracted with ethyl acetate (80.0 mL) three times. The organic layers were combined, washed with saturated brine (80.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **SC1-2** (3.8 g, 15.69mmol, 64.3% yield). LC-MS: m/z =243.2 $(M+H)^+$.

Step 2: Synthesis of compound **SC1-3** (4-(4-(trifluoromethyl)-1H-imidazol-2-yl)benzaldehyde)

Compound **SC1-2** (1.0 g, 4.1 mmol, 1.0 eq) and manganese dioxide (1.78 g, 20.5 mmol, 5.0 eq) were added to tetrahydrofuran (30.0 mL), stirred for 10 hours at 50°C under nitrogen atmosphere, cooled to room temperature, and

filtered to obtain a filtrate. The filtrate was concentrated under vacuum to obtain compound **SC1-3** (897.2mg, crude product) as a grey solid. LC-MS: m/z =241.2 $(M+H)^+$.

Step 3: Synthesis of compound **SC1-4** (4-(1-methyl-4-(trifluoromethyl)-1H-imidazol-2-yl)benzaldehyde)

Compound **SC1-3** (2.2 g, 9.3 mmol, 1.00 eq) was dissolved in tetrahydrofuran (30.0 mL), followed by the addition of sodium hydride (444.1 mg, 11.1 mmol, 1.2 eq) in batches at 0°C. After the addition was completed, the resulting mixture was reacted for 30 minutes at 0°C, followed by the addition of iodomethane (6.8 g, 48.1 mmol, 5.2 eq). After the addition was completed, the resulting mixture was heated up to 20°C and stirred for 15 hours. Ice water (20.0 mL) was added at 0°C to quench the reaction, and the resulting mixture was extracted with ethyl acetate (30.0 mL) three times. The organic layers were combined, washed with saturated brine (30.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **SC1-4** (1.3 g, 5.11 mmol, 55.0% yield). $^1H$ NMR (400 MHz, $CDCl_3$) $\delta$ 9.98 (s, 1H), 7.88 (d, $J$ = 8.2 Hz, 2H), 7.85 (d, $J$ = 8.0 Hz, 2H), 7.28 (s, 1H), 3.80 (s, 3H); LC-MS: m/z = 255.4 $(M+H)^+$.

Step 4: Synthesis of compound **SC1-5** (1-(4-(1-methyl-4-(trifluoromethyl-1H-imidazol-2-yl)phenyl)ethan-1-ol)

Compound **SC1-4** (0.8 g, 3.2 mmol, 1.0 eq) was dissolved in tetrahydrofuran (30.0 mL), followed by the dropwise addition of a solution of 1 M methyl magnesium bromide (4.7 mL, 4.7mmol, 1.5 eq) in tetrahydrofuran at 0°C. After the addition was completed, the resulting mixture was stirred for 1.5 hours at 0°C. Saturated aqueous ammonium chloride solution (20.0 mL) was added to quench the reaction, and the resulting mixture was extracted with ethyl acetate (100.0 mL) three times. The organic layers were combined, dried over anhydrous sodium sulfate, concentrated under vacuum to obtain a crude product, which was purified by column chromatography (eluent: petroleum ether/ethyl acetate = 1/0 - 0/1) to obtain compound **SC1-5** (386.0 mg, 1.43 mmol, 44.6% yield) as a colorless transparent oil. LC-MS: m/z =271.2 $(M+H)^+$.

Step 5: Synthesis of intermediate **SC1**

Compound **SC1-5** (540.0 mg, 2.0 mmol, 1.0 eq) was dissolved in dichloromethane (10.0 mL), followed by the addition of thionyl chloride (1.9 g, 15.6 mmol, 8.0 eq) at 0°C. After the addition was completed, the resulting mixture was heated up to 20°C and stirred for 15 hours. The reaction solution was concentrated under vacuum to obtain compound **SC1** (498.2 mg, 1.73 mmol, 86.3% yield) as a light yellow oil. $^1H$ NMR (400 MHz, DMSO-d6) $\delta$ 7.64 (d, $J$ = 8.4 Hz, 2H), 7.48 (d, $J$= 8.2 Hz, 2H), 7.37 (s, 1H), 5.15-4.98 (m, 1H), 3.80 (s, 3H), 1.88 (s, 3H),; LC-MS: m/z = 289.8 $(M+H)^+$.

**Synthesis of general intermediate SC2 (2-(4-(1-chloroethyl)-2-fluorophenyl)-1-methyl-4-(trifluoromethyl)-1H-imidazole)**

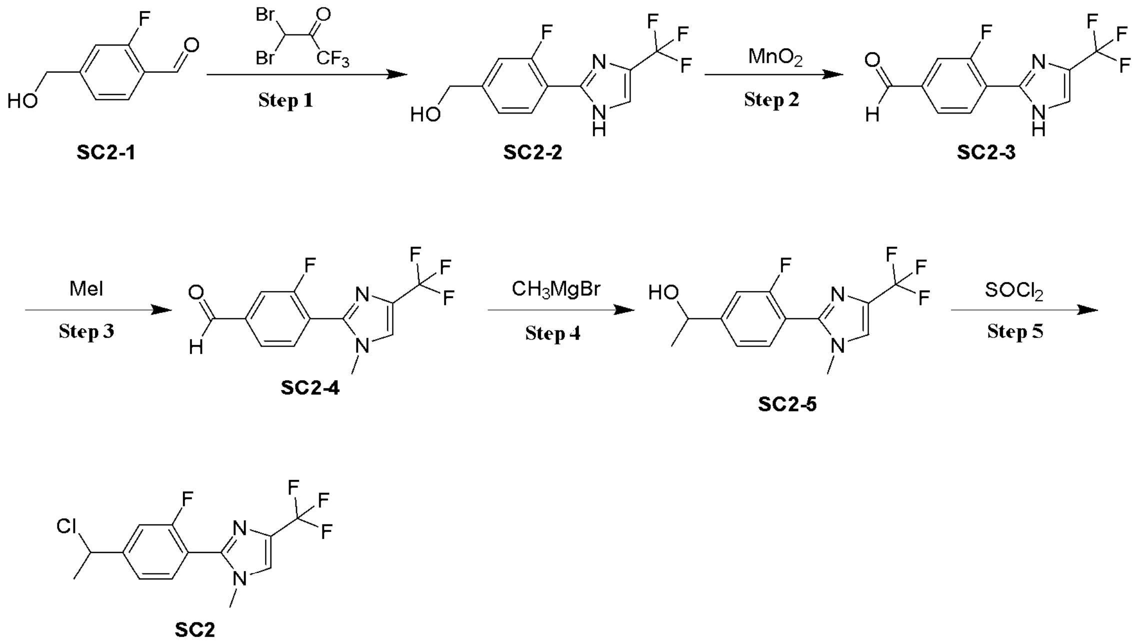


SC2-1 SC2-2 SC2-3 SC2-4 SC2-5 SC2

In accordance with the same steps of intermediate **SC1,** intermediate **SC2** (134.4 mg, 0.44 mmol, 68.9% yield) was obtained using 2-fluoro-4-hydroxymethylbenzaldehyde (compound **SC2-1,** 1.0 g, 6.49 mmol) and 1,1-dibromo-3,3,3-trifluoroacetone (1.93 g, 7.14 mmol, 1.1 eq) as starting materials. LC-MS: m/z =307.7 $(M+H)^+$.

**Synthesis of general intermediate SC4 (2-(4-(chloromethyl)phenyl)-1-(2,2,2-trifluoroethyl)-4-(trifluoromethyl)-1H-imidazole)**

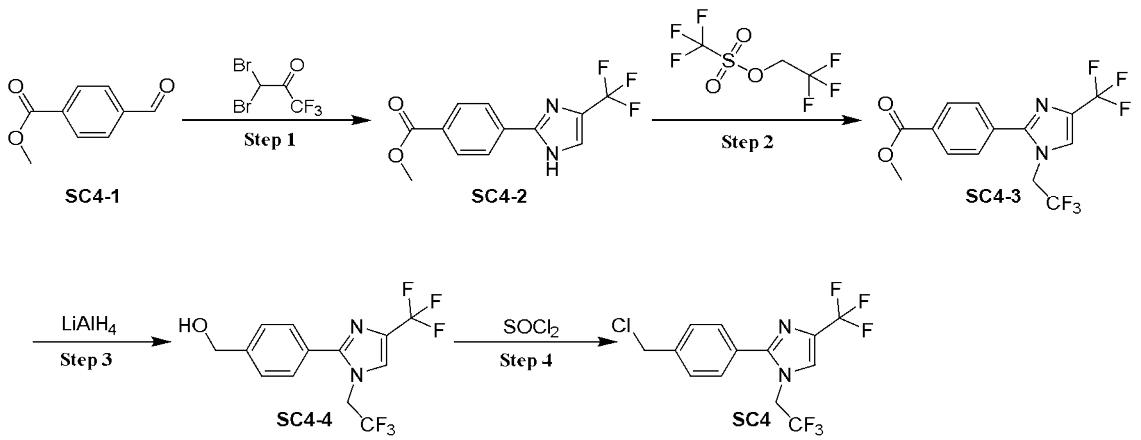


SC4-1 SC4-2 SC4-3 SC4-4 SC4

Step 1: Synthesis of compound **SC4-2** (methyl 4-(4-(trifluoromethyl)-1H-imidazol-2-yl)benzoate)

1,1-Dibromo-3,3,3-trifluoroacetone (7.2 g, 26.8 mmol, 1.1 eq) and sodium acetate (2.2 g, 27.3 mmol, 1.12 eq) were dissolved in water (8.0 mL), and stirred for 1 hour at 100°C, followed by the addition of methyl p-formylbenzoate (compound **SC4-1,** 4.0 g, 24.4 mmol, 1.0 eq) dissolved in methanol (80.0 mL) and ammonia water (22.0 mL) at 25°C. After the addition was completed, the resulting mixture was reacted for 40 minutes at 25°C, heated up to 100°C and stirred for 2 hours. Then, water (60.0 mL) was added to quench the reaction, and the resulting mixture was extracted with ethyl acetate (80.0 mL) three times. The organic layers were combined, washed with saturated brine (80.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **SC4-2** (4.5 g, 16.65 mmol, 62.1% yield). $^1$H NMR (400 MHz, $CDCl_3$) δ 13.4 (s, 1H), 8.10 - 8.12 (m, 2H), 8.04 - 8.06 (m, 2H), 7.99 (s, 1H), 3.87 (s, 3H); LC-MS: m/z = 271.0 $(M+H)^+$.

Step 2: Synthesis of compound **SC4-3** (methyl 4-(1-(2,2,2-trifluoroethyl)-4-(trifluoromethyl)-1H-imidazol-2-yl)benzoate)

Compound **SC4-2** (2.5 g, 9.3 mmol, 1.00 eq) was dissolved in tetrahydrofuran (60.0 mL), followed by the addition of sodium hydride (3.7 g, 93.0 mmol, 10.0 eq) in batches at 0°C. After the addition was completed, the resulting mixture was reacted for 30 minutes at 0°C, followed by the addition of 2,2,2-trifluoroethyl trifluoromethanesulfonate (6.48 g, 27.9 mmol, 3.0 eq). After the addition was completed, the resulting mixture was heated up to 20°C and stirred for 15 hours. Ice water (60.0 mL) was added at 0°C to quench the reaction, and the resulting mixture was extracted with ethyl acetate (100.0 mL) three times. The organic layers were combined, washed with saturated brine (100.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **SC4-3** (789.5 mg, 2.24 mmol, 24.1% yield). LC-MS: m/z =353.2 $(M+H)^+$.

Step 3: Synthesis of compound **SC4-4** ((4-(1-(2,2,2-trifluoroethyl)-4-(trifluoromethyl)-1H-imidazol-2-yl)phenyl)methanol)

Compound **SC4-3** (600.0 mg, 1.7 mmol, 1.0 eq) was dissolved in tetrahydrofuran (12.0 mL), followed by the addition of lithium aluminum tetrahydride (97.1 mg, 2.56 mmol, 1.5 eq) at 0°C. After the addition was completed, the resulting mixture was heated up to 25°C and stirred for 4 hours. Water (1.6 mL) and aqueous sodium hydroxide solution (1M, 0.4 mL) were added at 0°C, and the resulting mixture was stirred for 30 minutes at 0°C. Tetrahydrofuran (10.0 mL) was added, and the resulting mixture was dried over anhydrous sodium sulfate and filtered to obtain a filtrate. The filtrate was concentrated under vacuum to obtain crude compound **SC4-4** (493.3 mg, crude product), which was directly used for the next reaction step. LC-MS: m/z =325.3 $(M+H)^+$.

Step 4: Synthesis of intermediate S**C4**

Compound **SC4-4** (400.0 mg, 1.23 mmol, 1.0 eq) was dissolved in dichloromethane (8.0 mL), followed by the addition of thionyl chloride (880.6 mg, 7.4 mmol, 6.0 eq) at 0°C. After the addition was completed, the resulting mixture was heated up to 20°C and stirred for 15 hours. The reaction solution was concentrated under vacuum to obtain compound **SC4** (316.4 mg, 0.92 mmol, 75.1% yield) as a light yellow oil. LC-MS: m/z =343.8 $(M+H)^+$.

**Synthesis of general intermediate SC5 (2-(4-(chloromethyl)phenyl)-1-(2-**fluoroethyl)-4-(trifluoromethyl)-1H-imidazole)

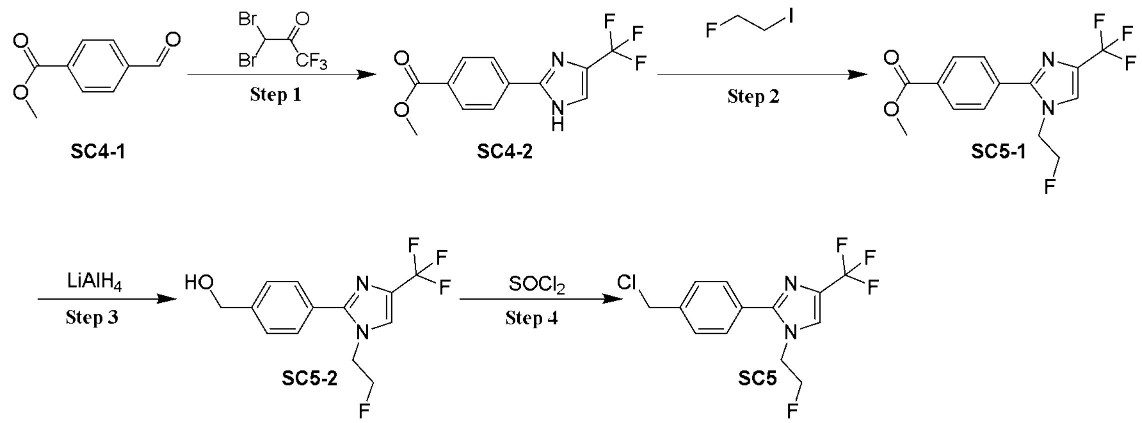

SC4-1
SC4-2
SC5-1
SC5-2
SC5

In accordance with the same steps of intermediate **SC4,** intermediate **SC5** (87.2 mg, 0.28 mmol, 73.3% yield) was obtained using compound **SC4-1** (4.0 g, 24.4 mmol, 1.0 eq) and 1,1-dibromo-3,3-trifluoroacetone (7.2 g, 26.8 mmol, 1.1 eq) as starting materials. LC-MS: m/z =307.8 $(M+H)^+$.

**Synthesis of general intermediate SC7 ((1-(1-cyclobutyl-4-(trifluoromethyl)-1H-imidazol-2-yl)-2-oxabicyclo [2.2.2]oct-4-yl)methyl-4-methylbenzenesulfonate)**

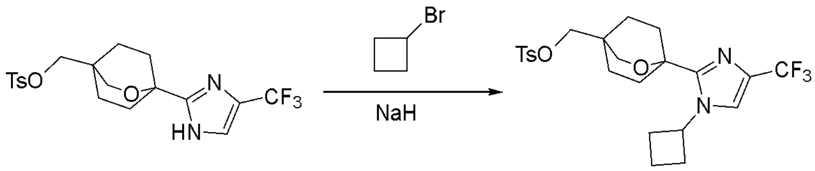


209-7 SC7

In accordance with the same steps of intermediate **C6,** intermediate **SC7** (186.5 mg, 0.38 mmol, 33.2% yield) as a white solid was obtained using compound **209-7** (0.5 g, 1.16 mmol, 1.0 eq) and cyclobutyl bromide (470.4 mg, 3.48 mmol, 3.0 eq) as starting materials. LC-MS: m/z =485.5 $(M+H)^+$.

**Synthesis of general intermediate SC8 ((1-(1-isopropyl-4-(trifluoromethyl)-1H-imidazol-2-yl)-2-oxabicyclo [2.2.2]oct-4-yl)methyl-4-methylbenzenesulfonate)**

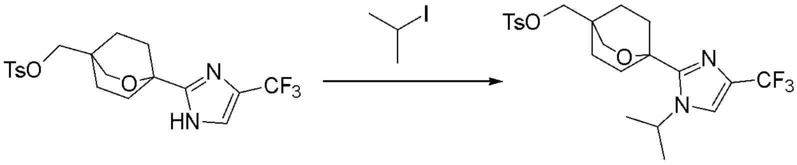

209-7 SC8

In accordance with the same steps of compound **209-8,** intermediate **SC8** (345.9 mg, 0.73 mmol, 63.1% yield) as a white solid was obtained using compound **209-7** (0.5 g, 1.16 mmol, 1.0 eq) and isopropyl iodide (591.6 mg, 3.48 mmol, 3.0 eq) as starting materials. LC-MS: m/z =473.6 $(M+H)^+$.

**Synthesis of general intermediate SC9 ((1-(1-cyclopropyl-4-(trifluoromethyl)-1H-imidazol-2-yl)-2-oxabicyclo [2.2.2]oct-4-yl)methyl-4-methylbenzenesulfonate)**

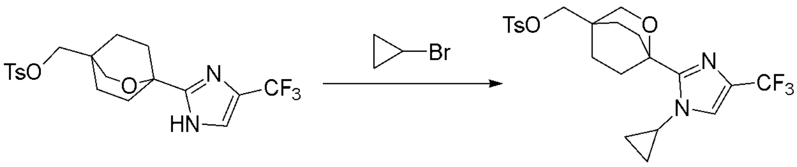

209-7 SC9

In accordance with the same steps of compound **209-8,** intermediate **SC9** (89.3 mg, 0.19 mmol, 16.4% yield) as a white solid was obtained using compound **209-7** (0.5 g, 1.16 mmol, 1.0 eq) and cyclopropyl bromide (421.0 mg, 3.48 mmol, 3.0 eq) as starting materials. LC-MS: m/z =471.7 $(M+H)^+$.

**Synthesis of general intermediate SC11 (2-((1R,4R)-4-(chloromethyl)cyclohexyl)-1-cyclopropyl-4-(trifluoromethyl)-1H-imidazole)**

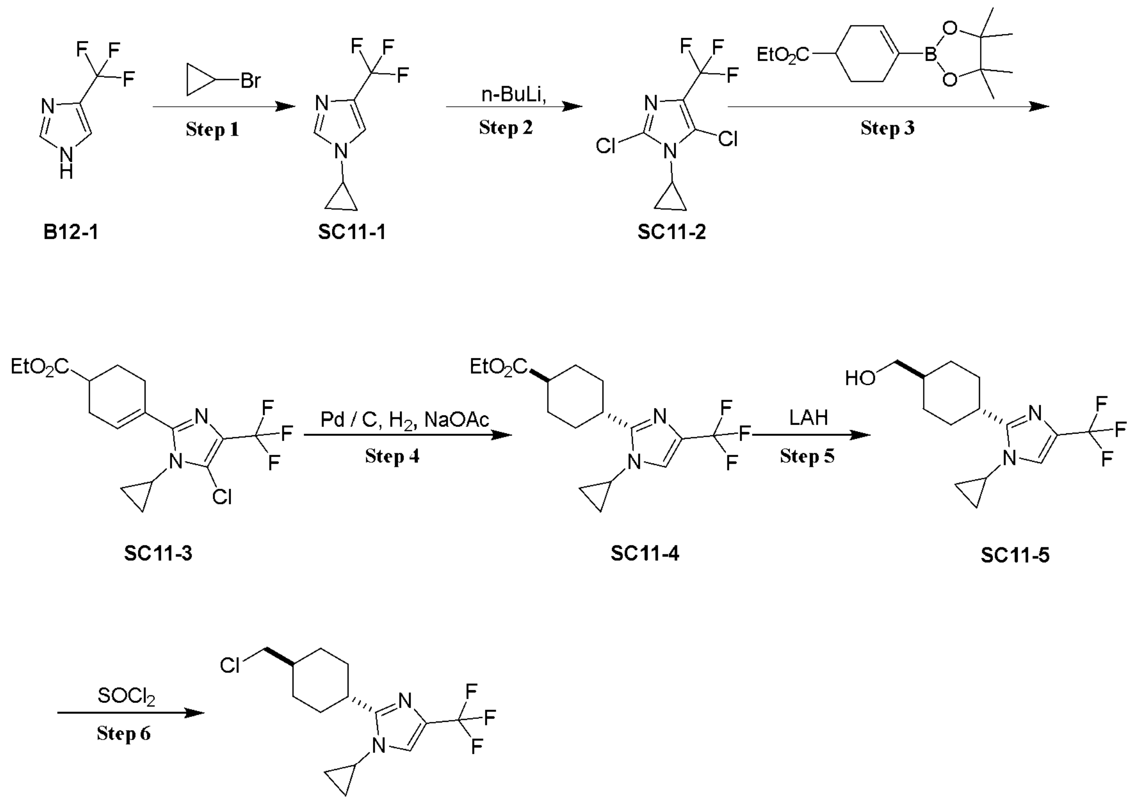


B12-1 SC11-1 SC11-2

SC11-3 SC11-4 SC11-5

SC11

In accordance with the same steps of intermediate B12, intermediate **SC11** (98.2 mg, 0.32 mmol, 89.8% yield) as a light yellow oil was obtained using compound **B12-1** (10.0 g, 73.49 mmol, 1.0 eq) and cyclopropyl bromide (26.67 g, 220.46 mmol, 3.0 eq) as starting materials. LC-MS: m/z =307.8 $(M+H)^+$.

**Synthesis of general intermediate SC12 (2-((1R,4R)-4-(chloromethyl)cyclohexyl)-1-isopropyl-4-(trifluoromethyl)-1H-imidazole)**

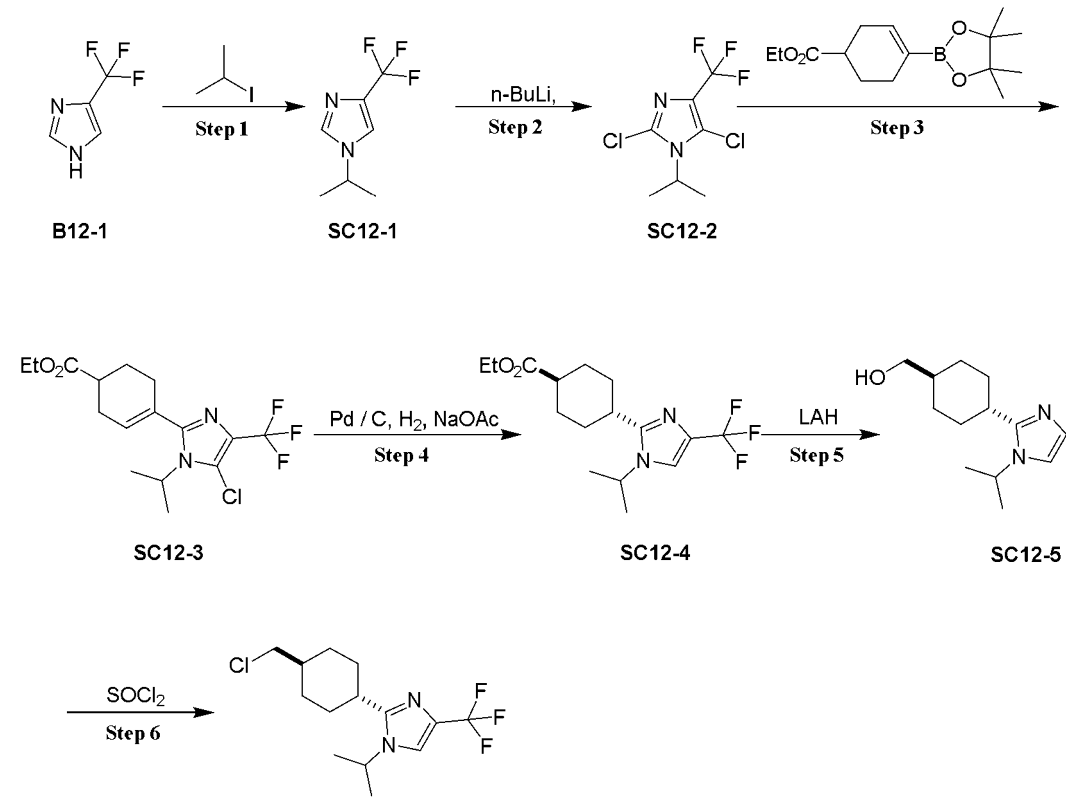


B12-1 SC12-1 SC12-2

SC12-3 SC12-4 SC12-5

SC12

In accordance with the same steps of intermediate **B12,** intermediate **SC12** (398.3 mg, 1.29 mmol, 91.9% yield) as a colorless transparent oil was obtained using compound **B12-1** (10.0 g, 73.49 mmol, 1.0 eq) and isopropyl iodide (37.5 g, 220.46 mmol, 3.0 eq) as starting materials. LC-MS: m/z =309.8 $(M+H)^+$.

**Intermediate BB2SC1 (2-chloro-5-methoxy-N-(1-(4-(1-methyl-4-(trifluoromethyl)-1H-imidazol-2-yl)phenyl)ethyl) pyrimidin-4-amine)**

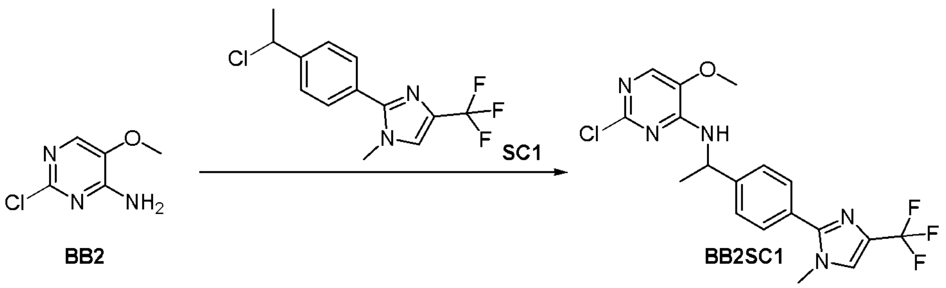


BB2 BB2SC1

Intermediate BB2 (500.0 mg, 3.13 mmol, 1.0 eq), intermediate **SC1** (995.1 mg, 3.45 mol, 1.1 eq) and cesium carbonate (4.08 g, 12.52 mmol, 4.0 eq) were added successively to dimethyl sulfoxide (10.0 mL), stirred for 16 hours at 90°C, and cooled to 25°C. Ethyl acetate (50.0 mL) was added, and the resulting mixture was stirred for 5 minutes, and filtered to obtain a filtrate. The filtrate was washed with saturated brine (50.0 mL) three times, dried over anhydrous sodium sulfate, concentrated under vacuum to obtain a crude product, which was purified by column chromatography (eluent: petroleum ether/ethyl acetate= 1/0 - 0/1) to obtain intermediate **BB2SC1** (1.1 g, 2.68 mmol, 85.6% yield) as a light yellow solid. LC-MS: m/z =412.2 $(M+H)^+$.

**Intermediate BB2C35 (2-chloro-5-methoxy-N-((4-(1-methyl-4-(trifluoromethyl)-1H-imidazol-2-yl)bicyclo[2.2.2]oct-1-yl)methyl)pyrimidin-4-amine)**

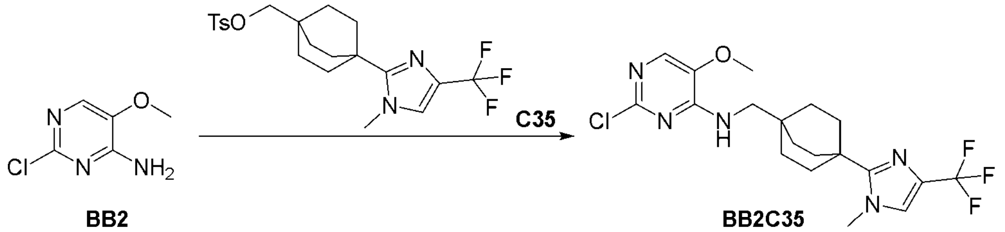


BB2 BB2C35

In accordance with the same steps of intermediate **BB2SC1,** intermediate **BB2C35** (427.7 mg, 0.99 mmol, 31.6% yield) as a white solid was obtained using intermediate **BB2** (500.0 mg, 3.13 mmol, 1.0 eq) and intermediate **C35** (1.53 g, 3.45 mol, 1.1 eq) as starting materials. LC-MS: m/z =430.2 $(M+H)^+$.

**Intermediate BB2SC4 (2-chloro-5-methoxy-N-(4-(1-(2,2,2-trifluoroethyl)-4-(trifluoromethyl)-1H-imidazol-2-yl)benzyl)pyrimidin-4-amine)**

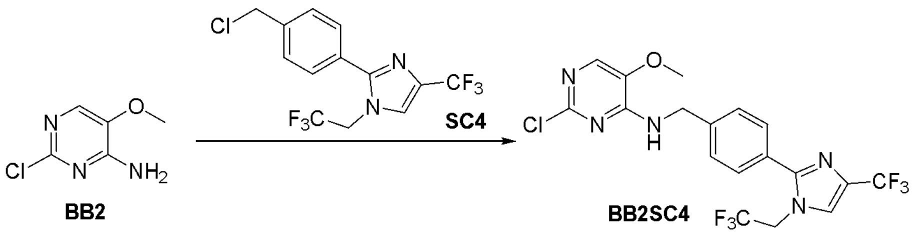


BB2 BB2SC4

In accordance with the same steps of intermediate **BB2SC1,** intermediate **BB2SC4** (1.33 g, 2.87 mmol, 91.2% yield) as a light yellow solid was obtained using intermediate **BB2** (500.0 mg, 3.13 mmol, 1.0 eq) and intermediate **SC4** (1.18 g, 3.45 mol, 1.1 eq) as starting materials. LC-MS: m/z =466.1 $(M+H)^+$.

**Intermediate BB2SC5 (2-chloro-N-(4-(1-(2-fluoroethyl)-4-(trifluoromethyl)-1H-imidazol-2-yl)benzyl)-5-methoxypyrimidin-4-amine)**

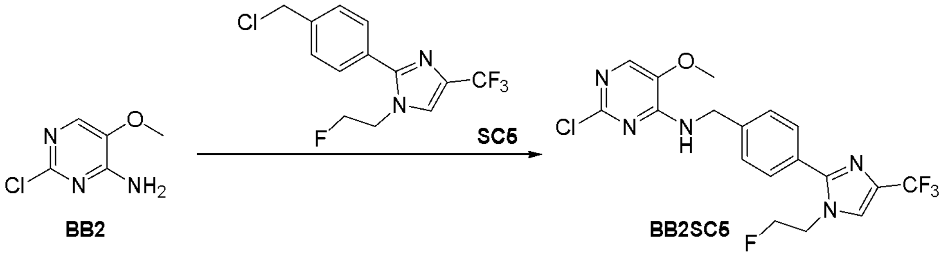


BB2 BB2SC5

In accordance with the same steps of intermediate **BB2SC1,** intermediate **BB2SC5** (181.2 mg, 0.42 mmol, 66.9% yield) as a light yellow solid was obtained using intermediate **BB2** (100.0 mg, 0.63 mmol, 1.0 eq) and intermediate **SC5** (211.4 mg, 0.69 mol, 1.1 eq) as starting materials. LC-MS: m/z =430.1 $(M+H)^+$.

**Intermediate BB2SC7 (2-chloro-N-((1-(1-cyclobutyl-4-(trifluoromethyl)-1H-imidazol-2-yl)-2-oxabicyclo[2.2.2]oct-4-yl)methyl)-5-methoxypyrimidin-4-amine)**

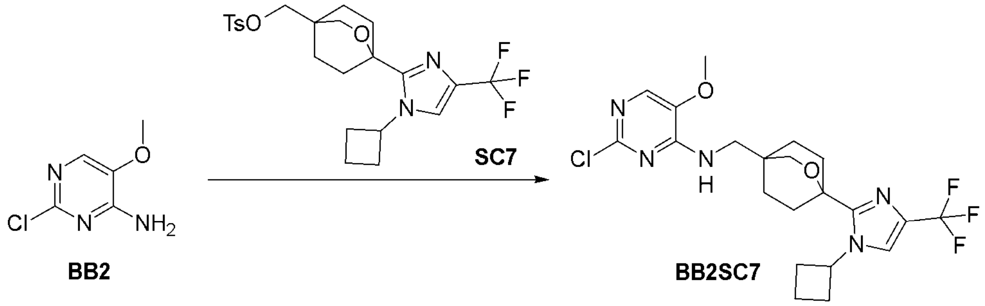


In accordance with the same steps of intermediate **BB2SC1,** intermediate BB2SC7 (195.0 mg, 0.41 mmol, 65.6% yield) as a white solid was obtained using intermediate **BB2** (100.0 mg, 0.63 mmol, 1.0 eq) and intermediate **SC7** (240.7 mg, 0.69 mol, 1.1 eq) as starting materials. LC-MS: m/z =472.2 $(M+H)^+$.

**Intermediate BB2SC8 (2-chloro-N-((1-(1-isopropyl-4-(trifluoromethyl)-1H-imidazol-2-yl)-2-oxabicyclo[2.2.2] oct-4-yl)methyl)-5-methoxypyrimidin-4-amine)**

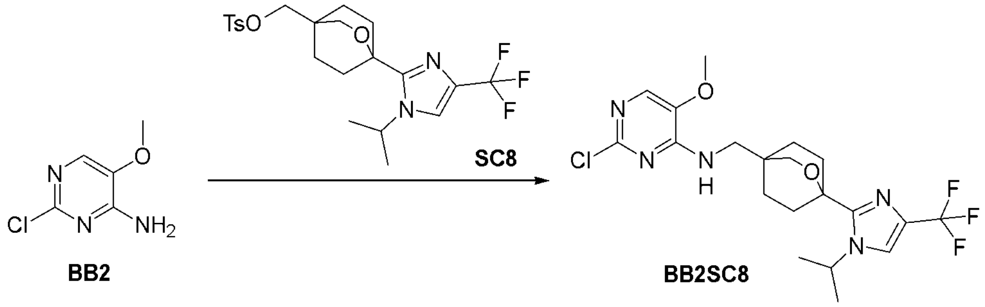


In accordance with the same steps of intermediate **BB2SC1,** intermediate **BB2SC8** (206.8 mg, 0.45 mmol, 71.4% yield) as a white solid was obtained using intermediate **BB2** (100.0 mg, 0.63 mmol, 1.0 eq) and intermediate **SC8** (232.4 mg, 0.69 mol, 1.1 eq) as starting materials. LC-MS: m/z =460.2 $(M+H)^+$.

**Intermediate BB2SC9 (2-chloro-N-((1-(1-cyclopropyl-4-(trifluoromethyl)-1H-imidazol-2-yl)-2-oxabicyclo[2.2.2] oct-4-yl)methyl)-5-methoxypyrimidin-4-amine)**

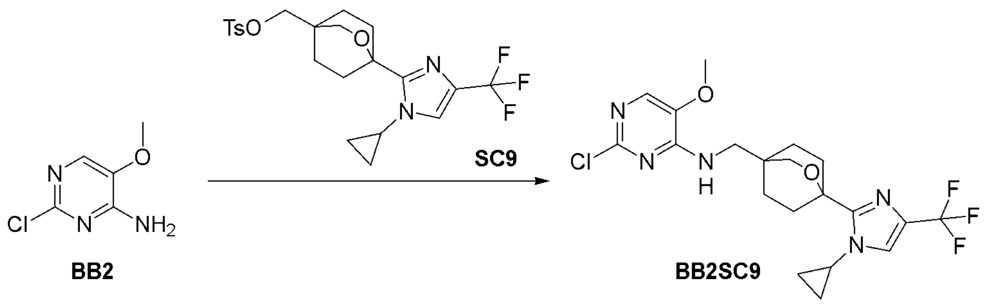


In accordance with the same steps of intermediate **BB2SC1,** intermediate **BB2SC9** (256.4 mg, 0.56 mmol, 88.8% yield) as a white solid was obtained using intermediate **BB2** (100.0 mg, 0.63 mmol, 1.0 eq) and intermediate **SC9** (231.0 mg, 0.69 mol, 1.1 eq) as starting materials. LC-MS: m/z =458.2 $(M+H)^+$.

**Intermediate BB2B12 (2-chloro-5-methoxy-N-((1R,4R)-4-(1-methyl-4-(trifluoromethyl)-1H-imidazol-2-yl)cyclohexyl)methyl)pyrimidin-4-amine)**

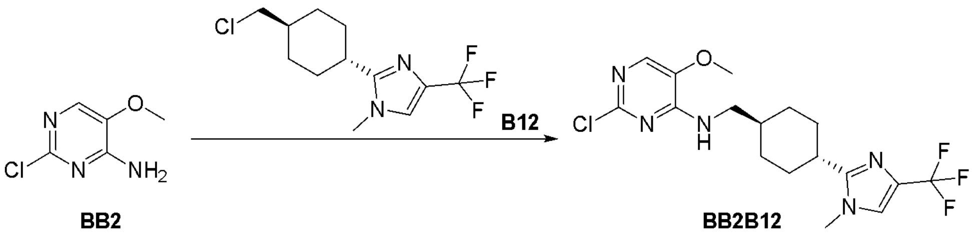


BB2 BB2B12

In accordance with the same steps of intermediate **BB2SC1,** intermediate **BB2B12** (183.2 mg, 0.45 mmol, 72.0% yield) as a colorless transparent oil was obtained using intermediate **BB2** (100.0 mg, 0.63 mmol, 1.0 eq) and intermediate **B12** (193.7 mg, 0.69 mol, 1.1 eq) as starting materials. LC-MS: m/z =404.2 $(M+H)^+$.

**Intermediate BB2SC11 (2-chloro-N-(((1R,4R)-4-(1-cyclopropyl-4-(trifluoromethyl)-1H-imidazol-2-yl)cyclohexyl) methyl)-5-methoxypyrimidin-4-amine)**

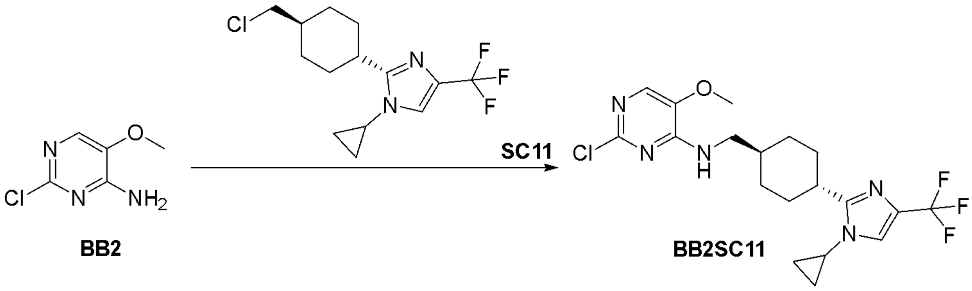


BB2 BB2SC11

In accordance with the same steps of intermediate **BB2SC1,** intermediate **BB2SC11** (104.1 mg, 0.24 mmol, 38.4% yield) as a colorless transparent oil was obtained using intermediate **BB2** (100.0 mg, 0.63 mmol, 1.0 eq) and intermediate **SC11** (211.7 mg, 0.69 mol, 1.1 eq) as starting materials. LC-MS: m/z =430.1 $(M+H)^+$.

**Intermediate BB2SC12 (2-chloro-N-(((1R,4R)-4-(1-isopropyl-4-(trifluoromethyl)-1H-imidazol-2-yl)cyclohexyl) methyl)-5-methoxypyrimidin-4-amine)**

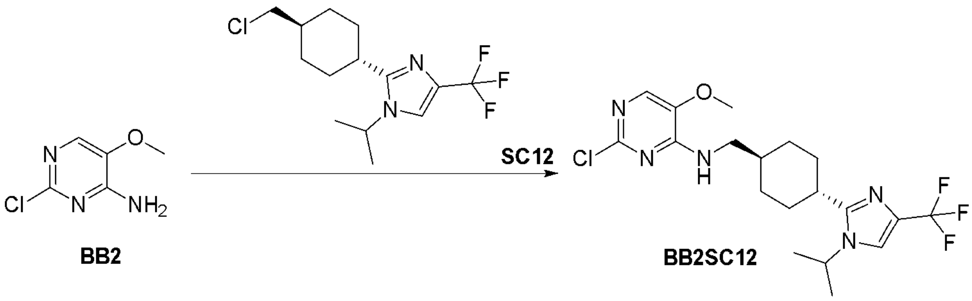


BB2 BB2SC12

In accordance with the same steps of intermediate **BB2SC1,** intermediate **BB2SC12** (151.6 mg, 0.35 mmol, 55.7% yield) as a colorless transparent oil was obtained using intermediate **BB2** (100.0 mg, 0.63 mmol, 1.0 eq) and intermediate **SC12** (213.1 mg, 0.69 mol, 1.1 eq) as starting materials. LC-MS: m/z =432.3 $(M+H)^+$.

**Intermediate BB1SC2 (2-chloro-N-(3-fluoro-4-(1-methyl-4-(trifluoromethyl)-1H-imidazol-2-yl)benzyl)-5-methoxy-N-methylpyrimidin-4-amine)**

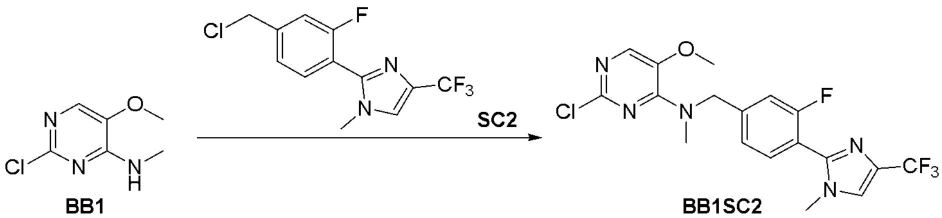


BB1 BB1SC2

In accordance with the same steps of intermediate **BB2SC1,** intermediate **BB1SC2** (55.6 mg, 0.13 mmol, 37.0% yield) as a white solid was obtained using intermediate **BB1** (60.0 mg, 0.35 mmol, 1.0 eq) and intermediate **SC2** (111.3 mg, 0.38 mol, 1.1 eq) as starting materials. LC-MS: m/z =430.2 (M+H)+.

**Intermediate BB1C35 (2-chloro-5-methoxy-N-methyl-N-((4-(1-methyl-4-(trifluoromethyl)-1H-imidazol-2-yl)bicyclo[2.2.2]oct-1-yl)methyl)pyrimidin-4-amine)**

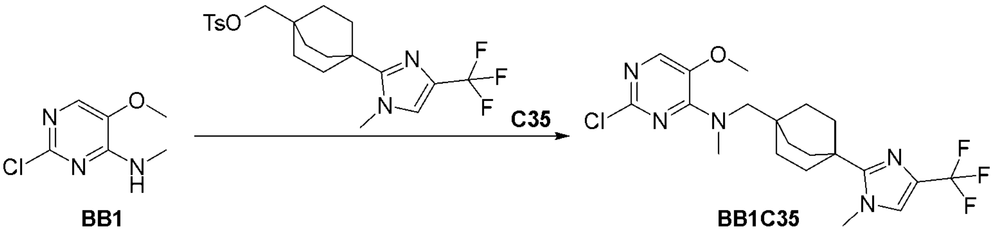


BB1 BB1C35

In accordance with the same steps of intermediate **BB2SC1,** intermediate **BB1C35** (2.4 g, 5.41 mmol, 46.9% yield) as a white solid was obtained using intermediate **BB1** (2.0 g, 11.52 mmol, 1.0 eq) and intermediate **C35** (5.61 g, 12.67 mol, 1.1 eq) as starting materials. LC-MS: m/z =444.2 (M+H)+.

**Intermediate BB1-209-8 (2-chloro-5-methoxy-N-methyl-N-((1-(1-methyl-4-(trifluoromethyl)-1H-imidazol-2-yl)-2-oxabicyclo[2.2.2]oct-4-yl)methyl)pyrimidin-**4-amine)

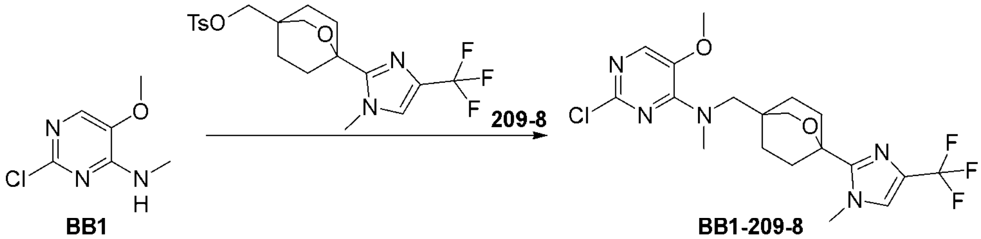


BB1 BB1-209-8

In accordance with the same steps of intermediate **BB2SC1,** intermediate **B2C6** (66.7 mg, 0.15 mmol, 25.8% yield) as a white solid was obtained using intermediate **BB1** (100.0 mg, 0.58 mmol, 1.0 eq) and intermediate 209-8 (194.5 mg, 0.63 mol, 1.1 eq) as starting materials. LC-MS: m/z =446.1 (M+H)+.

**Intermediate BB1SC7 (2-chloro-N-((1-(1-cyclobutyl-4-(trifluoromethyl)-1H-imidazol-2-yl)-2-oxabicyclo[2.2.2] oct-4-yl)methyl)-5-methoxy-N-methylpyrimidin-4-amine)**

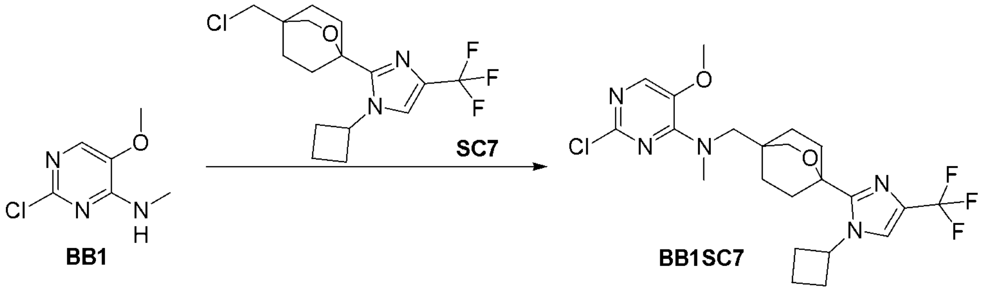


BB1 BB1SC7

In accordance with the same steps of intermediate **BB2SC1,** intermediate **BB1SC7** (68.3 mg, 0.14 mmol, 43.9% yield) as a white solid was obtained using intermediate **BB1** (55.0 mg, 0.32 mmol, 1.0 eq) and intermediate SC7 (121.6 mg, 0.35 mol, 1.1 eq) as starting materials. LC-MS: m/z =486.3 (M+H)$^+$.

**Intermediate BBISC8 (2-chloro-N-((1-(1-isopropyl-4-(trifluoromethyl)-1H-imidazol-2-yl)-2-oxabicyclo[2.2.2]oct-4-yl)methyl)-5-methoxy-N-methylpyrimidin-4-amine)**

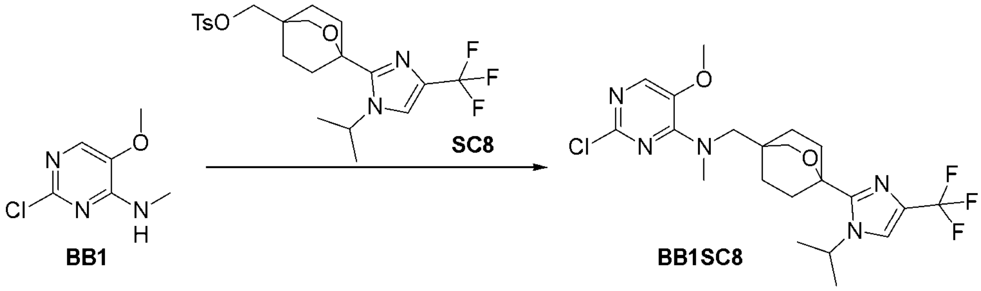


BB1 BB1SC8

In accordance with the same steps of intermediate **BB2SC1,** intermediate **BB1SC8** (110.8 mg, 0.23 mmol, 45.0% yield) as a white solid was obtained using intermediate **BB1** (90.0 mg, 0.52 mmol, 1.0 eq) and intermediate S**C8** (192.1 mg, 0.57 mol, 1.1 eq) as starting materials. LC-MS: m/z =474.0 (M+H)$^+$.

**Intermediate BB1B12 (2-chloro-5-methoxy-N-methyl-N-((1R,4R)-4-(1-methyl-4-(trifluoromethyl)-1H-imidazol-2-yl)cyclohexyl)methyl)pyrimidin-4-amine)**

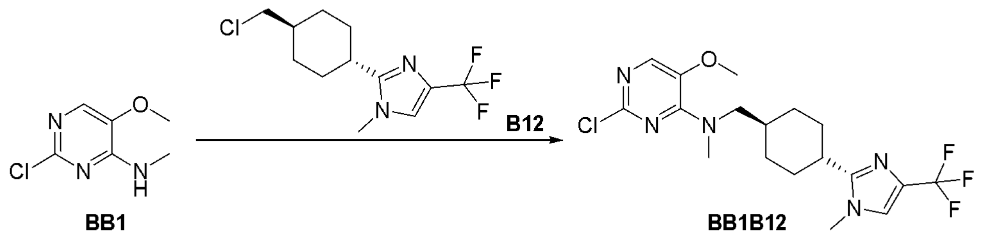


BB1 BB1B12

In accordance with the same steps of intermediate **BB2SC1,** intermediate **BB1B12** (4.5 g, 10.77 mmol, 62.3% yield) as a colorless transparent oil was obtained using intermediate **BB1** (3.0 g, 17.28 mmol, 1.0 eq) and intermediate **B12** (5.34 g, 19.0 mol, 1.1 eq) as starting materials. LC-MS: m/z =418.2 (M+H)$^+$.

**Intermediate BB1SC11 (2-chloro-N-(((1R,4R)-4-(1-cyclopropyl-4-(trifluoromethyl)-1H-imidazol-2-yl)cyclohexyl)methyl)-5-methoxy-N-methylpyrimidin-4-amine)**

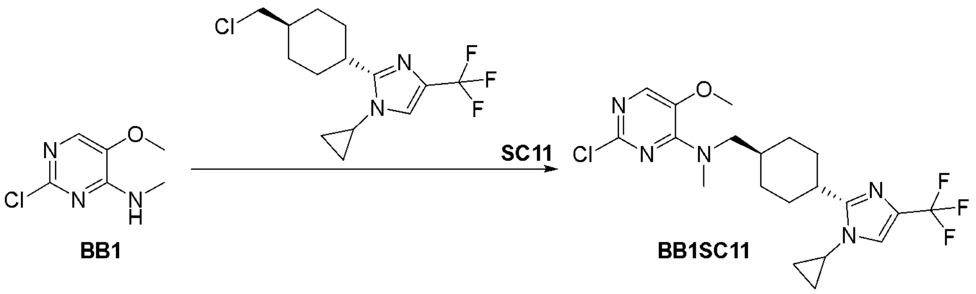


In accordance with the same steps of intermediate **BB2SC1,** intermediate **BB1SC11** (7.4 g, 16.7 mmol, 64.4% yield) as a colorless transparent oil was obtained using intermediate **BB1** (4.5 g, 25.9 mmol, 1.0 eq) and intermediate S**C11** (8.75 g, 28.5 mol, 1.1 eq) as starting materials. LC-MS: m/z =444.4 (M+H)$^+$.

**Intermediate BB1SC12 (2-chloro-N-(((1R,4R)-4-(1-isopropyl-4-(trifluoromethyl)-1H-imidazol-2-yl)cyclohexyl) methyl)-5-methoxy-N-methylpyrimidin-4-amine)**

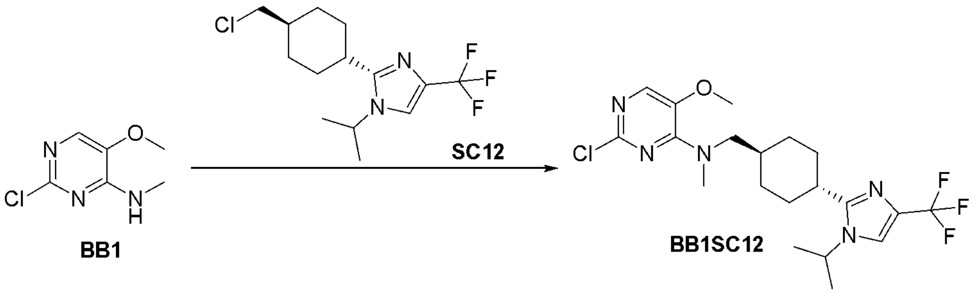


In accordance with the same steps of intermediate **BB2SC1,** intermediate **BB1SC12** (178.6 mg, 0.4 mmol, 46.6% yield) as a colorless transparent oil was obtained using intermediate **BB1** (150 mg, 0.86 mmol, 1.0 eq) and intermediate **SC12** (293.5 mg, 0.95 mol, 1.1 eq) as starting materials. LC-MS: m/z =446.1 (M+H)$^+$.

**Intermediate SB3SC1 (2-chloro-5-methoxy-N-(methyl-D3)-N-(1-(4-(1-methyl-4-(trifluoromethyl)-1H-imidazol-2-yl)phenyl)ethyl)pyrimidin-4-amine)**

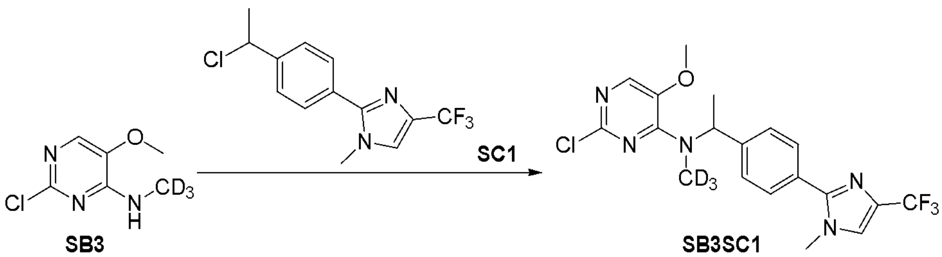


In accordance with the same steps of intermediate **BB2SC1,** intermediate SB**3SC1** (66.7 mg, 0.15 mmol, 97.2% yield) as a light yellow solid was obtained using intermediate S**B3** (28.7 mg, 0.16 mmol, 1.0 eq) and intermediate S**C1** (52.0 mg, 0.18 mol, 1.1 eq) as starting materials. LC-MS: m/z =429.4 (M+H)$^+$.

**Intermediate SB3C35 (2-chloro-5-methoxy-N-(methyl-D3)-N-((4-(1-methyl-4-(trifluoromethyl)-1H-imidazol-2-yl) bicyclo [2.2.2] octan-1-yl)methyl)pyrimidin-4-amine)**

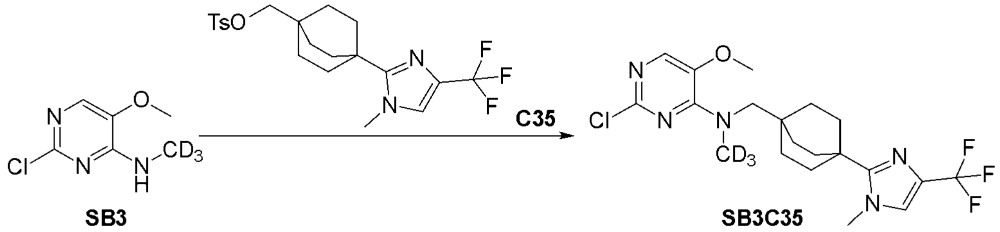


In accordance with the same steps of intermediate **BB2SC1,** intermediate **SB3C35** (679.2 mg, 1.52 mmol, 26.9% yield) as a white solid was obtained using intermediate **SB3** (1.0 g, 5.66 mmol, 1.0 eq) and intermediate **C35** (2.76 g, 6.23 mol, 1.1 eq) as starting materials. LC-MS: m/z =447.3 $(M+H)^+$.

**Intermediate SB3B12 (2-chloro-5-methoxy-N-(methyl-D3)-N-(((1R,4R)-4-(1-methyl-4-(trifluoromethyl)-1H-imidazol-2-yl)cyclohexyl)methyl)pyrimidin-4-amine)**

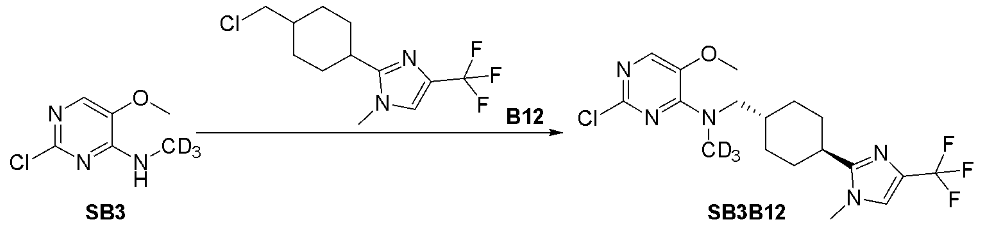


In accordance with the same steps of intermediate **BB2SC1,** intermediate **SB3B12** (394.6 mg, 0.94 mmol, 63.8% yield) as a colorless transparent oil was obtained using intermediate S**B3** (260.0 mg, 1.47 mmol, 1.0 eq) and intermediate **B12** (454.6 mg, 1.62 mol, 1.1 eq) as starting materials. LC-MS: m/z =421.1 $(M+H)^+$.

**Intermediate SB3SC11 (2-chloro-N-(((1R,4R)-4-(1-cyclopropyl-4-(trifluoromethyl)-1H-imidazol-2-yl)cyclohexyl)methyl)-5-methoxy-N-(methyl-D3)pyrimidin-4-amine)**

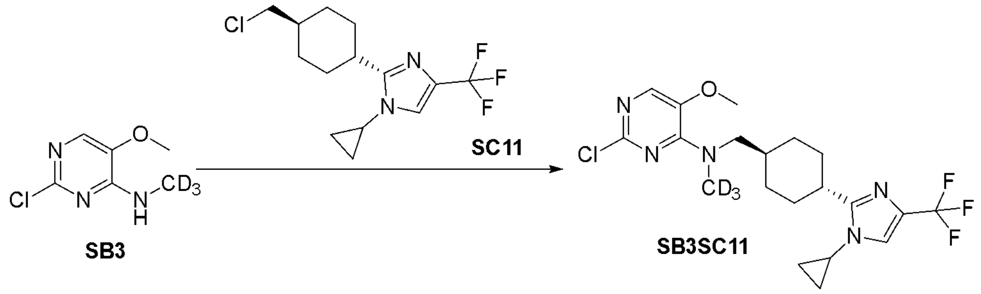


In accordance with the same steps of intermediate **BB2SC1,** intermediate **SB3SC11** (48.7 mg, 0.11 mmol, 25.9% yield) as a light yellow oil was obtained using intermediate S**B3** (75.0 mg, 0.42 mmol, 1.0 eq) and intermediate **SC11** (143.3 mg, 0.47 mol, 1.1 eq) as starting materials. LC-MS: m/z =447.2 $(M+H)^+$.

**Example S1: Synthesis of compound S1 (4'-cyclopropyl-5,6'-dimethoxy-N-(1-(1-methyl-4-(trifluoromethyl)-1H-imidazol-2-yl)phenyl)ethyl)-[2,5'-bipyrimidin]-4-**amine)

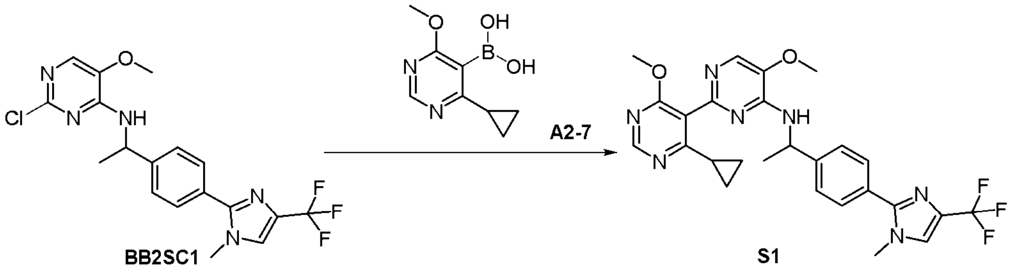


BB2SC1 S1

Compound **BB2SC1** (75.0 mg, 0.20 mmol, 1.0 eq), compound A2-7 (77.1 mg, 0.40 mmol, 2.0 eq), chloro(2-dicyclohexylphosphino-2',4',6'-triisopropyl-1,1'-biphenyl)[2-(2'-amino-1,1'-biphenyl)]palladium(II) (XPhos-Pd-G2, 31.4 mg, 0.04 mmol, 0.2 eq), 2-dicyclohexylphosphino-2',4',6'-triisopropylbiphenyl (XPhos, 76.3 mg, 0.16 mmol, 0.4 eq) and potassium phosphate (169.8 mg, 0.80 mmol, 4.0 eq) were added successively to dioxane (1.5 mL) and water (0.3 mL), purged with nitrogen three times, and stirred for 16 hours at 95°C under nitrogen atmosphere. Water (10.0 mL) was added, and the resulting mixture was extracted with ethyl acetate (20.0 mL) three times. The organic layers were combined, washed with saturated brine (20.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound S**1** (47.5 mg, 0.09 mmol, 45.0% yield) as a white solid. $^1H$ NMR (400 MHz, DMSO-$d_6$) δ 8.74 (s, 1H), 8.38 (d, *J* = 6.9 Hz, 2H), 7.93 (d, *J* = 7.2 Hz, 2H), 7.76 (s, 1H), 7.36 (s, 1H), 6.83 (s, 1H), 4.01-3.97 (m, 1H), 3.90 (s, 3H), 3.81 (s, 3H), 3.36 (s, 3H), 1.80-1.70 (m, 1H), 1.26-0.98 (m, 5H), 0.80-0.73 (m, 2H); LC-MS: m/z = 526.3 $(M+H)^+$.

The following compounds were prepared in accordance with the preparation method of compound S**1**, using above-mentioned intermediates as starting materials, XPhos-Pd-G2/XPhos/$K_3PO_4$ as reaction reagents, and dioxane and water as solvents.

| Example | Structure and name of compound | Reaction moiety | Analyzed data |
|---|---|---|---|
| **S2** | 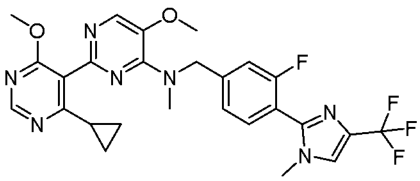 4'-Cyclopropyl-N-(3-fluoro-4-(1-methyl-4-(trifluoromethyl)-1H-imidazol-2-yl)benzyl)-5,6'-dimethoxy-N-methyl-[2,5'-bipyrimidin]-4-amine | **A2-7 BB1SC2** | LC-MS: m/z =544.4 $(M+H)^+$; $^1H$ NMR (400 MHz, DMSO-$d_6$) δ 8.71 (s, 1H), 8.23 (d, *J* = 7.4 Hz, 1H), 7.99 (s, 1H), 7.74 (d, *J*= 8.1 Hz, 1H), 7.64 (s, 1H), 7.46 (s, 1H), 4.91 (s, 2H), 3.94 (s, 3H), 3.88 (s, 3H), 3.78 (s, 3H), 3.36 (s, 3H), 1.80-1.73 (m, 1H), 1.06-0.98 (m, 2H), 0.89-0.78 (m, 2H). |
| **S3** | 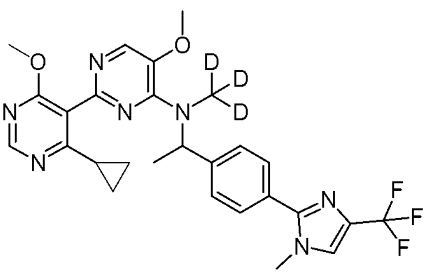 4'-Cyclopropyl-5,6'-dimethoxy-N-(methyl-D3)-N-(1-(4-(1-methyl-4-(trifluoromethyl)- 1 H-imidazol-2-yl)phenyl) ethyl)-[2,5'-bipyrimidin]-4-amine | **A2-7 SB3SC1** | LC-MS: m/z =543.3 $(M+H)^+$ ; $^1H$ NMR (400 MHz, DMSO-$d_6$) δ 8.77 (s, 1H), 8.39 (d, J = 6.8 Hz, 2H), 7.90 (d, J = 7.2 Hz, 2H), 7.78 (s, 1H), 7.38 (s, 1H), 4.08-3.97 (m, 1H), 3.95 (s, 3H), 3.88 (s, 3H), 3.33 (s, 3H), 1.81-1.73 (m, 1H), 1.26-1.01 (m, 5H), 0.82-0.76 (m, 2H). |

(continued)

| Example | Structure and name of compound | Reaction moiety | Analyzed data |
|---|---|---|---|
| S4 | 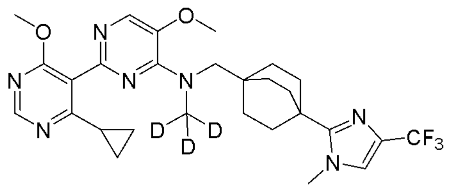 4'-Cyclopropyl-5,6'-dimethoxy-N-(methyl-d3)-N-((4-(1-methyl-4-(trifluoromethyl)-1H-imidazol-2-yl)bicyclo[2.2.2]oct-1-yl)methyl)-[2,5'-bipyrimidin]-4-amine | A2-7 SB3C35 | LC-MS: m/z =561.3 $(M+H)^+$ ; $^1H$ NMR (400 MHz, DMSO-$d_6$) δ 8.61 (s, 1H), 7.92 (s, 1H), 7.67 (s, 1H), 3.95 (s, 3H), 3.88 (s, 3H), 3.80 (s, 3H), 3.21 (s, 2H), 1.95-1.87 (m, 6H), 1.76-1.70 (m, 1H), 1.51-1.47 (m, 6H), 1.08-0.97 (m, 2H), 0.89- 0.78 (m, 2H). |
| S5 | 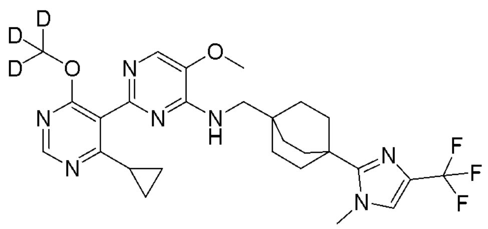 4'-Cyclopropyl-5-methoxy-6'-(methoxy-D3)-N-((4-(1-methyl-4-(trifluoromethyl)-1H-imidazol-2-yl)bicyclo[2.2.2]oct-1-yl)methyl)-[2,5'-bipyrimidin]-4-amine | SA2 BB2C35 | LC-MS: m/z =547.2 $(M+H)^+$ ; $^1H$ NMR (400 MHz, DMSO-$d_6$) δ 8.59 (s, 1H), 7.81 (s, 1H), 7.59 (s, 1H), 6.88 (t, *J* = 6.6 Hz, 1H), 3.78 (s, 3H), 3.69 (s, 3H), 3.19 (d, *J* = 7.2 Hz, 2H), 1.99-1.87 (m, 6H), 1.75-1.68 (m, 1H), 1.53-1.37 (m, 6H), 0.98-0.90 (m, 2H), 0.79-0.75 (m, 2H). |
| S6 | 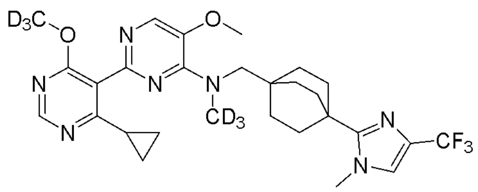 4'-Cyclopropyl-5-methoxy-6'-(methoxy-D3)-N-(methyl-D3)-N-((4-(1-methyl-4-(trifluoromethyl)- 1 H-imidazol-2-yl)bicyclo[2.2.2]oct-1-yl)methyl)-[2,5'-bipyrimidin]-4-amine | SA2 SB3C35 | LC-MS: m/z =564.3 $(M+H)^+$; $^1H$ NMR (400 MHz, DMSO-$d_6$) δ 8.64 (s, 1H), 8.02 (s, 1H), 7.76 (s, 1H), 3.88 (s, 3H), 3.76 (s, 3H), 3.27 (s, 2H), 1.96-1.89 (m, 6H), 1.75-1.69 (m, 1H), 1.53-1.48 (m, 6H), 1.10-0.98 (m, 2H), 0.93-0.81 (m, 2H). |
| S7 | 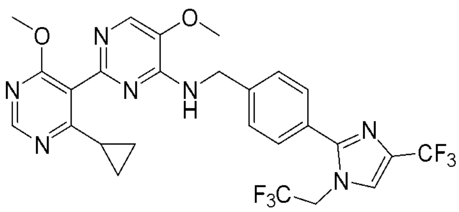 4'-Cyclopropyl-56'-dimethoxy-N-(4-(1-(2,2,2-trifluoroethyl)-4-(trifluoromethyl)-1H-imidazol-2-yl)benzyl)-[2,5'-bipyrimidin]-4-amine | A2-7 BB2SC4 | LC-MS: m/z =580.2 $(M+H)^+$; $^1H$ NMR (400 MHz, $CDCl_3$) δ 8.67 (s, 1H), 7.96 (s, 1H), 7.69 (d, *J* = 7.8 Hz, 2H), 7.54 (s, 1H), 7.50 (d, *J* = 8.0 Hz, 2H), 5.95 (s, 1H), 4.88 (s, 2H), 4.70 (s, 2H), 3.98 (s, 3H), 3.92 (s, 3H), 1.83-1.75 (m, 1H), 1.18-1.09 (m, 2H), 0.95-0.84 (m, 2H). |

(continued)

| Example | Structure and name of compound | Reaction moiety | Analyzed data |
|---|---|---|---|
| S8 | 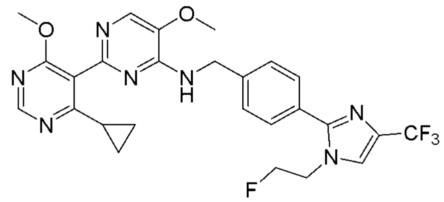 4'-Cyclopropyl-N-(4-(1-(2-fluoroethyl)-4-(trifluoromethyl)-1H-imidazol-2-yl)benzyl)-5,6'-dimethoxy-[2,5'-bipyrimidin]-4-amine | A2-7 BB2SC5 | LC-MS: m/z =544.1 $(M+H)^+$; $^1H$ NMR (400 MHz, $CDCl_3$) δ 8.62 (s, 1H), 7.95 (s, 1H), 7.71 (d, *J= 7.8* Hz, 2H), 7.53 (s, 1H), 7.49 (d, *J* = 8.2 Hz, 2H), 5.94 (s, 1H), 5.77-5.62 (m, 2H), 4.79 (d, J=7.0 Hz, 2H), 4.01-3.88 (m, 8H), 1.85-1.71 (m, 1H), 1.17-1.09 (m, 2H), 0.88 - 0.78 (m, 2H). |
| S9 | 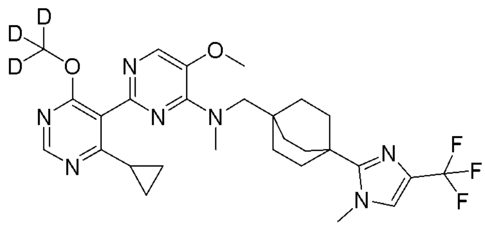 4'-Cyclopropyl-5-methoxy-6'-(methoxy-D3)-N-methyl-N-((4-(1-methyl-4-(trifluoromethyl)-1H-imidazol-2-yl)bicyclo [2.2.2]oct-1-yl)methyl)-[2,5'-bipyrimidin]-4-amine | SA2 BB1C35 | LC-MS: m/z =561.3 $(M+H)^+$; $^1H$ NMR (400 MHz, DMSO-$d_6$) δ 8.69 (s, 1H), 7.93 (s, 1H), 7.70 (s, 1H), 3.88 (s, 3H), 3.73 (s, 3H), 3.47 (s, 3H), 3.18 (s, 2H), 1.96-1.87 (m, 6H), 1.76 (m, 1H), 1.52-1.45 (m, 6H), 1.08-0.99 (m, 2H), 0.95-0.88 (m, 2H). |
| S10 | 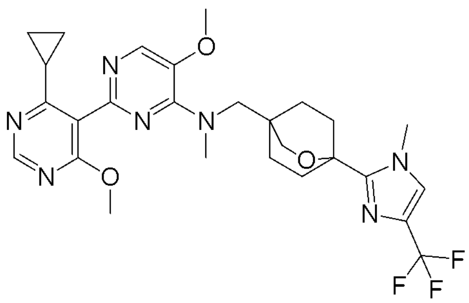 4'-Cyclopropyl-5,6'-dimethoxy-N-methyl-N-((1-(1-methyl-4-(trifluoromethyl)-1H-imidazol-2-yl)-2-oxabicyclo [2.2.2]oct-4-yl)methyl)-[2,5'-bipyrimidin]-4-amine | A2-7 BB1-209-8 | LC-MS: m/z =560.5 $(M+H)^+$; $^1H$ NMR (400 MHz, DMSO-$d_6$) δ 8.62 (s, 1H), 7.98 (s, 1H), 7.90 (s, 1H), 3.96 (s, 3H), 3.85 (s, 3H), 3.74 (s, 2H), 3.71 (s, 3H), 3.23-2.99 (m, 5H), 2.15-2.11 (m, 2H), 2.08-1.88 (m, 2H), 1.76-1.69 (m, 3H), 1.64-1.56 (m, 2H), 1.12-0.98 (m, 2H), 0.93-0.78 (m, 2H). |
| S11 | 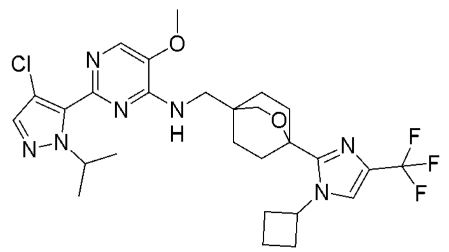 2-(4-Chloro-1-isopropyl-1H-pyrazol-5-yl)-N-((1-(1-cyclobutyl-4-(trifluoromethyl)-1H-imidazol-2-yl)-2-oxabicyclo[2.2.2] oct-4-yl)methyl)-5-methoxypyrimidin-4-amine | AA9 BB2SC7 | LC-MS: m/z =580.3 $(M+H)^+$; |

(continued)

| Example | Structure and name of compound | Reaction moiety | Analyzed data |
|---|---|---|---|
| **S12** | 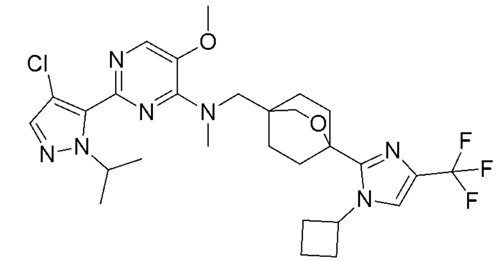 2-(4-Chloro-1-isopropyl-1H-pyrazol-5-yl)-N-((1-(1-cyclobutyl-4-(trifluoromethyl)-1H-imidazol-2-yl)-2-oxabicyclo[2.2.2]oct-4-yl)methyl)-5-methoxy-N-methylpyrimidin-4-amine | **AA9 BB1SC7** | LC-MS: m/z =594.2 $(M+H)^+$; |
| **S13** | 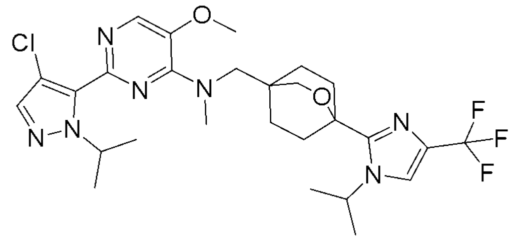 2-(4-Chloro-1-isopropyl-1H-pyrazol-5-yl)-N-((1-(1-isopropyl-4-(trifluoromethyl)-1H-imidazol-2-yl)-2-oxabicyclo[2.2.2]oct-4-yl)methyl)-5-methoxy-N-methylpyrimidin-4-amine | **AA9 BB1SC8** | LC-MS: m/z =582.4 $(M+H)^+$; $^1H$ NMR (400 MHz, DMSO-$d_6$) δ 8.15 (s, 1H), 8.00 (s, 1H), 7.64 (s, 1H), 5.37-5.18 (m, 1H), 5.13-5.05 (m, 1H), 3.92 (s, 3H), 3.80 (s, 2H), 3.76 (s, 2H), 3.26 (s, 3H), 2.17 (s, 2H), 2.08-1.96 (m, 2H), 1.71-.63 (m, 4H), 1.40 (d, *J* = 6.6 Hz, 6H), 1.35 (d, *J*= 6.6 Hz, 6H). |
| **S14** | 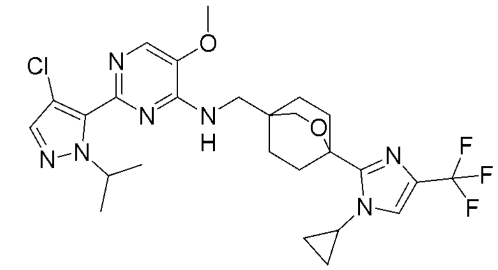 2-(4-Chloro-1-isopropyl-1H-pyrazol-5-yl)-N-((1-(1-cyclopropyl-4-(trifluoromethyl)-1H-imidazol-2-yl)-2-oxabicyclo[2.2.2]oct-4-yl)methyl)-5-methoxypyrimidin-4-amine | **AA9 BB2SC9** | LC-MS: m/z =566.2 $(M+H)^+$; |
| **S15** | 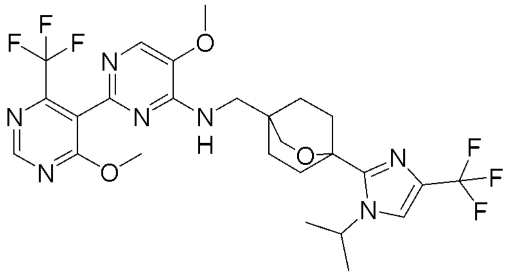 N-((1-(1-Isopropyl-4-(trifluoromethyl)-1H-imidazol-2-yl)-2-oxabicyclo[2.2.2]oct-4-yl)methyl)-4',5-dimethoxy-6'-(trifluoromethyl)-[2,5'-bipyrimidin]-4-amine | **SA4 BB2SC8** | LC-MS: m/z =602.4 $(M+H)^+$; $^1H$ NMR (400 MHz, DMSO-$d_6$) δ 9.00 (s, 1H), 7.98 (s, 1H), 7.88 (s, 1H), 7.20 (t, *J*= 6.9 Hz, 1H), 5.18-5.00 (m, 1H), 3.96 (s, 3H), 3.89 (s, 3H), 3.73 (s, 2H), 3.21 (d, *J* = 6.6 Hz, 2H), 2.15-2.11 (m, 2H), 2.06-1.87 (m, 2H), 1.73-1.69 (m, 2H), 1.63-1.55 (m, 2H), 1.39 (d, *J* = 6.6 Hz, 6H); |

(continued)

| Example | Structure and name of compound | Reaction moiety | Analyzed data |
|---|---|---|---|
| **S16** | 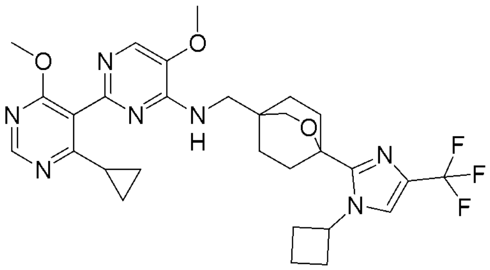<br>N-((1-(1-Cyclobutyl-4-(trifluoromethyl)-1H-imidazol-2-yl)-2-oxabicyclo[2.2.2]oct-4-yl)methyl)-4'-cyclopropyl-5,6'-dimethoxy-[2,5'-bipyrimidin]-4-amine | **A2-7**<br>**BB2SC7** | LC-MS: m/z =586.2 $(M+H)^+$; |
| **S17** | 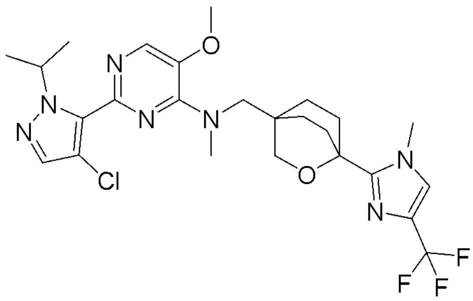<br>2-(4-Chloro-1-isopropyl-1H-pyrazol-5-yl)-5-methoxy-N-methyl-N-((1-(1-methyl-4-(trifluoromethyl)-1H-imidazol-2-yl)-2-oxabicyclo[2.2.2]oct-4-yl)methyl)pyrimidin-4-amine | **AA9**<br>**BB1-209-8** | LC-MS: m/z =554.4 $(M+H)^+$;<br>$^1$H NMR (400 MHz, DMSO-$d_6$) $\delta$ 8.16 (s, 1H), 8.00 (s, 1H), 7.65 (s, 1H), 5.06-4.99 (m, 1H), 3.94 (s, 3H), 3.88 (s, 3H), 3.81 (s, 2H), 3.74 (s, 2H), 3.26 (s, 3H), 2.13-1.88 (m, 4H), 1.75-1.53 (m, 4H), 1.35 (d, *J* = 6.7 Hz, 6H). |
| **S18** | 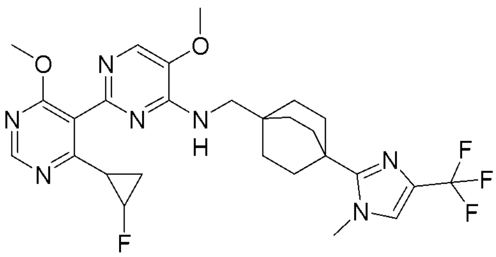<br>4'-(2-Fluorocyclopropyl)-5,6'-dimethoxy-N-((4-(1-methyl-4-(trifluoromethyl)- 1 H-imidazol-2-yl)bicyclo[2.2.2]oct-1-yl) methyl)-[2,5'-bipyrimidin]-4-amine | **SA5**<br>**BB2C35** | LC-MS: m/z =562.3 $(M+H)^+$;<br>$^1$H NMR (400 MHz, DMSO-$d_6$) $\delta$ 8.79 (s, 1H), 7.96 (s, 1H), 7.72 (s, 1H), 7.00-6.93 (m, 1H), 6.18-6.00 (m, 1H), 3.98 (s, 3H), 3.86 (s, 3H), 3.78 (s, 3H), 3.23-3.11 (m, 3H), 1.98-1.83 (m, 6H), 1.53-1.20 (m, 8H). |
| **S19** | 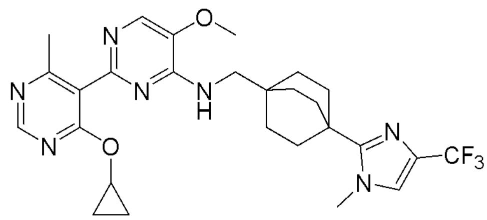<br>4'-Cyclopropoxy-5-methoxy-6'-methyl-N-(4-(1-methyl-4-(trifluoromethyl)-1H-imidazol-2-yl)bicyclo[2.2.2]oct-1-yl) methyl)-[2,5'-bipyrimidin]-4-amine | **SA6**<br>**BB2C35** | LC-MS: m/z =544.3 $(M+H)^+$;<br>$^1$H NMR (400 MHz, DMSO-$d_6$) $\delta$ 8.65 (s, 1H), 7.93 (s, 1H), 7.66 (s, 1H), 7.01 (t, *J* = 6.5 Hz, 1H), 3.86 (s, 3H), 3.79 (s, 3H), 3.46 (m, 1H), 3.21 (d, *J* = 6.8 Hz, 2H), 3.16 (s, 3H), 1.95-1.82 (m, 6H), 1.56-1.43 (m, 6H), 0.89- 0.67 (m, 4H). |

(continued)

| Example | Structure and name of compound | Reaction moiety | Analyzed data |
|---|---|---|---|
| **S20** | 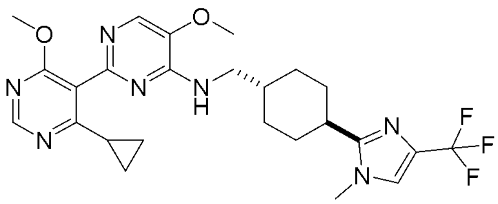 4'-Cyclopropyl-5,6'-dimethoxy-N-((1R,4R)-4-(1-methyl-4-(trifluoromethyl)-1H-imidazol-2-yl)cyclohexyl)methyl)-[2,5'-bipyrimidin]-4-amine | **A2-7 BB2B12** | LC-MS: m/z =518.4 $(M+H)^+$; $^1$H NMR (400 MHz, DMSO-$d_6$) δ 8.63 (s, 1H), 8.08 (s, 1H), 7.65 (s, 1H), 7.08 (t, *J* = 6.1 Hz, 1H), 3.93 (s, 3H), 3.86 (s, 3H), 3.63 (s, 3H), 3.56 (d, *J* = 6.4 Hz, 2H), 2.76-2.61 (m, 1H), 1.88-1.82 (m, 3H), 1.79-1.65 (m, 3H), 1.57-1.36 (m, 2H), 1.16-1.08 (m, 2H), 1.10-0.96 (m, 2H), 0.92-0.83 (m, 2H). |
| **S21** | 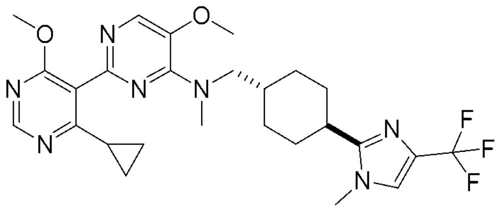 4'-Cyclopropyl-5,6'-dimethoxy-N-methyl-N-((1R,4R)-4-(1-methyl-4-(trifluoromethyl)-1H-imidazol-2-yl)cyclohexyl) methyl)-[2,5'-bipyrimidin]-4-amine | **A2-7 BB1B12** | LC-MS: m/z =532.3 $(M+H)^+$; $^1$H NMR (400 MHz, DMSO-$d_6$) δ 8.60 (s, 1H), 8.03 (s, 1H), 7.62 (s, 1H), 3.88 (s, 3H), 3.83 (s, 3H), 3.62 (s, 3H), 3.57 (d, *J*= 7.1 Hz, 2H), 3.14 (s, 3H), 2.77 - 2.63 (m, 1H), 1.85 (d, *J* = 9.3 Hz, 3H), 1.77 - 1.64 (m, 3H), 1.55 - 1.36 (m, 2H), 1.14-0.97 (m, 4H), 0.91-0.85 (m, 2H). |
| **S22** | 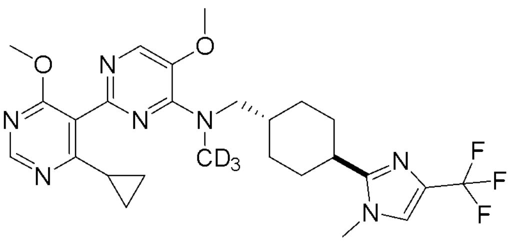 4'-Cyclopropyl-5,6'-dimethoxy-N-(methyl-d3)-N-(((1r,4r)-4-(1-methyl-4-(trifluoromethyl)-1H-imidazol-2-yl)cyclohexyl) methyl)-[2,5'-bipyrimidin]-4-amine | **A2-7 SB3B12** | LC-MS: m/z =535.4 $(M+H)^+$; $^1$H NMR (400 MHz, DMSO-$d_6$) δ 8.62 (s, 1H), 8.05 (s, 1H), 7.66 (s, 1H), 3.90 (s, 3H), 3.85 (s, 3H), 3.63 (s, 3H), 3.58 (d, *J* = 7.0 Hz, 2H), 2.80-2.64 (m, 1H), 1.88-1.79 (m, 3H), 1.79-1.67 (m, 3H), 1.59-1.39 (m, 2H), 1.23-1.08 (m, 2H), 1.06-0.99 (m, 2H), 0.90-0.86 (m, 2H). |

(continued)

| Example | Structure and name of compound | Reaction moiety | Analyzed data |
|---|---|---|---|
| **S23** | 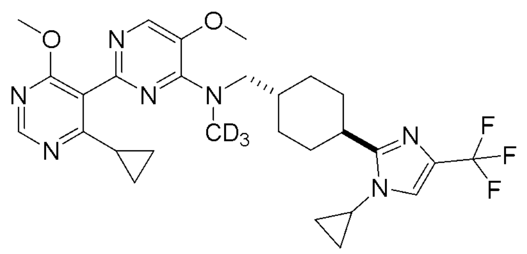<br>4'-Cyclopropyl-N-(((1R,4R)-4-(1-cyclopropyl-4-(trifluoromethyl)-1H-imidazol-2-yl)cyclohexyl)methyl)-5,6'-dimethoxy-N-(methyl-D3)-[2,5'-bipyrimidin]-4-amine | **A2-7 SB3SC11** | LC-MS: m/z =561.4 $(M+H)^+$; |
| **S24** | 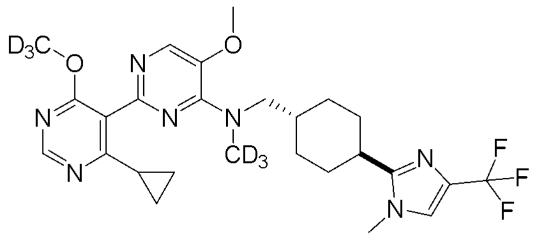<br>4'-Cyclopropyl-5-methoxy-6'-(methoxy-D3)-N-(methyl-D3)-N(((1R,4R)-4-(1-methyl-4-(trifluoromethyl)-1H-imidazol-2-yl)cyclohexyl)methyl)-[2,5'-bipyrimidin]-4-amine | **SA2 SB3B12** | LC-MS: m/z =538.3 $(M+H)^+$; $^1H$ NMR (400 MHz, DMSO-$d_6$) δ 8.63 (s, 1H), 8.06 (s, 1H), 7.66 (s, 1H), 3.91 (s, 3H), 3.65 (s, 3H), 3.59 (d, *J*= 7.1 Hz, 2H), 2.74-2.69 (m, 1H), 1.89-1.85 (m, 3H), 1.78-1.70 (m, 3H), 1.52-1.41 (m, 2H), 1.16-1.08 (m, 2H), 1.07-0.99 (m, 2H), 0.95-0.88 (m, 2H). |
| **S25** | 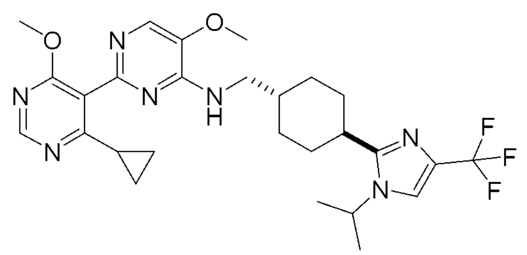<br>4'-Cyclopropyl-N-((1R,4R)-4-(1-isopropyl-4-(trifluoromethyl)-1H-imidazol-2-yl)cyclohexyl)methyl)-5,6'-dimethoxy-[2,5'-bipyrimidin]-4-amine | **A2-7 BB2SC12** | LC-MS: m/z =546.2 $(M+H)^+$; |
| **S26** | 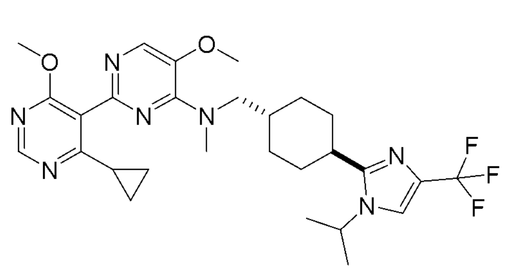<br>4'-Cyclopropyl-N-(((1R,4R)-4-(1-isopropyl-4-(trifluoromethyl)-1H-imidazol-2-yl)cyclohexyl)methyl)-5,6'-dimethoxy-N-methyl-[2,5'-bipyrimidin]-4-amine | **A2-7 BB1SC12** | LC-MS: m/z =560.4 $(M+H)^+$; $^1H$ NMR (400 MHz, DMSO-$d_6$) δ 8.61 (s, 1H), 7.98 (s, 1H), 7.59 (s, 1H), 5.26-5.13 (m, 1H), 3.86 (s, 3H), 3.82 (s, 3H), 3.57 (d, *J* = 7.4 Hz, 2H), 3.14 (s, 3H), 2.76-2.60 (m, 1H), 1.89-1.81 (m, 3H), 1.79-1.68 (m, 3H), 1.51-1.30 (m, 8H), 1.18-1.03(m, 4H), 0.99-0.85 (m, 2H). |

(continued)

| Example | Structure and name of compound | Reaction moiety | Analyzed data |
|---|---|---|---|
| **S27** | 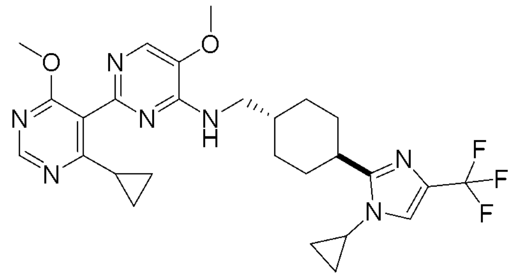 4'-Cyclopropyl-N-(((1R,4R)-4-(1-cyclopropyl-4-(trifluoromethyl)-1H-imidazol-2-yl)cyclohexyl)methyl)-5,6'-dimethoxy-[2,5'-bipyrimidin]-4-amine | **A2-7 BB2SC11** | LC-MS: m/z =544.3 $(M+H)^+$; |
| **S28** | 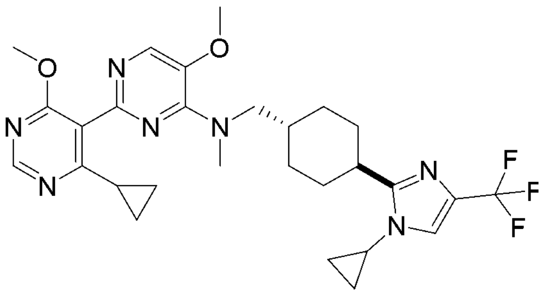 4'-Cyclopropyl-N-(((1R,4R)-4-(1-cyclopropyl-4-(trifluoromethyl)-1H-imidazol-2-yl)cyclohexyl)methyl)-5,6'-dimethoxy-N-methyl-[2,5'-bipyrimidin]-4-amine | **A2-7 BB1SC11** | LC-MS: m/z =558.4 $(M+H)^+$ |
| **S29** | 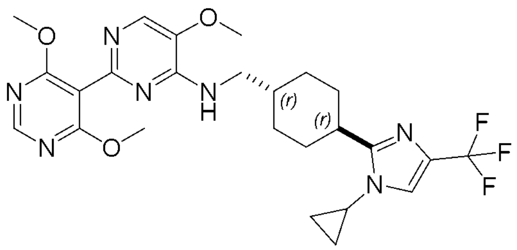 N-(((1R,4R)-4-(1-cyclopropyl-4-(trifluoromethyl)-1H-imidazol-2-yl)cyclohexyl)methyl)-4',5,6'-trimethoxy-[2,5'-bipyrimidin]-4-amine | **SA7 BB2SC11** | LC-MS: m/z =534.3 $(M+H)^+$ ; |
| **S30** | N-methyl-[2,5'-bipyrimidin]-4-amine 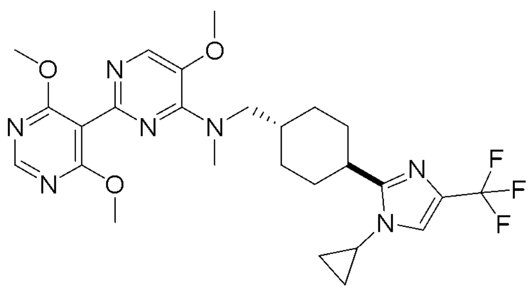 N-(((1R,4R)-4-(1-cyclopropyl-4-(trifluoromethyl)-1H-imidazol-2-yl)cyclohexyl)methyl)-4',5,6'-trimethoxy-N-methyl-[2,5'-bipyrimidin]-4-amine | **SA7 BB1SC11** | LC-MS: m/z =548.2 $(M+H)^+$ ; |

(continued)

| Example | Structure and name of compound | Reaction moiety | Analyzed data |
|---|---|---|---|
| S31 | 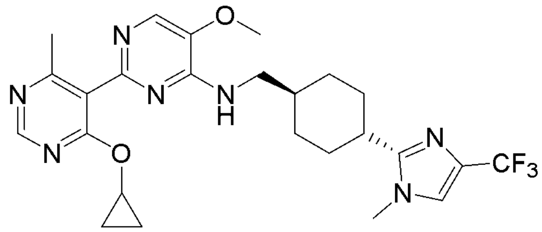 4'-Cyclopropoxy-5-methoxy-6'-methyl-N-((1R,4R)-4-(1-methyl-4-(trifluoromethyl)-1H-imidazol-2-yl)cyclohexyl)methyl)-[2,5'-bipyrimidin]-4-amine | SA6 BB2B12 | LC-MS: m/z =518.3 $(M+H)^+$ ; |
| S32 | 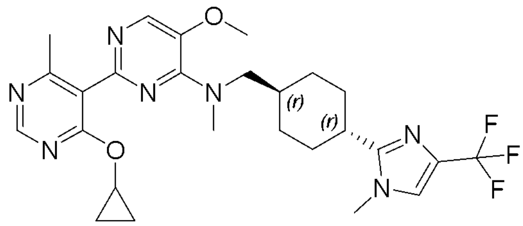 4'-Cyclopropoxy-5-methoxy-N,6'-dimethyl-N-((1R,4R)-4-(1-methyl-4-(trifluoromethyl)-1H-imidazol-2-yl)cyclohexyl)methyl)-[2,5'-bipyrimidin]-4-amine | SA6 BB1B12 | LC-MS: m/z =532.4 $(M+H)^+$ ; |
| S33 | 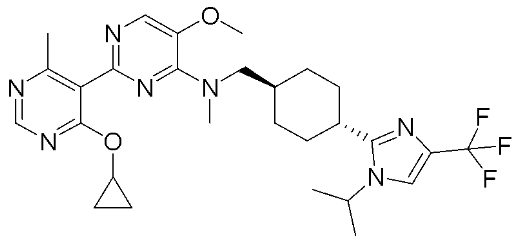 4'-Cyclopropoxy-N-((1R,4R)-4-(1-isopropyl-4-(trifluoromethyl)-1H-imidazol-2-yl)cyclohexyl)methyl)-5-methoxy-N, 6'-dimethyl-[2,5'-bipyrimidin]-4-amine | SA6 BB1SC12 | LC-MS: m/z =560.3 $(M+H)^+$ ; |
| S34 | 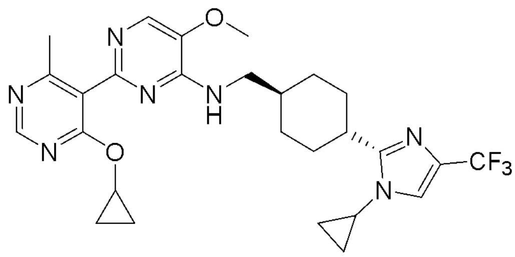 4'-Cyclopropoxy-N-((1R,4R)-4-(1-cyclopropyl-4-(trifluoromethyl)-1H-imidazol-2-yl)cyclohexyl)methyl)-5-methoxy-6-methyl-[2,5'-bipyrimidin]-4-amine | SA6 BB2SC11 | LC-MS: m/z =544.4 $(M+H)^+$; |

(continued)

| Example | Structure and name of compound | Reaction moiety | Analyzed data |
|---|---|---|---|
| S35 | 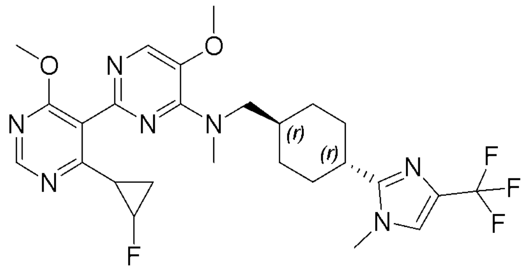 4'-(2-Fluorocyclopropyl)-5,6'-dimethoxy-N-methyl-N-((1R,4R)-4-(1-methyl-4-(trifluoromethyl)- 1H-imidazol-2-yl)cyclohexyl)methyl)-[2,5'-bipyrimidin]-4-amine | SA5 BBIB12 | LC-MS: m/z =550.3 $(M+H)^+$ ; |
| S36 | 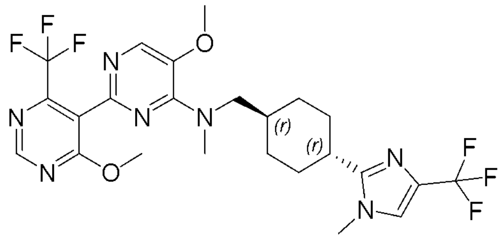 4',5-Dimethoxy-N-methyl-N-(((1R,4R)-4-(1-methyl-4-(trifluoromethyl)-1H-imidazol-2-yl)cyclohexyl)methyl)-6'-(trifluoromethyl)-[2,5'-bipyrimidin]-4-amine | SA4 BB1B12 | LC-MS: m/z =560.2 $(M+H)^+$; $^1H$ NMR (400 MHz, DMSO-$d_6$) δ 9.05 (s, 1H), 8.04 (s, 1H), 7.64 (s, 1H), 3.99 (s, 3H), 3.91 (s, 3H), 3.64 (s, 3H), 3.59 (d, *J*= 7.2 Hz, 2H), 3.14 (s, 3H), 2.74-2.70 (m, 1H), 1.89-1.85 (m, 2H), 1.78 (d, *J* = 7.7 Hz, 1H), 1.71-1.65 (m, 2H), 1.50-1.42 (m, 2H), 1.09-1.02 (m, 2H). |
| S37 | 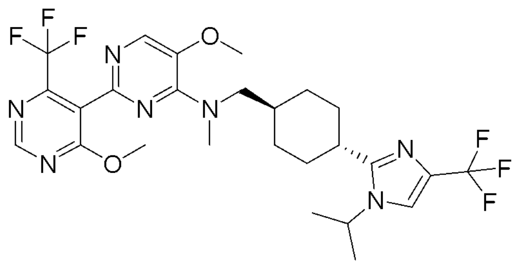 N-(((1R,4R)-4-(1-isopropyl-4-(trifluoromethyl)-1H-imidazol-2-yl)cyclohexyl)methyl)-4',5-dimethoxy-N-methyl-6'-(trifluoromethyl)-[2,5'-bipyrimidin]-4-amine | SA4 BB1SC12 | LC-MS: m/z =588.1 $(M+H)^+$; $^1H$ NMR (400 MHz, DMSO-$d_6$) δ 9.07 (s, 1H), 8.05 (s, 1H), 7.67 (s, 1H), 5.24-5.18 (m, 1H), 4.01 (s, 3H), 3.92 (s, 3H), 3.59 (d, *J*= 6.8 Hz, 2H), 3.14 (s, 3H), 2.76-2.69 (m, 1H), 1.90-1.83 (m, 2H), 1.78-1.72 (m, 1H), 1.68-1.59 (m, 8H), 1.55-1.48 (m, 2H), 1.19-1.06 (m, 2H). |

(continued)

| Example | Structure and name of compound | Reaction moiety | Analyzed data |
|---|---|---|---|
| **S38** | 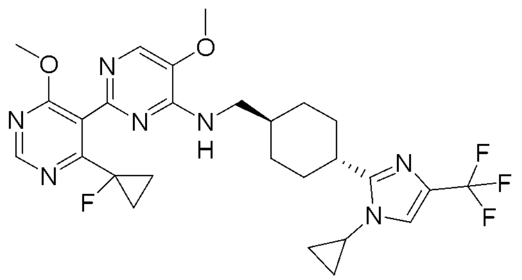 N-(((1R,4R)-4-(1-cyclopropyl-4-(trifluoromethyl)- 1H-imidazol-2-yl)cyclohexyl)methyl)-4'-(1-fluorocyclopropyl)-5,6'-dimethoxy-[2,5'-bipyrimidin]-4-amine | **SA8 BB2SC11** | LC-MS: m/z =562.3 $(M+H)^+$; |
| **S39** | 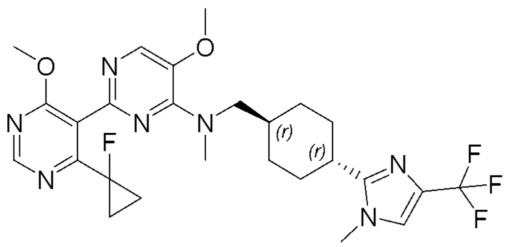 4'-(1-Fluorocyclopropyl)-5,6'-dimethoxy-N-methyl-N-((1R,4R)-4-(1-methyl-4-(trifluoromethyl)-1H-imidazol-2-yl) cyclohexyl)methyl)-[2,5'-bipyrimidin]-4-amine | **SA8 BB1B12** | LC-MS: m/z =550.4 $(M+H)^+$; |

**Example S40: Synthesis of compound S40 (4'-cyclopropyl-N-((4-(1-isopropyl-4-(trifluoromethyl)-1H-imidazol-2-yl)cuban-1-yl)methyl)-5,6'-dimethoxy-N-methyl-[2,5'-bipyrimidin]-4-amine)**

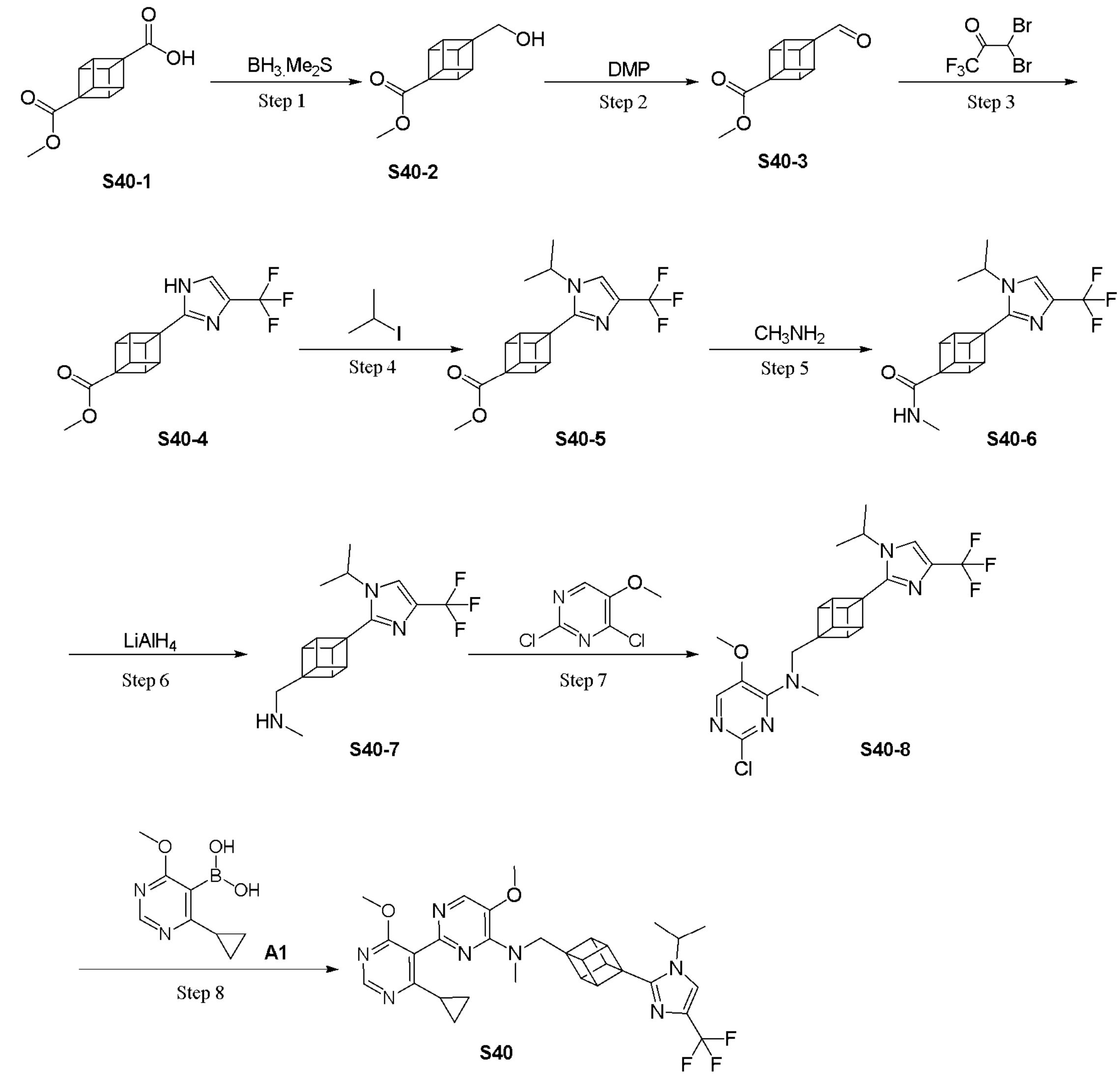


S40-1 S40-2 S40-3 S40-4 S40-5 S40-6 S40-7 S40-8 S40

Step 1: Synthesis of compound S**40-2** (methyl (2R,3R,4S,5S)-4-(hydroxymethyl)cuban-1-carboxylate)

(1S,2R,3R,8S)-4-(methoxycarbonyl)cubane-1-carboxylic acid (compound **S40-1,** 10.0 g, 48.5 mmol, 1.0 eq) was dissolved in tetrahydrofuran (300.0 mL), followed by the addition of a solution of 10 M borane dimethyl sulfide in tetrahydrofuran (5.82 mL, 1.2 eq) at 20°C, and stirred for 13 hours at 50°C. Methanol (300.0 mL) was added slowly at 0°C to quench the reaction, and the resulting mixture was filtered to obtain a filtrate. The filtrate was concentrated under vacuum to obtain compound **S40-2** (11.0 g, crude product) as a white solid. $^1$H NMR (400MHz, CDCl$_3$) δ 4.15 - 4.13 (m, 3H), 3.89 - 3.87 (m, 3H), 3.77 - 3.75 (m, 2H), 3.70 (s, 3H).

Step 2: Synthesis of compound **S40-3** (methyl (2R,3R,4S,5S)-4-formylcuban-1-carboxylate)

Compound **S40-2** (11.0 g, 57.2 mmol, 1.00 eq) was dissolved in dichloromethane (150 mL), followed by the addition of Dess-Martin periodinane (DMP, 29.1 g, 68.6 mmol, 21.2 mL, 1.20 eq) at 0°C, and stirred for 2 hours at 0°C. Saturated aqueous sodium bicarbonate solution (100 mL) was added, and the resulting mixture was extracted with dichloromethane (150 mL) three times. The organic layers were combined, washed with saturated brine (120 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **S40-3** (5.8 g, 30.5 mmol, 53.3% yield) as a white solid. $^1$H NMR (400MHz, CDCl$_3$) δ 9.75 (s, 1H), 4.38 - 4.36 (m, 3H), 4.27 - 4.25 (m, 3H), 3.72 (s, 3H).

Step 3: Synthesis of compound **S40-4** (methyl (2R,3R,4S,5S)-4-(4-(trifluoromethyl)-1H-imidazol-2-yl)cuban-1-carboxylate)

Sodium acetate (5.25 g, 64.0 mmol, 2.1 eq) was dissolved in water (25.0 mL), followed by the addition of 1,1-dibromo-3,3,3-trifluoroacetone (9.05 g, 33.5 mmol, 1.1 eq) at 20°C, heated up to 100°C and stirred for 1 hour. Compound **S40-3** (5.8 g, 30.5 mmol, 1.0 eq) dissolved in methanol (65.0 mL) and ammonia water (25.0 mL) was added at 20°C. After the addition was completed, the resulting mixture was stirred for 11 hours at 20°C. The reaction solution was filtered to obtain a filtrate, which was concentrated under vacuum to obtain compound **S40-4** (6.0 g, 18.5 mmol, 60.8% yield) as a white solid. LC-MS: m/z =297.0 $(M+H)^+$.

Step 4: Synthesis of compound **S40-5** (methyl (2R,3R,4S,5S)-4-(1-isopropyl-4-(trifluoromethyl)-1H-imidazol-2-yl)cuban-1-carboxylate)

Compound **S40-4** (4.5 g, 15.2 mmol, 1.0 eq) was dissolved in tetrahydrofuran (40.0 mL), followed by the addition of sodium hydride (911 mg, 22.7 mmol, 60% content, 1.50 eq) in batches at 0°C. After the addition was completed, the resulting mixture was stirred for 30 minutes at 20°C. Isopropyl iodide (5.17 g, 30.4 mmol, 2.0 eq) was added, and the resulting mixture was reacted for 2 hours at 20°C. Saturated aqueous ammonium chloride solution (100.0 mL) was added at 0°C to quench the reaction. Water (100 mL) was added, and the resulting mixture was extracted with ethyl acetate (150.0 mL) three times. The organic layers were combined, washed with saturated brine (150.0 mL) twice, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **S40-5** (3.3 g, 9.75 mmol, 64.2% yield) as a white solid. LC-MS: m/z = 339.3 $(M+H)^+$.

Step 5: Synthesis of compound **S40-6** ((2R,3R,4S,5S)-4-(1-isopropyl-4-(trifluoromethyl)-1H-imidazol-2-yl)-N-methylcubane-1-carboxamide)

Compound **S40-5** (1.0 g, 2.96 mmol, 1.0 eq) was dissolved in ethanol (20.0 mL), followed by the addition of a solution of methylamine in ethanol (15.3 g, 148.0 mmol, 30% content, 50.0 eq) at 25°C, stirred for 12 hours at 70°C and under a pressure of 50 Psi, and cooled to room temperature. Ice water (20.0 mL) was added, and the resulting mixture was concentrated under vacuum to remove ethanol, and extracted with ethyl acetate (50.0 mL) three times. The organic layers were combined, dried over anhydrous sodium sulfate, and filtered to obtain a filtrate. The filtrate was concentrated under vacuum to obtain compound **S40-6** (1.2 g, crude product) as a white solid. LC-MS: m/z =338.1 $(M+H)^+$.

Step 6: Synthesis of compound **S40-7** (1-((2R,3R,4S,5S)-4-(1-isopropyl-4-(trifluoromethyl)-1H-imidazol-2-yl)cuban-1-yl)-N-methylmethanamine)

Compound **S40-6** (1.2 g, 3.56 mmol, 1.0 eq) was dissolved in tetrahydrofuran (25.0 mL), followed by the addition of a solution of 2.5 M lithium aluminium hydride in tetrahydrofuran (10.7 mmol, 4.28 mL, 3.0 eq) at 0 °C, stirred for 30 minutes at 0°C, then heated up to 50°C and stirred for 3 hours. Sodium sulfate decahydrate (5.0 g) was added at 0°C and stirred for 30 minutes. Tetrahydrofuran (25.0 mL) was added and stirred for 5 minutes. The resulting mixture was filtered to obtain a filtrate, which was concentrated under vacuum to obtain compound **S40-7** (866.9 mg, crude product) as a white solid. LC-MS: m/z = 324.3 $(M+H)^+$.

Step 7: Synthesis of compound **S40-8** (2-chloro-N-(((2R,3R,4S,5S)-4-(1-isopropyl-4-(trifluoromethyl)-1H-imidazol-2-yl)cuban-1-yl)methyl)-5-methoxy-N-methylpyrimidin-4-amine)

Compound **S40-8** (800.0 mg, 2.47 mmol, 1.0 eq), N,N-diisopropylethylamine (1.28 g, 9.90 mmol, 4.0 eq) and 2,4-dichloro-5-methoxypyrimidine (663.2 mg, 3.71 mmol, 1.5 eq) were added successively to dioxane (15.0 mL), stirred for 8 hours at an external temperature of 60°C, and cooled to room temperature. Water (20.0 mL) was added, and the resulting mixture was extracted with ethyl acetate **(30.0** mL) three times. The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate= 1/0 - 0/1) to obtain compound **S40-8** (458.2 mg, 0.98 mmol, 39.8% yield) as a white solid. LC-MS: m/z = 466.4 $(M+H)^+$.

Step 8: Synthesis of compound **S40** (4'-cyclopropyl-5,6'-dimethoxy-N-methyl-N-(4-(1-methyl-4-(trifluoromethyl)-1H-imidazol-2-yl)cuban-1-yl)methyl)-[2,5'-bipyrimidin]-4-amine)

In accordance with the same steps of compound **S1,** compound **S40** (33.1 mg, 0.057 mmol, 27.2% yield) as a

white solid was obtained using intermediate **S40-8** (100.0 mg, 0.21 mmol, 1.0 eq) and compound **A2-7** (83.3 mg, 0.43 mmol, 2.0 eq) as starting materials. LC-MS: m/z = 580.3 $(M+H)^+$.

**Example S41: Synthesis of compound S41 (4'-cyclopropyl-N-((4-(1-cyclopropyl-4-(trifluoromethyl)-1H-imidazol-2-yl)cuban-1-yl)methyl)-5,6'-dimethoxy-N-methyl-[2,5'-bipyrimidin]-4-amine)**

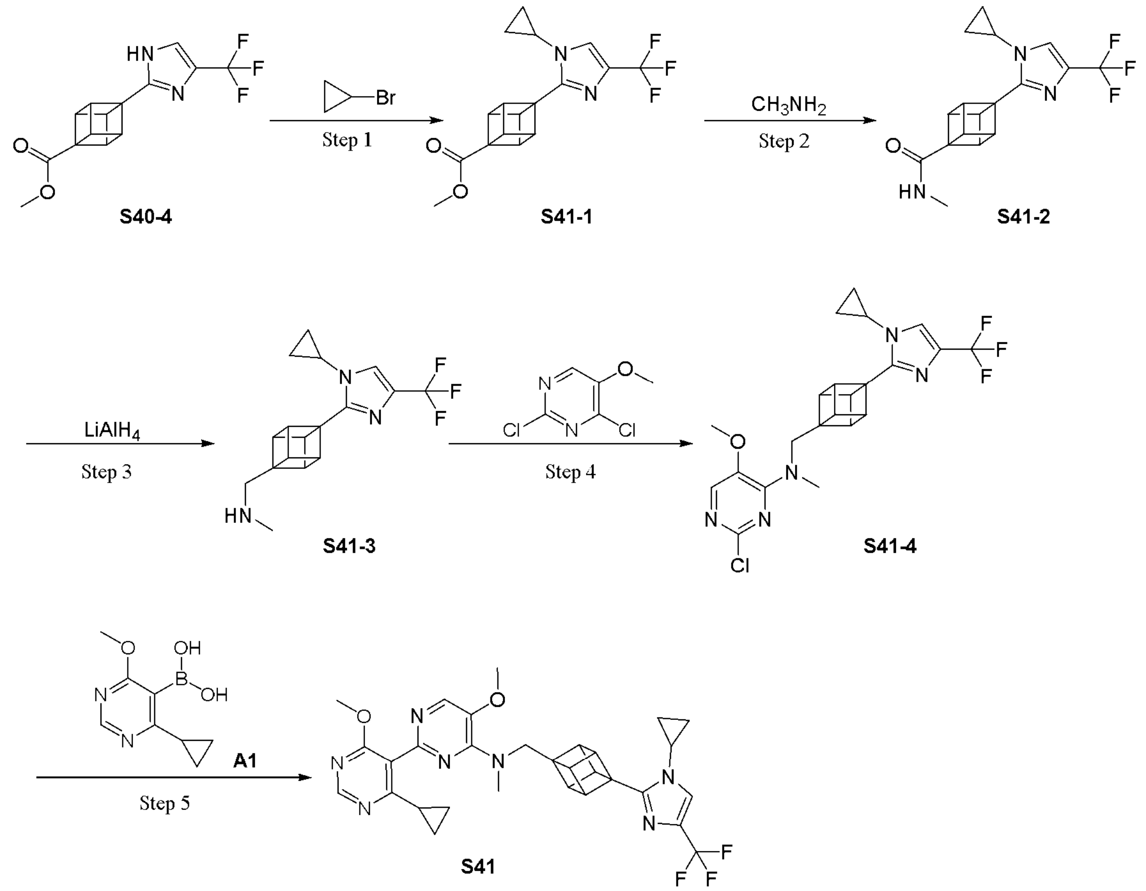


In accordance with the same steps of compound **S40,** compound **S41** (13.0 mg, 0.022 mmol, 16.8% yield) as a white solid was obtained using compound **S40-4** (2.1 g, 7.09 mmol, 1.0 eq) and cyclopropyl bromide (2.57 g, 21.3 mmol, 3.0 eq) as starting materials. LC-MS: m/z = 578.4 $(M+H)^+$.

**Example S42: Synthesis of compound S42 (2-(2-isopropylpyridin-3-yl)-5-methoxy-N-((4-(1-methyl-4-(trifluoromethyl)-1H-imidazol-2-yl)cuban-1-yl)methyl)pyrimidin-4-amine)**

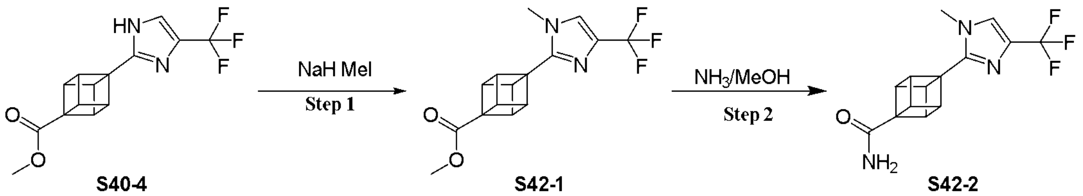


S40-4 S42-1 S42-2

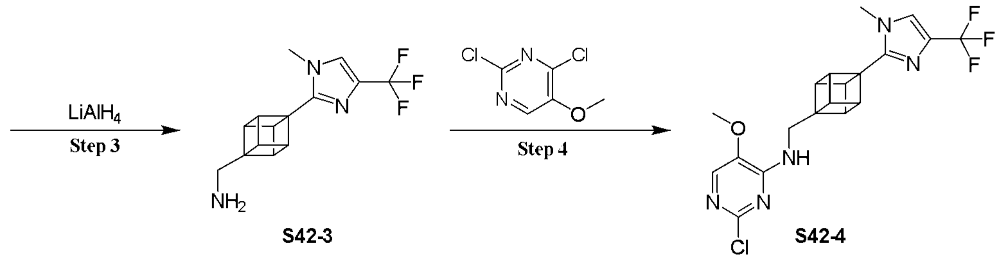


S42-3 S42-4

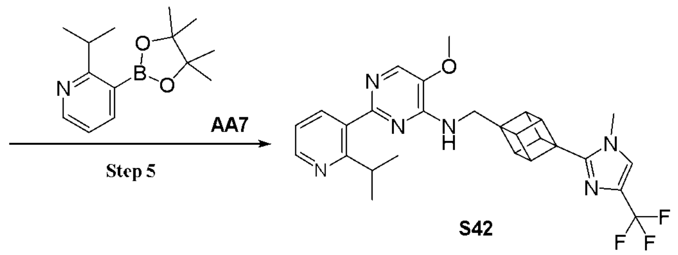


S42

In accordance with the same steps of compound **S40,** compound **S42** (8.7 mg, 0.017 mmol, 21.6% yield) as a white solid was obtained using compound **S40-4** (1.6 g, 5.4 mmol, 1.0 eq) and iodomethane (919.9 mg, 6.48 mmol, 1.2 eq) as starting materials. LC-MS: m/z = 509.3 $(M+H)^+$.

**Example S43: Synthesis of compound S43 (N-(4'-cyclopropyl-5,6'-dimethoxy-[2,5'-bipyrimidin]-4-yl)-N-(4-(1-methyl-4-(trifluoromethyl)-1H-imidazol-2-yl)bicyclo[2.2.2]oct-1-yl)methyl)cyanamide)**

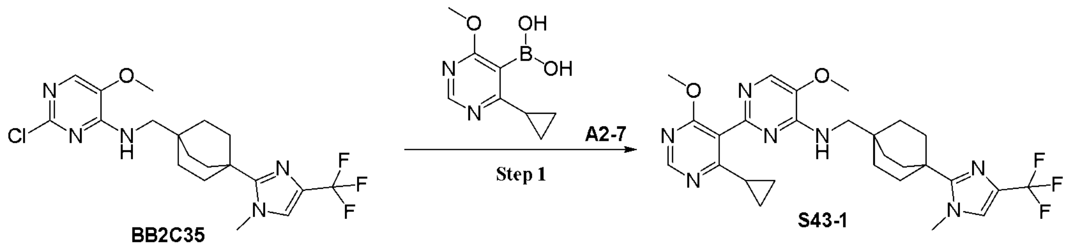


BB2C35 S43-1

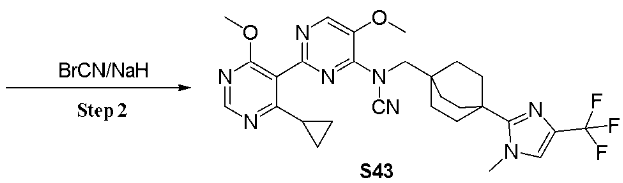


S43

Step 1: Synthesis of compound **S43-1** (4'-cyclopropyl-5,6'-dimethoxy-N-((4-(1-methyl-4-(trifluoromethyl)-1H-imidazol-2-yl)bicyclo[2.2.2]oct-1-yl)methyl)-[2,5'-bipyrimidin]-4-amine)

In accordance with the same steps of compound **S1,** compound **S43-1** (117.1 mg, 0.22 mmol, 71.0% yield) as a white solid was obtained using intermediate **BB2C35** (135.0 mg, 0.31 mmol, 1.0 eq) and **intermediate A2-7** (121.9 mg, 0.63 mmol, 2.0 eq) as starting materials. LC-MS: m/z = 544.3 (M+H)+.

Step 2: Synthesis of compound **S43**

Compound **S43-1** (100.0 mg, 0.18 mmol, 1.0 eq) was dissolved in N,N-dimethylformamide (2.0 mL), followed by the addition of sodium hydride (60% content, 73.6 mg, 18.4 mmol, 10.0 eq) in batches at 0°C, naturally heated up to 25°C and stirred for 1 hour. Cyanogen bromide (95.3 mg, 0.90 mmol, 5.0 eq) was added, and the resulting mixture was stirred for 16 hours at 45°C, concentrated under vacuum to obtain a crude product, which was purified by column chromatography (eluent: petroleum ether/ethyl acetate= 1/0 - 0/1) to obtain compound **S43** (12.4 mg, 0.02 mmol, 12.1% yield) as a white solid. LC-MS: m/z =569.3 (M+H)+.

**Example S49: Synthesis of compound S49 (4'-(2,2-difluorocyclopropyl)-5,6'-dimethoxy-N-methyl-N-((1R,4R)-4-(1-methyl-4-(trifluoromethyl)-1H-imidazol-2-yl)cyclohexyl)methyl)-[2,5'-bipyrimidin]-4-amine)**

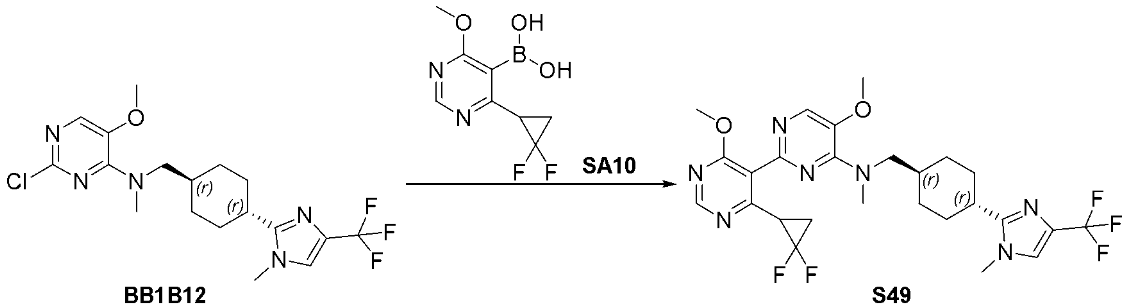


BB1B12 S49

In accordance with the same steps of compound **S1,** compound **S49** (11.3 mg, 0.02 mmol, 46.3% yield) as a white solid was obtained using intermediate **BB1B12** (18.0 mg, 0.043 mmol, 1.0 eq) and compound **SA10** (19.8 mg, 0.086 mmol, 2.0 eq) as starting materials. LC-MS: m/z = 568.4 (M+H)+.

**Example S50: Synthesis of compound S50 (4'-(2,2-difluorocyclopropyl)-5,6'-dimethoxy-N-((1R,4R)-4-(1-methyl-4-(trifluoromethyl)-1H-imidazol-2-yl)cyclohexyl)methyl)-[2,5'-bipyrimidin]-4-amine)**

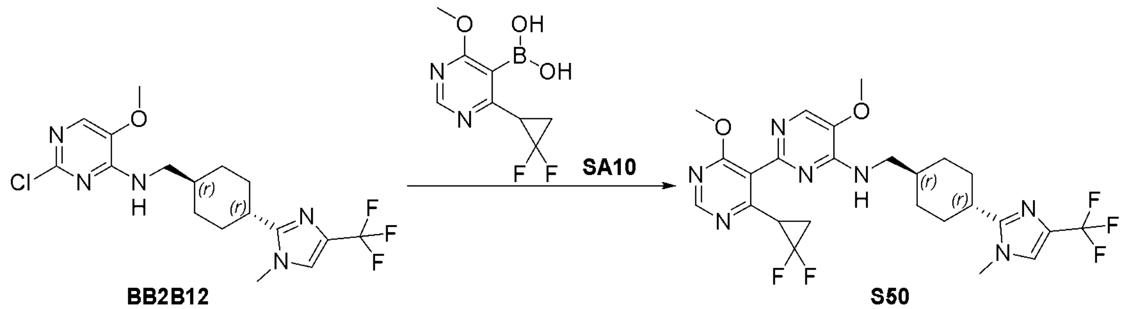


BB2B12 S50

In accordance with the same steps of compound **S1,** compound **S50** (19.2 mg, 0.035 mmol, 55.9% yield) as a white solid was obtained using intermediate **BB2B12** (25.0 mg, 0.062 mmol, 1.0 eq) and compound **SA10** (28.5 mg, 0.12 mmol, 2.0 eq) as starting materials. LC-MS: m/z = 554.3 (M+H)+.

**Example S51: Synthesis of compound S51 (4'-cyclopropyl-5,6'-dimethoxy-N-methyl-N-((4-(1-methyl-4-(trifluoromethyl)-1H-imidazol-2-yl)cuban-1-yl)methyl)-[2,5'-bipyrimidin]-4-amine)**

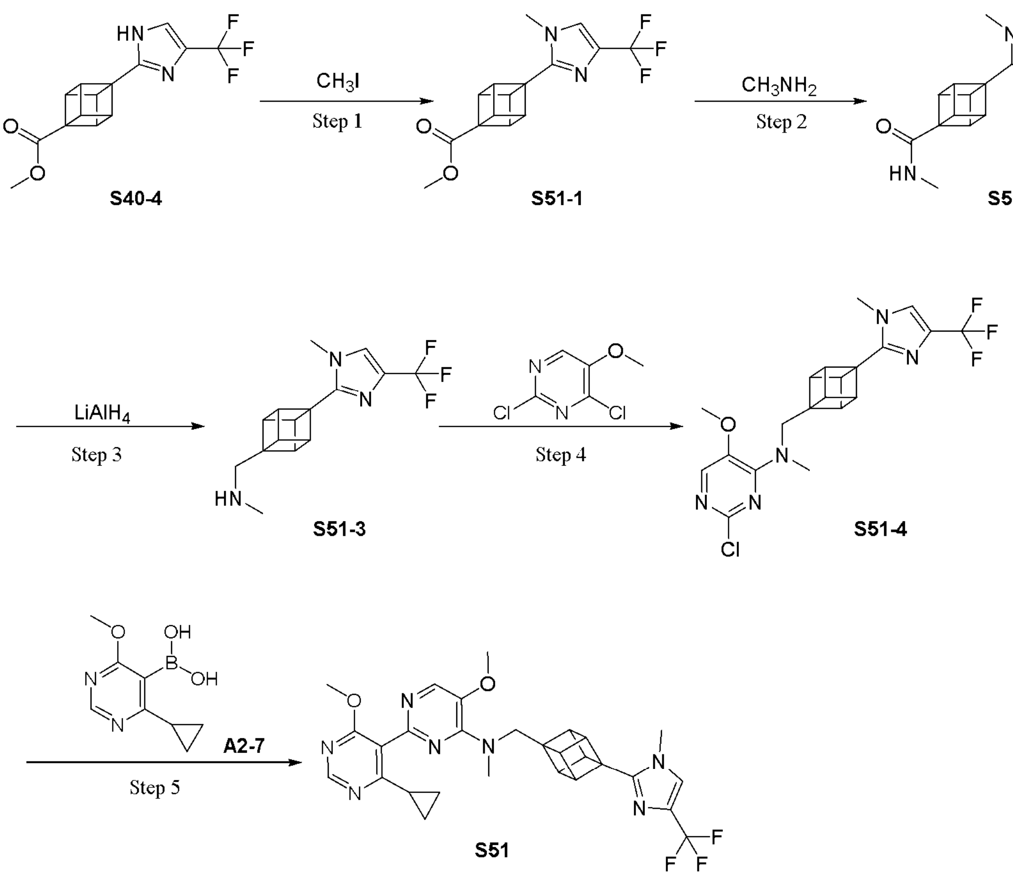


S40-4 S51-1 S51-2 S51-3 S51-4 S51

In accordance with the same steps of compound **S40,** compound **S51** (89.6 mg, 0.16 mmol, 45.9% yield) as a white solid was obtained using compound **S40-4** (4.0 g, 13.5 mmol, 1.0 eq) and iodomethane (2.3 g, 16.2 mmol, 1.2 eq) as starting materials. $^1$H NMR (400 MHz, DMSO-$d_6$) δ 8.60 (s, 1H), 7.88 (s, 1H), 7.20 (s, 1H), 4.31-4.28 (m, 3H), 4.15-3.96 (m, 6H), 3.95-3.89 (m, 3H), 3.85-3.78 (m, 2H), 3.56 (s, 3H), 3.25 (s, 3H), 1.79-1.66 (m, 1H), 1.20-1.17 (m, 2H), 0.91-0.88 (m, 2H). LC-MS: m/z = 552.3 $(M+H)^+$.

**Test Example 1: *In vitro* inhibitory activity against USP1 kinase**

Experimental object: to screen compounds with inhibitory activity against USP1 kinase by a fluorescence detection method, using a 384-well plate.

Experimental materials: Recombinant human His6-USP1/His6-UAF1 complex protein (R&D, Cat. No.: E-568-050), Ubiquitin Rhodamine 110 protein (Ub-Rho) (R&D, Cat. No.: U-555-050), 384-well plate (Perkin Elmer, Cat. No.: 6007279); all compounds were dissolved in 100% DMSO to obtain 50 mM stock solutions.

Experimental method:

1) Preparation of 1 × assay buffer: 1 × assay buffer (modified Tris buffer) was prepared;
2) Dilution of compounds: the compounds were transferred to an assay plate via Echo. DMSO had a final ratio of 1%;
3) Preparation of an enzyme solution: recombinant human His6-USP1/His6-UAF1 complex protein was added to 1 × assay buffer to prepare the enzyme solution;
4) Preparation of a substrate solution: Ubiquitin Rhodamine 110 protein CF (Ub-Rho) was added to 1 × assay buffer to obtain the substrate solution;

5) 10 μL of the enzyme solution was transferred to the assay plate, and 10 μL of 1 × assay buffer was added to the control wells;
6) The assay plate was incubated for 1 hour at room temperature;
7) 10 μL of the substrate solution was added to each well to start the reaction, and the plate was centrifuged for 30 seconds, and shaken for 30 seconds;
8) After 30 minutes, the plate was read using Envision with following parameters: excitation wavelength set to 480 nm, and emission wavelength set to 540 nm;
9) The data were collected and summarized;
10) Curve fitting: the data were fitted in Excel,

an inhibition rate formula: % inhibition rate = (maximum value - signal value)/(maximum value - minimum value) × 100; and the data were fitted in XL-Fit,

a half maximal inhibitory concentration formula: % inhibition rate = minimum concentration + (maximum concentration - minimum concentration)/(1+(half maximal inhibitory concentration/compound concentration)$^{slope}$);

and the half maximal inhibitory concentration was represented by $IC_{50}$ below.

**Test Example 2: CTG experiment for cell viability**

1. Object: to screen USP1 inhibitors by a CTG assay, using a 96-well plate.
2. Materials:

The cells used were MDA-MB-436 (manufacturer: Nanjing Cobioer Biotechnology Co., Ltd.) and cultured in DMEM medium,
CTG (CellTiter-Glo® Luminescent Cell Viability Assay kit) (Promega, Cat. No.: G7572),
96-well plate (Corning, Cat. No.: 3610).

3. Compounds:
all compounds were dissolved in 100% DMSO to obtain 50 mM stock solutions.
4. Experimental method:

① Plating:
The cells to be tested were spread into a 96-well plate at a certain density, with 200 μL per well, and 200 μL of cell medium was added to the blank wells. The spread cell plate was placed in an incubator at 37°C.
② Dilution of compounds:
The compounds were diluted into different concentrations with 100% DMSO, and DMSO had a final ratio of 4%.
③ Addition of the compounds:
The formulated compounds in Step ② were added to the cell plate in Step ①, with 10 μL per well. The cell plate with the compounds was placed in an incubator at 37°C, and further cultured for 7 days prior to detection.
④ Assay:
The substrate and the buffer of CTG kit were mixed evenly. The cell plate with the compounds in Step ③ was taken out from the incubator, and left at room temperature to equilibrate for 30 minutes. The medium containing the compounds was removed from the cell plate, 100 μL of PBS and 50 μL of CTG reagent were added to each well, shaken and mixed for 3 minutes, and then incubated for 10 minutes at room temperature. The plate was read by a Blotek microplate-reader, with a parameter set to 567 CPM.

5. Curve fitting

It was the same as that for the enzyme.

The $IC_{50}$ values for inhibitory activity against USP1 kinase and the $IC_{50}$ data for inhibitory activity against cells of the compounds of the present invention are shown as follows:

Table 1

| Example No. | USP1 $IC_{50}$ (nM) | Example No. | USP1 $IC_{50}$ (nM) |
|---|---|---|---|
| 1 | 26 | 141 | 9.5 |
| 2 | 14 | 142 | 25 |
| 3 | 2.8 | 143 | 31.0 |
| 4 | 1.2 | 144 | 68.6 |
| 5 | 7.6 | 145 | 19 |
| 6 | 1.2 | 146 | 18 |
| 7 | 4.5 | 147 | 364 |
| 8 | 1.4 | 149 | 69.2 |
| 9 | 35 | 150 | 431.2 |
| 10 | 564 | 151 | 72 |
| 11 | 22 | 152 | 7.5 |
| 13 | 1.9 | 153 | 3.4 |
| 14 | 60 | 154 | 6.2 |
| 15 | 6.8 | 155 | 12 |
| 16 | 30.6 | 156 | 2.2 |
| 17 | 3.9 | 157 | 2.9 |
| 18 | 4 | 158 | 2.1 |
| 19 | 24 | 159 | 1.7 |
| 20 | 91.5 | 160 | 3.6 |
| 21 | 7.4 | 161 | 6 |
| 22 | 24.4 | 162 | 3.9 |
| 23 | 33 | 163 | 2.3 |
| 24 | 8.8 | 164 | 1.1 |
| 25 | 3.7 | 165 | 1.4 |
| 26 | 4.6 | 166 | 2.1 |
| 27 | 195 | 167 | 0.45 |
| 28 | 33 | 168 | 1.2 |
| 29 | 4.8 | 169 | 0.8 |
| 30 | 37 | 170 | 2.9 |
| 32 | 2.8 | 171 | 2.3 |
| 33 | 1.2 | 172 | 12 |
| 34 | 6.9 | 173 | 1.0 |
| 35 | 9.6 | 174 | 5.1 |
| 36 | 22.4 | 175 | 3.6 |
| 37 | 51 | 176 | 4.0 |
| 38 | 1.1 | 177 | 75 |
| 39 | 2.8 | 178 | 110 |
| 40 | 2.3 | 179 | 56 |
| 41 | 1261 | 180 | 15 |

(continued)

| Example No. | USP1 $IC_{50}$ (nM) | Example No. | USP1 $IC_{50}$ (nM) |
|---|---|---|---|
| 50 | 5.2 | 181 | 27 |
| 57 | 4.4 | 182 | 7.4 |
| 61 | 11 | 183 | 164 |
| 62 | 2.1 | 184 | 94 |
| 63 | 3.3 | 185 | 9.8 |
| 64 | 3.7 | 186 | 66 |
| 65 | 2.1 | 187 | 23.5 |
| 66 | 5.6 | 188 | 15 |
| 67 | 5.3 | 189 | 16 |
| 68 | 3.2 | 190 | 33 |
| 69 | 9.2 | 191 | 4.3 |
| 70 | 19.4 | 192 | 3.8 |
| 71 | 9.9 | 193 | 87 |
| 72 | 6.1 | 194 | 51 |
| 73 | 3.6 | 195 | 243 |
| 74 | 872.8 | 196 | 12 |
| 75 | 3.7 | 197 | 133 |
| 76 | 30.1 | 198 | 3.2 |
| 77 | 28 | 199 | 5.6 |
| 78 | 6.4 | 200 | 2.8 |
| 79 | 4.5 | 201 | 12 |
| 80 | 26 | 202 | 3 |
| 81 | 3.7 | 203 | 1.4 |
| 82 | 1.7 | 204 | 25 |
| 83 | 4.4 | 205 | 43 |
| 84 | 9.0 | 206 | 3.5 |
| 85 | 0.47 | 207 | 3.5 |
| 86 | 1.1 | 209 | 1.6 |
| 87 | 6.2 | 210 | 85 |
| 88 | 10 | 211 | 6.1 |
| 89 | 14.4 | 212 | 19 |
| 90 | 101.7 | 215 | 2.2 |
| 91 | 1.3 | 216 | 1.4 |
| 92 | 3.3 | S3 | 2.7 |
| 93 | 1.5 | S4 | 4.9 |
| 94 | 6.5 | S5 | 14 |
| 95 | 8.4 | S7 | 12 |
| 96 | 4.8 | S9 | 3.5 |
| 97 | 263 | S10 | 19 |

(continued)

| Example No. | USP1 $IC_{50}$ (nM) | Example No. | USP1 $IC_{50}$ (nM) |
|---|---|---|---|
| 98 | 144.5 | S11 | 6.1 |
| 99 | 15.1 | S12 | 35 |
| 100 | 4.1 | S13 | 13 |
| 101 | 15 | S15 | 91 |
| 102 | 4.4 | S16 | 34 |
| 103 | 10 | S17 | 6.1 |
| 104 | 8.8 | S18 | 12 |
| 105 | 60 | S19 | 300 |
| 106 | 2.9 | S20 | 19 |
| 107 | 16 | S21 | 6 |
| 108 | 19.7 | S22 | 4.6 |
| 109 | 5.3 | S23 | 4.8 |
| 110 | 28.6 | S24 | 5.7 |
| 111 | 10.4 | S26 | 13 |
| 112 | 266.1 | S29 | 15 |
| 113 | 23 | S30 | 12 |
| 114 | 648 | S32 | 106 |
| 115 | 77 | S35 | 6.8 |
| 116 | 19.4 | S36 | 7.1 |
| 117 | 13.8 | S37 | 12 |
| 118 | 58 | S38 | 220 |
| 119 | 7.6 | S42 | 1.2 |
| 120 | 8.9 | S43 | 6.9 |
| 121 | 8.3 | S49 | 3.1 |
| 122 | 13 | S51 | 3.8 |
| 123 | 20 | | |
| 124 | 6.1 | | |
| 125 | 3.1 | | |
| 126 | 13 | | |
| 127 | 4.2 | | |
| 128 | 2.6 | | |
| 129 | 9 | | |
| 130 | 19 | | |
| 131 | 7.4 | | |
| 132 | 6.0 | | |
| 133 | 15 | | |
| 134 | 3.2 | | |
| 135 | 3.2 | | |
| 136 | 17 | | |

(continued)

| Example No. | USP1 $IC_{50}$ (nM) | Example No. | USP1 $IC_{50}$ (nM) |
|---|---|---|---|
| 137 | 24 | | |
| 138 | 9.0 | | |
| 139 | 5.2 | | |
| 140 | 73 | | |

Table 2

| Example No. | MDA-MB-436 $IC_{50}$ (nM) | Example No. | MDA-MB-436 $IC_{50}$ (nM) |
|---|---|---|---|
| 2 | 0.1 | 141 | 76.7 |
| 3 | 6.4 | 142 | 50.0 |
| 4 | 0.3 | 145 | 730.9 |
| 5 | 112.7 | 151 | 58.4 |
| 6 | 2 | 152 | 64.4 |
| 7 | 102.1 | 153 | 25.4 |
| 8 | 2.3 | 154 | 7.3 |
| 11 | 751 | 155 | 85.7 |
| 15 | 301 | 156 | 59.5 |
| 17 | 102.7 | 157 | 23.3 |
| 18 | 312.8 | 158 | 12.3 |
| 19 | 1.2 | 159 | 39.4 |
| 20 | 532.9 | 160 | 121.3 |
| 21 | 1.8 | 161 | 537.1 |
| 22 | 209 | 162 | 77.5 |
| 23 | 33.1 | 163 | 13.5 |
| 26 | 0.9 | 164 | 0.8 |
| 28 | 55.8 | 165 | 7.1 |
| 32 | 336 | 166 | 41.4 |
| 33 | 1.8 | 167 | 2.9 |
| 34 | 119 | 168 | 5.1 |
| 35 | 45 | 169 | 0.4 |
| 36 | 950.3 | 170 | 14.5 |
| 38 | 13.7 | 171 | 84.7 |
| 39 | 56.9 | 172 | 954.5 |
| 40 | 21.4 | 173 | 23.6 |
| 50 | 0.022 | 174 | 43.7 |
| 57 | 48.3 | 175 | 87.1 |
| 61 | 813 | 176 | 186.6 |
| 62 | 2.2 | 179 | 196.7 |
| 63 | 30 | 180 | 122.7 |
| 64 | 6.3 | 182 | 6.0 |

(continued)

| Example No. | MDA-MB-436 $IC_{50}$ (nM) | Example No. | MDA-MB-436 $IC_{50}$ (nM) |
|---|---|---|---|
| 65 | 2.4 | 185 | 119.9 |
| 66 | 14.6 | 189 | 307.2 |
| 68 | 266.9 | 190 | 339.1 |
| 69 | 709.1 | 191 | 10.7 |
| 70 | 599.8 | 192 | 20.4 |
| 71 | 274.3 | 194 | 58.4 |
| 72 | 181.17 | 196 | 82.1 |
| 73 | 112.3 | 198 | 5.6 |
| 75 | 330.6 | 199 | 28.7 |
| 81 | 66.1 | 200 | 2.8 |
| 82 | 278.3 | 201 | 40.5 |
| 83 | 99.1 | 202 | 15.9 |
| 84 | 704.4 | 203 | 3.3 |
| 85 | 22.5 | 204 | 15.7 |
| 86 | 29.4 | 207 | 5.8 |
| 88 | 156.8 | 209 | 31.6 |
| 89 | 243.1 | 210 | 168.6 |
| 91 | 479.4 | 215 | 8.7 |
| 92 | 27.3 | 216 | 2.7 |
| 93 | 3.5 | S3 | 0.3 |
| 94 | 275.4 | S4 | 2.3 |
| 95 | 75.1 | S5 | 20.2 |
| 96 | 12 | S7 | 17.1 |
| 98 | 694.4 | S9 | 0.7 |
| 99 | 445.0 | | 31.6 |
| 100 | 27.8 | S12 | 168.2 |
| 101 | 99.1 | S13 | 60.8 |
| 102 | 15.8 | S15 | 239.4 |
| 103 | 140.6 | S16 | 40.3 |
| 104 | 13.7 | S17 | 35.9 |
| 105 | 124.4 | S18 | 10.7 |
| 106 | 4.7 | S20 | 21.4 |
| 107 | 17.5 | S21 | 4.7 |
| 108 | 352.9 | S22 | 4.5 |
| 109 | 48.3 | S23 | 0.1 |
| 110 | 354.4 | S24 | 4.6 |
| 111 | 88.1 | S25 | 24.4 |
| 113 | 87.2 | S26 | 14.3 |
| 116 | 535.7 | S28 | 0.3 |

(continued)

| Example No. | MDA-MB-436 $IC_{50}$ (nM) | Example No. | MDA-MB-436 $IC_{50}$ (nM) |
|---|---|---|---|
| 117 | 278.3 | S29 | 338.2 |
| 119 | 16.9 | S30 | 31.9 |
| 120 | 50.5 | S32 | 234.7 |
| 121 | 23.9 | S35 | 3.5 |
| 122 | 82.8 | S36 | 1.8 |
| 123 | 169.3 | S37 | 32.6 |
| 124 | 13.2 | S42 | 3.5 |
| 125 | 0.5 | S43 | 12.1 |
| 126 | 22.7 | S49 | 0.7 |
| 127 | 12.2 | S51 | 5.8 |
| 128 | 5.7 | | |
| 129 | 12.6 | | |
| 130 | 45.6 | | |
| 131 | 45.5 | | |
| 132 | 8.8 | | |
| 133 | 134.8 | | |
| 134 | 34.1 | | |
| 135 | 15.8 | | |
| 136 | 24.9 | | |
| 137 | 216.2 | | |
| 138 | 12.4 | | |
| 139 | 5.0 | | |

As shown in Tables 1 and 2, the compounds of the present invention exhibited excellent inhibitory activity against USP1 kinase.

## Claims

**1.** A compound of formula (SI),

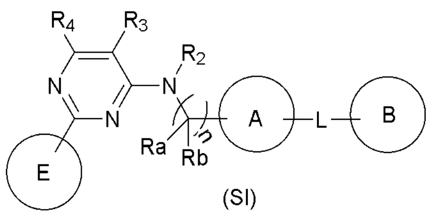

(SI)

or a pharmaceutically acceptable salt, hydrate, solvate, isotope substitute or stereoisomer thereof, wherein,

ring A and ring B are each independently selected from the group consisting of $C_{6-10}$ aryl, 5 to 10 membered heteroaryl, $C_{3-8}$ cycloalkyl and 3 to 8 membered heterocyclyl, and ring A and ring B are each independently and optionally substituted by one or more $R_1$;

ring E is selected from the group consisting of $C_{6-10}$ aryl, 5 to 6 membered heteroaryl, $C_{6-10}$ aryl fused with 5 to 6

membered heterocyclyl, and $C_{6-10}$ aryl fused with 5 to 6 membered heteroaryl, and ring E is optionally substituted by one or more $R_1$;
L is selected from the group consisting of a chemical bond, -O-, -S-, -$C_{1-6}$ alkylene-, -O-$C_{1-6}$ alkylene-, -$C_{1-6}$ alkylene-O-, -S-$C_{1-6}$ alkylene- and -$C_{1-6}$ alkylene-S-;
$R_a$ and $R_b$ are each independently selected from the group consisting of H atom, - CN, $C_{1-6}$ alkyl, -OH, halogen, $C_{2-6}$ alkenyl, $C_{2-6}$ alkynyl, $C_{1-6}$ hydroxyalkyl, $C_{1-6}$ haloalkyl, $C_{1-6}$ alkoxy, $C_{1-6}$ haloalkoxy; or $R_a$ and $R_b$ together form an oxo, $C_{3-8}$ cycloalkyl or 3 to 8 membered heterocyclyl; wherein the $C_{1-6}$ alkyl is optionally substituted by one or more $R_1$;
$R_2$ is selected from the group consisting of H atom, -OH, -CN, $C_{1-6}$ alkyl, -$C_{1-6}$ alkylene-$C_{6-10}$ aryl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl, wherein the $C_{1-6}$ alkyl or -$C_{1-6}$ alkylene-$C_{6-10}$ aryl is optionally substituted by one or more $R_1$;
$R_3$ is selected from the group consisting of H atom, -OH, -COOH, -$NH_2$, -CN, halogen, $C_{1-6}$ alkyl, -S-$C_{1-6}$ alkyl, -S(O)-$C_{1-6}$ alkyl, -S(O)$_2$-$C_{1-6}$ alkyl, phosphoryl, phosphonyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3 to 8 membered heterocyclyl and -$C_{1-6}$ alkylene-C(O)-O-$C_{1-6}$ alkyl, wherein the - $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl are each independently and optionally substituted by one or more $R_1$;
$R_4$ is selected from the group consisting of H atom, -OH, -COOH, -$NH_2$, -CN, halogen, $C_{1-6}$ alkyl, $C_{2-6}$ alkenyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{6-10}$ aryl, 5 to 10 membered heteroaryl, $C_{3-8}$ cycloalkyl and 3 to 8 membered heterocyclyl;
$R_1$ at each occurrence is independently selected from the group consisting of D atom, -OH, -COOH, -$NH_2$, -CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 10 membered heteroaryl, $C_{3-8}$ cycloalkyl, $C_{3-8}$ cycloalkyl-O-, 3 to 8 membered heterocyclyl, -O-$C_{1-6}$alkylene-O-$C_{1-6}$ alkyl, wherein the $C_{6-10}$ aryl, 5 to 10 membered heteroaryl, $C_{3-8}$ cycloalkyl and 3 to 8 membered heterocyclyl are each independently and optionally substituted by one or more substituents selected from the group consisting of D atom, - OH, -COOH, -$NH_2$, -CN, halogen, $C_{1-6}$ alkyl, $C_{1-6}$ haloalkyl, $C_{1-6}$ alkoxy, $C_{1-6}$ haloalkoxy and $C_{1-6}$ hydroxyalkyl; and
n is an integer from 0 to 8.

**2.** The compound according to claim 1, wherein,

ring B is $C_{6-10}$ aryl or 5 to 6 membered heteroaryl, and ring B is optionally substituted by one or more $R_1$, $R_1$ is as defined in claim 1;
specifically,
ring B is selected from the group consisting of phenyl, naphthyl, pyridinyl, pyrimidinyl, imidazolyl, pyrazolyl, thienyl, piperazinyl, naphthyl, pyrrolyl, pyridazinyl, triazolyl, tetrazolyl, and furanyl, and ring B is optionally substituted by one or more $R_1$, $R_1$ is as defined in claim 1;
more specifically,
ring B is imidazolyl, and ring B is optionally substituted by one or more $R_1$, $R_1$ is as defined in claim 1.

**3.** The compound according to claim 1 or 2, being a compound of formula (SII),

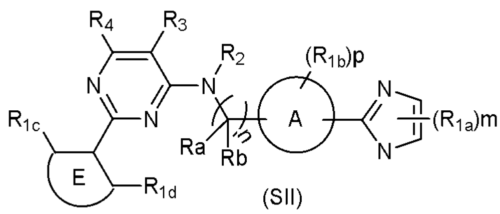

(SII)

wherein,

$R_{1a}$ at each occurrence is independently selected from the group consisting of D atom, -OH, -COOH, -$NH_2$, -CN, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;

$R_{1b}$ at each occurrence is independently selected from the group consisting of D atom, -OH, -COOH, -$NH_2$, -CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;
$R_{1c}$ is selected from the group consisting of H atom, D atom, -OH, -COOH, -$NH_2$, - CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;
$R_{1d}$ is selected from the group consisting of H atom, D atom, -OH, -COOH, -$NH_2$, - CN, oxo, halogen, $C_{1-6}$ alkyl, $C_{2-6}$ alkynyl, $C_{1-6}$ haloalkyl, $C_{1-6}$ alkoxy, $C_{1-6}$ deuterated alkoxy, $C_{1-6}$ haloalkoxy, $C_{1-6}$ hydroxyalkyl, $C_{6-10}$ aryl, 5 to 6 membered heteroaryl, $C_{3-6}$ cycloalkyl, $C_{3-6}$ halocycloalkyl, $C_{3-6}$ cycloalkyl-O- and 5 to 7 membered heterocyclyl;
m is 0, 1 or 2;
p is 0, 1 or 2;
ring A, ring E, $R_a$, $R_b$, $R_2$ to $R_4$ and n are as defined in claim 1.

**4.** The compound according to any one of claims 1 to 3, wherein,

ring A is selected from the group consisting of $C_{6-10}$ aryl, $C_{3-8}$ cycloalkyl and 3 to 8 membered heterocyclyl, and ring A is optionally substituted by one or more $R_1$, $R_1$ is as defined in claim 1;
specifically,
ring A is selected from the group consisting of phenyl, naphthyl, cyclohexyl, cyclopentyl, cyclobutyl, cyclopropyl, bicyclo[2.2.2]octanyl, 2-oxabicyclo[2.2.2]octanyl, cubanyl, piperidyl, pyranyl, pyrrolidinyl, piperazinyl and morpholinyl, and ring A is optionally substituted by one or more $R_{1b}$, $R_{1b}$ is as defined in claim 3;
more specifically,
ring A is selected from the group consisting of phenyl, cyclohexyl, bicyclo[2.2.2]octanyl, 2-oxabicyclo[2.2.2]octanyl and cubanyl, and ring A is optionally substituted by one or more $R_{1b}$, $R_{1b}$ is as defined in claim 3.

**5.** The compound according to any one of claims 1 to 4, wherein,

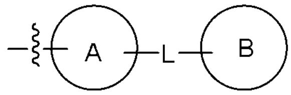 Or 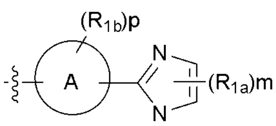

is selected from the group consisting of

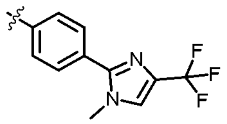, 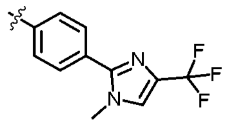, 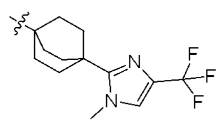, 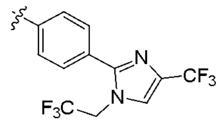,

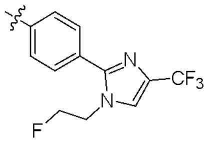, 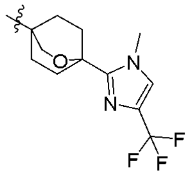, 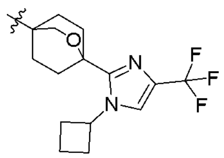, 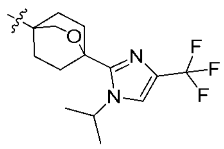,

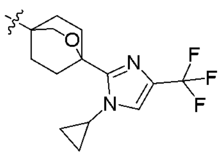, 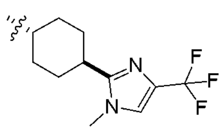, 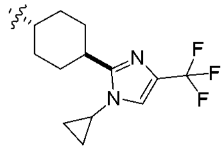, 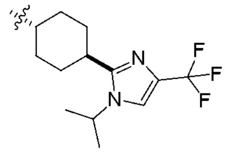,

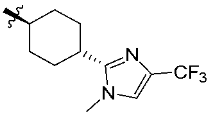 , 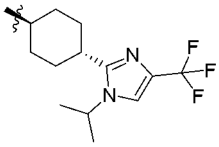 , 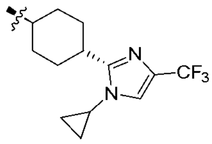 , 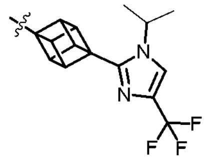 ,

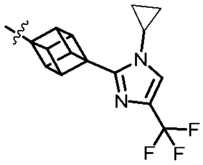 and 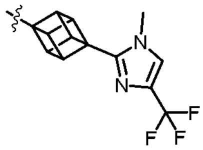 .

6. The compound according to any one of claims 1 to 5, wherein,

ring E is $C_{6-10}$ aryl or 5 to 6 membered heteroaryl, and ring E is optionally substituted by one or more $R_1$, $R_1$ is as defined in claim 1;
specifically,
ring E is selected from the group consisting of phenyl, pyridinyl, pyrimidinyl, pyrazolyl, imidazolyl, thiazolyl and isoxazolyl, and ring E is optionally substituted by one $R_{1c}$ and one $R_{1d}$, $R_{1c}$ and $R_{1d}$ is as defined in claim 3;
more specifically,
ring E is selected from the group consisting of pyridinyl, pyrimidinyl and imidazolyl, ring E is optionally substituted by one $R_{1c}$ and one $R_{1d}$, $R_{1c}$ and $R_{1d}$ are as defined in claim 3;
even more specifically,

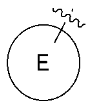

is selected from the group consisting of

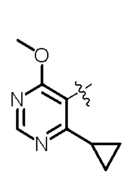 , 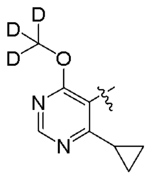 , 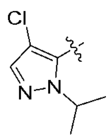 ,

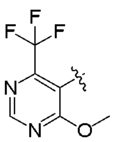 , 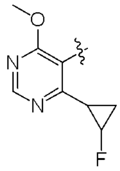 , 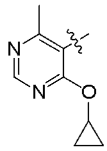 , 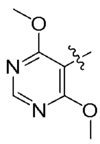 , 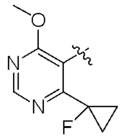 , 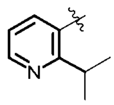 and 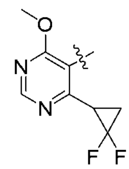 .

7. The compound according to any one of claims 1 to 6, wherein, n is 0 or 1;
and/or

$R_a$ and $R_b$ are each independently selected from the group consisting of H atom, - CN, $C_{1-6}$ alkyl, -OH and halogen;
preferably, $R_a$ and $R_b$ are each independently H atom or $C_{1-6}$ alkyl;
more preferably, both of $R_a$ and $R_b$ are H atom, or $R_a$ is H atom and $R_b$ is methyl.

8. The compound according to any one of claims 1 to 7, wherein,

$R_2$ is selected from the group consisting of H atom, -OH, -CN, $C_{1-6}$ alkyl, $C_{1-6}$ deuterated alkyl, $C_{2-6}$ alkynyl, -$C_{1-6}$

alkylene-$C_{6-10}$ aryl, $C_{1-6}$ alkoxy, $C_{1-6}$ haloalkyl, $C_{1-6}$ haloalkoxy, $C_{1-6}$ hydroxyalkyl, $C_{3-6}$ cycloalkyl and 5 to 7 membered heterocyclyl,
preferably, $R_2$ is selected from the group consisting of H atom, -CN, methyl and trideuteriomethyl;
and/or
$R_3$ is selected from the group consisting of H atom, -OH, -COOH, -$NH_2$, -CN, halogen, $C_{1-6}$ alkyl, $C_{2-6}$ alkynyl, $C_{1-6}$ alkoxy, $C_{1-6}$ haloalkyl, $C_{1-6}$ haloalkoxy, $C_{1-6}$ hydroxyalkyl, $C_{3-6}$ cycloalkyl-O-, 5 to 7 membered heterocyclyl and -$C_{1-6}$ alkylene-C(O)-O-$C_{1-6}$ alkyl;
preferably, $R_3$ is methoxy or cyclopropyl-O-;
and/or
$R_4$ is selected from the group consisting of H atom, -OH, -COOH, -$NH_2$, -CN, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;
preferably, $R_4$ is H atom.

**9.** The compound according to any one of claims 1 to 8, being selected from the group consisting of

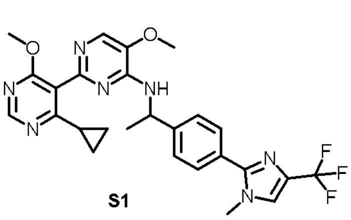
S1,

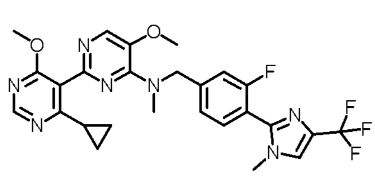
S2,

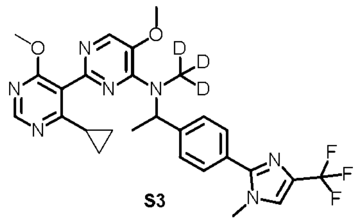
S3,

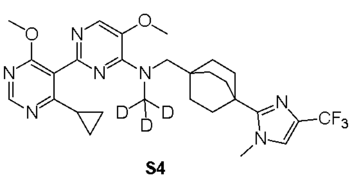
S4,

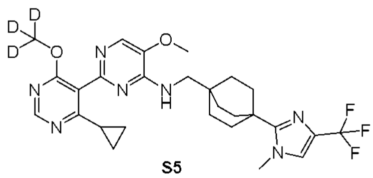
S5,

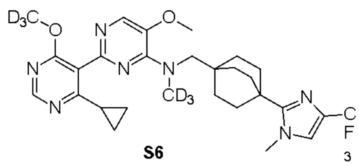
S6,

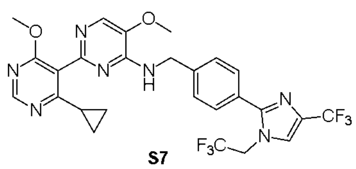
S7,

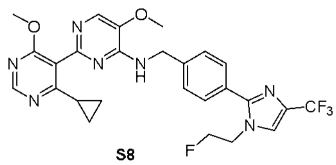
S8,

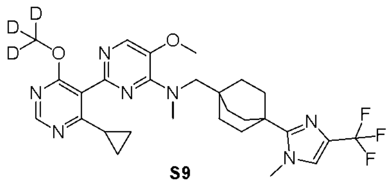
S9,

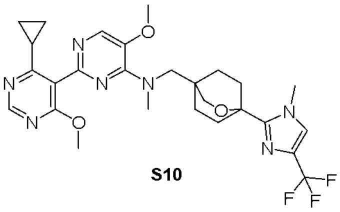
S10,

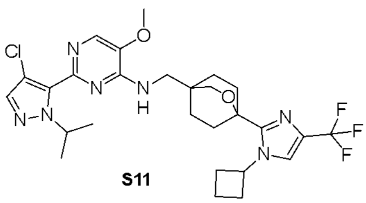
S11,

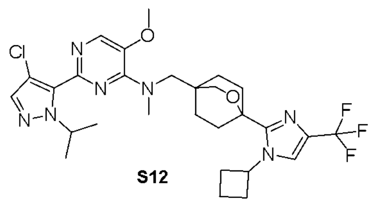
S12,

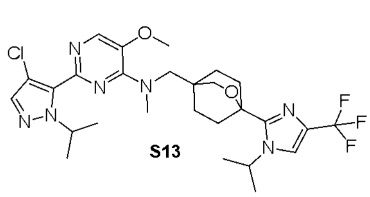
S13,

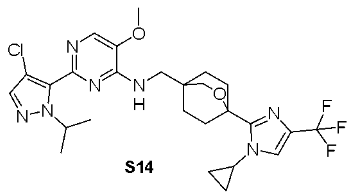
S14,

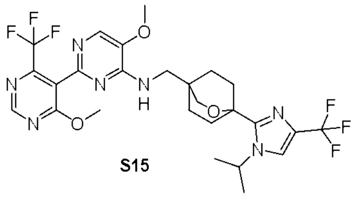
S15,

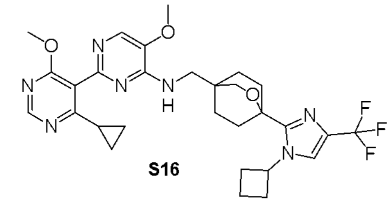
S16 ,
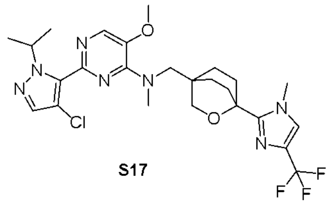
S17 ,
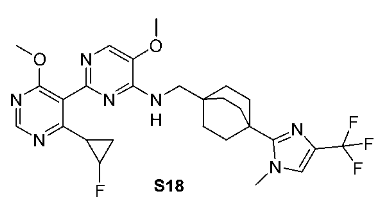
S18 ,

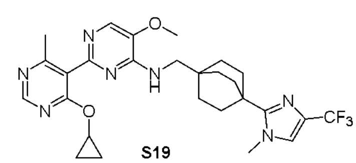
S19 ,
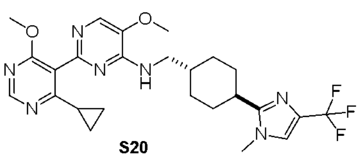
S20 ,
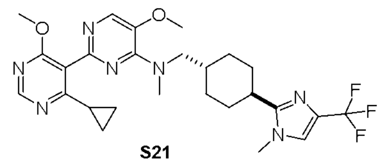
S21 ,

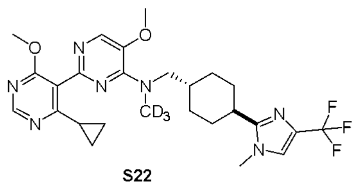
S22 ,
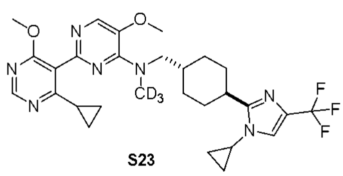
S23 ,
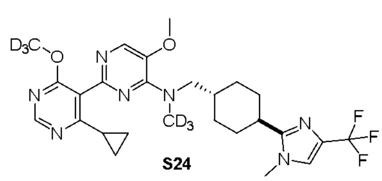
S24 ,

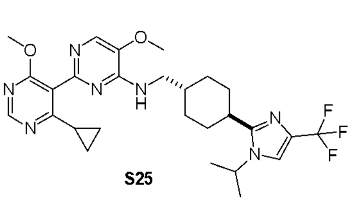
S25 ,
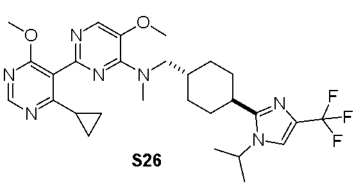
S26 ,
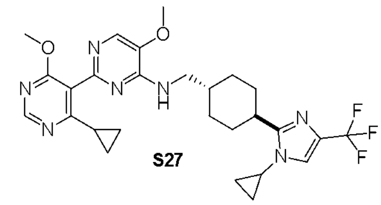
S27 ,

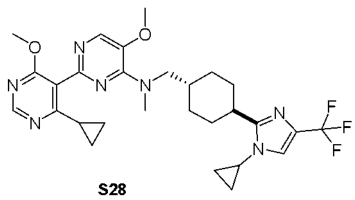
S28 ,
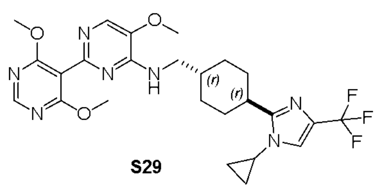
S29 ,
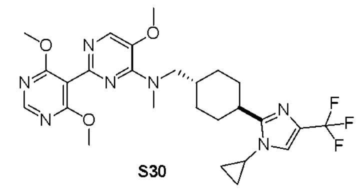
S30 ,

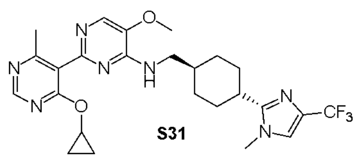
S31 ,
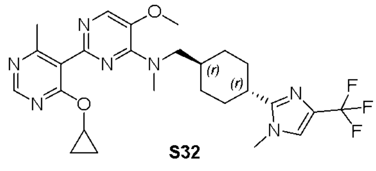
S32 ,
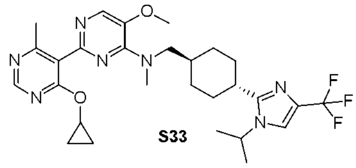
S33 ,

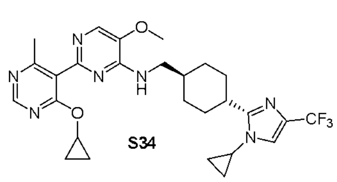
S34 ,
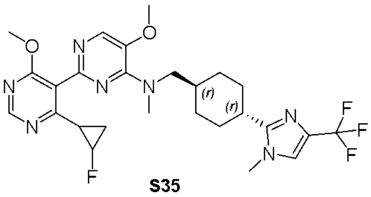
S35 ,
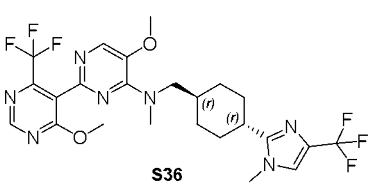
S36 ,

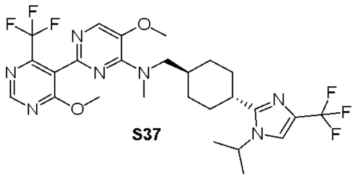
S37,
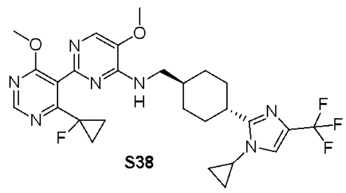
S38,
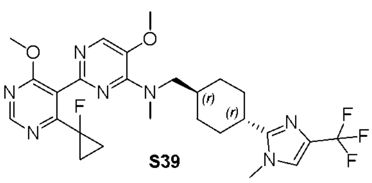
S39,
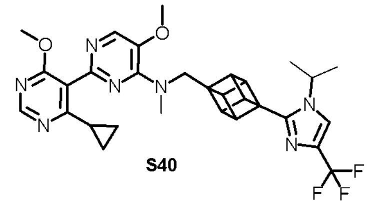
S40,
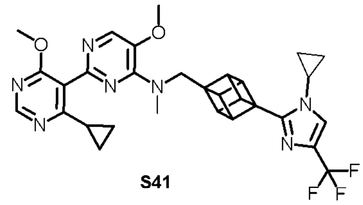
S41,
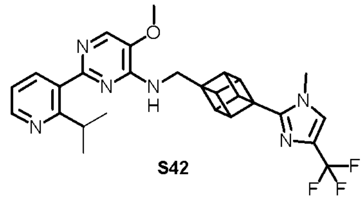
S42,
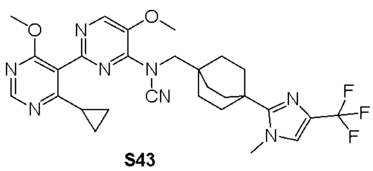
S43,
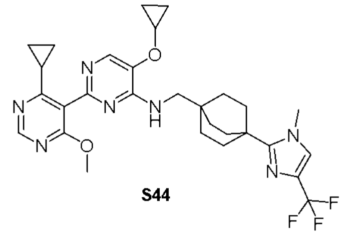
S44,
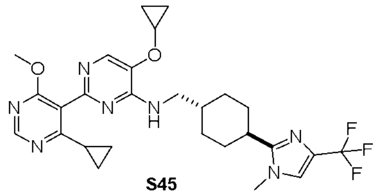
S45,
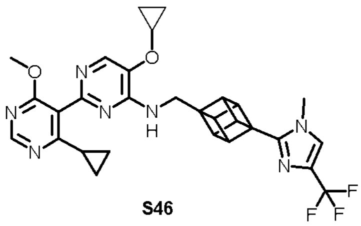
S46,
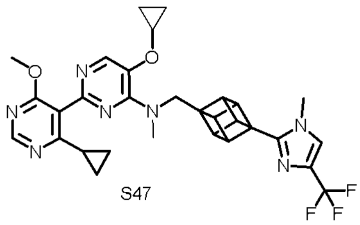
S47,
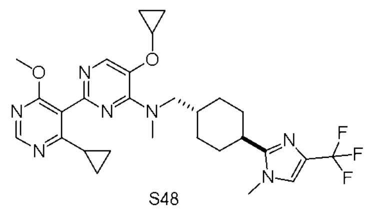
S48,
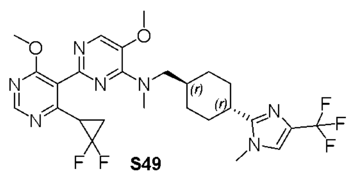
S49,
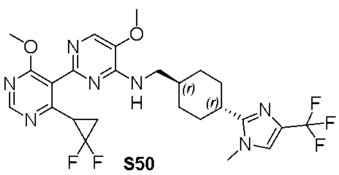
S50,
and
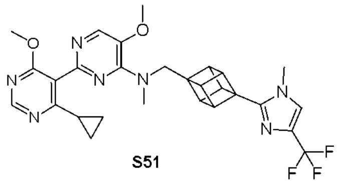
S51.

**10.** A method for preparing the compound according to claim 1, comprising:

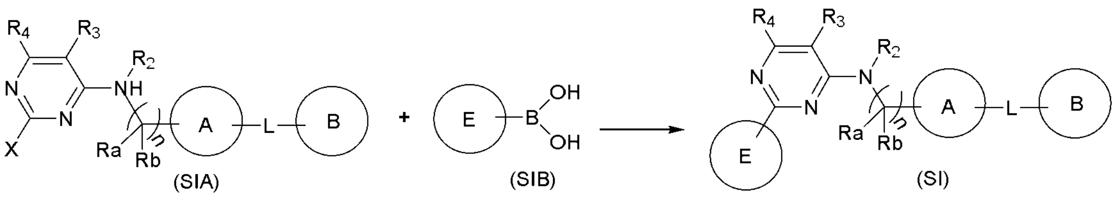
(SIA) (SIB) (SI)

reacting a compound of formula (SIA) with a compound of formula (SIB) to obtain the compound of formula (SI); X is a leaving group selected from the group consisting of halogen, sulfonate, boronic acid and borate; and ring A, ring B, ring E, L, $R_a$, $R_b$, $R_2$ to $R_4$ and n are as defined in claim 1;

or

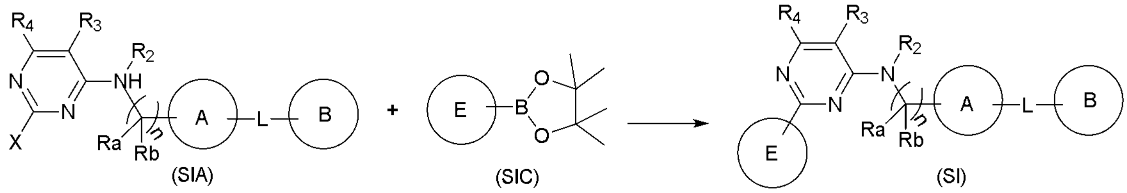

(SIA) (SIC) (SI)

reacting a compound of formula (SIA) with a compound of formula (SIC) to obtain the compound of formula (SI); X is a leaving group selected from the group consisting of halogen, sulfonate, boronic acid and borate; and ring A, ring B, ring E, L, $R_a$, $R_b$, $R_2$ to $R_4$ and n are as defined in claim 1.

**11.** The method according to claim 10, wherein the compound of formula (SI) is a compound of formula (SII), and the method comprises:

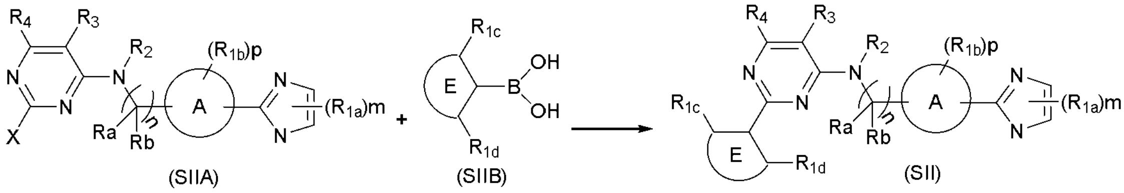

(SIIA) (SIIB) (SII)

reacting a compound of formula (SIIA) with a compound of formula (SIIB) to obtain the compound of formula (SII); X is a leaving group selected from the group consisting of halogen, sulfonate, boronic acid and borate; and ring A, ring B, ring E, $R_a$, $R_b$, $R_2$ to $R_4$, $R_{1a}$, $R_{1b}$, $R_{1c}$, $R_{1d}$, m, n and p are as defined in claim 3;

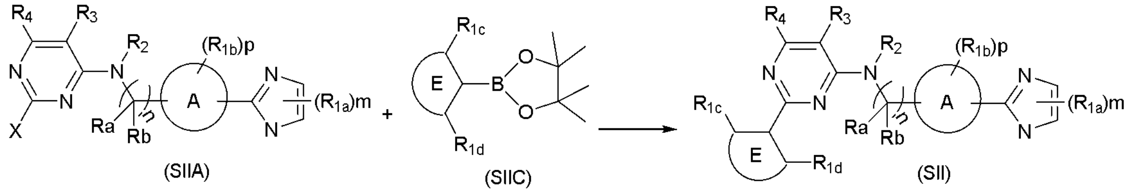

(SIIA) (SIIC) (SII)

reacting a compound of formula (SIIA) with a compound of formula (SIIC) to obtain the compound of formula (SII); X is a leaving group selected from the group consisting of halogen, sulfonate, boronic acid and borate; and ring A, ring B, ring E, $R_a$, $R_b$, $R_2$ to $R_4$, $R_{1a}$, $R_{1b}$, $R_{1c}$, $R_{1d}$, m, n and p are as defined in claim 3.

**12.** A pharmaceutical composition comprising the compound according to any one of claims 1 to 9, and one or more pharmaceutically acceptable excipients.

**13.** A use of the compound according to any one of claims 1 to 9 or the pharmaceutical composition according to claim 12 in the preparation of a medicament for treating or preventing diseases or conditions associated with inhibition of ubiquitin-specific protease 1 (USP1).

**14.** A use of the compound according to any one of claims 1 to 9 or the pharmaceutical composition according to claim 12 in the preparation of a medicament for treating or preventing a cancer,
specifically, the cancer being selected from the group consisting of lung cancer, non-small cell lung cancer (NSCLC), colon cancer, bladder cancer, osteosarcoma, ovarian cancer, skin cancer, and breast cancer.

## INTERNATIONAL SEARCH REPORT

International application No.
**PCT/CN2024/101346**

### A. CLASSIFICATION OF SUBJECT MATTER

C07D403/14(2006.01)i; C07D401/12(2006.01)i; C07D401/14(2006.01)i; C07D403/12(2006.01)i; C07D498/04(2006.01)i; C07D487/04(2006.01)i; C07D491/048(2006.01)i; C07D239/47(2006.01)i; C07D413/14(2006.01)i; C07D493/08(2006.01)i; A61K31/506(2006.01)i; A61K31/5383(2006.01)i; A61K31/519(2006.01)i; A61K31/5377(2006.01)i; A61P35/00(2006.01)i

According to International Patent Classification (IPC) or to both national classification and IPC

### B. FIELDS SEARCHED

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNKI; VEN; CNTXT; CNABS; WOTXT; STN CAPLUS; REGISTRY; EPTXT; USTXT; 万方, WANFANG: 江苏亚虹, 抑制剂, 肿瘤, 癌症, 泛素特异性蛋白酶, 嘧啶, 吡啶, 咪唑, 吡唑, USP1, inhibitor, cancer, tumor, tumour, pyrimidin+, pyrid+, imidazol, pyrazol, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| E | CN 118660872 A (JIANGSU YAHONG MEDITECH CO., LTD. et al.) 17 September 2024 (2024-09-17)<br>claims 1-15 | 1-14 |
| E | WO 2024153175 A1 (LAEKNA THERAPEUTICS SHANGHAI CO., LTD.) 25 July 2024 (2024-07-25)<br>claims 1-59 | 1-14 |
| PX | CN 116496252 A (JIANGSU YAHONG MEDITECH CO., LTD. et al.) 28 July 2023 (2023-07-28)<br>claims 1-15 | 1-14 |
| PX | CN 118184658 A (SHANGHAI QILU PHARMACEUTICAL RESEARCH AND DEVELOPMENT CENTRE LTD.) 14 June 2024 (2024-06-14)<br>embodiments 16 and 30, and claims 18-20 | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 September 2024** | **04 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/101346

C. DOCUMENTS CONSIDERED TO BE RELEVANT

| Category* | Citation of document, with indication, where appropriate, of the relevant passages | Relevant to claim No. |
|---|---|---|
| PX | WO 2023208130 A1 (JIANGSU YAHONG MEDITECH CO., LTD. et al.) 02 November 2023 (2023-11-02)<br>claims 1-3 and 6-15 | 1-14 |
| PX | WO 2024094170 A1 (SHENZHEN JINGTAI TECHNOLOGY CO., LTD.) 10 May 2024 (2024-05-10)<br>claims 1-25 | 1-14 |
| PX | WO 2024078436 A1 (HAINAN SIMCERE ZAIMING PHARMACEUTICAL CO., LTD.) 18 April 2024 (2024-04-18)<br>embodiment 14, compound 22B, and claims 13-15 | 1-14 |
| PX | WO 2024041634 A1 (SIMCERE ZAIMING PHARMACEUTICAL CO., LTD.) 29 February 2024 (2024-02-29)<br>embodiment 1, compound 1j, and claims 18-21 | 1-14 |
| PX | WO 2024022266 A1 (GUANGDONG NEWOPP BIOPHARMACEUTICALS CO., LTD.) 01 February 2024 (2024-02-01)<br>description, page 79, steps 3 and 4, intermediate compounds | 1-8 |
| PX | WO 2024006879 A1 (ZENTAUR THERAPEUTICS USA INC.) 04 January 2024 (2024-01-04)<br>description, page 140, I-24, step 2, compounds; page 143, I-25, step 5, compounds; page 140, I-24, step 2, compounds; page 147, I-27, step 4, compounds; page 153, I-32, step 2-3, compounds; page 179, I-50, step 4, compounds; and page 293, I-135, step 5-7, compounds | 1-8 |
| PX | CN 117003757 A (SUZHOU PUHE PHARMACEUTICAL TECHNOLOGY CO., LTD.) 07 November 2023 (2023-11-07)<br>description, page 48, intermediates d9 and d10 | 1-8 |
| PX | WO 2023148643 A1 (AURIGENE ONCOLOGY LTD.) 10 August 2023 (2023-08-10)<br>description, pages 87-88, intermediates I-165 to I-168 | 1-8 |
| PX | 美国化学会社 (American Chemical Society). "compound structure, and CAS registration number"<br>*STN REGISTRY database*, 21 December 2023 (2023-12-21),<br>compound CAS registration number 3022124-11-1, etc., see attachment for details | 1-2, 4, 6-8 |
| X | CN 113164485 A (KSQ THERAPEUTICS, INC.) 23 July 2021 (2021-07-23)<br>description, page 322, and page 276, and claims 27-34 | 1-14 |
| X | US 2015344443 A1 (US HEALTH et al.) 03 December 2015 (2015-12-03)<br>description, page 11, and embodiments 10-11 | 1-14 |
| X | DEXHEIMER, Thomas S. et al. "Synthesis and Structure-Activity Relationship Studies of N-Benzyl-2-phenylpyrimidin-4-amine Derivatives as Potent USP1/UAF1 Deubiquitinase Inhibitors with Anticancer Activity against Nonsmall Cell Lung Cancer"<br>*Cancer, Journal of Medicinal Chemistry,*<br>Vol. 57, No. 19, 31 December 2014 (2014-12-31), 8099-8110<br>tables 3-5 | 1-14 |
| X | WO 2023083297 A1 (INSILICO MEDICINE IP LTD.) 19 May 2023 (2023-05-19)<br>description, page 140, intermediate 3 | 1-8 |
| X | WO 2023083285 A1 (INSILICO MEDICINE IP LTD.) 19 May 2023 (2023-05-19)<br>description, page 135, paragraph 352, compounds; and page 137, paragraph 361, compounds | 1-8 |
| X | WO 2023030295 A1 (SIMCERE ZAIMING PHARMACEUTICAL CO., LTD.) 09 March 2023 (2023-03-09)<br>page 20, embodiment 3, compound 3E | 1-8 |
| X | WO 2022228399 A1 (SHANDONG XUANZHU PHARMA CO., LTD.) 03 November 2022 (2022-11-03)<br>page 41, step 7, compounds | 1-8 |

Form PCT/ISA/210 (second sheet) (July 2022)

**INTERNATIONAL SEARCH REPORT**

International application No.
**PCT/CN2024/101346**

**C. DOCUMENTS CONSIDERED TO BE RELEVANT**

| Category* | Citation of document, with indication, where appropriate, of the relevant passages | Relevant to claim No. |
|---|---|---|
| X | WO 2022216820 A1 (FORMA THERAPEUTICS INC.) 13 October 2022 (2022-10-13) page 52, embodiment 3, step 2, compounds; page 54, embodiment 4, step 3, compounds; page 57, embodiment 6, steps 2 and 3, compounds; page 60, embodiment 7, step 3, compounds; page 62, embodiment 8, step 2, compounds; page 70, embodiment 12, step 3, compounds; page 71, embodiment 13, step 1, compounds; and page 73, embodiment 14, step 2, compounds | 1-8 |
| X | WO 2022214053 A1 (HAINAN SPARKLE THERAPEUTICS CO., LTD.) 13 October 2022 (2022-10-13) page 46, embodiment 25, compounds 27C and 27D; page 59, embodiment 36, compound 39G; page 61, embodiment 39, compounds 35C and 42A; page 70, embodiment 48, compound 51K; page 75, embodiment 55, compound 58D; page 77, embodiment 56, compound 59A; and page 78, embodiment 57, compound 60A | 1-8 |
| X | CN 103649074 A (MERCK PATENT GMBH) 19 March 2014 (2014-03-19) description, pages 27-28, compounds 4 and 5; pages 30-31, compounds 10-11; page 35, compound 20; and page 39, compound 28; and claims 5-11 | 1-14 |
| X | CN 101490038 A (NOVARTIS AG) 22 July 2009 (2009-07-22) embodiments 1.22, 1.43, and 1.44, compounds; and claims 7-9 | 1-14 |
| X | 美国化学会社 (American Chemical Society). "compound structure, and CAS registration number" *STN REGISTRY database,* 21 April 2023 (2023-04-21), compound CAS registration number 3022124-11-1, etc., see attachment for details | 1-2, 4, 6-8 |

Form PCT/ISA/210 (second sheet) (July 2022)

**INTERNATIONAL SEARCH REPORT**
**Information on patent family members**

International application No.
**PCT/CN2024/101346**

| Patent document cited in search report | | | Publication date (day/month/year) | Patent family member(s) | | | Publication date (day/month/year) |
|---|---|---|---|---|---|---|---|
| CN | 118660872 | A | 17 September 2024 | WO | 2023208130 | A1 | 02 November 2023 |
| | | | | TW | 202400576 | A | 01 January 2024 |
| WO | 2024153175 | A1 | 25 July 2024 | None | | | |
| CN | 116496252 | A | 28 July 2023 | WO | 2023208130 | A1 | 02 November 2023 |
| | | | | TW | 202400576 | A | 01 January 2024 |
| CN | 118184658 | A | 14 June 2024 | None | | | |
| WO | 2023208130 | A1 | 02 November 2023 | None | | | |
| WO | 2024094170 | A1 | 10 May 2024 | None | | | |
| WO | 2024078436 | A1 | 18 April 2024 | None | | | |
| WO | 2024041634 | A1 | 29 February 2024 | None | | | |
| WO | 2024022266 | A1 | 01 February 2024 | None | | | |
| WO | 2024006879 | A1 | 04 January 2024 | US | 2024092779 | A1 | 21 March 2024 |
| CN | 117003757 | A | 07 November 2023 | WO | 2023216910 | A1 | 16 November 2023 |
| WO | 2023148643 | A1 | 10 August 2023 | None | | | |
| CN | 113164485 | A | 23 July 2021 | BR | 112021010715 | A2 | 16 November 2021 |
| | | | | EP | 3897652 | A1 | 27 October 2021 |
| | | | | EP | 3897652 | A4 | 14 September 2022 |
| | | | | JP | 2022511515 | A | 31 January 2022 |
| | | | | WO | 2020132269 | A1 | 25 June 2020 |
| | | | | CL | 2021001575 | A1 | 03 June 2022 |
| | | | | KR | 20210105887 | A | 27 August 2021 |
| | | | | SG | 11202106232 | TA | 29 July 2021 |
| | | | | TW | 202421630 | A | 01 June 2024 |
| | | | | CA | 3122108 | A1 | 25 June 2020 |
| | | | | MX | 2021007179 | A | 28 September 2021 |
| | | | | US | 2021115049 | A1 | 22 April 2021 |
| | | | | US | 11485736 | B2 | 01 November 2022 |
| | | | | US | 2023203046 | A1 | 29 June 2023 |
| | | | | US | 11787813 | B2 | 17 October 2023 |
| | | | | TW | 202039501 | A | 01 November 2020 |
| | | | | TWI | 834784 | B | 11 March 2024 |
| | | | | CO | 2021009078 | A2 | 29 October 2021 |
| | | | | IL | 284050 | A | 31 August 2021 |
| | | | | AU | 2019405925 | A1 | 29 July 2021 |
| US | 2015344443 | A1 | 03 December 2015 | JP | 2016504365 | A | 12 February 2016 |
| | | | | WO | 2014105952 | A2 | 03 July 2014 |
| | | | | WO | 2014105952 | A3 | 09 October 2014 |
| | | | | US | 9802904 | B2 | 31 October 2017 |
| | | | | EP | 2938610 | A2 | 04 November 2015 |
| | | | | AU | 2013370417 | A1 | 16 July 2015 |
| | | | | CA | 2896731 | A1 | 03 July 2014 |
| WO | 2023083297 | A1 | 19 May 2023 | CO | 2024006279 | A2 | 30 May 2024 |
| | | | | AR | 127645 | A1 | 14 February 2024 |
| | | | | KR | 20240101680 | A | 02 July 2024 |
| | | | | IL | 312641 | A | 01 July 2024 |
| | | | | TW | 202327602 | A | 16 July 2023 |
| | | | | EP | 4430041 | A1 | 18 September 2024 |
| | | | | AU | 2022384416 | A1 | 16 May 2024 |

Form PCT/ISA/210 (patent family annex) (July 2022)

**INTERNATIONAL SEARCH REPORT**
**Information on patent family members**

International application No.
**PCT/CN2024/101346**

| Patent document cited in search report | Publication date (day/month/year) | Patent family member(s) | Publication date (day/month/year) |
|---|---|---|---|
| WO 2023083285 A1 | 19 May 2023 | AU 2022387669 A1 | 16 May 2024 |
| | | CA 3235603 A1 | 19 May 2023 |
| | | KR 20240117555 A | 01 August 2024 |
| | | CO 2024006278 A2 | 30 May 2024 |
| | | EP 4430049 A1 | 18 September 2024 |
| | | PE 20241350 A1 | 03 July 2024 |
| | | IL 312640 A | 01 July 2024 |
| WO 2023030295 A1 | 09 March 2023 | None | |
| WO 2022228399 A1 | 03 November 2022 | None | |
| WO 2022216820 A1 | 13 October 2022 | CL 2023002956 A1 | 08 March 2024 |
| | | MA 62912 A1 | 31 May 2024 |
| | | KR 20230167071 A | 07 December 2023 |
| | | MX 2023011709 A | 12 October 2023 |
| | | AU 2022254062 A1 | 12 October 2023 |
| | | CA 3214040 A1 | 13 October 2022 |
| | | BR 112023019075 A2 | 17 October 2023 |
| | | EP 4319758 A1 | 14 February 2024 |
| | | IL 307157 A | 01 November 2023 |
| | | JP 2024514322 A | 01 April 2024 |
| | | US 2024239808 A1 | 18 July 2024 |
| WO 2022214053 A1 | 13 October 2022 | CA 3213709 A1 | 13 October 2022 |
| | | TW 202304933 A | 01 February 2023 |
| | | JP 2024513554 A | 26 March 2024 |
| | | BR 112023020442 A2 | 21 November 2023 |
| | | KR 20230170658 A | 19 December 2023 |
| | | AU 2022253524 A1 | 12 October 2023 |
| | | EP 4321515 A1 | 14 February 2024 |
| | | US 2024226107 A1 | 11 July 2024 |
| CN 103649074 A | 19 March 2014 | IL 230218 A | 29 December 2016 |
| | | CA 2840883 A1 | 10 January 2013 |
| | | CA 2840883 C | 16 July 2019 |
| | | AU 2012280725 A1 | 20 February 2014 |
| | | AU 2012280725 B2 | 02 February 2017 |
| | | JP 2014520767 A | 25 August 2014 |
| | | JP 6116554 B2 | 19 April 2017 |
| | | WO 2013004332 A1 | 10 January 2013 |
| | | US 2014221366 A1 | 07 August 2014 |
| | | US 9199962 B2 | 01 December 2015 |
| | | ES 2698847 T3 | 06 February 2019 |
| | | EP 2729459 A1 | 14 May 2014 |
| | | EP 2729459 B1 | 22 August 2018 |
| CN 101490038 A | 22 July 2009 | EP 1878733 A1 | 16 January 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Synthesis and Structure-Activity Relationship Studies of N-Benzyl-2-phenylpyrimidin-4-amine Derivatives as Potent USP1/UAF1 Deubiquitinase Inhibitors with Anticancer Activity against Nonsmall Cell Lung Cancer. *J. Med. Chem.*, 2014, vol. 57, 8099-8110
- **CUI S-Z** ; **LEI Z-Y** ; **GUAN T-P et al.** Targeting USP1-dependent KDM4A protein stability as a potential prostate cancer therapy. *Cancer Sci.*, 2020, vol. 00, 1-15